# EUROPEAN PATENT APPLICATION

(11) **EP 2 960 335 A1**
(43) Date of publication of application: **30.12.2015**
(21) Application number: 15175601.2
(22) Date of filing: 26.11.2009
(51) Int. Cl.: C12N 15/82

(54) **PLANTS HAVING ENHANCED ABIOTIC STRESS TOLERANCE AND/OR ENHANCED YIELD-RELATED TRAITS AND A METHOD FOR MAKING THE SAME**

(30) Priority: 03.12.2008 EP 08170613; 03.12.2008 EP 08170618; 10.12.2008 EP 08171212; 10.12.2008 EP 08171237; 11.12.2008 US 121570 P; 11.12.2008 US 121577 P; 11.12.2008 US 121572 P; 11.12.2008 US 121571 P; 17.12.2008 EP 08172034; 17.12.2008 EP 08172033; 17.12.2008 EP 08172038; 19.12.2008 US 138972 P; 19.12.2008 US 138965 P; 19.12.2008 US 138970 P
(62) Divisional of application: 09760151.2
(71) Applicant: BASF Plant Science GmbH, 67056 Ludwigshafen (DE)
(72) Inventor: Hatzfeld, Yves, 59000 Lille (FR); Frankard, Valerie, 1410 Waterloo (BE); Reuzeau, Christophe, 24350 La Chapelle Gonaguet (FR); Sanz Molinero, Ana Isabel, 9050 Gentbrugge (BE); Bruynseels, Koen, 9269 Wichelen (BE)
(74) Representative: Mistry, Meeta

(57) **Abstract**

The present invention relates generally to the field of molecular biology and concerns a method for improving various plant growth characteristics by modulating expression in a plant of a nucleic acid encoding an ARP6 (Actin Related Protein 6). The present invention also concerns plants having modulated expression of a nucleic acid encoding an ARP, which plants have improved growth characteristics relative to corresponding wild type plants or other control plants. The invention also provides constructs useful in the methods of the invention.

SEQUENCE LISTING

<110>  BASF Plant Science GmbH

<120>  Plants having enhanced abiotic stress tolerance and/or enhanced
       yield-related traits and a method for making the same

<130>  PF61492_PCT

<160>  213

<170>  PatentIn version 3.3

<210>  1
<211>  973
<212>  DNA
<213>  Allium cepa

<400>  1
ctcatttctc cttcctcatc tttcttcacc taaacccaaa cccttcctgt aaaaatttca      60

attctccctc tcctactttc cataccgtaa aatggacgga aattcagtgc cttataaccc     120

tagaacagtc gaggaagttt ttggggattt caaagccagg agagccggca tgattaaagc     180

gctcaccgcc gatgtggagg acttttacca gcagtgcgac ccagataagg aaaatttgtg     240

cctttatgga cttccgaatg ggaattggga agttactctg cctgcagagg aggttccccc     300

tgagcttcct gagcctgcat tgggcatcaa ttttgccaga gatggaatgc aagagaaaga     360

ttggttatcc ttagttgctg tgcacagtga tgcttggttg ctttccgttg cattttattt     420

tggagctaga tttggattcg acaagtcgga cagaaagcgt ctttttggga tgatgaatga     480

cttacctact atatttgaag tagtgaatgg aaagagcaaa gccaaaaccc ctagcaccaa     540

caaccatagc aacaacaagc ccaagtcgac cattaccaca aaagcgaagt catcggagtt     600

ccaatcgaag caaacaaaac cgacctacat ccctaaagac gaggaagaag aagaggatga     660

tgaagccatc gacgaagtcg agaacgagga tgcagaccac ggagaagacg aagagaacgg     720

aacacccaat atgcggtgtg tgcggggaca ctacgcatcg gacgagttct ggatctgctg     780

cgacatctgc aagaagtggt tccacggcaa gtgcgttaag ataaccccag cccgtgcaga     840

gcacatcaag cagtacaaat gcccttcctg cagcaccagc aacaagcgcg ctcgccctac     900

ttgaaaaatg cccaaagtgc ttaaaagccc tcagtgcttt tgggtgggat cataacgtgc     960

tttttatttg tgt                                                        973

<210>  2
<211>  270
<212>  PRT
<213>  Allium cepa

<400>  2

Met Asp Gly Asn Ser Val Pro Tyr Asn Pro Arg Thr Val Glu Glu Val
1               5                   10                  15

Phe Gly Asp Phe Lys Ala Arg Arg Ala Gly Met Ile Lys Ala Leu Thr
            20                  25                  30

Ala Asp Val Glu Asp Phe Tyr Gln Gln Cys Asp Pro Asp Lys Glu Asn
        35                  40                  45

Leu Cys Leu Tyr Gly Leu Pro Asn Gly Asn Trp Glu Val Thr Leu Pro
    50                  55                  60

Ala Glu Glu Val Pro Pro Glu Leu Pro Glu Pro Ala Leu Gly Ile Asn
65                  70                  75                  80

Phe Ala Arg Asp Gly Met Gln Glu Lys Asp Trp Leu Ser Leu Val Ala
                85                  90                  95

Val His Ser Asp Ala Trp Leu Leu Ser Val Ala Phe Tyr Phe Gly Ala
            100                 105                 110

Arg Phe Gly Phe Asp Lys Ser Asp Arg Lys Arg Leu Phe Gly Met Met
        115                 120                 125

Asn Asp Leu Pro Thr Ile Phe Glu Val Val Asn Gly Lys Ser Lys Ala
    130                 135                 140

Lys Thr Pro Ser Thr Asn Asn His Ser Asn Asn Lys Pro Lys Ser Thr
145                 150                 155                 160

Ile Thr Thr Lys Ala Lys Ser Ser Glu Phe Gln Ser Lys Gln Thr Lys
                165                 170                 175

Pro Thr Tyr Ile Pro Lys Asp Glu Glu Glu Glu Asp Asp Glu Ala
            180                 185                 190

Ile Asp Glu Val Glu Asn Glu Asp Ala Asp His Gly Glu Asp Glu Glu
            195                 200                 205

Asn Gly Thr Pro Asn Met Arg Cys Val Arg Gly His Tyr Ala Ser Asp
        210                 215                 220

Glu Phe Trp Ile Cys Cys Asp Ile Cys Lys Lys Trp Phe His Gly Lys
225 230 235 240

Cys Val Lys Ile Thr Pro Ala Arg Ala Glu His Ile Lys Gln Tyr Lys
245 250 255

Cys Pro Ser Cys Ser Thr Ser Asn Lys Arg Ala Arg Pro Thr

260                 265                 270

<210>  3
<211>  1276
<212>  DNA
<213>  Hordeum vulgare

<400>  3
cggcacgagg gctccccccg gctgttctaa aaagccttgc tactcgcctc ggccagtcgc      60

ctccgccacc aaaaccctaa ccctagcccc ggagctcagg cgacactcgc gcgccccgaa     120

tggacggagg agggacgcat cgcacgccgg aggacgtgtt cagggatttc cgcgcgcggc     180

gggccggcat gattaaggcg ctcaccaccg atgtggagaa gttctaccag cagtgcgacc     240

cagagaaaga gaatctgtgt ctgtatggtc ttcccaatga aacatgggaa gtgaacttgc     300

ctgcagagga ggttccccca gaacttccag agccagcact gggaattaat tttgctcggg     360

atgggatgga tgagaaagat tggttgtcac ttgttgcggt gcacagtgat gcctggttgt     420

tagcagtatc cttctacttt ggagcaagat tcgggtttga caaagaatcc aggaaacggc     480

tttttagcat gataaacaac ctccccacca tatatgaggt tgtcaccgga actgcgaaga     540

agcaggtcaa agaaaaacac cccaaaagca gcagcaagat aaataaatct ggcactaagc     600

catctcgcca gccagaaccc aactcaaggg gtccaaagat gccactacct ccgaaggatg     660

aggatgacag tggaggcgag gaagaagagg gagaagaaca cgaaaaggca ttatgtggtg     720

cgtgtaacga taactatggg caggatgaat tctggatctg ttgtgatgct tgcgagacat     780

ggttccacgg taagtgtgtg aagatcactc ctgccaaagc tgagcacatc aagcactaca     840

aatgcccgaa ttgcagcagc agtagcaaga gggccagagc atgatatcgg attcctccac     900

ggctcatgtg tgaagatcca agaatttgac ttactgtaaa gacgcatgtg ggacggaatg     960

cccaatgtgg atgtgcattc agttgttgct ttgagattag taccagcctt aggggtcttg    1020

ctgtgttgtt attcggtgta tgtcggtgat gctctagatt tacttgtagc cgtagcaact    1080

aattgtaaca gcttgtatgt cttgggggcc ttgtcagtgc cctgttctgg actcttggtg    1140

tcctagttcc agtgatgtat catgtcatca tgtacccttg ttgtaatttg ttggagaatc    1200

gcctgttatc ttagctttta cagcctagtt atttggacat ggtatccact gtagcatata    1260

ccggtagttc cactgt                                                    1276

<210>  4
<211>  254
<212>  PRT
<213>  Hordeum vulgare

<400>  4

Met Asp Gly Gly Gly Thr His Arg Thr Pro Glu Asp Val Phe Arg Asp
1               5                   10                  15

Phe Arg Ala Arg Arg Ala Gly Met Ile Lys Ala Leu Thr Thr Asp Val
            20                  25                  30

Glu Lys Phe Tyr Gln Gln Cys Asp Pro Glu Lys Glu Asn Leu Cys Leu
        35                  40                  45

Tyr Gly Leu Pro Asn Glu Thr Trp Glu Val Asn Leu Pro Ala Glu Glu
    50                  55                  60

Val Pro Pro Glu Leu Pro Glu Pro Ala Leu Gly Ile Asn Phe Ala Arg
65                  70                  75                  80

Asp Gly Met Asp Glu Lys Asp Trp Leu Ser Leu Val Ala Val His Ser
                85                  90                  95

Asp Ala Trp Leu Leu Ala Val Ser Phe Tyr Phe Gly Ala Arg Phe Gly
            100                 105                 110

Phe Asp Lys Glu Ser Arg Lys Arg Leu Phe Ser Met Ile Asn Asn Leu
        115                 120                 125

Pro Thr Ile Tyr Glu Val Val Thr Gly Thr Ala Lys Lys Gln Val Lys
    130                 135                 140

Glu Lys His Pro Lys Ser Ser Ser Lys Ile Asn Lys Ser Gly Thr Lys
145                 150                 155                 160

Pro Ser Arg Gln Pro Glu Pro Asn Ser Arg Gly Pro Lys Met Pro Leu
                165                 170                 175

Pro Pro Lys Asp Glu Asp Asp Ser Gly Gly Glu Glu Glu Glu Gly Glu
            180                 185                 190

Glu His Glu Lys Ala Leu Cys Gly Ala Cys Asn Asp Asn Tyr Gly Gln
        195                 200                 205

Asp Glu Phe Trp Ile Cys Cys Asp Ala Cys Glu Thr Trp Phe His Gly
    210                 215                 220

Lys Cys Val Lys Ile Thr Pro Ala Lys Ala Glu His Ile Lys His Tyr
225                 230                 235                 240

Lys Cys Pro Asn Cys Ser Ser Ser Ser Lys Arg Ala Arg Ala
                245                 250

<210> 5

<211>  2194
<212>  DNA
<213>  Oryza sativa

<400>  5
aatccgaaaa gtttctgcac cgttttcacc ccctaactaa caatataggg aacgtgtgct      60

aaatataaaa tgagacctta tatatgtagc gctgataact agaactatgc aagaaaaact     120

catccaccta ctttagtggc aatcgggcta aataaaaaag agtcgctaca ctagtttcgt     180

tttccttagt aattaagtgg gaaaatgaaa tcattattgc ttagaatata cgttcacatc     240

tctgtcatga agttaaatta ttcgaggtag ccataattgt catcaaactc ttcttgaata     300

aaaaaatctt tctagctgaa ctcaatgggt aaagagagag atttttttta aaaaaataga     360

atgaagatat tctgaacgta ttggcaaaga tttaaacata taattatata attttatagt     420

ttgtgcattc gtcatatcgc acatcattaa ggacatgtct tactccatcc caatttttat     480

ttagtaatta aagacaattg acttattttt attatttatc ttttttcgat tagatgcaag     540

gtacttacgc acacactttg tgctcatgtg catgtgtgag tgcacctcct caatacacgt     600

tcaactagca acacatctct aatatcactc gcctatttaa tacatttagg tagcaatatc     660

tgaattcaag cactccacca tcaccagacc acttttaata atatctaaaa tacaaaaaat     720

aattttacag aatagcatga aaagtatgaa acgaactatt taggtttttc acatacaaaa     780

aaaaaaagaa ttttgctcgt gcgcgagcgc caatctccca tattgggcac acaggcaaca     840

acagagtggc tgcccacaga acaacccaca aaaaacgatg atctaacgga ggacagcaag     900

tccgcaacaa ccttttaaca gcaggctttg cggccaggag agaggaggag aggcaaagaa     960

aaccaagcat cctccttctc ccatctataa attcctcccc ccttttcccc tctctatata    1020

ggaggcatcc aagccaagaa gagggagagc accaaggaca cgcgactagc agaagccgag    1080

cgaccgcctt ctcgatccat atcttccggt cgagttcttg gtcgatctct tccctcctcc    1140

acctcctcct cacagggtat gtgcctccct tcggttgttc ttggatttat tgttctaggt    1200

tgtgtagtac gggcgttgat gttaggaaag gggatctgta tctgtgatga ttcctgttct    1260

tggatttggg atagaggggt tcttgatgtt gcatgttatc ggttcggttt gattagtagt    1320

atggttttca atcgtctgga gagctctatg gaaatgaaat ggtttaggga tcggaatctt    1380

gcgattttgt gagtaccttt tgtttgaggt aaaatcagag caccggtgat tttgcttggt    1440

gtaataaagt acggttgttt ggtcctcgat tctggtagtg atgcttctcg atttgacgaa    1500

gctatccttt gtttattccc tattgaacaa aaataatcca actttgaaga cggtcccgtt    1560

gatgagattg aatgattgat tcttaagcct gtccaaaatt tcgcagctgg cttgtttaga    1620

tacagtagtc cccatcacga aattcatgga aacagttata atcctcagga acaggggatt    1680

ccctgttctt ccgatttgct tttagtccag aatttttttt cccaaatatc ttaaaaagtc    1740

actttctggt tcagttcaat gaattgattg ctacaaataa tgcttttata gcgttatcct 1800

agctgtagtt cagttaatag gtaatacccc tatagtttag tcaggagaag aacttatccg 1860

atttctgatc tccattttta attatatgaa atgaactgta gcataagcag tattcatttg 1920

gattattttt tttattagct ctcacccctt cattattctg agctgaaagt ctggcatgaa 1980

ctgtcctcaa ttttgttttc aaattcacat cgattatcta tgcattatcc tcttgtatct 2040

acctgtagaa gtttcttttt ggttattcct tgactgcttg attacagaaa gaaatttatg 2100

aagctgtaat cgggatagtt atactgcttg ttcttatgat tcatttcctt tgtgcagttc 2160

ttggtgtagc ttgccacttt caccagcaaa gttc 2194

<210> 6
<211> 54
<212> DNA
<213> Artificial sequence

<220>
<223> primer: prm18922

<400> 6
ggggacaagt ttgtacaaaa aagcaggctt aaacaatgga cggaaattca gtgc 54

<210> 7
<211> 50
<212> DNA
<213> Artificial sequence

<220>
<223> primer: prm18923

<400> 7
ggggaccact ttgtacaaga aagctgggtc tttgggcatt tttcaagtag 50

<210> 8
<211> 52
<212> DNA
<213> Artificial sequence

<220>
<223> primer: prm18924

<400> 8
ggggacaagt ttgtacaaaa aagcaggctt aaacaatgga cggaggaggg ac 52

<210> 9
<211> 49
<212> DNA
<213> Artificial sequence

<220>
<223> primer: prm18925

<400> 9
ggggaccact ttgtacaaga aagctgggta ggaatccgat atcatgctc 49

<210> 10
<211> 1498
<212> DNA
<213> Hordeum vulgare

<220>
<221> misc_feature
<222> (9)..(9)
<223> n is a, c, g, or t

<220>
<221> misc_feature
<222> (1444)..(1444)
<223> n is a, c, g, or t

<220>
<221> misc_feature
<222> (1498)..(1498)
<223> n is a, c, g, or t

<400> 10
ctgaaaggna tagctcagca tcacgcagga atgggcaatc catatggtgt gcctgcttct 60

agtgctcaag tggcctcctt gggaggacta gattttcaag ctttggctgc ttcaggtcaa 120

atccctcctc aagccctcgc agctttgcag gatgagctcc tagggcgacc tacgaacaac 180

ctggtgttgc ctggaaggga ccagtcatct ttacgacttg ctgcagtcaa aggaaataag 240

ccccatggag agcaaatagc atttgggcag ccgatataca aggttcagaa taattcatat 300

gcggcattac cccaaaacag ccctgcagtt ggaagaatgc cttctttttc agcttggccc 360

aataacaaac ttggtatggc agattcaatg agcgcactgg gtaatgtgaa taattctcag 420

aatagcaata taggtttgca cgaattgcaa cagcagccag ataccatgct gtcaggaacc 480

ctccacactc tagatgtcaa accttctggc atagttatgc catctcagtc attaaatact 540

ttcccagcta gcgagggtct ctctcctaat caaaatccct tgattgtacc ctctcaatcg 600

tcaggttatc tgacaggcat tcctccatcc atgaagcctg aacttgttct cccaacttct 660

cagtcatcaa acaatctatt aagtgggatt gatctgatta accaagcttc aaccagtcag 720

cctttcatta gtagccacgg aggaaatctt tctggtctca tgaaccgcaa ctcaaatgtg 780

ataccatcac aaggaataag taactttcaa actggtaata ctccgtatct ggttaatcag 840

aattcgatgg gaatgggctc taaaccaccc ggtgtcctaa agactgagag cactgactct 900

cttaaccaaa gctatggtta tgtcaaccat atggattccg gcttgctgtc ttctcagtca 960

aaaaatgcac aatttggttt tctgcagagt ccaaatgatg tcacaggtgg ctggtcttct 1020

ttgcaaaata tggattgctt tagaaatact gttgggccaa gtcagccagt gtccagttcg 1080

tctagctttc atagttctaa tgcagccctt gggaaattgc ctgatcaagg acgaggaaaa 1140

aaccttggat ttgttgggaa gggtacttgc atcccaaacc gctttgcagt ggatgagatc 1200

gaatctccaa ctaacagctt gagtcacagc attggaagca gtggagacat ccctgacatg 1260

tttgggttta gtggacagat gtgaaggctt cttggatttg taagtagggt aatcactgga 1320

aacagtaggg gtggtagaat gaatgggggc tctagttagg actgggtgcg aattcatgac 1380

gcttgcgtga aaccgtgatg ggatatatag ttgggacgga ttgtggactg agcggcgtta 1440

caanaagacg gattgtatat attaacacat tatatacccc catggaggaa cacaccan 1498

<210> 11
<211> 302
<212> PRT
<213> Hordeum vulgare

<400> 11

Met Ala Asp Ser Met Ser Ala Leu Gly Asn Val Asn Asn Ser Gln Asn
1               5                   10                  15

Ser Asn Ile Gly Leu His Glu Leu Gln Gln Gln Pro Asp Thr Met Leu
            20                  25                  30

Ser Gly Thr Leu His Thr Leu Asp Val Lys Pro Ser Gly Ile Val Met
        35                  40                  45

Pro Ser Gln Ser Leu Asn Thr Phe Pro Ala Ser Glu Gly Leu Ser Pro
    50                  55                  60

Asn Gln Asn Pro Leu Ile Val Pro Ser Gln Ser Ser Gly Tyr Leu Thr
65                  70                  75                  80

Gly Ile Pro Pro Ser Met Lys Pro Glu Leu Val Leu Pro Thr Ser Gln
                85                  90                  95

Ser Ser Asn Asn Leu Leu Ser Gly Ile Asp Leu Ile Asn Gln Ala Ser
            100                 105                 110

Thr Ser Gln Pro Phe Ile Ser Ser His Gly Gly Asn Leu Ser Gly Leu
        115                 120                 125

Met Asn Arg Asn Ser Asn Val Ile Pro Ser Gln Gly Ile Ser Asn Phe
    130                 135                 140

Gln Thr Gly Asn Thr Pro Tyr Leu Val Asn Gln Asn Ser Met Gly Met
145                 150                 155                 160

Gly Ser Lys Pro Pro Gly Val Leu Lys Thr Glu Ser Thr Asp Ser Leu
                165                 170                 175

Asn Gln Ser Tyr Gly Tyr Val Asn His Met Asp Ser Gly Leu Leu Ser
            180                 185                 190

Ser Gln Ser Lys Asn Ala Gln Phe Gly Phe Leu Gln Ser Pro Asn Asp
        195                 200                 205

Val Thr Gly Gly Trp Ser Ser Leu Gln Asn Met Asp Cys Phe Arg Asn
    210                 215                 220

Thr Val Gly Pro Ser Gln Pro Val Ser Ser Ser Ser Ser Phe His Ser
225                 230                 235                 240

Ser Asn Ala Ala Leu Gly Lys Leu Pro Asp Gln Gly Arg Gly Lys Asn
                245                 250                 255

Leu Gly Phe Val Gly Lys Gly Thr Cys Ile Pro Asn Arg Phe Ala Val
            260                 265                 270

Asp Glu Ile Glu Ser Pro Thr Asn Ser Leu Ser His Ser Ile Gly Ser
        275                 280                 285

Ser Gly Asp Ile Pro Asp Met Phe Gly Phe Ser Gly Gln Met
    290                 295                 300

<210>  12
<211>  1498
<212>  DNA
<213>  Hordeum vulgare

<220>
<221>  misc_feature
<222>  (9)..(9)
<223>  n is a, c, g, or t

<220>
<221>  misc_feature
<222>  (1444)..(1444)
<223>  n is a, c, g, or t

<220>
<221>  misc_feature
<222>  (1498)..(1498)
<223>  n is a, c, g, or t

<400>  12
ctgaaaggna tagctcagca tcacgcagga atgggcaatc catatggtgt gcctgcttct      60

agtgctcaag tggcctcctt gggaggacta gattttcaag ctttggctgc ttcaggtcaa     120

atccctcctc aagccctcgc agctttgcag gatgagctcc tagggcgacc tacgaacaac     180

ctggtgttgc ctggaaggga ccagtcatct ttacgacttg ctgcagtcaa aggaaataag     240

ccccatggag agcaaatagc atttgggcag ccgatataca aggttcagaa taattcatat     300

gcggcattac cccaaaacag ccctgcagtt ggaagaatgc cttctttttc agcttggccc 360

aataacaaac ttggtatggc agattcaatg agcgcactgg gtaatgtgaa taattctcag 420

aatagcaata taggtttgca cgaattgcaa cagcagccag ataccatgct gtcaggaacc 480

ctccacactc tagatgtcaa accttctggc atagttatgc catctcagtc attaaatact 540

ttcccagcta gcgagggtct ctctcctaat caaaatccct tgattgtacc ctctcaatcg 600

tcaggttatc tgacaggcat tcctccatcc atgaagcctg aacttgttct cccaacttct 660

cagtcatcaa acaatctatt aagtgggatt gatctgatta accaagcttc aaccagtcag 720

cctttcatta gtagccacgg aggaaatctt tctggtctca tgaaccgcaa ctcaaatgtg 780

ataccatcac aaggaataag taactttcaa actggtaata ctccgtatct ggttaatcag 840

aattcgatgg gaatgggctc taaaccaccc ggtgtcctaa agactgagag cactgactct 900

cttaaccaaa gctatggtta tgtcaaccat atggattccg gcttgctgtc ttctcagtca 960

aaaaatgcac aatttggttt tctgcagagt ccaaatgatg tcacaggtgg ctggtcttct 1020

ttgcaaaata tggattgctt tagaaatact gttgggccaa gtcagccagt gtccagttcg 1080

tctagctttc atagttctaa tgcagccctt gggaaattgc ctgatcaagg acgaggaaaa 1140

aaccttggat ttgttgggaa gggtacttgc atcccaaacc gctttgcagt ggatgagatc 1200

gaatctccaa ctaacagctt gagtcacagc attggaagca gtggagacat ccctgacatg 1260

tttgggttta gtggacagat gtgaaggctt cttggatttg taagtagggt aatcactgga 1320

aacagtaggg gtggtagaat gaatgggggc tctagttagg actgggtgcg aattcatgac 1380

gcttgcgtga aaccgtgatg ggatatatag ttgggacgga ttgtggactg agcggcgtta 1440

caanaagacg gattgtatat attaacacat tatatacccc catggaggaa cacaccan 1498

<210> 13
<211> 417
<212> PRT
<213> Hordeum vulgare

<400> 13

Met Gly Asn Pro Tyr Gly Val Pro Ala Ser Ser Ala Gln Val Ala Ser
1               5                   10                  15

Leu Gly Gly Leu Asp Phe Gln Ala Leu Ala Ala Ser Gly Gln Ile Pro
            20                  25                  30

Pro Gln Ala Leu Ala Ala Leu Gln Asp Glu Leu Leu Gly Arg Pro Thr
        35                  40                  45

Asn Asn Leu Val Leu Pro Gly Arg Asp Gln Ser Ser Leu Arg Leu Ala
    50                  55                  60

Ala Val Lys Gly Asn Lys Pro His Gly Glu Gln Ile Ala Phe Gly Gln
65                  70                  75                  80

Pro Ile Tyr Lys Val Gln Asn Asn Ser Tyr Ala Ala Leu Pro Gln Asn
                85                  90                  95

Ser Pro Ala Val Gly Arg Met Pro Ser Phe Ser Ala Trp Pro Asn Asn
            100                 105                 110

Lys Leu Gly Met Ala Asp Ser Met Ser Ala Leu Gly Asn Val Asn Asn
        115                 120                 125

Ser Gln Asn Ser Asn Ile Gly Leu His Glu Leu Gln Gln Gln Pro Asp
    130                 135                 140

Thr Met Leu Ser Gly Thr Leu His Thr Leu Asp Val Lys Pro Ser Gly
145                 150                 155                 160

Ile Val Met Pro Ser Gln Ser Leu Asn Thr Phe Pro Ala Ser Glu Gly
                165                 170                 175

Leu Ser Pro Asn Gln Asn Pro Leu Ile Val Pro Ser Gln Ser Ser Gly
            180                 185                 190

Tyr Leu Thr Gly Ile Pro Pro Ser Met Lys Pro Glu Leu Val Leu Pro
        195                 200                 205

Thr Ser Gln Ser Ser Asn Asn Leu Leu Ser Gly Ile Asp Leu Ile Asn
    210                 215                 220

Gln Ala Ser Thr Ser Gln Pro Phe Ile Ser Ser His Gly Gly Asn Leu
225                 230                 235                 240

Ser Gly Leu Met Asn Arg Asn Ser Asn Val Ile Pro Ser Gln Gly Ile
                245                 250                 255

Ser Asn Phe Gln Thr Gly Asn Thr Pro Tyr Leu Val Asn Gln Asn Ser
            260                 265                 270

Met Gly Met Gly Ser Lys Pro Pro Gly Val Leu Lys Thr Glu Ser Thr
        275                 280                 285

Asp Ser Leu Asn Gln Ser Tyr Gly Tyr Val Asn His Met Asp Ser Gly
    290                 295                 300

Leu Leu Ser Ser Gln Ser Lys Asn Ala Gln Phe Gly Phe Leu Gln Ser

305                 310                 315                 320

Pro Asn Asp Val Thr Gly Gly Trp Ser Ser Leu Gln Asn Met Asp Cys
                325                 330                 335

Phe Arg Asn Thr Val Gly Pro Ser Gln Pro Val Ser Ser Ser Ser Ser
            340                 345                 350

Phe His Ser Ser Asn Ala Ala Leu Gly Lys Leu Pro Asp Gln Gly Arg
        355                 360                 365

Gly Lys Asn Leu Gly Phe Val Gly Lys Gly Thr Cys Ile Pro Asn Arg
    370                 375                 380

Phe Ala Val Asp Glu Ile Glu Ser Pro Thr Asn Ser Leu Ser His Ser
385                 390                 395                 400

Ile Gly Ser Ser Gly Asp Ile Pro Asp Met Phe Gly Phe Ser Gly Gln
                405                 410                 415

Met

<210>  14
<211>  2194
<212>  DNA
<213>  Oryza sativa

<400>  14
aatccgaaaa gtttctgcac cgttttcacc ccctaactaa caatataggg aacgtgtgct      60

aaatataaaa tgagacctta tatatgtagc gctgataact agaactatgc aagaaaaact     120

catccaccta ctttagtggc aatcgggcta aataaaaaag agtcgctaca ctagtttcgt     180

tttccttagt aattaagtgg gaaaatgaaa tcattattgc ttagaatata cgttcacatc     240

tctgtcatga agttaaatta ttcgaggtag ccataattgt catcaaactc ttcttgaata     300

aaaaaatctt tctagctgaa ctcaatgggt aaagagagag atttttttta aaaaaataga     360

atgaagatat tctgaacgta ttggcaaaga tttaaacata taattatata attttatagt     420

ttgtgcattc gtcatatcgc acatcattaa ggacatgtct tactccatcc caatttttat     480

ttagtaatta aagacaattg acttattttt attatttatc ttttttcgat tagatgcaag     540

gtacttacgc acacactttg tgctcatgtg catgtgtgag tgcacctcct caatacacgt     600

tcaactagca acacatctct aatatcactc gcctatttaa tacatttagg tagcaatatc     660

tgaattcaag cactccacca tcaccagacc acttttaata atatctaaaa tacaaaaaat     720

aattttacag aatagcatga aaagtatgaa acgaactatt taggtttttc acatacaaaa     780

aaaaaaagaa ttttgctcgt gcgcgagcgc caatctccca tattgggcac acaggcaaca 840

acagagtggc tgcccacaga acaacccaca aaaaacgatg atctaacgga ggacagcaag 900

tccgcaacaa ccttttaaca gcaggctttg cggccaggag agaggaggag aggcaaagaa 960

aaccaagcat cctccttctc ccatctataa attcctcccc ccttttcccc tctctatata 1020

ggaggcatcc aagccaagaa gagggagagc accaaggaca cgcgactagc agaagccgag 1080

cgaccgcctt ctcgatccat atcttccggt cgagttcttg gtcgatctct tccctcctcc 1140

acctcctcct cacagggtat gtgcctccct tcggttgttc ttggatttat tgttctaggt 1200

tgtgtagtac gggcgttgat gttaggaaag gggatctgta tctgtgatga ttcctgttct 1260

tggatttggg atagaggggt tcttgatgtt gcatgttatc ggttcggttt gattagtagt 1320

atggttttca atcgtctgga gagctctatg gaaatgaaat ggtttaggga tcggaatctt 1380

gcgattttgt gagtaccttt tgtttgaggt aaaatcagag caccggtgat tttgcttggt 1440

gtaataaagt acggttgttt ggtcctcgat tctggtagtg atgcttctcg atttgacgaa 1500

gctatccttt gtttattccc tattgaacaa aaataatcca actttgaaga cggtcccgtt 1560

gatgagattg aatgattgat tcttaagcct gtccaaaatt tcgcagctgg cttgtttaga 1620

tacagtagtc cccatcacga aattcatgga aacagttata atcctcagga acaggggatt 1680

ccctgttctt ccgatttgct ttagtcccag aatttttttt cccaaatatc ttaaaaagtc 1740

actttctggt tcagttcaat gaattgattg ctacaaataa tgcttttata gcgttatcct 1800

agctgtagtt cagttaatag gtaatacccc tatagtttag tcaggagaag aacttatccg 1860

atttctgatc tccattttta attatatgaa atgaactgta gcataagcag tattcatttg 1920

gattattttt tttattagct ctcacccctt cattattctg agctgaaagt ctggcatgaa 1980

ctgtcctcaa ttttgttttc aaattcacat cgattatcta tgcattatcc tcttgtatct 2040

acctgtagaa gtttcttttt ggttattcct tgactgcttg attacagaaa gaaatttatg 2100

aagctgtaat cgggatagtt atactgcttg ttcttatgat tcatttcctt tgtgcagttc 2160

ttggtgtagc ttgccacttt caccagcaaa gttc 2194

<210> 15
<211> 54
<212> DNA
<213> Artificial sequence

<220>
<223> primer: prm18898

<400> 15
ggggacaagt ttgtacaaaa aagcaggctt aaacaatggg caatccatat ggtg 54

<210> 16
<211> 50

<212>  DNA
<213>  Artificial sequence

<220>
<223>  primer: prm18899

<400>  16
ggggaccact ttgtacaaga aagctgggta caaatccaag aagccttcac          50


<210>  17
<211>  1089
<212>  DNA
<213>  Populus trichocarpa

<400>  17
atgtttacgt gcatagcgtg taccaagcca gtggcagagg atggacgggg agaagaagga      60

ggagcgcgtg gaagtggtac cccaagtaca aaagaagccg tcaaaagcct cacttcacag     120

atcaaggaca tggcactgaa aatgtctggt gcttacaagc aatgcaagcc ctgcacaagt     180

cccagcagct acaagaaagg acagcgacct taccctgact ttgatgcagc ttcagaaggg     240

gttccatacc cctattttgg aggtggaagc tcaagctcaa ccccagcctg ggactttact     300

actcccaaac acaatcgagg tactagagct gactctaggt tttctacttt gtatggtgga     360

gaccggaccc ctggaggagc agagtcttgt gatgtggtgc tggaggatga ggatgagccc     420

aaggaatgga tggcacaggt ggagccaggt gttcacatta ctttcgtgtc tctccctaat     480

gggggaaatg atctaaagcg tattcgtttc agccgagaga tgtttaataa gtggcaagct     540

cagcgatggt ggggtgagaa ctatgacaga atcacggagc tctataatgt ccagagattt     600

aatcgtcaag ctcttcacac tcccccgagg tgtgaggatg agcaaagaga ttcctcctac     660

tcaaggctgg aatctgcaag ggaaagccct atggctccat cttttacccc aagaaactac     720

tataaacctg ctggaagtaa aggttatttc ccatctgata ctatggacca aggtggcagc     780

catcactacc acgctggttc aagtagctat ggtatggggg ggccaagatt cgaggcatct     840

tctttagagg catcacggac aactacatca tctagagatg agccttctat ttcagttagc     900

aatgctagtg acctggagac agaatgggtt gagcaagatg agccaggggt ttacatcaca     960

atcagacaac ttgccgatgg caccagggag ctcaggcgtg tcagattcag ccgcgaacaa    1020

ttcggagaag tgcacgctaa gacatggtgg gaacagaata gagaaagaat tcaagctcag    1080

tacctataa                                                            1089


<210>  18
<211>  362
<212>  PRT
<213>  Populus trichocarpa

<400>  18

Met Phe Thr Cys Ile Ala Cys Thr Lys Pro Val Ala Glu Asp Gly Arg

1               5                   10                  15

Gly Glu Glu Gly Gly Ala Arg Gly Ser Gly Thr Pro Ser Thr Lys Glu
            20                  25                  30

Ala Val Lys Ser Leu Thr Ser Gln Ile Lys Asp Met Ala Leu Lys Met
        35                  40                  45

Ser Gly Ala Tyr Lys Gln Cys Lys Pro Cys Thr Ser Pro Ser Ser Tyr
    50                  55                  60

Lys Lys Gly Gln Arg Pro Tyr Pro Asp Phe Asp Ala Ala Ser Glu Gly
65                  70                  75                  80

Val Pro Tyr Pro Tyr Phe Gly Gly Gly Ser Ser Ser Ser Thr Pro Ala
                85                  90                  95

Trp Asp Phe Thr Thr Pro Lys His Asn Arg Gly Thr Arg Ala Asp Ser
            100                 105                 110

Arg Phe Ser Thr Leu Tyr Gly Gly Asp Arg Thr Pro Gly Gly Ala Glu
        115                 120                 125

Ser Cys Asp Val Val Leu Glu Asp Glu Asp Glu Pro Lys Glu Trp Met
    130                 135                 140

Ala Gln Val Glu Pro Gly Val His Ile Thr Phe Val Ser Leu Pro Asn
145                 150                 155                 160

Gly Gly Asn Asp Leu Lys Arg Ile Arg Phe Ser Arg Glu Met Phe Asn
                165                 170                 175

Lys Trp Gln Ala Gln Arg Trp Trp Gly Glu Asn Tyr Asp Arg Ile Thr
            180                 185                 190

Glu Leu Tyr Asn Val Gln Arg Phe Asn Arg Gln Ala Leu His Thr Pro
        195                 200                 205

Pro Arg Cys Glu Asp Glu Gln Arg Asp Ser Ser Tyr Ser Arg Leu Glu
    210                 215                 220

Ser Ala Arg Glu Ser Pro Met Ala Pro Ser Phe Thr Pro Arg Asn Tyr
225                 230                 235                 240

Tyr Lys Pro Ala Gly Ser Lys Gly Tyr Phe Pro Ser Asp Thr Met Asp
                245                 250                 255

Gln Gly Gly Ser His His Tyr His Ala Gly Ser Ser Ser Tyr Gly Met
        260                 265                 270

Gly Gly Pro Arg Phe Glu Ala Ser Ser Leu Glu Ala Ser Arg Thr Thr
        275                 280                 285

Thr Ser Ser Arg Asp Glu Pro Ser Ile Ser Val Ser Asn Ala Ser Asp
    290                 295                 300

Leu Glu Thr Glu Trp Val Glu Gln Asp Glu Pro Gly Val Tyr Ile Thr
305                 310                 315                 320

Ile Arg Gln Leu Ala Asp Gly Thr Arg Glu Leu Arg Arg Val Arg Phe
                325                 330                 335

Ser Arg Glu Gln Phe Gly Glu Val His Ala Lys Thr Trp Trp Glu Gln
        340                 345                 350

Asn Arg Glu Arg Ile Gln Ala Gln Tyr Leu
        355                 360

<210>  19
<211>  1035
<212>  DNA
<213>  Arabidopsis thaliana

<400>  19
atgttttctt gcatagcttg taccaaagca gacggaggtg aagaagtcga acatggagcg      60

cgtggaggca ccactcccaa tactaaagaa gccgtcaaaa gcctaaccat tcagattaaa     120

gatatggctt tgaaattctc tggtgcttat aaacaatgca agccatgcac tggttcctct     180

agtagtccct tgaagaaagg acatagatca tttccggatt atgacaatgc ctctgaaggt     240

gttccatacc ctttcatggg tggaagtgct ggttcaactc ctgcttggga cttcacaaac     300

tcctctcatc atccagctgg acggttagaa tcaaagttca cttcaatata tggaaatgat     360

cgagaatcta tctctgcaca gtcttgtgat gttgtactgg atgatgatgg accaaaagag     420

tggatggctc aagtagaacc tggtgttcat atcacatttg cttctcttcc cactggagga     480

aatgatctca aacggatccg tttcagccga gagatgtttg ataagtggca agctcaacgg     540

tggtggggtg agaactatga caagatcgtt gagctttaca atgtacagag atttaaccgc     600

caagctctcc aaacgcctgc aagatccgac gatcagtcac agagagattc aacgtactca     660

aagatggatt cagcaagaga aagcaaagac tggactccaa gacacaattt cagacctcca     720

ggatctgtcc cgcatcactt ttatggcggc tctagcaact atggaccagg aagttatcat     780

ggaggaccac cgatggatgc agcaagaacc acaacttctt ccagagatga tccaccttcc     840

atgagcaatg ctagtgaaat gcaagctgag tggattgaag aggacgagcc tggtgtttac     900

attaccatca gacaattatc agatgggact agagagctac gtcgagtcag attcagccgg 960

gaacggtttg gggaagtgca tgcaaagaca tggtgggagc agaacagaga gagaatacaa 1020

acacagtacc tctaa 1035

<210> 20
<211> 344
<212> PRT
<213> Arabidopsis thaliana

<400> 20

Met Phe Ser Cys Ile Ala Cys Thr Lys Ala Asp Gly Gly Glu Glu Val
1               5                   10                  15

Glu His Gly Ala Arg Gly Gly Thr Thr Pro Asn Thr Lys Glu Ala Val
            20                  25                  30

Lys Ser Leu Thr Ile Gln Ile Lys Asp Met Ala Leu Lys Phe Ser Gly
        35                  40                  45

Ala Tyr Lys Gln Cys Lys Pro Cys Thr Gly Ser Ser Ser Ser Pro Leu
    50                  55                  60

Lys Lys Gly His Arg Ser Phe Pro Asp Tyr Asp Asn Ala Ser Glu Gly
65                  70                  75                  80

Val Pro Tyr Pro Phe Met Gly Gly Ser Ala Gly Ser Thr Pro Ala Trp
                85                  90                  95

Asp Phe Thr Asn Ser Ser His His Pro Ala Gly Arg Leu Glu Ser Lys
            100                 105                 110

Phe Thr Ser Ile Tyr Gly Asn Asp Arg Glu Ser Ile Ser Ala Gln Ser
        115                 120                 125

Cys Asp Val Val Leu Asp Asp Asp Gly Pro Lys Glu Trp Met Ala Gln
    130                 135                 140

Val Glu Pro Gly Val His Ile Thr Phe Ala Ser Leu Pro Thr Gly Gly
145                 150                 155                 160

Asn Asp Leu Lys Arg Ile Arg Phe Ser Arg Glu Met Phe Asp Lys Trp
                165                 170                 175

Gln Ala Gln Arg Trp Trp Gly Glu Asn Tyr Asp Lys Ile Val Glu Leu
            180                 185                 190

Tyr Asn Val Gln Arg Phe Asn Arg Gln Ala Leu Gln Thr Pro Ala Arg
        195                 200                 205

Ser Asp Asp Gln Ser Gln Arg Asp Ser Thr Tyr Ser Lys Met Asp Ser
    210                 215                 220

Ala Arg Glu Ser Lys Asp Trp Thr Pro Arg His Asn Phe Arg Pro Pro
225                 230                 235                 240

Gly Ser Val Pro His His Phe Tyr Gly Gly Ser Ser Asn Tyr Gly Pro
                245                 250                 255

Gly Ser Tyr His Gly Gly Pro Pro Met Asp Ala Ala Arg Thr Thr Thr
            260                 265                 270

Ser Ser Arg Asp Asp Pro Pro Ser Met Ser Asn Ala Ser Glu Met Gln
        275                 280                 285

Ala Glu Trp Ile Glu Glu Asp Glu Pro Gly Val Tyr Ile Thr Ile Arg
    290                 295                 300

Gln Leu Ser Asp Gly Thr Arg Glu Leu Arg Arg Val Arg Phe Ser Arg
305                 310                 315                 320

Glu Arg Phe Gly Glu Val His Ala Lys Thr Trp Trp Glu Gln Asn Arg
                325                 330                 335

Glu Arg Ile Gln Thr Gln Tyr Leu
            340

<210>  21
<211>  996
<212>  DNA
<213>  Arabidopsis thaliana

<400>  21
atgtttactt gcataaattg cactaaaatg gcagacagag gtgaggagga tgaagaagat      60

gaagcgcgtg ggagcaccac tcccaacact aaagaagccg ttaaaagcct aactacacag     120

atcaaagata tggcgtcgaa attctcaggt tctcataaac aaagcaagcc aactccgggc     180

tcttcgagca gcaacttgag gaagtttccg gattttgaca ctgcatcaga gagtgttcca     240

tacccttatc ctggtggaag cacgagctcc actcctgctt gggacttacc aagatcctcc     300

taccatcaat ctggacggcc agactcaaga ttcacatcaa tgtatggcgg tgaacgtgaa     360

tccatctctg ctcagtcgtg tgatgtggta ctagaggatg acgagccaaa ggagtggatg     420

gctcaagtag agcctggtgt tcatatcaca tttgtctcac ttcccagtgg aggaaatgat     480

cttaaacgga tacgtttcag ccgggaggtt ttcgacaagt ggcaggctca gaggtggtgg     540

ggtgagaact atgacagaat agttgagctt tacaatgttc aaagatttaa ccgacaagct 600

cttcaaactc ctggtagatc cgaggaccag tcgcagagag attcaactta cacaagaatt 660

gattcagcaa gagaaagcag agactggaca caaagagaca acaacttcag accaccagga 720

ggcagtgtcc ctcatcattt ttatggacct ccaatggacg cagcaagaat caccacctca 780

tcgagggacg aaccgccttc aatgagtaat gctagtgaaa tgcaaggtga gtgggttgaa 840

gaggacgagc ctggtgtcta cattaccatc agacagttac cagatggaac tagagaacta 900

cgccgcgtca ggttcagtcg ggaacggttt ggggaagtgc atgccaagac atggtgggag 960

cagaacaggg acagaataca aacccaatac ctctaa 996

<210> 22
<211> 331
<212> PRT
<213> Arabidopsis thaliana

<400> 22

Met Phe Thr Cys Ile Asn Cys Thr Lys Met Ala Asp Arg Gly Glu Glu
1               5                   10                  15

Asp Glu Glu Asp Glu Ala Arg Gly Ser Thr Thr Pro Asn Thr Lys Glu
            20                  25                  30

Ala Val Lys Ser Leu Thr Thr Gln Ile Lys Asp Met Ala Ser Lys Phe
        35                  40                  45

Ser Gly Ser His Lys Gln Ser Lys Pro Thr Pro Gly Ser Ser Ser Ser
    50                  55                  60

Asn Leu Arg Lys Phe Pro Asp Phe Asp Thr Ala Ser Glu Ser Val Pro
65                  70                  75                  80

Tyr Pro Tyr Pro Gly Gly Ser Thr Ser Ser Thr Pro Ala Trp Asp Leu
                85                  90                  95

Pro Arg Ser Ser Tyr His Gln Ser Gly Arg Pro Asp Ser Arg Phe Thr
            100                 105                 110

Ser Met Tyr Gly Gly Glu Arg Glu Ser Ile Ser Ala Gln Ser Cys Asp
        115                 120                 125

Val Val Leu Glu Asp Asp Glu Pro Lys Glu Trp Met Ala Gln Val Glu
    130                 135                 140

Pro Gly Val His Ile Thr Phe Val Ser Leu Pro Ser Gly Gly Asn Asp
145                 150                 155                 160

Leu Lys Arg Ile Arg Phe Ser Arg Glu Val Phe Asp Lys Trp Gln Ala
                165                 170                 175

Gln Arg Trp Trp Gly Glu Asn Tyr Asp Arg Ile Val Glu Leu Tyr Asn
            180                 185                 190

Val Gln Arg Phe Asn Arg Gln Ala Leu Gln Thr Pro Gly Arg Ser Glu
        195                 200                 205

Asp Gln Ser Gln Arg Asp Ser Thr Tyr Thr Arg Ile Asp Ser Ala Arg
    210                 215                 220

Glu Ser Arg Asp Trp Thr Gln Arg Asp Asn Asn Phe Arg Pro Pro Gly
225                 230                 235                 240

Gly Ser Val Pro His His Phe Tyr Gly Pro Pro Met Asp Ala Ala Arg
                245                 250                 255

Ile Thr Thr Ser Ser Arg Asp Glu Pro Pro Ser Met Ser Asn Ala Ser
            260                 265                 270

Glu Met Gln Gly Glu Trp Val Glu Glu Asp Glu Pro Gly Val Tyr Ile
        275                 280                 285

Thr Ile Arg Gln Leu Pro Asp Gly Thr Arg Glu Leu Arg Arg Val Arg
    290                 295                 300

Phe Ser Arg Glu Arg Phe Gly Glu Val His Ala Lys Thr Trp Trp Glu
305                 310                 315                 320

Gln Asn Arg Asp Arg Ile Gln Thr Gln Tyr Leu
                325                 330

<210>  23
<211>  1125
<212>  DNA
<213>  Arabidopsis thaliana

<400>  23
atgctgacat gcatagcttg tacgaagcag ctaaacacca acaatggtgg atctaagaaa      60

caagaagagg atgaagaaga agaagacaga gttattgaaa cacccaggtc taagcagatt     120

aagtccctga cgtcacagat taaagacatg gcagtaaaag catcaggtgc ttacaaaagc     180

tgcaaaccgt gttctgggtc gtctaatcag aataagaacc gaagctacgc tgattcggat     240

gttgcttcga attctgggag gtttcgttat gcgtacaaga gagcggggag tggaagctca     300

acaccgaaga ttttggggaa ggaaatggag tcaaggttga aaggtttttt gagtggagaa     360

ggaacacctg aatccatgag tggtaggaca gagtctacag tgttcatgga ggaagaagat 420

gagctcaagg aatgggttgc tcaagtggag cctggtgtcc tcatcacatt tgtatcattg 480

cctgagggag ggaatgatat gaagcggatt cggttcagcc gtgaaatgtt cgataaatgg 540

caagctcaaa agtggtgggc ggagaatttc gacaaggtca tggaattata caatgtacag 600

cagttcaatc agcagagcgt cccacttcca actcctccta gatctgaaga tgggagctcg 660

cgaattcagt ctaccaagaa tggtcctgca actccacctc tgaacaaaga atgctctcga 720

ggaaaaggct acgcttcttc tggctcactc gctcaccaac caacaaccca aacacaaagt 780

cgacaccatg attcatctgg tcttgctaca acaccaaaac tctctagcat aagtgggaca 840

aaaaccgaga catcatctgt tgatgagtct gcaagaagta gcttctcaag ggaagaagaa 900

gaagcagatc attcagggga ggagctatct gtaagtaatg caagtgacat tgaaacagaa 960

tgggtggaac aggacgaagc aggtgtttac atcacaatca gagctttacc agatgggact 1020

cgcgagctta ggcgtgttcg cttcagccga gagaagtttg gggaaacgaa tgcaagattg 1080

tggtgggagc agaacagagc tcggatacaa caacaatact tgtga 1125

<210> 24
<211> 374
<212> PRT
<213> Arabidopsis thaliana

<400> 24

Met Leu Thr Cys Ile Ala Cys Thr Lys Gln Leu Asn Thr Asn Asn Gly
1               5                   10                  15

Gly Ser Lys Lys Gln Glu Glu Asp Glu Glu Glu Glu Asp Arg Val Ile
            20                  25                  30

Glu Thr Pro Arg Ser Lys Gln Ile Lys Ser Leu Thr Ser Gln Ile Lys
        35                  40                  45

Asp Met Ala Val Lys Ala Ser Gly Ala Tyr Lys Ser Cys Lys Pro Cys
    50                  55                  60

Ser Gly Ser Ser Asn Gln Asn Lys Asn Arg Ser Tyr Ala Asp Ser Asp
65                  70                  75                  80

Val Ala Ser Asn Ser Gly Arg Phe Arg Tyr Ala Tyr Lys Arg Ala Gly
                85                  90                  95

Ser Gly Ser Ser Thr Pro Lys Ile Leu Gly Lys Glu Met Glu Ser Arg
            100                 105                 110

Leu Lys Gly Phe Leu Ser Gly Glu Gly Thr Pro Glu Ser Met Ser Gly
        115                 120                 125

Arg Thr Glu Ser Thr Val Phe Met Glu Glu Glu Asp Glu Leu Lys Glu
    130                 135                 140

Trp Val Ala Gln Val Glu Pro Gly Val Leu Ile Thr Phe Val Ser Leu
145                 150                 155                 160

Pro Glu Gly Gly Asn Asp Met Lys Arg Ile Arg Phe Ser Arg Glu Met
                165                 170                 175

Phe Asp Lys Trp Gln Ala Gln Lys Trp Trp Ala Glu Asn Phe Asp Lys
            180                 185                 190

Val Met Glu Leu Tyr Asn Val Gln Gln Phe Asn Gln Gln Ser Val Pro
        195                 200                 205

Leu Pro Thr Pro Pro Arg Ser Glu Asp Gly Ser Ser Arg Ile Gln Ser
    210                 215                 220

Thr Lys Asn Gly Pro Ala Thr Pro Pro Leu Asn Lys Glu Cys Ser Arg
225                 230                 235                 240

Gly Lys Gly Tyr Ala Ser Ser Gly Ser Leu Ala His Gln Pro Thr Thr
                245                 250                 255

Gln Thr Gln Ser Arg His His Asp Ser Ser Gly Leu Ala Thr Thr Pro
            260                 265                 270

Lys Leu Ser Ser Ile Ser Gly Thr Lys Thr Glu Thr Ser Ser Val Asp
        275                 280                 285

Glu Ser Ala Arg Ser Ser Phe Ser Arg Glu Glu Glu Glu Ala Asp His
    290                 295                 300

Ser Gly Glu Glu Leu Ser Val Ser Asn Ala Ser Asp Ile Glu Thr Glu
305                 310                 315                 320

Trp Val Glu Gln Asp Glu Ala Gly Val Tyr Ile Thr Ile Arg Ala Leu
                325                 330                 335

Pro Asp Gly Thr Arg Glu Leu Arg Arg Val Arg Phe Ser Arg Glu Lys
            340                 345                 350

Phe Gly Glu Thr Asn Ala Arg Leu Trp Trp Glu Gln Asn Arg Ala Arg
        355                 360                 365

Ile Gln Gln Gln Tyr Leu
    370

<210>  25
<211>  1035
<212>  DNA
<213>  Arabidopsis thaliana

<400>  25
atgctgacgt gtatagcttg cacgaagcag cttaacacca acaatggcgg atctactcga      60

gaagaagatg aagaacacgg agttattggt acccctagga ctaagcaagc gattaaatca     120

ctgacgtcac agctaaaaga catggcagta aaggcttcag gggcatacaa aaactgcaaa     180

ccgtgttctg ggacaacaaa ccggaatcag aatcgaaact atgctgattc ggatgctgct     240

tcagattctg gaagattcca ttattcatac cagagagctg ggactgcaac ctcaactcct     300

aagatttggg ggaatgaaat ggagtcaagg ttaaaaggga tttctagtga agaaggtaca     360

cctacgtcta tgagtggtcg aacagaatcc atagtgttca tggaggatga tgaggtcaag     420

gaatgggttg ctcaagtgga gcctggtgtt ctcatcacat ttgtgtcatt gcctcaggga     480

gggaatgatc ttaagagaat tcggttcagc cgtgagatgt ttaataaatg gcaagctcaa     540

aaatggtggg tggagaattt cgagaaggtc atggagttat acaacgtgca gttcaatcag     600

cagagcgtac cgcttcaaac tcctcctgta tctgaagatg ggggctcaca aatacagtcc     660

gtgaaggaca gtcctgtaac tccaccgctg gaaagagagc gacctcaccg caatattcca     720

ggctcttctg gttttgcttc aaccccaaag ctctctagca tcagcggaac caagactgaa     780

acatcatcta tcgatggttc tgctagaagt agctcggtag atcgatccga ggaggtctca     840

gtgagtaacg caagtgacat ggaaagtgaa tgggtagaac aagatgagcc tggtatctat     900

attactatca gagctttacc agatggaaat cgcgagctta ggcgagttcg attcagccga     960

gacaagtttg gggaaacaca tgcgaggtta tggtgggaac aaaacagagc gcgtatacaa    1020

cagcaatact tgtga                                                    1035

<210>  26
<211>  344
<212>  PRT
<213>  Arabidopsis thaliana

<400>  26

Met Leu Thr Cys Ile Ala Cys Thr Lys Gln Leu Asn Thr Asn Asn Gly
1               5                   10                  15

Gly Ser Thr Arg Glu Glu Asp Glu Glu His Gly Val Ile Gly Thr Pro
            20                  25                  30

Arg Thr Lys Gln Ala Ile Lys Ser Leu Thr Ser Gln Leu Lys Asp Met
        35                  40                  45

Ala Val Lys Ala Ser Gly Ala Tyr Lys Asn Cys Lys Pro Cys Ser Gly
    50                  55                  60

Thr Thr Asn Arg Asn Gln Asn Arg Asn Tyr Ala Asp Ser Asp Ala Ala
65                  70                  75                  80

Ser Asp Ser Gly Arg Phe His Tyr Ser Tyr Gln Arg Ala Gly Thr Ala
                85                  90                  95

Thr Ser Thr Pro Lys Ile Trp Gly Asn Glu Met Glu Ser Arg Leu Lys
            100                 105                 110

Gly Ile Ser Ser Glu Glu Gly Thr Pro Thr Ser Met Ser Gly Arg Thr
        115                 120                 125

Glu Ser Ile Val Phe Met Glu Asp Asp Glu Val Lys Glu Trp Val Ala
    130                 135                 140

Gln Val Glu Pro Gly Val Leu Ile Thr Phe Val Ser Leu Pro Gln Gly
145                 150                 155                 160

Gly Asn Asp Leu Lys Arg Ile Arg Phe Ser Arg Glu Met Phe Asn Lys
                165                 170                 175

Trp Gln Ala Gln Lys Trp Trp Val Glu Asn Phe Glu Lys Val Met Glu
            180                 185                 190

Leu Tyr Asn Val Gln Phe Asn Gln Gln Ser Val Pro Leu Gln Thr Pro
        195                 200                 205

Pro Val Ser Glu Asp Gly Gly Ser Gln Ile Gln Ser Val Lys Asp Ser
    210                 215                 220

Pro Val Thr Pro Pro Leu Glu Arg Glu Arg Pro His Arg Asn Ile Pro
225                 230                 235                 240

Gly Ser Ser Gly Phe Ala Ser Thr Pro Lys Leu Ser Ser Ile Ser Gly
                245                 250                 255

Thr Lys Thr Glu Thr Ser Ser Ile Asp Gly Ser Ala Arg Ser Ser Ser
            260                 265                 270

Val Asp Arg Ser Glu Glu Val Ser Val Ser Asn Ala Ser Asp Met Glu
        275                 280                 285

Ser Glu Trp Val Glu Gln Asp Glu Pro Gly Ile Tyr Ile Thr Ile Arg
    290                 295                 300

Ala Leu Pro Asp Gly Asn Arg Glu Leu Arg Arg Val Arg Phe Ser Arg
305                 310                 315                 320

Asp Lys Phe Gly Glu Thr His Ala Arg Leu Trp Trp Glu Gln Asn Arg
                325                 330                 335

Ala Arg Ile Gln Gln Gln Tyr Leu
            340

<210>  27
<211>  1155
<212>  DNA
<213>  Arabidopsis thaliana

<400>  27
atgctgacgt gtatagctcg ttcgaagcga gcaggcgatg aatcctccgg tcaaccagac      60

gatccagatt ctaaaaacgc caaatctcta acatctcagc tcaaagatat ggctctgaaa     120

gcatcaggag cttaccggca ttgtacgccg tgtacggcgg cacaaggtca gggacaagga     180

caaggtccga tcaagaacaa tccgtcgtcg tcgtcggtaa agtcggattt cgaatcggat     240

caacggttta aaatgcttta cggaagatca aacagttcga ttacagctac ggcggcggtg     300

gcggcgacgc aacaacaaca gcctagggtt tggggaaagg agatggaagc gagactaaaa     360

gggatttcga gcggcgaagc gactccgaaa tcggcgagtg ggagaaaccg ggtcgacccg     420

attgtgtttg tggaggagaa agagcctaaa gaatgggttg ctcaggttga gcccggagtt     480

ctcataacct tcgtttctct tcccggcggt ggtaatgatc tcaagcggat acgtttcagc     540

cgagacatgt tcaacaagtt acaagctcaa cgatggtggg cagataacta tgacaaagta     600

atggaacttt acaatgttca aaaactaagc cgccaagctt tcccgcttcc caccccgcct     660

agatccgaag acgagaatgc aaaagtggag taccatccag aagacactcc tgcaacaccg     720

cctctaaaca aagaacggtt gcctcgtact atccatcgtc cacctggatt ggctgcttac     780

tcatcctcag attcactcga ccataattca atgcagagcc agcagttcta tgactctggt     840

ctactcaact caactcctaa agtttcaagc atcagtgtag ccaagacaga aacttcttcc     900

atagatgctt ccataagaag cagctcgtcg agagatgcag accggtcaga ggaaatgtcg     960

gtaagcaatg cgagtgatgt tgacaacgaa tgggtggagc aagatgagcc tggcgtttat    1020

atcaccatta aagttttacc aggtgggaaa agagagcttc gaagagtcag attcagccga    1080

gagagattcg gggagatgca cgcgagatta tggtgggaag agaacagggc aaggatacat    1140

gaacaatact tgtga                                                     1155

<210>  28
<211>  384
<212>  PRT
<213>  Arabidopsis thaliana

<400>  28
   Met Leu Thr Cys Ile Ala Arg Ser Lys Arg Ala Gly Asp Glu Ser Ser
   1 5 10 15
   Gly Gln Pro Asp Asp Pro Asp Ser Lys Asn Ala Lys Ser Leu Thr Ser
   20 25 30
   Gln Leu Lys Asp Met Ala Leu Lys Ala Ser Gly Ala Tyr Arg His Cys
   35 40 45
   Thr Pro Cys Thr Ala Ala Gln Gly Gln Gly Gln Gly Gln Gly Pro Ile
   50 55 60
   Lys Asn Asn Pro Ser Ser Ser Ser Val Lys Ser Asp Phe Glu Ser Asp
   65 70 75 80
   Gln Arg Phe Lys Met Leu Tyr Gly Arg Ser Asn Ser Ser Ile Thr Ala
   85 90 95
   Thr Ala Ala Val Ala Ala Thr Gln Gln Gln Gln Pro Arg Val Trp Gly
   100 105 110
   Lys Glu Met Glu Ala Arg Leu Lys Gly Ile Ser Ser Gly Glu Ala Thr
   115 120 125
   Pro Lys Ser Ala Ser Gly Arg Asn Arg Val Asp Pro Ile Val Phe Val
   130 135 140
   Glu Glu Lys Glu Pro Lys Glu Trp Val Ala Gln Val Glu Pro Gly Val
   145 150 155 160
   Leu Ile Thr Phe Val Ser Leu Pro Gly Gly Gly Asn Asp Leu Lys Arg
   165 170 175
   Ile Arg Phe Ser Arg Asp Met Phe Asn Lys Leu Gln Ala Gln Arg Trp
   180 185 190
   Trp Ala Asp Asn Tyr Asp Lys Val Met Glu Leu Tyr Asn Val Gln Lys
   195 200 205
   Leu Ser Arg Gln Ala Phe Pro Leu Pro Thr Pro Pro Arg Ser Glu Asp
   210 215 220
   Glu Asn Ala Lys Val Glu Tyr His Pro Glu Asp Thr Pro Ala Thr Pro
   225                 230                 235                 240
   Pro Leu Asn Lys Glu Arg Leu Pro Arg Thr Ile His Arg Pro Pro Gly
                   245                 250                 255
   Leu Ala Ala Tyr Ser Ser Ser Asp Ser Leu Asp His Asn Ser Met Gln
               260                 265                 270
   Ser Gln Gln Phe Tyr Asp Ser Gly Leu Leu Asn Ser Thr Pro Lys Val
           275                 280                 285
   Ser Ser Ile Ser Val Ala Lys Thr Glu Thr Ser Ser Ile Asp Ala Ser
       290                 295                 300
   Ile Arg Ser Ser Ser Ser Arg Asp Ala Asp Arg Ser Glu Glu Met Ser
   305                 310                 315                 320
   Val Ser Asn Ala Ser Asp Val Asp Asn Glu Trp Val Glu Gln Asp Glu
                   325                 330                 335
   Pro Gly Val Tyr Ile Thr Ile Lys Val Leu Pro Gly Gly Lys Arg Glu
               340                 345                 350
   Leu Arg Arg Val Arg Phe Ser Arg Glu Arg Phe Gly Glu Met His Ala
           355                 360                 365
   Arg Leu Trp Trp Glu Glu Asn Arg Ala Arg Ile His Glu Gln Tyr Leu
       370                 375                 380

<210>  29
<211>  1101
<212>  DNA
<213>  Glycine max

<400>  29
atgcttacgt gcatagctcg tccaaagaaa cctgatgaat cggatccgga caacgcgaca      60

agcgccgcta aatcgcaagc catcaaatcc cttacttctc agataaggga tatggcgttg     120

aaggcttcgg gagcgtacaa gcattgcgca ccgtgcacgg ggccagcgac gcaggggcga     180

gttcggagca acgccaccga gttggacgcg gattcggacc ggttccggtg gtcgtaccgg     240

agaacgggga gctcgagctc gacgacgaca aggacgtggg ggaaggagat ggaggcgcgg     300

ctgaagggga tttcgagcgg ggagggaacg ccgaactccg ccagcggacg gagggcggag     360

ccggtggtgc tgttcgttga agagaacgag ccgaaggagt gggttgcgca ggtggagcct     420

ggcgttttga ttacgttcgt gtcgttgcca cgtggcggga acgatctcaa gcgtatacgg     480

ttcagtcgag agatgttcaa taaatggcaa gctcaaaggt ggtgggcaga gaactacgac 540

aaggttatgg aactttacaa tgttcaaagg tttaaccgcc aagcatttcc tcttccaacg 600

cctctaagat ctgaagatga gagctcaaag cttgaatcag tagaagaaag ccctgtaaca 660

cctccactga acagtgagcg gttacctcgt aatatgtacc gtccaacagg gatgggaatg 720

ggttactcat cctcagattc ctttgatcat cagtctatgc aatctcggca tttctatgac 780

tcaaatggta tgaactcaac accaaaagtg tccaccatta gtgcggccaa gacagagata 840

tcatcaatgg aggcttctat cagaagtagc tcatccagag aggctgatcg ctcgggtgat 900

ttttcaatca gtaatgctag tgaattggag actgaatggg ttgaacagga tgaacctggg 960

gtttacatta cgatcagagc attgccaggt ggtgcaagag agctcaaacg agtcaggttc 1020

agccgagaaa agtttgggga gatgcatgct agattatggt gggaagagaa ccgtgccaga 1080

atacatgaac aatacttgtg a 1101

<210> 30
<211> 366
<212> PRT
<213> Glycine max

<400> 30

Met Leu Thr Cys Ile Ala Arg Pro Lys Lys Pro Asp Glu Ser Asp Pro
1               5                   10                  15

Asp Asn Ala Thr Ser Ala Ala Lys Ser Gln Ala Ile Lys Ser Leu Thr
            20                  25                  30

Ser Gln Ile Arg Asp Met Ala Leu Lys Ala Ser Gly Ala Tyr Lys His
        35                  40                  45

Cys Ala Pro Cys Thr Gly Pro Ala Thr Gln Gly Arg Val Arg Ser Asn
    50                  55                  60

Ala Thr Glu Leu Asp Ala Asp Ser Asp Arg Phe Arg Trp Ser Tyr Arg
65                  70                  75                  80

Arg Thr Gly Ser Ser Ser Ser Thr Thr Thr Arg Thr Trp Gly Lys Glu
                85                  90                  95

Met Glu Ala Arg Leu Lys Gly Ile Ser Ser Gly Glu Gly Thr Pro Asn
            100                 105                 110

Ser Ala Ser Gly Arg Arg Ala Glu Pro Val Val Leu Phe Val Glu Glu
        115                 120                 125

Asn Glu Pro Lys Glu Trp Val Ala Gln Val Glu Pro Gly Val Leu Ile

130                 135                 140

Thr Phe Val Ser Leu Pro Arg Gly Gly Asn Asp Leu Lys Arg Ile Arg
145                 150                 155                 160

Phe Ser Arg Glu Met Phe Asn Lys Trp Gln Ala Gln Arg Trp Trp Ala
                165                 170                 175

Glu Asn Tyr Asp Lys Val Met Glu Leu Tyr Asn Val Gln Arg Phe Asn
            180                 185                 190

Arg Gln Ala Phe Pro Leu Pro Thr Pro Leu Arg Ser Glu Asp Glu Ser
        195                 200                 205

Ser Lys Leu Glu Ser Val Glu Glu Ser Pro Val Thr Pro Pro Leu Asn
    210                 215                 220

Ser Glu Arg Leu Pro Arg Asn Met Tyr Arg Pro Thr Gly Met Gly Met
225                 230                 235                 240

Gly Tyr Ser Ser Ser Asp Ser Phe Asp His Gln Ser Met Gln Ser Arg
                245                 250                 255

His Phe Tyr Asp Ser Asn Gly Met Asn Ser Thr Pro Lys Val Ser Thr
            260                 265                 270

Ile Ser Ala Ala Lys Thr Glu Ile Ser Ser Met Glu Ala Ser Ile Arg
        275                 280                 285

Ser Ser Ser Ser Arg Glu Ala Asp Arg Ser Gly Asp Phe Ser Ile Ser
    290                 295                 300

Asn Ala Ser Glu Leu Glu Thr Glu Trp Val Glu Gln Asp Glu Pro Gly
305                 310                 315                 320

Val Tyr Ile Thr Ile Arg Ala Leu Pro Gly Gly Ala Arg Glu Leu Lys
                325                 330                 335

Arg Val Arg Phe Ser Arg Glu Lys Phe Gly Glu Met His Ala Arg Leu
            340                 345                 350

Trp Trp Glu Glu Asn Arg Ala Arg Ile His Glu Gln Tyr Leu
        355                 360                 365

<210>  31
<211>  1050
<212>  DNA
<213>  Glycine max

<400>  31
atgtttacgt gcatagcgtg tacgaagacg gatgataagg atgaagaagg aggatctcgt      60

gagagtggca cgccgagtac aaaagaagcc gtcaaaagcc tgaccacgca gataaaggat     120

atggcactga agttttcagg tgcatacaag caatgcaaac catgcacagg gtccagtagc     180

tacaaaaaag gacataggcc atatccagat tttgatacca tctcagaagg ggttccatac     240

ccttatattg gaggtgcaag ctcaagttca acccctgcat gggacttcac aacctctcac     300

taccctggtg gaagatctga ccctagattt gctggggcat atggcggtga ccgcaccccg     360

agaggacgtg actcatcatc agtttgtgat gtagtcttag aggatgagga tgagcctaag     420

gagtggatgg cacaggtgga gccaggggtt cacattacct ttgtgtctct tcctaacgga     480

ggaaatgatc ttaagagaat tcgcttcagc cgagagatgt ttaataaatg gcaagctcaa     540

agatggtggg gtgagaatta cgacagaatc atggaacttt acaacgtcca gagatttaat     600

aaacaagctc ttaacactcc tccaaggtct gaggatgagc aaagagattc ttcttactca     660

agattgacat ctgcaaggga aagccccatg gcctcaaaca aggattggac gccgaggagt     720

cactataagc cctctgggag cagaggatat tacccgtccg aaccgttgga tcacggtgga     780

ggcagtggcc aataccatgc agggccatct atggaaccag caagggatac cactgcttct     840

agagatgagc cttctatcag caatgccagt gaaatggaga cagaatgggt agaacaagac     900

gagcctggag tttacattac aatcaggcag ttagctgatg gaactaggga gcttagacgt     960

gtcagattca gccgggaaag atttggggag gtgaatgcaa aaacttggtg ggaagagaac    1020

agagagagaa tccaagctca atatctttga                                     1050


<210>  32
<211>  349
<212>  PRT
<213>  Glycine max

<400>  32

Met Phe Thr Cys Ile Ala Cys Thr Lys Thr Asp Asp Lys Asp Glu Glu
1               5                   10                  15


Gly Gly Ser Arg Glu Ser Gly Thr Pro Ser Thr Lys Glu Ala Val Lys
            20                  25                  30


Ser Leu Thr Thr Gln Ile Lys Asp Met Ala Leu Lys Phe Ser Gly Ala
        35                  40                  45


Tyr Lys Gln Cys Lys Pro Cys Thr Gly Ser Ser Ser Tyr Lys Lys Gly
    50                  55                  60


His Arg Pro Tyr Pro Asp Phe Asp Thr Ile Ser Glu Gly Val Pro Tyr

65                  70                  75                  80

Pro Tyr Ile Gly Gly Ala Ser Ser Ser Ser Thr Pro Ala Trp Asp Phe
                85                  90                  95

Thr Thr Ser His Tyr Pro Gly Gly Arg Ser Asp Pro Arg Phe Ala Gly
            100                 105                 110

Ala Tyr Gly Gly Asp Arg Thr Pro Arg Gly Arg Asp Ser Ser Ser Val
        115                 120                 125

Cys Asp Val Val Leu Glu Asp Glu Asp Glu Pro Lys Glu Trp Met Ala
    130                 135                 140

Gln Val Glu Pro Gly Val His Ile Thr Phe Val Ser Leu Pro Asn Gly
145                 150                 155                 160

Gly Asn Asp Leu Lys Arg Ile Arg Phe Ser Arg Glu Met Phe Asn Lys
                165                 170                 175

Trp Gln Ala Gln Arg Trp Trp Gly Glu Asn Tyr Asp Arg Ile Met Glu
            180                 185                 190

Leu Tyr Asn Val Gln Arg Phe Asn Lys Gln Ala Leu Asn Thr Pro Pro
        195                 200                 205

Arg Ser Glu Asp Glu Gln Arg Asp Ser Ser Tyr Ser Arg Leu Thr Ser
    210                 215                 220

Ala Arg Glu Ser Pro Met Ala Ser Asn Lys Asp Trp Thr Pro Arg Ser
225                 230                 235                 240

His Tyr Lys Pro Ser Gly Ser Arg Gly Tyr Tyr Pro Ser Glu Pro Leu
                245                 250                 255

Asp His Gly Gly Gly Ser Gly Gln Tyr His Ala Gly Pro Ser Met Glu
            260                 265                 270

Pro Ala Arg Asp Thr Thr Ala Ser Arg Asp Glu Pro Ser Ile Ser Asn
        275                 280                 285

Ala Ser Glu Met Glu Thr Glu Trp Val Glu Gln Asp Glu Pro Gly Val
    290                 295                 300

Tyr Ile Thr Ile Arg Gln Leu Ala Asp Gly Thr Arg Glu Leu Arg Arg
305                 310                 315                 320

Val Arg Phe Ser Arg Glu Arg Phe Gly Glu Val Asn Ala Lys Thr Trp
                325                 330                 335

Trp Glu Glu Asn Arg Glu Arg Ile Gln Ala Gln Tyr Leu
            340                 345

<210>  33
<211>  1101
<212>  DNA
<213>  Glycine max

<400>  33
atgcttacgt gcatagctcg tccaaagaaa cctgatgagt cggatccaaa caacgcgaca      60

agcgccgcta aatcgcaagc cgtcaaatct ctcacttctc agataaggga tatggcgctg     120

aaggcttcgg gagcgtacaa gcattgcgca ccgtgcacgg ggccagcgac gcaggggcga     180

tttcggagca acaccaccga gtcggacgcg gattcggacc ggttccggtg gtcgtaccgg     240

agaacgggga gttcgagctc gacgacgaca aggacgtggg ggaaggagat ggaggcgcgg     300

ctgaagggga tttcgagcgg ggaggggacg ccgaactccg ccagcgggag gcgggcggag     360

ccggtggtgc tgtttgttga agagaacgag ccgaaggagt gggtggcgca ggtggagcct     420

ggcgttttga ttacgttcgt ttcgttgcca cgtggcggga acgatctgaa gcgcatacgg     480

ttcagtcgag agatgttcaa taaatggcaa gctcaaaggt ggtgggcaga gaactacgac     540

aaagttatgg aactttacaa tgttcaaagg tttaaccgcc aagcatttcc tcttccaacg     600

ccaccaagat ctgaagatga gagctcaaag cttgaatcag ctgaagaaag ccccgtaaca     660

cctccactga acagggaacg gttacctcgt aatatgtacc gtccaacagg aatgggaatg     720

ggatactcat cctcagattc ctttgatcat cagtcaatgc aatctcggca tttctatgac     780

tcaaatggta tgaactcaac accgaaagtg tccaccatta gtgcggccaa aacggagata     840

tcatcaatgg atgcttctat cagaagtagc tcatcgagag aggctgatcg ctccggtgat     900

ttttcaatca gcaatgctag tgatttggag actgaatggg ttgaacagga tgaacctggg     960

gtttacatta cgatcagagc attgccaggt ggtgcaagag agctcaaacg agtcaggttc    1020

agccgagaaa aatttgggga gatgcatgct agattatggt gggaagagaa ccgtgccaga    1080

atacatgaac aatacttgtg a                                              1101

<210>  34
<211>  366
<212>  PRT
<213>  Glycine max

<400>  34

Met Leu Thr Cys Ile Ala Arg Pro Lys Lys Pro Asp Glu Ser Asp Pro
1               5                   10                  15

Asn Asn Ala Thr Ser Ala Ala Lys Ser Gln Ala Val Lys Ser Leu Thr
            20                  25                  30

Ser Gln Ile Arg Asp Met Ala Leu Lys Ala Ser Gly Ala Tyr Lys His
        35                  40                  45

Cys Ala Pro Cys Thr Gly Pro Ala Thr Gln Gly Arg Phe Arg Ser Asn
    50                  55                  60

Thr Thr Glu Ser Asp Ala Asp Ser Asp Arg Phe Arg Trp Ser Tyr Arg
65                  70                  75                  80

Arg Thr Gly Ser Ser Ser Ser Thr Thr Thr Arg Thr Trp Gly Lys Glu
                85                  90                  95

Met Glu Ala Arg Leu Lys Gly Ile Ser Ser Gly Glu Gly Thr Pro Asn
            100                 105                 110

Ser Ala Ser Gly Arg Arg Ala Glu Pro Val Val Leu Phe Val Glu Glu
        115                 120                 125

Asn Glu Pro Lys Glu Trp Val Ala Gln Val Glu Pro Gly Val Leu Ile
    130                 135                 140

Thr Phe Val Ser Leu Pro Arg Gly Gly Asn Asp Leu Lys Arg Ile Arg
145                 150                 155                 160

Phe Ser Arg Glu Met Phe Asn Lys Trp Gln Ala Gln Arg Trp Trp Ala
                165                 170                 175

Glu Asn Tyr Asp Lys Val Met Glu Leu Tyr Asn Val Gln Arg Phe Asn
            180                 185                 190

Arg Gln Ala Phe Pro Leu Pro Thr Pro Pro Arg Ser Glu Asp Glu Ser
        195                 200                 205

Ser Lys Leu Glu Ser Ala Glu Glu Ser Pro Val Thr Pro Pro Leu Asn
    210                 215                 220

Arg Glu Arg Leu Pro Arg Asn Met Tyr Arg Pro Thr Gly Met Gly Met
225                 230                 235                 240

Gly Tyr Ser Ser Ser Asp Ser Phe Asp His Gln Ser Met Gln Ser Arg
                245                 250                 255

His Phe Tyr Asp Ser Asn Gly Met Asn Ser Thr Pro Lys Val Ser Thr
            260                 265                 270

Ile Ser Ala Ala Lys Thr Glu Ile Ser Ser Met Asp Ala Ser Ile Arg
        275                 280                 285

Ser Ser Ser Ser Arg Glu Ala Asp Arg Ser Gly Asp Phe Ser Ile Ser
    290                 295                 300

Asn Ala Ser Asp Leu Glu Thr Glu Trp Val Glu Gln Asp Glu Pro Gly
305                 310                 315                 320

Val Tyr Ile Thr Ile Arg Ala Leu Pro Gly Gly Ala Arg Glu Leu Lys
                325                 330                 335

Arg Val Arg Phe Ser Arg Glu Lys Phe Gly Glu Met His Ala Arg Leu
            340                 345                 350

Trp Trp Glu Glu Asn Arg Ala Arg Ile His Glu Gln Tyr Leu
        355                 360                 365

<210>  35
<211>  1074
<212>  DNA
<213>  Glycine max

<400>  35
atgcttacgt gcatagcacg tccgaagaaa cccggtggcg actcggcaag cgacgatccg      60

agttcgcgga gccagcaggg agtgaagtcg ctgacgtgtc agctgaagga gatggcgctg     120

aaggcgtcgg gagcgtacaa gcagtgcggc ccgtgcgcga cggcgccgag taggccgagt     180

cgcagcggaa ccgagtcgga ctcggagtcg tcgtcgtccc ggcggcggtg ggggaaggag     240

ctggaggcgc ggctgaaggg gatatcgagc ggggaaggga cgccgagctc gagcgggagg     300

agggtggtga tgctgctcga ggacgaggag gagccgaagg agtgggtggc gcaggtggag     360

cctggcgttt tgatctcctt tgtgtcgctt ccacgtggcg ggaaccatct caaacggata     420

cgattcagtc gcgagatatt taacaaatgg caagctcaaa gatggtgggc agagaactat     480

gacaaggtga tggaacttta caatgttcaa aggcttgatc gccaagcttt ccctcttcca     540

actccaccaa ggtctgaaga cgagagttca aagcgtgaat caatagaaga tttcccagtg     600

acacctccac tgagcaggga aaggccacct tgtaatctat accgtgccgg gggaagagga     660

ggaggaggca tgggaatggg gtactcatcc tcagattcct ttgatcacac ctcaatgcaa     720

tcatcccggc attactatga ccccaatggt gtgaactcaa ccccaaaggc ttccaccatt     780

agtgctgctg caaagacaga tatctcatca ataatggatg ctgatgcttc cataagaagt     840

agttcatcca gggaagctga tcgctctggg gatctttcaa tcagcaatgc cagtgacttt     900

gacaatgaat gggttgagca ggatgagcct ggggtttaca tcaccatcag agctctcctg     960

ggtggcaaaa aggagctcag acgagtcagg ttcagtcgag aaaagtttgg ggagatgcat 1020

gctagactat ggtgggaaga gaaccgtgcc agaatacatg aacaatactt gtga 1074

<210> 36
<211> 357
<212> PRT
<213> Glycine max

<400> 36

Met Leu Thr Cys Ile Ala Arg Pro Lys Lys Pro Gly Gly Asp Ser Ala
1               5                   10                  15

Ser Asp Asp Pro Ser Ser Arg Ser Gln Gln Gly Val Lys Ser Leu Thr
            20                  25                  30

Cys Gln Leu Lys Glu Met Ala Leu Lys Ala Ser Gly Ala Tyr Lys Gln
        35                  40                  45

Cys Gly Pro Cys Ala Thr Ala Pro Ser Arg Pro Ser Arg Ser Gly Thr
    50                  55                  60

Glu Ser Asp Ser Glu Ser Ser Ser Ser Arg Arg Arg Trp Gly Lys Glu
65                  70                  75                  80

Leu Glu Ala Arg Leu Lys Gly Ile Ser Ser Gly Glu Gly Thr Pro Ser
                85                  90                  95

Ser Ser Gly Arg Arg Val Val Met Leu Leu Glu Asp Glu Glu Glu Pro
            100                 105                 110

Lys Glu Trp Val Ala Gln Val Glu Pro Gly Val Leu Ile Ser Phe Val
        115                 120                 125

Ser Leu Pro Arg Gly Gly Asn His Leu Lys Arg Ile Arg Phe Ser Arg
    130                 135                 140

Glu Ile Phe Asn Lys Trp Gln Ala Gln Arg Trp Trp Ala Glu Asn Tyr
145                 150                 155                 160

Asp Lys Val Met Glu Leu Tyr Asn Val Gln Arg Leu Asp Arg Gln Ala
                165                 170                 175

Phe Pro Leu Pro Thr Pro Pro Arg Ser Glu Asp Glu Ser Ser Lys Arg
            180                 185                 190

Glu Ser Ile Glu Asp Phe Pro Val Thr Pro Pro Leu Ser Arg Glu Arg
        195                 200                 205

Pro Pro Cys Asn Leu Tyr Arg Ala Gly Gly Arg Gly Gly Gly Gly Met
    210                 215                 220

Gly Met Gly Tyr Ser Ser Ser Asp Ser Phe Asp His Thr Ser Met Gln
225                 230                 235                 240

Ser Ser Arg His Tyr Tyr Asp Pro Asn Gly Val Asn Ser Thr Pro Lys
                245                 250                 255

Ala Ser Thr Ile Ser Ala Ala Ala Lys Thr Asp Ile Ser Ser Ile Met
            260                 265                 270

Asp Ala Asp Ala Ser Ile Arg Ser Ser Ser Ser Arg Glu Ala Asp Arg
        275                 280                 285

Ser Gly Asp Leu Ser Ile Ser Asn Ala Ser Asp Phe Asp Asn Glu Trp
    290                 295                 300

Val Glu Gln Asp Glu Pro Gly Val Tyr Ile Thr Ile Arg Ala Leu Leu
305                 310                 315                 320

Gly Gly Lys Lys Glu Leu Arg Arg Val Arg Phe Ser Arg Glu Lys Phe
                325                 330                 335

Gly Glu Met His Ala Arg Leu Trp Trp Glu Glu Asn Arg Ala Arg Ile
            340                 345                 350

His Glu Gln Tyr Leu
        355

<210>  37
<211>  1062
<212>  DNA
<213>  Glycine max

<400>  37
atgtttacat gcattgcgtg tacgaagcaa gcggcggagg agaaggaaga agaagaagag      60

ggtgagagtg gcacaacgag tacaaataaa gaagccgtca aaagcctgag tgctcagtta     120

aaggatatgg cactgaagtt ttcaggtgcc tacaagcagt gcaaaccttg cacagggtct     180

agtacctaca agaatggaca gaggtcatat cctgactttg acaccatttc agaaggggtt     240

ccataccctt atattggagg tgcaagctca acttcaaccc ctgcttggga cttcacaagc     300

tccaatttcc ctggtggtag atcagaccaa agatttatgg ggagattcag tggtgatagg     360

acccctagag ggcctcaatc agcaccagcc tctgatgtag tagttgttga ggatgaggat     420

gaaaccaagg agtggatggc acaggtggaa ccaggggttc acattacctt tgtgtctctt     480

cctaatgggg gaaatgatct caaaagaatt cgcttcagcc gagagatttt cgacaagtgg 540

caagctcaaa aatggtgggg tgagaattat gacagaatca tggaacttta caacgtgcag 600

agattcaatc gacaagctct caacactcct tcaagatctg aggatgagcg aagagattct 660

tcttactcaa gaatgacatc tggacaagat agccccatgc actccatgtc tttaaagggt 720

tggacaccaa ggaatcacta taaaccctca gggaataacc cttctgaagc tatggaacaa 780

ggtagtggtg gtggccaaga cttccatgca gcagcttcat ccgtggaagc atcaaggaca 840

accacctctt ctagagacga gcgttcaatg agcaatgcca gtgacttgga gactgaatgg 900

atagagcaag atgaacctgg agtctacatc acaatcaggc aattagctga tggaacaaag 960

gagcttcgcc gtgtcagatt cagccgggag agatttgggg agggacatgc aaagaaatgg 1020

tgggaagaca acagagaaag aatacaagct caatatcttt ga 1062

<210> 38
<211> 353
<212> PRT
<213> Glycine max

<400> 38

Met Phe Thr Cys Ile Ala Cys Thr Lys Gln Ala Ala Glu Glu Lys Glu
1               5                   10                  15

Glu Glu Glu Glu Gly Glu Ser Gly Thr Thr Ser Thr Asn Lys Glu Ala
            20                  25                  30

Val Lys Ser Leu Ser Ala Gln Leu Lys Asp Met Ala Leu Lys Phe Ser
        35                  40                  45

Gly Ala Tyr Lys Gln Cys Lys Pro Cys Thr Gly Ser Ser Thr Tyr Lys
    50                  55                  60

Asn Gly Gln Arg Ser Tyr Pro Asp Phe Asp Thr Ile Ser Glu Gly Val
65                  70                  75                  80

Pro Tyr Pro Tyr Ile Gly Gly Ala Ser Ser Thr Ser Thr Pro Ala Trp
                85                  90                  95

Asp Phe Thr Ser Ser Asn Phe Pro Gly Gly Arg Ser Asp Gln Arg Phe
            100                 105                 110

Met Gly Arg Phe Ser Gly Asp Arg Thr Pro Arg Gly Pro Gln Ser Ala
        115                 120                 125

Pro Ala Ser Asp Val Val Val Val Glu Asp Glu Asp Glu Thr Lys Glu
    130                 135                 140

Trp Met Ala Gln Val Glu Pro Gly Val His Ile Thr Phe Val Ser Leu
145                 150                 155                 160

Pro Asn Gly Gly Asn Asp Leu Lys Arg Ile Arg Phe Ser Arg Glu Ile
                165                 170                 175

Phe Asp Lys Trp Gln Ala Gln Lys Trp Trp Gly Glu Asn Tyr Asp Arg
            180                 185                 190

Ile Met Glu Leu Tyr Asn Val Gln Arg Phe Asn Arg Gln Ala Leu Asn
        195                 200                 205

Thr Pro Ser Arg Ser Glu Asp Glu Arg Arg Asp Ser Ser Tyr Ser Arg
    210                 215                 220

Met Thr Ser Gly Gln Asp Ser Pro Met His Ser Met Ser Leu Lys Gly
225                 230                 235                 240

Trp Thr Pro Arg Asn His Tyr Lys Pro Ser Gly Asn Asn Pro Ser Glu
                245                 250                 255

Ala Met Glu Gln Gly Ser Gly Gly Gly Gln Asp Phe His Ala Ala Ala
            260                 265                 270

Ser Ser Val Glu Ala Ser Arg Thr Thr Thr Ser Ser Arg Asp Glu Arg
        275                 280                 285

Ser Met Ser Asn Ala Ser Asp Leu Glu Thr Glu Trp Ile Glu Gln Asp
    290                 295                 300

Glu Pro Gly Val Tyr Ile Thr Ile Arg Gln Leu Ala Asp Gly Thr Lys
305                 310                 315                 320

Glu Leu Arg Arg Val Arg Phe Ser Arg Glu Arg Phe Gly Glu Gly His
                325                 330                 335

Ala Lys Lys Trp Trp Glu Asp Asn Arg Glu Arg Ile Gln Ala Gln Tyr
            340                 345                 350

Leu

<210>  39
<211>  1080
<212>  DNA
<213>  Glycine max

<400>  39
atgcttacgt gcatagcacg tccgaagaaa ctcgtcggcg actcggcagc gagcgaggat      60

ccgagttcgc ggggagtgaa gtcgctgacg ggtcagctga aggagatggc gctgaaggcg     120

tcgggggcgt acaagcagtg cggcgggccg tgcgcgacgg cgccgccgag tcgagtgagt     180

cgcggcggcg gaaccgagtt ggactcggag tcgtcgtcgt cgtcttcgtc tcggcggcgg     240

tgggggaagg agctggaggc acggctgaag gggatatcga gcggggaggg gacgccgagc     300

tcgagcggga ggagggtggt gctgctgctt gaagacgagg aagagccgaa cgagtgggtg     360

gcgcaggttg agcccggcgt tttgatcacc ttcgtgtcgc ttccccgtgg cgggaaccat     420

ctcaaacgga tacgattcag ccgtgagata tttaacaaat ggcaagctca aagatggtgg     480

gcagagaact atgacaaggt gatggaactt tacaatgtgc aaaggcttaa tcgtcaagct     540

ttccctcttc caactccgcc tagatctgaa gacgagagtt caaagcgtga atcaatagaa     600

gatttcccag tgacacctcc actgagcagg gaaaggccac catgtaactt attccgtgca     660

ggaggaggag ggatgggaat ggggtactca tcctcagatt catttgatca tcactcaatg     720

caatcatccc ggcattacta tgaccccaat gacgttaact caaccccaaa agcttcttcc     780

accattagtg ctgctgccaa gacagatatc tcatcatcaa tggatgttga tgcttccata     840

agaagtagct cttctagaga agctgatcgc tctggggacc tgtcaatcag caatgccagt     900

gaccttgaca ctgaatgggt tgagcaggat gagcctgggg tttacatcac catcagagct     960

ctcccaggtg gcaaaaagga gctcagacga gtcaggttca gtcgagaaaa gtttggtgag    1020

atgcatgcta gactgtggtg ggaagagaac cgtgccagaa tacatgaaca atacttgtga    1080

<210>  40
<211>  359
<212>  PRT
<213>  Glycine max

<400>  40

Met Leu Thr Cys Ile Ala Arg Pro Lys Lys Leu Val Gly Asp Ser Ala
1               5                   10                  15

Ala Ser Glu Asp Pro Ser Ser Arg Gly Val Lys Ser Leu Thr Gly Gln
            20                  25                  30

Leu Lys Glu Met Ala Leu Lys Ala Ser Gly Ala Tyr Lys Gln Cys Gly
        35                  40                  45

Gly Pro Cys Ala Thr Ala Pro Pro Ser Arg Val Ser Arg Gly Gly Gly
    50                  55                  60

Thr Glu Leu Asp Ser Glu Ser Ser Ser Ser Ser Ser Ser Arg Arg Arg
65                  70                  75                  80

Trp Gly Lys Glu Leu Glu Ala Arg Leu Lys Gly Ile Ser Ser Gly Glu
                85                  90                  95

Gly Thr Pro Ser Ser Ser Gly Arg Arg Val Val Leu Leu Leu Glu Asp
            100                 105                 110

Glu Glu Glu Pro Asn Glu Trp Val Ala Gln Val Glu Pro Gly Val Leu
        115                 120                 125

Ile Thr Phe Val Ser Leu Pro Arg Gly Gly Asn His Leu Lys Arg Ile
    130                 135                 140

Arg Phe Ser Arg Glu Ile Phe Asn Lys Trp Gln Ala Gln Arg Trp Trp
145                 150                 155                 160

Ala Glu Asn Tyr Asp Lys Val Met Glu Leu Tyr Asn Val Gln Arg Leu
                165                 170                 175

Asn Arg Gln Ala Phe Pro Leu Pro Thr Pro Pro Arg Ser Glu Asp Glu
            180                 185                 190

Ser Ser Lys Arg Glu Ser Ile Glu Asp Phe Pro Val Thr Pro Pro Leu
        195                 200                 205

Ser Arg Glu Arg Pro Pro Cys Asn Leu Phe Arg Ala Gly Gly Gly Gly
    210                 215                 220

Met Gly Met Gly Tyr Ser Ser Ser Asp Ser Phe Asp His His Ser Met
225                 230                 235                 240

Gln Ser Ser Arg His Tyr Tyr Asp Pro Asn Asp Val Asn Ser Thr Pro
                245                 250                 255

Lys Ala Ser Ser Thr Ile Ser Ala Ala Ala Lys Thr Asp Ile Ser Ser
            260                 265                 270

Ser Met Asp Val Asp Ala Ser Ile Arg Ser Ser Ser Ser Arg Glu Ala
        275                 280                 285

Asp Arg Ser Gly Asp Leu Ser Ile Ser Asn Ala Ser Asp Leu Asp Thr
    290                 295                 300

Glu Trp Val Glu Gln Asp Glu Pro Gly Val Tyr Ile Thr Ile Arg Ala
305                 310                 315                 320

Leu Pro Gly Gly Lys Lys Glu Leu Arg Arg Val Arg Phe Ser Arg Glu

325                 330                 335

Lys Phe Gly Glu Met His Ala Arg Leu Trp Trp Glu Glu Asn Arg Ala
            340                 345                 350

Arg Ile His Glu Gln Tyr Leu
        355

<210>  41
<211>  1110
<212>  DNA
<213>  Medicago truncatula

<400>  41
atgttgactt gcatagcttg ttcaaagcaa ctcaataatg gatctcttca tcaacaagaa      60

gaagatgaag ctgtgcaaac acctagcacc aaacaagcca tcaaggctct cactgctcag     120

atcaaggaca tggcagtaaa ggcttctggg gcatataaga attgcaagcc ttgttcagga     180

tcttcaaatg gtaacaagaa caaaaaatat gctgattctg acatgggatc agattcagca     240

aggttcaact gggcgtaccg aagaaccggg agcgcaagtt caacaccaag aatgtggggg     300

aaggaaatgg aagcgagact caaagggatt tcaagtggag aaggaacacc aacatcggtg     360

agcggacgta ccgagtcagt tgtgttcatg gaagaagagg atgaacctaa ggaatgggtt     420

gcacaagttg aacctggtgt gcttattact tttgtttcat tgcctgaagg tgggaatgat     480

ttgaagagga tacggttcag tcgcgaaatg tttaataaat ggcaagctca gagatggtgg     540

gcggagaact atgacaaggt tatggaactg tacaatgttc aaaggttcaa tcaagaagca     600

ggtcctcttc caaccccacc tagatctgaa gatgagagtt cgaagattga atctgcgagg     660

gacagccccg tcacacctcc tctaaccaaa gagcgcctgc cccgtcattt gcaccaccca     720

atgacaatgg gctactcctc atcagattca ctggatcacc accatatgca acctcaacct     780

tgctatgaaa caagtggtct gccatcaaaa tctaatcttt ccaacattgg tgtgccaaag     840

accgaaagat catccattga tgcttctgta aggacgagtt catcagaaga ggaagatcac     900

tcaggtgagc tctcaatcag caatgccagt gacatggaaa ctgaatgggt tgaacaggac     960

gaaccgggag tatacatcac tatcagagca ctaccaggtg gaaccagaga acttaggcgt    1020

gtccgtttca gccgagagaa gtttggagaa atgcatgcga gattgtggtg ggaagagaac    1080

cgtgcgagga tacaagagca atatttgtga                                     1110

<210>  42
<211>  369
<212>  PRT
<213>  Medicago truncatula

<400>  42

Met Leu Thr Cys Ile Ala Cys Ser Lys Gln Leu Asn Asn Gly Ser Leu
1               5                   10                  15

His Gln Gln Glu Glu Asp Glu Ala Val Gln Thr Pro Ser Thr Lys Gln
            20                  25                  30

Ala Ile Lys Ala Leu Thr Ala Gln Ile Lys Asp Met Ala Val Lys Ala
        35                  40                  45

Ser Gly Ala Tyr Lys Asn Cys Lys Pro Cys Ser Gly Ser Ser Asn Gly
    50                  55                  60

Asn Lys Asn Lys Lys Tyr Ala Asp Ser Asp Met Gly Ser Asp Ser Ala
65                  70                  75                  80

Arg Phe Asn Trp Ala Tyr Arg Arg Thr Gly Ser Ala Ser Ser Thr Pro
                85                  90                  95

Arg Met Trp Gly Lys Glu Met Glu Ala Arg Leu Lys Gly Ile Ser Ser
            100                 105                 110

Gly Glu Gly Thr Pro Thr Ser Val Ser Gly Arg Thr Glu Ser Val Val
        115                 120                 125

Phe Met Glu Glu Glu Asp Glu Pro Lys Glu Trp Val Ala Gln Val Glu
    130                 135                 140

Pro Gly Val Leu Ile Thr Phe Val Ser Leu Pro Glu Gly Gly Asn Asp
145                 150                 155                 160

Leu Lys Arg Ile Arg Phe Ser Arg Glu Met Phe Asn Lys Trp Gln Ala
                165                 170                 175

Gln Arg Trp Trp Ala Glu Asn Tyr Asp Lys Val Met Glu Leu Tyr Asn
            180                 185                 190

Val Gln Arg Phe Asn Gln Glu Ala Gly Pro Leu Pro Thr Pro Pro Arg
        195                 200                 205

Ser Glu Asp Glu Ser Ser Lys Ile Glu Ser Ala Arg Asp Ser Pro Val
    210                 215                 220

Thr Pro Pro Leu Thr Lys Glu Arg Leu Pro Arg His Leu His His Pro
225                 230                 235                 240

Met Thr Met Gly Tyr Ser Ser Ser Asp Ser Leu Asp His His His Met
                245                 250                 255

Gln Pro Gln Pro Cys Tyr Glu Thr Ser Gly Leu Pro Ser Lys Ser Asn
            260                 265                 270

Leu Ser Asn Ile Gly Val Pro Lys Thr Glu Arg Ser Ser Ile Asp Ala
        275                 280                 285

Ser Val Arg Thr Ser Ser Ser Glu Glu Glu Asp His Ser Gly Glu Leu
    290                 295                 300

Ser Ile Ser Asn Ala Ser Asp Met Glu Thr Glu Trp Val Glu Gln Asp
305                 310                 315                 320

Glu Pro Gly Val Tyr Ile Thr Ile Arg Ala Leu Pro Gly Gly Thr Arg
                325                 330                 335

Glu Leu Arg Arg Val Arg Phe Ser Arg Glu Lys Phe Gly Glu Met His
            340                 345                 350

Ala Arg Leu Trp Trp Glu Glu Asn Arg Ala Arg Ile Gln Glu Gln Tyr
        355                 360                 365

Leu

<210> 43
<211> 1116
<212> DNA
<213> Nicotiana benthamiana

<400> 43
atgcttacgt gcatcgctcg ttcgaaacaa ccgagcgatg attcgcgtga tcatcaacct 60

gagaaattca atccaaattc agctccggcc aataaacaag ctgttaaaac actcacttct 120

cagattaagg acatggcatt gaaagcatca ggagcatata agcagtgtag tccatgtaca 180

actcagccaa cgactctgcg gaagaacgga agccaaaacg agtcgtcgga ctcggcgtcg 240

ttggataaat tccggtggtc ttacaagaga acaggaagtt caagttcgag ctcgacggct 300

gggagaaagg aactggaggc gaggctgaaa gggatatcga gcggagaagt tacgccggtg 360

tctgcgtcag cgagtggccg gcgagttgaa ccggtcgtgt tcgttgaaga aagcgagcca 420

aaagagtggg tggcgcaagt tgaaccgggt gtgctcatca ctttcgtttc gttaccacgt 480

ggcggcaatg atctcaagcg gattcgattc agtcgagaaa tatttaataa atatcaagct 540

cagaggtggt gggcagagaa ttgtgagaag gtaatggagc tttataatgt ccagaggttg 600

aatcgccaag ctttccccct gccaacacct ccaagatctg aagacgggag ttcaaaaatt 660

gaatccatcg aagacagtcc agtgacaccc ccgctcacta aagaacgact gccccataca 720

ttgtaccgtc caatgtattc atcttcagat tctcttgaac aacactcgac acattttcgc 780

tacaattatg attcttgtgg tgtcgcctca actccaaaac tctcaagcat cagtggagca 840

aagacagaga tatcatctat ggatgcctct attaggacta gcacatcaag agatgcagat 900

cgctctggag agctatccat cagcaatgcc agtgatcttg aaactgaatg ggttgagcaa 960

gatgagcctg gagtttacat cactatacaa gcgctaccaa atggcagaag agaactcaaa 1020

cgagttagat tcagccgaga aaaatttgga gaagtgcacg ccaggttatg gtgggaagag 1080

aatcgtgcta ggatacacaa acaatacctg ggatag 1116

<210> 44
<211> 252
<212> DNA
<213> Nicotiana benthamiana

<220>
<221> misc_feature
<222> (16)..(17)
<223> n is a, c, g, or t

<220>
<221> misc_feature
<222> (21)..(21)
<223> n is a, c, g, or t

<220>
<221> misc_feature
<222> (49)..(49)
<223> n is a, c, g, or t

<220>
<221> misc_feature
<222> (52)..(52)
<223> n is a, c, g, or t

<220>
<221> misc_feature
<222> (116)..(116)
<223> n is a, c, g, or t

<220>
<221> misc_feature
<222> (123)..(123)
<223> n is a, c, g, or t

<220>
<221> misc_feature
<222> (131)..(131)
<223> n is a, c, g, or t

<220>
<221> misc_feature
<222> (137)..(137)
<223> n is a, c, g, or t

<220>
<221> misc_feature

<222>  (140)..(140)
<223>  n is a, c, g, or t

<220>
<221>  misc_feature
<222>  (176)..(176)
<223>  n is a, c, g, or t

<220>
<221>  misc_feature
<222>  (214)..(214)
<223>  n is a, c, g, or t

<220>
<221>  misc_feature
<222>  (227)..(227)
<223>  n is a, c, g, or t

<220>
<221>  misc_feature
<222>  (245)..(245)
<223>  n is a, c, g, or t

<400>  44
mtcarsksdd srdhknnsaa nkavkttskd makasgaykc scttttrkng snssdsasdk      60

rwsykrtgss sssstagrka rkgssgvtvs asasgrrvvv vskwvavgvt vsrggndkrr     120

srnkyarwwa nckvmynvrn ratrsdgssk sdsvttkrht yrmysssdsh sthrynydsc     180

gvastksssg aktssmdasr tstsrdadrs gssnasdtwv dgvytangrr krvrsrkgvh     240

arwwnrarhk yg                                                         252


<210>  45
<211>  1194
<212>  DNA
<213>  Oryza sativa

<400>  45
atgctcacgt gcatcgcgtg ctccaagcag ctcgccggcg gcgcgccgcc gctgcgtgag      60

cagtccgacg acgccgacga cgccgccgtt gccagaggcg ccggcgagtg cgccacgccc     120

agcacgaggc aggccatcaa ggcgctcacc gcccagatca aggacatggc gctgaaggcg     180

tcgggcgcgt accggcactg caagccgtgc gccggctcgt cgtcgtcgtc accggcggcg     240

ggggcgcggc ggcaccaccc gtaccacgcg tacgcggact ccgggtccga ccgcttccac     300

tacgcgtaca ggcgagccgg cagcggtggc gacgccaccc cgtcggtgag cgcgcgcacc     360

gacttcctcg ccggggacga ggaggaggag gaggaggagg aggaggagga ggggacgacg     420

gcggacggca gcgaggacga cgaggcgaag gagtgggtcg cccaggtgga gcccggcgtg     480

ctcatcacct tcctctcgct gccggagggc ggcaacgacc tcaaacggat ccgattcagc     540

cgtgagatat tcaacaaatg gcaagcgcaa agatggtggg ccgaaaatta tgagaaagtc     600

atggagcttt acaatgttca gaggttcaac cagcaaactc cccttcctac tactccaaag     660

tctgaagatg agagcttgaa ggaggatatc ccagcaacac caccactcaa cagtgaacgt 720

ctaccccaca ctttgcacag atcactaact ggtggcagaa caactgggta tggacaacca 780

gattctcttg ggcatcagca caaccttggt aatggtcatc gccaacagca ccatcactgc 840

tacactggac accagtgcta tggttcagtt gggctggcat caacacctaa gttatcaagc 900

attagtggag caaagacaga aacttcgtca atggatgcat cgatgaggtc aagctcatct 960

cctgaagagg tggatcgatc tcgtgaactt tcagtctctg ttagcaatgc aagtgaccaa 1020

gagagggaat gggtagaaga ggatgaacct ggtgtataca tcaccattcg ggccttgcct 1080

ggtggtatta gagagctccg acgggtccga ttcagccggg agaaattcag cgagatgcac 1140

gccaggctat ggtgggaaga gaaccgggca aggatacatg atcagtatct ctga 1194

<210> 46
<211> 397
<212> PRT
<213> Oryza sativa

<400> 46

Met Leu Thr Cys Ile Ala Cys Ser Lys Gln Leu Ala Gly Gly Ala Pro
1               5                   10                  15

Pro Leu Arg Glu Gln Ser Asp Asp Ala Asp Asp Ala Ala Val Ala Arg
            20                  25                  30

Gly Ala Gly Glu Cys Ala Thr Pro Ser Thr Arg Gln Ala Ile Lys Ala
        35                  40                  45

Leu Thr Ala Gln Ile Lys Asp Met Ala Leu Lys Ala Ser Gly Ala Tyr
    50                  55                  60

Arg His Cys Lys Pro Cys Ala Gly Ser Ser Ser Ser Ser Pro Ala Ala
65                  70                  75                  80

Gly Ala Arg Arg His His Pro Tyr His Ala Tyr Ala Asp Ser Gly Ser
                85                  90                  95

Asp Arg Phe His Tyr Ala Tyr Arg Arg Ala Gly Ser Gly Gly Asp Ala
            100                 105                 110

Thr Pro Ser Val Ser Ala Arg Thr Asp Phe Leu Ala Gly Asp Glu Glu
        115                 120                 125

Glu Glu Glu Glu Glu Glu Glu Glu Glu Gly Thr Thr Ala Asp Gly Ser
    130                 135                 140

Glu Asp Asp Glu Ala Lys Glu Trp Val Ala Gln Val Glu Pro Gly Val

145                 150                 155                 160

Leu Ile Thr Phe Leu Ser Leu Pro Glu Gly Gly Asn Asp Leu Lys Arg
                165                 170                 175

Ile Arg Phe Ser Arg Glu Ile Phe Asn Lys Trp Gln Ala Gln Arg Trp
            180                 185                 190

Trp Ala Glu Asn Tyr Glu Lys Val Met Glu Leu Tyr Asn Val Gln Arg
        195                 200                 205

Phe Asn Gln Gln Thr Pro Leu Pro Thr Thr Pro Lys Ser Glu Asp Glu
    210                 215                 220

Ser Leu Lys Glu Asp Ile Pro Ala Thr Pro Pro Leu Asn Ser Glu Arg
225                 230                 235                 240

Leu Pro His Thr Leu His Arg Ser Leu Thr Gly Gly Arg Thr Thr Gly
                245                 250                 255

Tyr Gly Gln Pro Asp Ser Leu Gly His Gln His Asn Leu Gly Asn Gly
            260                 265                 270

His Arg Gln Gln His His His Cys Tyr Thr Gly His Gln Cys Tyr Gly
        275                 280                 285

Ser Val Gly Leu Ala Ser Thr Pro Lys Leu Ser Ser Ile Ser Gly Ala
    290                 295                 300

Lys Thr Glu Thr Ser Ser Met Asp Ala Ser Met Arg Ser Ser Ser Ser
305                 310                 315                 320

Pro Glu Glu Val Asp Arg Ser Arg Glu Leu Ser Val Ser Val Ser Asn
                325                 330                 335

Ala Ser Asp Gln Glu Arg Glu Trp Val Glu Glu Asp Glu Pro Gly Val
            340                 345                 350

Tyr Ile Thr Ile Arg Ala Leu Pro Gly Gly Ile Arg Glu Leu Arg Arg
        355                 360                 365

Val Arg Phe Ser Arg Glu Lys Phe Ser Glu Met His Ala Arg Leu Trp
    370                 375                 380

Trp Glu Glu Asn Arg Ala Arg Ile His Asp Gln Tyr Leu
385                 390                 395

<210>  47
<211>  1236
<212>  DNA
<213>  Oryza sativa

<400>  47
atgcttgcgt gcatcgcgtg ttcgaccaag gacggcgggg agggcgggca ccggtccgcc      60

accgccacgc ccaactccgg caagtctctg acttcacagt tgaaggacat ggtgctcaag     120

ttctccggca gcggcaggca ccaatacaag tccggcggga gcccgtcgtt gaggaccagc     180

cgcttccacc gctccagccg cctcgccgcg tacccggcca tcatcgacga gtcgggcttc     240

acgtcggacg gggccggcga ggcctatact tacatgagga cgacgacggc gagcgccggc     300

gcccgggccg cgccgtcgac gtgggacttg ccgcccaagg tgaaccaccg cagcttccag     360

ccgcgcgtga tcaggagccc gagcgcgagc ggggtaccga gcatcgggga ggaagactac     420

gacgacgacg acgacgacga tgacgaggag acagtcctcc tggaggagga ccgcgtgccg     480

cgggagtgga cggcgcaggt ggagcccggc gtgcagatca ccttcgtctc catccccggc     540

ggcgccggca acgatctgaa gcgcatccgt ttcagccggg agatgtttaa caagtgggag     600

gcgcagcggt ggtgggggga gaactacgac cgcgtggtgg agctctacaa cgtgcagacg     660

ttcagccggc agcagggctt ctcgacgccg acgtcctccg tcgacgaagc catgcagaga     720

gattcgttct actcccgcgt cggctcgacg agggagagcc cggcgatgat gatgccgccg     780

ccgccgccgc tgccgtcgtc gggtgctggc agggagcacc cgatcagccg gacagcgtcg     840

agcaaggcgc agctgtcgtc gtcgtcgtcg gtggcggcgg ctcggccgcc gttctacccg     900

tccacggccg tgccggaccc gtccgaccac gtgtgggcgc accacttcaa cctcctcaac     960

tccgcggccg ccggaccagc ggcgccgtac gacccgtcgc gcggcacgac gtcgtcccgg    1020

gacgaggcgt ccgtgtccat cagcaacgcg agcgacctgg aggccacgga gtgggtggag    1080

caggacgagc ctggggtgtc catcaccatc cgcgagttcg gcgacggcac ccgcgagctc    1140

cgccgcgtcc ggttcagccg cgagagattt ggcgaggagc gggccaaggt gtggtgggag    1200

cagaaccggg accggataca cgcgcagtat ctgtga                              1236


<210>  48
<211>  411
<212>  PRT
<213>  Oryza sativa

<400>  48

Met Leu Ala Cys Ile Ala Cys Ser Thr Lys Asp Gly Gly Glu Gly Gly
1               5                   10                  15


His Arg Ser Ala Thr Ala Thr Pro Asn Ser Gly Lys Ser Leu Thr Ser
            20                  25                  30

Gln Leu Lys Asp Met Val Leu Lys Phe Ser Gly Ser Gly Arg His Gln
        35                  40                  45

Tyr Lys Ser Gly Gly Ser Pro Ser Leu Arg Thr Ser Arg Phe His Arg
    50                  55                  60

Ser Ser Arg Leu Ala Ala Tyr Pro Gly Ile Ile Asp Glu Ser Gly Phe
65                  70                  75                  80

Thr Ser Asp Gly Ala Gly Glu Ala Tyr Thr Tyr Met Arg Thr Thr Thr
                85                  90                  95

Ala Ser Ala Gly Ala Arg Ala Ala Pro Ser Thr Trp Asp Leu Pro Pro
            100                 105                 110

Lys Val Asn His Arg Ser Phe Gln Pro Arg Val Ile Arg Ser Pro Ser
        115                 120                 125

Ala Ser Gly Val Pro Ser Ile Gly Glu Glu Asp Tyr Asp Asp Asp Asp
    130                 135                 140

Asp Asp Asp Asp Glu Glu Thr Val Leu Leu Glu Glu Asp Arg Val Pro
145                 150                 155                 160

Arg Glu Trp Thr Ala Gln Val Glu Pro Gly Val Gln Ile Thr Phe Val
                165                 170                 175

Ser Ile Pro Gly Gly Ala Gly Asn Asp Leu Lys Arg Ile Arg Phe Ser
            180                 185                 190

Arg Glu Met Phe Asn Lys Trp Glu Ala Gln Arg Trp Trp Gly Glu Asn
        195                 200                 205

Tyr Asp Arg Val Val Glu Leu Tyr Asn Val Gln Thr Phe Ser Arg Gln
    210                 215                 220

Gln Gly Phe Ser Thr Pro Thr Ser Ser Val Asp Glu Ala Met Gln Arg
225                 230                 235                 240

Asp Ser Phe Tyr Ser Arg Val Gly Ser Thr Arg Glu Ser Pro Ala Met
                245                 250                 255

Met Met Pro Pro Pro Pro Pro Leu Pro Ser Ser Gly Ala Gly Arg Glu
            260                 265                 270

His Pro Ile Ser Arg Thr Ala Ser Ser Lys Ala Gln Leu Ser Ser Ser
        275                 280                 285

Ser Ser Val Ala Ala Ala Arg Pro Pro Phe Tyr Pro Ser Thr Ala Val
    290                 295                 300

Pro Asp Pro Ser Asp His Val Trp Ala His His Phe Asn Leu Leu Asn
305                 310                 315                 320

Ser Ala Ala Ala Gly Pro Ala Ala Pro Tyr Asp Pro Ser Arg Gly Thr
                325                 330                 335

Thr Ser Ser Arg Asp Glu Ala Ser Val Ser Ile Ser Asn Ala Ser Asp
            340                 345                 350

Leu Glu Ala Thr Glu Trp Val Glu Gln Asp Glu Pro Gly Val Ser Ile
        355                 360                 365

Thr Ile Arg Glu Phe Gly Asp Gly Thr Arg Glu Leu Arg Arg Val Arg
    370                 375                 380

Phe Ser Arg Glu Arg Phe Gly Glu Glu Arg Ala Lys Val Trp Trp Glu
385                 390                 395                 400

Gln Asn Arg Asp Arg Ile His Ala Gln Tyr Leu
                405                 410

<210>  49
<211>  642
<212>  DNA
<213>  Oryza sativa

<400>  49
atgctggcat gcatcgcgtg ctcctccaag gaaggcgggg aggacgggtc ccgtggcgcg      60

gccacgcctc acggcagaga tgccgtcaag tccctcacct cacagctcaa ggacatggtg     120

ctcaagttct ccggctccaa caagcatcag cactacaagg cggcgacggc cggtagccct     180

tcgttcagga gcaggagcta ccgccgtccg tacccgggct tcatcgatga ctccgcgttc     240

atgacgacga ccaggcctgg aggtgaggcc tacatgtaca cgagagcggc gccgccgccg     300

cccgtccggg ccgcgtcgac gtcgatggcg acatgggaca tgaccaggag caagagcaac     360

cgagggtggc agcaggatgc cggcaggagc cccggcggca cgacgtggat acagagcatc     420

gaggaggagg ccggcgccga cgacgtcacc gtcgtggagg atgccgtgcc gagggagtgg     480

acggcgcaga tggagcccgg cgtccagatc accttcgtca cgctccccgg cggtggcaac     540

gacctcaagc gcatccggtt cagccgcgag aggtttggtg aggacagggc gaaggtttgg     600

tgggagcaca acagagacag aatacaagca cagtatctgt aa                        642

<210>  50
<211>  213
<212>  PRT
<213>  Oryza sativa

<400>  50

Met Leu Ala Cys Ile Ala Cys Ser Ser Lys Glu Gly Gly Glu Asp Gly
1               5                   10                  15

Ser Arg Gly Ala Ala Thr Pro His Gly Arg Asp Ala Val Lys Ser Leu
            20                  25                  30

Thr Ser Gln Leu Lys Asp Met Val Leu Lys Phe Ser Gly Ser Asn Lys
        35                  40                  45

His Gln His Tyr Lys Ala Ala Thr Ala Gly Ser Pro Ser Phe Arg Ser
    50                  55                  60

Arg Ser Tyr Arg Arg Pro Tyr Pro Gly Phe Ile Asp Asp Ser Ala Phe
65                  70                  75                  80

Met Thr Thr Thr Arg Pro Gly Gly Glu Ala Tyr Met Tyr Thr Arg Ala
                85                  90                  95

Ala Pro Pro Pro Pro Val Arg Ala Ala Ser Thr Ser Met Ala Thr Trp
            100                 105                 110

Asp Met Thr Arg Ser Lys Ser Asn Arg Gly Trp Gln Gln Asp Ala Gly
        115                 120                 125

Arg Ser Pro Gly Gly Thr Thr Trp Ile Gln Ser Ile Glu Glu Glu Ala
    130                 135                 140

Gly Ala Asp Asp Val Thr Val Val Glu Asp Ala Val Pro Arg Glu Trp
145                 150                 155                 160

Thr Ala Gln Met Glu Pro Gly Val Gln Ile Thr Phe Val Thr Leu Pro
                165                 170                 175

Gly Gly Gly Asn Asp Leu Lys Arg Ile Arg Phe Ser Arg Glu Arg Phe
            180                 185                 190

Gly Glu Asp Arg Ala Lys Val Trp Trp Glu His Asn Arg Asp Arg Ile
        195                 200                 205

Gln Ala Gln Tyr Leu
    210

<210>  51
<211>  1110
<212>  DNA
<213>  Oryza sativa

<400>  51
atgctggcct gcatcgcctg cgtcaagcag gaagatggcg gccgccgcca ggacacgccg      60

cgcgccggcg ccggcgccgg ggacgacacg cccacctgca gggaccccgt caagacgctc     120

acctcccagc tcaaggacat ggtgatgaag ctgtccggca cgagccgtca tcacggtcag     180

cagaggcgtg gcgggtcgcc gccgccgagg ggccggacga cgtcggtgta ccggagcggg     240

tactaccggc cgggcatggt ccaggacgac atggcggtgc cgccggcgac gtacctgggc     300

ggcggcggga cgtcgatgag ctcggcgagc tcgacgccgg cgtgggactt cgcgcggccg     360

gcggagggcg aggctcggga gtgggtggcg caggtggagc ccggggtgca gatcacgttc     420

gtgtccctcg ccggcggcgg cggcaacgac ctgaaacgca tcaggttcag ccgggagatg     480

tacgacaagt ggcaggcgca gaagtggtgg ggcgagaaca acgagcggat catggagctc     540

tacaacgtcc gccgcttcag ccgccaggtc ctccccacgc cgccgcgctc cgacgacggc     600

gagagggaat cgttctactc tcaagtgggg tcgacgaggg ggagccccgc cgccacgccg     660

tcgccggcgc cgctcacccc ggaccgcgtc accagctgga gcgccttcgt ccggccgccg     720

tcggcctcgc gccagcagca gcagcacagc ttccggccgc tgtcgccgcc gccgccgtcg     780

tcgtccaacc cgtcggagcg ggcctggcag cagcagcagc agccgcagcg cgctggcaag     840

agccccgcgg cggcgtcgga cgccatggac gcggcgagaa cgaccagctg ctcgtcgagg     900

gacgaggtgt ccatcagcaa cgccagcgag ctggaggtga cggagtgggt catacaggac     960

gagcccggcg tctacatcac cgtcagggag ctcgccgacg gcaccaggga gctccgccgc    1020

gtccgtttca gccgggagag gtttgcagag ctgaatgcta agctatggtg ggaggagaac    1080

aaggagagga tacaggcgca gtacctctag                                     1110

<210>  52
<211>  369
<212>  PRT
<213>  Oryza sativa

<400>  52

Met Leu Ala Cys Ile Ala Cys Val Lys Gln Glu Asp Gly Gly Arg Arg
1               5                   10                  15

Gln Asp Thr Pro Arg Ala Gly Ala Gly Ala Gly Asp Asp Thr Pro Thr
            20                  25                  30

Cys Arg Asp Pro Val Lys Thr Leu Thr Ser Gln Leu Lys Asp Met Val
        35                  40                  45

Met Lys Leu Ser Gly Thr Ser Arg His His Gly Gln Gln Arg Arg Gly
    50                  55                  60

Gly Ser Pro Pro Pro Arg Gly Arg Thr Thr Ser Val Tyr Arg Ser Gly
65                  70                  75                  80

Tyr Tyr Arg Pro Gly Met Val Gln Asp Asp Met Ala Val Pro Pro Ala
                85                  90                  95

Thr Tyr Leu Gly Gly Gly Gly Thr Ser Met Ser Ser Ala Ser Ser Thr
            100                 105                 110

Pro Ala Trp Asp Phe Ala Arg Pro Ala Glu Gly Glu Ala Arg Glu Trp
        115                 120                 125

Val Ala Gln Val Glu Pro Gly Val Gln Ile Thr Phe Val Ser Leu Ala
    130                 135                 140

Gly Gly Gly Gly Asn Asp Leu Lys Arg Ile Arg Phe Ser Arg Glu Met
145                 150                 155                 160

Tyr Asp Lys Trp Gln Ala Gln Lys Trp Trp Gly Glu Asn Asn Glu Arg
                165                 170                 175

Ile Met Glu Leu Tyr Asn Val Arg Arg Phe Ser Arg Gln Val Leu Pro
            180                 185                 190

Thr Pro Pro Arg Ser Asp Asp Gly Glu Arg Glu Ser Phe Tyr Ser Gln
        195                 200                 205

Val Gly Ser Thr Arg Gly Ser Pro Ala Ala Thr Pro Ser Pro Ala Pro
    210                 215                 220

Leu Thr Pro Asp Arg Val Thr Ser Trp Ser Ala Phe Val Arg Pro Pro
225                 230                 235                 240

Ser Ala Ser Arg Gln Gln Gln Gln His Ser Phe Arg Pro Leu Ser Pro
                245                 250                 255

Pro Pro Pro Ser Ser Ser Asn Pro Ser Glu Arg Ala Trp Gln Gln Gln
            260                 265                 270

Gln Gln Pro Gln Arg Ala Gly Lys Ser Pro Ala Ala Ala Ser Asp Ala
        275                 280                 285

Met Asp Ala Ala Arg Thr Thr Ser Cys Ser Ser Arg Asp Glu Val Ser
    290                 295                 300

Ile Ser Asn Ala Ser Glu Leu Glu Val Thr Glu Trp Val Ile Gln Asp
305                 310                 315                 320

Glu Pro Gly Val Tyr Ile Thr Val Arg Glu Leu Ala Asp Gly Thr Arg
                325                 330                 335

Glu Leu Arg Arg Val Arg Phe Ser Arg Glu Arg Phe Ala Glu Leu Asn
            340                 345                 350

Ala Lys Leu Trp Trp Glu Glu Asn Lys Glu Arg Ile Gln Ala Gln Tyr
        355                 360                 365

Leu

<210>  53
<211>  1272
<212>  DNA
<213>  Oryza sativa

<400>  53
atgctcacgt gcatcgcgtg ctcgaagcag cccggcggcg gcggcgggga gccgctgcac      60

gagccgccgg aggacgagga cgccgtcgat ggcggcggcg gcggcggcgg ggcgacgccc     120

agcacgcggc tggccatcaa ggcgctcact gcgcagatca aggacattgc gttgaaggcg     180

tcgggcgcgt accggcactg caagccctgc gccggctcgt cgtcggcggc gggggcctcg     240

cgccggcacc acccgtacca ccaccgcggc ggcggcggcg gcttcggcga ccccgacgcg     300

gcctcgggct ccgaccggtt ccactacgcg taccgccgcg ccacgagctc ggcggcctcg     360

acgccgcgct tccgcggcgg cggcggcggc ggcgcgctgt cgagcgggga cgctacgccg     420

tccatgagcg cccgctccga cttccccatc ggcgacgagg aggatgagga ggaagacgac     480

gacgacgaga tggtgtcaac cggcggcggc ggcggcggca aggaggagga cgcgaaggag     540

tgggtggcgc agatggagcc cggcgtgctg ataaccttcg tctcgctgcc acagggcggc     600

aacgacctca aacgcatccg gttcagccgt gagatgttca acaagtggca agcacaaaga     660

tggtgggctg aaaattatga caaagttatg gagctttaca atgtacagag atttaaccat     720

caagctgttc ctcttcctgc tactccaaaa tctgaagacg agagctcaaa ggaggacagc     780

ccggtgacac caccactggg gaaggaacgg ttacctcgtt cgttccatag accattgtca     840

ggtggtggtg cagtgggttc atcttcatca gattctcttg agcatcattc aaaccactac     900

tgtaatggtg gccaccacca ccatggacac cagtgctatg attcagtggg gctggtctca     960

acaccgaagc tgtcaagtat aagtggagcc aagacagaaa cctcgtcaat ggacgcatca    1020

atgaggacga gctcatctcc tgaagaggtc gacagatctg gtgagctctc ggtgtccatc    1080

agcaatgcaa gtgaccaaga gcgggagtgg gtcgaggaag acgagcctgg tgtatatatt 1140

accatccggg ctttgcctgg tggtatcaga gaactccggc gtgttcgatt cagccgagag 1200

aggttcagcg agatgcatgc caggctatgg tgggaagaaa accgagcgag gatacatgaa 1260

cagtatctct ga 1272

<210> 54
<211> 423
<212> PRT
<213> Oryza sativa

<400> 54

Met Leu Thr Cys Ile Ala Cys Ser Lys Gln Pro Gly Gly Gly Gly Gly
1               5                   10                  15

Glu Pro Leu His Glu Pro Pro Glu Asp Glu Asp Ala Val Asp Gly Gly
            20                  25                  30

Gly Gly Gly Gly Gly Ala Thr Pro Ser Thr Arg Leu Ala Ile Lys Ala
        35                  40                  45

Leu Thr Ala Gln Ile Lys Asp Ile Ala Leu Lys Ala Ser Gly Ala Tyr
    50                  55                  60

Arg His Cys Lys Pro Cys Ala Gly Ser Ser Ser Ala Ala Gly Ala Ser
65                  70                  75                  80

Arg Arg His His Pro Tyr His His Arg Gly Gly Gly Gly Gly Phe Gly
                85                  90                  95

Asp Pro Asp Ala Ala Ser Gly Ser Asp Arg Phe His Tyr Ala Tyr Arg
            100                 105                 110

Arg Ala Thr Ser Ser Ala Ala Ser Thr Pro Arg Phe Arg Gly Gly Gly
        115                 120                 125

Gly Gly Gly Ala Leu Ser Ser Gly Asp Ala Thr Pro Ser Met Ser Ala
    130                 135                 140

Arg Ser Asp Phe Pro Ile Gly Asp Glu Glu Asp Glu Glu Glu Asp Asp
145                 150                 155                 160

Asp Asp Glu Met Val Ser Thr Gly Gly Gly Gly Gly Gly Lys Glu Glu
                165                 170                 175

Asp Ala Lys Glu Trp Val Ala Gln Val Glu Pro Gly Val Leu Ile Thr
            180                 185                 190

Phe Val Ser Leu Pro Gln Gly Gly Asn Asp Leu Lys Arg Ile Arg Phe
        195                 200                 205

Ser Arg Glu Met Phe Asn Lys Trp Gln Ala Gln Arg Trp Trp Ala Glu
    210                 215                 220

Asn Tyr Asp Lys Val Met Glu Leu Tyr Asn Val Gln Arg Phe Asn His
225                 230                 235                 240

Gln Ala Val Pro Leu Pro Ala Thr Pro Lys Ser Glu Asp Glu Val Ser
                245                 250                 255

Lys Glu Asp Ser Pro Val Thr Pro Pro Leu Gly Lys Glu Arg Leu Pro
            260                 265                 270

Arg Ser Phe His Arg Pro Leu Ser Gly Gly Gly Ala Val Gly Ser Ser
        275                 280                 285

Ser Ser Asp Ser Leu Glu His His Ser Asn His Tyr Cys Asn Gly Gly
    290                 295                 300

His His His His Gly His Gln Cys Tyr Asp Ser Val Gly Leu Val Ser
305                 310                 315                 320

Thr Pro Lys Leu Ser Ser Ile Ser Gly Ala Lys Thr Glu Thr Ser Ser
                325                 330                 335

Met Asp Ala Ser Met Arg Thr Ser Ser Ser Pro Glu Glu Val Asp Arg
            340                 345                 350

Ser Gly Glu Leu Ser Val Ser Ile Ser Asn Ala Ser Asp Gln Glu Arg
        355                 360                 365

Glu Trp Val Glu Glu Asp Glu Pro Gly Val Tyr Ile Thr Ile Arg Ala
    370                 375                 380

Leu Pro Gly Gly Ile Arg Glu Leu Arg Arg Val Arg Phe Ser Arg Glu
385                 390                 395                 400

Arg Phe Ser Glu Met His Ala Arg Leu Trp Trp Glu Glu Asn Arg Ala
                405                 410                 415

Arg Ile His Glu Gln Tyr Leu
            420

<210>  55

<211>  2007
<212>  DNA
<213>  Physcomitrella patens

<400>  55
atgggctcca ctcactgcca tctcattacg agacccttac ttctggcccg gcttgcggcg      60

tttggtgcgg ggaacgtggc cctcttcagg gcaggaattg aactctttcg agggttgatg     120

gtgagacttg ccgaagtcgg aaggtgctcc gggtgcaagt gctggtggtg gtggttgatg     180

gagccagcaa gaagggcggg gttgatagat tgtgagatgg agggcggggc tcgcggaggc     240

ggcgaagctt gttgttcgca gggtcacggg aagtcgtgga gggagaatgt tgcaggagtg     300

cgacttcctc tcgatgagaa gatcttggtt gaggctctcg cgaaaggatt ttatttcagt     360

gttgatgtag agcagctggt ggattgcagc agaagccatc ttggtcttcg ggaagcgttt     420

atgtcctgga tcgcctgcaa tgccaggtta tgtactttga acgtagcagt gagctattgt     480

tatcttcggg atcatgcaac agttaagcgg gttttctcaa gaacaatggc cgtcaaaatg     540

ctggcttgca tgagttcgaa aaccttggaa gaggccgtcg aagccgacta tgagaaggac     600

aagacatcgt ccaagccgga gaccaaatct gacttgcgaa aaatgaagtc atttacttgt     660

cagaccctga tccatccgtc ttggtctttt aaaagccaag gaaatcaagg tgtgtcccca     720

cctctagcaa ctccacctca agcaagtcgt cagtcagagg gaaaggagga ggtcgcgtcc     780

gggaaacctg atggaaaagg tcgttgcagt actcggttgg gggcactgat gttcatattc     840

gctagtttcg atgtaatatc cctcgattgc gctgatgaca ctacgttcaa gggaatggcg     900

gctaagttga cagcaggagc ttgtacaact tcttgcaagc cttgttcaac ttctgtttca     960

gagcaagacg ctgcgcatgc tgtccatcca gaggcttcat tggacaaata cagtgaggct    1020

gatttttcgc taccagtggc caattatcga aatcctaatg tgttgacaga tgatcaagga    1080

aataacctcg gaagtaataa tggagacggc tcaggttctc cgcgttctgc tcaagcggtc    1140

tccccggagc agaaaatcaa tgcaagtctt tttagtaaaa ctcctacccc aattgaaagc    1200

ccagttgttg cgagctatca catgagcaat ggaactgctt tgaagattcc tgaagaacct    1260

caaaatttga atcgtcttat gccggctgaa ccaggtgttc ctggcactga atggttttca    1320

caagtagagc ttggagtatt cataacgttt gtgactctac ctaacggttg caatgctcta    1380

aaacgaatta gattcagccg agacattttc agcaaaaaag aagctgaatc gtggtgggcg    1440

gagaatggaa atcgtgtccg tgaagtctat aacgtaccag ctttcgaaag gacaacgaca    1500

aatggtcacc aggctacttc aagttcagaa gaagaggttt caggggtatc agggtatgct    1560

accccctcct atagtccgca ggggtcacga ggagcttcaa caagggattc accggctggc    1620

tacagttctg ggatatcgcg tggagcatct ttgagggata catcgagcag agaggcctca    1680

atgagagaac cgtcgatcag agaatccatc agacagtcga tgagggatgc ggtcagcgaa    1740

cattccgaat ccgcaacgtg tactgagaga gagactgaaa cagatacagt tgctggaagc 1800

gtggcaggta gcgacagaac ttatgacggc gaagaatcca cttgggttga ggaagacgtt 1860

cctggtgtat atttaacatt gaagaatttg actggaggtg gtagagagtt gaagcgtgtc 1920

aggttcagtc gtgaaaagtt caccgagaaa caggccaaaa tatggtggga cgagaaccgc 1980

gggcgaattc acaaacagta tctatga 2007

<210> 56
<211> 668
<212> PRT
<213> Physcomitrella patens

<400> 56

Met Gly Ser Thr His Cys His Leu Ile Thr Arg Pro Leu Leu Leu Ala
1               5                   10                  15

Arg Leu Ala Ala Phe Gly Ala Gly Asn Val Ala Leu Phe Arg Ala Gly
            20                  25                  30

Ile Glu Leu Phe Arg Gly Leu Met Val Arg Leu Ala Glu Val Gly Arg
        35                  40                  45

Cys Ser Gly Cys Lys Cys Trp Trp Trp Trp Leu Met Glu Pro Ala Arg
    50                  55                  60

Arg Ala Gly Leu Ile Asp Cys Glu Met Glu Gly Gly Ala Arg Gly Gly
65                  70                  75                  80

Gly Glu Ala Cys Cys Ser Gln Gly His Gly Lys Ser Trp Arg Glu Asn
                85                  90                  95

Val Ala Gly Val Arg Leu Pro Leu Asp Glu Lys Ile Leu Val Glu Ala
            100                 105                 110

Leu Ala Lys Gly Phe Tyr Phe Ser Val Asp Val Glu Gln Leu Val Asp
        115                 120                 125

Cys Ser Arg Ser His Leu Gly Leu Arg Glu Ala Phe Met Ser Trp Ile
    130                 135                 140

Ala Cys Asn Ala Arg Leu Cys Thr Leu Asn Val Ala Val Ser Tyr Cys
145                 150                 155                 160

Tyr Leu Arg Asp His Ala Thr Val Lys Arg Val Phe Ser Arg Thr Met
                165                 170                 175

Ala Val Lys Met Leu Ala Cys Met Ser Ser Lys Thr Leu Glu Glu Ala

            180                 185                 190

Val Glu Ala Asp Tyr Glu Lys Asp Lys Thr Ser Ser Lys Pro Glu Thr
        195                 200                 205

Lys Ser Asp Leu Arg Lys Met Lys Ser Phe Thr Cys Gln Thr Leu Ile
    210                 215                 220

His Pro Ser Trp Ser Phe Lys Ser Gln Gly Asn Gln Gly Val Ser Pro
225                 230                 235                 240

Pro Leu Ala Thr Pro Pro Gln Ala Ser Arg Gln Ser Glu Gly Lys Glu
                245                 250                 255

Glu Val Ala Ser Gly Lys Pro Asp Gly Lys Gly Arg Cys Ser Thr Arg
            260                 265                 270

Leu Gly Ala Leu Met Phe Ile Phe Ala Ser Phe Asp Val Ile Ser Leu
        275                 280                 285

Asp Cys Ala Asp Asp Thr Thr Phe Lys Gly Met Ala Ala Lys Leu Thr
    290                 295                 300

Ala Gly Ala Cys Thr Thr Ser Cys Lys Pro Cys Ser Thr Ser Val Ser
305                 310                 315                 320

Glu Gln Asp Ala Ala His Ala Val His Pro Glu Ala Ser Leu Asp Lys
                325                 330                 335

Tyr Ser Glu Ala Asp Phe Ser Leu Pro Val Ala Asn Tyr Arg Asn Pro
            340                 345                 350

Asn Val Leu Thr Asp Asp Gln Gly Asn Asn Leu Gly Ser Asn Asn Gly
        355                 360                 365

Asp Gly Ser Gly Ser Pro Arg Ser Ala Gln Ala Val Ser Pro Glu Gln
    370                 375                 380

Lys Ile Asn Ala Ser Leu Phe Ser Lys Thr Pro Thr Pro Ile Glu Ser
385                 390                 395                 400

Pro Val Val Ala Ser Tyr His Met Ser Asn Gly Thr Ala Leu Lys Ile
                405                 410                 415

Pro Glu Glu Pro Gln Asn Leu Asn Arg Leu Met Pro Ala Glu Pro Gly
            420                 425                 430

Val Pro Gly Thr Glu Trp Phe Ser Gln Val Glu Leu Gly Val Phe Ile
        435                 440                 445

Thr Phe Val Thr Leu Pro Asn Gly Cys Asn Ala Leu Lys Arg Ile Arg
    450                 455                 460

Phe Ser Arg Asp Ile Phe Ser Lys Lys Glu Ala Glu Ser Trp Trp Ala
465                 470                 475                 480

Glu Asn Gly Asn Arg Val Arg Glu Val Tyr Asn Val Pro Ala Phe Glu
                485                 490                 495

Arg Thr Thr Thr Asn Gly His Gln Ala Thr Ser Ser Ser Glu Glu Glu
            500                 505                 510

Val Ser Gly Val Ser Gly Tyr Ala Thr Pro Ser Tyr Ser Pro Gln Gly
        515                 520                 525

Ser Arg Gly Ala Ser Thr Arg Asp Ser Pro Ala Gly Tyr Ser Ser Gly
    530                 535                 540

Ile Ser Arg Gly Ala Ser Leu Arg Asp Thr Ser Ser Arg Glu Ala Ser
545                 550                 555                 560

Met Arg Glu Pro Ser Ile Arg Glu Ser Ile Arg Gln Ser Met Arg Asp
                565                 570                 575

Ala Val Ser Glu His Ser Glu Ser Ala Thr Cys Thr Glu Arg Glu Thr
            580                 585                 590

Glu Thr Asp Thr Val Ala Gly Ser Val Ala Gly Ser Asp Arg Thr Tyr
        595                 600                 605

Asp Gly Glu Glu Ser Thr Trp Val Glu Glu Asp Val Pro Gly Val Tyr
    610                 615                 620

Leu Thr Leu Lys Asn Leu Thr Gly Gly Gly Arg Glu Leu Lys Arg Val
625                 630                 635                 640

Arg Phe Ser Arg Glu Lys Phe Thr Glu Lys Gln Ala Lys Ile Trp Trp
                645                 650                 655

Asp Glu Asn Arg Gly Arg Ile His Lys Gln Tyr Leu
            660                 665

<210>  57
<211>  1029
<212>  DNA

<213>  Picea sitchensis

<400>  57
atgctggcgt gcatcgcgtg ttcgaagcgg ctaaatgatg gctcactgga tgccgcagac      60

gaggatggtt ccggtactcc aagatcacct gcctccaggg aggccatcaa gaatcttact     120

tctcagataa aggatatggc gttaaagtta tctggagcac atagacattg caggcctttc     180

gcggtatcca accttagccg cgagggacag ctacaaagat gcacagcatc agaagtagga     240

tctgaaaacg gaacgccgcg tggaggaagt tccagttcca caccggcctg gagtattgtc     300

agttcttcta gtaaaggcca tgttctcggc gataggctgt gcgcaaatac ctcaagaatc     360

ggcacgccga tgttgcatac ctcctcagga ccagtcgaaa caacgatgga agaagtggag     420

gaggaggagt ccaaagagtg gatatcacag gtcgaaccag gcgtcctcat cactttagtt     480

tctgtcaagg gaggaggaaa tgagcttaaa cggatcagat tcagccgtga attattcaac     540

aaatggcaag cacagcgttg gtgggcggaa aattacgata aagttatgga gctttacaat     600

gtccatgcgc atgccaagga cgactctgcc gttgccgttc ccactcctcc aaggtccgag     660

gacgagagag attcgaagat gcaggaatcc ggagctgaca gtcccgtgac gccgccgata     720

caaaacatga gccttcttcc aagaggtttg tacgcatcaa gaacgagttc ttctcactat     780

gcggatcgat ccgacgactt tcagagcagc ggcaattcaa gcgaacaaga gcaagaacaa     840

gaacaagaat gggtcgaaga agacgaacct ggcgtctacg ttaccattcg ctgttcccct     900

gcaggctcaa gggaaatcag gcgcgtcaga ttcagccgcg agaagttcag tgagatgcaa     960

gcgagattgt ggtgggaaga aaatcgccta agaatccatg aacagtacat aagtggacgt    1020

tctctttaa                                                            1029

<210>  58
<211>  342
<212>  PRT
<213>  Picea sitchensis

<400>  58

Met Leu Ala Cys Ile Ala Cys Ser Lys Arg Leu Asn Asp Gly Ser Leu
1               5                   10                  15

Asp Ala Ala Asp Glu Asp Gly Ser Gly Thr Pro Arg Ser Pro Ala Ser
            20                  25                  30

Arg Glu Ala Ile Lys Asn Leu Thr Ser Gln Ile Lys Asp Met Ala Leu
        35                  40                  45

Lys Leu Ser Gly Ala His Arg His Cys Arg Pro Phe Ala Val Ser Asn
    50                  55                  60

Leu Ser Arg Glu Gly Gln Leu Gln Arg Cys Thr Ala Ser Glu Val Gly
65                  70                  75                  80

Ser Glu Asn Gly Thr Pro Arg Gly Gly Ser Ser Ser Ser Thr Pro Ala
                85                  90                  95

Trp Ser Ile Val Ser Ser Ser Ser Lys Gly His Val Leu Gly Asp Arg
            100                 105                 110

Leu Cys Ala Asn Thr Ser Arg Ile Gly Thr Pro Met Leu His Thr Ser
        115                 120                 125

Ser Gly Pro Val Glu Thr Thr Met Glu Glu Val Glu Glu Glu Glu Ser
    130                 135                 140

Lys Glu Trp Ile Ser Gln Val Glu Pro Gly Val Leu Ile Thr Leu Val
145                 150                 155                 160

Ser Val Lys Gly Gly Gly Asn Glu Leu Lys Arg Ile Arg Phe Ser Arg
                165                 170                 175

Glu Leu Phe Asn Lys Trp Gln Ala Gln Arg Trp Trp Ala Glu Asn Tyr
            180                 185                 190

Asp Lys Val Met Glu Leu Tyr Asn Val His Ala His Ala Lys Asp Asp
        195                 200                 205

Ser Ala Val Ala Val Pro Thr Pro Pro Arg Ser Glu Asp Glu Arg Asp
    210                 215                 220

Ser Lys Met Gln Glu Ser Gly Ala Asp Ser Pro Val Thr Pro Pro Ile
225                 230                 235                 240

Gln Asn Met Ser Leu Leu Pro Arg Gly Leu Tyr Ala Ser Arg Thr Ser
                245                 250                 255

Ser Ser His Tyr Ala Asp Arg Ser Asp Asp Phe Gln Ser Ser Gly Asn
            260                 265                 270

Ser Ser Glu Gln Glu Gln Glu Gln Glu Gln Glu Trp Val Glu Glu Asp
        275                 280                 285

Glu Pro Gly Val Tyr Val Thr Ile Arg Cys Ser Pro Ala Gly Ser Arg
    290                 295                 300

Glu Ile Arg Arg Val Arg Phe Ser Arg Glu Lys Phe Ser Glu Met Gln
305                 310                 315                 320

Ala Arg Leu Trp Trp Glu Glu Asn Arg Leu Arg Ile His Glu Gln Tyr
                325                 330                 335

Ile Ser Gly Arg Ser Leu
            340

<210>  59
<211>  1095
<212>  DNA
<213>  Populus trichocarpa

<400>  59
atgctgacct gtatagctcg ttctaaacaa cctggtgatg actcgctgac catcaaatcc      60

ctcacttctc agttaaagga tatggcatta aaggcatcag gtgcataccg ccactgcaac     120

ccatgcacgg tgccaacaac aacaacaaca actcagagtc gactcaggag caactggact     180

gcatcagacg cagagtcgga gagattcagg tggccgctgc agaggacggg aagctcgagc     240

tcgattacac cgcgtacgtg gggaaaagag atggaggcga ggctgaaagg tatatcgagc     300

tccagcggtg aagggacccc gaattcggta aatagcagcg ggcgtcgggt cgacccgccg     360

atagctttcg tggaagaaaa ggagcctaaa gaatgggtgg cccaagtgga gcccggtgta     420

ctcatcaccc tagtttcgct tccaagggga gggaatgatc tcaagcggat acgcttcagt     480

cgagacatgt ttaacaagtg gcaagctcaa agatggtggg cagagaacta cgacaggatc     540

atggagcttt acaatgttca gaggtttaac tgccaagctt ttccactccc gccaccccca     600

agatccgagg atgagagctc aaagatggaa tctgcagaag acatccctgt aacaccacca     660

ctgaatagag agcggttacc tcgtaatttg tatcgtccaa cagggacggg aatgggctac     720

tcatcctctg attcacttga ccatcaccca attcaggctc gtcattactg tgactctact     780

ggtctcacct ccaccccaaa actctccagc atcagtggag ctaagacgga aacctcttca     840

atggatgcat ctataagaag tagctcatcg agggaggctg attgctcagg agagctctcc     900

atcagcaatg ccagtgatat ggaaactgaa tgggttgaac aggatgaaca aggtgtttac     960

atcactataa gagccttgcc aggtggtaaa agagagatca gacgagtcag attcagccga    1020

gaaagattcg gggagacgca tgccaaagtg tggtgggaag agaaccgagc caggatacat    1080

caacaataca tgtga                                                     1095

<210>  60
<211>  364
<212>  PRT
<213>  Populus trichocarpa

<400>  60

Met Leu Thr Cys Ile Ala Arg Ser Lys Gln Pro Gly Asp Asp Ser Leu
1               5                   10                  15

Thr Ile Lys Ser Leu Thr Ser Gln Leu Lys Asp Met Ala Leu Lys Ala
                20                  25                  30

Ser Gly Ala Tyr Arg His Cys Asn Pro Cys Thr Val Pro Thr Thr Thr
        35                  40                  45

Thr Thr Thr Gln Ser Arg Leu Arg Ser Asn Trp Thr Ala Ser Asp Ala
    50                  55                  60

Glu Ser Glu Arg Phe Arg Trp Pro Leu Gln Arg Thr Gly Ser Ser Ser
65                  70                  75                  80

Ser Ile Thr Pro Arg Thr Trp Gly Lys Glu Met Glu Ala Arg Leu Lys
                85                  90                  95

Gly Ile Ser Ser Ser Ser Gly Glu Gly Thr Pro Asn Ser Val Asn Ser
            100                 105                 110

Ser Gly Arg Arg Val Asp Pro Pro Ile Ala Phe Val Glu Glu Lys Glu
        115                 120                 125

Pro Lys Glu Trp Val Ala Gln Val Glu Pro Gly Val Leu Ile Thr Leu
    130                 135                 140

Val Ser Leu Pro Arg Gly Gly Asn Asp Leu Lys Arg Ile Arg Phe Ser
145                 150                 155                 160

Arg Asp Met Phe Asn Lys Trp Gln Ala Gln Arg Trp Trp Ala Glu Asn
                165                 170                 175

Tyr Asp Arg Ile Met Glu Leu Tyr Asn Val Gln Arg Phe Asn Cys Gln
            180                 185                 190

Ala Phe Pro Leu Pro Pro Pro Pro Arg Ser Glu Asp Glu Ser Ser Lys
        195                 200                 205

Met Glu Ser Ala Glu Asp Ile Pro Val Thr Pro Pro Leu Asn Arg Glu
    210                 215                 220

Arg Leu Pro Arg Asn Leu Tyr Arg Pro Thr Gly Thr Gly Met Gly Tyr
225                 230                 235                 240

Ser Ser Ser Asp Ser Leu Asp His His Pro Ile Gln Ala Arg His Tyr
                245                 250                 255

Cys Asp Ser Thr Gly Leu Thr Ser Thr Pro Lys Leu Ser Ser Ile Ser

260                 265                 270

Gly Ala Lys Thr Glu Thr Ser Ser Met Asp Ala Ser Ile Arg Ser Ser
        275                 280                 285

Ser Ser Arg Glu Ala Asp Cys Ser Gly Glu Leu Ser Ile Ser Asn Ala
    290                 295                 300

Ser Asp Met Glu Thr Glu Trp Val Glu Gln Asp Glu Gln Gly Val Tyr
305                 310                 315                 320

Ile Thr Ile Arg Ala Leu Pro Gly Gly Lys Arg Glu Ile Arg Arg Val
                325                 330                 335

Arg Phe Ser Arg Glu Arg Phe Gly Glu Thr His Ala Lys Val Trp Trp
            340                 345                 350

Glu Glu Asn Arg Ala Arg Ile His Gln Gln Tyr Met
        355                 360

<210>  61
<211>  1170
<212>  DNA
<213>  Populus trichocarpa

<400>  61
atgctgacgt gtatagctcg ttctaaacaa cccggtgatg actcactgag tcaagccgat      60

gacgactcag ccgccaccac cgccaatcat catccctccg ctgccaaaca acaacaacaa     120

caacaacaag ccatcaaatc cctaacttct cagttgaagg acatggcatt aaaggcatca     180

ggtgcatacc gccactgcaa cccatgcacg gcgccaaaca caactactca gagtcgactc     240

aggagcaact cgactgagtc agacgccgag tcagagaaat tcaggtggtc gctgcggcgg     300

acgggaagct cgagctcgac gacaccgcgt acgtggggaa aggagatgga ggcgaggctg     360

aaaggaatat cgagctccag tggcgaaggg actccgaatt cggtaaatgg tagtgggcgt     420

cgggtcgacc cgccaatagt attcgttgaa gaaaaggaac ctaaagaatg ggtggcccaa     480

gtggagcccg gtgttctcat cacattcgtt tcgcttccaa ggggagggaa tgatctcaag     540

cggatacgct tcagtcgaga catgtttaac aagtggcaag ctcaaagatg gtgggcagag     600

aactatgaca ggatcatgga gctttacaat gtccagaggt ttaaccgcca agctttccca     660

ctaccgacac ccccaagatc cgaggatgag agctcaaaga tggaatctgc agaagacagc     720

ccggtaacac ctccactgaa tagagaacgg ctaccccgta acttgtatcg tccaacaggg     780

atgggaatgg gctactcgtc ctcagattca cttgaccatc acccattgca ggcccgtcat     840

tactgtgatt ctattggttt cacctccacc ccaaaactct ccagcatcag tggagctaag     900

acagaaacat catcaatgga tgcatctata agaagtagct catcgaggga ggctgatcgc 960

tcaggagagc tttccatcag caatgccagt gatatggaga ctgaatgggt cgagcaggat 1020

gaaccaggtg tttacatcac tatcagagcc ttgccaggtg gaaaaagaga gctcagacga 1080

gtcagattca gccgagaaag attcggggag atgcatgcca gagtgtggtg ggaagagaac 1140

cgggccagga tacatgaaca atacttgtaa 1170

<210> 62
<211> 389
<212> PRT
<213> Populus trichocarpa

<400> 62

Met Leu Thr Cys Ile Ala Arg Ser Lys Gln Pro Gly Asp Asp Ser Leu
1               5                   10                  15

Ser Gln Ala Asp Asp Asp Ser Ala Ala Thr Thr Ala Asn His His Pro
            20                  25                  30

Ser Ala Ala Lys Gln Gln Gln Gln Gln Gln Gln Ala Ile Lys Ser Leu
        35                  40                  45

Thr Ser Gln Leu Lys Asp Met Ala Leu Lys Ala Ser Gly Ala Tyr Arg
    50                  55                  60

His Cys Asn Pro Cys Thr Ala Pro Asn Thr Thr Thr Gln Ser Arg Leu
65                  70                  75                  80

Arg Ser Asn Ser Thr Glu Ser Asp Ala Glu Ser Glu Lys Phe Arg Trp
                85                  90                  95

Ser Leu Arg Arg Thr Gly Ser Ser Ser Ser Thr Thr Pro Arg Thr Trp
            100                 105                 110

Gly Lys Glu Met Glu Ala Arg Leu Lys Gly Ile Ser Ser Ser Ser Gly
        115                 120                 125

Glu Gly Thr Pro Asn Ser Val Asn Gly Ser Gly Arg Arg Val Asp Pro
    130                 135                 140

Pro Ile Val Phe Val Glu Glu Lys Glu Pro Lys Glu Trp Val Ala Gln
145                 150                 155                 160

Val Glu Pro Gly Val Leu Ile Thr Phe Val Ser Leu Pro Arg Gly Gly
                165                 170                 175

Asn Asp Leu Lys Arg Ile Arg Phe Ser Arg Asp Met Phe Asn Lys Trp

180                 185                 190

Gln Ala Gln Arg Trp Trp Ala Glu Asn Tyr Asp Arg Ile Met Glu Leu
        195                 200                 205

Tyr Asn Val Gln Arg Phe Asn Arg Gln Ala Phe Pro Leu Pro Thr Pro
    210                 215                 220

Pro Arg Ser Glu Asp Glu Ser Ser Lys Met Glu Ser Ala Glu Asp Ser
225                 230                 235                 240

Pro Val Thr Pro Pro Leu Asn Arg Glu Arg Leu Pro Arg Asn Leu Tyr
                245                 250                 255

Arg Pro Thr Gly Met Gly Met Gly Tyr Ser Ser Ser Asp Ser Leu Asp
            260                 265                 270

His His Pro Leu Gln Ala Arg His Tyr Cys Asp Ser Ile Gly Phe Thr
        275                 280                 285

Ser Thr Pro Lys Leu Ser Ser Ile Ser Gly Ala Lys Thr Glu Thr Ser
    290                 295                 300

Ser Met Asp Ala Ser Ile Arg Ser Ser Ser Ser Arg Glu Ala Asp Arg
305                 310                 315                 320

Ser Gly Glu Leu Ser Ile Ser Asn Ala Ser Asp Met Glu Thr Glu Trp
                325                 330                 335

Val Glu Gln Asp Glu Pro Gly Val Tyr Ile Thr Ile Arg Ala Leu Pro
            340                 345                 350

Gly Gly Lys Arg Glu Leu Arg Arg Val Arg Phe Ser Arg Glu Arg Phe
        355                 360                 365

Gly Glu Met His Ala Arg Val Trp Trp Glu Glu Asn Arg Ala Arg Ile
    370                 375                 380

His Glu Gln Tyr Leu
385

<210>  63
<211>  1125
<212>  DNA
<213>  Populus trichocarpa

<400>  63
atgttgactt gtattgcgtg ttcaaagcga ctcaacaacc gatgttcgcc gccgagagac      60

agagaagagg atgttgatgt tgctgctttt gagacgctta ggactaagca cgccatgaag 120

gcactcacag ctcaaatgaa ggatatggct gtaaaggctt caggagcata tagaaactgc 180

aagccatgtt caggatcctc gagcaataac aataacagga actatgctga gtctgatgct 240

gcctcagact cagcgaggtt ccactgcttg tatcgtagag caggaagttc caattcgacc 300

ccgagaaagt gggggaaaga gtcggaggca agactaaaag ggctatcgag tggcgaaggc 360

acacctgctt cagttagtgg gcggacggag tcggtggttt ttatggagga agatgagcct 420

aaggagtggg ttgcgcaagt tgagcctggt gtgctcatca cttttgtttc attgcctgat 480

ggcggtaatg atctgaagcg aattcgattc agtcgtgaaa tgttcaataa atggcaagct 540

caaagctggt gggctgagaa ctatgacaag gtcatggaat tatacaatgt tcaacagttc 600

aatcaccaat cagtcccact tccacctcca ccaagatctg aagatgagag ctcaaagcct 660

gaatcagcca aagacagtcc cacgactcct ccactgggca aagaacgccc aagcaatttc 720

caccatccaa caggaatggg ttattcgtca tcagattcac ttgaccacca cccaatgcaa 780

tctcaccaat attatgagtc agctggtctt gcttcaacac caaagctctc tagcattgct 840

ggggctaaaa ctgagacatc atcaatagat ggttctgtga ggactagtat gtcaagagag 900

tcagatcgct cagaagaact ttccatcagt aatgcaagtg atatggagac tgaatgggtt 960

gaacaggatg aaccaggggt atacatcact atcagagcac tgccaggtgg caccagggag 1020

cttagacgtg tcaggttcag ccgagaaaca tttggagaaa cacgtgcaag gttgtggtgg 1080

gaggagaacc gagacagggt acacgaacag tacttgtaca gatga 1125

<210> 64
<211> 374
<212> PRT
<213> Populus trichocarpa

<400> 64

Met Leu Thr Cys Ile Ala Cys Ser Lys Arg Leu Asn Asn Arg Cys Ser
1               5                   10                  15

Pro Pro Arg Asp Arg Glu Glu Asp Val Asp Val Ala Ala Phe Glu Thr
            20                  25                  30

Leu Arg Thr Lys His Ala Met Lys Ala Leu Thr Ala Gln Met Lys Asp
        35                  40                  45

Met Ala Val Lys Ala Ser Gly Ala Tyr Arg Asn Cys Lys Pro Cys Ser
    50                  55                  60

Gly Ser Ser Ser Asn Asn Asn Asn Arg Asn Tyr Ala Glu Ser Asp Ala
65                  70                  75                  80

Ala Ser Asp Ser Ala Arg Phe His Cys Leu Tyr Arg Arg Ala Gly Ser
                85                  90                  95
Ser Asn Ser Thr Pro Arg Lys Trp Gly Lys Glu Ser Glu Ala Arg Leu
            100                 105                 110
Lys Gly Leu Ser Ser Gly Glu Gly Thr Pro Ala Ser Val Ser Gly Arg
        115                 120                 125
Thr Glu Ser Val Val Phe Met Glu Glu Asp Glu Pro Lys Glu Trp Val
    130                 135                 140
Ala Gln Val Glu Pro Gly Val Leu Ile Thr Phe Val Ser Leu Pro Asp
145                 150                 155                 160
Gly Gly Asn Asp Leu Lys Arg Ile Arg Phe Ser Arg Glu Met Phe Asn
                165                 170                 175
Lys Trp Gln Ala Gln Ser Trp Trp Ala Glu Asn Tyr Asp Lys Val Met
            180                 185                 190
Glu Leu Tyr Asn Val Gln Gln Phe Asn His Gln Ser Val Pro Leu Pro
        195                 200                 205
Pro Pro Pro Arg Ser Glu Asp Glu Ser Ser Lys Pro Glu Ser Ala Lys
    210                 215                 220
Asp Ser Pro Thr Thr Pro Pro Leu Gly Lys Glu Arg Pro Ser Asn Phe
225                 230                 235                 240
His His Pro Thr Gly Met Gly Tyr Ser Ser Ser Asp Ser Leu Asp His
                245                 250                 255
His Pro Met Gln Ser His Gln Tyr Tyr Glu Ser Ala Gly Leu Ala Ser
            260                 265                 270
Thr Pro Lys Leu Ser Ser Ile Ala Gly Ala Lys Thr Glu Thr Ser Ser
        275                 280                 285
Ile Asp Gly Ser Val Arg Thr Ser Met Ser Arg Glu Ser Asp Arg Ser
    290                 295                 300
Glu Glu Leu Ser Ile Ser Asn Ala Ser Asp Met Glu Thr Glu Trp Val
305                 310                 315                 320
Glu Gln Asp Glu Pro Gly Val Tyr Ile Thr Ile Arg Ala Leu Pro Gly
                325                 330                 335

Gly Thr Arg Glu Leu Arg Arg Val Arg Phe Ser Arg Glu Thr Phe Gly
            340                 345                 350

Glu Thr Arg Ala Arg Leu Trp Trp Glu Glu Asn Arg Asp Arg Val His
        355                 360                 365

Glu Gln Tyr Leu Tyr Arg
    370

<210>  65
<211>  1230
<212>  DNA
<213>  Sorghum bicolor

<400>  65
atgctcacgt gcatcgcgtg ctccaagcac ctcccgggcg gcgcgccgcc gctgcgcgag      60

ccgccggaag aggaggagga ggaggacgac gacgaccacc acgccatcgc cggaggcgcc     120

ggtgaatcgg cgacgacgcc cggcacgagg cacgccgtca agtcgctcac cgcccagatc     180

aaggacatgg cgctgaaggc gtcgggcgcg taccggcact gcaagccgtg cgccgggtcg     240

tcgtccccgg cggcgtcgcg gcggcagcaa ccgtactacc acggcgcgta cgcggagtcc     300

aggtccgacc gcttccacta cgcgtaccag tgcgccggca gctccgcagc atcgacgccg     360

aggctgcgca cgggggcgcc gatgtccagc ggtgacgtca cgccgtcggt cagcgcgcgc     420

actgacttcc tcgccggcga tgaggatgaa gaggaaacgg cggctggcag cagcgaggag     480

gatgaggcga aggagtgggt cgcccaggtg gaacccgggg tgctcatcac cttcctctcg     540

ctgccacggg gcggcaatgg tctgaagcgc atccgattca gccgtgaaat gttcaacaaa     600

tggcaagcac aaagatggtg gactgaaaat tacgagaagg tcatggagct ttacaacgtt     660

cagaagtttg acagtcaagc tgcttctctg ccaagcattc caaggtctga aaatgagagc     720

tccaaagacg ataactcagc tacagctcca cttaacaagg ggcaactact agatactttg     780

cacagaccac taaaagttag tggagccata ggatattcat cttcagattg tcttcagcac     840

caacccaatc atcttggcaa catttaccgc caagaccgct accttgggca ccaatgttgt     900

gattcagttg gactggcatc aacgcctaag ttatcaagca ttagtggagc aaagacagaa     960

acttctattg atgcatcagt gaggacaagc tcatctcctg aagaggtgga tcgatcaggt    1020

gaactttcag cctctgttag caacgcaagt gaccaagaga gggaatgggt agaagaggac    1080

gagcctggtg tgtatattac tattcgagct ttgcctggtg gcatcagaga actacgtcgc    1140

gtcagattca gccgggagag attcaatgag atgcatgcca ggttgtggtg ggaagaaaac    1200

cgagcgagga tacatgatca atatctctga                                      1230

<210>  66
<211>  409
<212>  PRT
<213>  Sorghum bicolor

<400>  66

Met Leu Thr Cys Ile Ala Cys Ser Lys His Leu Pro Gly Gly Ala Pro
1               5                   10                  15

Pro Leu Arg Glu Pro Pro Glu Glu Glu Glu Glu Glu Asp Asp Asp Asp
            20                  25                  30

His His Ala Ile Ala Gly Gly Ala Gly Glu Ser Ala Thr Thr Pro Gly
        35                  40                  45

Thr Arg His Ala Val Lys Ser Leu Thr Ala Gln Ile Lys Asp Met Ala
    50                  55                  60

Leu Lys Ala Ser Gly Ala Tyr Arg His Cys Lys Pro Cys Ala Gly Ser
65                  70                  75                  80

Ser Ser Pro Ala Ala Ser Arg Arg Gln Gln Pro Tyr Tyr His Gly Ala
                85                  90                  95

Tyr Ala Glu Ser Arg Ser Asp Arg Phe His Tyr Ala Tyr Gln Cys Ala
            100                 105                 110

Gly Ser Ser Ala Ala Ser Thr Pro Arg Leu Arg Thr Gly Gly Ala Met
        115                 120                 125

Ser Ser Gly Asp Val Thr Pro Ser Val Ser Ala Arg Thr Asp Phe Leu
    130                 135                 140

Ala Gly Asp Glu Asp Glu Glu Glu Thr Ala Ala Gly Ser Ser Glu Glu
145                 150                 155                 160

Asp Glu Ala Lys Glu Trp Val Ala Gln Val Glu Pro Gly Val Leu Ile
                165                 170                 175

Thr Phe Leu Ser Leu Pro Arg Gly Gly Asn Gly Leu Lys Arg Ile Arg
            180                 185                 190

Phe Ser Arg Glu Met Phe Asn Lys Trp Gln Ala Gln Arg Trp Trp Thr
        195                 200                 205

Glu Asn Tyr Glu Lys Val Met Glu Leu Tyr Asn Val Gln Lys Phe Asp
    210                 215                 220

Ser Gln Ala Ala Ser Leu Pro Ser Ile Pro Arg Ser Glu Asn Glu Ser
225                 230                 235                 240

Ser Lys Asp Asp Asn Ser Ala Thr Ala Pro Leu Asn Lys Gly Gln Leu
                245                 250                 255

Leu Asp Thr Leu His Arg Pro Leu Lys Val Ser Gly Ala Ile Gly Tyr
            260                 265                 270

Ser Ser Ser Asp Cys Leu Gln His Gln Pro Asn His Leu Gly Asn Ile
        275                 280                 285

Tyr Arg Gln Asp Arg Tyr Leu Gly His Gln Cys Cys Asp Ser Val Gly
    290                 295                 300

Leu Ala Ser Thr Pro Lys Leu Ser Ser Ile Ser Gly Ala Lys Thr Glu
305                 310                 315                 320

Thr Ser Ile Asp Ala Ser Val Arg Thr Ser Ser Ser Pro Glu Glu Val
                325                 330                 335

Asp Arg Ser Gly Glu Leu Ser Ala Ser Val Ser Asn Ala Ser Asp Gln
            340                 345                 350

Glu Arg Glu Trp Val Glu Glu Asp Glu Pro Gly Val Tyr Ile Thr Ile
        355                 360                 365

Arg Ala Leu Pro Gly Gly Ile Arg Glu Leu Arg Arg Val Arg Phe Ser
    370                 375                 380

Arg Glu Arg Phe Asn Glu Met His Ala Arg Leu Trp Trp Glu Glu Asn
385                 390                 395                 400

Arg Ala Arg Ile His Asp Gln Tyr Leu
                405

<210>  67
<211>  1251
<212>  DNA
<213>  Sorghum bicolor

<400>  67
atgctcacgt gcatcgcctg ctccaagcag cagttcgccg ccggcggcgg cccgccactg      60

catgagccgc cggaggacga cgatgtcgtt gacggaggag gcgccatcgg cggcgcgggt     120

acgcccagca cacggcacgc catcaaggcg ctcaccgccc agatcaagga catggcgctc     180

aaggcgtcgg gcgcgtaccg gcactgcaag ccctgcgcgg gctcctccgc ggcggcgtcg     240

cggcggcacc acccgtacca ccaccgcggc ggcagcggcg tcttcggggg ctccgacgcc     300

ggctcggcct ccgaccgctt ccactacgcg taccgccgcg ccgggagctc agcggcctcc 360

actccgcgac tgcgcagcgg gggcgccgcc ctgtcgagtg gcgacgccac gccgtccatg 420

agcgtgcgca ccgacttccc tgccggcgac gacgaggagg acgacgagat ggcgtcggaa 480

gccgctggcg gatgtggtgg tggtggcaag gacgacgacg ccagggagtg ggtagcgcag 540

gtggagcccg gcgtgctcat taccttcgtc tcgctggcgc aaggtggcaa cgacctgaaa 600

cgcattcgat tcagccgtga gatgttcaac aaatggcaag cacaaagatg gtgggctgaa 660

aattatgaca aagttatgga actttacaat gtccagaggt ttaatcaaac tgtccctctc 720

ccgactaccc caaaatctga agatgagagc tccaaagagg acagcccagt aacaccacca 780

ctggacaagg aacggctacc tcgcacattc cacagacaag gtggtggagc tatgggctac 840

tcttcatcag attctctcga gcatcactca aaccactact gtactggcca ccaccaccat 900

catggacacc aatgctgtga ttcaatgggc ctggcatcaa caccaaagct gtcaagtatc 960

agtggagcca agacagaaac ctcatcaatg gacgcatcaa tgaggacaag ctcgtcacct 1020

gaagaggtcg acaggtctgg tgagctctcg gtgtccatca gcaatgcaag cgaccaggag 1080

agggagtggg tcgaggaaga cgagcctggt gtatatatta caatccgggc tttacctggt 1140

gggatcagag agcttcgccg tgttcggttc agccgggaga agttcagcga gatgcatgcc 1200

aggctatggt gggaagagaa ccgagcgagg atacacgaac agtacctctg a 1251

<210> 68
<211> 416
<212> PRT
<213> Sorghum bicolor

<400> 68

Met Leu Thr Cys Ile Ala Cys Ser Lys Gln Gln Phe Ala Ala Gly Gly
1               5                   10                  15

Gly Pro Pro Leu His Glu Pro Pro Glu Asp Asp Asp Val Val Asp Gly
            20                  25                  30

Gly Gly Ala Ile Gly Gly Ala Gly Thr Pro Ser Thr Arg His Ala Ile
        35                  40                  45

Lys Ala Leu Thr Ala Gln Ile Lys Asp Met Ala Leu Lys Ala Ser Gly
    50                  55                  60

Ala Tyr Arg His Cys Lys Pro Cys Ala Gly Ser Ser Ala Ala Ala Ser
65                  70                  75                  80

Arg Arg His His Pro Tyr His His Arg Gly Gly Ser Gly Val Phe Gly
                85                  90                  95

Gly Ser Asp Ala Gly Ser Ala Ser Asp Arg Phe His Tyr Ala Tyr Arg
            100                 105                 110

Arg Ala Gly Ser Ser Ala Ala Ser Thr Pro Arg Leu Arg Ser Gly Gly
        115                 120                 125

Ala Ala Leu Ser Ser Gly Asp Ala Thr Pro Ser Met Ser Val Arg Thr
    130                 135                 140

Asp Phe Pro Ala Gly Asp Asp Glu Glu Asp Asp Glu Met Ala Ser Glu
145                 150                 155                 160

Ala Ala Gly Gly Cys Gly Gly Gly Gly Lys Asp Asp Asp Ala Arg Glu
                165                 170                 175

Trp Val Ala Gln Val Glu Pro Gly Val Leu Ile Thr Phe Val Ser Leu
            180                 185                 190

Ala Gln Gly Gly Asn Asp Leu Lys Arg Ile Arg Phe Ser Arg Glu Met
        195                 200                 205

Phe Asn Lys Trp Gln Ala Gln Arg Trp Trp Ala Glu Asn Tyr Asp Lys
    210                 215                 220

Val Met Glu Leu Tyr Asn Val Gln Arg Phe Asn Gln Thr Val Pro Leu
225                 230                 235                 240

Pro Thr Thr Pro Lys Ser Glu Asp Glu Ser Ser Lys Glu Asp Ser Pro
                245                 250                 255

Val Thr Pro Pro Leu Asp Lys Glu Arg Leu Pro Arg Thr Phe His Arg
            260                 265                 270

Gln Gly Gly Gly Ala Met Gly Tyr Ser Ser Ser Asp Ser Leu Glu His
        275                 280                 285

His Ser Asn His Tyr Cys Thr Gly His His His His His Gly His Gln
    290                 295                 300

Cys Cys Asp Ser Met Gly Leu Ala Ser Thr Pro Lys Leu Ser Ser Ile
305                 310                 315                 320

Ser Gly Ala Lys Thr Glu Thr Ser Ser Met Asp Ala Ser Met Arg Thr
                325                 330                 335

Ser Ser Ser Pro Glu Glu Val Asp Arg Ser Gly Glu Leu Ser Val Ser

340                 345                 350

Ile Ser Asn Ala Ser Asp Gln Glu Arg Glu Trp Val Glu Glu Asp Glu
        355                 360                 365

Pro Gly Val Tyr Ile Thr Ile Arg Ala Leu Pro Gly Gly Ile Arg Glu
    370                 375                 380

Leu Arg Arg Val Arg Phe Ser Arg Glu Lys Phe Ser Glu Met His Ala
385                 390                 395                 400

Arg Leu Trp Trp Glu Glu Asn Arg Ala Arg Ile His Glu Gln Tyr Leu
                405                 410                 415

<210>  69
<211>  1083
<212>  DNA
<213>  Vitis vinifera

<400>  69
atgctgacgt gtatatcgtg ctcgaagcaa acggaggagg atgggagagg agaggagggc      60

agtggaggtg gggcgcgtgg gacaccaagt acaaaagaag ccgtcaaaag cctgacggcg     120

cagatcaagg atatggcctt gaaattctcg ggtgcttaca ggcaatgcaa gccctgcact     180

gggtccagca gctacaagaa gggacaccgg ccttatcctg actttgatac catttcagaa     240

ggggttccgt acccctactt aaggccgggg agctcaagct caacgcctgc atgggatttc     300

acaaccagca gccataaccc tggtgcaggg tctgactcaa ggttcactgg ggtgttgaga     360

ggtgatcaga cgcccggagg ggtgtccatc tcggctcagt cttgtgatgt tgtgctggag     420

gatgaggatg agcccaaaga gtggatggct caggttgagc ctggagttca catcactttt     480

gtgtccctcc cccatggggg aaatgactta aaacggatcc gtttcagccg ggagatgttt     540

aataaatggc aagctcagcg atggtgggga gagaattatg accggatcat ggagctgtac     600

aatgtccaga gattcaaccg ccaagctcta cacactcctc ctaggtctga ggacgaggta     660

aacagagatt catcttactc gaggatggga tcggcaaggg aaagccctat gactccatca     720

ctaaacaagg aatggattcc gaggaactat tataaaccat ctggaagtaa aggtcatcaa     780

tacaatgcgg gctcaagtgc ttatggcaca ggcggcccaa ggggcgagac atcttccatg     840

gatgcttcac ggacaacaac ctcatctagg gatgaggcgt cagtttccat tagcaatgcc     900

agtgacatgg agactgagtg ggttgagcaa gatgaaccag gagtctacat taccatcaga     960

cagttggctg atggcacaag ggagcttcgg cgcgtcagat tcagccgtga aagatttggt    1020

gaggtccatg caaagacgtg gtgggaagag aatcgagaga gaatacaagc tcaatacctc    1080

taa                                                                  1083

<210>  70
<211>  360
<212>  PRT
<213>  Vitis vinifera

<400>  70

Met Leu Thr Cys Ile Ser Cys Ser Lys Gln Thr Glu Glu Asp Gly Arg
1               5                   10                  15

Gly Glu Glu Gly Ser Gly Gly Gly Ala Arg Gly Thr Pro Ser Thr Lys
            20                  25                  30

Glu Ala Val Lys Ser Leu Thr Ala Gln Ile Lys Asp Met Ala Leu Lys
        35                  40                  45

Phe Ser Gly Ala Tyr Arg Gln Cys Lys Pro Cys Thr Gly Ser Ser Ser
    50                  55                  60

Tyr Lys Lys Gly His Arg Pro Tyr Pro Asp Phe Asp Thr Ile Ser Glu
65                  70                  75                  80

Gly Val Pro Tyr Pro Tyr Leu Arg Pro Gly Ser Ser Ser Ser Thr Pro
                85                  90                  95

Ala Trp Asp Phe Thr Thr Ser Ser His Asn Pro Gly Ala Gly Ser Asp
            100                 105                 110

Ser Arg Phe Thr Gly Val Leu Arg Gly Asp Gln Thr Pro Gly Gly Val
        115                 120                 125

Ser Ile Ser Ala Gln Ser Cys Asp Val Val Leu Glu Asp Glu Asp Glu
    130                 135                 140

Pro Lys Glu Trp Met Ala Gln Val Glu Pro Gly Val His Ile Thr Phe
145                 150                 155                 160

Val Ser Leu Pro His Gly Gly Asn Asp Leu Lys Arg Ile Arg Phe Ser
                165                 170                 175

Arg Glu Met Phe Asn Lys Trp Gln Ala Gln Arg Trp Trp Gly Glu Asn
            180                 185                 190

Tyr Asp Arg Ile Met Glu Leu Tyr Asn Val Gln Arg Phe Asn Arg Gln
        195                 200                 205

Ala Leu His Thr Pro Pro Arg Ser Glu Asp Glu Val Asn Arg Asp Ser
    210                 215                 220

Ser Tyr Ser Arg Met Gly Ser Ala Arg Glu Ser Pro Met Thr Pro Ser
225                 230                 235                 240

Leu Asn Lys Glu Trp Ile Pro Arg Asn Tyr Tyr Lys Pro Ser Gly Ser
                245                 250                 255

Lys Gly His Gln Tyr Asn Ala Gly Ser Ser Ala Tyr Gly Thr Gly Gly
            260                 265                 270

Pro Arg Gly Glu Thr Ser Ser Met Asp Ala Ser Arg Thr Thr Thr Ser
        275                 280                 285

Ser Arg Asp Glu Ala Ser Val Ser Ile Ser Asn Ala Ser Asp Met Glu
    290                 295                 300

Thr Glu Trp Val Glu Gln Asp Glu Pro Gly Val Tyr Ile Thr Ile Arg
305                 310                 315                 320

Gln Leu Ala Asp Gly Thr Arg Glu Leu Arg Arg Val Arg Phe Ser Arg
                325                 330                 335

Glu Arg Phe Gly Glu Val His Ala Lys Thr Trp Trp Glu Glu Asn Arg
            340                 345                 350

Glu Arg Ile Gln Ala Gln Tyr Leu
        355                 360

<210>  71
<211>  1116
<212>  DNA
<213>  Vitis vinifera

<400>  71
atgttgacgt gcatagcttg ttccaagcag gtgagtggta gatctctcca tgaacaggag      60

gagggagagg gagaggctgt tgcaactcca agcaccaagc acgccatcaa ggcccttact     120

gctcagatca aggacatggc gttgaaggct tccggggcgt acagaaactg caagccttgt     180

tctgggtcat cggggcaaaa ccaggaccgg aactatgccg attccgagtc cgcgtcggac     240

tcggcgaggt tccattgctc gtaccgtaga accggcagct cgagctccac gcccaggctg     300

ttggggaagg aaatggaggc gagatcgaaa cggctttcta gcggagaagg cacgccggcc     360

tcggtcagcg gccgggcgga gtcggttgtg tttatggagg aagatgagcc taaggagtgg     420

attgcccaag tggagcctgg tgtcctcatc acttttgttt ccatgcctca gggtggaaac     480

gatctcaagc ggattcgatt cagtcgtgag atctttaaca aatggcaagc ccaaaggtgg     540

tgggcagaga actatgacaa agtcatggaa ttatacaatg ttcagaggtt caaccgccaa     600

gctgtccccc ttccaacacc gccaagatct gaagatgaga gctcgaggat ggaatctata 660

cagaacagcc ctgtgacacc gccactgagc aaagaacgct taccccgcaa cttccaccac 720

cgcccaacag gaatgggtta ctcttcatcc gattcacttg accaccaccc tttacaatcc 780

cgacattact atgactcggc tggccttgct tcaacaccaa aactctccag catcagcggg 840

gcaaagaccg agacatcatc aatagatgct tcggtaagga ctagttcatc gagagaggca 900

gaccgctcag gagagctctc cataagcaac gccagtgata tggagactga atgggttgag 960

caggacgagc caggagtcta catcaccatc agagcattgc cagatggcac tcgggagctc 1020

agacgtgtcc gattcagccg agaaaggttt ggggaaatgc acgcgaggct gtggtgggaa 1080

gagaaccgag ccaggataca agaacaatac ttgtga 1116

<210> 72
<211> 371
<212> PRT
<213> Vitis vinifera

<400> 72

Met Leu Thr Cys Ile Ala Cys Ser Lys Gln Val Ser Gly Arg Ser Leu
1               5                   10                  15

His Glu Gln Glu Glu Gly Glu Gly Glu Ala Val Ala Thr Pro Ser Thr
            20                  25                  30

Lys His Ala Ile Lys Ala Leu Thr Ala Gln Ile Lys Asp Met Ala Leu
        35                  40                  45

Lys Ala Ser Gly Ala Tyr Arg Asn Cys Lys Pro Cys Ser Gly Ser Ser
    50                  55                  60

Gly Gln Asn Gln Asp Arg Asn Tyr Ala Asp Ser Glu Ser Ala Ser Asp
65                  70                  75                  80

Ser Ala Arg Phe His Cys Ser Tyr Arg Arg Thr Gly Ser Ser Ser Ser
                85                  90                  95

Thr Pro Arg Leu Leu Gly Lys Glu Met Glu Ala Arg Ser Lys Arg Leu
            100                 105                 110

Ser Ser Gly Glu Gly Thr Pro Ala Ser Val Ser Gly Arg Ala Glu Ser
        115                 120                 125

Val Val Phe Met Glu Glu Asp Glu Pro Lys Glu Trp Ile Ala Gln Val
    130                 135                 140

Glu Pro Gly Val Leu Ile Thr Phe Val Ser Met Pro Gln Gly Gly Asn

145                 150                 155                 160

Asp Leu Lys Arg Ile Arg Phe Ser Arg Glu Ile Phe Asn Lys Trp Gln
                165                 170                 175

Ala Gln Arg Trp Trp Ala Glu Asn Tyr Asp Lys Val Met Glu Leu Tyr
            180                 185                 190

Asn Val Gln Arg Phe Asn Arg Gln Ala Val Pro Leu Pro Thr Pro Pro
        195                 200                 205

Arg Ser Glu Asp Glu Ser Ser Arg Met Glu Ser Ile Gln Asn Ser Pro
    210                 215                 220

Val Thr Pro Pro Leu Ser Lys Glu Arg Leu Pro Arg Asn Phe His His
225                 230                 235                 240

Arg Pro Thr Gly Met Gly Tyr Ser Ser Ser Asp Ser Leu Asp His His
                245                 250                 255

Pro Leu Gln Ser Arg His Tyr Tyr Asp Ser Ala Gly Leu Ala Ser Thr
            260                 265                 270

Pro Lys Leu Ser Ser Ile Ser Gly Ala Lys Thr Glu Thr Ser Ser Ile
        275                 280                 285

Asp Ala Ser Val Arg Thr Ser Ser Ser Arg Glu Ala Asp Arg Ser Gly
    290                 295                 300

Glu Leu Ser Ile Ser Asn Ala Ser Asp Met Glu Thr Glu Trp Val Glu
305                 310                 315                 320

Gln Asp Glu Pro Gly Val Tyr Ile Thr Ile Arg Ala Leu Pro Asp Gly
                325                 330                 335

Thr Arg Glu Leu Arg Arg Val Arg Phe Ser Arg Glu Arg Phe Gly Glu
            340                 345                 350

Met His Ala Arg Leu Trp Trp Glu Glu Asn Arg Ala Arg Ile Gln Glu
        355                 360                 365

Gln Tyr Leu
    370

<210>  73
<211>  1257
<212>  DNA
<213>  Zea mays

<400>  73
atgctcacgt gcatcgcgtg ctccaagcag cagttctccg gcggcggccc gccactgcac      60

gagccgccgg aggacgacga tgtcgttggt ggaggcgccg gcaccagcgc cggcgcggcg     120

acgccgagca cacggcacgc catcaaggcg ctcaccgccc agatcaagga catggcgctc     180

aaggcgtcgg gcgcgtaccg gcaatgcaag ccctgcgcgg gctcctcggc ggcggcctcg     240

cggcggcacc acccgtacca ccaccgcggc ggcagcgcct tcgggggctc cgacgccgac     300

tcgggggcct ccgaccgctt ccactacgcg taccgccgcg cggggagctc ggcggcctcg     360

actccgcgat tgcgcagcgg aggcgccgcc ctgtcgagtg gcgacgccac gccctccatc     420

agcgtgcgca ccggcaccga cttccccgcc ggcgatgacg acgacgacga gatgacgcca     480

gaagccactg gcggatgtgg tggcaaggac gacgacgcca aggagtgggt ggcgcaggtg     540

gagcccggtg tgctcatcac tttcgtctca ctggcggaag gtggcaacga cctaaaacgc     600

attcgattca gccgtgagat gttcaacaaa tgggaagcac aaagatggtg ggctgaaaac     660

tatgacaaag ttatggaact ttacaatgtg cagaagttta atcaaactgt ccctctccca     720

gctaccccaa aatctgaaga tgagagctcc aaggaggaca gcccggtgac accaccactg     780

gacaaggaaa ggctaccccg cactttccac agacaaggtg gtggagcgat gggctcctct     840

tcatcagatt ctctcgagca tcactcaaac cactactgta ctggccgcca ccaccaccac     900

caccaccatg gacaccaatg ctgtgattca atgggcctgg catcaacgcc aaagctgtca     960

agtatcagtg gagccaaaac agaaacctca tcgatggacg cgtcaatgag gacaagctcg    1020

tcacctgagg agatcgacag gtctggtgag ctctcggtgt ccgtcagcaa tgcgagcgac    1080

caggagaggg agtgggtcga agaagacgag ccaggcgtat atatcacaat ccgggcttta    1140

cctggtggga tcagagagct tcgccgcgtt cggttcagcc gggagaagtt cagcgagatg    1200

catgccaggc tgtggtggga agagaaccga gcgaggatac acgaacaata cctctga       1257


<210>  74
<211>  418
<212>  PRT
<213>  Zea mays

<400>  74

Met Leu Thr Cys Ile Ala Cys Ser Lys Gln Gln Phe Ser Gly Gly Gly
1               5                   10                  15


Pro Pro Leu His Glu Pro Pro Glu Asp Asp Asp Val Val Gly Gly Gly
            20                  25                  30


Ala Gly Thr Ser Ala Gly Ala Ala Thr Pro Ser Thr Arg His Ala Ile
        35                  40                  45

Lys Ala Leu Thr Ala Gln Ile Lys Asp Met Ala Leu Lys Ala Ser Gly
    50                  55                  60

Ala Tyr Arg Gln Cys Lys Pro Cys Ala Gly Ser Ser Ala Ala Ala Ser
65                  70                  75                  80

Arg Arg His His Pro Tyr His His Arg Gly Gly Ser Ala Phe Gly Gly
                85                  90                  95

Ser Asp Ala Asp Ser Gly Ala Ser Asp Arg Phe His Tyr Ala Tyr Arg
            100                 105                 110

Arg Ala Gly Ser Ser Ala Ala Ser Thr Pro Arg Leu Arg Ser Gly Gly
        115                 120                 125

Ala Ala Leu Ser Ser Gly Asp Ala Thr Pro Ser Ile Ser Val Arg Thr
    130                 135                 140

Gly Thr Asp Phe Pro Ala Gly Asp Asp Asp Asp Asp Glu Met Thr Pro
145                 150                 155                 160

Glu Ala Thr Gly Gly Cys Gly Gly Lys Asp Asp Asp Ala Lys Glu Trp
                165                 170                 175

Val Ala Gln Val Glu Pro Gly Val Leu Ile Thr Phe Val Ser Leu Ala
            180                 185                 190

Glu Gly Gly Asn Asp Leu Lys Arg Ile Arg Phe Ser Arg Glu Met Phe
        195                 200                 205

Asn Lys Trp Glu Ala Gln Arg Trp Trp Ala Glu Asn Tyr Asp Lys Val
    210                 215                 220

Met Glu Leu Tyr Asn Val Gln Lys Phe Asn Gln Thr Val Pro Leu Pro
225                 230                 235                 240

Ala Thr Pro Lys Ser Glu Asp Glu Ser Ser Lys Glu Asp Ser Pro Val
                245                 250                 255

Thr Pro Pro Leu Asp Lys Glu Arg Leu Pro Arg Thr Phe His Arg Gln
            260                 265                 270

Gly Gly Gly Ala Met Gly Ser Ser Ser Ser Asp Ser Leu Glu His His
        275                 280                 285

Ser Asn His Tyr Cys Thr Gly Arg His His His His His His His Gly
    290                 295                 300

His Gln Cys Cys Asp Ser Met Gly Leu Ala Ser Thr Pro Lys Leu Ser
305                 310                 315                 320

Ser Ile Ser Gly Ala Lys Thr Glu Thr Ser Ser Met Asp Ala Ser Met
                325                 330                 335

Arg Thr Ser Ser Ser Pro Glu Glu Ile Asp Arg Ser Gly Glu Leu Ser
            340                 345                 350

Val Ser Val Ser Asn Ala Ser Asp Gln Glu Arg Glu Trp Val Glu Glu
        355                 360                 365

Asp Glu Pro Gly Val Tyr Ile Thr Ile Arg Ala Leu Pro Gly Gly Ile
    370                 375                 380

Arg Glu Leu Arg Arg Val Arg Phe Ser Arg Glu Lys Phe Ser Glu Met
385                 390                 395                 400

His Ala Arg Leu Trp Trp Glu Glu Asn Arg Ala Arg Ile His Glu Gln
                405                 410                 415

Tyr Leu

<210>  75
<211>  1218
<212>  DNA
<213>  Zea mays

<400>  75
atgctcacgt gcatcgcgtg ctccaagcag ctcccgggcg acgcgccgcc gctgcgcgag      60

ccgtcggacg acgatgaccg cgccaacgcc ggaggcgggg gcgaatcggc ggcaacgccc     120

ggcacgaggc aggccatcag ggtgctcacc gcccagatca aggacatggc gctgaaggca     180

tcgggcgcgt accggcactg caagccctgc gccggttcgt cctccccggc ggcgtcgcgg     240

cggcagcaac cgtactacca cggcgcgtac gcggagtccg ggtccgaccg cttccactgc     300

gcgtaccagc gcgccggcag ctccgcagca tccacgccgg ggctgcgcac ggggggcgcg      360

atgtccagcg gtgacatcac gccatcggtc agcgcgcgca ccgacttcct cgccgacgac     420

gaggaagggg acgatgaaga gggaacggcg actggcagca gcgaggagga tgaggagaag     480

gagtgggtcg cccaggtgga gcccggggtg ctcatcacct tcctctcgct gccgcggggc     540

ggcaatggtc tgaagcgcat ccgattcagc cgtgaaatgt tcaacaaatg gcaagcacaa     600

agatggtgga ctgaaaatta cgagaaggtc atggagcttt acaacgttca gaagtctaac     660

agtcaagttg atcctctgcc aagcattcca aggtctgaca gtgagatctc caaagacgat     720

accccagcta cagcaccgct taacaagggg caattgctac gtacttcacc cagaccacta 780

aaaggcagtg aagccatagg ctattcatct tcggattgtc ctcagcacca atccagtcat 840

tttcgcaacg tttaccgcaa agaccgctac cttgggcacc agttctgtga tccagttgaa 900

ctggcatcga cgcctgagtt atcaagcatc agtggtgcga agacagagac gtctattggt 960

gcatcagtga ggacaagctc atctcctgaa gaggtcgatg gatcgggcga actttcagcc 1020

tctgtcagca acgcaagtga cgaagagagg gaatgggtag aagaggacga gcctggcgtg 1080

tatattacta ttcgagcttt gcctggtagc atcagagaac tacgccgcgt cagattcagc 1140

cgggagagat tcagtgagat gcatgccagg ttatggtggg aagaaaacca agcgagaata 1200

tatgagcagt atctctga 1218

<210> 76
<211> 405
<212> PRT
<213> Zea mays

<400> 76

Met Leu Thr Cys Ile Ala Cys Ser Lys Gln Leu Pro Gly Asp Ala Pro
1               5                   10                  15

Pro Leu Arg Glu Pro Ser Asp Asp Asp Asp Arg Ala Asn Ala Gly Gly
            20                  25                  30

Gly Gly Glu Ser Ala Ala Thr Pro Gly Thr Arg Gln Ala Ile Arg Val
        35                  40                  45

Leu Thr Ala Gln Ile Lys Asp Met Ala Leu Lys Ala Ser Gly Ala Tyr
    50                  55                  60

Arg His Cys Lys Pro Cys Ala Gly Ser Ser Ser Pro Ala Ala Ser Arg
65                  70                  75                  80

Arg Gln Gln Pro Tyr Tyr His Gly Ala Tyr Ala Glu Ser Gly Ser Asp
                85                  90                  95

Arg Phe His Cys Ala Tyr Gln Arg Ala Gly Ser Ser Ala Ala Ser Thr
            100                 105                 110

Pro Gly Leu Arg Thr Gly Gly Ala Met Ser Ser Gly Asp Ile Thr Pro
        115                 120                 125

Ser Val Ser Ala Arg Thr Asp Phe Leu Ala Asp Asp Glu Glu Gly Asp
    130                 135                 140

Asp Glu Glu Gly Thr Ala Thr Gly Ser Ser Glu Glu Asp Glu Glu Lys
145                 150                 155                 160

Glu Trp Val Ala Gln Val Glu Pro Gly Val Leu Ile Thr Phe Leu Ser
                165                 170                 175

Leu Pro Arg Gly Gly Asn Gly Leu Lys Arg Ile Arg Phe Ser Arg Glu
            180                 185                 190

Met Phe Asn Lys Trp Gln Ala Gln Arg Trp Trp Thr Glu Asn Tyr Glu
        195                 200                 205

Lys Val Met Glu Leu Tyr Asn Val Gln Lys Ser Asn Ser Gln Val Asp
    210                 215                 220

Pro Leu Pro Ser Ile Pro Arg Ser Asp Ser Glu Ile Ser Lys Asp Asp
225                 230                 235                 240

Thr Pro Ala Thr Ala Pro Leu Asn Lys Gly Gln Leu Leu Arg Thr Ser
                245                 250                 255

Pro Arg Pro Leu Lys Gly Ser Glu Ala Ile Gly Tyr Ser Ser Ser Asp
            260                 265                 270

Cys Pro Gln His Gln Ser Ser His Phe Arg Asn Val Tyr Arg Lys Asp
        275                 280                 285

Arg Tyr Leu Gly His Gln Phe Cys Asp Pro Val Glu Leu Ala Ser Thr
    290                 295                 300

Pro Glu Leu Ser Ser Ile Ser Gly Ala Lys Thr Glu Thr Ser Ile Gly
305                 310                 315                 320

Ala Ser Val Arg Thr Ser Ser Ser Pro Glu Glu Val Asp Gly Ser Gly
                325                 330                 335

Glu Leu Ser Ala Ser Val Ser Asn Ala Ser Asp Glu Glu Arg Glu Trp
            340                 345                 350

Val Glu Glu Asp Glu Pro Gly Val Tyr Ile Thr Ile Arg Ala Leu Pro
        355                 360                 365

Gly Ser Ile Arg Glu Leu Arg Arg Val Arg Phe Ser Arg Glu Arg Phe
    370                 375                 380

Ser Glu Met His Ala Arg Leu Trp Trp Glu Glu Asn Gln Ala Arg Ile
385                 390                 395                 400

Tyr Glu Gln Tyr Leu
                405

<210>  77
<211>  1155
<212>  DNA
<213>  Zea mays

<400>  77
atgctgtcct gcatcgcgtg cgtcaataag gaagaagacg gcggccggga ccgcgaggag      60

catggcggcg acaccccgag ctgcagagac cccgtgaagt cactcacctc ccagctcaag     120

gacatggtgc tgaagctgtc gggcacccac caccgccagc acggcgcgca gcacaggcgc     180

ggcgggtcgc cgccgccgcc acggggccgg gccacctccc tgtaccgcag cggctactac     240

cgccccggcg tcgtgcagga cgacatggcc gtgcccccgg ccacgtacct gggaggtgga     300

ggcgccggcg cgtcgagcgc gagctccacc ccggcgtggg acctgcccgc cgccgcccgc     360

gcggacggcg aggcctgcag ggagtgggtg gcgcaggtgg agcccggcgt gcagatcacc     420

ttcgtgtccc tccccggcgg cgccggcaac gacctcaagc gcatccggtt cagccgcgag     480

atgtacgaca agtggcaggc gcagaagtgg tggggcgaca acaacgagcg catcatggag     540

ctctacaacg tccgccgctt cagccgccag gtgctcccca cgccgccgcg ctccgacgac     600

gccgagaggg agtcgttcta ctcgcagtct caggtaggga gcccctcggc caccccgtcg     660

ccggcgccgc tcacgccgga gaggatcagc tggggcgcgt tcgcgcggca ggtggcagcg     720

ccgccagcat catcaggtgc cacaggcgcc gcgcggcagc acagcttccg gccgatgtcg     780

ccgccgccgc cgtcgtcctc caacccttcg gagcgggcct ggcagcagca gcagcgacac     840

atcggtggtg gtggtggtgg tggagcggcc ggcggcaaga gccccgccgc ctccgaggcc     900

gccgccgccg ccacggaagc ggcgcggacc accacgtcgt ccagggacga cgtgtccgtc     960

agcaacgcca gcgagctgga ggtgtcggag tggatcatcc aggaccagcc cggcgtctac    1020

atcaccgtca gggagctcgc cgacggcagc cgggagctgc gccgcgtcag gttcagccgc    1080

gagaggttcg cggagctgaa tgccaagctg tggtgggagg agaacaagga gaggatacag    1140

gctcagtacc tttga                                                     1155

<210>  78
<211>  384
<212>  PRT
<213>  Zea mays

<400>  78

Met Leu Ser Cys Ile Ala Cys Val Asn Lys Glu Glu Asp Gly Gly Arg
1               5                   10                  15

Asp Arg Glu Glu His Gly Gly Asp Thr Pro Ser Cys Arg Asp Pro Val
            20                  25                  30

Lys Ser Leu Thr Ser Gln Leu Lys Asp Met Val Leu Lys Leu Ser Gly
        35                  40                  45

Thr His His Arg Gln His Gly Ala Gln His Arg Arg Gly Gly Ser Pro
    50                  55                  60

Pro Pro Pro Arg Gly Arg Ala Thr Ser Leu Tyr Arg Ser Gly Tyr Tyr
65                  70                  75                  80

Arg Pro Gly Val Val Gln Asp Asp Met Ala Val Pro Pro Ala Thr Tyr
                85                  90                  95

Leu Gly Gly Gly Gly Ala Gly Ala Ser Ser Ala Ser Ser Thr Pro Ala
            100                 105                 110

Trp Asp Leu Pro Ala Ala Ala Arg Ala Asp Gly Glu Ala Cys Arg Glu
        115                 120                 125

Trp Val Ala Gln Val Glu Pro Gly Val Gln Ile Thr Phe Val Ser Leu
    130                 135                 140

Pro Gly Gly Ala Gly Asn Asp Leu Lys Arg Ile Arg Phe Ser Arg Glu
145                 150                 155                 160

Met Tyr Asp Lys Trp Gln Ala Gln Lys Trp Trp Gly Asp Asn Asn Glu
                165                 170                 175

Arg Ile Met Glu Leu Tyr Asn Val Arg Arg Phe Ser Arg Gln Val Leu
            180                 185                 190

Pro Thr Pro Pro Arg Ser Asp Asp Ala Glu Arg Glu Ser Phe Tyr Ser
        195                 200                 205

Gln Ser Gln Val Gly Ser Pro Ser Ala Thr Pro Ser Pro Ala Pro Leu
    210                 215                 220

Thr Pro Glu Arg Ile Ser Trp Gly Ala Phe Ala Arg Gln Val Ala Ala
225                 230                 235                 240

Pro Pro Ala Ser Ser Gly Ala Thr Gly Ala Ala Arg Gln His Ser Phe
                245                 250                 255

Arg Pro Met Ser Pro Pro Pro Pro Ser Ser Ser Asn Pro Ser Glu Arg
            260                 265                 270

Ala Trp Gln Gln Gln Gln Arg His Ile Gly Gly Gly Gly Gly Gly Gly
        275                 280                 285

Ala Ala Gly Gly Lys Ser Pro Ala Ala Ser Glu Ala Ala Ala Ala Ala
    290                 295                 300

Thr Glu Ala Ala Arg Thr Thr Thr Ser Ser Arg Asp Asp Val Ser Val
305                 310                 315                 320

Ser Asn Ala Ser Glu Leu Glu Val Ser Glu Trp Ile Ile Gln Asp Gln
                325                 330                 335

Pro Gly Val Tyr Ile Thr Val Arg Glu Leu Ala Asp Gly Ser Arg Glu
            340                 345                 350

Leu Arg Arg Val Arg Phe Ser Arg Glu Arg Phe Ala Glu Leu Asn Ala
        355                 360                 365

Lys Leu Trp Trp Glu Glu Asn Lys Glu Arg Ile Gln Ala Gln Tyr Leu
    370                 375                 380

<210>  79
<211>  1182
<212>  DNA
<213>  Zea mays

<400>  79
atgctggcgt gcatcgcctg ctcggccaag gacggcgggg atcaggacgg ctcccgcgcc      60

gccacgcccc acggaaggga cgccggcaag tccctcacct cccagctcaa ggacatggtg     120

ctcaagttct ccggctccgg caggcagtac aaggccgcgg cggcgagccc gtcgttccgg     180

ggcaaccgct tccaccgcaa cagccgcctg gccgcgtaca cgggcgtcat tgacgactcg     240

ggcttcacgt cggacggggc caccgagggc tacggctaca tgaggacgac gacgcacgcg     300

acaggcgcca ccgcgggaac caaggtcggc cgtggcttcc cgcagcatgt caggagcccg     360

agcgcgagct ggataccgag catcggggag gacgacgagg aggaggatga agaggttgtc     420

gtcgtggaag aggaccgcgt gccgcgggag tggacggccc aggtggagcc cggcgtgcag     480

atcaccttcg tctccaccgc gggcggcgct ggcaacgaca taaagcgaat ccgcttcagc     540

cgcgacatgt tcaacaagtg ggaggcgcag cggtggtggg gagagaacta cgaccgcgtg     600

gtggagctct acaacgtgca gacgttcagc cggcagcagg gcgtctcaac gccgacgtcc     660

tccatcgacg acgccacgca gagagactcg tcgttctact cccgcgccgg ctcgacgagg     720

gacagcccgg tgatcctgcc gccgacagcc gtgggcaggg agcagccgat cgcccgcgcc     780

acctcgtgca gggccacggc cgcggcggct tccacggccc gagccgcgtg caacccatcg     840

tccagtgcgg tgccggaccc gtcggaccac gtgtgggcgc accacttcaa cctgctcaac 900

tccgcgccgg cgccggcacc ggccgctccg cacctcgacc cgtcgcgcgc taccacgtcg 960

tccctggacg aggcgtccgt gtccgtgagc aacgcgagcg acctggaggc cacggagtgg 1020

gtggagcagg acgagcccgg cgtgtccatc accatccgcg agttcggcga tggcacccgc 1080

gagctccgcc gcgtccgctt cagccgggag aggttcggcg aggagagggc caaggtgtgg 1140

tgggaccaga acaggaaccg aatacacgcg cagtacctgt ga 1182

<210> 80
<211> 393
<212> PRT
<213> Zea mays

<400> 80

Met Leu Ala Cys Ile Ala Cys Ser Ala Lys Asp Gly Gly Asp Gln Asp
1               5                   10                  15

Gly Ser Arg Ala Ala Thr Pro His Gly Arg Asp Ala Gly Lys Ser Leu
            20                  25                  30

Thr Ser Gln Leu Lys Asp Met Val Leu Lys Phe Ser Gly Ser Gly Arg
        35                  40                  45

Gln Tyr Lys Ala Ala Ala Ala Ser Pro Ser Phe Arg Gly Asn Arg Phe
    50                  55                  60

His Arg Asn Ser Arg Leu Ala Ala Tyr Thr Gly Val Ile Asp Asp Ser
65                  70                  75                  80

Gly Phe Thr Ser Asp Gly Ala Thr Glu Gly Tyr Gly Tyr Met Arg Thr
                85                  90                  95

Thr Thr His Ala Thr Gly Ala Thr Ala Gly Thr Lys Val Gly Arg Gly
            100                 105                 110

Phe Pro Gln His Val Arg Ser Pro Ser Ala Ser Trp Ile Pro Ser Ile
        115                 120                 125

Gly Glu Asp Asp Glu Glu Glu Asp Glu Glu Val Val Val Val Glu Glu
    130                 135                 140

Asp Arg Val Pro Arg Glu Trp Thr Ala Gln Val Glu Pro Gly Val Gln
145                 150                 155                 160

Ile Thr Phe Val Ser Thr Ala Gly Gly Ala Gly Asn Asp Ile Lys Arg
                165                 170                 175

Ile Arg Phe Ser Arg Asp Met Phe Asn Lys Trp Glu Ala Gln Arg Trp
            180                 185                 190

Trp Gly Glu Asn Tyr Asp Arg Val Val Glu Leu Tyr Asn Val Gln Thr
        195                 200                 205

Phe Ser Arg Gln Gln Gly Val Ser Thr Pro Thr Ser Ser Ile Asp Asp
    210                 215                 220

Ala Thr Gln Arg Asp Ser Ser Phe Tyr Ser Arg Ala Gly Ser Thr Arg
225                 230                 235                 240

Asp Ser Pro Val Ile Leu Pro Pro Thr Ala Val Gly Arg Glu Gln Pro
                245                 250                 255

Ile Ala Arg Ala Thr Ser Cys Arg Ala Thr Ala Ala Ala Ala Ser Thr
            260                 265                 270

Ala Arg Ala Ala Cys Asn Pro Ser Ser Ser Ala Val Pro Asp Pro Ser
        275                 280                 285

Asp His Val Trp Ala His His Phe Asn Leu Leu Asn Ser Ala Pro Ala
    290                 295                 300

Pro Ala Pro Ala Ala Pro His Leu Asp Pro Ser Arg Ala Thr Thr Ser
305                 310                 315                 320

Ser Leu Asp Glu Ala Ser Val Ser Val Ser Asn Ala Ser Asp Leu Glu
                325                 330                 335

Ala Thr Glu Trp Val Glu Gln Asp Glu Pro Gly Val Ser Ile Thr Ile
            340                 345                 350

Arg Glu Phe Gly Asp Gly Thr Arg Glu Leu Arg Arg Val Arg Phe Ser
        355                 360                 365

Arg Glu Arg Phe Gly Glu Glu Arg Ala Lys Val Trp Trp Asp Gln Asn
    370                 375                 380

Arg Asn Arg Ile His Ala Gln Tyr Leu
385                 390

<210> 81
<211> 36
<212> PRT
<213> Artificial sequence

<220>

<223>  IPR013591 DZC domain (PFAM entry PF08381 DZC)

<400>  81

Asn Gly Gly Asn Asp Leu Lys Arg Ile Arg Phe Ser Arg Glu Met Phe
1               5                   10                  15

Asn Lys Trp Gln Ala Gln Arg Trp Trp Gly Glu Asn Tyr Asp Arg Ile
            20                  25                  30

Thr Glu Leu Tyr
        35

<210>  82
<211>  36
<212>  PRT
<213>  Artificial sequence

<220>
<223>  C-terminal IPR013591 DZC domain (PFAM entry PF08381 DZC)

<400>  82

Asp Gly Thr Arg Glu Leu Arg Arg Val Arg Phe Ser Arg Glu Gln Phe
1               5                   10                  15

Gly Glu Val His Ala Lys Thr Trp Trp Glu Gln Asn Arg Glu Arg Ile
            20                  25                  30

Gln Ala Gln Tyr
        35

<210>  83
<211>  63
<212>  PRT
<213>  Artificial sequence

<220>
<223>  Conserved domain 1

<400>  83

Glu Pro Lys Glu Trp Met Ala Gln Val Glu Pro Gly Val His Ile Thr
1               5                   10                  15

Phe Val Ser Leu Pro Asn Gly Gly Asn Asp Leu Lys Arg Ile Arg Phe
            20                  25                  30

Ser Arg Glu Met Phe Asn Lys Trp Gln Ala Gln Arg Trp Trp Gly Glu
        35                  40                  45

Asn Tyr Asp Arg Ile Thr Glu Leu Tyr Asn Val Gln Arg Phe Asn
    50                  55                  60

<210>  84
<211>  55
<212>  PRT
<213>  Artificial sequence

<220>
<223>  Conserved domain 2

<400>  84

Glu Trp Val Glu Gln Asp Glu Pro Gly Val Tyr Ile Thr Ile Arg Gln
1               5                   10                  15

Leu Ala Asp Gly Thr Arg Glu Leu Arg Arg Val Arg Phe Ser Arg Glu
            20                  25                  30

Gln Phe Gly Glu Val His Ala Lys Thr Trp Trp Glu Gln Asn Arg Glu
        35                  40                  45

Arg Ile Gln Ala Gln Tyr Leu
    50                  55

<210>  85
<211>  25
<212>  PRT
<213>  Artificial sequence

<220>
<223>  Conserved domain 3

<400>  85

Lys Ser Leu Thr Ser Gln Ile Lys Asp Met Ala Leu Lys Met Ser Gly
1               5                   10                  15

Ala Tyr Lys Gln Cys Lys Pro Cys Thr
            20                  25

<210>  86
<211>  9
<212>  PRT
<213>  Artificial sequence

<220>
<223>  Conserved domain 4

<400>  86

Met Phe Thr Cys Ile Ala Cys Thr Lys
1               5

<210>  87
<211>  2194
<212>  DNA
<213>  Oryza sativa

<400> 87
aatccgaaaa gtttctgcac cgttttcacc ccctaactaa caatataggg aacgtgtgct 60

aaatataaaa tgagacctta tatatgtagc gctgataact agaactatgc aagaaaaact 120

catccaccta ctttagtggc aatcgggcta aataaaaaag agtcgctaca ctagtttcgt 180

tttccttagt aattaagtgg gaaaatgaaa tcattattgc ttagaatata cgttcacatc 240

tctgtcatga agttaaatta ttcgaggtag ccataattgt catcaaactc ttcttgaata 300

aaaaaatctt tctagctgaa ctcaatgggt aaagagagag atttttttta aaaaaataga 360

atgaagatat tctgaacgta ttggcaaaga tttaaacata taattatata attttatagt 420

ttgtgcattc gtcatatcgc acatcattaa ggacatgtct tactccatcc caatttttat 480

ttagtaatta aagacaattg acttattttt attatttatc ttttttcgat tagatgcaag 540

gtacttacgc acacactttg tgctcatgtg catgtgtgag tgcacctcct caatacacgt 600

tcaactagca acacatctct aatatcactc gcctatttaa tacatttagg tagcaatatc 660

tgaattcaag cactccacca tcaccagacc acttttaata atatctaaaa tacaaaaaat 720

aattttacag aatagcatga aaagtatgaa acgaactatt taggtttttc acatacaaaa 780

aaaaaaagaa ttttgctcgt gcgcgagcgc caatctccca tattgggcac acaggcaaca 840

acagagtggc tgcccacaga acaacccaca aaaaacgatg atctaacgga ggacagcaag 900

tccgcaacaa ccttttaaca gcaggctttg cggccaggag agaggaggag aggcaaagaa 960

aaccaagcat cctccttctc ccatctataa attcctcccc ccttttcccc tctctatata 1020

ggaggcatcc aagccaagaa gagggagagc accaaggaca cgcgactagc agaagccgag 1080

cgaccgcctt ctcgatccat atcttccggt cgagttcttg gtcgatctct tccctcctcc 1140

acctcctcct cacagggtat gtgcctccct tcggttgttc ttggatttat tgttctaggt 1200

tgtgtagtac gggcgttgat gttaggaaag gggatctgta tctgtgatga ttcctgttct 1260

tggatttggg atagaggggt tcttgatgtt gcatgttatc ggttcggttt gattagtagt 1320

atggttttca atcgtctgga gagctctatg gaaatgaaat ggtttaggga tcggaatctt 1380

gcgattttgt gagtaccttt tgtttgaggt aaaatcagag caccggtgat tttgcttggt 1440

gtaataaagt acggttgttt ggtcctcgat tctggtagtg atgcttctcg atttgacgaa 1500

gctatccttt gtttattccc tattgaacaa aaataatcca actttgaaga cggtcccgtt 1560

gatgagattg aatgattgat tcttaagcct gtccaaaatt tcgcagctgg cttgtttaga 1620

tacagtagtc cccatcacga aattcatgga aacagttata atcctcagga acaggggatt 1680

ccctgttctt ccgatttgct ttagtcccag aatttttttt cccaaatatc ttaaaaagtc 1740

actttctggt tcagttcaat gaattgattg ctacaaataa tgcttttata gcgttatcct 1800

agctgtagtt cagttaatag gtaatacccc tatagtttag tcaggagaag aacttatccg 1860

atttctgatc tccattttta attatatgaa atgaactgta gcataagcag tattcatttg 1920

gattattttt tttattagct ctcacccctt cattattctg agctgaaagt ctggcatgaa 1980

ctgtcctcaa ttttgttttc aaattcacat cgattatcta tgcattatcc tcttgtatct 2040

acctgtagaa gtttcttttt ggttattcct tgactgcttg attacagaaa gaaatttatg 2100

aagctgtaat cgggatagtt atactgcttg ttcttatgat tcatttcctt tgtgcagttc 2160

ttggtgtagc ttgccacttt caccagcaaa gttc 2194

<210> 88
<211> 52
<212> DNA
<213> Artificial sequence

<220>
<223> primer: prm11475

<400> 88
ggggacaagt ttgtacaaaa aagcaggctt aaacaatgtt tacgtgcata gc 52

<210> 89
<211> 50
<212> DNA
<213> Artificial sequence

<220>
<223> primer: prm11476

<400> 89
ggggaccact ttgtacaaga aagctgggtg caatttaggt catgggaaat 50

<210> 90
<211> 800
<212> DNA
<213> Populus trichocarpa

<400> 90
cagctttcag aaaaaaagta aaggaaaact atgggtcgtg gaaagattga gatcaaaaag 60

attgaaaacc ccactaacag gcaagtcacc tactcaaaaa gaagaaatgg tattttcaag 120

aaagcccagg aactcactgt tctttgtgat gctaaggtct ctcttatcat gttctccaac 180

actaacaaat tccatgagta cattagcccc tccacaacga caaagaagat ctacgatcaa 240

tatcagaagg ctttaggcat agatctgtgg agcgctcaat acgagaaaat gcaagagcaa 300

ttgcggaagc tgaaagatat caatcataag ctgaaaaaag aaatcaggca gaggatagga 360

gaggacctta atgaattgag cattgatcat ctgcgcgttc ttgagcaaaa tatgactgaa 420

gccttgaatg gtgtccgtgg caggaagtac catgttatca aaacacaaac cgaaacctat 480

aaaaagaagg taaggagttt ggaggagaga catggaaacc tctggatgga atatgaagca 540

aaaatggagg atccacgata tggattagtg gacagtgaag gagattatga atctgctgct 600

gctcttgtaa atggggcttc caacctctat gcattccgcc tgcaccaggg gcaccaccac 660

caccatccta atcttcacct tgctggtggg tttggacccc atgacctccg tcttccttga 720

gtggtcctag tcttcagaca tctaatctaa gtgctctcgt gataattaga gatgctatct 780

ctgatattga ataatgaaga 800

<210> 91
<211> 229
<212> PRT
<213> Populus trichocarpa

<400> 91

Met Gly Arg Gly Lys Ile Glu Ile Lys Lys Ile Glu Asn Pro Thr Asn
1               5                   10                  15

Arg Gln Val Thr Tyr Ser Lys Arg Arg Asn Gly Ile Phe Lys Lys Ala
            20                  25                  30

Gln Glu Leu Thr Val Leu Cys Asp Ala Lys Val Ser Leu Ile Met Phe
        35                  40                  45

Ser Asn Thr Asn Lys Phe His Glu Tyr Ile Ser Pro Ser Thr Thr Thr
    50                  55                  60

Lys Lys Ile Tyr Asp Gln Tyr Gln Lys Ala Leu Gly Ile Asp Leu Trp
65                  70                  75                  80

Ser Ala Gln Tyr Glu Lys Met Gln Glu Gln Leu Arg Lys Leu Lys Asp
                85                  90                  95

Ile Asn His Lys Leu Lys Lys Glu Ile Arg Gln Arg Ile Gly Glu Asp
            100                 105                 110

Leu Asn Glu Leu Ser Ile Asp His Leu Arg Val Leu Glu Gln Asn Met
        115                 120                 125

Thr Glu Ala Leu Asn Gly Val Arg Gly Arg Lys Tyr His Val Ile Lys
    130                 135                 140

Thr Gln Thr Glu Thr Tyr Lys Lys Lys Val Arg Ser Leu Glu Glu Arg
145                 150                 155                 160

His Gly Asn Leu Trp Met Glu Tyr Glu Ala Lys Met Glu Asp Pro Arg
                165                 170                 175

Tyr Gly Leu Val Asp Ser Glu Gly Asp Tyr Glu Ser Ala Ala Ala Leu
            180                 185                 190

Val Asn Gly Ala Ser Asn Leu Tyr Ala Phe Arg Leu His Gln Gly His
        195                 200                 205

His His His His Pro Asn Leu His Leu Ala Gly Gly Phe Gly Pro His
    210                 215                 220

Asp Leu Arg Leu Pro
225

<210>  92
<211>  959
<212>  DNA
<213>  Solanum lycopersicum

<400>  92
ctcaatttct acttcttcaa aaatgggccg tggaaaaatt gagatcaaga agattgaaaa      60

ctcgacaaac aggcaggtca cttactccaa gagaagaaac ggtattttca agaaagctaa     120

agaacttact gttctttgtg acgctaagat ctctctcatc atgctatcaa gcaccaggaa     180

gtatcatgag tacacaagcc caaacactac gacaaaaaag atgattgatc agtatcagag     240

tgcacttgga gttgatatct ggagcattca ctacgagaaa atgcaagaaa acttgaagag     300

attgaaagag atcaataaca agctaagaag agagataagg cagagaacag gggaagacat     360

gagcggacta aatttgcagg aactatgtca cttgcaggag aacatcactg aatctgttgc     420

tgagattcgt gaacgaaagt accacgtgat caagaatcaa acagacacct gcaagaagaa     480

ggcgaggaac ttagaagagc aaaatggaaa ccttgtactt gacttggaag caaaatgtga     540

agatccaaag tatggtgttg tggaaaatga ggggcattac cactctgctg tggcatttgc     600

gaatggagta cacaatcttt atgcttttcg cctacaacca ttgcacccca atcttcaaaa     660

cgaaggagga tttggttctc gtgatctacg tctctcctga agatatcagt tcacagtaat     720

ggcgttaaac attaatgctg agttacttat tcaaatcaac ttcccgaatt atatcttatt     780

cctaaaaaaa attaaatatt gcaagctgca aacactactt tatctaacta atttgctccg     840

agtctggatt tggttctgtg ttaagcactc tattattcta ggtgtttcaa ctcctttcta     900

tatatgaatt atgatgcctt gaacgcatgt tcaattaatc aattttcctt ttcgacttg      959

<210>  93
<211>  225
<212>  PRT
<213>  Solanum lycopersicum

<400>  93

Met Gly Arg Gly Lys Ile Glu Ile Lys Lys Ile Glu Asn Ser Thr Asn
1               5                   10                  15

Arg Gln Val Thr Tyr Ser Lys Arg Arg Asn Gly Ile Phe Lys Lys Ala
            20                  25                  30

Lys Glu Leu Thr Val Leu Cys Asp Ala Lys Ile Ser Leu Ile Met Leu
        35                  40                  45

Ser Ser Thr Arg Lys Tyr His Glu Tyr Thr Ser Pro Asn Thr Thr Thr
    50                  55                  60

Lys Lys Met Ile Asp Gln Tyr Gln Ser Ala Leu Gly Val Asp Ile Trp
65                  70                  75                  80

Ser Ile His Tyr Glu Lys Met Gln Glu Asn Leu Lys Arg Leu Lys Glu
                85                  90                  95

Ile Asn Asn Lys Leu Arg Arg Glu Ile Arg Gln Arg Thr Gly Glu Asp
            100                 105                 110

Met Ser Gly Leu Asn Leu Gln Glu Leu Cys His Leu Gln Glu Asn Ile
        115                 120                 125

Thr Glu Ser Val Ala Glu Ile Arg Glu Arg Lys Tyr His Val Ile Lys
    130                 135                 140

Asn Gln Thr Asp Thr Cys Lys Lys Lys Ala Arg Asn Leu Glu Glu Gln
145                 150                 155                 160

Asn Gly Asn Leu Val Leu Asp Leu Glu Ala Lys Cys Glu Asp Pro Lys
                165                 170                 175

Tyr Gly Val Val Glu Asn Glu Gly His Tyr His Ser Ala Val Ala Phe
            180                 185                 190

Ala Asn Gly Val His Asn Leu Tyr Ala Phe Arg Leu Gln Pro Leu His
        195                 200                 205

Pro Asn Leu Gln Asn Glu Gly Gly Phe Gly Ser Arg Asp Leu Arg Leu
    210                 215                 220

Ser
225

<210>  94
<211>  1117
<212>  DNA
<213>  Triticum aestivum

<400>  94

ccagcagctc gagaagcagc agcagcagca gctagccgat ctcccctcct tccccccata 60

ctctttcttc tccacccgtc gctgcgtcgg ccgacctagc tagccagctc gctcgctcgc 120

ggtggcgcgc gcgattgcgg ggtcggagga ggtggatcgg gcggcggaga tggggcgggg 180

gaagatcgag ataaagcgga tcgagaacgc cacaaacagg caggtgacct actccaagcg 240

ccggtcgggg atcatgaaga aggcgcggga gctcaccgtg ctctgcgacg cccaggtcgc 300

catcatcatg ttctcctcca ccggcaagta ccacgagttc tgcagcaccg gcaccgacat 360

caaggggatc tttgaccgct accagcaggc catcgggacc agcctgtgga tcgagcagta 420

tgagaatatg cagcgcacgc tgagccatct caaggacatc aatcggaacc tgcgcaccga 480

gatcaggcaa aggatgggtg aagatctgga cgcgctggag ttcgaggagc tgcgcgacct 540

tgagcaaaat gtcgatgccg ctctcaagga ggttcgccag aggaagtatc atgtgatcac 600

cacgcagact gaaacctaca agaagaaggt gaagcactcc caggaggcat acaagaatct 660

gcagcaggag ctgggtatgc gcgaggaccc ggcgtacggg ttcgtggaca acccggctgc 720

gggcgggtgg gacggcgtgg cagcggtggc gatgggcggc ggctcggcgg cggacatgta 780

cgccttccgc gtggtgccca gccagcccaa cctgcacggc atggcctacg gcggctccca 840

cgacctgcgc ctcggctaat cgatcacttc gatcgctcct actagcttat atatcaagtg 900

atcgatcaag ttaccacaat aagaaaaaaa ttctgtgtgt ttgtatttgt gaaatgctgt 960

gatcgatgat gccttatctc ggtctcgtgc acatgagtca gttcaatgtg taattaacgc 1020

cgtagtgctc gactgtgtat tgtattgtat gatctgctat gactttggtt gtgagctact 1080

ttgccagtac tataatatat tttaaaaaaa aaaaaaa 1117

<210> 95
<211> 229
<212> PRT
<213> Triticum aestivum

<400> 95

Met Gly Arg Gly Lys Ile Glu Ile Lys Arg Ile Glu Asn Ala Thr Asn
1               5                   10                  15

Arg Gln Val Thr Tyr Ser Lys Arg Arg Ser Gly Ile Met Lys Lys Ala
            20                  25                  30

Arg Glu Leu Thr Val Leu Cys Asp Ala Gln Val Ala Ile Ile Met Phe
        35                  40                  45

Ser Ser Thr Gly Lys Tyr His Glu Phe Cys Ser Thr Gly Thr Asp Ile
    50                  55                  60

Lys Gly Ile Phe Asp Arg Tyr Gln Gln Ala Ile Gly Thr Ser Leu Trp

65                  70                  75                  80

Ile Glu Gln Tyr Glu Asn Met Gln Arg Thr Leu Ser His Leu Lys Asp
                85                  90                  95

Ile Asn Arg Asn Leu Arg Thr Glu Ile Arg Gln Arg Met Gly Glu Asp
            100                 105                 110

Leu Asp Ala Leu Glu Phe Glu Glu Leu Arg Asp Leu Glu Gln Asn Val
        115                 120                 125

Asp Ala Ala Leu Lys Glu Val Arg Gln Arg Lys Tyr His Val Ile Thr
    130                 135                 140

Thr Gln Thr Glu Thr Tyr Lys Lys Lys Val Lys His Ser Gln Glu Ala
145                 150                 155                 160

Tyr Lys Asn Leu Gln Gln Glu Leu Gly Met Arg Glu Asp Pro Ala Tyr
                165                 170                 175

Gly Phe Val Asp Asn Pro Ala Ala Gly Gly Trp Asp Gly Val Ala Ala
            180                 185                 190

Val Ala Met Gly Gly Gly Ser Ala Ala Asp Met Tyr Ala Phe Arg Val
        195                 200                 205

Val Pro Ser Gln Pro Asn Leu His Gly Met Ala Tyr Gly Gly Ser His
    210                 215                 220

Asp Leu Arg Leu Gly
225

<210>  96
<211>  2194
<212>  DNA
<213>  Oryza sativa

<400>  96
aatccgaaaa gtttctgcac cgttttcacc ccctaactaa caatataggg aacgtgtgct      60

aaatataaaa tgagacctta tatatgtagc gctgataact agaactatgc aagaaaaact     120

catccaccta ctttagtggc aatcgggcta aataaaaaag agtcgctaca ctagtttcgt     180

tttccttagt aattaagtgg gaaaatgaaa tcattattgc ttagaatata cgttcacatc     240

tctgtcatga agttaaatta ttcgaggtag ccataattgt catcaaactc ttcttgaata     300

aaaaaatctt tctagctgaa ctcaatgggt aaagagagag atttttttta aaaaaataga     360

atgaagatat tctgaacgta ttggcaaaga tttaaacata taattatata attttatagt     420

ttgtgcattc gtcatatcgc acatcattaa ggacatgtct tactccatcc caatttttat 480

ttagtaatta aagacaattg acttattttt attatttatc ttttttcgat tagatgcaag 540

gtacttacgc acacactttg tgctcatgtg catgtgtgag tgcacctcct caatacacgt 600

tcaactagca acacatctct aatatcactc gcctatttaa tacatttagg tagcaatatc 660

tgaattcaag cactccacca tcaccagacc acttttaata atatctaaaa tacaaaaaat 720

aattttacag aatagcatga aaagtatgaa acgaactatt taggtttttc acatacaaaa 780

aaaaaaagaa ttttgctcgt gcgcgagcgc caatctccca tattgggcac acaggcaaca 840

acagagtggc tgcccacaga acaacccaca aaaaacgatg atctaacgga ggacagcaag 900

tccgcaacaa ccttttaaca gcaggctttg cggccaggag agaggaggag aggcaaagaa 960

aaccaagcat cctccttctc ccatctataa attcctcccc ccttttcccc tctctatata 1020

ggaggcatcc aagccaagaa gagggagagc accaaggaca cgcgactagc agaagccgag 1080

cgaccgcctt ctcgatccat atcttccggt cgagttcttg gtcgatctct tccctcctcc 1140

acctcctcct cacagggtat gtgcctccct tcggttgttc ttggatttat tgttctaggt 1200

tgtgtagtac gggcgttgat gttaggaaag gggatctgta tctgtgatga ttcctgttct 1260

tggatttggg atagaggggt tcttgatgtt gcatgttatc ggttcggttt gattagtagt 1320

atggttttca atcgtctgga gagctctatg gaaatgaaat ggtttaggga tcggaatctt 1380

gcgattttgt gagtaccttt tgtttgaggt aaaatcagag caccggtgat tttgcttggt 1440

gtaataaagt acggttgttt ggtcctcgat tctggtagtg atgcttctcg atttgacgaa 1500

gctatccttt gtttattccc tattgaacaa aaataatcca actttgaaga cggtcccgtt 1560

gatgagattg aatgattgat tcttaagcct gtccaaaatt tcgcagctgg cttgtttaga 1620

tacagtagtc cccatcacga aattcatgga aacagttata atcctcagga acaggggatt 1680

ccctgttctt ccgatttgct ttagtcccag aatttttttt cccaaatatc ttaaaaagtc 1740

actttctggt tcagttcaat gaattgattg ctacaaataa tgcttttata gcgttatcct 1800

agctgtagtt cagttaatag gtaatacccc tatagtttag tcaggagaag aacttatccg 1860

atttctgatc tccattttta attatatgaa atgaactgta gcataagcag tattcatttg 1920

gattattttt tttattagct ctcacccctt cattattctg agctgaaagt ctggcatgaa 1980

ctgtcctcaa ttttgttttc aaattcacat cgattatcta tgcattatcc tcttgtatct 2040

acctgtagaa gtttcttttt ggttattcct tgactgcttg attacagaaa gaaatttatg 2100

aagctgtaat cgggatagtt atactgcttg ttcttatgat tcatttcctt tgtgcagttc 2160

ttggtgtagc ttgccacttt caccagcaaa gttc 2194

<210> 97
<211> 54

<212>  DNA
<213>  Artificial sequence

<220>
<223>  primer: prm18902

<400>  97
ggggacaagt ttgtacaaaa aagcaggctt aaacaatggg tcgtggaaag attg          54


<210>  98
<211>  51
<212>  DNA
<213>  Artificial sequence

<220>
<223>  primer: prm18903

<400>  98
ggggaccact ttgtacaaga aagctgggtg aagactagga ccactcaagg a             51


<210>  99
<211>  52
<212>  DNA
<213>  Artificial sequence

<220>
<223>  primer: prm18904

<400>  99
ggggacaagt ttgtacaaaa aagcaggctt aaacaatggg ccgtggaaaa at            52


<210>  100
<211>  50
<212>  DNA
<213>  Artificial sequence

<220>
<223>  primer: prm18905

<400>  100
ggggaccact ttgtacaaga aagctgggta acgccattac tgtgaactga               50


<210>  101
<211>  1474
<212>  DNA
<213>  Arabidopsis thaliana

<400>  101
aaaccgattc atttaaacaa caaaaaacca cttacagagt tcgttcgccg gcggcggcga      60

gtcatcgtca tcaatgtcaa acatcgttgt tctagacaac ggcggtggtc taatcaaagc     120

cggacaaggc ggcgagcgtg atcccaccac cgtaatcccg aactgtctct acaaacctct     180

ctcttccaaa aaattcatcc acccatcacc actcaccact ctctccgacg aaatcgacct     240

cacctccgcc gccgtacgcc gcccaatcga ccgtggctac ctcataaact ccgacctaca     300

acgcgaaatc tggtcacacc tattcacttc cctcctccac atagctcctt cctcctcctc     360

tctcctcctc accgaagcac cactctcaat cccttccgtt caacgaacca ccgacgaact 420

cgtcttcgaa gatttcggat tctcttctct ctatatagct catcctcaat ctcttgttca 480

tctctatgaa gctagtcgtc agcctgattc aatcctctca aagactcagt gtagtctcgt 540

tgttgattgt ggtttctcct tcactcacgc tgttcctgtt cttcacaatt tcactcttaa 600

tcacgccatt aagaggattg atttaggagg aaaagctttt actaattact tgaaggaatt 660

ggtttcttat agatctatta atgttatgga tgaaactttt ttagttgatg atgctaaaga 720

gaagctttgt tttgtttcac ttgatcttct tcgtgatctc cgccttgcta gaaatgggaa 780

cactcttatc aagtctactt atgttcttcc tgatggtgtt acacatacca aaggctatgt 840

taaagaccct caagctgcta agaggtttct tagtttgtca gagaaagagt ctgtggtggt 900

gatggataag gttggggaga gaaagaaggc tgacatgaac aaaaatgaga ttgatttaac 960

gaatgagcgt tttcttgtac ctgagacgtt attccagcct gcagatttag ggatgaatca 1020

ggcgggactt gcagagtgca ttgtccgagc tataaactca tgccattctt acttgcaacc 1080

agttttgtat caaagcatta tcttaactgg tggaagtaca ttatttccac aacttaagga 1140

gagactagaa ggagagcttc gaccacttgt cccagatcac tttgatgtga agataacaac 1200

tcaggaggac cccatactag gtgtatggag aggtggttca cttttggctt ccagcccgga 1260

tttcgagtcc atgtgtgtca ccaaggctga gtacgaagaa cttggatcag ctcggtgtcg 1320

tagacgattc tttcattgag gctaaccaaa acaatatcac atgagttggt tgtaagttga 1380

aattcttcac attatttgtc gtatattgta atgcatgtat tgtttttctt cttcctctaa 1440

tttgataaac ttcaaaccgt ttaagtaata acca 1474

<210> 102
<211> 421
<212> PRT
<213> Arabidopsis thaliana

<400> 102

Met Ser Asn Ile Val Val Leu Asp Asn Gly Gly Gly Leu Ile Lys Ala
1               5                   10                  15

Gly Gln Gly Gly Glu Arg Asp Pro Thr Thr Val Ile Pro Asn Cys Leu
            20                  25                  30

Tyr Lys Pro Leu Ser Ser Lys Lys Phe Ile His Pro Ser Pro Leu Thr
        35                  40                  45

Thr Leu Ser Asp Glu Ile Asp Leu Thr Ser Ala Ala Val Arg Arg Pro
    50                  55                  60

Ile Asp Arg Gly Tyr Leu Ile Asn Ser Asp Leu Gln Arg Glu Ile Trp
65                  70                  75                  80

Ser His Leu Phe Thr Ser Leu Leu His Ile Ala Pro Ser Ser Ser Ser
                85                  90                  95

Leu Leu Leu Thr Glu Ala Pro Leu Ser Ile Pro Ser Val Gln Arg Thr
            100                 105                 110

Thr Asp Glu Leu Val Phe Glu Asp Phe Gly Phe Ser Ser Leu Tyr Ile
        115                 120                 125

Ala His Pro Gln Ser Leu Val His Leu Tyr Glu Ala Ser Arg Gln Pro
    130                 135                 140

Asp Ser Ile Leu Ser Lys Thr Gln Cys Ser Leu Val Val Asp Cys Gly
145                 150                 155                 160

Phe Ser Phe Thr His Ala Val Pro Val Leu His Asn Phe Thr Leu Asn
                165                 170                 175

His Ala Ile Lys Arg Ile Asp Leu Gly Gly Lys Ala Phe Thr Asn Tyr
            180                 185                 190

Leu Lys Glu Leu Val Ser Tyr Arg Ser Ile Asn Val Met Asp Glu Thr
        195                 200                 205

Phe Leu Val Asp Asp Ala Lys Glu Lys Leu Cys Phe Val Ser Leu Asp
    210                 215                 220

Leu Leu Arg Asp Leu Arg Leu Ala Arg Asn Gly Asn Thr Leu Ile Lys
225                 230                 235                 240

Ser Thr Tyr Val Leu Pro Asp Gly Val Thr His Thr Lys Gly Tyr Val
                245                 250                 255

Lys Asp Pro Gln Ala Ala Lys Arg Phe Leu Ser Leu Ser Glu Lys Glu
            260                 265                 270

Ser Val Val Val Met Asp Lys Val Gly Glu Arg Lys Lys Ala Asp Met
        275                 280                 285

Asn Lys Asn Glu Ile Asp Leu Thr Asn Glu Arg Phe Leu Val Pro Glu
    290                 295                 300

Thr Leu Phe Gln Pro Ala Asp Leu Gly Met Asn Gln Ala Gly Leu Ala
305                 310                 315                 320

Glu Cys Ile Val Arg Ala Ile Asn Ser Cys His Ser Tyr Leu Gln Pro
                325                 330                 335

Val Leu Tyr Gln Ser Ile Ile Leu Thr Gly Gly Ser Thr Leu Phe Pro
            340                 345                 350

Gln Leu Lys Glu Arg Leu Glu Gly Glu Leu Arg Pro Leu Val Pro Asp
        355                 360                 365

His Phe Asp Val Lys Ile Thr Thr Gln Glu Asp Pro Ile Leu Gly Val
    370                 375                 380

Trp Arg Gly Gly Ser Leu Leu Ala Ser Ser Pro Asp Phe Glu Ser Met
385                 390                 395                 400

Cys Val Thr Lys Ala Glu Tyr Glu Glu Leu Gly Ser Ala Arg Cys Arg
                405                 410                 415

Arg Arg Phe Phe His
            420

<210>  103
<211>  2283
<212>  DNA
<213>  Oryza sativa

<400>  103
aaacgctact acccctccgc cacagcgtcc acacccgcgc cgccgccgcc ctcaccctcg      60

ccgccgcgca tcccgcggtc ccaccgtcgc cgattccacc gctgccacgg acaaccccgc     120

cgcccctttc cttcctcgcc gtgggagcgc cccccccctc caggttggct agcagctagc     180

tccgccggcc gcccttcccc gatgggggca gcggcgtagg cgccgccatc agccgccgta     240

cgtatgtgag cggcgcctgc gcacgcctcc agctggtccg cgcgagaaga agaagaagca     300

gaagagagaa acagtggctt ttttttgctg aaccagatgc cgatgcagtg acgattcgtt     360

ggccgaggat cgcgttctta taggcccgat ttgcaaagga gtgcttgtaa atccaatcat     420

accatttcga ttttcatttg accaaggata ggcaagaata ggaggaaaaa ggagagtggc     480

atataatgac gggtggatca ggtgttgtgg tgctagacaa tgggggtggt cttctgaagg     540

ctggatttgg tggggacatg aatccgactg ctgttgtccc caactgtatg gccaagcccc     600

ctggttccaa gaaatggcta gttgctgacc agctgcaggc acaagatgtt gatgttactg     660

gcatgacatt gaggcgtcct attgatcgtg gctatctcat caatcaagaa gtgcaacggg     720

aggtgtggga gcgggttata cgcaacctac tgcaggtgga tcctaacaac tcatcgttgc     780

tactggtgga accacagttc aaccctccag cactgcagca tgcaaccgat gagcttgttt     840

ttgaggagct tggtttcaaa tctctttgtg ttgcagatgc cccttccctt gttcaccttt 900

atgaggctag ccgccagcca tcgctgtttc gagctcaatg tagccttgtt gttgactgtg 960

gcttctcttt cactcatgca tctcccgtgc ttcaaaactt tacactgaat tatgctgtgc 1020

ggcgcatgga ccttggtgga aaggccctca caaactatct caaagagctc atttcatatc 1080

gctcccttaa tgtcatggat gaaacactcc tcattgatga tgcaaaggaa aaactatgct 1140

ttgtatccct tgatgtccct ggtgatcttc gtcttgccag gttatcatct aatgacaacc 1200

cttttagatg ctcctacatt ctccctgatg gtataacata caagaaaggg tttgtgaagg 1260

acttggatga ggcatgcaga tacagctctc tgcctgctaa tggagaatcg gttagaaagg 1320

atagttctga cagcgatagg agcaagtttg aggataagaa aaagcctgaa cttagtcaaa 1380

atgaatttgt gttgaccaat gagaggttcc tagtgccaga gatgcttttc catccaattg 1440

atctgggtat gaatcaagct gggcttgctg agtgcatagt tcgtgctata caagcttgcc 1500

acccacatct tcaacctgtg ctttttgaga gaattatcct gacaggagga agcacgctat 1560

tccctcgatt caccgaaaga ttggaaaagg aacttcgtcc tcttgtgcct gatgactacc 1620

aagtaaagat aattgctcag gaggacccaa ttcttggtgc ctggagaggt ggatctcttt 1680

tggcgcacag gcctgatttt gaatcaatgt gcattacaaa atcagagtat gaagagatgg 1740

gttcaatgcg gtgccgtcgt agattctttc actgaaagtt gtgtgccagc agctcagtag 1800

aagtgcaatt tgtaagtatg attcagcact atctagttca ggtcttgaag aaatactcat 1860

taattaggca aacgagaagt ttggttctag aaggtaatga tgcacaattt taacacgtgg 1920

tcattttttt acataggaat tagaagctat tactccatgt atctggtccc ccttattact 1980

ggcaaccaat tctttcagcc ttcctaccag ctaaatatgc agatatagtc cttaccaggg 2040

aaaacctttg tggtctaaca ccctcggaac acagttgctc tgagataaat ggtgaatttt 2100

gcttttctgc tcggtgaaaa gttttcttaa tttttttccg agtaaacagc gtgcatggaa 2160

atattttatt agtcatttac tctggcaata cctgacctgt gtacgccaca cagagcttta 2220

gtttagaaac aacagtatgc atgatcgtct tttagaagca taaatattag tatgttagtt 2280

tgt 2283

<210> 104
<211> 429
<212> PRT
<213> Oryza sativa

<400> 104

Met Thr Gly Gly Ser Gly Val Val Val Leu Asp Asn Gly Gly Gly Leu
1               5                   10                  15

Leu Lys Ala Gly Phe Gly Gly Asp Met Asn Pro Thr Ala Val Val Pro

20                  25                  30

Asn Cys Met Ala Lys Pro Pro Gly Ser Lys Lys Trp Leu Val Ala Asp
        35                  40                  45

Gln Leu Gln Ala Gln Asp Val Asp Val Thr Gly Met Thr Leu Arg Arg
    50                  55                  60

Pro Ile Asp Arg Gly Tyr Leu Ile Asn Gln Glu Val Gln Arg Glu Val
65                  70                  75                  80

Trp Glu Arg Val Ile Arg Asn Leu Leu Gln Val Asp Pro Asn Asn Ser
                85                  90                  95

Ser Leu Leu Leu Val Glu Pro Gln Phe Asn Pro Pro Ala Leu Gln His
            100                 105                 110

Ala Thr Asp Glu Leu Val Phe Glu Glu Leu Gly Phe Lys Ser Leu Cys
        115                 120                 125

Val Ala Asp Ala Pro Ser Leu Val His Leu Tyr Glu Ala Ser Arg Gln
    130                 135                 140

Pro Ser Leu Phe Arg Ala Gln Cys Ser Leu Val Val Asp Cys Gly Phe
145                 150                 155                 160

Ser Phe Thr His Ala Ser Pro Val Leu Gln Asn Phe Thr Leu Asn Tyr
                165                 170                 175

Ala Val Arg Arg Met Asp Leu Gly Gly Lys Ala Leu Thr Asn Tyr Leu
            180                 185                 190

Lys Glu Leu Ile Ser Tyr Arg Ser Leu Asn Val Met Asp Glu Thr Leu
        195                 200                 205

Leu Ile Asp Asp Ala Lys Glu Lys Leu Cys Phe Val Ser Leu Asp Val
    210                 215                 220

Pro Gly Asp Leu Arg Leu Ala Arg Leu Ser Ser Asn Asp Asn Pro Phe
225                 230                 235                 240

Arg Cys Ser Tyr Ile Leu Pro Asp Gly Ile Thr Tyr Lys Lys Gly Phe
                245                 250                 255

Val Lys Asp Leu Asp Glu Ala Cys Arg Tyr Ser Ser Leu Pro Ala Asn
            260                 265                 270

Gly Glu Ser Val Arg Lys Asp Ser Ser Asp Ser Asp Arg Ser Lys Phe
        275                 280                 285

Glu Asp Lys Lys Lys Pro Glu Leu Ser Gln Asn Glu Phe Val Leu Thr
    290                 295                 300

Asn Glu Arg Phe Leu Val Pro Glu Met Leu Phe His Pro Ile Asp Leu
305                 310                 315                 320

Gly Met Asn Gln Ala Gly Leu Ala Glu Cys Ile Val Arg Ala Ile Gln
                325                 330                 335

Ala Cys His Pro His Leu Gln Pro Val Leu Phe Glu Arg Ile Ile Leu
            340                 345                 350

Thr Gly Gly Ser Thr Leu Phe Pro Arg Phe Thr Glu Arg Leu Glu Lys
        355                 360                 365

Glu Leu Arg Pro Leu Val Pro Asp Asp Tyr Gln Val Lys Ile Ile Ala
    370                 375                 380

Gln Glu Asp Pro Ile Leu Gly Ala Trp Arg Gly Gly Ser Leu Leu Ala
385                 390                 395                 400

His Arg Pro Asp Phe Glu Ser Met Cys Ile Thr Lys Ser Glu Tyr Glu
                405                 410                 415

Glu Met Gly Ser Met Arg Cys Arg Arg Arg Phe Phe His
            420                 425

<210>  105
<211>  1320
<212>  DNA
<213>  Physcomitrella patens

<400>  105
atgaagagga ctggcggagg ggtgacgtcc tccgtggtag ttctggacaa tggagcagga      60

tactgtaagg caggtatggc ggggcaatca gagcctacag cagtggtgcc taattgtatg     120

gcgcggccac gctcggcgaa gaaatggctc atcgcggacc agctgttgga gtgtgacgac     180

attcagagta ttgccatcaa gaggccgatc gacaggggtt atatgatcag cccggaaatc     240

gaacgcgaaa tatgggaccg cgtttttaag gtccacctta aggttaatcc tacggagtgt     300

gggttgatgc ttgttgaacc tttgttcacc ttgtcgtcaa ttcaaaaggc gacagatgag     360

cttgtgtttg aagatttggg gtttcaatcg ttttgtatat cgaattcggc tgtctctgcg     420

catgcgtacc aagcacagaa agcgccgacg agcatccttg ccaggggaag gactagctta     480

gtcgtggact cgggattttc tttcacgcat accgtgcccg tgttccagaa taaagctgtg     540

acgactgcgg cgaagcgtat taatctagga ggcaaagctc tcacaaatta tcttaaggag 600

ttggtgtcgt accgggcatg gaatgtaatg gacgagacat ataccatgga ggacgtgaag 660

gaaaaactgt gttttgtctc tttggacatt gaacgtgatc tcaacattgc tcgggtgaaa 720

ggcaaatcga acgctctgcg gcgcgagtat gtactgccgg atggcgtcaa acataagcgt 780

ggttttgtga aggaacttga acccctcgta aacaagcacc acctcaaatc aagcaagaag 840

atcaggtatt atctgcctcc ttgtgcagaa aaattgcggg agttttttgt caatcaagag 900

attactttaa caaatgagcg ctttctggtc ccggagatgc tatttcaccc tgctgacctt 960

gggatgaatg aggcgggact ggcagagtgc atcgttcgag caatcaatgc ttgtgaacct 1020

gaacttcatg ctctccttta tcagagtgtg ctgctaacgg gtgggaatac tttgttacct 1080

ggtttcaaga accgtttaga gcatgagctt cgtcctctgg taactgacga tttcaatatc 1140

aacattgaga tcgtggactg tccaatttta gcagcttgga agggtgcgtc tcttatggca 1200

gctagacccg agtttcacac ctgggctgtg accaaggccg agtacgaaga ggatggtact 1260

ctgcgatgtc gacaaaggtt tgtttcagct gattggtctc tttttaaaca atcaccataa 1320

<210> 106
<211> 439
<212> PRT
<213> Physcomitrella patens

<400> 106

Met Lys Arg Thr Gly Gly Gly Val Thr Ser Ser Val Val Val Leu Asp
1               5                   10                  15

Asn Gly Ala Gly Tyr Cys Lys Ala Gly Met Ala Gly Gln Ser Glu Pro
            20                  25                  30

Thr Ala Val Val Pro Asn Cys Met Ala Arg Pro Arg Ser Ala Lys Lys
        35                  40                  45

Trp Leu Ile Ala Asp Gln Leu Leu Glu Cys Asp Asp Ile Gln Ser Ile
    50                  55                  60

Ala Ile Lys Arg Pro Ile Asp Arg Gly Tyr Met Ile Ser Pro Glu Ile
65                  70                  75                  80

Glu Arg Glu Ile Trp Asp Arg Val Phe Lys Val His Leu Lys Val Asn
                85                  90                  95

Pro Thr Glu Cys Gly Leu Met Leu Val Glu Pro Leu Phe Thr Leu Ser
            100                 105                 110

Ser Ile Gln Lys Ala Thr Asp Glu Leu Val Phe Glu Asp Phe Gly Phe
        115                 120                 125

Gln Ser Phe Cys Ile Ser Asn Ser Ala Val Ser Ala His Ala Tyr Gln
    130                 135                 140

Ala Gln Lys Ala Pro Thr Ser Ile Leu Ala Arg Gly Arg Thr Ser Leu
145                 150                 155                 160

Val Val Asp Ser Gly Phe Ser Phe Thr His Thr Val Pro Val Phe Gln
                165                 170                 175

Asn Lys Ala Val Thr Thr Ala Ala Lys Arg Ile Asn Leu Gly Gly Lys
            180                 185                 190

Ala Leu Thr Asn Tyr Leu Lys Glu Leu Val Ser Tyr Arg Ala Trp Asn
        195                 200                 205

Val Met Asp Glu Thr Tyr Thr Met Glu Asp Val Lys Glu Lys Leu Cys
    210                 215                 220

Phe Val Ser Leu Asp Ile Glu Arg Asp Leu Asn Ile Ala Arg Val Lys
225                 230                 235                 240

Gly Lys Ser Asn Ala Leu Arg Arg Glu Tyr Val Leu Pro Asp Gly Val
                245                 250                 255

Lys His Lys Arg Gly Phe Val Lys Glu Leu Glu Pro Leu Val Asn Lys
            260                 265                 270

His His Leu Lys Ser Ser Lys Lys Ile Arg Tyr Tyr Leu Pro Pro Cys
        275                 280                 285

Ala Glu Lys Leu Arg Glu Phe Phe Val Asn Gln Glu Ile Thr Leu Thr
    290                 295                 300

Asn Glu Arg Phe Leu Val Pro Glu Met Leu Phe His Pro Ala Asp Leu
305                 310                 315                 320

Gly Met Asn Glu Ala Gly Leu Ala Glu Cys Ile Val Arg Ala Ile Asn
                325                 330                 335

Ala Cys Glu Pro Glu Leu His Ala Leu Leu Tyr Gln Ser Val Leu Leu
            340                 345                 350

Thr Gly Gly Asn Thr Leu Leu Pro Gly Phe Lys Asn Arg Leu Glu His
        355                 360                 365

Glu Leu Arg Pro Leu Val Thr Asp Asp Phe Asn Ile Asn Ile Glu Ile
    370                 375                 380

Val Asp Cys Pro Ile Leu Ala Ala Trp Lys Gly Ala Ser Leu Met Ala
385                 390                 395                 400

Ala Arg Pro Glu Phe His Thr Trp Ala Val Thr Lys Ala Glu Tyr Glu
                405                 410                 415

Glu Asp Gly Thr Leu Arg Cys Arg Gln Arg Phe Val Ser Ala Asp Trp
            420                 425                 430

Ser Leu Phe Lys Gln Ser Pro
        435

<210>  107
<211>  1385
<212>  DNA
<213>  Populus trichocarpa

<400>  107
aacccaccac ctccctaatc ctcaaacgac atgtcaagcg tcgtcgttct cgacaacggc      60

ggtggcctaa tcaaagccgg ctacggcggg gaacgtgacc cctccaccat aatcccaaac     120

tgcctctatc gtcctctctc ctccaaaaaa ttcctccacc ccactcccac caccgaagaa     180

gatctcacct ccgccgccgt ccgccgcccc atagaccgag gctacctaat aaacccagac     240

ctccaacgcg atatctggaa ccacctcttc tccaatctcc tccacataaa cccatcaaac     300

tcttctttac tactaacaga acccttgttt tctctccctt caattgaacg tgcaacggat     360

gagcttgttt ttgaagattt tgggttcaat tctctgttca tttctgatcc accgaagttg     420

gttcatcttt atgaggcgag tagaaggcca tatgggttag tttcaaaagc gcaatgtagc     480

ttagttgtgg attgtgggtt tagttttacg catgctgcgc ctgtgtttca gaactttacg     540

ttgaattatg gagtgaaaag gattgattta ggaggaaaag cgttgacgaa ttttttgaag     600

gaattggtgt cttacaggag tgttagtgtt atggatgaaa gttttattat ggatgatgtc     660

aaggagaagt tgtgctttgt ttctcttgat gttgctagag atttgaagat tgcaaggaga     720

cgaggaaacg acaatttttt taggtgtact tatgttctac ctgatggagt gacccacaca     780

aaaggttttg ttaaagaccc agatgaagca aagaagtatc tcactgtggg tgatggagca     840

tatttagaaa cgagaaagga tatggatcgc actgaaatta tggaccgaaa gaaagttgat     900

ttaactaaaa atgagtttga cttgacaaat gaacggttcc tggttccaga gatgattttc      960

cacccagcag atttaggtat gaatcaggct ggactagcag agtgcattgt tcgagctgtg    1020

aactcttgcc atcctcttct tcatcctcta ctctaccaaa gcattatatt aactggtgga    1080

agcacattgt tccctagatt tgctgagaga cttgaaatgg agcttcgacc tcttgtccca 1140

gatgactatc aagtgaagat aactacacaa gaagatccta ttctaggtgt gtggcgaggt 1200

ggatcccttt tggcatccag tcctgatttt gaagcaatgt gtgttaccaa ggcagagtat 1260

gaggaacttg gatctgctcg atgtcgaagg agattctttc attgagaaac aaaactgctc 1320

aaggtatttt atctccttac tgcatccacc tttcaagtta acatgtgctg ttttattgag 1380

aaggg 1385

<210> 108
<211> 424
<212> PRT
<213> Populus trichocarpa

<400> 108

Met Ser Ser Val Val Val Leu Asp Asn Gly Gly Gly Leu Ile Lys Ala
1               5                   10                  15

Gly Tyr Gly Gly Glu Arg Asp Pro Ser Thr Ile Ile Pro Asn Cys Leu
            20                  25                  30

Tyr Arg Pro Leu Ser Ser Lys Lys Phe Leu His Pro Thr Pro Thr Thr
        35                  40                  45

Glu Glu Asp Leu Thr Ser Ala Ala Val Arg Arg Pro Ile Asp Arg Gly
    50                  55                  60

Tyr Leu Ile Asn Pro Asp Leu Gln Arg Asp Ile Trp Asn His Leu Phe
65                  70                  75                  80

Ser Asn Leu Leu His Ile Asn Pro Ser Asn Ser Ser Leu Leu Leu Thr
                85                  90                  95

Glu Pro Leu Phe Ser Leu Pro Ser Ile Glu Arg Ala Thr Asp Glu Leu
            100                 105                 110

Val Phe Glu Asp Phe Gly Phe Asn Ser Leu Phe Ile Ser Asp Pro Pro
        115                 120                 125

Lys Leu Val His Leu Tyr Glu Ala Ser Arg Arg Pro Tyr Gly Leu Val
    130                 135                 140

Ser Lys Ala Gln Cys Ser Leu Val Val Asp Cys Gly Phe Ser Phe Thr
145                 150                 155                 160

His Ala Ala Pro Val Phe Gln Asn Phe Thr Leu Asn Tyr Gly Val Lys
                165                 170                 175

Arg Ile Asp Leu Gly Gly Lys Ala Leu Thr Asn Phe Leu Lys Glu Leu
        180                 185                 190

Val Ser Tyr Arg Ser Val Ser Val Met Asp Glu Ser Phe Ile Met Asp
    195                 200                 205

Asp Val Lys Glu Lys Leu Cys Phe Val Ser Leu Asp Val Ala Arg Asp
    210                 215                 220

Leu Lys Ile Ala Arg Arg Arg Gly Asn Asp Asn Phe Phe Arg Cys Thr
225                 230                 235                 240

Tyr Val Leu Pro Asp Gly Val Thr His Thr Lys Gly Phe Val Lys Asp
                245                 250                 255

Pro Asp Glu Ala Lys Lys Tyr Leu Thr Val Gly Asp Gly Ala Tyr Leu
            260                 265                 270

Glu Thr Arg Lys Asp Met Asp Arg Thr Glu Ile Met Asp Arg Lys Lys
        275                 280                 285

Val Asp Leu Thr Lys Asn Glu Phe Asp Leu Thr Asn Glu Arg Phe Leu
    290                 295                 300

Val Pro Glu Met Ile Phe His Pro Ala Asp Leu Gly Met Asn Gln Ala
305                 310                 315                 320

Gly Leu Ala Glu Cys Ile Val Arg Ala Val Asn Ser Cys His Pro Leu
                325                 330                 335

Leu His Pro Leu Leu Tyr Gln Ser Ile Ile Leu Thr Gly Gly Ser Thr
            340                 345                 350

Leu Phe Pro Arg Phe Ala Glu Arg Leu Glu Met Glu Leu Arg Pro Leu
        355                 360                 365

Val Pro Asp Asp Tyr Gln Val Lys Ile Thr Thr Gln Glu Asp Pro Ile
    370                 375                 380

Leu Gly Val Trp Arg Gly Gly Ser Leu Leu Ala Ser Ser Pro Asp Phe
385                 390                 395                 400

Glu Ala Met Cys Val Thr Lys Ala Glu Tyr Glu Glu Leu Gly Ser Ala
                405                 410                 415

Arg Cys Arg Arg Arg Phe Phe His
            420

<210>  109
<211>  1311
<212>  DNA
<213>  Glycine max

<400>  109
atgtcgacct cgacgaacgt ggtagtcctg gacaacggcg gcgggctgat caaggcgggc      60

atcggcggcg agcgcgaccc ctccgccata gtcccgaact gcctctaccg ccccccgtcg     120

tcgaagaagt ggctccacct ccactccggc gacgaggacc tcacctccgc cgccgtgcgc     180

cgccccatgg accgcggcta cctcataaac cccgacctcc agcgcgaaat ctggtcccac     240

ctcttctctt ccgtcctcca cataaaccct tcccagtcct cgctcctcct cacggagcct     300

ctcttcactc ccccctccat ccagcgctcg gtggacgagc tcgtcttcga ggacttcaac     360

ttccgggccc tgtacgtggc ccactccccc tccctcgtcc acctccacga ggccagccgc     420

aacaacgcca acgggctcct ctccaaggcc cagtgcagcc tcgtcctcga cgccgggttc     480

tccttcaccc acgcctcccc cgtcttccac aacttcgccc tcaactacgc cgtcaagagg     540

atcgacctcg gcggcaaggc cctcaccaac tacctcaagg agctcgtctc cttccgctcc     600

gttaatgtca tggaagagac cttcatcatc gacgacgtca aggagaaact ctgctttgtc     660

tcactcgacg tcaaccgcga cctcaccatc gccaggaaga gtgggaaaga gaatctgttc     720

aggtgcacct acgtgcttcc ggatggtgtc acgtacacaa aggggtttgt taagtatcca     780

gatcaggcgc agcgatatct tgcattgagg gagggtggcc ttcattcttc atcaccagtg     840

caagcgcagg aggatgtgaa tttcacggaa attgccgagc acccagagaa caggaagaga     900

gttgatttga caaaaaatga atttgacttg acaaatgaac ggtttcttgt gccagagatg     960

atcttccgtc ctgctgattt gggaatgaac caggctgggc tagcagaatg cattgtacgt    1020

gctgttaatg cgtgccatcc acatctccgc cctgttctct atgaaagcat cattttaact    1080

ggtggaagca ccttatttcc tcagtttgcc gagagactag agaaggagct tcggcctcta    1140

gttcctgatg actatcgtgt gaagataacg actcaagaag atcccatact aggtgtttgg    1200

cgtggaggtt cactgttggc atcaagtccg gattttgaag ctatgtgtgt gaccaagtct    1260

gagtatgagg agcttggttc tgctagatgt cgcaagagat tctttcatta a             1311


<210>  110
<211>  436
<212>  PRT
<213>  Glycine max

<400>  110

Met Ser Thr Ser Thr Asn Val Val Val Leu Asp Asn Gly Gly Gly Leu
1               5                   10                  15

Ile Lys Ala Gly Ile Gly Gly Glu Arg Asp Pro Ser Ala Ile Val Pro
            20                  25                  30

Asn Cys Leu Tyr Arg Pro Pro Ser Ser Lys Lys Trp Leu His Leu His
        35                  40                  45

Ser Gly Asp Glu Asp Leu Thr Ser Ala Ala Val Arg Arg Pro Met Asp
    50                  55                  60

Arg Gly Tyr Leu Ile Asn Pro Asp Leu Gln Arg Glu Ile Trp Ser His
65                  70                  75                  80

Leu Phe Ser Ser Val Leu His Ile Asn Pro Ser Gln Ser Ser Leu Leu
                85                  90                  95

Leu Thr Glu Pro Leu Phe Thr Pro Pro Ser Ile Gln Arg Ser Val Asp
            100                 105                 110

Glu Leu Val Phe Glu Asp Phe Asn Phe Arg Ala Leu Tyr Val Ala His
        115                 120                 125

Ser Pro Ser Leu Val His Leu His Glu Ala Ser Arg Asn Asn Ala Asn
    130                 135                 140

Gly Leu Leu Ser Lys Ala Gln Cys Ser Leu Val Leu Asp Ala Gly Phe
145                 150                 155                 160

Ser Phe Thr His Ala Ser Pro Val Phe His Asn Phe Ala Leu Asn Tyr
                165                 170                 175

Ala Val Lys Arg Ile Asp Leu Gly Gly Lys Ala Leu Thr Asn Tyr Leu
            180                 185                 190

Lys Glu Leu Val Ser Phe Arg Ser Val Asn Val Met Glu Glu Thr Phe
        195                 200                 205

Ile Ile Asp Asp Val Lys Glu Lys Leu Cys Phe Val Ser Leu Asp Val
    210                 215                 220

Asn Arg Asp Leu Thr Ile Ala Arg Lys Ser Gly Lys Glu Asn Leu Phe
225                 230                 235                 240

Arg Cys Thr Tyr Val Leu Pro Asp Gly Val Thr Tyr Thr Lys Gly Phe
                245                 250                 255

Val Lys Tyr Pro Asp Gln Ala Gln Arg Tyr Leu Ala Leu Arg Glu Gly
            260                 265                 270

Gly Leu His Ser Ser Ser Pro Val Gln Ala Gln Glu Asp Val Asn Phe
        275                 280                 285

Thr Glu Ile Ala Glu His Pro Glu Asn Arg Lys Arg Val Asp Leu Thr
    290                 295                 300

Lys Asn Glu Phe Asp Leu Thr Asn Glu Arg Phe Leu Val Pro Glu Met
305                 310                 315                 320

Ile Phe Arg Pro Ala Asp Leu Gly Met Asn Gln Ala Gly Leu Ala Glu
                325                 330                 335

Cys Ile Val Arg Ala Val Asn Ala Cys His Pro His Leu Arg Pro Val
            340                 345                 350

Leu Tyr Glu Ser Ile Ile Leu Thr Gly Gly Ser Thr Leu Phe Pro Gln
        355                 360                 365

Phe Ala Glu Arg Leu Glu Lys Glu Leu Arg Pro Leu Val Pro Asp Asp
    370                 375                 380

Tyr Arg Val Lys Ile Thr Thr Gln Glu Asp Pro Ile Leu Gly Val Trp
385                 390                 395                 400

Arg Gly Gly Ser Leu Leu Ala Ser Ser Pro Asp Phe Glu Ala Met Cys
                405                 410                 415

Val Thr Lys Ser Glu Tyr Glu Glu Leu Gly Ser Ala Arg Cys Arg Lys
            420                 425                 430

Arg Phe Phe His
        435

<210>  111
<211>  1083
<212>  DNA
<213>  Glycine max

<400>  111
atgtcgacat cgacgaacct ggtggtcctg gacaatggcg gtgggctaat aaaagcaggc      60

atcgacggcg agcacgaccc cttcgccata tggctccacc tctactctgg caatgaagac     120

ctcacctcta ccgccatgcg ctaccccgtg gaccgcggct acctcataaa cccggaccta     180

caacacaaaa tctggtccca cctcttctcc tccgtcctcc acacaaaccc ttccaaatcc     240

tctctcatcc tcacagagcc tctattcaca gccccctcca tccaatgctc catggatgaa     300

ctcatcttca aggactttaa cttttgggcc ctctacctgg ccgattctgc ctctgtcgtc     360

tacctctaca aggccagccg caacaatgcc aacgggatcc tctccaaggc ccaacaaagc 420

ctcgtcatgg acttgggctt ctccttcacc cacacctccc ccgtctttca caacttcgcc 480

ctcaactacg ccatcaggag aatcgacctc agtggcaagg ccctcactaa ctacctcaag 540

gatctcgtct ccttccgctc cgtcaacatc atggaagaga ctttcatcat caatgatgaa 600

tttgttaagt atccagatca ggcacaacac tatcttgcat tgagggagtg tggccttcct 660

tcttcaccat cagtggatgc accaggggat gtgaaatgcc tggaaattgc taagcagcca 720

gaggacagga agatagttga tttgacaaaa aattttcttg tgccaaagat gatatttcgt 780

cctgctgatt tgggtctatc aatacaatat atttgcattt ttcttaattt gaaacactca 840

tttttaaatg ttgtgtgtgt caccttattt cctcagtttg ttgagagact cgagaaggag 900

cttcggcctc tagttcctaa tgactatcgt gtgaagatag caactcaaga agatccctta 960

ctaggtgttt ggcgtggagg gtcactgttg gcatcaagtc cagactttga agctatgtgt 1020

gtgaccaagt ctgagtatga ggagcttggt tctgctagat gtcgcaagag attctttcat 1080

taa 1083

<210> 112
<211> 360
<212> PRT
<213> Glycine max

<400> 112

Met Ser Thr Ser Thr Asn Leu Val Val Leu Asp Asn Gly Gly Gly Leu
1               5                   10                  15

Ile Lys Ala Gly Ile Asp Gly Glu His Asp Pro Phe Ala Ile Trp Leu
            20                  25                  30

His Leu Tyr Ser Gly Asn Glu Asp Leu Thr Ser Thr Ala Met Arg Tyr
        35                  40                  45

Pro Val Asp Arg Gly Tyr Leu Ile Asn Pro Asp Leu Gln His Lys Ile
    50                  55                  60

Trp Ser His Leu Phe Ser Ser Val Leu His Thr Asn Pro Ser Lys Ser
65                  70                  75                  80

Ser Leu Ile Leu Thr Glu Pro Leu Phe Thr Ala Pro Ser Ile Gln Cys
                85                  90                  95

Ser Met Asp Glu Leu Ile Phe Lys Asp Phe Asn Phe Trp Ala Leu Tyr
            100                 105                 110

Leu Ala Asp Ser Ala Ser Val Val Tyr Leu Tyr Lys Ala Ser Arg Asn
        115                 120                 125

Asn Ala Asn Gly Ile Leu Ser Lys Ala Gln Gln Ser Leu Val Met Asp
    130                 135                 140

Leu Gly Phe Ser Phe Thr His Thr Ser Pro Val Phe His Asn Phe Ala
145                 150                 155                 160

Leu Asn Tyr Ala Ile Arg Arg Ile Asp Leu Ser Gly Lys Ala Leu Thr
                165                 170                 175

Asn Tyr Leu Lys Asp Leu Val Ser Phe Arg Ser Val Asn Ile Met Glu
            180                 185                 190

Glu Thr Phe Ile Ile Asn Asp Glu Phe Val Lys Tyr Pro Asp Gln Ala
        195                 200                 205

Gln His Tyr Leu Ala Leu Arg Glu Cys Gly Leu Pro Ser Ser Pro Ser
    210                 215                 220

Val Asp Ala Pro Gly Asp Val Lys Cys Leu Glu Ile Ala Lys Gln Pro
225                 230                 235                 240

Glu Asp Arg Lys Ile Val Asp Leu Thr Lys Asn Phe Leu Val Pro Lys
                245                 250                 255

Met Ile Phe Arg Pro Ala Asp Leu Gly Leu Ser Ile Gln Tyr Ile Cys
            260                 265                 270

Ile Phe Leu Asn Leu Lys His Ser Phe Leu Asn Val Val Cys Val Thr
        275                 280                 285

Leu Phe Pro Gln Phe Val Glu Arg Leu Glu Lys Glu Leu Arg Pro Leu
    290                 295                 300

Val Pro Asn Asp Tyr Arg Val Lys Ile Ala Thr Gln Glu Asp Pro Leu
305                 310                 315                 320

Leu Gly Val Trp Arg Gly Gly Ser Leu Leu Ala Ser Ser Pro Asp Phe
                325                 330                 335

Glu Ala Met Cys Val Thr Lys Ser Glu Tyr Glu Glu Leu Gly Ser Ala
            340                 345                 350

Arg Cys Arg Lys Arg Phe Phe His
        355                 360

<210>  113
<211>  56
<212>  DNA
<213>  Artificial sequence

<220>
<223>  primer 1

<400>  113
ggggacaagt ttgtacaaaa aagcaggctt aaacaatgtc aaacatcgtt gttcta          56


<210>  114
<211>  50
<212>  DNA
<213>  Artificial sequence

<220>
<223>  primer 2

<400>  114
ggggaccact ttgtacaaga aagctgggtt catgtgatat tgttttggtt                 50


<210>  115
<211>  2194
<212>  DNA
<213>  Oryza sativa

<400>  115
aatccgaaaa gtttctgcac cgttttcacc ccctaactaa caatataggg aacgtgtgct      60

aaatataaaa tgagacctta tatatgtagc gctgataact agaactatgc aagaaaaact     120

catccaccta ctttagtggc aatcgggcta aataaaaaag agtcgctaca ctagtttcgt     180

tttccttagt aattaagtgg gaaaatgaaa tcattattgc ttagaatata cgttcacatc     240

tctgtcatga agttaaatta ttcgaggtag ccataattgt catcaaactc ttcttgaata     300

aaaaaatctt tctagctgaa ctcaatgggt aaagagagag atttttttta aaaaaataga     360

atgaagatat tctgaacgta ttggcaaaga tttaaacata taattatata attttatagt     420

ttgtgcattc gtcatatcgc acatcattaa ggacatgtct tactccatcc caatttttat     480

ttagtaatta aagacaattg acttattttt attatttatc ttttttcgat tagatgcaag     540

gtacttacgc acacactttg tgctcatgtg catgtgtgag tgcacctcct caatacacgt     600

tcaactagca acacatctct aatatcactc gcctatttaa tacatttagg tagcaatatc     660

tgaattcaag cactccacca tcaccagacc acttttaata atatctaaaa tacaaaaaat     720

aattttacag aatagcatga aaagtatgaa acgaactatt taggtttttc acatacaaaa     780

aaaaaaagaa ttttgctcgt gcgcgagcgc caatctccca tattgggcac acaggcaaca     840

acagagtggc tgcccacaga acaacccaca aaaaacgatg atctaacgga ggacagcaag     900

tccgcaacaa ccttttaaca gcaggctttg cggccaggag agaggaggag aggcaaagaa     960

aaccaagcat cctccttctc ccatctataa attcctcccc ccttttcccc tctctatata 1020

ggaggcatcc aagccaagaa gagggagagc accaaggaca cgcgactagc agaagccgag 1080

cgaccgcctt ctcgatccat atcttccggt cgagttcttg gtcgatctct tccctcctcc 1140

acctcctcct cacagggtat gtgcctccct tcggttgttc ttggatttat tgttctaggt 1200

tgtgtagtac gggcgttgat gttaggaaag gggatctgta tctgtgatga ttcctgttct 1260

tggatttggg atagaggggt tcttgatgtt gcatgttatc ggttcggttt gattagtagt 1320

atggttttca atcgtctgga gagctctatg gaaatgaaat ggtttaggga tcggaatctt 1380

gcgattttgt gagtaccttt tgtttgaggt aaaatcagag caccggtgat tttgcttggt 1440

gtaataaagt acggttgttt ggtcctcgat tctggtagtg atgcttctcg atttgacgaa 1500

gctatccttt gtttattccc tattgaacaa aaataatcca actttgaaga cggtcccgtt 1560

gatgagattg aatgattgat tcttaagcct gtccaaaatt tcgcagctgg cttgtttaga 1620

tacagtagtc cccatcacga aattcatgga aacagttata atcctcagga acaggggatt 1680

ccctgttctt ccgatttgct ttagtcccag aatttttttt cccaaatatc ttaaaaagtc 1740

actttctggt tcagttcaat gaattgattg ctacaaataa tgcttttata gcgttatcct 1800

agctgtagtt cagttaatag gtaatacccc tatagtttag tcaggagaag aacttatccg 1860

atttctgatc tccattttta attatatgaa atgaactgta gcataagcag tattcatttg 1920

gattattttt tttattagct ctcacccctt cattattctg agctgaaagt ctggcatgaa 1980

ctgtcctcaa ttttgttttc aaattcacat cgattatcta tgcattatcc tcttgtatct 2040

acctgtagaa gtttcttttt ggttattcct tgactgcttg attacagaaa gaaatttatg 2100

aagctgtaat cgggatagtt atactgcttg ttcttatgat tcatttcctt tgtgcagttc 2160

ttggtgtagc ttgccacttt caccagcaaa gttc 2194

<210> 116
<211> 2241
<212> DNA
<213> Arabidopsis thaliana

<400> 116
atggcggata aggacgttcc ttttggggtt gaagatattg ttcagacgcc tttacctggt 60

tatgtagctc caactgcagt tagctttagt cctgatgatt ctttgatcac ttatttgttt 120

agccctgaaa agaatttgaa aaggagggtg tatgcttttg atgtcaataa aggggaatct 180

aatttggttt ttagtcctcc tgatggtgga gttgatgaga gtaacatttc acctgaagag 240

aagttgagga gggagaggtt gcgggagcgt ggtttaggtg taactcggta tgaatgggtc 300

aagactaact caaagatgag attcattgtg gttcctttac ctgccggggt gtatatgaag 360

gacctttctt catcaccaaa tccggagctc atagttccaa gttcacccac ttctccaatt 420

attgatcctc gtctatctcc taatggctta tttcttgcat acgtaagaga atccgagttg 480

catgtcctca atttgttaaa aaaccagaca caacagttaa ctagcggtgc caatggaagt 540

actttgagtc atggccttgc tgagtacata gctcaggagg agatggatcg gagaaatggg 600

tattggtggt cattagatag caagtttatc gcctatacag aagttgactc ctcacagatt 660

cctttgttca gaataatgca tcagggaaaa cgttcggttg gttcagaggc gcaagaagat 720

catgcttatc cttttgcagg agctctcaat tctacacttc gtctcgggat agtttcttca 780

gctggtggtg gaaagacgac ttggatgaat cttgtgtgcg gaggaagagg caatacagag 840

gacgagtatc ttggtagagt caactggctg cccgggaatg tcctcatagt gcaggttctg 900

aataggtcgc agagtaaact gaaaatcatc agctttgaca taaacactgg tcaaggaaac 960

gtactgttga cggaagaatc cgacacgtgg gtgactttac atgactgttt tactcctctg 1020

gagaaagtcc cttcatcgag aggttcagga ggattcattt gggccagtga gaggactggt 1080

tttagacatt tgtatcttta tgagtctgat gggacatgcc ttggagctat tactagcggt 1140

gaatggatgg ttgagcaaat agcaggtgta aatgagccga tgagtctggt gtatttcaca 1200

ggaacccttg atggaccact tgagactaat ctttactgcg caaaattgga agctgggaat 1260

acatctcggt gtcaacccat gagacttaca catgggaaag gaaaacatat cgttgtgctc 1320

gatcaccaga tgaagaactt cgttgacatt catgattcag ttgattcgcc tccgcgggtt 1380

tctctttgct ctctgagtga cggaactgta ctcaagattc cctacgagca gacatctcct 1440

atacagatac tgaaaagcct taaactagag cctccagagt ttgttcaaat acaagcaaat 1500

gacggaaaga caacactgta tggcgcggtt tacaaacccg acagttcgaa atttggtcct 1560

cctccatata aaacaatgat taacgtttat ggaggtccca gcgttcagtt ggtctatgat 1620

tcttggatta atacagtcga catgaggaca cagtatctga gaagcagagg catcttagtt 1680

tggaagcttg ataacagagg aactgcacgg cgtgggctga aatttgaatc ttggatgaag 1740

cataactgtg gatatgttga tgcagaagat caggtaactg gggccaaatg gctaatcgag 1800

caaggtctgg ctaaaccaga ccacattgga gtgtatggtt ggagttacgg tgggtacctt 1860

tcagctacac tcttgacccg ataccctgag atctttaact gcgctgtctc gggtgctcct 1920

gttacatctt gggatggcta tgactcgttc tacacggaga agtacatggg tcttcccacg 1980

gaggaggagc gctacctaaa gagctcggta atgcaccatg tcgggaactt gaccgataag 2040

cagaagctga tgttagtaca cggaatgatc gatgagaatg tgcatttcag acacacggct 2100

aggcttgtga acgcgcttgt tgaagcggga aagcggtacg agttgttgat atttcccgat 2160

gaacggcata tgccacggag gaagaaagac cggatatata tggaacagag gatttgggag 2220

tttatagaga agaacttatg a 2241

<210>  117
<211>  746
<212>  PRT
<213>  Arabidopsis thaliana

<400>  117

Met Ala Asp Lys Asp Val Pro Phe Gly Val Glu Asp Ile Val Gln Thr
1               5                   10                  15

Pro Leu Pro Gly Tyr Val Ala Pro Thr Ala Val Ser Phe Ser Pro Asp
            20                  25                  30

Asp Ser Leu Ile Thr Tyr Leu Phe Ser Pro Glu Lys Asn Leu Lys Arg
        35                  40                  45

Arg Val Tyr Ala Phe Asp Val Asn Lys Gly Glu Ser Asn Leu Val Phe
    50                  55                  60

Ser Pro Pro Asp Gly Gly Val Asp Glu Ser Asn Ile Ser Pro Glu Glu
65                  70                  75                  80

Lys Leu Arg Arg Glu Arg Leu Arg Glu Arg Gly Leu Gly Val Thr Arg
                85                  90                  95

Tyr Glu Trp Val Lys Thr Asn Ser Lys Met Arg Phe Ile Val Val Pro
            100                 105                 110

Leu Pro Ala Gly Val Tyr Met Lys Asp Leu Ser Ser Ser Pro Asn Pro
        115                 120                 125

Glu Leu Ile Val Pro Ser Ser Pro Thr Ser Pro Ile Ile Asp Pro Arg
    130                 135                 140

Leu Ser Pro Asn Gly Leu Phe Leu Ala Tyr Val Arg Glu Ser Glu Leu
145                 150                 155                 160

His Val Leu Asn Leu Leu Lys Asn Gln Thr Gln Gln Leu Thr Ser Gly
                165                 170                 175

Ala Asn Gly Ser Thr Leu Ser His Gly Leu Ala Glu Tyr Ile Ala Gln
            180                 185                 190

Glu Glu Met Asp Arg Arg Asn Gly Tyr Trp Trp Ser Leu Asp Ser Lys
        195                 200                 205

Phe Ile Ala Tyr Thr Glu Val Asp Ser Ser Gln Ile Pro Leu Phe Arg
    210                 215                 220

Ile Met His Gln Gly Lys Arg Ser Val Gly Ser Glu Ala Gln Glu Asp
225                 230                 235                 240

His Ala Tyr Pro Phe Ala Gly Ala Leu Asn Ser Thr Leu Arg Leu Gly
                245                 250                 255

Ile Val Ser Ser Ala Gly Gly Gly Lys Thr Thr Trp Met Asn Leu Val
            260                 265                 270

Cys Gly Gly Arg Gly Asn Thr Glu Asp Glu Tyr Leu Gly Arg Val Asn
        275                 280                 285

Trp Leu Pro Gly Asn Val Leu Ile Val Gln Val Leu Asn Arg Ser Gln
    290                 295                 300

Ser Lys Leu Lys Ile Ile Ser Phe Asp Ile Asn Thr Gly Gln Gly Asn
305                 310                 315                 320

Val Leu Leu Thr Glu Glu Ser Asp Thr Trp Val Thr Leu His Asp Cys
                325                 330                 335

Phe Thr Pro Leu Glu Lys Val Pro Ser Ser Arg Gly Ser Gly Gly Phe
            340                 345                 350

Ile Trp Ala Ser Glu Arg Thr Gly Phe Arg His Leu Tyr Leu Tyr Glu
        355                 360                 365

Ser Asp Gly Thr Cys Leu Gly Ala Ile Thr Ser Gly Glu Trp Met Val
    370                 375                 380

Glu Gln Ile Ala Gly Val Asn Glu Pro Met Ser Leu Val Tyr Phe Thr
385                 390                 395                 400

Gly Thr Leu Asp Gly Pro Leu Glu Thr Asn Leu Tyr Cys Ala Lys Leu
                405                 410                 415

Glu Ala Gly Asn Thr Ser Arg Cys Gln Pro Met Arg Leu Thr His Gly
            420                 425                 430

Lys Gly Lys His Ile Val Val Leu Asp His Gln Met Lys Asn Phe Val
        435                 440                 445

Asp Ile His Asp Ser Val Asp Ser Pro Pro Arg Val Ser Leu Cys Ser
    450                 455                 460

Leu Ser Asp Gly Thr Val Leu Lys Ile Pro Tyr Glu Gln Thr Ser Pro
465                 470                 475                 480

Ile Gln Ile Leu Lys Ser Leu Lys Leu Glu Pro Pro Glu Phe Val Gln
                485                 490                 495

Ile Gln Ala Asn Asp Gly Lys Thr Thr Leu Tyr Gly Ala Val Tyr Lys
            500                 505                 510

Pro Asp Ser Ser Lys Phe Gly Pro Pro Pro Tyr Lys Thr Met Ile Asn
        515                 520                 525

Val Tyr Gly Gly Pro Ser Val Gln Leu Val Tyr Asp Ser Trp Ile Asn
    530                 535                 540

Thr Val Asp Met Arg Thr Gln Tyr Leu Arg Ser Arg Gly Ile Leu Val
545                 550                 555                 560

Trp Lys Leu Asp Asn Arg Gly Thr Ala Arg Arg Gly Leu Lys Phe Glu
                565                 570                 575

Ser Trp Met Lys His Asn Cys Gly Tyr Val Asp Ala Glu Asp Gln Val
            580                 585                 590

Thr Gly Ala Lys Trp Leu Ile Glu Gln Gly Leu Ala Lys Pro Asp His
        595                 600                 605

Ile Gly Val Tyr Gly Trp Ser Tyr Gly Gly Tyr Leu Ser Ala Thr Leu
    610                 615                 620

Leu Thr Arg Tyr Pro Glu Ile Phe Asn Cys Ala Val Ser Gly Ala Pro
625                 630                 635                 640

Val Thr Ser Trp Asp Gly Tyr Asp Ser Phe Tyr Thr Glu Lys Tyr Met
                645                 650                 655

Gly Leu Pro Thr Glu Glu Glu Arg Tyr Leu Lys Ser Ser Val Met His
            660                 665                 670

His Val Gly Asn Leu Thr Asp Lys Gln Lys Leu Met Leu Val His Gly
        675                 680                 685

Met Ile Asp Glu Asn Val His Phe Arg His Thr Ala Arg Leu Val Asn
    690                 695                 700

Ala Leu Val Glu Ala Gly Lys Arg Tyr Glu Leu Leu Ile Phe Pro Asp
705                 710                 715                 720

Glu Arg His Met Pro Arg Arg Lys Lys Asp Arg Ile Tyr Met Glu Gln
                725                 730                 735

Arg Ile Trp Glu Phe Ile Glu Lys Asn Leu
            740                 745

<210>  118
<211>  7
<212>  PRT
<213>  Artificial sequence

<220>
<223>  motif 1

<220>
<221>  VARIANT
<222>  (1)..(1)
<223>  / replace = "Ser" / replace = "Leu"

<220>
<221>  VARIANT
<222>  (2)..(2)
<223>  / replace = "His"

<400>  118

Val Ser Leu Tyr Gly Gly Pro
1               5

<210>  119
<211>  23
<212>  PRT
<213>  Artificial sequence

<220>
<223>  motif 2

<220>
<221>  VARIANT
<222>  (1)..(1)
<223>  / replace = "Ala"

<220>
<221>  VARIANT
<222>  (2)..(2)
<223>  / replace = "Phe"

<220>
<221>  VARIANT
<222>  (3)..(3)
<223>  / replace = "Trp"

<220>
<221>  VARIANT
<222>  (4)..(4)
<223>  / replace = "Thr" / replace = "Ser"

<220>
<221>  VARIANT
<222>  (5)..(5)

<223>  / replace = "Asn"

<220>
<221>  VARIANT
<222>  (6)..(6)
<223>  / replace = "Gln" / replace = "Lys" / replace = "Ile"

<220>
<221>  VARIANT
<222>  (8)..(8)
<223>  / replace = "Trp" / replace = "Tyr"

<220>
<221>  VARIANT
<222>  (9)..(9)
<223>  / replace = "Ala" / replace = "Ser"

<220>
<221>  VARIANT
<222>  (10)..(10)
<223>  / replace = "Phe" / replace = "Leu"

<220>
<221>  VARIANT
<222>  (11)..(11)
<223>  / replace = "Val" / replace = "Ala" / replace = "Leu"

<220>
<221>  VARIANT
<222>  (12)..(12)
<223>  / replace = "Asp" / replace = "Ile"

<220>
<221>  VARIANT
<222>  (12)..(12)
<223>  / = replace = "Arg" / = replace = "Glu"

<220>
<221>  VARIANT
<222>  (13)..(13)
<223>  / replace = "Met" / replace = "Val" / replace = "Ile"

<220>
<221>  VARIANT
<222>  (14)..(14)
<223>  / replace = "Asn"

<220>
<221>  VARIANT
<222>  (15)..(15)
<223>  / replace = "Tyr"

<220>
<221>  VARIANT
<222>  (16)..(16)
<223>  / replace = "Gly"

<220>
<221>  VARIANT
<222>  (18)..(18)
<223>  / = replace = "Thr"

<220>

<221>  VARIANT
<222>  (19)..(19)
<223>  / = replace = "Ser" / = replace = "Thr" / = replace = "Leu"

<220>
<221>  VARIANT
<222>  (20)..(20)
<223>  / = replace = "Gly"

<220>
<221>  VARIANT
<222>  (21)..(21)
<223>  / = replace = "Tyr"

<400>  119

Gln Tyr Leu Arg Ser Arg Gly Ile Leu Val Trp Lys Leu Asp Asn Arg
1               5                   10                  15

Gly Ser Ala Arg Arg Gly Leu
            20

<210>  120
<211>  14
<212>  PRT
<213>  Artificial sequence

<220>
<223>  motif 3

<220>
<221>  VARIANT
<222>  (1)..(1)
<223>  / = replace = "His"

<220>
<221>  VARIANT
<222>  (2)..(2)
<223>  / = replace = "Leu"

<220>
<221>  VARIANT
<222>  (3)..(3)
<223>  / = replace = "Cys" / = replace = "Thr"

<220>
<221>  VARIANT
<222>  (4)..(4)
<223>  / = replace = "Leu" / = replace = "Val"

<220>
<221>  VARIANT
<222>  (5)..(5)
<223>  / = replace = "Cys" / = replace = "Ser" / = replace = "Thr" / =
      replace = "Leu"

<220>
<221>  VARIANT
<222>  (7)..(7)
<223>  / = replace = "Glu" / = replace = "Arg"

<220>
<221>  VARIANT
<222>  (9)..(9)
<223>  / = replace = "Ala" / = replace = "His"

<220>
<221>  VARIANT
<222>  (12)..(12)
<223>  / = replace = "Phe"

<220>
<221>  VARIANT
<222>  (13)..(13)
<223>  / = replace = "Leu" / = replace = "Thr"

<220>
<221>  VARIANT
<222>  (14)..(14)
<223>  / = replace = "Ser" / = replace = "Thr"

<400>  120

Arg Ile Gly Ile Tyr Gly Trp Ser Tyr Gly Gly Tyr Met Ala
1               5                   10


<210>  121
<211>  11
<212>  PRT
<213>  Artificial sequence

<220>
<223>  motif 4


<220>
<221>  VARIANT
<222>  (1)..(1)
<223>  / = replace = "Phe"

<220>
<221>  VARIANT
<222>  (2)..(2)
<223>  / = replace = "Glu"

<220>
<221>  VARIANT
<222>  (3)..(3)
<223>  / = replace = "Ser" / = replace = "Ala"

<220>
<221>  VARIANT
<222>  (4)..(4)
<223>  / = replace = "His" / = replace = "Phe" / = replace = "Arg"

<220>
<221>  VARIANT
<222>  (5)..(5)
<223>  / = replace = "Gly"

<220>
<221>  VARIANT

<222>  (6)..(6)
<223>  / = replace = "Ile" / = replace = "Asp"

<220>
<221>  VARIANT
<222>  (7)..(7)
<223>  / = replace = "Asp" / = replace = "Gln"

<220>
<221>  VARIANT
<222>  (8)..(8)
<223>  / = replace = "Asn" / = replace = "Ser"

<220>
<221>  VARIANT
<222>  (9)..(9)
<223>  / = replace = "Leu" / = replace = "His"

<220>
<221>  VARIANT
<222>  (10)..(10)
<223>  / = replace = "Vzl" / = replace = "Tyr"

<220>
<221>  VARIANT
<222>  (11)..(11)
<223>  / = replace = "Thr"

<400>  121

Tyr Asp Thr Tyr Tyr Thr Glu Lys Tyr Met Gly
1               5                   10


<210>  122
<211>  6
<212>  PRT
<213>  Artificial sequence

<220>
<223>  motif 5



<220>
<221>  VARIANT
<222>  (2)..(2)
<223>  / = replace = "Ile" / = replace = "Pro"

<220>
<221>  VARIANT
<222>  (3)..(3)
<223>  / = replace = "Ile"

<220>
<221>  VARIANT
<222>  (4)..(4)
<223>  / = replace = "Asn" / = replace = "Ser"

<220>
<221>  VARIANT
<222>  (5)..(5)
<223>  / = replace = "Phe"

<220>
<221>  VARIANT
<222>  (6)..(6)
<223>  / = replace = "Ile"

<400>  122

Ser Val Met His His Val
1               5


<210>  123
<211>  10
<212>  PRT
<213>  Artificial sequence

<220>
<223>  motif 6


<220>
<221>  VARIANT
<222>  (1)..(1)
<223>  / = replace = "Gln" / = replace = "Leu"

<220>
<221>  VARIANT
<222>  (3)..(3)
<223>  / = replace = "Leu" / = replace = "Thr"

<220>
<221>  VARIANT
<222>  (4)..(4)
<223>  / = replace = "Glu" / = replace = "Lys"

<220>
<221>  VARIANT
<222>  (6)..(6)
<223>  / = replace = "Lys" / = replace = "Leu"

<220>
<221>  VARIANT
<222>  (7)..(7)
<223>  / = replace = "Val" / = replace = "Arg"

<220>
<221>  VARIANT
<222>  (9)..(9)
<223>  / = replace = "Thr" / = replace = "Pro"

<220>
<221>  VARIANT
<222>  (10)..(10)
<223>  / = replace = "Pro" / = replace = "Ile"

<400>  123

His Gly Met Ile Asp Glu Asn Val His Phe
1               5                   10


<210>  124
<211>  10

<212>  PRT
<213>  Artificial sequence

<220>
<223>  motif 7


<220>
<221>  VARIANT
<222>  (1)..(1)
<223>  / = replace = "Tyr"

<220>
<221>  VARIANT
<222>  (2)..(2)
<223>  / = replace = "Glu"

<220>
<221>  VARIANT
<222>  (3)..(3)
<223>  / = replace = "Gly" / = replace = "Asn"

<220>
<221>  VARIANT
<222>  (4)..(4)
<223>  / = replace = "Asp"

<220>
<221>  VARIANT
<222>  (5)..(5)
<223>  / = replace = "Gln" / = replace = "Asn"

<220>
<221>  VARIANT
<222>  (7)..(7)
<223>  / = replace = "Gly" / = replace = "Pro"

<220>
<221>  VARIANT
<222>  (8)..(8)
<223>  / = replace = "Phe" / = replace = "Leu"

<220>
<221>  VARIANT
<222>  (9)..(9)
<223>  / = replace = "Asp"

<220>
<221>  VARIANT
<222>  (10)..(10)
<223>  / = replace = "Arg" / = replace = "Lys"

<400>  124

Phe Pro Asp Glu Arg His Met Pro Arg Gly
1               5                   10


<210>  125
<211>  19
<212>  PRT
<213>  Artificial sequence

<220>
<223>  motif 8

<220>
<221>  VARIANT
<222>  (9)..(9)
<223>  / = replace = "Glu"

<220>
<221>  VARIANT
<222>  (16)..(16)
<223>  / = replace = "Arg"

<400>  125

Lys Leu Arg Arg Glu Arg Leu Arg Gln Arg Gly Leu Gly Val Thr Cys
1               5                   10                  15

Tyr Glu Trp

<210>  126
<211>  24
<212>  PRT
<213>  Artificial sequence

<220>
<223>  motif 9

<220>
<221>  VARIANT
<222>  (3)..(3)
<223>  / = replace = "Ile"

<220>
<221>  VARIANT
<222>  (13)..(13)
<223>  / = replace = "Glu"

<220>
<221>  VARIANT
<222>  (15)..(15)
<223>  / = replace = "Arg"

<220>
<221>  VARIANT
<222>  (16)..(16)
<223>  / = replace = "Thr" / = replace = "Met"

<220>
<221>  VARIANT
<222>  (18)..(18)
<223>  / = replace = "Phe"

<220>
<221>  VARIANT
<222>  (22)..(22)
<223>  / = replace = "Pro"

<400>  126

His Gly Leu Ala Glu Tyr Ile Ala Gln Glu Glu Met Asp Arg Lys Asn
1               5                   10                  15

Gly Tyr Trp Trp Ser Leu Asp Ser
            20

<210>  127
<211>  14
<212>  PRT
<213>  Artificial sequence

<220>
<223>  motif 10

<220>
<221>  VARIANT
<222>  (8)..(8)
<223>  / = replace = "Arg"

<220>
<221>  VARIANT
<222>  (9)..(9)
<223>  / = replace = "Thr"

<400>  127

Gly Phe Ile Trp Ala Ser Glu Lys Ser Gly Phe Arg His Leu
1               5                   10

<210>  128
<211>  12
<212>  PRT
<213>  Artificial sequence

<220>
<223>  motif 11

<220>
<221>  VARIANT
<222>  (3)..(3)
<223>  / = replace = "Asn"

<220>
<221>  VARIANT
<222>  (4)..(4)
<223>  / = replace = "Lys" / = replace = "Arg"

<220>
<221>  VARIANT
<222>  (11)..(11)
<223>  / = replace = "Met"

<400>  128

Leu Arg Ser Gln Gly Ile Leu Val Trp Lys Leu Asp
1               5                   10

<210>  129
<211>  11
<212>  PRT
<213>  Artificial sequence

<220>
<223>  motif 12


<220>
<221>  VARIANT
<222>  (3)..(3)
<223>  / = replace = "Val" / = replace = "Ile"

<220>
<221>  VARIANT
<222>  (4)..(4)
<223>  / = replace = "Tyr"

<220>
<221>  VARIANT
<222>  (11)..(11)
<223>  / = replace = "Phe"

<400>  129

Ile Gly Leu Cys Gly Trp Ser Tyr Gly Gly Tyr
1               5                   10


<210>  130
<211>  15
<212>  PRT
<213>  Artificial sequence

<220>
<223>  motif 11


<220>
<221>  VARIANT
<222>  (4)..(4)
<223>  / = replace = "Ala"

<220>
<221>  VARIANT
<222>  (10)..(10)
<223>  / = replace = "Ala"

<400>  130

Cys Ala Val Ser Gly Ala Pro Val Thr Ser Trp Asp Gly Tyr Asp
1               5                   10                  15


<210>  131
<211>  16
<212>  PRT
<213>  Artificial sequence

<220>

<223>  motif 12

<400>  131

His Gly Met Ile Asp Glu Asn Val His Phe Arg His Thr Ala Arg Leu
1               5                   10                  15


<210>  132
<211>  53
<212>  DNA
<213>  Artificial sequence

<220>
<223>  primer: prm05611

<400>  132
ggggacaagt ttgtacaaaa aagcaggctt aaacaatggc ggataaggac gtt          53


<210>  133
<211>  50
<212>  DNA
<213>  Artificial sequence

<220>
<223>  primer: prm05612

<400>  133
ggggaccact ttgtacaaga aagctgggta agcaacaaca ggttctgtga              50


<210>  134
<211>  2194
<212>  DNA
<213>  Oryza sativa

<400>  134
aatccgaaaa gtttctgcac cgttttcacc ccctaactaa caatataggg aacgtgtgct      60

aaatataaaa tgagacctta tatatgtagc gctgataact agaactatgc aagaaaaact     120

catccaccta ctttagtggc aatcgggcta aataaaaaag agtcgctaca ctagtttcgt     180

tttccttagt aattaagtgg gaaaatgaaa tcattattgc ttagaatata cgttcacatc     240

tctgtcatga agttaaatta ttcgaggtag ccataattgt catcaaactc ttcttgaata     300

aaaaaatctt tctagctgaa ctcaatgggt aaagagagag atttttttta aaaaaataga     360

atgaagatat tctgaacgta ttggcaaaga tttaaacata taattatata attttatagt     420

ttgtgcattc gtcatatcgc acatcattaa ggacatgtct tactccatcc caatttttat     480

ttagtaatta aagacaattg acttattttt attatttatc ttttttcgat tagatgcaag     540

gtacttacgc acacactttg tgctcatgtg catgtgtgag tgcacctcct caatacacgt     600

tcaactagca acacatctct aatatcactc gcctatttaa tacatttagg tagcaatatc     660

tgaattcaag cactccacca tcaccagacc acttttaata atatctaaaa tacaaaaaat     720

aattttacag aatagcatga aaagtatgaa acgaactatt taggtttttc acatacaaaa     780

aaaaaaagaa ttttgctcgt gcgcgagcgc caatctccca tattgggcac acaggcaaca 840

acagagtggc tgcccacaga acaacccaca aaaaacgatg atctaacgga ggacagcaag 900

tccgcaacaa ccttttaaca gcaggctttg cggccaggag agaggaggag aggcaaagaa 960

aaccaagcat cctccttctc ccatctataa attcctcccc ccttttcccc tctctatata 1020

ggaggcatcc aagccaagaa gagggagagc accaaggaca cgcgactagc agaagccgag 1080

cgaccgcctt ctcgatccat atcttccggt cgagttcttg gtcgatctct tccctcctcc 1140

acctcctcct cacagggtat gtgcctccct tcggttgttc ttggatttat tgttctaggt 1200

tgtgtagtac gggcgttgat gttaggaaag gggatctgta tctgtgatga ttcctgttct 1260

tggatttggg atagaggggt tcttgatgtt gcatgttatc ggttcggttt gattagtagt 1320

atggttttca atcgtctgga gagctctatg gaaatgaaat ggtttaggga tcggaatctt 1380

gcgattttgt gagtaccttt tgtttgaggt aaaatcagag caccggtgat tttgcttggt 1440

gtaataaagt acggttgttt ggtcctcgat tctggtagtg atgcttctcg atttgacgaa 1500

gctatccttt gtttattccc tattgaacaa aaataatcca actttgaaga cggtcccgtt 1560

gatgagattg aatgattgat tcttaagcct gtccaaaatt tcgcagctgg cttgtttaga 1620

tacagtagtc cccatcacga aattcatgga aacagttata atcctcagga acaggggatt 1680

ccctgttctt ccgatttgct ttagtcccag aatttttttt cccaaatatc ttaaaaagtc 1740

actttctggt tcagttcaat gaattgattg ctacaaataa tgcttttata gcgttatcct 1800

agctgtagtt cagttaatag gtaatacccc tatagtttag tcaggagaag aacttatccg 1860

atttctgatc tccattttta attatatgaa atgaactgta gcataagcag tattcatttg 1920

gattattttt tttattagct ctcacccctt cattattctg agctgaaagt ctggcatgaa 1980

ctgtcctcaa ttttgttttc aaattcacat cgattatcta tgcattatcc tcttgtatct 2040

acctgtagaa gtttcttttt ggttattcct tgactgcttg attacagaaa gaaatttatg 2100

aagctgtaat cgggatagtt atactgcttg ttcttatgat tcatttcctt tgtgcagttc 2160

ttggtgtagc ttgccacttt caccagcaaa gttc 2194

<210> 135
<211> 2313
<212> DNA
<213> Medicago truncatula

<400> 135
atgagaagag gaatccgaag cgtcaaaaac cacttccttt caaaatgcct gcctgtgaca 60

gacttcaatg atgtgcaaaa tcttgatgac ggcattcttt tcccggttga agagattgca 120

caatatccat taccgggata tgtatcacca acttcaataa gttttagtcc agatgatagt 180

ttgatttctt atttgtttag tcctgataat acattaaaca gaaagatttt cacttttgat 240

ctgaagacca ataaacaaga attgttattt agcccgcctg atggcggact tgatgagagt 300

aatatttctc cggaagaaaa gttgaggagg gagaggttga gggaacgcgg tttaggagtg 360

acgcggtatg agtgggtgaa gacgaactca aaaaggaaag cagtcctggt gccgttacct 420

gctgggattt atgtccatga tatttcccat tcgaaaacag agctcaagct tcctagtata 480

ccagcttcgc ccattattga tcctcatctg tctccagatg gatctatgct tgcttatgta 540

agagactgtg agttgcatgt tatgaatctc ttatctgatg aatcaaagca gttgacccat 600

ggtgcgaagg aaaatggctt tactcacggg cttgccgaat atatagcaca ggaggagatg 660

gataggaaaa ctggctactg gtggtcactg gacagtaaat atattgcttt tactgaagtt 720

gattattctg aaataccgct tttcagaatt atgcaccaag gtagaagctc agtcggcaca 780

gatgcacagg aagaccatcc ttatcctttt gcaggagctt caaatgctaa aatacgccta 840

ggggttgttt cggtagctgg aggctccacc acttggatgg atcttgtctg tgggggcgtg 900

aaagaactag acaacgagga tgaatatttg gcaagagtta attggatgca tggaaacatt 960

ctcactgctc agatcataaa taggcaccag actaaaataa agatcgttaa gtttgatatt 1020

agaacaggac aaagaagaga tatattggta gaagagaaca aaacttggat caacatacat 1080

gactgtttca caccttttga taaaggagtt accaagtttt caggtggatt tatctgggct 1140

agtgagaaat caggatttag acatctctat cttcatgatg cgaatgggat ttgtttagga 1200

cccatcactg aaggtgaatg gatggttgag caaatcgccg gtgtgaatga ggcaacaggt 1260

ctagtatatt tcaccgggac cttagatagt cctcttgaat ccaacttata ctgtgctaaa 1320

cttttcgtag atggaactca accacttcaa gcccctacca gactaacaca tagcaaggga 1380

aagcacattg tagtccttga tcatcacatg cgaacatttg ttgatataca cgactccctt 1440

agttgtccac ctagggtatt actatgctcg ttagaggatg gaaccataat catgcctttg 1500

tatgagcagc aaataccaat tccaaaatcc aaaaagcttc agcttgagcc tccagagatt 1560

gttgaaatac agtccgacga tggtactacc ttgtatggag ctctttacaa gcctgaccct 1620

tcaagatttg gacctccacc ttacaaaacc atgattaatg tttatggtgg tccaagtgta 1680

cagcttgttt ctaactcttg gcttaataca gtagacctga gagcgcaata cttgagaaat 1740

aaaggcatct tagtttggaa gttagacaac agaggaactt ctagacgggg gttgaagttt 1800

gaaggctatt taaagcagaa acttggacaa attgatgctg atgatcaatt tactggagca 1860

gagtggcttg taaaaaatgg gcttgcagaa tttggccaca ttgggttgta cggttggagc 1920

tatggcgggt atctgtctgc tatgaccctt tcaagatatc cagatttctt caagtgtgct 1980

gtagctggtg cacccgtcac atcgtgggat ggatacgaca cattctacac agagaaatac 2040

atgggattgc catctgaata taaatcaggg tatgcaagag cttctgtcat gaaccatgtg 2100

cacaagatga ggggaaggtt gttgattgtg catggaatga ttgatgagaa tgtacatttt 2160

cggcacactg caaggcttat caatgcactt gtagcggctg gaaaaacgta tgagctgata 2220

attttcccgg atgagcgcca catgcctcgg cgatatagtg atcgagttta tatggaagag 2280

aggatgtggg aattcattga caggaatctg tga 2313

<210> 136
<211> 1791
<212> DNA
<213> Oryza sativa

<400> 136
atgattgcgt atgtgcggga tgatgagttg catacagtgg ggttctctga tgggcaaact 60

acccagttga cctatggtgc aagcgaaagt gggaagatcc acggacttgc tgagtatatt 120

gcacaggaag agatggaaag gaagatggga ttctggtggt ctcctgatag caaacacctt 180

gcatttactg aagttgactc atctgaaatc ccactgtata gaattatgca ccagggtaaa 240

agttcggttg gtccagatgc tcaagaagat catgcttacc cctttgcagg ggctgctaat 300

gtcaaagtgc gccttggagt tgtttcttcc catggaggag aaataacttg gatggatctc 360

ctatgtggag aaccaaatag tatccatggt gatgaagaat atcttgctag agtaaactgg 420

atgcataata gtgctattgc tgttcaagtt ctcaatagaa ctcactcaaa acttaagcta 480

cttaagtttg acatcgcttc aggtaaaagg gaggtcatac tagaggaaga gcatgatacg 540

tggataacat tgcacgattg ttttactcct ctggataaag gagtgaatag taaacatcca 600

ggtggattta tttgggccag tgagaagaca ggatttagac acctgtatct tcatgacaag 660

aatggtgtgt gcttggggcc cctcacacaa ggtgattgga tgatcgacca aattgctggt 720

gtcaatgaga gttctggagt tatatatttt acaggcacat tggatgggcc attggagaca 780

aatctttact ccaccaacct atttccggat tggagccttc ccttgcaagt ccccaagagg 840

ttgactcatg gcacaggacg tcattcagta attcttgacc atcagttgct gaggtttatt 900

gatgtgtatg actcaataaa atctccacct gtgatcctgc tatgctcttt gcttgatgga 960

agtgtgataa tgcctctata tgagcagcct ctgactgttc agccgcttaa aaagtttcag 1020

cagctgtctc cagagatagt acagattgaa ggaaaggatg ggactgcttt atatggaact 1080

ctttatctcc ctgatgagaa aaaatatgga ccacccccat acaaaacact cgttaacgtt 1140

tatggcggtc ccagtgtcca gcttgtaagt gactcatgga taagtacagt tgacatgaga 1200

gctcagttcc tgcgtagtaa gggtatatta gtttggaaga tggacaatcg aggaaccgca 1260

agacgcggtt tgcagtttga gggacagctc aaatacaaca tcggtcgtgt cgatgctgaa 1320

gatcagttag caggtgccga gtggctaata aagaaaggcc tagcaaaacc cggacatata 1380

ggtctctacg gctggagcta tggtggcttt ctctcggcaa tgtgcctcgc gagatttccc 1440

gacacgttca gttgtgcagt ttctggcgct ccggtgacag catgggatgg gtatgacact 1500

ttctataccg agaagtacat gggtctgccc tcggagcagc gcgacgctta cagatacggg 1560

tcgatcatgc atcatgtgaa gaacctccga gggaggctgc tgctgatcca cggtatgata 1620

gacgagaacg tgcatttcag gcacacggcg aggctcatca actcgctgat ggcggagggg 1680

aagccctacg atatcctcct cttccccgac gagaggcaca tgccgcgccg gttaggggac 1740

cggatctata tggaagagag gatctgggat tttgtagaga ggaacctgtg a 1791

<210> 137
<211> 2371
<212> DNA
<213> Physcomitrella patens

<400> 137
atgcccacca ctgacagcca aagccttgga gatgaataca cgcttttccc agttgaggac 60

attgttcaaa accctcttcc aggatatgtt gcgcctggat cagtgcagtt tagtcctgat 120

gataagctca tcacttattt gtacagtcct gacagcactc tcagcagaaa aatatacgct 180

tttgatgtgg tcgcgaaaca gcagcggttg ctggtcaatc ctcccggtgg aggagtggac 240

gagggcaatc tctccacagt tgagaagctg aggagggaga ggctgcgaga gagaggactg 300

ggagtgacac gctatgagtg ggccaagggt gttcctcacc ggctcatggt tccccttcct 360

agtgggattt atgtgcaagg agtgggatct gaattacgac tgcgagtggc tagcacgcct 420

tccaatccga tacttgaccc tcaactttcg ccagacgcgt cctctatagc ttatgtgcgt 480

gatgatgaac tatttgttgt tccactagca tttggggagc ccatccaaat tacgtatggc 540

gcacgtggaa ctggcaagac tcatgggctc gccgagtaca tagcgcagga agaaatggat 600

agacgtaatg gcttctggtg gtcgcctgat agtcgctaca ttgcttttgc ggaagtagat 660

tcatccagga tcccaccctt tacgattgtg caccagggga aggtgacaac gggtggggag 720

gctgaagaag tccacgccta cccttttgcg ggccactcaa acgtgagtgt gcgattaggc 780

gtggtgccat caaatgggga tagtgtagtg tggatggatt tggaggttgg cctttgtggt 840

gaaggtcatg aggatgaaga gtatttagca agagtcacgt ggttgcccaa tggttatctt 900

acagctcaag tgcaaaatcg aactcaatca aaattaaagc tgctgagatt tgatccactg 960

acaggcaaga ggcttgtgtt actcaccgag gagagcgaag tttgggttaa cttgcacgat 1020

agttttactc ctctacgaaa ataccatgga cccctttcag gtgggtttat atgggcaagc 1080

gaaaagacag gatttcggca cttgtatatg catgatgagt ttggcaattg cattagggct 1140

ctcacacagg ggagctggat ggtagagcaa gtggcaggag tggacgaaga cgccggcttg 1200

gtctacttta caggagcatt tgatagccct ttagaagtgc atttgtatag cgctagtctg 1260

gattgtacgg aaccgaacct ggcagagccc aagcggctga ctcaaggggc aggtcggcac 1320

attgtggttc tagatcacga gatgaagaag tttgttgata tacacgactc tttagacact 1380

ccacccaggc tgttgctgcg gtctttagat actggaaagc tgctcatctc aatatatgag 1440

caaccagccc ccacttacca cgcgcggagg ttgcaactca cccctcccga gttcaaatct 1500

ctcactgcaa gcgacggaac tgtcctacat ggactaattt acattccaga ttctaaacaa 1560

tttggacgcc ccccttatag aacggtggtg agtgtttatg gtggccctaa tgtgcaaata 1620

gtgtgcaatt cctggatgaa tacggtggat atgcgtgctc aatatctgag gagtagaggc 1680

atccttgttt ggaagctgga taacagaggt agtgcgagaa gaggtttgga ttttgaaggt 1740

gcaatcaagc acaacatggg gatgatcgac gttgacgatc agcaaacagg agtgcagtgg 1800

ctagtaaatc aaggtcttgc aatgccgaac cgaatcggca tatatggatg gagctatgga 1860

ggttacatgg cttcaatggc actagcacga tgcccagaaa ctttttcttg cgcagtggct 1920

ggggcaccag tcacgtcatg ggatggctac gatacgtact atactgagaa gtacatggga 1980

accccggcca gcaacccagc aggataccaa tacagctctg tgatgcacca cgtttatcag 2040

ataactggga aactgttgct ggtccatggc atgatcgatg agaatgtaca ttttcggcat 2100

acggctcggc tgatcaacac actgaccgct gcctccaagg attatgaact gctggtgttc 2160

ccggacgaga gacacatgcc tcgggggcta agagaccgga tgtatatgga ggagcggatt 2220

tgtgattttt tagagagaaa tatttgactg gtaaaatttt tatgtatttg aaacttttag 2280

gttattattc atctaaagtt ccacacagaa tgaactagtc tcggaaagcc ctgctagatt 2340

agtatagtag gttcactgta taataactct a 2371

<210> 138
<211> 2486
<212> DNA
<213> Physcomitrella patens

<400> 138
atgcccacca ctgacagcca agctcttgga gatgaataca cgctcttccc agtcgaggac 60

attgttcagt accctcttcc aggatatgta gcgccggctt cagtgcagtt cagtcccgat 120

gataagctaa tcacatattt gtacagtccc gacagcactc tcagcaggaa aatctacgct 180

tttgatgtgg ctgccaggca acagcggttg ctggtcagtc cacctggtgg cggagtggac 240

gagggtaacc tttccacagt tgagaagctg aggagggaga ggttgcgaga aaggggattg 300

ggagtgacac gttatgagtg gggcaagggt gttcctcacc gtctgatggt tccccttcct 360

agtgggattt atgttcaaga aggagtacga gctgaactac aacggcgagt ggctagcacc 420

tcttggtctc ctattctgga tccccagctt tcaccagatg catgttctat agcttacgtt 480

cgtgatgatg agatatttgt tgttccagta gcattcgggg atcccattca aattacatct 540

ggtgcacgtg gaactggcaa gactcatgga cttgccgagt atatagcgca ggaagaaatg 600

gatagacgaa atggtttctg gtggtcgcct gatagtcgct acattgcatt tgcggaagta 660
gattcaacca ggattccacc ttttaggatc atgcaccagg ggaaggcgtc aactggtggg 720
gaggctgaag aaattcacgc ttatcctttt gcaggccact caaacgtgag tgtgcgatta 780
ggtgtggttc catcaaatgg cggtagtgta gtctggatgg atttggaggt tggtttgtgc 840
ggtgaaggtc gagatgatga ggagtatcta gcacgagtca cgtggttgcc caatggtagt 900
ctcactgcgc aagtacagaa tcggactcag acaaaactta agctattgaa atttgatcca 960
ctcactggca agaggactgt gttgttcact gaggagagcg atgtttgggt taacttgcac 1020
gattgtttca cttctctacg aaaatgtcat ggacttcttt caggggggtt catctgggca 1080
agtgaaaaaa caggatttcg gcatttgtat gtgcacgatg aatttggcaa ttgcattagg 1140
gctctcacgc aagggagctg gatggtagag caagttgcag gagtggacga agaggccggc 1200
ctggtgtact ttacaggaac acatgatagt cctttagaaa tgcatctgta cagcgtcagt 1260
ctggactgca cgcaaccaaa cctcgtagag cccaagaggc tgacgcaagg ggcaggtcgg 1320
catattgtcg tattagatca ccagatgaag atgtttgttg atatacacga ctctttagat 1380
acaccaccca ggctgttgct gcgttccttg gacaccggaa agctactcgt taccatttat 1440
gagcaaccag cccctacgta ccacacacgt aggttgcaat taacacctcc ggagttcaaa 1500
tcactcactg ctagcgatgg gactgtgctg catgggctag tttacattcc agattctaaa 1560
cagttcgggc gcccacctta tagaacagtg gtgagtgttt atggtggccc taatgtgcaa 1620
gtagtgtgca attcctggat gaacacggtg gatatgcgtg cccagtattt gaggagtcga 1680
ggcattcttg tttggaagct ggacaacaga ggtagtgcaa gaagaggtct ggattttgaa 1740
ggtgcgatca agcacaacat gggaaagatc gacgtagagg atcagcaaac aggagtgcag 1800
tggctggtaa gccaaggcct tgcaatgtcg aaccgaattg gaatatatgg atggagctat 1860
ggcggttaca tggcttcaat ggccctagca cgatgtccac aaactttttc ctgtgctgtt 1920
gctggggcgc ctgtcacctc ttgggatggc tacgacaccc actacacgga gaaatacatg 1980
ggaaccccgg ccagcaaccc agcagggtac caatacagct ctgtcatgca ccacgtttac 2040
cagattactg gaaaactgtt gctggtgcat ggaatgatcg atgagaatgt acattttcgg 2100
cacacggctc ggcttatcaa ttcactgacc gctgcatcca aggattatga gctgttggtg 2160
ttcccagacg agagacacat gccacggggg ctcagagacc ggatgtacat ggaggagcgg 2220
atttgtgatt ttctagaaag aaatatatga gtggttaaat ttgtatgtag ttgaaactta 2280
aactcttagg ttcttgctca tctaaaggtt cacactgaat gaatgagtct cggaaatccc 2340
cgcgagtagg ttcactgtat aataacttta taatactgtg attccatccc tgtaaagtgg 2400
gaaaacgtat gatgtcggct gcatttttac tctgaaatgt ggaggattag acatggtgta 2460
acttttgggc ttacccatcg aaagca 2486

<210>  139
<211>  2432
<212>  DNA
<213>  Populus trichocarpa

<400>  139
tttagtttat agaattttag ttcatatgtg atgcaatcag ttgatgagaa cgagagccag      60

aacaagaaat taaggatatt gagatcatta aataacgata tgcctctgac tgataacacc     120

atcccacaaa atgttgagga cagtattctt tttcctattg aagagatagt gcaatccccg     180

ttgcctggat atgaggcgcc aacttcgata ggtttcagtg ctgatgatag tttacttact     240

tacttattta gtcctgatca caccttgagt aggaaggttt ttgcttttga tctcaagagt     300

ggcaagcaag aattgttttt tggcccccct gatggtggac ttgatgagag taatatatca     360

ccggaagaga agttgcgaag ggagaggtta aggcaacgtg ggctgggagt gacatgttat     420

gaatgggtga agacaggttc gaagaagaaa gcaattatgg tgccactgcc tgcaggggaa     480

ctgcactctt caaaaccaga gctcaagctt cccagctctg cattgtcccc tgttatagat     540

ccacatgtct ctcctgatgg taccatgctt gcttatataa gggacagtga gctgcatgtt     600

ctaaatttat tgtacaacga gtccaaacaa ttaacacatg gtgctcaggg aaatacagtg     660

actcatggcc ttgctgaata tatagctcag gaggagatgg accggaagaa tggttactgg     720

tggtcacttg acagcaaatt cattgcattt acacaagttg attcatctga gatacctctt     780

tttagaatta tgcaccaagg caaaagctct gtaggttcag aagcacagga agatcatcct     840

tatccctttg ctggagcttc aaatgtcaaa gttcgccttg gggtagtttc tgttcatggt     900

gattctataa cttggatgga tcttctatgt ggaggaacaa aagaaccaga taatgaggat     960

gaatatttgg cccgagtcaa ttggatgcat ggaaatgttc tcatagctca agttttgaac    1020

aggtctcatt caaaattaaa acttcttaag tttgatatta agacggggaa aaaagaggtg    1080

ttatatgcag aagaacaact cccatggatt aatttacatg actgcttcac tcctttggac    1140

aaaggaatca ctaaatattc tggaggattc atttgggcga gtgaaaagtc aggatttagg    1200

catttgtgtg tgcatgatgc caatggggca tgcttaggac caattactga aggtgaatgg    1260

atggttgagc aaattgccgg tgtaaatgag gctgcaggga tcatatattt tactgcaact    1320

ctagatgggc ctttggaatc acatctttac cgtgctaaac tgtatccaat tgaaaacaat    1380

cccttgcagg ctccggtgag attaacaaat ggtaaaggga aacactcggt tgtccttgat    1440

caccacttgc agaattttgt tgatatccat gattcccttg attctccccc aagagtttct    1500

ctctgctccc tgtttgatgg aagagaaatt atgcctctgt ttgagcagtc ttttaccatt    1560

ccaagatata aaaggctgga acttgagcct ccaaagatag ttcagataca agcaaatgac    1620

gggaccatat tgtatggggc tttatatgac cctgacccta caagatttgg accaccacca    1680

tacaaaaccg tgatcagtgt gtatggaggc cccggtgtac agtatgtatg tgattcttgg 1740

ataggtacag ctgacatgag agctcaatat cttcggagcc aaggcatctt agtgtggaag 1800

ttggataata gaggaagtgc tcgccgtggg ctaaagtttg aaggtgctct gaaaggaaat 1860

cctggccgct ttgatgcaga ggatcagctt acaggagcag aatggctcat taaacaagga 1920

ttggcaaaag ctggccacat tgggttgtgt ggatggagtt atggtggata tatgtcagct 1980

gtgatcttgg caaggttccc tgatgtattc tgttgtgctg tctctggtgc acctgtaacc 2040

tcctgggatg gatatgacac attttacact gagaagtaca tgggattgcc ttctgataat 2100

ccaaagggct acgagtacgg ctctgtgatg catcatgtgc acaagttaaa agggaggttg 2160

ttgctggtgc atggcatgat tgatgaaaat gtgcatttta gacacactgc aaggcttgtc 2220

aatgcactcg tggcagctgg aaaaccctat gaactattaa ttttccctga tgaacgacac 2280

atgccccgtc ggcataatga ccgaatttac atggaagaga gaatttggga gttcttccag 2340

agaagtttat gaagtaattt ttatttatag attcatgtcg ctgtagaaaa aattctgcat 2400

ctttttgctt cgatatattt tttctttttt ca 2432

<210> 140
<211> 2432
<212> DNA
<213> Populus trichocarpa

<400> 140
tttagtagat agaagtgtag ttcatttgta atgcaatcag ttgatgagaa cgagagcgag 60

aacaagaaat tgaagcgttt gagatcatta agtaacaata tgcctttgac tgacaacacc 120

accccacaaa atgttgagga cagcattctt tttcctattg aagagatagt gcaatcaccg 180

ttgcctggat atgtggcgcc gacttcgata ggttttagtg ctgatgatag tttagttact 240

tgcttattta gtcctgatca caccttgagt aggaaggttt ttgcttttga tctcaagaat 300

ggcaagcaag aattgttttt tggtccccct gatggcggac tcgatgagag taatatatca 360

gcggaagaga agctgcggag ggagaggttg agagaacgtg ggctgggagt gacacggtat 420

gaatgggtga agacaggctt gaagaagaaa gcaattatgg tgccattgcc tgcaggggaa 480

ctctactctc ccaaacccga gctcaagctt cctagctcct cattatcacc tattatcgat 540

ccgcatatct ctcctgacgg taccatgctt gcttatgtac gggacagtga gctgcatgtt 600

ctaaatttct tgttcaatga gtccaaacaa ttaacacatg gtgctcaggg aaatacagtg 660

actcatggca ttgctgaata tatagctcag gaggaaatgg accggaagaa tggttactgg 720

tggtcacttg acagccaatt tattgcattt acacaagttg attcatctga gatacctctt 780

tttagaatta tgcaccaagg caaaagctct gttggttcag aagcacagga agaccatcct 840

tatccctttg caggagcttc aaatgtcaaa gttcacctcg gggtagtttc tgttcatggt 900

ggttctgtaa cttggttgga tcttctctgt ggaggaacag aaaaaccaga taacgaggat 960

gaatatttgg ccagaatcaa ttggatgcat ggaaatattc tcatagctca agttttgaac 1020

aggtctcatt caaaattaaa acttattaag tttgatatca aggcggggag aaaagaagtc 1080

atatatgtgg aagaacaatt cccatggatt aatttacatg actgcttcac tcctctggac 1140

aaaggaatca ctaaatattc tgaaggattc atttgggcga gtgaaaagac aggatttaga 1200

catttgtatc tgcatgatgc aaatgggaca tgcttaggac caattactga aggtgactgg 1260

atggttgagc aaattgctgg tgtaaatgag gctgctggaa tgatatattt tactgcaact 1320

cgagatgggc cattggaatc gcatctttat cgtgctaaac tgttcccaga tgaaaaaaac 1380

gccttgcagg ctccagtgag attaacaaat ggtaagggga aacactcggt tgtgcttgat 1440

caccacttgc agaattttgt tgatatccac gattccctcg attgtccccc tagagttttg 1500

ctctgctcct tgatcgatgg aagagaaatt atgcctctgt ttgaacaggc tttcaccatt 1560

ccaagattta aaaggctgga acttgagcct ccaaagatag ttcagataca ggcaaacgat 1620

gggaccatat tgtatggggc tttatatgag cctgacccaa ctagatttgg accgccacca 1680

tacaaaacct tgatcagtgt gtatggtggc cccagtgtac agtatgtatg tgattcttgg 1740

ataagtacag ttgacatgag agcacaatat cttcggagca aaggcatctt agtgtggaag 1800

ttggataaca gaggaagtgc tcgtcgtggg ctaaagtttg aaggtgctct gaaaggaaat 1860

cccggccgct ttgatgctga ggatcagctt actggagctg aatggctcat taaacaagga 1920

ctggcaaaag ctggtcatat tgggttgtat ggatggagtt atgggggata tatgtcagct 1980

atgatcttgg caaggttccc tgatgtcttc tgttgtgcag tctctggtgc acctgtaacc 2040

tcctgggatg gatatgacac attttatact gagaaataca tgggattgcc ttatgagaat 2100

ccaacaggct atgagtacgg ctctgtgatg catcatgtgc acaagttaaa agggaggttg 2160

ttactggtgc atgggatgat tgatgaaaat gtgcatttta gacacactgc aaggcttgtc 2220

aatgcactcg tggcagctgg aaaaccctat gaactattaa tttttccaga cgaacgacac 2280

atgccccgtc ggcatacaga ccgaatttac atggaagaga gaatttggga gttcttcgag 2340

agaaatctgt gaagtaattt ttgtttatag attcatgttg tcgcagagaa aattctgcat 2400

cttttttctt caatatttct tgcatatcat aa 2432

<210> 141
<211> 2529
<212> DNA
<213> Arabidopsis thaliana

<400> 141
gagctcttct catcgtcatc ttcgtcgcct tcttttccga tctactttca tcactccttc 60

actcactcag tctccttaca agaattggat tcgaagattt gtagctatgg attcttctgg 120

aactgattcg gctaaagaat tgcatgttgg tttggaccca actacagagg aagagtatgc 180

cacacagtca aagttacttc aagagttcat taatattccc agcattgata aagcttggat 240

ttttaattct gattctggtt ctcaggcaat gtttgccttg agtcaagcaa accttttggc 300

taataaaaag aagaagttta tgttgtcggg tcatatttcg aacgaaagta accaatctgt 360

aaactttcac tgggcgccat ttcccatcga gatgactggt gcatcggctt ttgtcccatc 420

tccatcggga ttgaagctcc ttgtaattcg aaatcctgaa aatgaatctc ctacaaagtt 480

tgagatatgg aattcttctc agctagagaa ggagttccat attccacaga aagttcatgg 540

ctctgtatac gttgatggat ggtttgaagg gatctcttgg gattcagatg agactcatgt 600

tgcgtatgtc gccgaggagc catctcgtcc caagcctaca tttgatcatc ttggttatta 660

caagaaagaa aattctttgg acaaggtatt tggaagctgg aaaggtgaag gggattggga 720

agaggaatgg ggagaagcat acgccggaaa aaggcagcct gcactgtttg ttatcaatgt 780

tgacagtgga gaggtcgagc caatcaaagg aattcctaga tcaataagtg ttggacaggt 840

tgtttggagt ccaaacagta atggatcagc tcaatatttg gtttttgccg gttggttagg 900

agataaaaga aagtttggta ttaagtactg ctacaacaga ccatgtgcca tatacgcaat 960

taagttcaca tcggatgaac caaaagatga tgacgcaaat gaattcccta ttcataattt 1020

gactaagagc ataagcagcg ggttttgtcc tcggttcagc aaagatggca aatttcttgt 1080

gtttgtatcc gcaaagaccg ctgttgattc tggggcgcat tgggcaaccg agtcacttca 1140

taggattgac tggccaagtg atgggaaact tcctgagtca acaaatatcg ttgatgtgat 1200

tcaagttgtg aattgtccta aggacggttg tttccctggg ctctatgtta ccggccttct 1260

gagtgatcca tggctgtcag atggacatag ccttatgttg tctacctact ggcgcagttg 1320

tcgagttata ctcagcgtaa atttgctaag cggtgaagtg tcacgtgcca gccctagtga 1380

ttcagattat tcatggaacg ctcttgcgct agatggtgat agtattgttg ctgtgtctag 1440

cagcccggtg agtgttcctg aaattaagta tggaaagaaa ggtctcgatt cagctgggaa 1500

gccttcatgg ctctggtcga atatccaaag cccgatcaga tactctgaga aggttatggc 1560

agggctttca tctcttcagt ttaaaattct aaaagtacca attagtgatg tttctgaagg 1620

tcttgccgaa ggagccaaaa atcctattga agcaatatat gtatcgtcat ccaagtctaa 1680

ggagaatggg aaatgtgatc ccttaattgc tgttcttcat ggaggtcctc attctgtttc 1740

accatgcagc ttctccagga ctatggccta tctctcctca attggatata gtcagctgat 1800

tataaattac aggggttcat taggatatgg ggaagatgct ttgcagtctc tacctggcaa 1860

agttggatcg caggacgtga aagattgtct cttggctgta gatcatgcca ttgaaatggg 1920

aattgcagac ccgtctagaa taaccgtgct aggtggttct catggtgggt ttctgaccac 1980

tcacttgatt ggccaggccc cggataaatt tgtggcagca gctgcaagga atcccgtatg 2040

caacatggca tcaatggttg ggattacaga tatacctgat tggtgtttct ttgaagccta 2100

tggggaccag agtcactata cagaagcccc ctcagccgaa gatctctctc ggtttcatca 2160

aatgtctcct atatcacaca tctcaaaggt gaaaacacct actttgtttc ttttgggaac 2220

taaggatctc cgtgttccca tatcaaacgg atttcaatac gtgagggcgt tgaaggagaa 2280

aggagttgag gttaaagttc ttgtatttcc caatgacaat catcccttag atagaccaca 2340

gacggattat gaaagctttc tcaacattgc tgtctggttc aacaagtact gcaagctgtg 2400

aacacttgat tttttttccg tatactgagt ttgttttctt acaaaattgg agttcgaaga 2460

aatgaacgtt taaagaataa tcatttggtt caccaatctt agtgcaatga aagactgcca 2520

ctttctggc 2529

<210> 142
<211> 2505
<212> DNA
<213> Arabidopsis thaliana

<400> 142
gcaaagagtg gatgagtaaa taaatggcat tgttgttgtt gacctcactc aatcacctag 60

tctcattctc actcactcgc ttgccttctt cttccgctca caatctcttc ctttctcgtt 120

ccttctcttc ttcgatcaga cgattcaatc gcttctcact caaaccactt cgctccttcg 180

catccatgtc ttcttcttcc cccgacgctg ctcagactcc tctaaccacc gctccttatg 240

gctcctggaa atctccaatc accgccgaca tcgtttccgg agcttcaaag cgtcttggcg 300

gcaccgccgt cgattctcat ggccgtctcg tcttgctcga gtctcgccct aacgaatccg 360

ggagaggagt tttggtgcta caaggagaaa catcaattga tattactcca aaagacttcg 420

ctgtgagaac tttaacacaa gaatacggtg gtggtgcttt ccagatttca tcagatgata 480

cacttgtgtt ctctaattac aaggatcagc gattgtacaa gcaagacatt actgataaag 540

attcatctcc aaagccaatt actccagatt atggtacacc agctgttact tatgcagatg 600

gagtctttga ttcacgcttt aaccgttatg ttactgttag ggaagatggt cgccaggata 660

gatcaaaccc aattacaaca atcgtggagg ttaatctaag tggagagaca cttgaagaac 720

caaaagtact cgtaagtggc aatgatttct atgcatttcc acggttggat cccaagtgtg 780

agcgattggc atggattgaa tggagtcacc ctaatatgcc atgggataaa gcagagctgt 840

gggttgggta catttccgag ggcggaaata ttgataagcg cgtatgtgtt gctggttgtg 900

atcccaaata cgtcgaatca cccactgagc cgaagtggtc atcaagaggg gaactctttt 960

ttgtaactga cagaaagaat ggatgttgga atattcataa atggattgag agtaccaatg 1020

aggttgtttc tgtatatcct ctcgacggtg agtttgcgaa accactatgg attttcggta 1080

ctaactccta cgagataatc gagtgttctg aagagaagaa cctaattgca tgcagctata 1140

ggcagaaagg gaagtcatat ctaggcattg tagatgattc acagggatca tgttctctgc 1200

tcgatattcc tttaacggat tttgacagca ttacattggg aaatcagtgc ctttatgttg 1260

agggagcatc agcagttctt ccaccatcag ttgccagggt aacactggat cagcataaga 1320

cgaaagcact cagttctgag attgtttggt cgtcgtcacc cgatgttttg aagtacaagg 1380

cttacttcag cgtgccagag ttgattgaat ttccaacaga ggttcctggt cagaacgctt 1440

atgcatactt ttatcctcca accaatccgc tctacaatgc tagcatggaa gagaaacctc 1500

cgttgttagt gaagagtcat ggaggaccta ctgctgaatc acgtggatcc ttaaatctga 1560

atatccaata ctggacaagt cgaggttggg catttgttga tgtcaattat ggtggaagca 1620

caggttatgg tcgagagtat cgagagcggt tgttaaggca gtggggaata gtcgacgttg 1680

atgactgttg tggctgtgct aaatacttgg tatcttctgg caaggcagat gttaagcggc 1740

tctgtatatc tggcggttct gcaggaggtt acacaactct tgcatcattg gcgttcaggg 1800

atgttttcaa agctggagcg tccttatacg gagtggctga tttaaaaatg ttgaaagaag 1860

aaggtcataa atttgaatcc cgttatatcg acaatcttgt tggagatgaa aaagatttct 1920

atgagagatc accaatcaat ttcgtcgata agttctcttg tcccatcatc cttttccaag 1980

gactagaaga caaggttgta actccagatc aatcacgtaa aatctatgaa gcattaaaga 2040

aaaaaggtct gcctgttgct cttgtcgagt acgaaggaga acaacacggt tttcgcaagg 2100

cggaaaacat caaatacaca cttgagcaac agatggtgtt ctttgcccga gtggttggag 2160

ggttcaaagt tgctgatgac atcactcctt tgaaaattga caacttcgac acttagaaaa 2220

cgatgcttaa atagatatgt ttggaaaaga tttgcttttt ttttattatt tagagaaaat 2280

acatgcttgg actaacgttt tcggataatt tgttcatcaa aagtccaaaa ccttctatat 2340

tcactagaat gggaatgcct aggaggtggc tcggcaaata accacagtgt taaggtcaat 2400

gttggtttgg gccaatgttg cttaacgtat gtcttttcag ctttagtcat tatcatcgaa 2460

atggttgatg aagctgtcag tgatatgata tcggtagttg gtatt 2505

<210> 143
<211> 2727
<212> DNA
<213> Picea sitchensis

<400> 143
gataactcta atgaattaca aaaacccaat ataacatata tggttgttta acttgaaaag 60

atgtagaaac aggaagaatt accctgccga gtagaaggaa atttttgatg caacggtatg 120

gatccctaac acgaacgtgg tggagatgcc tgatcacaat aaaatacgtt atccggacaa 180

actataactg tggtgtcaca gtgcaccaat ggcttcattc tctttctctt gtgcatattt 240

gagaccctta ttctattgta ggtcaatcac cagtcgaaga ggaatataca cggtaaactg 300
tttgaggcag agctccgcgg atcacaaaac cacagatatg tatgcagaga gaattgaggt 360
gcacaaggag aagatgagtg cgccttatgg ttcatggaag tcaccaatca cagcagatat 420
cgtatctgga gccgataagc gcctgggagg cttcgccctc gacggggaag ggcgtgtcat 480
atggttggaa ggtcgtccaa ctgaagctgg acggtcagtt ttagtaaggg aggctgcaga 540
tgaagaaggc acagctgaag atatcacccc tgctggattt aatgttagga ccttggtaca 600
tgaatatggt ggaggtgcat tcactgtctc gggagatgtt gttgtgttct ctaactacaa 660
ggaccaaaga ctctataagc agtctatcaa aggaggacat gcaccgattg cactcactcc 720
agattatgga gcacctgtgg tgcgttatgc tgatggagtg atggatcttc acttaggctg 780
ttatgtaact gtaagagaag accatcggga aagtgataca aatccaacta caacgattgt 840
ttctgtggag ctcaatgggg ctggcacaac agaaccacat gtattagtca gtggcagcga 900
cttctatgct tttccacgct tgacaccaga tgggggaaag atggcatgga ttgaatggaa 960
tcacccaaat atgccttggg acaaatcgga actttgggtt ggctacatgt ctgcagaagg 1020
taaggttgag aaacggatat gcattgcagg caatgatcca aatatgatag aatcccctac 1080
tgaaccaaag tggtcatctc aaggagaact tttctttgtt acagatagga agagtggatt 1140
ttggaatttg tacaaatggg ttgagtcaac taatgaggtg aaagcattgt acccattgga 1200
tgcagagttt acaaggccgt catgggtatt tggtaatagt tcttatgctt tcattgagca 1260
gaagggacaa aataaaaaca ttgcatgcac atacaggcaa aaaggatgtt cctatctggg 1320
gattcttgat catgttcttg gttccttctc cttagttgat cttcctttta cagatattta 1380
caacattacc tccattggaa gtcatttgta cctagaggga gcatcccctc tgcatccttt 1440
atcgatagtg aaggtttcct acgaagagaa tctgattgca gtgagaggca tctctataat 1500
ttggtcatca tcgtctttga acatttccga gtatagtgca tttataagct ccccagaaat 1560
agttgaattt tctactaaag ttcctggtca gacggcattt gcttatctct atctgccttc 1620
caattacaat tacgaagctc ctgaaggaga aaagccacca ttacttgtaa aaagtcatgg 1680
gggtcccaca tctgaatcac attcagcatt ggatttgagc atccaatatt ggacaagcag 1740
agggtgggct tttgctgacg tcaactatgg aggaagcact gggtatggca gggaatatcg 1800
agaaaggctt aatggctcat ggggtatcgt agatgttaat gattgttgca gctgtgcaga 1860
atttctggta acaacaggga gggtagacgg tgagaggttg tgtattactg ggagatcagc 1920
tggaggttac acaacgctgg ctgctcttgt atttagggaa acatttaaag ctggggcttc 1980
attgtttgga gttgccgacg tgtcactatt gaaagctgat acacacaagt ttgaatcata 2040
ttacactgac agtcttgttg ggaaggatga atcattactg tatgagcgat cacctatcaa 2100
ttttgttgat aggctttcat gtccaatgat tctatttcaa ggattagaag acaaggttgt 2160

cccgcctgaa caagcacgta agatctatgc agcagtgaag gaaaaagggt tacctgttgc 2220

actcgttgag tatgaaggcg aacaacatgg cttccggaag gcagagaaca tcaagtacac 2280

tcttgaacag caaatggtct tttttgcacg tttgattgga aatttcaagg tggccgatga 2340

tatcattcct gtacatatag aaaatttcga ttagaagttt agacaatgct accaatgtgc 2400

tattgtccct attgctatgt acgcatagaa aactagatta aaagtttaga caatgcaaca 2460

ggtgctgttg cctcaacccc tataaacatg atttatgtta tcctatgtga tgaaacatct 2520

cttatgtatt tgtccactta ttaatgggaa ttactgttgg gcctattaaa ggttaattag 2580

gccagatcat aagcatatac tgaagttaag cacaatcttt tacaagtaaa atgacccgca 2640

cataaatcta tgaattttct tatgtatttg tccacttatt aatgggaatt actgttgggc 2700

ctattaaagg taaaaaaaaa aaaaaaa 2727

<210> 144
<211> 1671
<212> DNA
<213> Zea mays

<400> 144
gcaccagctt ggatggatct cctttgtgga gatccaaatg gtccccatag tgatgaagaa 60

tatttagcta gagtcaactg gatgcataat agtgctcttg ctgttcaagt tctcaacagg 120

tcacatacga aacttaaatt acttaagttt gatattagta caggtgaaag agaagtctta 180

ctagaagagc agcatgaggt atggatcaca ttgcatgatt gcttcactcg actagacaaa 240

ggagtgaata ataaacatcg aggtggcttt atttgggcca gtgaaaaaac aggattcagg 300

catttatatg ttcatggcaa tgatggagca tgcttaggac ctctcacaca aggtgattgg 360

atggttgagc acattgctgg tatcaatgag agtaatggac ttatatattt cactggaaca 420

ttggatggac cattggagac aaatctctac cacaccaatc tctttccaga ttggagcctt 480

cccttgcaaa cccctaaaag ggctgactcg tgggaactgg ccggcattca gtaattctcg 540

accatcagtt gctgaagttt attgatgtgt atgacacagc aaaatctcca cctgtgatct 600

tgctgtgctc tttgcttgat ggaagtgtaa ttatacccct gtttgagcag ccactgacta 660

ttccgccact taaaaagttc cagcagttgt ctccagagat agttgaaatt acagcgaagg 720

atgggaccaa tttatatggc gctctctacc ttcctgacga gagaaaatat gggccacctc 780

cctacaaaac actggttaat gtttatggtg gccccagtgt ccagcttgtt agtgattcat 840

ggatgtgtac agttgacatg agagctcaat atctacggag caagggtata ttagtttgga 900

agatggataa tcgaggatcg gcaaggcgag ggctgcattt tgagggacaa ctgaagtaca 960

acattggtcg tgttgatgct gaagatcaat tagaaggggc tgagtggtta ataaagaagg 1020

gccttgcaaa acctggccat attggtatct atggctggag ctacggagga tttctctcag 1080

caatgtgcct cgcaaggttt ccagacacat tctgttgtgc ggtgtctggt gctccggtaa 1140

cagcatggga tggttacgac accttttaca cagagaagta cttgggtctg cccgcggagc 1200

atccggatgc ttacgagtac gggtcaataa tgtaccacgc caagaacctg aaagggaagc 1260

tgctcctcat ccatgggatg attgacgaga acgtgcattt caggcacacg gcaaggctca 1320

tcaactcgct aatggcagag ggcaagccct atgaaatcct ccttttccct gacgagaggc 1380

acatgccacg acgccttggt gatcggatct acatggagga gaggatcttt ggttttttcg 1440

agaggagcct ttgagagggc gtttgtataa atttgttgcc cacatctggg ctgtgattgg 1500

gatgtttttg gcatatggat tcaggttttc tcattcacac ctgtatttat cactagcgca 1560

gttatctttg attcttttgc cagtcttgga tggttatcag ctgtttggct gcgtttacag 1620

gtttcagaat aagcaaaaac ttcatatcaa aaaaaaaaaa aaaaaaaaaa a 1671

<210> 145
<211> 770
<212> PRT
<213> Medicago truncatula

<400> 145

Met Arg Arg Gly Ile Arg Ser Val Lys Asn His Phe Leu Ser Lys Cys
1               5                   10                  15

Leu Pro Val Thr Asp Phe Asn Asp Val Gln Asn Leu Asp Asp Gly Ile
            20                  25                  30

Leu Phe Pro Val Glu Glu Ile Ala Gln Tyr Pro Leu Pro Gly Tyr Val
        35                  40                  45

Ser Pro Thr Ser Ile Ser Phe Ser Pro Asp Asp Ser Leu Ile Ser Tyr
    50                  55                  60

Leu Phe Ser Pro Asp Asn Thr Leu Asn Arg Lys Ile Phe Thr Phe Asp
65                  70                  75                  80

Leu Lys Thr Asn Lys Gln Glu Leu Leu Phe Ser Pro Pro Asp Gly Gly
                85                  90                  95

Leu Asp Glu Ser Asn Ile Ser Pro Glu Glu Lys Leu Arg Arg Glu Arg
            100                 105                 110

Leu Arg Glu Arg Gly Leu Gly Val Thr Arg Tyr Glu Trp Val Lys Thr
        115                 120                 125

Asn Ser Lys Arg Lys Ala Val Leu Val Pro Leu Pro Ala Gly Ile Tyr
    130                 135                 140

Val His Asp Ile Ser His Ser Lys Thr Glu Leu Lys Leu Pro Ser Ile
145                 150                 155                 160

Pro Ala Ser Pro Ile Ile Asp Pro His Leu Ser Pro Asp Gly Ser Met
                165                 170                 175

Leu Ala Tyr Val Arg Asp Cys Glu Leu His Val Met Asn Leu Leu Ser
            180                 185                 190

Asp Glu Ser Lys Gln Leu Thr His Gly Ala Lys Glu Asn Gly Phe Thr
        195                 200                 205

His Gly Leu Ala Glu Tyr Ile Ala Gln Glu Glu Met Asp Arg Lys Thr
    210                 215                 220

Gly Tyr Trp Trp Ser Leu Asp Ser Lys Tyr Ile Ala Phe Thr Glu Val
225                 230                 235                 240

Asp Tyr Ser Glu Ile Pro Leu Phe Arg Ile Met His Gln Gly Arg Ser
                245                 250                 255

Ser Val Gly Thr Asp Ala Gln Glu Asp His Pro Tyr Pro Phe Ala Gly
            260                 265                 270

Ala Ser Asn Ala Lys Ile Arg Leu Gly Val Val Ser Val Ala Gly Gly
        275                 280                 285

Ser Thr Thr Trp Met Asp Leu Val Cys Gly Gly Val Lys Glu Leu Asp
    290                 295                 300

Asn Glu Asp Glu Tyr Leu Ala Arg Val Asn Trp Met His Gly Asn Ile
305                 310                 315                 320

Leu Thr Ala Gln Ile Ile Asn Arg His Gln Thr Lys Ile Lys Ile Val
                325                 330                 335

Lys Phe Asp Ile Arg Thr Gly Gln Arg Arg Asp Ile Leu Val Glu Glu
            340                 345                 350

Asn Lys Thr Trp Ile Asn Ile His Asp Cys Phe Thr Pro Phe Asp Lys
        355                 360                 365

Gly Val Thr Lys Phe Ser Gly Gly Phe Ile Trp Ala Ser Glu Lys Ser
    370                 375                 380

Gly Phe Arg His Leu Tyr Leu His Asp Ala Asn Gly Ile Cys Leu Gly

385                 390                 395                 400

Pro Ile Thr Glu Gly Glu Trp Met Val Glu Gln Ile Ala Gly Val Asn
                405                 410                 415

Glu Ala Thr Gly Leu Val Tyr Phe Thr Gly Thr Leu Asp Ser Pro Leu
            420                 425                 430

Glu Ser Asn Leu Tyr Cys Ala Lys Leu Phe Val Asp Gly Thr Gln Pro
        435                 440                 445

Leu Gln Ala Pro Thr Arg Leu Thr His Ser Lys Gly Lys His Ile Val
    450                 455                 460

Val Leu Asp His His Met Arg Thr Phe Val Asp Ile His Asp Ser Leu
465                 470                 475                 480

Ser Cys Pro Pro Arg Val Leu Leu Cys Ser Leu Glu Asp Gly Thr Ile
                485                 490                 495

Ile Met Pro Leu Tyr Glu Gln Gln Ile Pro Ile Pro Lys Ser Lys Lys
            500                 505                 510

Leu Gln Leu Glu Pro Pro Glu Ile Val Glu Ile Gln Ser Asp Asp Gly
        515                 520                 525

Thr Thr Leu Tyr Gly Ala Leu Tyr Lys Pro Asp Pro Ser Arg Phe Gly
    530                 535                 540

Pro Pro Pro Tyr Lys Thr Met Ile Asn Val Tyr Gly Gly Pro Ser Val
545                 550                 555                 560

Gln Leu Val Ser Asn Ser Trp Leu Asn Thr Val Asp Leu Arg Ala Gln
                565                 570                 575

Tyr Leu Arg Asn Lys Gly Ile Leu Val Trp Lys Leu Asp Asn Arg Gly
            580                 585                 590

Thr Ser Arg Arg Gly Leu Lys Phe Glu Gly Tyr Leu Lys Gln Lys Leu
        595                 600                 605

Gly Gln Ile Asp Ala Asp Asp Gln Phe Thr Gly Ala Glu Trp Leu Val
    610                 615                 620

Lys Asn Gly Leu Ala Glu Phe Gly His Ile Gly Leu Tyr Gly Trp Ser
625                 630                 635                 640

Tyr Gly Gly Tyr Leu Ser Ala Met Thr Leu Ser Arg Tyr Pro Asp Phe
                645                 650                 655

Phe Lys Cys Ala Val Ala Gly Ala Pro Val Thr Ser Trp Asp Gly Tyr
            660                 665                 670

Asp Thr Phe Tyr Thr Glu Lys Tyr Met Gly Leu Pro Ser Glu Tyr Lys
        675                 680                 685

Ser Gly Tyr Ala Arg Ala Ser Val Met Asn His Val His Lys Met Arg
    690                 695                 700

Gly Arg Leu Leu Ile Val His Gly Met Ile Asp Glu Asn Val His Phe
705                 710                 715                 720

Arg His Thr Ala Arg Leu Ile Asn Ala Leu Val Ala Ala Gly Lys Thr
                725                 730                 735

Tyr Glu Leu Ile Ile Phe Pro Asp Glu Arg His Met Pro Arg Arg Tyr
            740                 745                 750

Ser Asp Arg Val Tyr Met Glu Glu Arg Met Trp Glu Phe Ile Asp Arg
        755                 760                 765

Asn Leu
    770

<210>  146
<211>  596
<212>  PRT
<213>  Oryza sativa

<400>  146

Met Ile Ala Tyr Val Arg Asp Asp Glu Leu His Thr Val Gly Phe Ser
1               5                   10                  15

Asp Gly Gln Thr Thr Gln Leu Thr Tyr Gly Ala Ser Glu Ser Gly Lys
            20                  25                  30

Ile His Gly Leu Ala Glu Tyr Ile Ala Gln Glu Glu Met Glu Arg Lys
        35                  40                  45

Met Gly Phe Trp Trp Ser Pro Asp Ser Lys His Leu Ala Phe Thr Glu
    50                  55                  60

Val Asp Ser Ser Glu Ile Pro Leu Tyr Arg Ile Met His Gln Gly Lys
65                  70                  75                  80

Ser Ser Val Gly Pro Asp Ala Gln Glu Asp His Ala Tyr Pro Phe Ala
                    85                  90                  95

Gly Ala Ala Asn Val Lys Val Arg Leu Gly Val Val Ser Ser His Gly
            100                 105                 110

Gly Glu Ile Thr Trp Met Asp Leu Leu Cys Gly Glu Pro Asn Ser Ile
        115                 120                 125

His Gly Asp Glu Glu Tyr Leu Ala Arg Val Asn Trp Met His Asn Ser
    130                 135                 140

Ala Ile Ala Val Gln Val Leu Asn Arg Thr His Ser Lys Leu Lys Leu
145                 150                 155                 160

Leu Lys Phe Asp Ile Ala Ser Gly Lys Arg Glu Val Ile Leu Glu Glu
                165                 170                 175

Glu His Asp Thr Trp Ile Thr Leu His Asp Cys Phe Thr Pro Leu Asp
            180                 185                 190

Lys Gly Val Asn Ser Lys His Pro Gly Gly Phe Ile Trp Ala Ser Glu
        195                 200                 205

Lys Thr Gly Phe Arg His Leu Tyr Leu His Asp Lys Asn Gly Val Cys
    210                 215                 220

Leu Gly Pro Leu Thr Gln Gly Asp Trp Met Ile Asp Gln Ile Ala Gly
225                 230                 235                 240

Val Asn Glu Ser Ser Gly Val Ile Tyr Phe Thr Gly Thr Leu Asp Gly
                245                 250                 255

Pro Leu Glu Thr Asn Leu Tyr Ser Thr Asn Leu Phe Pro Asp Trp Ser
            260                 265                 270

Leu Pro Leu Gln Val Pro Lys Arg Leu Thr His Gly Thr Gly Arg His
        275                 280                 285

Ser Val Ile Leu Asp His Gln Leu Leu Arg Phe Ile Asp Val Tyr Asp
    290                 295                 300

Ser Ile Lys Ser Pro Pro Val Ile Leu Leu Cys Ser Leu Leu Asp Gly
305                 310                 315                 320

Ser Val Ile Met Pro Leu Tyr Glu Gln Pro Leu Thr Val Gln Pro Leu
                325                 330                 335

Lys Lys Phe Gln Gln Leu Ser Pro Glu Ile Val Gln Ile Glu Gly Lys
            340                 345                 350

Asp Gly Thr Ala Leu Tyr Gly Thr Leu Tyr Leu Pro Asp Glu Lys Lys
        355                 360                 365

Tyr Gly Pro Pro Pro Tyr Lys Thr Leu Val Asn Val Tyr Gly Gly Pro
    370                 375                 380

Ser Val Gln Leu Val Ser Asp Ser Trp Ile Ser Thr Val Asp Met Arg
385                 390                 395                 400

Ala Gln Phe Leu Arg Ser Lys Gly Ile Leu Val Trp Lys Met Asp Asn
                405                 410                 415

Arg Gly Thr Ala Arg Arg Gly Leu Gln Phe Glu Gly Gln Leu Lys Tyr
            420                 425                 430

Asn Ile Gly Arg Val Asp Ala Glu Asp Gln Leu Ala Gly Ala Glu Trp
        435                 440                 445

Leu Ile Lys Lys Gly Leu Ala Lys Pro Gly His Ile Gly Leu Tyr Gly
    450                 455                 460

Trp Ser Tyr Gly Gly Phe Leu Ser Ala Met Cys Leu Ala Arg Phe Pro
465                 470                 475                 480

Asp Thr Phe Ser Cys Ala Val Ser Gly Ala Pro Val Thr Ala Trp Asp
                485                 490                 495

Gly Tyr Asp Thr Phe Tyr Thr Glu Lys Tyr Met Gly Leu Pro Ser Glu
            500                 505                 510

Gln Arg Asp Ala Tyr Arg Tyr Gly Ser Ile Met His His Val Lys Asn
        515                 520                 525

Leu Arg Gly Arg Leu Leu Leu Ile His Gly Met Ile Asp Glu Asn Val
    530                 535                 540

His Phe Arg His Thr Ala Arg Leu Ile Asn Ser Leu Met Ala Glu Gly
545                 550                 555                 560

Lys Pro Tyr Asp Ile Leu Leu Phe Pro Asp Glu Arg His Met Pro Arg
                565                 570                 575

Arg Leu Gly Asp Arg Ile Tyr Met Glu Glu Arg Ile Trp Asp Phe Val
            580                 585                 590

Glu Arg Asn Leu
        595

<210>  147
<211>  748
<212>  PRT
<213>  Physcomitrella patens

<400>  147

Met Pro Thr Thr Asp Ser Gln Ser Leu Gly Asp Glu Tyr Thr Leu Phe
1               5                   10                  15

Pro Val Glu Asp Ile Val Gln Asn Pro Leu Pro Gly Tyr Val Ala Pro
            20                  25                  30

Gly Ser Val Gln Phe Ser Pro Asp Asp Lys Leu Ile Thr Tyr Leu Tyr
        35                  40                  45

Ser Pro Asp Ser Thr Leu Ser Arg Lys Ile Tyr Ala Phe Asp Val Val
    50                  55                  60

Ala Lys Gln Gln Arg Leu Leu Val Asn Pro Pro Gly Gly Gly Val Asp
65                  70                  75                  80

Glu Gly Asn Leu Ser Thr Val Glu Lys Leu Arg Arg Glu Arg Leu Arg
                85                  90                  95

Glu Arg Gly Leu Gly Val Thr Arg Tyr Glu Trp Ala Lys Gly Val Pro
            100                 105                 110

His Arg Leu Met Val Pro Leu Pro Ser Gly Ile Tyr Val Gln Gly Val
        115                 120                 125

Gly Ser Glu Leu Arg Leu Arg Val Ala Ser Thr Pro Ser Asn Pro Ile
    130                 135                 140

Leu Asp Pro Gln Leu Ser Pro Asp Ala Ser Ser Ile Ala Tyr Val Arg
145                 150                 155                 160

Asp Asp Glu Leu Phe Val Val Pro Leu Ala Phe Gly Glu Pro Ile Gln
                165                 170                 175

Ile Thr Tyr Gly Ala Arg Gly Thr Gly Lys Thr His Gly Leu Ala Glu
            180                 185                 190

Tyr Ile Ala Gln Glu Glu Met Asp Arg Arg Asn Gly Phe Trp Trp Ser
        195                 200                 205

Pro Asp Ser Arg Tyr Ile Ala Phe Ala Glu Val Asp Ser Ser Arg Ile
    210                 215                 220

Pro Pro Phe Thr Ile Val His Gln Gly Lys Val Thr Thr Gly Gly Glu
225                 230                 235                 240

Ala Glu Glu Val His Ala Tyr Pro Phe Ala Gly His Ser Asn Val Ser
                245                 250                 255

Val Arg Leu Gly Val Val Pro Ser Asn Gly Asp Ser Val Val Trp Met
            260                 265                 270

Asp Leu Glu Val Gly Leu Cys Gly Glu Gly His Glu Asp Glu Glu Tyr
        275                 280                 285

Leu Ala Arg Val Thr Trp Leu Pro Asn Gly Tyr Leu Thr Ala Gln Val
    290                 295                 300

Gln Asn Arg Thr Gln Ser Lys Leu Lys Leu Leu Arg Phe Asp Pro Leu
305                 310                 315                 320

Thr Gly Lys Arg Leu Val Leu Leu Thr Glu Glu Ser Glu Val Trp Val
                325                 330                 335

Asn Leu His Asp Ser Phe Thr Pro Leu Arg Lys Tyr His Gly Pro Leu
            340                 345                 350

Ser Gly Gly Phe Ile Trp Ala Ser Glu Lys Thr Gly Phe Arg His Leu
        355                 360                 365

Tyr Met His Asp Glu Phe Gly Asn Cys Ile Arg Ala Leu Thr Gln Gly
    370                 375                 380

Ser Trp Met Val Glu Gln Val Ala Gly Val Asp Glu Asp Ala Gly Leu
385                 390                 395                 400

Val Tyr Phe Thr Gly Ala Phe Asp Ser Pro Leu Glu Val His Leu Tyr
                405                 410                 415

Ser Ala Ser Leu Asp Cys Thr Glu Pro Asn Leu Ala Glu Pro Lys Arg
            420                 425                 430

Leu Thr Gln Gly Ala Gly Arg His Ile Val Val Leu Asp His Glu Met
        435                 440                 445

Lys Lys Phe Val Asp Ile His Asp Ser Leu Asp Thr Pro Pro Arg Leu

450                 455                 460

Leu Leu Arg Ser Leu Asp Thr Gly Lys Leu Leu Ile Ser Ile Tyr Glu
465                 470                 475                 480

Gln Pro Ala Pro Thr Tyr His Ala Arg Arg Leu Gln Leu Thr Pro Pro
                485                 490                 495

Glu Phe Lys Ser Leu Thr Ala Ser Asp Gly Thr Val Leu His Gly Leu
            500                 505                 510

Ile Tyr Ile Pro Asp Ser Lys Gln Phe Gly Arg Pro Pro Tyr Arg Thr
        515                 520                 525

Val Val Ser Val Tyr Gly Gly Pro Asn Val Gln Ile Val Cys Asn Ser
    530                 535                 540

Trp Met Asn Thr Val Asp Met Arg Ala Gln Tyr Leu Arg Ser Arg Gly
545                 550                 555                 560

Ile Leu Val Trp Lys Leu Asp Asn Arg Gly Ser Ala Arg Arg Gly Leu
                565                 570                 575

Asp Phe Glu Gly Ala Ile Lys His Asn Met Gly Met Ile Asp Val Asp
            580                 585                 590

Asp Gln Gln Thr Gly Val Gln Trp Leu Val Asn Gln Gly Leu Ala Met
        595                 600                 605

Pro Asn Arg Ile Gly Ile Tyr Gly Trp Ser Tyr Gly Gly Tyr Met Ala
    610                 615                 620

Ser Met Ala Leu Ala Arg Cys Pro Glu Thr Phe Ser Cys Ala Val Ala
625                 630                 635                 640

Gly Ala Pro Val Thr Ser Trp Asp Gly Tyr Asp Thr Tyr Tyr Thr Glu
                645                 650                 655

Lys Tyr Met Gly Thr Pro Ala Ser Asn Pro Ala Gly Tyr Gln Tyr Ser
            660                 665                 670

Ser Val Met His His Val Tyr Gln Ile Thr Gly Lys Leu Leu Leu Val
        675                 680                 685

His Gly Met Ile Asp Glu Asn Val His Phe Arg His Thr Ala Arg Leu
    690                 695                 700

Ile Asn Thr Leu Thr Ala Ala Ser Lys Asp Tyr Glu Leu Leu Val Phe
705                 710                 715                 720

Pro Asp Glu Arg His Met Pro Arg Gly Leu Arg Asp Arg Met Tyr Met
                725                 730                 735

Glu Glu Arg Ile Cys Asp Phe Leu Glu Arg Asn Ile
            740                 745

<210>  148
<211>  749
<212>  PRT
<213>  Physcomitrella patens

<400>  148

Met Pro Thr Thr Asp Ser Gln Ala Leu Gly Asp Glu Tyr Thr Leu Phe
1               5                   10                  15

Pro Val Glu Asp Ile Val Gln Tyr Pro Leu Pro Gly Tyr Val Ala Pro
            20                  25                  30

Ala Ser Val Gln Phe Ser Pro Asp Asp Lys Leu Ile Thr Tyr Leu Tyr
        35                  40                  45

Ser Pro Asp Ser Thr Leu Ser Arg Lys Ile Tyr Ala Phe Asp Val Ala
    50                  55                  60

Ala Arg Gln Gln Arg Leu Leu Val Ser Pro Pro Gly Gly Gly Val Asp
65                  70                  75                  80

Glu Gly Asn Leu Ser Thr Val Glu Lys Leu Arg Arg Glu Arg Leu Arg
                85                  90                  95

Glu Arg Gly Leu Gly Val Thr Arg Tyr Glu Trp Gly Lys Gly Val Pro
            100                 105                 110

His Arg Leu Met Val Pro Leu Pro Ser Gly Ile Tyr Val Gln Glu Gly
        115                 120                 125

Val Arg Ala Glu Leu Gln Arg Arg Val Ala Ser Thr Ser Trp Ser Pro
    130                 135                 140

Ile Leu Asp Pro Gln Leu Ser Pro Asp Ala Cys Ser Ile Ala Tyr Val
145                 150                 155                 160

Arg Asp Asp Glu Ile Phe Val Val Pro Val Ala Phe Gly Asp Pro Ile
                165                 170                 175

Gln Ile Thr Ser Gly Ala Arg Gly Thr Gly Lys Thr His Gly Leu Ala
        180                 185                 190

Glu Tyr Ile Ala Gln Glu Glu Met Asp Arg Arg Asn Gly Phe Trp Trp
    195                 200                 205

Ser Pro Asp Ser Arg Tyr Ile Ala Phe Ala Glu Val Asp Ser Thr Arg
    210                 215                 220

Ile Pro Pro Phe Arg Ile Met His Gln Gly Lys Ala Ser Thr Gly Gly
225                 230                 235                 240

Glu Ala Glu Glu Ile His Ala Tyr Pro Phe Ala Gly His Ser Asn Val
                245                 250                 255

Ser Val Arg Leu Gly Val Val Pro Ser Asn Gly Gly Ser Val Val Trp
            260                 265                 270

Met Asp Leu Glu Val Gly Leu Cys Gly Glu Gly Arg Asp Asp Glu Glu
        275                 280                 285

Tyr Leu Ala Arg Val Thr Trp Leu Pro Asn Gly Ser Leu Thr Ala Gln
    290                 295                 300

Val Gln Asn Arg Thr Gln Thr Lys Leu Lys Leu Leu Lys Phe Asp Pro
305                 310                 315                 320

Leu Thr Gly Lys Arg Thr Val Leu Phe Thr Glu Glu Ser Asp Val Trp
                325                 330                 335

Val Asn Leu His Asp Cys Phe Thr Ser Leu Arg Lys Cys His Gly Leu
            340                 345                 350

Leu Ser Gly Gly Phe Ile Trp Ala Ser Glu Lys Thr Gly Phe Arg His
        355                 360                 365

Leu Tyr Val His Asp Glu Phe Gly Asn Cys Ile Arg Ala Leu Thr Gln
    370                 375                 380

Gly Ser Trp Met Val Glu Gln Val Ala Gly Val Asp Glu Glu Ala Gly
385                 390                 395                 400

Leu Val Tyr Phe Thr Gly Thr His Asp Ser Pro Leu Glu Met His Leu
                405                 410                 415

Tyr Ser Val Ser Leu Asp Cys Thr Gln Pro Asn Leu Val Glu Pro Lys
            420                 425                 430

Arg Leu Thr Gln Gly Ala Gly Arg His Ile Val Val Leu Asp His Gln
        435                 440                 445

Met Lys Met Phe Val Asp Ile His Asp Ser Leu Asp Thr Pro Pro Arg
    450                 455                 460

Leu Leu Leu Arg Ser Leu Asp Thr Gly Lys Leu Leu Val Thr Ile Tyr
465                 470                 475                 480

Glu Gln Pro Ala Pro Thr Tyr His Thr Arg Arg Leu Gln Leu Thr Pro
                485                 490                 495

Pro Glu Phe Lys Ser Leu Thr Ala Ser Asp Gly Thr Val Leu His Gly
            500                 505                 510

Leu Val Tyr Ile Pro Asp Ser Lys Gln Phe Gly Arg Pro Pro Tyr Arg
        515                 520                 525

Thr Val Val Ser Val Tyr Gly Gly Pro Asn Val Gln Val Val Cys Asn
    530                 535                 540

Ser Trp Met Asn Thr Val Asp Met Arg Ala Gln Tyr Leu Arg Ser Arg
545                 550                 555                 560

Gly Ile Leu Val Trp Lys Leu Asp Asn Arg Gly Ser Ala Arg Arg Gly
                565                 570                 575

Leu Asp Phe Glu Gly Ala Ile Lys His Asn Met Gly Lys Ile Asp Val
            580                 585                 590

Glu Asp Gln Gln Thr Gly Val Gln Trp Leu Val Ser Gln Gly Leu Ala
        595                 600                 605

Met Ser Asn Arg Ile Gly Ile Tyr Gly Trp Ser Tyr Gly Gly Tyr Met
    610                 615                 620

Ala Ser Met Ala Leu Ala Arg Cys Pro Gln Thr Phe Ser Cys Ala Val
625                 630                 635                 640

Ala Gly Ala Pro Val Thr Ser Trp Asp Gly Tyr Asp Thr His Tyr Thr
                645                 650                 655

Glu Lys Tyr Met Gly Thr Pro Ala Ser Asn Pro Ala Gly Tyr Gln Tyr
            660                 665                 670

Ser Ser Val Met His His Val Tyr Gln Ile Thr Gly Lys Leu Leu Leu
        675                 680                 685

Val His Gly Met Ile Asp Glu Asn Val His Phe Arg His Thr Ala Arg
    690                 695                 700

Leu Ile Asn Ser Leu Thr Ala Ala Ser Lys Asp Tyr Glu Leu Leu Val
705                 710                 715                 720

Phe Pro Asp Glu Arg His Met Pro Arg Gly Leu Arg Asp Arg Met Tyr
                725                 730                 735

Met Glu Glu Arg Ile Cys Asp Phe Leu Glu Arg Asn Ile
            740                 745

<210>  149
<211>  773
<212>  PRT
<213>  Populus trichocarpa

<400>  149

Met Gln Ser Val Asp Glu Asn Glu Ser Gln Asn Lys Lys Leu Arg Ile
1               5                   10                  15

Leu Arg Ser Leu Asn Asn Asp Met Pro Leu Thr Asp Asn Thr Ile Pro
            20                  25                  30

Gln Asn Val Glu Asp Ser Ile Leu Phe Pro Ile Glu Glu Ile Val Gln
        35                  40                  45

Ser Pro Leu Pro Gly Tyr Glu Ala Pro Thr Ser Ile Gly Phe Ser Ala
    50                  55                  60

Asp Asp Ser Leu Leu Thr Tyr Leu Phe Ser Pro Asp His Thr Leu Ser
65                  70                  75                  80

Arg Lys Val Phe Ala Phe Asp Leu Lys Ser Gly Lys Gln Glu Leu Phe
                85                  90                  95

Phe Gly Pro Pro Asp Gly Gly Leu Asp Glu Ser Asn Ile Ser Pro Glu
            100                 105                 110

Glu Lys Leu Arg Arg Glu Arg Leu Arg Gln Arg Gly Leu Gly Val Thr
        115                 120                 125

Cys Tyr Glu Trp Val Lys Thr Gly Ser Lys Lys Lys Ala Ile Met Val
    130                 135                 140

Pro Leu Pro Ala Gly Glu Leu His Ser Ser Lys Pro Glu Leu Lys Leu
145                 150                 155                 160

Pro Ser Ser Ala Leu Ser Pro Val Ile Asp Pro His Val Ser Pro Asp
                165                 170                 175

Gly Thr Met Leu Ala Tyr Ile Arg Asp Ser Glu Leu His Val Leu Asn
            180                 185                 190

Leu Leu Tyr Asn Glu Ser Lys Gln Leu Thr His Gly Ala Gln Gly Asn
        195                 200                 205

Thr Val Thr His Gly Leu Ala Glu Tyr Ile Ala Gln Glu Glu Met Asp
    210                 215                 220

Arg Lys Asn Gly Tyr Trp Trp Ser Leu Asp Ser Lys Phe Ile Ala Phe
225                 230                 235                 240

Thr Gln Val Asp Ser Ser Glu Ile Pro Leu Phe Arg Ile Met His Gln
                245                 250                 255

Gly Lys Ser Ser Val Gly Ser Glu Ala Gln Glu Asp His Pro Tyr Pro
            260                 265                 270

Phe Ala Gly Ala Ser Asn Val Lys Val Arg Leu Gly Val Val Ser Val
        275                 280                 285

His Gly Asp Ser Ile Thr Trp Met Asp Leu Leu Cys Gly Gly Thr Lys
    290                 295                 300

Glu Pro Asp Asn Glu Asp Glu Tyr Leu Ala Arg Val Asn Trp Met His
305                 310                 315                 320

Gly Asn Val Leu Ile Ala Gln Val Leu Asn Arg Ser His Ser Lys Leu
                325                 330                 335

Lys Leu Leu Lys Phe Asp Ile Lys Thr Gly Lys Lys Glu Val Leu Tyr
            340                 345                 350

Ala Glu Glu Gln Leu Pro Trp Ile Asn Leu His Asp Cys Phe Thr Pro
        355                 360                 365

Leu Asp Lys Gly Ile Thr Lys Tyr Ser Gly Gly Phe Ile Trp Ala Ser
    370                 375                 380

Glu Lys Ser Gly Phe Arg His Leu Cys Val His Asp Ala Asn Gly Ala
385                 390                 395                 400

Cys Leu Gly Pro Ile Thr Glu Gly Glu Trp Met Val Glu Gln Ile Ala

405                 410                 415

Gly Val Asn Glu Ala Ala Gly Ile Ile Tyr Phe Thr Ala Thr Leu Asp
            420                 425                 430

Gly Pro Leu Glu Ser His Leu Tyr Arg Ala Lys Leu Tyr Pro Ile Glu
        435                 440                 445

Asn Asn Pro Leu Gln Ala Pro Val Arg Leu Thr Asn Gly Lys Gly Lys
    450                 455                 460

His Ser Val Val Leu Asp His His Leu Gln Asn Phe Val Asp Ile His
465                 470                 475                 480

Asp Ser Leu Asp Ser Pro Pro Arg Val Ser Leu Cys Ser Leu Phe Asp
                485                 490                 495

Gly Arg Glu Ile Met Pro Leu Phe Glu Gln Ser Phe Thr Ile Pro Arg
            500                 505                 510

Tyr Lys Arg Leu Glu Leu Glu Pro Pro Lys Ile Val Gln Ile Gln Ala
        515                 520                 525

Asn Asp Gly Thr Ile Leu Tyr Gly Ala Leu Tyr Asp Pro Asp Pro Thr
    530                 535                 540

Arg Phe Gly Pro Pro Pro Tyr Lys Thr Val Ile Ser Val Tyr Gly Gly
545                 550                 555                 560

Pro Gly Val Gln Tyr Val Cys Asp Ser Trp Ile Gly Thr Ala Asp Met
                565                 570                 575

Arg Ala Gln Tyr Leu Arg Ser Gln Gly Ile Leu Val Trp Lys Leu Asp
            580                 585                 590

Asn Arg Gly Ser Ala Arg Arg Gly Leu Lys Phe Glu Gly Ala Leu Lys
        595                 600                 605

Gly Asn Pro Gly Arg Phe Asp Ala Glu Asp Gln Leu Thr Gly Ala Glu
    610                 615                 620

Trp Leu Ile Lys Gln Gly Leu Ala Lys Ala Gly His Ile Gly Leu Cys
625                 630                 635                 640

Gly Trp Ser Tyr Gly Gly Tyr Met Ser Ala Val Ile Leu Ala Arg Phe
                645                 650                 655

Pro Asp Val Phe Cys Cys Ala Val Ser Gly Ala Pro Val Thr Ser Trp
            660                 665                 670

Asp Gly Tyr Asp Thr Phe Tyr Thr Glu Lys Tyr Met Gly Leu Pro Ser
        675                 680                 685

Asp Asn Pro Lys Gly Tyr Glu Tyr Gly Ser Val Met His His Val His
    690                 695                 700

Lys Leu Lys Gly Arg Leu Leu Leu Val His Gly Met Ile Asp Glu Asn
705                 710                 715                 720

Val His Phe Arg His Thr Ala Arg Leu Val Asn Ala Leu Val Ala Ala
                725                 730                 735

Gly Lys Pro Tyr Glu Leu Leu Ile Phe Pro Asp Glu Arg His Met Pro
            740                 745                 750

Arg Arg His Asn Asp Arg Ile Tyr Met Glu Glu Arg Ile Trp Glu Phe
        755                 760                 765

Phe Gln Arg Ser Leu
    770

<210> 150
<211> 773
<212> PRT
<213> Populus trichocarpa

<400> 150

Met Gln Ser Val Asp Glu Asn Glu Ser Glu Asn Lys Lys Leu Lys Arg
1               5                   10                  15

Leu Arg Ser Leu Ser Asn Asn Met Pro Leu Thr Asp Asn Thr Thr Pro
            20                  25                  30

Gln Asn Val Glu Asp Ser Ile Leu Phe Pro Ile Glu Glu Ile Val Gln
        35                  40                  45

Ser Pro Leu Pro Gly Tyr Val Ala Pro Thr Ser Ile Gly Phe Ser Ala
    50                  55                  60

Asp Asp Ser Leu Val Thr Cys Leu Phe Ser Pro Asp His Thr Leu Ser
65                  70                  75                  80

Arg Lys Val Phe Ala Phe Asp Leu Lys Asn Gly Lys Gln Glu Leu Phe
                85                  90                  95

Phe Gly Pro Pro Asp Gly Gly Leu Asp Glu Ser Asn Ile Ser Ala Glu
            100                 105                 110

Glu Lys Leu Arg Arg Glu Arg Leu Arg Glu Arg Gly Leu Gly Val Thr
        115                 120                 125

Arg Tyr Glu Trp Val Lys Thr Gly Leu Lys Lys Lys Ala Ile Met Val
    130                 135                 140

Pro Leu Pro Ala Gly Glu Leu Tyr Ser Pro Lys Pro Glu Leu Lys Leu
145                 150                 155                 160

Pro Ser Ser Ser Leu Ser Pro Ile Ile Asp Pro His Ile Ser Pro Asp
                165                 170                 175

Gly Thr Met Leu Ala Tyr Val Arg Asp Ser Glu Leu His Val Leu Asn
            180                 185                 190

Phe Leu Phe Asn Glu Ser Lys Gln Leu Thr His Gly Ala Gln Gly Asn
        195                 200                 205

Thr Val Thr His Gly Ile Ala Glu Tyr Ile Ala Gln Glu Glu Met Asp
    210                 215                 220

Arg Lys Asn Gly Tyr Trp Trp Ser Leu Asp Ser Gln Phe Ile Ala Phe
225                 230                 235                 240

Thr Gln Val Asp Ser Ser Glu Ile Pro Leu Phe Arg Ile Met His Gln
                245                 250                 255

Gly Lys Ser Ser Val Gly Ser Glu Ala Gln Glu Asp His Pro Tyr Pro
            260                 265                 270

Phe Ala Gly Ala Ser Asn Val Lys Val His Leu Gly Val Val Ser Val
        275                 280                 285

His Gly Gly Ser Val Thr Trp Leu Asp Leu Leu Cys Gly Gly Thr Glu
    290                 295                 300

Lys Pro Asp Asn Glu Asp Glu Tyr Leu Ala Arg Ile Asn Trp Met His
305                 310                 315                 320

Gly Asn Ile Leu Ile Ala Gln Val Leu Asn Arg Ser His Ser Lys Leu
                325                 330                 335

Lys Leu Ile Lys Phe Asp Ile Lys Ala Gly Arg Lys Glu Val Ile Tyr
            340                 345                 350

Val Glu Glu Gln Phe Pro Trp Ile Asn Leu His Asp Cys Phe Thr Pro
        355                 360                 365

Leu Asp Lys Gly Ile Thr Lys Tyr Ser Glu Gly Phe Ile Trp Ala Ser
    370                 375                 380

Glu Lys Thr Gly Phe Arg His Leu Tyr Leu His Asp Ala Asn Gly Thr
385                 390                 395                 400

Cys Leu Gly Pro Ile Thr Glu Gly Asp Trp Met Val Glu Gln Ile Ala
                405                 410                 415

Gly Val Asn Glu Ala Ala Gly Met Ile Tyr Phe Thr Ala Thr Arg Asp
            420                 425                 430

Gly Pro Leu Glu Ser His Leu Tyr Arg Ala Lys Leu Phe Pro Asp Glu
        435                 440                 445

Lys Asn Ala Leu Gln Ala Pro Val Arg Leu Thr Asn Gly Lys Gly Lys
    450                 455                 460

His Ser Val Val Leu Asp His His Leu Gln Asn Phe Val Asp Ile His
465                 470                 475                 480

Asp Ser Leu Asp Cys Pro Pro Arg Val Leu Leu Cys Ser Leu Ile Asp
                485                 490                 495

Gly Arg Glu Ile Met Pro Leu Phe Glu Gln Ala Phe Thr Ile Pro Arg
            500                 505                 510

Phe Lys Arg Leu Glu Leu Glu Pro Pro Lys Ile Val Gln Ile Gln Ala
        515                 520                 525

Asn Asp Gly Thr Ile Leu Tyr Gly Ala Leu Tyr Glu Pro Asp Pro Thr
    530                 535                 540

Arg Phe Gly Pro Pro Pro Tyr Lys Thr Leu Ile Ser Val Tyr Gly Gly
545                 550                 555                 560

Pro Ser Val Gln Tyr Val Cys Asp Ser Trp Ile Ser Thr Val Asp Met
                565                 570                 575

Arg Ala Gln Tyr Leu Arg Ser Lys Gly Ile Leu Val Trp Lys Leu Asp
            580                 585                 590

Asn Arg Gly Ser Ala Arg Arg Gly Leu Lys Phe Glu Gly Ala Leu Lys
        595                 600                 605

Gly Asn Pro Gly Arg Phe Asp Ala Glu Asp Gln Leu Thr Gly Ala Glu
    610                 615                 620

Trp Leu Ile Lys Gln Gly Leu Ala Lys Ala Gly His Ile Gly Leu Tyr
625                 630                 635                 640

Gly Trp Ser Tyr Gly Gly Tyr Met Ser Ala Met Ile Leu Ala Arg Phe
                645                 650                 655

Pro Asp Val Phe Cys Cys Ala Val Ser Gly Ala Pro Val Thr Ser Trp
            660                 665                 670

Asp Gly Tyr Asp Thr Phe Tyr Thr Glu Lys Tyr Met Gly Leu Pro Tyr
        675                 680                 685

Glu Asn Pro Thr Gly Tyr Glu Tyr Gly Ser Val Met His His Val His
    690                 695                 700

Lys Leu Lys Gly Arg Leu Leu Leu Val His Gly Met Ile Asp Glu Asn
705                 710                 715                 720

Val His Phe Arg His Thr Ala Arg Leu Val Asn Ala Leu Val Ala Ala
                725                 730                 735

Gly Lys Pro Tyr Glu Leu Leu Ile Phe Pro Asp Glu Arg His Met Pro
            740                 745                 750

Arg Arg His Thr Asp Arg Ile Tyr Met Glu Glu Arg Ile Trp Glu Phe
        755                 760                 765

Phe Glu Arg Asn Leu
    770

<210>  151
<211>  764
<212>  PRT
<213>  Arabidopsis thaliana

<400>  151

Met Asp Ser Ser Gly Thr Asp Ser Ala Lys Glu Leu His Val Gly Leu
1               5                   10                  15

Asp Pro Thr Thr Glu Glu Glu Tyr Ala Thr Gln Ser Lys Leu Leu Gln
            20                  25                  30

Glu Phe Ile Asn Ile Pro Ser Ile Asp Lys Ala Trp Ile Phe Asn Ser
        35                  40                  45

Asp Ser Gly Ser Gln Ala Met Phe Ala Leu Ser Gln Ala Asn Leu Leu
    50                  55                  60

Ala Asn Lys Lys Lys Lys Phe Met Leu Ser Gly His Ile Ser Asn Glu
65                  70                  75                  80

Ser Asn Gln Ser Val Asn Phe His Trp Ala Pro Phe Pro Ile Glu Met
                85                  90                  95

Thr Gly Ala Ser Ala Phe Val Pro Ser Pro Ser Gly Leu Lys Leu Leu
            100                 105                 110

Val Ile Arg Asn Pro Glu Asn Glu Ser Pro Thr Lys Phe Glu Ile Trp
        115                 120                 125

Asn Ser Ser Gln Leu Glu Lys Glu Phe His Ile Pro Gln Lys Val His
    130                 135                 140

Gly Ser Val Tyr Val Asp Gly Trp Phe Glu Gly Ile Ser Trp Asp Ser
145                 150                 155                 160

Asp Glu Thr His Val Ala Tyr Val Ala Glu Glu Pro Ser Arg Pro Lys
                165                 170                 175

Pro Thr Phe Asp His Leu Gly Tyr Tyr Lys Lys Glu Asn Ser Leu Asp
            180                 185                 190

Lys Gly Ile Gly Ser Trp Lys Gly Glu Gly Asp Trp Glu Glu Glu Trp
        195                 200                 205

Gly Glu Ala Tyr Ala Gly Lys Arg Gln Pro Ala Leu Phe Val Ile Asn
    210                 215                 220

Val Asp Ser Gly Glu Val Glu Pro Ile Lys Gly Ile Pro Arg Ser Ile
225                 230                 235                 240

Ser Val Gly Gln Val Val Trp Ser Pro Asn Ser Asn Gly Ser Ala Gln
                245                 250                 255

Tyr Leu Val Phe Ala Gly Trp Leu Gly Asp Lys Arg Lys Phe Gly Ile
            260                 265                 270

Lys Tyr Cys Tyr Asn Arg Pro Cys Ala Ile Tyr Ala Ile Lys Phe Thr
        275                 280                 285

Ser Asp Glu Pro Lys Asp Asp Asp Ala Asn Glu Phe Pro Ile His Asn

290                 295                 300

Leu Thr Lys Ser Ile Ser Ser Gly Phe Cys Pro Arg Phe Ser Lys Asp
305                 310                 315                 320

Gly Lys Phe Leu Val Phe Val Ser Ala Lys Thr Ala Val Asp Ser Gly
                325                 330                 335

Ala His Trp Ala Thr Glu Ser Leu His Arg Ile Asp Trp Pro Ser Asp
            340                 345                 350

Gly Lys Leu Pro Glu Ser Thr Asn Ile Val Asp Val Ile Gln Val Val
        355                 360                 365

Asn Cys Pro Lys Asp Gly Cys Phe Pro Gly Leu Tyr Val Thr Gly Leu
    370                 375                 380

Leu Ser Asp Pro Trp Leu Ser Asp Gly His Ser Leu Met Leu Ser Thr
385                 390                 395                 400

Tyr Trp Arg Ser Cys Arg Val Ile Leu Ser Val Asn Leu Leu Ser Gly
                405                 410                 415

Glu Val Ser Arg Ala Ser Pro Ser Asp Ser Asp Tyr Ser Trp Asn Ala
            420                 425                 430

Leu Ala Leu Asp Gly Asp Ser Ile Val Ala Val Ser Ser Ser Pro Val
        435                 440                 445

Ser Val Pro Glu Ile Lys Tyr Gly Lys Lys Gly Leu Asp Ser Ala Gly
    450                 455                 460

Lys Pro Ser Trp Leu Trp Ser Asn Ile Gln Ser Pro Ile Arg Tyr Ser
465                 470                 475                 480

Glu Lys Val Met Ala Gly Leu Ser Ser Leu Gln Phe Lys Ile Leu Lys
                485                 490                 495

Val Pro Ile Ser Asp Val Ser Glu Gly Leu Ala Glu Gly Ala Lys Asn
            500                 505                 510

Pro Ile Glu Ala Ile Tyr Val Ser Ser Ser Lys Ser Lys Glu Asn Gly
        515                 520                 525

Lys Cys Asp Pro Leu Ile Ala Val Leu His Gly Gly Pro His Ser Val
    530                 535                 540

Ser Pro Cys Ser Phe Ser Arg Thr Met Ala Tyr Leu Ser Ser Ile Gly
545                 550                 555                 560

Tyr Ser Gln Leu Ile Ile Asn Tyr Arg Gly Ser Leu Gly Tyr Gly Glu
                565                 570                 575

Asp Ala Leu Gln Ser Leu Pro Gly Lys Val Gly Ser Gln Asp Val Lys
            580                 585                 590

Asp Cys Leu Leu Ala Val Asp His Ala Ile Glu Met Gly Ile Ala Asp
        595                 600                 605

Pro Ser Arg Ile Thr Val Leu Gly Gly Ser His Gly Gly Phe Leu Thr
    610                 615                 620

Thr His Leu Ile Gly Gln Ala Pro Asp Lys Phe Val Ala Ala Ala Ala
625                 630                 635                 640

Arg Asn Pro Val Cys Asn Met Ala Ser Met Val Gly Ile Thr Asp Ile
                645                 650                 655

Pro Asp Trp Cys Phe Phe Glu Ala Tyr Gly Asp Gln Ser His Tyr Thr
            660                 665                 670

Glu Ala Pro Ser Ala Glu Asp Leu Ser Arg Phe His Gln Met Ser Pro
        675                 680                 685

Ile Ser His Ile Ser Lys Val Lys Thr Pro Thr Leu Phe Leu Leu Gly
    690                 695                 700

Thr Lys Asp Leu Arg Val Pro Ile Ser Asn Gly Phe Gln Tyr Val Arg
705                 710                 715                 720

Ala Leu Lys Glu Lys Gly Val Glu Val Lys Val Leu Val Phe Pro Asn
                725                 730                 735

Asp Asn His Pro Leu Asp Arg Pro Gln Thr Asp Tyr Glu Ser Phe Leu
            740                 745                 750

Asn Ile Ala Val Trp Phe Asn Lys Tyr Cys Lys Leu
        755                 760

<210>  152
<211>  730
<212>  PRT
<213>  Arabidopsis thaliana

<400>  152

Met Ala Leu Leu Leu Leu Thr Ser Leu Asn His Leu Val Ser Phe Ser
1               5                   10                  15

Leu Thr Arg Leu Pro Ser Ser Ser Ala His Asn Leu Phe Leu Ser Arg
            20                  25                  30

Ser Phe Ser Ser Ser Ile Arg Arg Phe Asn Arg Phe Ser Leu Lys Pro
        35                  40                  45

Leu Arg Ser Phe Ala Ser Met Ser Ser Ser Ser Pro Asp Ala Ala Gln
    50                  55                  60

Thr Pro Leu Thr Thr Ala Pro Tyr Gly Ser Trp Lys Ser Pro Ile Thr
65                  70                  75                  80

Ala Asp Ile Val Ser Gly Ala Ser Lys Arg Leu Gly Gly Thr Ala Val
                85                  90                  95

Asp Ser His Gly Arg Leu Val Leu Leu Glu Ser Arg Pro Asn Glu Ser
            100                 105                 110

Gly Arg Gly Val Leu Val Leu Gln Gly Glu Thr Ser Ile Asp Ile Thr
        115                 120                 125

Pro Lys Asp Phe Ala Val Arg Thr Leu Thr Gln Glu Tyr Gly Gly Gly
    130                 135                 140

Ala Phe Gln Ile Ser Ser Asp Asp Thr Leu Val Phe Ser Asn Tyr Lys
145                 150                 155                 160

Asp Gln Arg Leu Tyr Lys Gln Asp Ile Thr Asp Lys Asp Ser Ser Pro
                165                 170                 175

Lys Pro Ile Thr Pro Asp Tyr Gly Thr Pro Ala Val Thr Tyr Ala Asp
            180                 185                 190

Gly Val Phe Asp Ser Arg Phe Asn Arg Tyr Val Thr Val Arg Glu Asp
        195                 200                 205

Gly Arg Gln Asp Arg Ser Asn Pro Ile Thr Thr Ile Val Glu Val Asn
    210                 215                 220

Leu Ser Gly Glu Thr Leu Glu Glu Pro Lys Val Leu Val Ser Gly Asn
225                 230                 235                 240

Asp Phe Tyr Ala Phe Pro Arg Leu Asp Pro Lys Cys Glu Arg Leu Ala
                245                 250                 255

Trp Ile Glu Trp Ser His Pro Asn Met Pro Trp Asp Lys Ala Glu Leu
            260                 265                 270

Trp Val Gly Tyr Ile Ser Glu Gly Gly Asn Ile Asp Lys Arg Val Cys
        275                 280                 285

Val Ala Gly Cys Asp Pro Lys Tyr Val Glu Ser Pro Thr Glu Pro Lys
    290                 295                 300

Trp Ser Ser Arg Gly Glu Leu Phe Phe Val Thr Asp Arg Lys Asn Gly
305                 310                 315                 320

Cys Trp Asn Ile His Lys Trp Ile Glu Ser Thr Asn Glu Val Val Ser
                325                 330                 335

Val Tyr Pro Leu Asp Gly Glu Phe Ala Lys Pro Leu Trp Ile Phe Gly
            340                 345                 350

Thr Asn Ser Tyr Glu Ile Ile Glu Cys Ser Glu Glu Lys Asn Leu Ile
        355                 360                 365

Ala Cys Ser Tyr Arg Gln Lys Gly Lys Ser Tyr Leu Gly Ile Val Asp
    370                 375                 380

Asp Ser Gln Gly Ser Cys Ser Leu Leu Asp Ile Pro Leu Thr Asp Phe
385                 390                 395                 400

Asp Ser Ile Thr Leu Gly Asn Gln Cys Leu Tyr Val Glu Gly Ala Ser
                405                 410                 415

Ala Val Leu Pro Pro Ser Val Ala Arg Val Thr Leu Asp Gln His Lys
            420                 425                 430

Thr Lys Ala Leu Ser Ser Glu Ile Val Trp Ser Ser Ser Pro Asp Val
        435                 440                 445

Leu Lys Tyr Lys Ala Tyr Phe Ser Val Pro Glu Leu Ile Glu Phe Pro
    450                 455                 460

Thr Glu Val Pro Gly Gln Asn Ala Tyr Ala Tyr Phe Tyr Pro Pro Thr
465                 470                 475                 480

Asn Pro Leu Tyr Asn Ala Ser Met Glu Glu Lys Pro Pro Leu Leu Val
                485                 490                 495

Lys Ser His Gly Gly Pro Thr Ala Glu Ser Arg Gly Ser Leu Asn Leu
            500                 505                 510

Asn Ile Gln Tyr Trp Thr Ser Arg Gly Trp Ala Phe Val Asp Val Asn
        515                 520                 525

Tyr Gly Gly Ser Thr Gly Tyr Gly Arg Glu Tyr Arg Glu Arg Leu Leu
    530                 535                 540

Arg Gln Trp Gly Ile Val Asp Val Asp Asp Cys Cys Gly Cys Ala Lys
545                 550                 555                 560

Tyr Leu Val Ser Ser Gly Lys Ala Asp Val Lys Arg Leu Cys Ile Ser
                565                 570                 575

Gly Gly Ser Ala Gly Gly Tyr Thr Thr Leu Ala Ser Leu Ala Phe Arg
            580                 585                 590

Asp Val Phe Lys Ala Gly Ala Ser Leu Tyr Gly Val Ala Asp Leu Lys
        595                 600                 605

Met Leu Lys Glu Glu Gly His Lys Phe Glu Ser Arg Tyr Ile Asp Asn
    610                 615                 620

Leu Val Gly Asp Glu Lys Asp Phe Tyr Glu Arg Ser Pro Ile Asn Phe
625                 630                 635                 640

Val Asp Lys Phe Ser Cys Pro Ile Ile Leu Phe Gln Gly Leu Glu Asp
                645                 650                 655

Lys Val Val Thr Pro Asp Gln Ser Arg Lys Ile Tyr Glu Ala Leu Lys
            660                 665                 670

Lys Lys Gly Leu Pro Val Ala Leu Val Glu Tyr Glu Gly Glu Gln His
        675                 680                 685

Gly Phe Arg Lys Ala Glu Asn Ile Lys Tyr Thr Leu Glu Gln Gln Met
    690                 695                 700

Val Phe Phe Ala Arg Val Val Gly Gly Phe Lys Val Ala Asp Asp Ile
705                 710                 715                 720

Thr Pro Leu Lys Ile Asp Asn Phe Asp Thr
                725                 730

<210>  153
<211>  721
<212>  PRT
<213>  Picea sitchensis

<400>  153

Met Ala Ser Phe Ser Phe Ser Cys Ala Tyr Leu Arg Pro Leu Phe Tyr
1               5                   10                  15

Cys Arg Ser Ile Thr Ser Arg Arg Gly Ile Tyr Thr Val Asn Cys Leu
            20                  25                  30

Arg Gln Ser Ser Ala Asp His Lys Thr Thr Asp Met Tyr Ala Glu Arg
        35                  40                  45

Ile Glu Val His Lys Glu Lys Met Ser Ala Pro Tyr Gly Ser Trp Lys
    50                  55                  60

Ser Pro Ile Thr Ala Asp Ile Val Ser Gly Ala Asp Lys Arg Leu Gly
65                  70                  75                  80

Gly Phe Ala Leu Asp Gly Glu Gly Arg Val Ile Trp Leu Glu Gly Arg
                85                  90                  95

Pro Thr Glu Ala Gly Arg Ser Val Leu Val Arg Glu Ala Ala Asp Glu
            100                 105                 110

Glu Gly Thr Ala Glu Asp Ile Thr Pro Ala Gly Phe Asn Val Arg Thr
        115                 120                 125

Leu Val His Glu Tyr Gly Gly Gly Ala Phe Thr Val Ser Gly Asp Val
    130                 135                 140

Val Val Phe Ser Asn Tyr Lys Asp Gln Arg Leu Tyr Lys Gln Ser Ile
145                 150                 155                 160

Lys Gly Gly His Ala Pro Ile Ala Leu Thr Pro Asp Tyr Gly Ala Pro
                165                 170                 175

Val Val Arg Tyr Ala Asp Gly Val Met Asp Leu His Leu Gly Cys Tyr
            180                 185                 190

Val Thr Val Arg Glu Asp His Arg Glu Ser Asp Thr Asn Pro Thr Thr
        195                 200                 205

Thr Ile Val Ser Val Glu Leu Asn Gly Ala Gly Thr Thr Glu Pro His
    210                 215                 220

Val Leu Val Ser Gly Ser Asp Phe Tyr Ala Phe Pro Arg Leu Thr Pro
225                 230                 235                 240

Asp Gly Gly Lys Met Ala Trp Ile Glu Trp Asn His Pro Asn Met Pro

245                 250                 255

Trp Asp Lys Ser Glu Leu Trp Val Gly Tyr Met Ser Ala Glu Gly Lys
            260                 265                 270

Val Glu Lys Arg Ile Cys Ile Ala Gly Asn Asp Pro Asn Met Ile Glu
        275                 280                 285

Ser Pro Thr Glu Pro Lys Trp Ser Ser Gln Gly Glu Leu Phe Phe Val
    290                 295                 300

Thr Asp Arg Lys Ser Gly Phe Trp Asn Leu Tyr Lys Trp Val Glu Ser
305                 310                 315                 320

Thr Asn Glu Val Lys Ala Leu Tyr Pro Leu Asp Ala Glu Phe Thr Arg
                325                 330                 335

Pro Ser Trp Val Phe Gly Asn Ser Ser Tyr Ala Phe Ile Glu Gln Lys
            340                 345                 350

Gly Gln Asn Lys Asn Ile Ala Cys Thr Tyr Arg Gln Lys Gly Met Ser
        355                 360                 365

Tyr Leu Gly Ile Leu Asp His Val Leu Gly Ser Phe Ser Leu Val Asp
    370                 375                 380

Leu Pro Phe Thr Asp Ile Tyr Asn Ile Thr Ser Ile Gly Ser His Leu
385                 390                 395                 400

Tyr Leu Glu Gly Ala Ser Pro Leu His Pro Leu Ser Ile Val Lys Val
                405                 410                 415

Ser Tyr Glu Glu Asn Leu Ile Ala Val Arg Gly Ile Ser Ile Ile Trp
            420                 425                 430

Ser Ser Ser Ser Leu Asn Ile Ser Glu Tyr Ser Ala Phe Ile Ser Ser
        435                 440                 445

Pro Glu Ile Val Glu Phe Ser Thr Lys Val Pro Gly Gln Thr Ala Phe
    450                 455                 460

Ala Tyr Leu Tyr Leu Pro Ser Asn Tyr Asn Tyr Glu Ala Pro Glu Gly
465                 470                 475                 480

Glu Lys Pro Pro Leu Leu Val Lys Ser His Gly Gly Pro Thr Ser Glu
                485                 490                 495

Ser His Ser Ala Leu Asp Leu Ser Ile Gln Tyr Trp Thr Ser Arg Gly
        500                 505                 510

Trp Ala Phe Ala Asp Val Asn Tyr Gly Gly Ser Thr Gly Tyr Gly Arg
    515                 520                 525

Glu Tyr Arg Glu Arg Leu Asn Gly Ser Trp Gly Ile Val Asp Val Asn
    530                 535                 540

Asp Cys Cys Ser Cys Ala Glu Phe Leu Val Thr Thr Gly Arg Val Asp
545                 550                 555                 560

Gly Glu Arg Leu Cys Ile Thr Gly Arg Ser Ala Gly Gly Tyr Thr Thr
                565                 570                 575

Leu Ala Ala Leu Val Phe Arg Glu Thr Phe Lys Ala Gly Ala Ser Leu
            580                 585                 590

Phe Gly Val Ala Asp Val Ser Leu Leu Lys Ala Asp Thr His Lys Phe
        595                 600                 605

Glu Ser Tyr Tyr Thr Asp Ser Leu Val Gly Lys Asp Glu Ser Leu Leu
    610                 615                 620

Tyr Glu Arg Ser Pro Ile Asn Phe Val Asp Arg Leu Ser Cys Pro Met
625                 630                 635                 640

Ile Leu Phe Gln Gly Leu Glu Asp Lys Val Val Pro Pro Glu Gln Ala
                645                 650                 655

Arg Lys Ile Tyr Ala Ala Val Lys Glu Lys Gly Leu Pro Val Ala Leu
            660                 665                 670

Val Glu Tyr Glu Gly Glu Gln His Gly Phe Arg Lys Ala Glu Asn Ile
        675                 680                 685

Lys Tyr Thr Leu Glu Gln Gln Met Val Phe Phe Ala Arg Leu Ile Gly
    690                 695                 700

Asn Phe Lys Val Ala Asp Asp Ile Ile Pro Val His Ile Glu Asn Phe
705                 710                 715                 720

Asp

<210>  154
<211>  203
<212>  PRT

<213>  Zea mays

<400>  154

Met Cys Thr Val Asp Met Arg Ala Gln Tyr Leu Arg Ser Lys Gly Ile
1               5                   10                  15

Leu Val Trp Lys Met Asp Asn Arg Gly Ser Ala Arg Arg Gly Leu His
            20                  25                  30

Phe Glu Gly Gln Leu Lys Tyr Asn Ile Gly Arg Val Asp Ala Glu Asp
        35                  40                  45

Gln Leu Glu Gly Ala Glu Trp Leu Ile Lys Lys Gly Leu Ala Lys Pro
    50                  55                  60

Gly His Ile Gly Ile Tyr Gly Trp Ser Tyr Gly Gly Phe Leu Ser Ala
65                  70                  75                  80

Met Cys Leu Ala Arg Phe Pro Asp Thr Phe Cys Cys Ala Val Ser Gly
                85                  90                  95

Ala Pro Val Thr Ala Trp Asp Gly Tyr Asp Thr Phe Tyr Thr Glu Lys
            100                 105                 110

Tyr Leu Gly Leu Pro Ala Glu His Pro Asp Ala Tyr Glu Tyr Gly Ser
        115                 120                 125

Ile Met Tyr His Ala Lys Asn Leu Lys Gly Lys Leu Leu Leu Ile His
    130                 135                 140

Gly Met Ile Asp Glu Asn Val His Phe Arg His Thr Ala Arg Leu Ile
145                 150                 155                 160

Asn Ser Leu Met Ala Glu Gly Lys Pro Tyr Glu Ile Leu Leu Phe Pro
                165                 170                 175

Asp Glu Arg His Met Pro Arg Arg Leu Gly Asp Arg Ile Tyr Met Glu
            180                 185                 190

Glu Arg Ile Phe Gly Phe Phe Glu Arg Ser Leu
        195                 200


<210>  155
<211>  1133
<212>  DNA
<213>  Arabidopsis thaliana

<400>  155
aaccttactc ctcctcctct tcctctttct ctaatcggca aaattttctg ctcctgagaa      60

acaagtagag atactaaaga tggaatcttt gaactaaatt cgaaaccttt taatgggtac 120

cgagtcgggt tcggatccag aatcgagctc aaacgggtgg agccgtgctc gtggtctagt 180

tgtcaagact ctggttttaa ttggcggtgc tcttctcatc aagcgtctca ctaaatccac 240

cactcgtaga gatcacgccc gtgtcgtctc tcgttctctc accggagaga agtttacgag 300

ggagcaagcg tcaagagatc ctgataatta cttcaacata agaatgctga gttgtccagc 360

tgctgaaatg gtggatggtt cagaggtttt gtatctcgaa caggcatttt ggaggactcc 420

tcagaaaccg tttcgacaaa gattatatat ggtgaaaccg tgtccaaaag agctaaaatg 480

tgatgttgag gtaagttcat atgcgatcag agatgctgag gaatacaaaa atttctgtga 540

ccggcctaag gaccaacgcc cacttcctga agaagttatt ggtgacattg gagagcattt 600

gacgactata cacctgaatt gttgtgaccg tgggaaacgt tgcttgtacg aaggctcaac 660

ttcacctggt ggatttccaa attcatggaa cggggctagc tattgtactt cagatcttgc 720

agttctgaaa aacaatgaga tacacctctg ggatcgcggc tttgatgaga atcgaaacca 780

ggtttgggga ccaaaggaag gtccgtacga gttcaagcca gcgacatcat cgagcatcaa 840

cgaaaacttg tctgctttga acatccttta tcaatcttct atcgataaac caatccaagg 900

gtccctcatc ttgcaagact agccaatttg cacccccttt tctattgtct gatcttctct 960

gtatcacact tagcctcttt attcatctca tctcagccac tttcaatata gttttggtac 1020

atagaaagct caatacttta cttcattaca gtagtggtca acaagtcgaa gatgaatgtt 1080

tatacagaac aaaaaaatga ttccaaaagc agagtgtagt ctctcttgat act 1133

<210> 156
<211> 269
<212> PRT
<213> Arabidopsis thaliana

<400> 156

Met Gly Thr Glu Ser Gly Ser Asp Pro Glu Ser Ser Ser Asn Gly Trp
1               5                   10                  15

Ser Arg Ala Arg Gly Leu Val Val Lys Thr Leu Val Leu Ile Gly Gly
            20                  25                  30

Ala Leu Leu Ile Lys Arg Leu Thr Lys Ser Thr Thr Arg Arg Asp His
        35                  40                  45

Ala Arg Val Val Ser Arg Ser Leu Thr Gly Glu Lys Phe Thr Arg Glu
    50                  55                  60

Gln Ala Ser Arg Asp Pro Asp Asn Tyr Phe Asn Ile Arg Met Leu Ser
65                  70                  75                  80

Cys Pro Ala Ala Glu Met Val Asp Gly Ser Glu Val Leu Tyr Leu Glu
                85                  90                  95

Gln Ala Phe Trp Arg Thr Pro Gln Lys Pro Phe Arg Gln Arg Leu Tyr
            100                 105                 110

Met Val Lys Pro Cys Pro Lys Glu Leu Lys Cys Asp Val Glu Val Ser
        115                 120                 125

Ser Tyr Ala Ile Arg Asp Ala Glu Glu Tyr Lys Asn Phe Cys Asp Arg
    130                 135                 140

Pro Lys Asp Gln Arg Pro Leu Pro Glu Glu Val Ile Gly Asp Ile Gly
145                 150                 155                 160

Glu His Leu Thr Thr Ile His Leu Asn Cys Cys Asp Arg Gly Lys Arg
                165                 170                 175

Cys Leu Tyr Glu Gly Ser Thr Ser Pro Gly Gly Phe Pro Asn Ser Trp
            180                 185                 190

Asn Gly Ala Ser Tyr Cys Thr Ser Asp Leu Ala Val Leu Lys Asn Asn
        195                 200                 205

Glu Ile His Leu Trp Asp Arg Gly Phe Asp Glu Asn Arg Asn Gln Val
    210                 215                 220

Trp Gly Pro Lys Glu Gly Pro Tyr Glu Phe Lys Pro Ala Thr Ser Ser
225                 230                 235                 240

Ser Ile Asn Glu Asn Leu Ser Ala Leu Asn Ile Leu Tyr Gln Ser Ser
                245                 250                 255

Ile Asp Lys Pro Ile Gln Gly Ser Leu Ile Leu Gln Asp
            260                 265

<210>  157
<211>  1060
<212>  DNA
<213>  Brassica napus

<400>  157
cgttctcctc atccatcttc tgctctcacg agctttttga ttcggctaaa gctaaggcct      60

ttctttcgat cttgaggaaa cgtcagatgc taacgttaca aggattgaac tgagacgaaa     120

ggaagcttct ttctttttat gggtaccggg tcaggttcgg atccggagtc gagttcgtcc     180

gggtggagca gggctcctgg tttggtagtg aagacgctgg ttctgatcgg cggcgccgtt     240

ctcctgaagc gtctcacgaa atccaccact cgctgggacc actctcacgt cgtctctcgc 300

tctctcagcg gcgaaaagtt ttctaaggag caagcatcaa gggatcctga taattacttc 360

aacataagaa tgatgagctg cccagcagct gagatggtgg atggttcgca ggttttgtat 420

ctcgaacagg cattttggag aactcctcaa aaaccctttc ggcaaagatt gtatatggtt 480

aagccttgcc caaaggaact gaaatgtgat gttgaggtga gctcatatgc aatcagagat 540

gctgaggagt acaaaaattt ctgtgaccgc ccgaaggacc aacgcccact tccggaagaa 600

gttattggtg acataggaga gcatttgaca accatacaac ttagctgttg tgaccgcgga 660

aagcgttgct tgtatgaagg atcagctcca cctggtggtt tcccaaattc atggaatggt 720

gcaagctatt gtacatctga tcttacagtc ctgaaaaaca atgagataca tctctgggat 780

cgcgggtttg atgatgatgg aaaccaggtg tggggaccaa aggaaggccc gtacgagttc 840

aaaccggcgc cttcatcgtc aagcatcaac agcgatgtgt tctctccttt gaatatgttt 900

cctcaatctg cacttgataa accaatcaaa ggatctttca ttttgcaaga gtaggtaaag 960

gaaggccgtt cccgcttttg ttttctgtct ttgtaattct ctatgtagtg gcaaccaaca 1020

ctcacttatg tatcaccatc atatacagtt tttacttgct 1060

<210> 158
<211> 271
<212> PRT
<213> Brassica napus

<400> 158

Met Gly Thr Gly Ser Gly Ser Asp Pro Glu Ser Ser Ser Ser Gly Trp
1               5                   10                  15

Ser Arg Ala Pro Gly Leu Val Val Lys Thr Leu Val Leu Ile Gly Gly
            20                  25                  30

Ala Val Leu Leu Lys Arg Leu Thr Lys Ser Thr Thr Arg Trp Asp His
        35                  40                  45

Ser His Val Val Ser Arg Ser Leu Ser Gly Glu Lys Phe Ser Lys Glu
    50                  55                  60

Gln Ala Ser Arg Asp Pro Asp Asn Tyr Phe Asn Ile Arg Met Met Ser
65                  70                  75                  80

Cys Pro Ala Ala Glu Met Val Asp Gly Ser Gln Val Leu Tyr Leu Glu
                85                  90                  95

Gln Ala Phe Trp Arg Thr Pro Gln Lys Pro Phe Arg Gln Arg Leu Tyr
            100                 105                 110

Met Val Lys Pro Cys Pro Lys Glu Leu Lys Cys Asp Val Glu Val Ser
        115                 120                 125

Ser Tyr Ala Ile Arg Asp Ala Glu Glu Tyr Lys Asn Phe Cys Asp Arg
    130                 135                 140

Pro Lys Asp Gln Arg Pro Leu Pro Glu Glu Val Ile Gly Asp Ile Gly
145                 150                 155                 160

Glu His Leu Thr Thr Ile Gln Leu Ser Cys Cys Asp Arg Gly Lys Arg
                165                 170                 175

Cys Leu Tyr Glu Gly Ser Ala Pro Pro Gly Gly Phe Pro Asn Ser Trp
            180                 185                 190

Asn Gly Ala Ser Tyr Cys Thr Ser Asp Leu Thr Val Leu Lys Asn Asn
        195                 200                 205

Glu Ile His Leu Trp Asp Arg Gly Phe Asp Asp Asp Gly Asn Gln Val
    210                 215                 220

Trp Gly Pro Lys Glu Gly Pro Tyr Glu Phe Lys Pro Ala Pro Ser Ser
225                 230                 235                 240

Ser Ser Ile Asn Ser Asp Val Phe Ser Pro Leu Asn Met Phe Pro Gln
                245                 250                 255

Ser Ala Leu Asp Lys Pro Ile Lys Gly Ser Phe Ile Leu Gln Glu
            260                 265                 270

<210>  159
<211>  1176
<212>  DNA
<213>  Citrus clementina

<400>  159
caatttcatc atcataaaat ctttatgttc ccattattca aaaaaaaaaa aaaaaaagag      60

gcgaattatt gaactggata ctgctccgtt gactgttgtt gatacgaatt tgttgaatgt     120

ggtggtggtg gtggtgtagg tgagacgaga ccaaaacgaa ttggttgctt agttgctgct     180

agctacatgg gcccggactc ggactcggcc tcaaacccga acggaggatg gggcgggcga     240

gctcaaggtc tcttcgtgaa agcagcggtg cttattggcg gcgctgttct cctcaaacga     300

ctcaccaaat ccaagactcg ctgggaccat gcccgtattg ttgctgactc tctttctggc     360

gagaagtttt cgaaagaaca ggcatcgaga gatcctgata atttcttcaa catcagaatg     420

cttacttgcc cggcagctga aatggttgat ggttcgaagc ttttatatct tgagcaagca     480

ttttggagga ctcctcagaa accctttcgg cagaggtttt atatggtgaa gccttgccca 540

aaagaattga aatgcgacgt tgaggtaagt tcatatgcga ttagagaagt tgaggaatat 600

aaaaatttct gtgatcgtcc tagggatcag cgcccgctac ctgaagaagt tattggtgac 660

attggggaac atttgacaac tatacatctc agacgctgtg atcgtggaaa acgatgctta 720

tatgagggct caactccacc aggcggattc ccaaattcat ggcagaatgg agcaacctac 780

tctacctcgg agcttgcggt cttgaaaaat aatgaaatac acacctggga tagaggcttt 840

gacgatgatg gaaatcaagt ctggggagtg aaggcaggtc catatgaatt caagccggcg 900

cctagttcta gttatagtga catgttctcc ccccttgaat ttccctcccc agcagttctt 960

ttaaaaagag atttgaagga tccttttgtc ttgccaaaaa tgataacctc ggtacatggt 1020

aactaaaatt tatgtaaatt ttaccaactt ttcccttttt ccggggtaaa tttaaaccaa 1080

aaaattcccc ttttcctgtt tccccctatt ccccttttgg ttcatggctt gatttttttg 1140

ggcccaaaat taaattttcc ccccaacccc ccctaa 1176

<210> 160
<211> 279
<212> PRT
<213> Citrus clementina

<400> 160

Met Gly Pro Asp Ser Asp Ser Ala Ser Asn Pro Asn Gly Gly Trp Gly
1               5                   10                  15

Gly Arg Ala Gln Gly Leu Phe Val Lys Ala Ala Val Leu Ile Gly Gly
            20                  25                  30

Ala Val Leu Leu Lys Arg Leu Thr Lys Ser Lys Thr Arg Trp Asp His
        35                  40                  45

Ala Arg Ile Val Ala Asp Ser Leu Ser Gly Glu Lys Phe Ser Lys Glu
    50                  55                  60

Gln Ala Ser Arg Asp Pro Asp Asn Phe Phe Asn Ile Arg Met Leu Thr
65                  70                  75                  80

Cys Pro Ala Ala Glu Met Val Asp Gly Ser Lys Leu Leu Tyr Leu Glu
                85                  90                  95

Gln Ala Phe Trp Arg Thr Pro Gln Lys Pro Phe Arg Gln Arg Phe Tyr
            100                 105                 110

Met Val Lys Pro Cys Pro Lys Glu Leu Lys Cys Asp Val Glu Val Ser
        115                 120                 125

Ser Tyr Ala Ile Arg Glu Val Glu Glu Tyr Lys Asn Phe Cys Asp Arg
    130                 135                 140

Pro Arg Asp Gln Arg Pro Leu Pro Glu Glu Val Ile Gly Asp Ile Gly
145                 150                 155                 160

Glu His Leu Thr Thr Ile His Leu Arg Arg Cys Asp Arg Gly Lys Arg
                165                 170                 175

Cys Leu Tyr Glu Gly Ser Thr Pro Pro Gly Gly Phe Pro Asn Ser Trp
            180                 185                 190

Gln Asn Gly Ala Thr Tyr Ser Thr Ser Glu Leu Ala Val Leu Lys Asn
        195                 200                 205

Asn Glu Ile His Thr Trp Asp Arg Gly Phe Asp Asp Asp Gly Asn Gln
    210                 215                 220

Val Trp Gly Val Lys Ala Gly Pro Tyr Glu Phe Lys Pro Ala Pro Ser
225                 230                 235                 240

Ser Ser Tyr Ser Asp Met Phe Ser Pro Leu Glu Phe Pro Ser Pro Ala
                245                 250                 255

Val Leu Leu Lys Arg Asp Leu Lys Asp Pro Phe Val Leu Pro Lys Met
            260                 265                 270

Ile Thr Ser Val His Gly Asn
        275

<210>  161
<211>  1072
<212>  DNA
<213>  Cichorium endivia

<400>  161
tatcatgata ttacagacgc aatcacagta aaaatctctc tcccaaaaga aaatcccaac      60

tccttttccc cattggattt caatccccct ttttataaac agtttctttc tgcacctgaa     120

tctaaaccct agattgagta ctccgtagat acgtatatat aaaatttcga cacacacggt     180

atgtgtatgg gaccaccaag tgggtggagc agagctcgag ggttagtggt gaagacgctg     240

gttttaatcg gaggtgctct tctgatcaag agattgacca agtctaccac tcgttgggac     300

catgctcgca tcgtttctca atccatcacc ggcgaaaagt tctcaaagga acaagcatct     360

agagatcccg ataatttctt taatttaaga tggctttcat gcccagctgc agatatggtg     420

gatggatcaa aggttctata tttcgagcaa gcattttgga gaactcccca aaagccattc     480

agacagagat tttgtatggt gaaaccttgc ccaaaggaga tgaaatgtga tgttgagtta 540

agtacatatg ccattcggga tgcagaggag tacaagaact tttgtgatcg accaagggac 600

caacgtccac agcctgaaga agtaattggg gatgtagcag aacatttgac caccatatat 660

ctcaagcgct gtgaaagagg gaagcggtgt ttgtacgagg gttcaacacc acccgacggt 720

ttcccaaatt catggaatgg tgcagcatat tgtacctcag aactggcagt cttgaagaac 780

aacgaggttc atatgtggga taggggctat gatgacgatg gaaaccaagt ttggggagta 840

aagaatggtc cttatgaatt caaggctgca cctggacctg gaccaggacc tgcatctaca 900

tctgttgata tgttatctcc tttaaatttc cctcctcttt ctatcggtaa aaggatagaa 960

ggttcttttg ttcttcaaga ataatgacat gtgttgcatt gcttgtaata tatagctaca 1020

aagtgtaaca ttcaatattt ttattcaata taccaatttc gtcttctcaa aa 1072

<210> 162
<211> 267
<212> PRT
<213> Cichorium endivia

<400> 162

Met Cys Met Gly Pro Pro Ser Gly Trp Ser Arg Ala Arg Gly Leu Val
1               5                   10                  15

Val Lys Thr Leu Val Leu Ile Gly Gly Ala Leu Leu Ile Lys Arg Leu
            20                  25                  30

Thr Lys Ser Thr Thr Arg Trp Asp His Ala Arg Ile Val Ser Gln Ser
        35                  40                  45

Ile Thr Gly Glu Lys Phe Ser Lys Glu Gln Ala Ser Arg Asp Pro Asp
    50                  55                  60

Asn Phe Phe Asn Leu Arg Trp Leu Ser Cys Pro Ala Ala Asp Met Val
65                  70                  75                  80

Asp Gly Ser Lys Val Leu Tyr Phe Glu Gln Ala Phe Trp Arg Thr Pro
                85                  90                  95

Gln Lys Pro Phe Arg Gln Arg Phe Cys Met Val Lys Pro Cys Pro Lys
            100                 105                 110

Glu Met Lys Cys Asp Val Glu Leu Ser Thr Tyr Ala Ile Arg Asp Ala
        115                 120                 125

Glu Glu Tyr Lys Asn Phe Cys Asp Arg Pro Arg Asp Gln Arg Pro Gln
    130                 135                 140

Pro Glu Glu Val Ile Gly Asp Val Ala Glu His Leu Thr Thr Ile Tyr
145                 150                 155                 160

Leu Lys Arg Cys Glu Arg Gly Lys Arg Cys Leu Tyr Glu Gly Ser Thr
                165                 170                 175

Pro Pro Asp Gly Phe Pro Asn Ser Trp Asn Gly Ala Ala Tyr Cys Thr
            180                 185                 190

Ser Glu Leu Ala Val Leu Lys Asn Asn Glu Val His Met Trp Asp Arg
        195                 200                 205

Gly Tyr Asp Asp Asp Gly Asn Gln Val Trp Gly Val Lys Asn Gly Pro
    210                 215                 220

Tyr Glu Phe Lys Ala Ala Pro Gly Pro Gly Pro Gly Pro Ala Ser Thr
225                 230                 235                 240

Ser Val Asp Met Leu Ser Pro Leu Asn Phe Pro Pro Leu Ser Ile Gly
                245                 250                 255

Lys Arg Ile Glu Gly Ser Phe Val Leu Gln Glu
            260                 265

<210>  163
<211>  1343
<212>  DNA
<213>  Citrus sinensis

<400>  163
tttttcacta gcggacgcca agagccaaaa ctccaattaa aatcagacaa tttcatcatc      60

ataaaatctt tatgttccca ttattccaaa aaaaaaaaaa ggcgaattat tgaactggat     120

actgctccgt tgactgttgt tgatacgaat ttgttgaatg tggtggtggt ggtgtcggtg     180

agacgagacc aaaacgaatt ggttgcttag ttgctgctag ctacatgggc ccggactcgg     240

actcggactc ggcctcaaac ccgaacggag gatggggcgg ccgagctcaa ggtctcttcg     300

tgaaagcagc ggtgcttatt ggcggcgctg ttctcctcaa acgactcacc aaatccaaga     360

cccgctggga ccatgcccgt attgttgctg actctctgtc tggcgagaag ttttcgaaag     420

aacaggcatc gagagatcct gataatttct tcaacatcag aatgcttact tgcccggcag     480

ctgaaatggt tgatggttcg aagcttttat atcttgagca agcattttgg aggactcctc     540

agaaaccctt tcggcagagg ttttatatgg tgaagccttg cccaaaagaa ttgaaatgcg     600

acgttgaggt aagttcatat gcgattagag aagttgagga atataaaaat ttctgtgatc     660

gtcctaggga tcagcgcccg ctaccagaag aagttattgg tgacattggg gaacatttga     720

caactataca tctcagacgc tgtgatcgtg gaaaacgatg cttatatgag ggctcaactc 780

caccaggcgg attcccaaat tcatggcaga atggagcaac ctactctacc tcggagcttg 840

cggtcttgaa aaataatgaa atacacacct gggatagagg ctttgacgat gatggaaatc 900

aagtctgggg agtgaaggca ggtccatatg aattcaagcc ggcgcctagt tctagttata 960

gtgacatgtt ctcccccttg aatttccctc cacagcagtt cttggagaag agaattgaag 1020

gatcctttgt cttgcaagaa tgataactcg gtacatgtaa ctaaagtatg taaatttaca 1080

actttcactt atctggtaaa ttaaacaaga aatccctttc tgttctctat caactttgtc 1140

atgctgatct tggccgagta atatctcaac cactaatcag cttgcaccgg agaaataggt 1200

gtctggtagg gcaccgtcgg atccattatt caccatagaa tttggaagca agtgtagtga 1260

cctaataaac acagtttgga ggagggtttt cgatcatgta atctgcatca gttgtattat 1320

agggaaatta aagttgaaaa aaa 1343

<210> 164
<211> 272
<212> PRT
<213> Citrus sinensis

<400> 164

Met Gly Pro Asp Ser Asp Ser Asp Ser Ala Ser Asn Pro Asn Gly Gly
1               5                   10                  15

Trp Gly Gly Arg Ala Gln Gly Leu Phe Val Lys Ala Ala Val Leu Ile
            20                  25                  30

Gly Gly Ala Val Leu Leu Lys Arg Leu Thr Lys Ser Lys Thr Arg Trp
        35                  40                  45

Asp His Ala Arg Ile Val Ala Asp Ser Leu Ser Gly Glu Lys Phe Ser
    50                  55                  60

Lys Glu Gln Ala Ser Arg Asp Pro Asp Asn Phe Phe Asn Ile Arg Met
65                  70                  75                  80

Leu Thr Cys Pro Ala Ala Glu Met Val Asp Gly Ser Lys Leu Leu Tyr
                85                  90                  95

Leu Glu Gln Ala Phe Trp Arg Thr Pro Gln Lys Pro Phe Arg Gln Arg
            100                 105                 110

Phe Tyr Met Val Lys Pro Cys Pro Lys Glu Leu Lys Cys Asp Val Glu
        115                 120                 125

Val Ser Ser Tyr Ala Ile Arg Glu Val Glu Glu Tyr Lys Asn Phe Cys
    130                 135                 140

Asp Arg Pro Arg Asp Gln Arg Pro Leu Pro Glu Glu Val Ile Gly Asp
145                 150                 155                 160

Ile Gly Glu His Leu Thr Thr Ile His Leu Arg Arg Cys Asp Arg Gly
                165                 170                 175

Lys Arg Cys Leu Tyr Glu Gly Ser Thr Pro Pro Gly Gly Phe Pro Asn
            180                 185                 190

Ser Trp Gln Asn Gly Ala Thr Tyr Ser Thr Ser Glu Leu Ala Val Leu
        195                 200                 205

Lys Asn Asn Glu Ile His Thr Trp Asp Arg Gly Phe Asp Asp Asp Gly
    210                 215                 220

Asn Gln Val Trp Gly Val Lys Ala Gly Pro Tyr Glu Phe Lys Pro Ala
225                 230                 235                 240

Pro Ser Ser Ser Tyr Ser Asp Met Phe Ser Pro Leu Asn Phe Pro Pro
                245                 250                 255

Gln Gln Phe Leu Glu Lys Arg Ile Glu Gly Ser Phe Val Leu Gln Glu
            260                 265                 270

<210>  165
<211>  801
<212>  DNA
<213>  Glycine max

<400>  165
atgggtaccg gggattcgga ttcggaatcg aatggatgga accgtgctcg tgggttggcg      60

cttaagactc tgctactaat tggtggcgca cttctcgtta agcgcctccg caagtccacc     120

acgcgttggg accacgctca tttcgtctcc aactccctca ccggcgaaaa gtattccaag     180

gagcaagctt ccagagaccc ggataactat ttcaacatta gaatgcttac atgccccgca     240

gcggagctag tggatggttc caaggtcttg tattttgaac aggctttttg gaggactcca     300

caaaaaccct ttcggcagag gctttttatg gtgaaacctt gtcctaaaga gttgaaatgt     360

gatgttgagt taagtacata tgccattaga gacatggagg agtacaaaaa tttctgtgat     420

cgaccaaggg atcagcgtcc acagccggaa gaagtcattg gagatattgc tgaacatttg     480

acaacagtac atcttaagcg ttgtccacgt ggaaaacgtt gcttatatga aggttcaacc     540

ccacctggtg gatttcctaa ttcatggaat ggggcaacct actgtacttc agagcttgcg     600

attttaaaga acaatgagat acatacctgg gacaggggtt atgatgatgg tggaaatcaa     660

gtttgggggc aaaaagaagg cccttacgag ttcaagcctg caccaacctc cagttttaat     720

gatatgtttt ctcctttgaa tttcccccct ccaccatcca tggagagaag aatagagggt     780

tcatttgttt tgcaagaatg a                                              801

<210>  166
<211>  266
<212>  PRT
<213>  Glycine max

<400>  166

Met Gly Thr Gly Asp Ser Asp Ser Glu Ser Asn Gly Trp Asn Arg Ala
1               5                   10                  15

Arg Gly Leu Ala Leu Lys Thr Leu Leu Leu Ile Gly Gly Ala Leu Leu
            20                  25                  30

Val Lys Arg Leu Arg Lys Ser Thr Thr Arg Trp Asp His Ala His Phe
        35                  40                  45

Val Ser Asn Ser Leu Thr Gly Glu Lys Tyr Ser Lys Glu Gln Ala Ser
    50                  55                  60

Arg Asp Pro Asp Asn Tyr Phe Asn Ile Arg Met Leu Thr Cys Pro Ala
65                  70                  75                  80

Ala Glu Leu Val Asp Gly Ser Lys Val Leu Tyr Phe Glu Gln Ala Phe
                85                  90                  95

Trp Arg Thr Pro Gln Lys Pro Phe Arg Gln Arg Leu Phe Met Val Lys
            100                 105                 110

Pro Cys Pro Lys Glu Leu Lys Cys Asp Val Glu Leu Ser Thr Tyr Ala
        115                 120                 125

Ile Arg Asp Met Glu Glu Tyr Lys Asn Phe Cys Asp Arg Pro Arg Asp
    130                 135                 140

Gln Arg Pro Gln Pro Glu Glu Val Ile Gly Asp Ile Ala Glu His Leu
145                 150                 155                 160

Thr Thr Val His Leu Lys Arg Cys Pro Arg Gly Lys Arg Cys Leu Tyr
                165                 170                 175

Glu Gly Ser Thr Pro Pro Gly Gly Phe Pro Asn Ser Trp Asn Gly Ala
            180                 185                 190

Thr Tyr Cys Thr Ser Glu Leu Ala Ile Leu Lys Asn Asn Glu Ile His
        195                 200                 205

Thr Trp Asp Arg Gly Tyr Asp Asp Gly Gly Asn Gln Val Trp Gly Gln
    210                 215                 220

Lys Glu Gly Pro Tyr Glu Phe Lys Pro Ala Pro Thr Ser Ser Phe Asn
225                 230                 235                 240

Asp Met Phe Ser Pro Leu Asn Phe Pro Pro Pro Pro Ser Met Glu Arg
                245                 250                 255

Arg Ile Glu Gly Ser Phe Val Leu Gln Glu
            260                 265

<210> 167
<211> 1147
<212> DNA
<213> Gossypium raimondii

<400> 167
cttcatttct gaaaaaggaa gaggaataat aatatggtgg atgtttggat gaggtgagaa 60

gattaaggaa ttaattacaa ataaatatat aaaaattata tgggaaggaa ttcagagtcg 120

gactcgaatg gatggagtcg agctcgaggt ttggtagtga agacgctggt gttaattgga 180

ggcgcccttt tgctcaagag gtttaccaaa tccaccactc gttgggacca cgctagaatc 240

gttgctcgtt ctcttagcgg cgaaaagttc tcgcgggagc aagcctctag aaatccagat 300

agttacttca acatcagaac acttacttgc ccagcaacgg agatggtgga tggttcaaat 360

gttttatatt tcgaacaggc attttggagg actccccaga aacctttcgg gcagaggttt 420

ttcatggtga agccttgtcc aaaggatttg aaatgtgatg ttgaggtaag ttcttacgca 480

attagagatg cagatgaata caggaatttc tgtgatcgtc caaggatcaa atgcccacca 540

cctgaagaag ttattgatga tgttgctgaa catctgacaa ctatatatct caaacgctgt 600

gagaggggga aacgctgttt atacgaaggt tcaactccac caggtggatt cccaaattca 660

tggaatggag caacatactg cacttcagaa cttacaattt tgaagaacaa tgagatacat 720

acctgggata ggggttatga cgatgatgga aatcaggttt ggggagtgaa ggaaggtcct 780

tatgaattca agcctgcacc tgcctctagt ttcaatggta tgttttcacc actaaatttt 840

gccccttcac agccaatgga gaaaaggata gagggatcgt ttgtcttgca agaatgattc 900

atggttatat ataaatatat atggtcatag tctgtaaaat tttaactcct ttacataatc 960

ttgagtgaag acccttggct ttactcttgg ctatattttg tcttttattt gggtgcaatt 1020

ttctgggttt ttttggtcat taatttacag atacaagata gattatgcag tacatgtaac 1080

actgaaatct gtgatacagg gttaaaccca gggtataaat gagcttaacc aaactcaagt 1140

tttaagt                                                                 1147

<210>  168
<211>  265
<212>  PRT
<213>  Gossypium raimondii

<400>  168

Met Gly Arg Asn Ser Glu Ser Asp Ser Asn Gly Trp Ser Arg Ala Arg
1               5                   10                  15

Gly Leu Val Val Lys Thr Leu Val Leu Ile Gly Gly Ala Leu Leu Leu
            20                  25                  30

Lys Arg Phe Thr Lys Ser Thr Thr Arg Trp Asp His Ala Arg Ile Val
        35                  40                  45

Ala Arg Ser Leu Ser Gly Glu Lys Phe Ser Arg Glu Gln Ala Ser Arg
    50                  55                  60

Asn Pro Asp Ser Tyr Phe Asn Ile Arg Thr Leu Thr Cys Pro Ala Thr
65                  70                  75                  80

Glu Met Val Asp Gly Ser Asn Val Leu Tyr Phe Glu Gln Ala Phe Trp
                85                  90                  95

Arg Thr Pro Gln Lys Pro Phe Arg Gln Arg Phe Phe Met Val Lys Pro
            100                 105                 110

Cys Pro Lys Asp Leu Lys Cys Asp Val Glu Val Ser Ser Tyr Ala Ile
        115                 120                 125

Arg Asp Ala Asp Glu Tyr Arg Asn Phe Cys Asp Arg Pro Arg Asp Gln
    130                 135                 140

Cys Pro Pro Pro Glu Glu Val Ile Asp Asp Val Ala Glu His Leu Thr
145                 150                 155                 160

Thr Ile Tyr Leu Lys Arg Cys Glu Arg Gly Lys Arg Cys Leu Tyr Glu
                165                 170                 175

Gly Ser Thr Pro Pro Gly Gly Phe Pro Asn Ser Trp Asn Gly Ala Thr
            180                 185                 190

Tyr Cys Thr Ser Glu Leu Thr Ile Leu Lys Asn Asn Glu Ile His Thr
        195                 200                 205

Trp Asp Arg Gly Tyr Asp Asp Asp Gly Asn Gln Val Trp Gly Val Lys
    210                 215                 220

Glu Gly Pro Tyr Glu Phe Lys Pro Ala Pro Ala Ser Ser Phe Asn Gly
225                 230                 235                 240

Met Phe Ser Pro Leu Asn Phe Ala Pro Ser Gln Pro Met Glu Lys Arg
                245                 250                 255

Ile Glu Gly Ser Phe Val Leu Gln Glu
            260                 265

<210>  169
<211>  908
<212>  DNA
<213>  Helianthus paradoxus

<400>  169
   aaagagtcca aaagaaaccc tcactggatt tctatcccct ttttcaccac catcatcatc      60
   aatctataat aaaccaaacc ctagggtagg gtgggtgggt atgtgtgaag gaccaccaaa     120
   tgggtggagc agagctcgag gactggtggt gaagacgctg gtcttaatcg gcggtgctct     180
   tttgatcaaa cgcttcacta aatccaccac tcgttgggac catgctcgca tcgtttccaa     240
   ctccctcacc ggcgaaaagt tttcgaagga acaagcggct agagatccgg ataatttctt     300
   taatttaagg tggctttgct gcccggctgc ggatatggtg gatggatcaa aggttctata     360
   ttttgagcaa gcattttgga gaactcctca taagccgttt agacagagat tttgtacggt     420
   gaagccttgc ccaaaagaga tgaaatgtga cgttgagttg agtacgtatg ccatcaggga     480
   tgcagaggaa tacaagaact tttgtgatcg ttcaagggac cagcgtccac tgcctgaaga     540
   agttatcggg gatgtagcag aacatctaac aaccatacac cttaaccgtt gtgaacgagg     600
   gaaacgatgc ttatacgagg gttcaaccct gcctgaaggc ttccccaatt catggaatgg     660
   tgcttcatat tgtacctcag aactggcggt cttgaagaat aacgagatcc atacatggga     720
   caggggttat gatgatgatg ggaaacaagt ttggggggtg aaaaatggtc cttacgaatt     780
   caagcctgct ccagaacctg caactggacc tgcatatact tctgttgata tgttatccac     840
   tttaaatttc cccctttcta ttgataagag gatagaaggt tcctttgttc tgcaagaata     900
   gttatgtt                                                              908

<210>  170
<211>  266
<212>  PRT
<213>  Helianthus paradoxus

<400>  170

Met Cys Glu Gly Pro Pro Asn Gly Trp Ser Arg Ala Arg Gly Leu Val

1               5                   10                  15

Val Lys Thr Leu Val Leu Ile Gly Gly Ala Leu Leu Ile Lys Arg Phe
            20                  25                  30

Thr Lys Ser Thr Thr Arg Trp Asp His Ala Arg Ile Val Ser Asn Ser
        35                  40                  45

Leu Thr Gly Glu Lys Phe Ser Lys Glu Gln Ala Ala Arg Asp Pro Asp
    50                  55                  60

Asn Phe Phe Asn Leu Arg Trp Leu Cys Cys Pro Ala Ala Asp Met Val
65                  70                  75                  80

Asp Gly Ser Lys Val Leu Tyr Phe Glu Gln Ala Phe Trp Arg Thr Pro
                85                  90                  95

His Lys Pro Phe Arg Gln Arg Phe Cys Thr Val Lys Pro Cys Pro Lys
            100                 105                 110

Glu Met Lys Cys Asp Val Glu Leu Ser Thr Tyr Ala Ile Arg Asp Ala
        115                 120                 125

Glu Glu Tyr Lys Asn Phe Cys Asp Arg Ser Arg Asp Gln Arg Pro Leu
    130                 135                 140

Pro Glu Glu Val Ile Gly Asp Val Ala Glu His Leu Thr Thr Ile His
145                 150                 155                 160

Leu Asn Arg Cys Glu Arg Gly Lys Arg Cys Leu Tyr Glu Gly Ser Thr
                165                 170                 175

Leu Pro Glu Gly Phe Pro Asn Ser Trp Asn Gly Ala Ser Tyr Cys Thr
            180                 185                 190

Ser Glu Leu Ala Val Leu Lys Asn Asn Glu Ile His Thr Trp Asp Arg
        195                 200                 205

Gly Tyr Asp Asp Asp Gly Lys Gln Val Trp Gly Val Lys Asn Gly Pro
    210                 215                 220

Tyr Glu Phe Lys Pro Ala Pro Glu Pro Ala Thr Gly Pro Ala Tyr Thr
225                 230                 235                 240

Ser Val Asp Met Leu Ser Thr Leu Asn Phe Pro Leu Ser Ile Asp Lys
                245                 250                 255

Arg Ile Glu Gly Ser Phe Val Leu Gln Glu
            260                 265

<210>  171
<211>  1012
<212>  DNA
<213>  Ipomoea nil

<400>  171
gacacgaaga gtgagatagg gcgcacggaa ggagaaaata taatttgcaa ttctggagtt      60

gaagagtgta taagttgttg attgatggcg tgaagaatca tccggttttg gggagctggt     120

gagtgcggtt caatcgagta tgtgtacggg ttcggactcg ggtttggacc ccaagtcgga     180

ggagaatccg aatgggtggg atcgagcccg tggggtggtg ctgaagacgc tggttttgat     240

cggaggggca cttctcgtta ggcgtctcac caagtccacc acccgttggg accatgctgg     300

gattgtggct cagtctctca gtggagaaaa gttttccaaa cagcaagctg ctagagaccc     360

tgatagttac ttcaatttga gatggctttc atgcccagct gctgacatgg tagatggctc     420

gaaggtctta tattttgagc aggcattctg gaggacacct cataagcctt ttcggcagag     480

attttgcatt gtcaagcctt gtccaaagga gatgagatgt gatgttgagg tcagcacata     540

tgctctcaga gatgcagagg agtacaagaa cttctgtgat cggcctaagg accagcgccc     600

acaacctgaa gaagttattg gggatattgc tgaacatttg actaccattc atctgaaacg     660

ctgtgagcgt gggaagcgat gcttatatga aggatctaca cctgcagatg gatttcctaa     720

tacatggaac ggtgcatcat attgtacgtc agaacttgca gtgctgaaga ataatgagat     780

acattcctgg gatagaggct acgatgagag tggaaatcag gtctggggtg taaagggagg     840

tccatatgag ttcaaacctg caccagcttc tagttttgat gacctttccg ctttgatgtt     900

gtcttcccca tccatagaga aaagaataga gggttcattt gttattcaag attgatactt     960

tgttctgtag tattgtaatg taaatttcaa actcccatat cgagtctatc aa            1012

<210>  172
<211>  271
<212>  PRT
<213>  Ipomoea nil

<400>  172

Met Cys Thr Gly Ser Asp Ser Gly Leu Asp Pro Lys Ser Glu Glu Asn
1               5                   10                  15

Pro Asn Gly Trp Asp Arg Ala Arg Gly Val Val Leu Lys Thr Leu Val
            20                  25                  30

Leu Ile Gly Gly Ala Leu Leu Val Arg Arg Leu Thr Lys Ser Thr Thr
        35                  40                  45

Arg Trp Asp His Ala Gly Ile Val Ala Gln Ser Leu Ser Gly Glu Lys
    50                  55                  60

Phe Ser Lys Gln Gln Ala Ala Arg Asp Pro Asp Ser Tyr Phe Asn Leu
65                  70                  75                  80

Arg Trp Leu Ser Cys Pro Ala Ala Asp Met Val Asp Gly Ser Lys Val
                85                  90                  95

Leu Tyr Phe Glu Gln Ala Phe Trp Arg Thr Pro His Lys Pro Phe Arg
            100                 105                 110

Gln Arg Phe Cys Ile Val Lys Pro Cys Pro Lys Glu Met Arg Cys Asp
        115                 120                 125

Val Glu Val Ser Thr Tyr Ala Leu Arg Asp Ala Glu Glu Tyr Lys Asn
    130                 135                 140

Phe Cys Asp Arg Pro Lys Asp Gln Arg Pro Gln Pro Glu Glu Val Ile
145                 150                 155                 160

Gly Asp Ile Ala Glu His Leu Thr Thr Ile His Leu Lys Arg Cys Glu
                165                 170                 175

Arg Gly Lys Arg Cys Leu Tyr Glu Gly Ser Thr Pro Ala Asp Gly Phe
            180                 185                 190

Pro Asn Thr Trp Asn Gly Ala Ser Tyr Cys Thr Ser Glu Leu Ala Val
        195                 200                 205

Leu Lys Asn Asn Glu Ile His Ser Trp Asp Arg Gly Tyr Asp Glu Ser
    210                 215                 220

Gly Asn Gln Val Trp Gly Val Lys Gly Gly Pro Tyr Glu Phe Lys Pro
225                 230                 235                 240

Ala Pro Ala Ser Ser Phe Asp Asp Leu Ser Ala Leu Met Leu Ser Ser
                245                 250                 255

Pro Ser Ile Glu Lys Arg Ile Glu Gly Ser Phe Val Ile Gln Asp
            260                 265                 270

<210>  173
<211>  1172
<212>  DNA
<213>  Lactuca saligna

<400>  173

cttgcatcca ttcaaaaatc tgtccctaag aaaccctggc ttggcttggc tccttttcca 60

cattggattt caatcccctc tttttatcca cagtttcttt ctgcatcgaa ttgagcccta 120

gattgagtgc tcagtagata catatataat atatagaatt tccacacaca cggtatgtgt 180

atgggaccac caagtgggtg gagcagagct cgagggttag tagtgaagac gctggttttg 240

atcggaggtg ctcttttggt taagcgatta accaagtcca ccactcggtg ggaccatgct 300

cgcatcgttt ctcaatctat cgccggcgaa aagttctcaa aagaacaagc atccagagat 360

cccgataatt tctttaattt aagatggctt tcctgcccag ctgcagatat ggtggatgga 420

tcgaaggttc tatatttcga gcaagcattt tggagaactc cccataagcc atttagacag 480

agattttgta tggtcaaacc ttgcccaaag gagatgaaat gtgatgttga gttgagtaca 540

tatgccatac gcgacgcaga ggagtacaag aacttttgtg atcgcccaag ggaccaacgt 600

ccacagcctg aagaagtaat tggggatgta gcagaacatc ttaccaccat atatctcaag 660

cgttgtgaac gagggaaacg gtgtttgtac gagggttcaa cacctcctga cggcttcccc 720

aattcatgga atggggcagc atattgtaca tcagaactgg cagtcttgaa gaaaaatgag 780

gttcatatgt gggatagggg atatgatgat gatggaaacc aagtttgggg agtgaagaat 840

ggtccttatg aattcaaggc tgcacctgga cctgcatctg catctacttc tgcttctgct 900

tctgttgata tgttatctcc attaaatttc cctcctcttt ctataggtaa gcggatagaa 960

ggttcctttg ttcttcaaga ataatcacat atgttgcatt attgccttgt ggcttgtaat 1020

atataccata attcttttta atatatgcca ttaaatccct tattatgttt ttgagcacat 1080

gcttcaatga agtttgtaac aacaggagta gaataagcag ttccaaaact ctgctttacc 1140

tgttttctac tatgtaataa tttgttcttt ct 1172

<210> 174
<211> 269
<212> PRT
<213> Lactuca saligna

<400> 174

Met Cys Met Gly Pro Pro Ser Gly Trp Ser Arg Ala Arg Gly Leu Val
1               5                   10                  15

Val Lys Thr Leu Val Leu Ile Gly Gly Ala Leu Leu Val Lys Arg Leu
            20                  25                  30

Thr Lys Ser Thr Thr Arg Trp Asp His Ala Arg Ile Val Ser Gln Ser
        35                  40                  45

Ile Ala Gly Glu Lys Phe Ser Lys Glu Gln Ala Ser Arg Asp Pro Asp
    50                  55                  60

Asn Phe Phe Asn Leu Arg Trp Leu Ser Cys Pro Ala Ala Asp Met Val
65                  70                  75                  80

Asp Gly Ser Lys Val Leu Tyr Phe Glu Gln Ala Phe Trp Arg Thr Pro
                85                  90                  95

His Lys Pro Phe Arg Gln Arg Phe Cys Met Val Lys Pro Cys Pro Lys
            100                 105                 110

Glu Met Lys Cys Asp Val Glu Leu Ser Thr Tyr Ala Ile Arg Asp Ala
        115                 120                 125

Glu Glu Tyr Lys Asn Phe Cys Asp Arg Pro Arg Asp Gln Arg Pro Gln
    130                 135                 140

Pro Glu Glu Val Ile Gly Asp Val Ala Glu His Leu Thr Thr Ile Tyr
145                 150                 155                 160

Leu Lys Arg Cys Glu Arg Gly Lys Arg Cys Leu Tyr Glu Gly Ser Thr
                165                 170                 175

Pro Pro Asp Gly Phe Pro Asn Ser Trp Asn Gly Ala Ala Tyr Cys Thr
            180                 185                 190

Ser Glu Leu Ala Val Leu Lys Lys Asn Glu Val His Met Trp Asp Arg
        195                 200                 205

Gly Tyr Asp Asp Asp Gly Asn Gln Val Trp Gly Val Lys Asn Gly Pro
    210                 215                 220

Tyr Glu Phe Lys Ala Ala Pro Gly Pro Ala Ser Ala Ser Thr Ser Ala
225                 230                 235                 240

Ser Ala Ser Val Asp Met Leu Ser Pro Leu Asn Phe Pro Pro Leu Ser
                245                 250                 255

Ile Gly Lys Arg Ile Glu Gly Ser Phe Val Leu Gln Glu
            260                 265

<210>  175
<211>  804
<212>  DNA
<213>  Medicago truncatula

<400>  175
atgtgtaagg aatccgaatc cgaatccgat tccaatggat ggaaccgtgc tcaaggttta      60

gcactcaaag ctctcttact cctcggtggc gctcttctcg tcaagcgtct ccgtaagtcc     120

accactcgct gggaccatac tcatttagtc actcaatccc tcaccggcga aaagtattcc 180

aaggaccaag cttctagaga ccctgataac tatttcaata ttagaatgct tacatgtcca 240

gctgcagagc tagttgatgg ttcaaatgtc ctatattatg agcaggcttt ttggaggagt 300

ccacagaaac cctttcgcca gaggttgtta atgaccaaac cttgtccaaa agagttgaaa 360

tgtgatgttg agttaagtac atatgccatc agagacatgg aggaatacaa aaatttctgt 420

gatcggccaa aggatcagcg cccacaacca gaggaagtca ttggggatat tggagagcat 480

ttgacaacaa tacatcttaa gcgttgttca cgtggaaaac gctgcttata tgaaggttca 540

accccacccg aaggatttcc taattcatgg aatggagcaa cctactgtac ttcagagctt 600

gctgttatga agaacaatga gatacacacc tgggatcggg gttatgacga cgatggaaat 660

caagtttggg gacaaaaaga aggcccttac gagttcaagc ctgcaccaac ctcctgtttt 720

aatgatacat tttcgccctt gaattttccc cctccaccgt ccatggatag aagaatagag 780

ggttcatttg ttttgcaaga atga 804

<210> 176
<211> 267
<212> PRT
<213> Medicago truncatula

<400> 176

Met Cys Lys Glu Ser Glu Ser Glu Ser Asp Ser Asn Gly Trp Asn Arg
1               5                   10                  15

Ala Gln Gly Leu Ala Leu Lys Ala Leu Leu Leu Leu Gly Gly Ala Leu
            20                  25                  30

Leu Val Lys Arg Leu Arg Lys Ser Thr Thr Arg Trp Asp His Thr His
        35                  40                  45

Leu Val Thr Gln Ser Leu Thr Gly Glu Lys Tyr Ser Lys Asp Gln Ala
    50                  55                  60

Ser Arg Asp Pro Asp Asn Tyr Phe Asn Ile Arg Met Leu Thr Cys Pro
65                  70                  75                  80

Ala Ala Glu Leu Val Asp Gly Ser Asn Val Leu Tyr Tyr Glu Gln Ala
                85                  90                  95

Phe Trp Arg Ser Pro Gln Lys Pro Phe Arg Gln Arg Leu Leu Met Thr
            100                 105                 110

Lys Pro Cys Pro Lys Glu Leu Lys Cys Asp Val Glu Leu Ser Thr Tyr
        115                 120                 125

Ala Ile Arg Asp Met Glu Glu Tyr Lys Asn Phe Cys Asp Arg Pro Lys
    130                 135                 140

Asp Gln Arg Pro Gln Pro Glu Glu Val Ile Gly Asp Ile Gly Glu His
145                 150                 155                 160

Leu Thr Thr Ile His Leu Lys Arg Cys Ser Arg Gly Lys Arg Cys Leu
                165                 170                 175

Tyr Glu Gly Ser Thr Pro Pro Glu Gly Phe Pro Asn Ser Trp Asn Gly
            180                 185                 190

Ala Thr Tyr Cys Thr Ser Glu Leu Ala Val Met Lys Asn Asn Glu Ile
        195                 200                 205

His Thr Trp Asp Arg Gly Tyr Asp Asp Asp Gly Asn Gln Val Trp Gly
    210                 215                 220

Gln Lys Glu Gly Pro Tyr Glu Phe Lys Pro Ala Pro Thr Ser Cys Phe
225                 230                 235                 240

Asn Asp Thr Phe Ser Pro Leu Asn Phe Pro Pro Pro Pro Ser Met Asp
                245                 250                 255

Arg Arg Ile Glu Gly Ser Phe Val Leu Gln Glu
            260                 265

<210>  177
<211>  1083
<212>  DNA
<213>  Nicotiana tabacum

<400>  177
ccggggactt caaaaatttc cccattttct gaagtttgta attttgcaga aatttgagaa      60

gaaaaagaag ctgaagcatt tgaattcaat tcaagtttgt aaaattcgag gatgatttat     120

aggtcaaatc atctgctatt agagagtcca ctgtaaagtg ttaaagacca gtatgtgccc     180

gggctcggac tcggtttcgg actcaaagtc ggatccgaac tcaaacgggt ggagccgagc     240

tcgtggagcg gttctcaagt cgctggtgct tatcgggggc gctttgttgg tacggcggct     300

cactaagtcg accacacgtt gggaccatgc tcgaattgtc gcacagtcac ttagcggtga     360

aaagttttcc aaggagcaag ctgttaggga tcctgataat tattttaatt tcagatggct     420

ttcctgtcct gctgccgaca tggtagatgg ctctaaagtt ctatattttg aacaggcatt     480

ttggcggact ccccataaac ccttccgaca gaggtttttc atggtcaagc cttgtgcaaa     540

ggagctgaaa tgtgatgttg aggtaagcac atatgccatc agagatgcag aggagtacaa     600

gaacttctgc gatcgcccta gggaccaacg tccggaacct gaagaagtta ttggggatat 660

tgctgaacat ttgactacca ttcatctaaa gcgctgtgaa cgtgggaaac gatgcttata 720

tgaaggttca acacctgcag atggatttcc taattcatgg aatggtgcaa cgcgctgtac 780

ctccgaacta gctgtgttaa agaacaatga gatacatgcc tgggatagag gctatgatgt 840

tgatggcaat caagtttggg gtgtaaaagg aggtccttat gaattcaagc ccgctcctgc 900

ttcaagtttt gacgatgcat ttaatccttt gagttttgct tcccaacctg tggggaaaag 960

aatagagggt tcgtttgtcc tccaggagtg atttcttgtt ttatattcac atgtccatac 1020

ataacaatgt aaatcattga actcaattaa cattatgttt ggaatctact attacttgag 1080

tac 1083

<210> 178
<211> 272
<212> PRT
<213> Nicotiana tabacum

<400> 178

Met Cys Pro Gly Ser Asp Ser Val Ser Asp Ser Lys Ser Asp Pro Asn
1               5                   10                  15

Ser Asn Gly Trp Ser Arg Ala Arg Gly Ala Val Leu Lys Ser Leu Val
            20                  25                  30

Leu Ile Gly Gly Ala Leu Leu Val Arg Arg Leu Thr Lys Ser Thr Thr
        35                  40                  45

Arg Trp Asp His Ala Arg Ile Val Ala Gln Ser Leu Ser Gly Glu Lys
    50                  55                  60

Phe Ser Lys Glu Gln Ala Val Arg Asp Pro Asp Asn Tyr Phe Asn Phe
65                  70                  75                  80

Arg Trp Leu Ser Cys Pro Ala Ala Asp Met Val Asp Gly Ser Lys Val
                85                  90                  95

Leu Tyr Phe Glu Gln Ala Phe Trp Arg Thr Pro His Lys Pro Phe Arg
            100                 105                 110

Gln Arg Phe Phe Met Val Lys Pro Cys Ala Lys Glu Leu Lys Cys Asp
        115                 120                 125

Val Glu Val Ser Thr Tyr Ala Ile Arg Asp Ala Glu Glu Tyr Lys Asn
    130                 135                 140

Phe Cys Asp Arg Pro Arg Asp Gln Arg Pro Glu Pro Glu Glu Val Ile

145                 150                 155                 160

Gly Asp Ile Ala Glu His Leu Thr Thr Ile His Leu Lys Arg Cys Glu
                165                 170                 175

Arg Gly Lys Arg Cys Leu Tyr Glu Gly Ser Thr Pro Ala Asp Gly Phe
            180                 185                 190

Pro Asn Ser Trp Asn Gly Ala Thr Arg Cys Thr Ser Glu Leu Ala Val
        195                 200                 205

Leu Lys Asn Asn Glu Ile His Ala Trp Asp Arg Gly Tyr Asp Val Asp
    210                 215                 220

Gly Asn Gln Val Trp Gly Val Lys Gly Gly Pro Tyr Glu Phe Lys Pro
225                 230                 235                 240

Ala Pro Ala Ser Ser Phe Asp Asp Ala Phe Asn Pro Leu Ser Phe Ala
                245                 250                 255

Ser Gln Pro Val Gly Lys Arg Ile Glu Gly Ser Phe Val Leu Gln Glu
            260                 265                 270

<210>  179
<211>  882
<212>  DNA
<213>  Taraxum officinale

<400>  179
atatagagtt ttcgacagac acggtatgtg tatgggacca ccaagtgggt ggagcagagc      60

tcgagggtta gtggtgaaga cgctggtttt gatcggaggt gctcttttgg ttaagcgatt     120

aaccaagtcc accactcgtt gggaccatgc tcgcatcgtt tctcaatcca tcgccggcga     180

aaagttctcg aaggaacaag catccagaga tcccgataat ttcttcaatt taagatggct     240

ttcttgtcca gctgcagaca tggtagatgg atcaaaggtt ctatattttg agcaagcgtt     300

ttggagaact cccaataagc cattcagaca gagattttgt atggtgaaac cttgcccaaa     360

ggaaatgaaa tgtgatgttg agttaagtac atacgcgata cgggacgcag aggagtacaa     420

gaacttttgt gatcggccaa gggaccaacg tccacagcct gaagaagtaa tcggggatgt     480

agcagaacat cttactacaa tatatctcaa tcgatgtgaa cgagggaaac ggtgtttgta     540

cgagggttca acaccacccg acgggttccc taattcatgg aatggtgcag catattgtac     600

ctcacaactt gcagtcttga agaacaacga ggttcacatg tgggatagag gctatgatga     660

tgatggaaac caagtttggg gagtgaaaaa cggtccttat gaattcaagg ctgcacctgg     720

acctgcatct gctcctgctg acgtggtatc tcctctgaat tttcctcccc cttctattgg     780

taaaaggata gaaggttcct tttggcctca agaatgatca catgtgtcgc attgtttata     840

atatataatg taccttaatt atatttatta aaaatttgag ct                        882

<210>  180
<211>  263
<212>  PRT
<213>  Taraxum officinale

<400>  180

Met Cys Met Gly Pro Pro Ser Gly Trp Ser Arg Ala Arg Gly Leu Val
1               5                   10                  15

Val Lys Thr Leu Val Leu Ile Gly Gly Ala Leu Leu Val Lys Arg Leu
            20                  25                  30

Thr Lys Ser Thr Thr Arg Trp Asp His Ala Arg Ile Val Ser Gln Ser
        35                  40                  45

Ile Ala Gly Glu Lys Phe Ser Lys Glu Gln Ala Ser Arg Asp Pro Asp
    50                  55                  60

Asn Phe Phe Asn Leu Arg Trp Leu Ser Cys Pro Ala Ala Asp Met Val
65                  70                  75                  80

Asp Gly Ser Lys Val Leu Tyr Phe Glu Gln Ala Phe Trp Arg Thr Pro
                85                  90                  95

Asn Lys Pro Phe Arg Gln Arg Phe Cys Met Val Lys Pro Cys Pro Lys
            100                 105                 110

Glu Met Lys Cys Asp Val Glu Leu Ser Thr Tyr Ala Ile Arg Asp Ala
        115                 120                 125

Glu Glu Tyr Lys Asn Phe Cys Asp Arg Pro Arg Asp Gln Arg Pro Gln
    130                 135                 140

Pro Glu Glu Val Ile Gly Asp Val Ala Glu His Leu Thr Thr Ile Tyr
145                 150                 155                 160

Leu Asn Arg Cys Glu Arg Gly Lys Arg Cys Leu Tyr Glu Gly Ser Thr
                165                 170                 175

Pro Pro Asp Gly Phe Pro Asn Ser Trp Asn Gly Ala Ala Tyr Cys Thr
            180                 185                 190

Ser Gln Leu Ala Val Leu Lys Asn Asn Glu Val His Met Trp Asp Arg
        195                 200                 205

Gly Tyr Asp Asp Asp Gly Asn Gln Val Trp Gly Val Lys Asn Gly Pro
    210                 215                 220

Tyr Glu Phe Lys Ala Ala Pro Gly Pro Ala Ser Ala Pro Ala Asp Val
225                 230                 235                 240

Val Ser Pro Leu Asn Phe Pro Pro Pro Ser Ile Gly Lys Arg Ile Glu
                245                 250                 255

Gly Ser Phe Trp Pro Gln Glu
            260

<210>  181
<211>  1143
<212>  DNA
<213>  Vitis vinifera

<400>  181
tcacattgat ttttcttctg gaattggaca gacaaggatt caacatctac aatagtttgt      60

agatggtttt atcacagaag aaccactggg tttgtggtgg atggtgtatc cgcaattaag     120

gaagaaaaaa cgaaggagtg tttgaaaggg agagagtgag agatgggaac ggggtctgac     180

tcagagtcgg agggtaacgg atggggcaga gcaagaggaa ttctggtgaa ggcggcggtg     240

ttgataggag gggctattct ccttaagaga ctcaccaagt ctaccactcg ctgggatcat     300

gcccgctttg tttcccactc cctctccggc gaaaagttct caatggagca ggcttccaga     360

gaccctgaca actacttcaa tttcagaatg gtcacgtgcc cagcagcaga gctggtggat     420

ggttcccggg tcttatattt tgagcaggca ttttggagaa ctccttccaa gccctttcgg     480

cagagatttt atatggtaaa gccttgtcca aaagagatga aatgtgatgt tgagctaagt     540

tcatatgcca ttagagatgt ggaggagtac aagaacttct gtgatcgctc caaggcccag     600

cgcccacttc ctgaggaagt tataggggac attgcagagc atttgacgac catatatctc     660

aaacgctgtg aacgtggcaa acgctgttta tatgaaggtt caactccatc agggggattc     720

cctaattcat ggagtggtgc aacctactgt acttcagaac ttgcagtctt gaagaataat     780

gagattcata tctgggatag gggctatgat gatgaaggaa atcaagtttg gggagtgaag     840

gagggtcctt acgagttcaa gccggcacct gcctcaagtt ccaatgacat gttttctcct     900

ttaaattttg ccccccctct gcccatggag aaaagaatag atggttcatt tgttttacaa     960

gaatgatcac tactttataa ccatgacatg taaattttca actttcatga ttcttagatc    1020

ttacatattg tgtttttgtc tattctattt cctccgaatg aacttgatgc attgaacaat    1080

tagcaatgta aaattgcact caatagctga agtgcaataa aatgtatagt gatggttggg    1140

agc                                                                  1143

<210>  182
<211>  267
<212>  PRT
<213>  Vitis vinifera

<400>  182

Met Gly Thr Gly Ser Asp Ser Glu Ser Glu Gly Asn Gly Trp Gly Arg
1               5                   10                  15

Ala Arg Gly Ile Leu Val Lys Ala Ala Val Leu Ile Gly Gly Ala Ile
            20                  25                  30

Leu Leu Lys Arg Leu Thr Lys Ser Thr Thr Arg Trp Asp His Ala Arg
        35                  40                  45

Phe Val Ser His Ser Leu Ser Gly Glu Lys Phe Ser Met Glu Gln Ala
    50                  55                  60

Ser Arg Asp Pro Asp Asn Tyr Phe Asn Phe Arg Met Val Thr Cys Pro
65                  70                  75                  80

Ala Ala Glu Leu Val Asp Gly Ser Arg Val Leu Tyr Phe Glu Gln Ala
                85                  90                  95

Phe Trp Arg Thr Pro Ser Lys Pro Phe Arg Gln Arg Phe Tyr Met Val
            100                 105                 110

Lys Pro Cys Pro Lys Glu Met Lys Cys Asp Val Glu Leu Ser Ser Tyr
        115                 120                 125

Ala Ile Arg Asp Val Glu Glu Tyr Lys Asn Phe Cys Asp Arg Ser Lys
    130                 135                 140

Ala Gln Arg Pro Leu Pro Glu Glu Val Ile Gly Asp Ile Ala Glu His
145                 150                 155                 160

Leu Thr Thr Ile Tyr Leu Lys Arg Cys Glu Arg Gly Lys Arg Cys Leu
                165                 170                 175

Tyr Glu Gly Ser Thr Pro Ser Gly Gly Phe Pro Asn Ser Trp Ser Gly
            180                 185                 190

Ala Thr Tyr Cys Thr Ser Glu Leu Ala Val Leu Lys Asn Asn Glu Ile
        195                 200                 205

His Ile Trp Asp Arg Gly Tyr Asp Asp Glu Gly Asn Gln Val Trp Gly
    210                 215                 220

Val Lys Glu Gly Pro Tyr Glu Phe Lys Pro Ala Pro Ala Ser Ser Ser
225                 230                 235                 240

Asn Asp Met Phe Ser Pro Leu Asn Phe Ala Pro Pro Leu Pro Met Glu
                245                 250                 255

Lys Arg Ile Asp Gly Ser Phe Val Leu Gln Glu
            260                 265

<210>  183
<211>  1059
<212>  DNA
<213>  Populus trichocarpa

<400>  183
aggaacttca atcaacaact tggtgcttgc taactatcta gctctgtaat tcagacattc      60

gattcccatc ttccttatct ttaactcact gagtcccaag atggtattgg tggtggctgt     120

ttggtttttg tttagagtgg aaaaacagtg aaattgcgtt gcgtgtgttc ttttgttttc     180

ttaaatggta acgggtttgg gttcgggctc agggtccgat cctacttcag actcaaacgg     240

gtggggacga gctcgagggt tagcgctgaa gtctctggtt ttgattggtg gggtgttact     300

agtgaagaga ctgacgaagt ctactactcg ttgggaccat gcgaaaattg taactcaatc     360

acttactggt gaaaagtttt cgaaggagca agcatctaga gaccctgata attacttcaa     420

tatcagaatg cttacttgcc cggcagcaga gatggtggat ggttcaaagg ttttatattt     480

tgaacaggca ttctggagaa ctcctcaaaa gccctttcgg cagaggtttt atatggttaa     540

gccttgtcca aaggagttga aatgcgatgt tgaggtaggt tcgtatgcca ttagagatgc     600

agaggagtac aagaattttt gcgatcgatc aaaggaccag cgcccactgc cagaagaagt     660

aattggtgac attgcagaac atctgacaac aatacatctc aaacgctgtg accgtggaaa     720

acgctgctta tatgaaggct ccaatccacc tggtggattc ccaaattcct ggaatggagc     780

aacctactgc acttcggaac ttgcaatctt gaagaataat gaaatacata cctgggatag     840

gggatacgat gacggtggaa atcaggtttg gggagtgaaa gaaggacctt acgagttcaa     900

gcctgcacca gcttctagtg tcagtgaatt attttctcct ttaaacctcc cccctctcca     960

gtcgatggag aaaagaatag aaggttcatt tgttttgcaa gtgtgatcgc ttgctacatg    1020

tataatcaac cacggcatgt aacgtatcaa acttcaaat                           1059

<210>  184
<211>  273
<212>  PRT
<213>  Populus trichocarpa

<400>  184

Met Val Thr Gly Leu Gly Ser Gly Ser Gly Ser Asp Pro Thr Ser Asp
1               5                   10                  15

Ser Asn Gly Trp Gly Arg Ala Arg Gly Leu Ala Leu Lys Ser Leu Val
            20                  25                  30

Leu Ile Gly Gly Val Leu Leu Val Lys Arg Leu Thr Lys Ser Thr Thr
        35                  40                  45

Arg Trp Asp His Ala Lys Ile Val Thr Gln Ser Leu Thr Gly Glu Lys
    50                  55                  60

Phe Ser Lys Glu Gln Ala Ser Arg Asp Pro Asp Asn Tyr Phe Asn Ile
65                  70                  75                  80

Arg Met Leu Thr Cys Pro Ala Ala Glu Met Val Asp Gly Ser Lys Val
                85                  90                  95

Leu Tyr Phe Glu Gln Ala Phe Trp Arg Thr Pro Gln Lys Pro Phe Arg
            100                 105                 110

Gln Arg Phe Tyr Met Val Lys Pro Cys Pro Lys Glu Leu Lys Cys Asp
        115                 120                 125

Val Glu Val Gly Ser Tyr Ala Ile Arg Asp Ala Glu Glu Tyr Lys Asn
    130                 135                 140

Phe Cys Asp Arg Ser Lys Asp Gln Arg Pro Leu Pro Glu Glu Val Ile
145                 150                 155                 160

Gly Asp Ile Ala Glu His Leu Thr Thr Ile His Leu Lys Arg Cys Asp
                165                 170                 175

Arg Gly Lys Arg Cys Leu Tyr Glu Gly Ser Asn Pro Pro Gly Gly Phe
            180                 185                 190

Pro Asn Ser Trp Asn Gly Ala Thr Tyr Cys Thr Ser Glu Leu Ala Ile
        195                 200                 205

Leu Lys Asn Asn Glu Ile His Thr Trp Asp Arg Gly Tyr Asp Asp Gly
    210                 215                 220

Gly Asn Gln Val Trp Gly Val Lys Glu Gly Pro Tyr Glu Phe Lys Pro
225                 230                 235                 240

Ala Pro Ala Ser Ser Val Ser Glu Leu Phe Ser Pro Leu Asn Leu Pro
                245                 250                 255

Pro Leu Gln Ser Met Glu Lys Arg Ile Glu Gly Ser Phe Val Leu Gln
            260                 265                 270

Val

<210>  185
<211>  1237
<212>  DNA
<213>  Solanum lycopersicum

<400>  185
gtgaggcaac atttatacaa attactccat tttcagtagt acagattttg cagaaagctg      60

acaagcacaa gctaagatga tttatcggtt aaatgatctg ctgttgaaga gtccacttta     120

agtattaaag cgccggcatg tgtacgggct cggactcggg tttgggttcg gattctaagt     180

cggatcccga ctcaaacggg tggagccgag ctcgtggagc ggttctcaag tcgctggtgc     240

tcgtcggagg agccttattg ctccggcggc taactaagtc gactacacgt tgggaccatg     300

ctcgaattgt cgcagagtca cttaacggtg aaaagttttc aaaggagcaa gctgctaggg     360

atcctgataa ttattttaat ttcagatggc tttcctgtcc tgctgcagac atggtagatg     420

gctctaaggt tttatatttt gagcaggcat tttggcgaac accacacaaa ccctttagac     480

agagattttt catggtcaag ccttgtgcaa aggagttgaa atgtgatgtt gagttaagca     540

catatgctat cagagatgca gaggagtaca agaacttttg tgatcgccct agggatcagc     600

gtccacaacc cgaagaagtt attggggaca ttgctgaaca tttgactacc attcatctaa     660

agcgctgtga gcgtgggaaa cggtgcttgt atgaaggttc aacacctgca gatggatttc     720

ctaattcatg gcagaatggt gcgacatact gtacctcaga acttgctgtg ttaaagaata     780

atgagataca tgcctgggat agaggctttg atgatgatgg caatcaagtt tggggtgtaa     840

aaggaggtcc ttatgaattc aagcctgctc cttcttcaag ttttaacgat gtgctaaatc     900

ctttgagttt tgcttcacaa cccctgggga aaagaataga aggctcattt gtcctccagg     960

aatgatttct tgttatataa tccccccacc ccaactgccc acatcttgta aatcataact    1020

caattagtat tatgtttaaa ttatagtaaa tcataaggtg ttttatattt caaagctaac    1080

tgttttctga tgatagttct gatctttctc tgagaatgcc tgagagaatt ttgcagaaac    1140

ggtaatcacc gcttgaatta agattgatgt ttgtcatgta tgactgatat agtgttattg    1200

agagaatttg ctcgtgccca aacaagacgt gaagaag                             1237

<210>  186
<211>  275
<212>  PRT
<213>  Solanum lycopersicum

<400>  186

Met Cys Thr Gly Ser Asp Ser Gly Leu Gly Ser Asp Ser Lys Ser Asp
1               5                   10                  15

Pro Asp Ser Asn Gly Trp Ser Arg Ala Arg Gly Ala Val Leu Lys Ser
            20                  25                  30

Leu Val Leu Val Gly Gly Ala Leu Leu Leu Arg Arg Leu Thr Lys Ser
        35                  40                  45

Thr Thr Arg Trp Asp His Ala Arg Ile Val Ala Glu Ser Leu Asn Gly
    50                  55                  60

Glu Lys Phe Ser Lys Glu Gln Ala Ala Arg Asp Pro Asp Asn Tyr Phe
65                  70                  75                  80

Asn Phe Arg Trp Leu Ser Cys Pro Ala Ala Asp Met Val Asp Gly Ser
                85                  90                  95

Lys Val Leu Tyr Phe Glu Gln Ala Phe Trp Arg Thr Pro His Lys Pro
            100                 105                 110

Phe Arg Gln Arg Phe Phe Met Val Lys Pro Cys Ala Lys Glu Leu Lys
        115                 120                 125

Cys Asp Val Glu Leu Ser Thr Tyr Ala Ile Arg Asp Ala Glu Glu Tyr
    130                 135                 140

Lys Asn Phe Cys Asp Arg Pro Arg Asp Gln Arg Pro Gln Pro Glu Glu
145                 150                 155                 160

Val Ile Gly Asp Ile Ala Glu His Leu Thr Thr Ile His Leu Lys Arg
                165                 170                 175

Cys Glu Arg Gly Lys Arg Cys Leu Tyr Glu Gly Ser Thr Pro Ala Asp
            180                 185                 190

Gly Phe Pro Asn Ser Trp Gln Asn Gly Ala Thr Tyr Cys Thr Ser Glu
        195                 200                 205

Leu Ala Val Leu Lys Asn Asn Glu Ile His Ala Trp Asp Arg Gly Phe
    210                 215                 220

Asp Asp Asp Gly Asn Gln Val Trp Gly Val Lys Gly Gly Pro Tyr Glu
225                 230                 235                 240

Phe Lys Pro Ala Pro Ser Ser Ser Phe Asn Asp Val Leu Asn Pro Leu

245                 250                 255

Ser Phe Ala Ser Gln Pro Leu Gly Lys Arg Ile Glu Gly Ser Phe Val
            260                 265                 270

Leu Gln Glu
        275

<210>  187
<211>  1159
<212>  DNA
<213>  Solanum tuberosum

<400>  187
tcggacggcg tgaggcaacg tttgtacaaa ttattactct attttcagta gttcacattt      60

tgcagaaagc tgggaagcag aagcttaaga tgatttatcg gttaaataat ctgctgttga     120

agagtccact ttaagtgtta agcgccggca tgtgtacggg ctcggactcg ggtttgggtt     180

cgaattctaa gtcggatcct gactcaaacg ggtggagccg agctcgtgga gcggtcctca     240

agtcactggt gctcgtcgga ggagccttat tgctccggcg gctaactaag tcgaccacac     300

gttgggacca tgctcgaatt gtcgcagagt cacttaacgg tgaaaagttt tcaaaggagc     360

aagcggttag ggatcctgat aattatttta atttcagatg gctttcctgt cctgctgcag     420

acatggtaga tggctctaag gttttatatt ttgagcaggc attttggcga acaccgcaca     480

aacccttcag acagagattt ttcatggtca agccttgtgc aaaggagttg aaatgtgatg     540

ttgaggtaag cacatatgct atcagagatg cagaggagta caagaacttc tgtgatcgcc     600

ctagggatca gcgtccacaa cccgaagaag ttattgggga tattgctgaa catttgacta     660

ccattcatct aaagcgctgt gagcgtggga aacgatgctt atatgaaggt tcaacaccag     720

cagatggatt tcctaattca tggcagaatg gtgcgacata ctgtacctca gaacttgctg     780

tgttgaagaa taatgagata catgcctggg atagaggttt tgatgatgat ggcaatcaag     840

tttggggtgt aaaaggaggt ccttatgaat tcaagcctgc tccttcttca agttttaacg     900

atgtgctgaa tcctttgagt tttgcttcac aacccctggg gaaaagaata gagggttcat     960

ttgtcctcca ggaatgattt cttgttatat aagccccccc aaactgccca catcttgtaa    1020

attataactc aactaggtaa tcaaggcttt gaacaacatt tccaataatc gactgttgta    1080

agaaataaaa agagagtaaa ttttaatagg ttttaagaac atttcaatag aaattaaatg    1140

atttgaaatt aaaaaaaaa                                                  1159

<210>  188
<211>  275
<212>  PRT
<213>  Solanum tuberosum

<400> 188

Met Cys Thr Gly Ser Asp Ser Gly Leu Gly Ser Asn Ser Lys Ser Asp
1               5                   10                  15

Pro Asp Ser Asn Gly Trp Ser Arg Ala Arg Gly Ala Val Leu Lys Ser
            20                  25                  30

Leu Val Leu Val Gly Gly Ala Leu Leu Leu Arg Arg Leu Thr Lys Ser
        35                  40                  45

Thr Thr Arg Trp Asp His Ala Arg Ile Val Ala Glu Ser Leu Asn Gly
    50                  55                  60

Glu Lys Phe Ser Lys Glu Gln Ala Val Arg Asp Pro Asp Asn Tyr Phe
65                  70                  75                  80

Asn Phe Arg Trp Leu Ser Cys Pro Ala Ala Asp Met Val Asp Gly Ser
                85                  90                  95

Lys Val Leu Tyr Phe Glu Gln Ala Phe Trp Arg Thr Pro His Lys Pro
            100                 105                 110

Phe Arg Gln Arg Phe Phe Met Val Lys Pro Cys Ala Lys Glu Leu Lys
        115                 120                 125

Cys Asp Val Glu Val Ser Thr Tyr Ala Ile Arg Asp Ala Glu Glu Tyr
    130                 135                 140

Lys Asn Phe Cys Asp Arg Pro Arg Asp Gln Arg Pro Gln Pro Glu Glu
145                 150                 155                 160

Val Ile Gly Asp Ile Ala Glu His Leu Thr Thr Ile His Leu Lys Arg
                165                 170                 175

Cys Glu Arg Gly Lys Arg Cys Leu Tyr Glu Gly Ser Thr Pro Ala Asp
            180                 185                 190

Gly Phe Pro Asn Ser Trp Gln Asn Gly Ala Thr Tyr Cys Thr Ser Glu
        195                 200                 205

Leu Ala Val Leu Lys Asn Asn Glu Ile His Ala Trp Asp Arg Gly Phe
    210                 215                 220

Asp Asp Asp Gly Asn Gln Val Trp Gly Val Lys Gly Gly Pro Tyr Glu
225                 230                 235                 240

Phe Lys Pro Ala Pro Ser Ser Ser Phe Asn Asp Val Leu Asn Pro Leu

                245                 250                 255

Ser Phe Ala Ser Gln Pro Leu Gly Lys Arg Ile Glu Gly Ser Phe Val
            260                 265                 270

Leu Gln Glu
        275

<210> 189
<211> 1048
<212> DNA
<213> Saccharum officinarum

<400> 189
gacccccggc gatgggctcc ggcgaagagg acacaggagg cggaggaggg gcggtgcggg 60

gcgcggtgct gaaggcgctc gtggtcgtcg gcggcgtcct gctgctccgc cgcctgcgcc 120

gctccaccac ccgctgggac cacgcgcgag ccgtcgccga cgcgctctcc ggtgagaagt 180

tctctaggga gcaggcaagg aaggatcctg acaacttctt caatttgaga atgctcacat 240

gtcctgcaac cgagatggtg gatggctcaa gggtgcttta ctttgagcag gcattttgga 300

gatctccaga aaagcctttt agacaaagat tctacatggt aaagccctgt ccgaaggaga 360

tgaaatgcga tgttgagttg agttcatatg caattaggga tgctgaagag tacaagaatt 420

tctgtgaccg tcaaaaggat cagaggccac agccagaaga agtaattgcg gatatcgcag 480

agcatctgac caccttacac ttgtcacgat gtggccgtgg taaacgttgt ttgtatgaag 540

gatctacccc acctgaaggt tttcccaaca attggagtgg tgcatcatat tgtacatcgg 600

atttgtccat ccacaaaaac ggtgaagtac atatctggga caaaggtttt gacgacgaag 660

ggaaccaggt ttggggaacc aaggttggcc cttacgagtt caagcctgcc cccaaatcca 720

aatatgacga catgttctcg ccattaaatt tctccgcccc tttgtcacta gagaagaagt 780

tggataaagc atatgtaatc gatgaccagt agagcctgag ccctaaattt tgttcatagg 840

aatgcaacaa gtgaatagca tgtatgtata tattgtactg agttctattg catttttttt 900

accaacctgt gcatcttggt tgtccatggt tagtatggtg atgcatgatg actagtcaac 960

cgatttaagt cagtttgtta gatgacccaa tcgcattgta tcctatggtg tattatgcta 1020

gtcagaaaag atttgttgta gtcacatt 1048

<210> 190
<211> 266
<212> PRT
<213> Saccharum officinarum

<400> 190
   Met Gly Ser Gly Glu Glu Asp Thr Gly Gly Gly Gly Gly Ala Val Arg
   1 5 10 15
   Gly Ala Val Leu Lys Ala Leu Val Val Val Gly Gly Val Leu Leu Leu
               20                  25                  30
   Arg Arg Leu Arg Arg Ser Thr Thr Arg Trp Asp His Ala Arg Ala Val
           35                  40                  45
   Ala Asp Ala Leu Ser Gly Glu Lys Phe Ser Arg Glu Gln Ala Arg Lys
       50                  55                  60
   Asp Pro Asp Asn Phe Phe Asn Leu Arg Met Leu Thr Cys Pro Ala Thr
   65                  70                  75                  80
   Glu Met Val Asp Gly Ser Arg Val Leu Tyr Phe Glu Gln Ala Phe Trp
                   85                  90                  95
   Arg Ser Pro Glu Lys Pro Phe Arg Gln Arg Phe Tyr Met Val Lys Pro
               100                 105                 110
   Cys Pro Lys Glu Met Lys Cys Asp Val Glu Leu Ser Ser Tyr Ala Ile
           115                 120                 125
   Arg Asp Ala Glu Glu Tyr Lys Asn Phe Cys Asp Arg Gln Lys Asp Gln
       130                 135                 140
   Arg Pro Gln Pro Glu Glu Val Ile Ala Asp Ile Ala Glu His Leu Thr
   145                 150                 155                 160
   Thr Leu His Leu Ser Arg Cys Gly Arg Gly Lys Arg Cys Leu Tyr Glu
                   165                 170                 175
   Gly Ser Thr Pro Pro Glu Gly Phe Pro Asn Asn Trp Ser Gly Ala Ser
               180                 185                 190
   Tyr Cys Thr Ser Asp Leu Ser Ile His Lys Asn Gly Glu Val His Ile
           195                 200                 205
   Trp Asp Lys Gly Phe Asp Asp Glu Gly Asn Gln Val Trp Gly Thr Lys
       210                 215                 220
   Val Gly Pro Tyr Glu Phe Lys Pro Ala Pro Lys Ser Lys Tyr Asp Asp
   225                 230                 235                 240
   Met Phe Ser Pro Leu Asn Phe Ser Ala Pro Leu Ser Leu Glu Lys Lys
                   245                 250                 255
   Leu Asp Lys Ala Tyr Val Ile Asp Asp Gln
   260                 265

<210>  191
<211>  1128
<212>  DNA
<213>  Triticum aestivum

<400>  191
tcggcacgag ggaaatactt tccctttctc tttcttggtt gattgatcct cttccgcctc      60

cgtacctcgt gtcggtcctc gcgagttcgc cgtggaacac cccatgggct ccggcgagga     120

cacccccgga ggaaccggtg gaggagtcgg cgggatagtc cggggtgcgg tgctgaaggc     180

gctcgtcgtc ttcggcggcg ttattctgat ccggcggctg cgccgctcca ccacccggtg     240

ggaccacgcc cgcgccgtcg ccgacgccct ctctggtgaa aagttctcga gggagcaggc     300

gaggcaggat cctggaaact atttcaacct tagaatgctc acttgccctg caactgagat     360

ggtggatggt tctggagtgc tctactttga gcaagcattt tggagagctc cagaaaaacc     420

tttccgacaa agattctaca tggtaaagcc atgtccaaag gagatgaaat gtgacgttga     480

gttgagttct tatgcaatta gagatgttga agagtacaag aatttctgtg accgaccaaa     540

agatcagagg ccgcagccag aagaagtaat tgcggatatc gcagagcatt tgaccaccat     600

acacttatca cgatgtgaac gtggaaaacg ctgcttatat gaaggatcaa ccccaccggg     660

aggttttccc aacagttgga gcggtgcagc atattgtaca tctgatctgt ccattcataa     720

gaatggtgaa atacacattt gggacaaggg ttttgacgac gatgggagcc aggtttgggg     780

aaccaaggct ggcccttacg agtttaagcc tgctcccaag tccaattatg atgacatgtt     840

ctcaccttta aatttctctg cccccttgtc actagagaag atggaaagat catatgcaat     900

tgatgaccag tagatgagca ttgttaatac attttattgt tagagaacac cataagaata     960

tcatgtgttt atatattttg acagtatctc tgatcatcaa caaatggtaa ttctgtgggt    1020

gaacacttgc aatgcctgac aacatagtga gatataacaa ttgtcatatt gcagctaaca    1080

tgagttatgt aagcagacgg agcattaatt ctaatgttcg ttgggtgg                 1128

<210>  192
<211>  269
<212>  PRT
<213>  Triticum aestivum

<400>  192

Met Gly Ser Gly Glu Asp Thr Pro Gly Gly Thr Gly Gly Gly Val Gly
1               5                   10                  15

Gly Ile Val Arg Gly Ala Val Leu Lys Ala Leu Val Val Phe Gly Gly
            20                  25                  30

Val Ile Leu Ile Arg Arg Leu Arg Arg Ser Thr Thr Arg Trp Asp His
        35                  40                  45

Ala Arg Ala Val Ala Asp Ala Leu Ser Gly Glu Lys Phe Ser Arg Glu
    50                  55                  60

Gln Ala Arg Gln Asp Pro Gly Asn Tyr Phe Asn Leu Arg Met Leu Thr
65                  70                  75                  80

Cys Pro Ala Thr Glu Met Val Asp Gly Ser Gly Val Leu Tyr Phe Glu
                85                  90                  95

Gln Ala Phe Trp Arg Ala Pro Glu Lys Pro Phe Arg Gln Arg Phe Tyr
            100                 105                 110

Met Val Lys Pro Cys Pro Lys Glu Met Lys Cys Asp Val Glu Leu Ser
        115                 120                 125

Ser Tyr Ala Ile Arg Asp Val Glu Glu Tyr Lys Asn Phe Cys Asp Arg
    130                 135                 140

Pro Lys Asp Gln Arg Pro Gln Pro Glu Glu Val Ile Ala Asp Ile Ala
145                 150                 155                 160

Glu His Leu Thr Thr Ile His Leu Ser Arg Cys Glu Arg Gly Lys Arg
                165                 170                 175

Cys Leu Tyr Glu Gly Ser Thr Pro Pro Gly Gly Phe Pro Asn Ser Trp
            180                 185                 190

Ser Gly Ala Ala Tyr Cys Thr Ser Asp Leu Ser Ile His Lys Asn Gly
        195                 200                 205

Glu Ile His Ile Trp Asp Lys Gly Phe Asp Asp Asp Gly Ser Gln Val
    210                 215                 220

Trp Gly Thr Lys Ala Gly Pro Tyr Glu Phe Lys Pro Ala Pro Lys Ser
225                 230                 235                 240

Asn Tyr Asp Asp Met Phe Ser Pro Leu Asn Phe Ser Ala Pro Leu Ser
                245                 250                 255

Leu Glu Lys Met Glu Arg Ser Tyr Ala Ile Asp Asp Gln
            260                 265

<210>  193
<211>  1232
<212>  DNA

<213>  Sorghum bicolor

<400>  193
cccctccaaa aaccgctaat ttcctcccta ctccccagtc aatccccgcc ccgtgtcgcc      60

gtgtcggcca ggcccttgcg cggccgccgt ggaaggaccc ccagcgatgg gctccggcga     120

ggaagacaca ggaggcggag gaggggcggt gcggggcgcg gtgctgaagg cgctcgtcgt     180

cggcggcggc gtcctgctgc tccgccgcct gcgccgctcc accacccgtt gggaccacgc     240

gcgagccgtc gccgacgcgc tctccggaga aaagttctcg agggagcagg cgaggaagga     300

tcctgacaac ttcttcaatt tgagaatgct cacatgtcct gcaaccgaga cggtggatgg     360

ttcaagggtg ctttactttg agcatgcatt ttggagatct ccagaaaagc cttttagaca     420

aagattctac atggtaaagc cctgtccgaa ggagatgaaa tgcgatgttg agttgagttc     480

atatgcaatt agggatgctg aagagtacaa gaatttctgt gaccgtcaaa aggatcagag     540

gccacagcca gaagaagtaa ttgcggatat cgcagagcat ctgaccacca tacacttgtc     600

acggtgtggc cgtggaaaac gttgcttata tgaaggatct accccacctg aaggttttcc     660

caacaactgg aatggtgcat catattgtac atcggatttg tccatccaca aaaacggtga     720

agtacatatc tgggacaaag gttttgacga tgaagggaac caggtttggg gaaccaaggt     780

tggcccttac gagttcaagc ctgcccccaa atccaaatat gacgacatgt tctcgccatt     840

aaatttctcc gcccctttgt cactagagaa gaagttggat aaagcatatg taattgatga     900

ccagtagagg ctgagcccaa aattttgttc ataggaatgc aagaatagca tgtatgtata     960

tattgtactg agttctaatg catttttttt gaccaacctg tggatcttgg ttgtccatgt    1020

ttagtatggt gaggcatgat acctagtctg aaccaattta agctagtatg caagatgacc    1080

cagtcgcatt gtatttgtat cctatggtgt attatgccat gtcagaaaag atttgttcta    1140

agcaaattac atcagatggt atatagtgta ctatgacagt gacattaaga ttcctgatgt    1200

atatcgaatt atttgcaacc ctggagcatt ca                                  1232

<210>  194
<211>  266
<212>  PRT
<213>  Sorghum bicolor

<400>  194

Met Gly Ser Gly Glu Glu Asp Thr Gly Gly Gly Gly Gly Ala Val Arg
1               5                   10                  15

Gly Ala Val Leu Lys Ala Leu Val Val Gly Gly Gly Val Leu Leu Leu
            20                  25                  30

Arg Arg Leu Arg Arg Ser Thr Thr Arg Trp Asp His Ala Arg Ala Val
        35                  40                  45

Ala Asp Ala Leu Ser Gly Glu Lys Phe Ser Arg Glu Gln Ala Arg Lys
    50                  55                  60

Asp Pro Asp Asn Phe Phe Asn Leu Arg Met Leu Thr Cys Pro Ala Thr
65                  70                  75                  80

Glu Thr Val Asp Gly Ser Arg Val Leu Tyr Phe Glu His Ala Phe Trp
                85                  90                  95

Arg Ser Pro Glu Lys Pro Phe Arg Gln Arg Phe Tyr Met Val Lys Pro
            100                 105                 110

Cys Pro Lys Glu Met Lys Cys Asp Val Glu Leu Ser Ser Tyr Ala Ile
        115                 120                 125

Arg Asp Ala Glu Glu Tyr Lys Asn Phe Cys Asp Arg Gln Lys Asp Gln
    130                 135                 140

Arg Pro Gln Pro Glu Glu Val Ile Ala Asp Ile Ala Glu His Leu Thr
145                 150                 155                 160

Thr Ile His Leu Ser Arg Cys Gly Arg Gly Lys Arg Cys Leu Tyr Glu
                165                 170                 175

Gly Ser Thr Pro Pro Glu Gly Phe Pro Asn Asn Trp Asn Gly Ala Ser
            180                 185                 190

Tyr Cys Thr Ser Asp Leu Ser Ile His Lys Asn Gly Glu Val His Ile
        195                 200                 205

Trp Asp Lys Gly Phe Asp Asp Glu Gly Asn Gln Val Trp Gly Thr Lys
    210                 215                 220

Val Gly Pro Tyr Glu Phe Lys Pro Ala Pro Lys Ser Lys Tyr Asp Asp
225                 230                 235                 240

Met Phe Ser Pro Leu Asn Phe Ser Ala Pro Leu Ser Leu Glu Lys Lys
                245                 250                 255

Leu Asp Lys Ala Tyr Val Ile Asp Asp Gln
            260                 265

<210>  195
<211>  1239
<212>  DNA
<213>  Zea mays

<400>  195
ctccgccatt agtttgggtt gcccacagct tccgcgatcc acagaaaata catcgccatt      60

tccccaattt accgctgaca ccctccacaa accgcgaatt tcctccccgg ctcctctccc     120

tcctccccag tcaatcccca cacagtctcg gccgggccct cgcgaggccg ccgtggaagg     180

acccccagcg atgggctccg gcgaggagga cacaggaggc ggaggagggg cggtgcgggg     240

cgcggtgctg aaggcgctcg tggttgtcgg cggcgtcctg ctgctccgcc gcctgcgccg     300

ctccactacc cgatgggacc acgcgcgagc cgtcgccgac gcgctctccg gagaaaagtt     360

ctcgagggag caggcgagga aggatcctga caacttcttc aatttgagaa tgctcacatg     420

tcctgcaacc gagatggtgg atggttcaag ggtgctttac tttgagcagg cattttggag     480

atctccagaa aagcctttta gacaaagatt ctacatggta aagccctgtc cgaaggagat     540

gaaatgcgat gttgagttga gttcatatgc aattagggat gctgaagagt acaagaattt     600

ctgtgaccgt caaaaggatc agaggccaca ggcagaagaa gtaattgcag atatcgcaga     660

gcatctgacc accatacact tgtcacgatg cggacgagga aagcgttgct tatacgaagg     720

atctacccca cctgaaggtt ttcccaacaa ctggagcggc gcgtcgtact gtacgtcgga     780

tctgtccatc cacaaaaacg gcgaagtaca tatctgggac aaaggttttg acgacgaagg     840

gaaccaggtt tgggggacca aggctggccc ctacgagttc aagcctgctc ccaaatccaa     900

atatgacgac atgttctcgc cgttaaattt ctcggctcct ttgtcgctag agaagaagct     960

ggataaagca tatgtaattg atgaccagta gggcctgccc taaatttttg ttcataggaa    1020

tggaataagt gaatagaatg tatatgtata tactgtactg agttataatg cattctttgt    1080

tttgagcaaa aaaaaaaact tagttgtcca tgggtagcat ggtgaggcat ggtgatgcct    1140

tgtccgacca gatttgagtc agtacgcaag atgacccagt cactgtatac tatggtatgt    1200

tatttatgcc atgtcaggaa agatttgttc cagtcgcat                            1239

<210>  196
<211>  266
<212>  PRT
<213>  Zea mays

<400>  196

Met Gly Ser Gly Glu Glu Asp Thr Gly Gly Gly Gly Gly Ala Val Arg
1               5                   10                  15

Gly Ala Val Leu Lys Ala Leu Val Val Val Gly Gly Val Leu Leu Leu
            20                  25                  30

Arg Arg Leu Arg Arg Ser Thr Thr Arg Trp Asp His Ala Arg Ala Val
        35                  40                  45

Ala Asp Ala Leu Ser Gly Glu Lys Phe Ser Arg Glu Gln Ala Arg Lys
    50                  55                  60

Asp Pro Asp Asn Phe Phe Asn Leu Arg Met Leu Thr Cys Pro Ala Thr
65                  70                  75                  80

Glu Met Val Asp Gly Ser Arg Val Leu Tyr Phe Glu Gln Ala Phe Trp
                85                  90                  95

Arg Ser Pro Glu Lys Pro Phe Arg Gln Arg Phe Tyr Met Val Lys Pro
            100                 105                 110

Cys Pro Lys Glu Met Lys Cys Asp Val Glu Leu Ser Ser Tyr Ala Ile
        115                 120                 125

Arg Asp Ala Glu Glu Tyr Lys Asn Phe Cys Asp Arg Gln Lys Asp Gln
    130                 135                 140

Arg Pro Gln Ala Glu Glu Val Ile Ala Asp Ile Ala Glu His Leu Thr
145                 150                 155                 160

Thr Ile His Leu Ser Arg Cys Gly Arg Gly Lys Arg Cys Leu Tyr Glu
                165                 170                 175

Gly Ser Thr Pro Pro Glu Gly Phe Pro Asn Asn Trp Ser Gly Ala Ser
            180                 185                 190

Tyr Cys Thr Ser Asp Leu Ser Ile His Lys Asn Gly Glu Val His Ile
        195                 200                 205

Trp Asp Lys Gly Phe Asp Asp Glu Gly Asn Gln Val Trp Gly Thr Lys
    210                 215                 220

Ala Gly Pro Tyr Glu Phe Lys Pro Ala Pro Lys Ser Lys Tyr Asp Asp
225                 230                 235                 240

Met Phe Ser Pro Leu Asn Phe Ser Ala Pro Leu Ser Leu Glu Lys Lys
                245                 250                 255

Leu Asp Lys Ala Tyr Val Ile Asp Asp Gln
            260                 265

<210>  197
<211>  828
<212>  DNA
<213>  Oryza sativa

<400>  197
   atgggctccg gcgaggacac cggcgcgggc gtcgcgggag gagggggagg cggaggcgcc      60
   ggtggggtgg tgcggggcgc ggtgctgaag gcgctcgtgg tcgtcggcgg cgtgctgctg 120
   ctccggcggc tgcgccgctc caccacccgg tgggaccacg cccgcgccgt cgtggacgcg 180
   ctctccggtg agaagttctc gagggagcag gcgaggaagg atcctgataa ctactttaat 240
   ttgaggatgc ttacatgccc tgcaacagag atggtggatg gttctagagt gctttacttt 300
   gagcaagcat tttggagaag tccagaaaaa cctttccgac aaagattcta catggtaaag 360
   ccatgcccaa aggatatgaa atgtgatgtt gagttgagtt catatgcaat tagagatgtt 420
   gaagagtaca agaatttctg tgaccgtcca aaggatcaga ggccacaacc agaagaagtc 480
   attgcggaca ttgcagagca cctgactacc atacacttgt cgcggtgtga gcgtggaaag 540
   cgctgcttgt acaaaggatc aacccctcct gaaggctttc ccaacagctg gagcggtgcg 600
   acatattgta catcggattt gtccattcac aagaatggtg aggtgcatat ctgggacaag 660
   ggttttgacg atgatgggaa tcaggtttgg ggaaccaaag ctggccctta cgagttcaag 720
   cctgccccca agtcgaatta cgacgacatg ttctcgccgt tgaatttttc tgctccattg 780
   acgctggaga agaagattga gagctcgttc gcaatcgatg atcagtag 828

<210> 198
<211> 275
<212> PRT
<213> Oryza sativa

<400> 198

Met Gly Ser Gly Glu Asp Thr Gly Ala Gly Val Ala Gly Gly Gly Gly
1               5                   10                  15

Gly Gly Gly Ala Gly Gly Val Val Arg Gly Ala Val Leu Lys Ala Leu
            20                  25                  30

Val Val Val Gly Gly Val Leu Leu Leu Arg Arg Leu Arg Arg Ser Thr
        35                  40                  45

Thr Arg Trp Asp His Ala Arg Ala Val Val Asp Ala Leu Ser Gly Glu
    50                  55                  60

Lys Phe Ser Arg Glu Gln Ala Arg Lys Asp Pro Asp Asn Tyr Phe Asn
65                  70                  75                  80

Leu Arg Met Leu Thr Cys Pro Ala Thr Glu Met Val Asp Gly Ser Arg
                85                  90                  95

Val Leu Tyr Phe Glu Gln Ala Phe Trp Arg Ser Pro Glu Lys Pro Phe
            100                 105                 110

Arg Gln Arg Phe Tyr Met Val Lys Pro Cys Pro Lys Asp Met Lys Cys
        115                 120                 125

Asp Val Glu Leu Ser Ser Tyr Ala Ile Arg Asp Val Glu Glu Tyr Lys
    130                 135                 140

Asn Phe Cys Asp Arg Pro Lys Asp Gln Arg Pro Gln Pro Glu Glu Val
145                 150                 155                 160

Ile Ala Asp Ile Ala Glu His Leu Thr Thr Ile His Leu Ser Arg Cys
                165                 170                 175

Glu Arg Gly Lys Arg Cys Leu Tyr Lys Gly Ser Thr Pro Pro Glu Gly
            180                 185                 190

Phe Pro Asn Ser Trp Ser Gly Ala Thr Tyr Cys Thr Ser Asp Leu Ser
        195                 200                 205

Ile His Lys Asn Gly Glu Val His Ile Trp Asp Lys Gly Phe Asp Asp
    210                 215                 220

Asp Gly Asn Gln Val Trp Gly Thr Lys Ala Gly Pro Tyr Glu Phe Lys
225                 230                 235                 240

Pro Ala Pro Lys Ser Asn Tyr Asp Asp Met Phe Ser Pro Leu Asn Phe
                245                 250                 255

Ser Ala Pro Leu Thr Leu Glu Lys Lys Ile Glu Ser Ser Phe Ala Ile
            260                 265                 270

Asp Asp Gln
        275

<210>  199
<211>  828
<212>  DNA
<213>  Oryza sativa

<400>  199
atgggctccg gcgaggacac cggcgcgggc gtcgcgggag gagggggagg cggaggcgcc      60

ggtggggtgg tgcggggcgc ggtgctgaag gcgctcgtgg tcgtcggcgg cgtgctgctg     120

ctccggcggc tgcgccgctc caccacccgg tgggaccacg cccgcgccgt cgtggacgcg     180

ctctccggtg agaagttctc gagggagcag gcgaggaagg atcctgataa ctactttaat     240

ttgaggatgc ttacatgccc tgcaacagag atggtggatg gttctagagt gctttacttt     300

gagcaagcat tttggagaag tccagaaaaa cctttccgac aaagattcta catggtaaag     360

ccatgcccaa aggatatgaa atgtgatgtt gagttgagtt catatgcaat tagagatgtt     420

gaagagtaca agaatttctg tgaccgtcca aaggatcaga ggccacaacc agaagaagtc 480

attgcggaca ttgcagagca cctgactacc atacacttgt cgcggtgtga gcgtggaaag 540

cgctgcttgt acaaaggatc aacccctcct gaaggctttc ccaacagctg gagcggtgcg 600

acatattgta catcggattt gtccattcac aagaatggtg aggtgcatat ctgggacaag 660

ggttttgacg atgatgggaa tcaggtttgg ggaaccaaag ctggccctta cgagttcaag 720

cctgccccca agtcgaatta cgacgacatg ttctcgccgt tgaatttttc tgctccattg 780

acgctggaga agaagattga gagctcgttc gcaatcgatg atcagtag 828

<210> 200
<211> 275
<212> PRT
<213> Oryza sativa

<400> 200

Met Gly Ser Gly Glu Asp Thr Gly Ala Gly Val Ala Gly Gly Gly Gly
1               5                   10                  15

Gly Gly Gly Ala Gly Gly Val Val Arg Gly Ala Val Leu Lys Ala Leu
            20                  25                  30

Val Val Val Gly Gly Val Leu Leu Leu Arg Arg Leu Arg Arg Ser Thr
        35                  40                  45

Thr Arg Trp Asp His Ala Arg Ala Val Val Asp Ala Leu Ser Gly Glu
    50                  55                  60

Lys Phe Ser Arg Glu Gln Ala Arg Lys Asp Pro Asp Asn Tyr Phe Asn
65                  70                  75                  80

Leu Arg Met Leu Thr Cys Pro Ala Thr Glu Met Val Asp Gly Ser Arg
                85                  90                  95

Val Leu Tyr Phe Glu Gln Ala Phe Trp Arg Ser Pro Glu Lys Pro Phe
            100                 105                 110

Arg Gln Arg Phe Tyr Met Val Lys Pro Cys Pro Lys Asp Met Lys Cys
        115                 120                 125

Asp Val Glu Leu Ser Ser Tyr Ala Ile Arg Asp Val Glu Glu Tyr Lys
    130                 135                 140

Asn Phe Cys Asp Arg Pro Lys Asp Gln Arg Pro Gln Pro Glu Glu Val
145                 150                 155                 160

Ile Ala Asp Ile Ala Glu His Leu Thr Thr Ile His Leu Ser Arg Cys
                165                 170                 175

Glu Arg Gly Lys Arg Cys Leu Tyr Lys Gly Ser Thr Pro Pro Glu Gly
            180                 185                 190

Phe Pro Asn Ser Trp Ser Gly Ala Thr Tyr Cys Thr Ser Asp Leu Ser
        195                 200                 205

Ile His Lys Asn Gly Glu Val His Ile Trp Asp Lys Gly Phe Asp Asp
    210                 215                 220

Asp Gly Asn Gln Val Trp Gly Thr Lys Ala Gly Pro Tyr Glu Phe Lys
225                 230                 235                 240

Pro Ala Pro Lys Ser Asn Tyr Asp Asp Met Phe Ser Pro Leu Asn Phe
                245                 250                 255

Ser Ala Pro Leu Thr Leu Glu Lys Lys Ile Glu Ser Ser Phe Ala Ile
            260                 265                 270

Asp Asp Gln
        275

<210>  201
<211>  834
<212>  DNA
<213>  Physcomitrella patens

<400>  201
atggctgggg ctagtgatgc cggcccaagc ttcagtttag gtgagaggta taatccgggg      60

ggaagctata aaatcaatgg gagctatgag gtggctggga gcgggagcgg tggtggtggc     120

ggtggcggcg gcggcgggag gatggtgaga ggattggtaa ttaaagctgc gtgtctcatt     180

ggaggagctt ttctccttcg caagttgacc aagagcacaa ctcgttggga ccatgctcgc     240

aaggttgccc aatccctcag cggcgagaag ttttccacag agcaagcagc ccgagatcct     300

acgacgtatt ttaatctcag cagattgctc acttgcccag ccaccgtatt agcagatgga     360

gctcgtgtca tgtatttcga gcaggctttt tggaggaccc cagaaagacc gtatcgtcag     420

agattctaca gcattaagcc ttgtcccaag gaaatgaaat gtgacgtaga ggtcagttca     480

tacgctgtgc gagatattga agaatacaaa aacttttgtg accgctccaa agatgaaagg     540

cctcaacctg atgaggtgct caaggacatg gcagagcatc tgaacactgt ctatctttct     600

gtctgtgaac gtggacggcg ttgtttgtat gaggggtcta ctcctcctgg aggttttccc     660

aactcctgga atggagcgtc cagatgcaca tcagaattga ccatctacaa aaatggggaa     720

gttcattgct gggaccgtgc ctacgacgat gagggcaatc aggtttgggg cgtaagacaa     780

gggccttacg aattcaagac tgcaacgtct ccacggatca caactgaaat ttaa          834

<210>  202
<211>  277
<212>  PRT
<213>  Physcomitrella patens

<400>  202

Met Ala Gly Ala Ser Asp Ala Gly Pro Ser Phe Ser Leu Gly Glu Arg
1               5                   10                  15

Tyr Asn Pro Gly Gly Ser Tyr Lys Ile Asn Gly Ser Tyr Glu Val Ala
            20                  25                  30

Gly Ser Gly Ser Gly Gly Gly Gly Gly Gly Gly Gly Gly Gly Arg Met
        35                  40                  45

Val Arg Gly Leu Val Ile Lys Ala Ala Cys Leu Ile Gly Gly Ala Phe
    50                  55                  60

Leu Leu Arg Lys Leu Thr Lys Ser Thr Thr Arg Trp Asp His Ala Arg
65                  70                  75                  80

Lys Val Ala Gln Ser Leu Ser Gly Glu Lys Phe Ser Thr Glu Gln Ala
                85                  90                  95

Ala Arg Asp Pro Thr Thr Tyr Phe Asn Leu Ser Arg Leu Leu Thr Cys
            100                 105                 110

Pro Ala Thr Val Leu Ala Asp Gly Ala Arg Val Met Tyr Phe Glu Gln
        115                 120                 125

Ala Phe Trp Arg Thr Pro Glu Arg Pro Tyr Arg Gln Arg Phe Tyr Ser
    130                 135                 140

Ile Lys Pro Cys Pro Lys Glu Met Lys Cys Asp Val Glu Val Ser Ser
145                 150                 155                 160

Tyr Ala Val Arg Asp Ile Glu Glu Tyr Lys Asn Phe Cys Asp Arg Ser
                165                 170                 175

Lys Asp Glu Arg Pro Gln Pro Asp Glu Val Leu Lys Asp Met Ala Glu
            180                 185                 190

His Leu Asn Thr Val Tyr Leu Ser Val Cys Glu Arg Gly Arg Arg Cys
        195                 200                 205

Leu Tyr Glu Gly Ser Thr Pro Pro Gly Gly Phe Pro Asn Ser Trp Asn
    210                 215                 220

Gly Ala Ser Arg Cys Thr Ser Glu Leu Thr Ile Tyr Lys Asn Gly Glu
225                 230                 235                 240

Val His Cys Trp Asp Arg Ala Tyr Asp Asp Glu Gly Asn Gln Val Trp
                245                 250                 255

Gly Val Arg Gln Gly Pro Tyr Glu Phe Lys Thr Ala Thr Ser Pro Arg
            260                 265                 270

Ile Thr Thr Glu Ile
        275

<210>  203
<211>  1241
<212>  DNA
<213>  Pinus taeda

<400>  203
caaacgctct agcaacgttg ggcattgaat cctctaacac gcgacacatt ccagtgtggc      60

aaacaaatga ttgaatcaat cctctgaagg caaggcagta caagatggtt gatggaattt     120

gtttgcggga ggaggaaacg agcaggtgca gtggagcgcc tgcttatacg gatgataata     180

acaaagccgg aagtggaagt ggaagtggaa gcggaagaag ggttataaat gcagtgctca     240

aggcattatg tgtggctgga gggggtttcc tgattaggaa attcaccaag ttcaccactc     300

ggcaggatca gacgcgaatt gttgcagaag ccctctgcgg agagaagtca tcaagcgagc     360

aagcagcagg gcagcctatg acctatttca atctcagatg gctgacatgt ccggcaacta     420

caattgtgaa tggatccaga gttctttatt ttgaacaggc attttggaga acgcccaaga     480

agccttatcg tcagaggttt tttgttgtca agccgtgtcc taaagagatg aagtgtgatg     540

tggaggtggc ttcctttgca gtaagggaca ttgaagagta ccaaaacttt tgtgaaagac     600

caaagagtca aagaccagat gcccaaaatg tcatagggga cattgcagaa catctgaaca     660

cagtttatct gtcaaaatgt gaaaagggga ggaggtgctt atatcaaggt tccactccac     720

tagggggttt ccccaattca tggaatggtg ccactcattg cacatcagag cttacaatct     780

ataggaatgg agaaattcat tgctgggatc gggcctatga tgatgaagga aatcaggtat     840

ggggagtcag ggaaggtcct tatgaattca aacctgcaac aacaactact ttttgaggtc     900

tctcttttcc actagggttt tcatcttctt ctacagggaa ctacaatcca agtccgttta     960

gttgcagcag agaagaatct tctcatcggt aatttttata gggtgaatat atcaaaatgg    1020

ccttgtaaag tgaatattat ttgatctata atgttttcat gatggagatt ttaaaacagt    1080

caatatatca aatgcagaaa ataaggtgta tatatatgta acgacatgga ttacaaggaa    1140

gtcttttgtt atatcatttc tagaacataa ccgaaagctg aaatgtattt ttctccactg   1200

aactccaatt cttctgctct gaattagagt tcaatttttt t                        1241

<210>  204
<211>  263
<212>  PRT
<213>  Pinus taeda

<400>  204

Met Val Asp Gly Ile Cys Leu Arg Glu Glu Glu Thr Ser Arg Cys Ser
1               5                   10                  15

Gly Ala Pro Ala Tyr Thr Asp Asp Asn Asn Lys Ala Gly Ser Gly Ser
            20                  25                  30

Gly Ser Gly Ser Gly Arg Arg Val Ile Asn Ala Val Leu Lys Ala Leu
        35                  40                  45

Cys Val Ala Gly Gly Gly Phe Leu Ile Arg Lys Phe Thr Lys Phe Thr
    50                  55                  60

Thr Arg Gln Asp Gln Thr Arg Ile Val Ala Glu Ala Leu Cys Gly Glu
65                  70                  75                  80

Lys Ser Ser Ser Glu Gln Ala Ala Gly Gln Pro Met Thr Tyr Phe Asn
                85                  90                  95

Leu Arg Trp Leu Thr Cys Pro Ala Thr Thr Ile Val Asn Gly Ser Arg
            100                 105                 110

Val Leu Tyr Phe Glu Gln Ala Phe Trp Arg Thr Pro Lys Lys Pro Tyr
        115                 120                 125

Arg Gln Arg Phe Phe Val Val Lys Pro Cys Pro Lys Glu Met Lys Cys
    130                 135                 140

Asp Val Glu Val Ala Ser Phe Ala Val Arg Asp Ile Glu Glu Tyr Gln
145                 150                 155                 160

Asn Phe Cys Glu Arg Pro Lys Ser Gln Arg Pro Asp Ala Gln Asn Val
                165                 170                 175

Ile Gly Asp Ile Ala Glu His Leu Asn Thr Val Tyr Leu Ser Lys Cys
            180                 185                 190

Glu Lys Gly Arg Arg Cys Leu Tyr Gln Gly Ser Thr Pro Leu Gly Gly
        195                 200                 205

Phe Pro Asn Ser Trp Asn Gly Ala Thr His Cys Thr Ser Glu Leu Thr
    210                 215                 220

Ile Tyr Arg Asn Gly Glu Ile His Cys Trp Asp Arg Ala Tyr Asp Asp
225                 230                 235                 240

Glu Gly Asn Gln Val Trp Gly Val Arg Glu Gly Pro Tyr Glu Phe Lys
                245                 250                 255

Pro Ala Thr Thr Thr Thr Phe
            260

<210>  205
<211>  907
<212>  DNA
<213>  Selaginella moellendorffii

<400>  205
gcttattgtc ctcttcccct ctcggctatg gcgggaggcg acagggagga cgcctcctcg      60

ctccaatcct ccggtggcgc tggaaatgtg agcgtcggcg gcggtgacgg aggaggcggc     120

ggcgttcgca gcttcgtctt gaaagcggca tgtctgctgg gtggtgttct tctgctccgc     180

aagctcacca aagcaaagac gcggtgggat cacactcgtc tcgtcgccga tgctctcaca     240

ggcgagaaat tttcccaaga gcaagcggca agggatccaa tgacgtactt caatctcagg     300

atgctggcat gccccgcgac tgttctggac gatggagcga aagttctcta ctttgaacag     360

gcattctgga gaacacctga taagccatat agacagagat tctacgttgt gaggccctgt     420

ccgaaagaaa tgaagtgcga tgtggaggtt ggatcctacg ctgttcgtga cattgaggaa     480

tacaagaact tctgtgagag gccaaaggat cagcgaccac agccggaaga gatccctgga     540

gacatctccg agcatttaac ttccgtctat ctctccgcct gtgcgcgagg ccaacgttgt     600

ctctacgaag gatcaacacc tcccggaggc tttccaaaca actggaacgg tgcttcccgg     660

tgcacatccg agctcacaat cctcaaaagt ggagagatcc actgctggga tcgcgcctac     720

gacgacgaag gaaatcaggt gtggggtgta agacaggggc catacgaatt caagcctgga     780

acctccaaga acagatctta cgttgagcat gacacagcgt ccctttccat tgacaactag     840

aaactaagat agtgtgcctt ttgtaacata atagattttg gaataatcct actcgagatt     900

gggtttc                                                               907

<210>  206
<211>  270
<212>  PRT
<213>  Selaginella moellendorffii

<400>  206

Met Ala Gly Gly Asp Arg Glu Asp Ala Ser Ser Leu Gln Ser Ser Gly
1               5                   10                  15

Gly Ala Gly Asn Val Ser Val Gly Gly Gly Asp Gly Gly Gly Gly Gly
            20                  25                  30

Val Arg Ser Phe Val Leu Lys Ala Ala Cys Leu Leu Gly Gly Val Leu
        35                  40                  45

Leu Leu Arg Lys Leu Thr Lys Ala Lys Thr Arg Trp Asp His Thr Arg
    50                  55                  60

Leu Val Ala Asp Ala Leu Thr Gly Glu Lys Phe Ser Gln Glu Gln Ala
65                  70                  75                  80

Ala Arg Asp Pro Met Thr Tyr Phe Asn Leu Arg Met Leu Ala Cys Pro
                85                  90                  95

Ala Thr Val Leu Asp Asp Gly Ala Lys Val Leu Tyr Phe Glu Gln Ala
            100                 105                 110

Phe Trp Arg Thr Pro Asp Lys Pro Tyr Arg Gln Arg Phe Tyr Val Val
        115                 120                 125

Arg Pro Cys Pro Lys Glu Met Lys Cys Asp Val Glu Val Gly Ser Tyr
    130                 135                 140

Ala Val Arg Asp Ile Glu Glu Tyr Lys Asn Phe Cys Glu Arg Pro Lys
145                 150                 155                 160

Asp Gln Arg Pro Gln Pro Glu Glu Ile Pro Gly Asp Ile Ser Glu His
                165                 170                 175

Leu Thr Ser Val Tyr Leu Ser Ala Cys Ala Arg Gly Gln Arg Cys Leu
            180                 185                 190

Tyr Glu Gly Ser Thr Pro Pro Gly Gly Phe Pro Asn Asn Trp Asn Gly
        195                 200                 205

Ala Ser Arg Cys Thr Ser Glu Leu Thr Ile Leu Lys Ser Gly Glu Ile
    210                 215                 220

His Cys Trp Asp Arg Ala Tyr Asp Asp Glu Gly Asn Gln Val Trp Gly
225                 230                 235                 240

Val Arg Gln Gly Pro Tyr Glu Phe Lys Pro Gly Thr Ser Lys Asn Arg
                245                 250                 255

Ser Tyr Val Glu His Asp Thr Ala Ser Leu Ser Ile Asp Asn
            260                 265                 270

<210>  207
<211>  15
<212>  PRT
<213>  Artificial sequence

<220>
<223>  motif 15

<220>
<221>  misc_feature
<222>  (7)..(7)
<223>  Xaa can be any naturally occurring amino acid

<220>
<221>  misc_feature
<222>  (9)..(9)
<223>  Xaa can be any naturally occurring amino acid

<400>  207

Glu Gln Ala Phe Trp Arg Xaa Pro Xaa Lys Pro Phe Arg Gln Arg
1               5                   10                  15

<210>  208
<211>  5
<212>  PRT
<213>  Artificial sequence

<220>
<223>  motif 16

<400>  208

Asn Phe Cys Asp Arg
1               5

<210>  209
<211>  10
<212>  PRT
<213>  Artificial sequence

<220>
<223>  motif 17

<400>  209

Arg Gly Lys Arg Cys Leu Tyr Glu Gly Ser
1               5                   10

<210>  210
<211>  13
<212>  PRT
<213>  Artificial sequence

<220>
<223>  motif 18


<220>
<221>  misc_feature
<222>  (5)..(5)
<223>  Xaa can be any naturally occurring amino acid

<220>
<221>  misc_feature
<222>  (7)..(7)
<223>  Xaa can be any naturally occurring amino acid

<400>  210

Gln Val Trp Gly Xaa Lys Xaa Gly Pro Tyr Glu Phe Lys
1               5                   10


<210>  211
<211>  52
<212>  DNA
<213>  Artificial sequence

<220>
<223>  primer 3

<400>  211
ggggacaagt ttgtacaaaa aagcaggctt aaacaatggg taccgagtcg gg          52


<210>  212
<211>  50
<212>  DNA
<213>  Artificial sequence

<220>
<223>  primer 4

<400>  212
ggggaccact ttgtacaaga aagctgggtt cagacaatag aaaagggggt             50


<210>  213
<211>  2194
<212>  DNA
<213>  Oryza sativa

<400>  213
aatccgaaaa gtttctgcac cgttttcacc ccctaactaa caatataggg aacgtgtgct      60

aaatataaaa tgagacctta tatatgtagc gctgataact agaactatgc aagaaaaact     120

catccaccta ctttagtggc aatcgggcta aataaaaaag agtcgctaca ctagtttcgt     180

tttccttagt aattaagtgg gaaaatgaaa tcattattgc ttagaatata cgttcacatc     240

tctgtcatga agttaaatta ttcgaggtag ccataattgt catcaaactc ttcttgaata     300

aaaaaatctt tctagctgaa ctcaatgggt aaagagagag atttttttta aaaaaataga     360

atgaagatat tctgaacgta ttggcaaaga tttaaacata taattatata attttatagt 420

ttgtgcattc gtcatatcgc acatcattaa ggacatgtct tactccatcc caatttttat 480

ttagtaatta aagacaattg acttattttt attatttatc ttttttcgat tagatgcaag 540

gtacttacgc acacactttg tgctcatgtg catgtgtgag tgcacctcct caatacacgt 600

tcaactagca acacatctct aatatcactc gcctatttaa tacatttagg tagcaatatc 660

tgaattcaag cactccacca tcaccagacc acttttaata atatctaaaa tacaaaaaat 720

aattttacag aatagcatga aaagtatgaa acgaactatt taggtttttc acatacaaaa 780

aaaaaaagaa ttttgctcgt gcgcgagcgc caatctccca tattgggcac acaggcaaca 840

acagagtggc tgcccacaga acaacccaca aaaaacgatg atctaacgga ggacagcaag 900

tccgcaacaa ccttttaaca gcaggctttg cggccaggag agaggaggag aggcaaagaa 960

aaccaagcat cctccttctc ccatctataa attcctcccc ccttttcccc tctctatata 1020

ggaggcatcc aagccaagaa gagggagagc accaaggaca cgcgactagc agaagccgag 1080

cgaccgcctt ctcgatccat atcttccggt cgagttcttg gtcgatctct tccctcctcc 1140

acctcctcct cacagggtat gtgcctccct tcggttgttc ttggatttat tgttctaggt 1200

tgtgtagtac gggcgttgat gttaggaaag gggatctgta tctgtgatga ttcctgttct 1260

tggatttggg atagaggggt tcttgatgtt gcatgttatc ggttcggttt gattagtagt 1320

atggttttca atcgtctgga gagctctatg gaaatgaaat ggtttaggga tcggaatctt 1380

gcgattttgt gagtaccttt tgtttgaggt aaaatcagag caccggtgat tttgcttggt 1440

gtaataaagt acggttgttt ggtcctcgat tctggtagtg atgcttctcg atttgacgaa 1500

gctatccttt gtttattccc tattgaacaa aaataatcca actttgaaga cggtcccgtt 1560

gatgagattg aatgattgat tcttaagcct gtccaaaatt tcgcagctgg cttgtttaga 1620

tacagtagtc cccatcacga aattcatgga aacagttata atcctcagga acaggggatt 1680

ccctgttctt ccgatttgct ttagtcccag aatttttttt cccaaatatc ttaaaaagtc 1740

actttctggt tcagttcaat gaattgattg ctacaaataa tgcttttata gcgttatcct 1800

agctgtagtt cagttaatag gtaatacccc tatagtttag tcaggagaag aacttatccg 1860

atttctgatc tccattttta attatatgaa atgaactgta gcataagcag tattcatttg 1920

gattattttt tttattagct ctcacccctt cattattctg agctgaaagt ctggcatgaa 1980

ctgtcctcaa ttttgttttc aaattcacat cgattatcta tgcattatcc tcttgtatct 2040

acctgtagaa gtttcttttt ggttattcct tgactgcttg attacagaaa gaaatttatg 2100

aagctgtaat cgggatagtt atactgcttg ttcttatgat tcatttcctt tgtgcagttc 2160

ttggtgtagc ttgccacttt caccagcaaa gttc 2194

## Description

The present invention relates generally to the field of molecular biology and concerns a method for enhancing abiotic stress tolerance in plants by modulating expression in a plant of a nucleic acid encoding an alfin-like. The present invention also concerns plants having modulated expression of a nucleic acid encoding an alfin-like polypeptide, which plants have enhanced abiotic stress tolerance relative to corresponding wild type plants or other control plants. The invention also provides constructs useful in the methods of the invention.

Furthermore, he present invention relates generally to the field of molecular biology and concerns a method for enhancing abiotic stress tolerance in plants by modulating expression in a plant of a nucleic acid encoding a YRP. The present invention also concerns plants having modulated expression of a nucleic acid encoding a YRP, which plants have enhanced abiotic stress tolerance relative to corresponding wild type plants or other control plants. The invention also provides constructs useful in the methods of the invention.

Furthermore, the present invention relates generally to the field of molecular biology and concerns a method for increasing various plant yield-related traits by increasing expression in a plant of a nucleic acid sequence encoding a Brevis Radix-like (BRXL) polypeptide. The present invention also concerns plants having increased expression of a nucleic acid sequence encoding a BRXL polypeptide, which plants have increased yield-related traits relative to control plants. The invention additionally relates to nucleic acid sequences, nucleic acid constructs, vectors and plants containing said nucleic acid sequences.

Furthermore, the present invention relates generally to the field of molecular biology and concerns a method for enhancing abiotic stress tolerance in plants by modulating expression in a plant of a nucleic acid encoding a silky-1-like polypeptide. The present invention also concerns plants having modulated expression of a nucleic acid encoding a silky-1-like polypeptide, which plants have enhanced abiotic stress tolerance relative to corresponding wild type plants or other control plants. The invention also provides constructs useful in the methods of the invention.

Furthermore, the present invention relates generally to the field of molecular biology and concerns a method for improving various plant growth characteristics by modulating expression in a plant of a nucleic acid encoding an ARP6 (Actin Related Protein 6). The present invention also concerns plants having modulated expression of a nucleic acid encoding an ARP, which plants have improved growth characteristics relative to corresponding wild type plants or other control plants. The invention also provides constructs useful in the methods of the invention.

Furthermore, the present invention relates generally to the field of molecular biology and concerns a method for enhancing yield-relating traits in plants by modulating expression in a plant of a nucleic acid encoding a POP (Prolyl-oligopeptidase) polypeptide. The present invention also concerns plants having modulated expression of a nucleic acid encoding a POP polypeptide, which plants have enhanced yield-relating traits in plants relative to corresponding wild type plants or other control plants. The invention also provides constructs useful in the methods of the invention.

Furthermore, the present invention relates generally to the field of molecular biology and concerns a method for enhancing yield-related traits by modulating expression in a plant of a nucleic acid encoding a CRL (Crampled Leaf). The present invention also concerns plants having modulated expression of a nucleic acid encoding a CRL, which plants have enhanced yield-related traits relative to corresponding wild type plants or other control plants. The invention also provides constructs useful in the methods of the invention.

The ever-increasing world population and the dwindling supply of arable land available for agriculture fuels research towards increasing the efficiency of agriculture. Conventional means for crop and horticultural improvements utilise selective breeding techniques to identify plants having desirable characteristics. However, such selective breeding techniques have several drawbacks, namely that these techniques are typically labour intensive and result in plants that often contain heterogeneous genetic components that may not always result in the desirable trait being passed on from parent plants. Advances in molecular biology have allowed mankind to modify the germplasm of animals and plants. Genetic engineering of plants entails the isolation and manipulation of genetic material (typically in the form of DNA or RNA) and the subsequent introduction of that genetic material into a plant. Such technology has the capacity to deliver crops or plants having various improved economic, agronomic or horticultural traits.

A trait of particular economic interest is increased yield. Yield is normally defined as the measurable produce of economic value from a crop. This may be defined in terms of quantity and/or quality. Yield is directly dependent on several factors, for example, the number and size of the organs, plant architecture (for example, the number of branches), seed production, leaf senescence and more. Root development, nutrient uptake, stress tolerance and early vigour may also be important factors in determining yield. Optimizing the abovementioned factors may therefore contribute to increasing crop yield.

Seed yield is a particularly important trait, since the seeds of many plants are important for human and animal nutrition. Crops such as corn, rice, wheat, canola and soybean account for over half the total human caloric intake, whether through direct consumption of the seeds themselves or through consumption of meat products raised on processed seeds. They are also a source of sugars, oils and many kinds of metabolites used in industrial processes. Seeds contain an embryo (the source of new shoots and roots) and an endosperm (the source of nutrients for embryo growth during germination and during early growth of seedlings). The development of a seed involves many genes, and requires the transfer of metabolites from the roots, leaves and stems into the growing seed. The endosperm, in particular, assimilates the metabolic precursors of carbohydrates, oils and proteins and synthesizes them into storage macromolecules to fill out the grain.

Plant biomass is yield for forage crops like alfalfa, silage corn and hay. Many proxies for yield have been used in grain crops. Chief amongst these are estimates of plant size. Plant size can be measured in many ways depending on species and developmental stage, but include total plant dry weight, above-ground dry weight, above-ground fresh weight, leaf area, stem volume, plant height, rosette diameter, leaf length, root length, root mass, tiller number and leaf number. Many species maintain a conservative ratio between the size of different parts of the plant at a given developmental stage. These allometric relationships are used to extrapolate from one of these measures of size to another (e.g. Tittonell et al 2005 Agric Ecosys & Environ 105: 213). Plant size at an early developmental stage will typically correlate with plant size later in development. A larger plant with a greater leaf area can typically absorb more light and carbon dioxide than a smaller plant and therefore will likely gain a greater weight during the same period (Fasoula & Tollenaar 2005 Maydica 50:39). This is in addition to the potential continuation of the micro-environmental or genetic advantage that the plant had to achieve the larger size initially. There is a strong genetic component to plant size and growth rate (e.g. ter Steege et al 2005 Plant Physiology 139:1078), and so for a range of diverse genotypes plant size under one environmental condition is likely to correlate with size under another (Hittalmani et al 2003 Theoretical Applied Genetics 107:679). In this way a standard environment is used as a proxy for the diverse and dynamic environments encountered at different locations and times by crops in the field.

Another important trait for many crops is early vigour. Improving early vigour is an important objective of modern rice breeding programs in both temperate and tropical rice cultivars. Long roots are important for proper soil anchorage in water-seeded rice. Where rice is sown directly into flooded fields, and where plants must emerge rapidly through water, longer shoots are associated with vigour. Where drill-seeding is practiced, longer mesocotyls and coleoptiles are important for good seedling emergence. The ability to engineer early vigour into plants would be of great importance in agriculture. For example, poor early vigour has been a limitation to the introduction of maize (Zea mays L.) hybrids based on Corn Belt germplasm in the European Atlantic.

Harvest index, the ratio of seed yield to aboveground dry weight, is relatively stable under many environmental conditions and so a robust correlation between plant size and grain yield can often be obtained (e.g. Rebetzke et al 2002 Crop Science 42:739). These processes are intrinsically linked because the majority of grain biomass is dependent on current or stored photosynthetic productivity by the leaves and stem of the plant (Gardener et al. (1985) Physiology of Crop Plants. Iowa State University Press, pp 68-73). Therefore, selecting for plant size, even at early stages of development, has been used as an indicator for future potential yield (e.g. Tittonell et al 2005 Agric Ecosys & Environ 105: 213). When testing for the impact of genetic differences on stress tolerance, the ability to standardize soil properties, temperature, water and nutrient availability and light intensity is an intrinsic advantage of greenhouse or plant growth chamber environments compared to the field. However, artificial limitations on yield due to poor pollination due to the absence of wind or insects, or insufficient space for mature root or canopy growth, can restrict the use of these controlled environments for testing yield differences. Therefore, measurements of plant size in early development, under standardized conditions in a growth chamber or greenhouse, are standard practices to provide indication of potential genetic yield advantages.

Another trait of particular importance is that of improved abiotic stress tolerance. Abiotic stress is a primary cause of crop loss worldwide, reducing average yields for most major crop plants by more than 50% (Wang et al. (2003) Planta 218: 1-14). Abiotic stresses may be caused by drought, salinity, extremes of temperature, chemical toxicity, excess or deficiency of nutrients (macroelements and/or microelements), radiation and oxidative stress. The ability to increase plant tolerance to abiotic stress would be of great economic advantage to farmers worldwide and would allow for the cultivation of crops during adverse conditions and in territories where cultivation of crops may not otherwise be possible.

Crop yield may therefore be increased by optimising one of the above-mentioned factors.

Depending on the end use, the modification of certain yield traits may be favoured over others. For example for applications such as forage or wood production, or bio-fuel resource, an increase in the vegetative parts of a plant may be desirable, and for applications such as flour, starch or oil production, an increase in seed parameters may be particularly desirable. Even amongst the seed parameters, some may be favoured over others, depending on the application. Various mechanisms may contribute to increasing seed yield, whether that is in the form of increased seed size or increased seed number.

One approach to increase yield and/or yield-related traits (seed yield and/or biomass) in plants may be through modification of the inherent growth mechanisms of a plant, such as the cell cycle or various signalling pathways involved in plant growth or in defense mechanisms.

It has now been found that tolerance to various abiotic stresses may be enhanced in plants by modulating expression in a plant of a nucleic acid encoding an alfin-like polypeptide.

It has now been found that tolerance to various abiotic stresses may be enhanced in plants by modulating expression in a plant of a nucleic acid encoding a YRP polypeptide.

It has now been found that various yield-related traits may be increased in plants relative to control plants, by increasing expression in a plant of a nucleic acid sequence encoding a Brevis Radix-like (BRXL) polypeptide. The increased yield-related traits comprise one or more of: increased plant height, and increased Thousand Kernel Weight (TKW).

It has now been found that tolerance to various abiotic stresses may be enhanced in plants by modulating expression in a plant of a nucleic acid encoding a silky-1-like polypeptide.

It has now been found that various growth characteristics may be improved in plants by modulating expression in a plant of a nucleic acid encoding an ARP6 polypeptide in a plant.

It has now been found that various growth characteristics may be improved in plants by modulating expression in a plant of a nucleic acid encoding a POP in a plant.

It has now been found that various yield-related traits may be improved in plants by modulating expression in a plant of a nucleic acid encoding a CRL (Crumpled Leaf) in a plant.

### Background

### 1. Alfin-like polypeptides

The PHD finger, a Cys₄-His-Cys₃ zinc finger, is found in many regulatory proteins from plants to animals and which are frequently associated with chromatin-mediated transcriptional regulation. The PHD finger has been shown to activate transcription in yeast, plant and animal cells (Halbach et al., Nucleic Acids Res. 2000 September 15; 28(18): 3542-3550).

Alfin-like-derived zinc-finger domains belong to the PHD-finger domain family (R. Aasland, et al., Trends Biochem Sci (1995) 20:56-9). It was speculated that the Alfin-like PHD domain plays the role of binding DNA in a EDTA-sensitive manner inferring the need for zinc for binding at a core hexamer motif of either GNGGTG or GTGGNG (D. Bastola, et al., Plant Mol Biol. (1998) 38:1123-35). Eight Alfin-like-Like Factor (ALF) genes were identified in Arabidopsis (J.L. Riechmann, et al., Science (2000) 290:2105-10). Expressing an antisense version of alfin-like caused transgenic alfalfa to grow more poorly, whereas constitutive overexpression by a constitutive promoter enhanced root growth both in normal and salt-stressed conditions (I. Winicov Planta (2000) 210:416-22).

### 2. YRP polypeptides

The YRP of Hordeum vulgare (SEQ ID NO: 11 and 13) are transcription factors encoding members of the GARP class of transcription factors.

### 3. Brevis Radix-like (BRXL) polypeptides

Brevis Radix (BRX) polypeptide has been identified through a natural loss-of-function allele in the Arabidopsis accession Umkirch-1 (Uk-1), and results in reduced root meristem size, reduced mature cell size, and thus reduced root growth (Mouchel et al. (2004) Genes Dev 18: 700-714), owing to disturbed plant hormone brassinosteroid and auxin signaling pathways.

BRX and paralogs BRX-like (BRXL) belong to a conserved, plant-specific gene family (collectively called BRXL) that encodes proteins that are predicted to regulate transcription directly or indirectly. BRXL genes are found in all higher plants for which data are available, but not in unicellular organisms and animals. In the entirely sequenced plant genomes of Arabidopsis, poplar (Populus trichocarpa) and rice (Oryza sativa), five BRXL genes can be found (Briggs et al. (2006) Plant Physiol 140: 1307-1316).

Four domains of high conservation can be distinguished in BRX family proteins. The homology among BRX family proteins within and between species is especially conserved in these regions:
1. at the N terminus, two short domains of approximately 10 and 25 amino acids respectively, are conserved, containing conserved Cys's, whose spacing is indicative of a potential zinc-binding motif.
2. the middle region of BRX family proteins contains a highly conserved domain of approximately 65 amino acids.
3. a second highly conserved domain of approximately 60 amino acids is present, homologous to the first middle domain, constituting a novel type of tandem repeat, which is the main characteristic of BRX family proteins (therefore named the BRX domain).

Alpha-helical regions, which are characteristic of DNA binding and protein-protein interaction domains, have been identified within the conserved BRX domains. Yeast two-hybrid experiments demonstrated that the BRX domain is a novel protein-protein interaction domain, which likely mediates homodimerization and heterodimerization within and/or between the BRXL and also PRAF-like (PH, RCC1, and FYVE) protein families

(Briggs et al. (2006) Plant Physiol 140: 1307-1316; van Leeuwen et al. (2004) Trends Plant Sci 9: 378-384). PRAF-like proteins also contain regulator of chromosome condensation 1 (RCC1) repeats, which often provide guanine nucleotide exchange activity.

In US patent 7214786 "Nucleic acid molecules and other molecules associated with plants and uses thereof for plant improvement", are described nucleic acid sequences encoding BRXL polypeptides (SEQ IDs NO: 35674, 30290, 17003), and constructs comprising these. The disclosed recombinant polynucleotides and recombinant polypeptides find use in production of transgenic plants to produce plants having improved properties. In US patent 7365185 "Genomic plant sequences and uses thereof", are described rice nucleic acid promoter sequences of BRXL polypeptides (SEQ IDs NO: 59178, 70484, 70442, 37078, 78410, 64873), and constructs comprising these. The invention further discloses compositions, transformed host cells, transgenic plants, and seeds containing the rice genomic promoter sequences, and methods for preparing and using the same.

### 4. silky-1-like polypeptides

Silky-1 is a member of the family of MADS transcription factors and which is involved in flower development.

### 5. ARP6 polypeptides

Actin-related proteins (ARPs) constitute a family of eukaryotic proteins whose primary sequences display homology to conventionalactins. Whereas actins play well-characterized cytoskeletal roles, the ARPs are implicated in various cellular functions in both the cytoplasm and in the nucleus. Cytoplasmic ARPs, for example, are known to participate in the assembly of branched actin filamentsand dynein-mediated movement of vesicles in manyeukaryotes. Nuclear ARPs are components of various chromatin-modifying complexes involved in transcriptional regulation. In plants, for example it has been recently described the existence of a SWR1/ SRCAP-like complex. These complexes appear to destabilize protein-protein and protein-DNA interactions within the nucleosome, allowing chromatin to remodel and therefore influencing gene expression. Yeast and mammalian ARP6 proteins function with the SWR1 and SRCAP complexes, respectively, which deposit the histone variantH2A.Z into chromatin. The Arabidopsis thalianan ARP6 interacts with the ARP6, PIE1 and SEF proteins indicating that the ARP6 function is conserved also in the plant kingdom (March-Diaz, R. et al. 2007. Plant Physiol. 143, 893-901). Knockout mutations in AtARP6 results in misregulation of the expression of a number of genes and in early flowering and dwarf phenotypes in Arabidopsis thaliana (Deal 2007, The Plant Cell, Vol. 19: 74-83).

The ARPs and actins possess a common tertiary structure centered on the nucleotide-binding pocket known as the actin fold (Kabach et al. 1995. FASEB J. 9,167-1745). ARPs are grouped into several classes or subfamilies that are highly conserved in a wide range of eukaryotes. Each class or subfamily is distinguished by its degree of similarity to conventional actin (Kandasamy et al. 2004 Trends Plant Sci 9: 196-202).

### 6. POP polypeptides

Proteases catalyse the hydrolysis of peptide bonds, either at the end of a polypeptide chain (exopeptidases) or within the polypeptide chain (endopeptidases). They are classified according to their structural conservation, see for example the MEROPS database (Rawlings et al., Nucleic Acids Research 34, D270-272, 2006). Serine proteases have in their active site a serine that plays a role in the hydrolysis of the substrate, and form within the plants the largest group of proteinases. Within the group of Serine proteases, several subgroups are discriminated, such as the subtilisin family, the chymotrypsin family, D-Ala-D-Ala carboxypeptidase B family or the Prolyl oligopeptidase family.

Prolyl-oligopeptidases are postulated to play a role in the formation, processing and degradation of biologically active peptides, and have been described in bacteria, archaea as well as in eukaryotes. Within the group of Prolyl-oligopeptidases 4 subfamilies can be discriminated: prolyl-oligopeptidase (S9A), dipeptidyl-peptidase IV (S9B), aminoacylpeptidase (S9C) and glutamyl endopeptidase (S9D).

Despite the fact that so many proteases have been identified, little is known about the substrates of these enzymes. Therefore also the function and the regulation of proteases are hardly characterised.

### 7. Crumpled Leaf (CRL) polypeptides

Crumpled leaf (crl) is the name give to an Arabidopsis thaliana mutant having abnormal morphogenesis of all plant organs and division of plastids. Histological analysis revealed that planes of cell division were distorted in shoot apical meristems (SAMs), root tips, and embryos in plants that possess the crl mutation. Furthermore, differentiation patterns of cortex and endodermis cells in inflorescence stems and root endodermis cells were disturbed in the crl mutant. These results suggest that morphological abnormalities observed in the crl mutant were because of aberrant cell division and differentiation. In addition, cells of the crl mutant contained a reduced number of enlarged plastids, indicating that the division of plastids was inhibited in the crl. The gene mutated and responsible for the phenotype was named CRL (Crumpled leaf). The CRL gene encodes a protein with a molecular mass of 30 kDa that is localized in the plastid envelope. The CRL protein is conserved in various plant species, including a fern, and in cyanobacteria. CRL protein of Arabidopsis has a putative membrane domain localized between amino acids 19-36 and a conserved domain between amino acid residues 42-236. This domain is highly conserved amongst the CRL proteins present in other plant species.

### Summary

### 1. Alfin-like polypeptides

Surprisingly, it has now been found that modulating expression of a nucleic acid encoding an alfin-like polypeptide gives plants having enhanced tolerance to various abiotic stresses relative to control plants.

According one embodiment, there is provided a method for enhancing tolerance in plants to various abiotic stresses, relative to tolerance in control plants, comprising modulating expression of a nucleic acid encoding an alfin-like polypeptide in a plant.

### 2. YRP polypeptides

Surprisingly, it has now been found that modulating expression of a nucleic acid encoding a YRP polypeptide gives plants having enhanced tolerance to various abiotic stresses relative to control plants.

According one embodiment, there is provided a method for enhancing tolerance in plants to various abiotic stresses, relative to tolerance in control plants, comprising modulating expression of a nucleic acid encoding a YRP polypeptide in a plant.

### 3. Brevis Radix-like (BRXL) polypeptides

Surprisingly, it has now been found that increasing expression in a plant of a nucleic acid sequence encoding a BRXL polypeptide as defined herein, gives plants having increased yield-related traits relative to control plants.

According to one embodiment, there is provided a method for increasing yield-related traits in plants relative to control plants, comprising increasing expression in a plant of a nucleic acid sequence encoding a BRXL polypeptide as defined herein. The increased yield-related traits comprise one or more of: increased plant height, and increased Thousand Kernel Weight (TKW).

### 4. silky-1-like polypeptides

Surprisingly, it has now been found that modulating expression of a nucleic acid encoding a silky-1-like polypeptide gives plants having enhanced tolerance to various abiotic stresses relative to control plants.

According one embodiment, there is provided a method for enhancing tolerance in plants to various abiotic stresses, relative to tolerance in control plants, comprising modulating expression of a nucleic acid encoding a silky-1-like polypeptide in a plant.

### 5. ARP6 polypeptides

Surprisingly, it has now been found that modulating expression of a nucleic acid encoding an ARP6 polypeptide gives plants having enhanced yield-related traits relative to control plants.

According one embodiment, there is provided a method for enhancing yield-related traits of a plant relative to control plants, comprising modulating expression of a nucleic acid encoding an ARP6 polypeptide in a plant.

### 6. POP polypeptides

Surprisingly, it has now been found that modulating expression of a nucleic acid encoding a POP polypeptide gives plants having enhanced yield-related traits relative to control plants.

According one embodiment, there is provided a method for enhanced yield-related traits of a plant relative to control plants, comprising modulating expression of a nucleic acid encoding a POP polypeptide in a plant.

### 7. Crumpled Leaf (CRL) polypeptides

Surprisingly, it has now been found that modulating expression of a nucleic acid encoding a CRL polypeptide gives plants having enhanced yield-related traits relative to control plants.

According one embodiment, there is provided a method for enhancing yield related traits of a plant relative to control plants, comprising modulating expression of a nucleic acid encoding a CRL polypeptide in a plant.

### Definitions

### Polypeptide(s)/Protein(s)

The terms "polypeptide" and "protein" are used interchangeably herein and refer to amino acids in a polymeric form of any length, linked together by peptide bonds.

### Polynucleotide(s)/Nucleic acid(s)/Nucleic acid sequence(s)/nucleotide sequence(s)

The terms "polynucleotide(s)", "nucleic acid sequence(s)", "nucleotide sequence(s)", "nucleic acid(s)", "nucleic acid molecule" are used interchangeably herein and refer to nucleotides, either ribonucleotides or deoxyribonucleotides or a combination of both, in a polymeric unbranched form of any length.

### Control plant(s)

The choice of suitable control plants is a routine part of an experimental setup and may include corresponding wild type plants or corresponding plants without the gene of interest. The control plant is typically of the same plant species or even of the same variety as the plant to be assessed. The control plant may also be a nullizygote of the plant to be assessed. Nullizygotes are individuals missing the transgene by segregation. A "control plant" as used herein refers not only to whole plants, but also to plant parts, including seeds and seed parts.

### Homologue(s)

"Homologues" of a protein encompass peptides, oligopeptides, polypeptides, proteins and enzymes having amino acid substitutions, deletions and/or insertions relative to the unmodified protein in question and having similar biological and functional activity as the unmodified protein from which they are derived.

A deletion refers to removal of one or more amino acids from a protein.

An insertion refers to one or more amino acid residues being introduced into a predetermined site in a protein. Insertions may comprise N-terminal and/or C-terminal fusions as well as intra-sequence insertions of single or multiple amino acids. Generally, insertions within the amino acid sequence will be smaller than N- or C-terminal fusions, of the order of about 1 to 10 residues. Examples of N- or C-terminal fusion proteins or peptides include the binding domain or activation domain of a transcriptional activator as used in the yeast two-hybrid system, phage coat proteins, (histidine)-6-tag, glutathione S-transferase-tag, protein A, maltose-binding protein, dihydrofolate reductase, Tag•100 epitope, c-myc epitope, FLAG^{®}-epitope, lacZ, CMP (calmodulin-binding peptide), HA epitope, protein C epitope and VSV epitope.

A substitution refers to replacement of amino acids of the protein with other amino acids having similar properties (such as similar hydrophobicity, hydrophilicity, antigenicity, propensity to form or break α-helical structures or β-sheet structures). Amino acid substitutions are typically of single residues, but may be clustered depending upon functional constraints placed upon the polypeptide; insertions will usually be of the order of about 1 to 10 amino acid residues. The amino acid substitutions are preferably conservative amino acid substitutions. Conservative substitution tables are well known in the art (see for example Creighton (1984) Proteins. W.H. Freeman and Company (Eds) and Table 1 below).

**Table 1: Examples of conserved amino acid substitutions**

| Residue | Conservative Substitutions | Residue | Conservative Substitutions |
|---|---|---|---|
| Ala | Ser | Leu | Ile; Val |
| Arg | Lys | Lys | Arg; Gln |
| Asn | Gln; His | Met | Leu; lie |
| Asp | Glu | Phe | Met; Leu; Tyr |
| Gln | Asn | Ser | Thr; Gly |
| Cys | Ser | Thr | Ser; Val |
| Glu | Asp | Trp | Tyr |
| Gly | Pro | Tyr | Trp; Phe |
| His | Asn; Gln | Val | lie; Leu |
| Ile | Leu, Val | | |

Amino acid substitutions, deletions and/or insertions may readily be made using peptide synthetic techniques well known in the art, such as solid phase peptide synthesis and the like, or by recombinant DNA manipulation. Methods for the manipulation of DNA sequences to produce substitution, insertion or deletion variants of a protein are well known in the art. For example, techniques for making substitution mutations at predetermined sites in DNA are well known to those skilled in the art and include M13 mutagenesis, T7-Gen in vitro mutagenesis (USB, Cleveland, OH), QuickChange Site Directed mutagenesis (Stratagene, San Diego, CA), PCR-mediated site-directed mutagenesis or other site-directed mutagenesis protocols.

### Derivatives

"Derivatives" include peptides, oligopeptides, polypeptides which may, compared to the amino acid sequence of the naturally-occurring form of the protein, such as the protein of interest, comprise substitutions of amino acids with non-naturally occurring amino acid residues, or additions of non-naturally occurring amino acid residues. "Derivatives" of a protein also encompass peptides, oligopeptides, polypeptides which comprise naturally occurring altered (glycosylated, acylated, prenylated, phosphorylated, myristoylated, sulphated etc.) or non-naturally altered amino acid residues compared to the amino acid sequence of a naturally-occurring form of the polypeptide. A derivative may also comprise one or more non-amino acid substituents or additions compared to the amino acid sequence from which it is derived, for example a reporter molecule or other ligand, covalently or non-covalently bound to the amino acid sequence, such as a reporter molecule which is bound to facilitate its detection, and non-naturally occurring amino acid residues relative to the amino acid sequence of a naturally-occurring protein. Furthermore, "derivatives" also include fusions of the naturally-occurring form of the protein with tagging peptides such as FLAG, HIS6 or thioredoxin (for a review of tagging peptides, see Terpe, Appl. Microbiol. Biotechnol. 60, 523-533, 2003).

### Orthologue(s)/Paralogue(s)

Orthologues and paralogues encompass evolutionary concepts used to describe the ancestral relationships of genes. Paralogues are genes within the same species that have originated through duplication of an ancestral gene; orthologues are genes from different organisms that have originated through speciation, and are also derived from a common ancestral gene.

### Domain

The term "domain" refers to a set of amino acids conserved at specific positions along an alignment of sequences of evolutionarily related proteins. While amino acids at other positions can vary between homologues, amino acids that are highly conserved at specific positions indicate amino acids that are likely essential in the structure, stability or function of a protein. Identified by their high degree of conservation in aligned sequences of a family of protein homologues, they can be used as identifiers to determine if any polypeptide in question belongs to a previously identified polypeptide family.

### Motif/Consensus sequence/Signature

The term "motif" or "consensus sequence" or "signature" refers to a short conserved region in the sequence of evolutionarily related proteins. Motifs are frequently highly conserved parts of domains, but may also include only part of the domain, or be located outside of conserved domain (if all of the amino acids of the motif fall outside of a defined domain).

### Hybridisation

The term "hybridisation" as defined herein is a process wherein substantially homologous complementary nucleotide sequences anneal to each other. The hybridisation process can occur entirely in solution, i.e. both complementary nucleic acids are in solution. The hybridisation process can also occur with one of the complementary nucleic acids immobilised to a matrix such as magnetic beads, Sepharose beads or any other resin. The hybridisation process can furthermore occur with one of the complementary nucleic acids immobilised to a solid support such as a nitro-cellulose or nylon membrane or immobilised by e.g. photolithography to, for example, a siliceous glass support (the latter known as nucleic acid arrays or microarrays or as nucleic acid chips). In order to allow hybridisation to occur, the nucleic acid molecules are generally thermally or chemically denatured to melt a double strand into two single strands and/or to remove hairpins or other secondary structures from single stranded nucleic acids.

The term "stringency" refers to the conditions under which a hybridisation takes place. The stringency of hybridisation is influenced by conditions such as temperature, salt concentration, ionic strength and hybridisation buffer composition. Generally, low stringency conditions are selected to be about 30°C lower than the thermal melting point (Tₘ) for the specific sequence at a defined ionic strength and pH. Medium stringency conditions are when the temperature is 20°C below Tₘ, and high stringency conditions are when the temperature is 10°C below Tₘ. High stringency hybridisation conditions are typically used for isolating hybridising sequences that have high sequence similarity to the target nucleic acid sequence. However, nucleic acids may deviate in sequence and still encode a substantially identical polypeptide, due to the degeneracy of the genetic code. Therefore medium stringency hybridisation conditions may sometimes be needed to identify such nucleic acid molecules.

The Tm is the temperature under defined ionic strength and pH, at which 50% of the target sequence hybridises to a perfectly matched probe. The Tₘ is dependent upon the solution conditions and the base composition and length of the probe. For example, longer sequences hybridise specifically at higher temperatures. The maximum rate of hybridisation is obtained from about 16°C up to 32°C below Tₘ. The presence of monovalent cations in the hybridisation solution reduce the electrostatic repulsion between the two nucleic acid strands thereby promoting hybrid formation; this effect is visible for sodium concentrations of up to 0.4M (for higher concentrations, this effect may be ignored). Formamide reduces the melting temperature of DNA-DNA and DNA-RNA duplexes with 0.6 to 0.7°C for each percent formamide, and addition of 50% formamide allows hybridisation to be performed at 30 to 45°C, though the rate of hybridisation will be lowered. Base pair mismatches reduce the hybridisation rate and the thermal stability of the duplexes. On average and for large probes, the Tm decreases about 1°C per % base mismatch. The Tm may be calculated using the following equations, depending on the types of hybrids:
1) DNA-DNA hybrids (Meinkoth and Wahl, Anal. Biochem., 138: 267-284, 1984): Tₘ= 81.5°C + 16.6xlog₁₀[Na⁺]^{a} + 0.41x%[G/C^{b}] - 500x[Lc]⁻¹ - 0.61x% formamide
2) DNA-RNA or RNA-RNA hybrids: Tm= 79.8 + 18.5 (log₁₀[Na⁺]^{a}) + 0.58 (%G/C^{b}) + 11.8 (%G/C^{b})² - 820/L^{c}
3) oligo-DNA or oligo-RNA^{d} hybrids:
   For <20 nucleotides: Tₘ= 2 (Iₙ)
   For 20-35 nucleotides: Tₘ= 22 + 1.46 (Iₙ)

a or for other monovalent cation, but only accurate in the 0.01-0.4 M range.
b only accurate for %GC in the 30% to 75% range.
c L = length of duplex in base pairs.
d oligo, oligonucleotide; Iₙ, = effective length of primer = 2×(no. of G/C)+(no. of A/T).

Non-specific binding may be controlled using any one of a number of known techniques such as, for example, blocking the membrane with protein containing solutions, additions of heterologous RNA, DNA, and SDS to the hybridisation buffer, and treatment with Rnase. For non-homologous probes, a series of hybridizations may be performed by varying one of (i) progressively lowering the annealing temperature (for example from 68°C to 42°C) or (ii) progressively lowering the formamide concentration (for example from 50% to 0%). The skilled artisan is aware of various parameters which may be altered during hybridisation and which will either maintain or change the stringency conditions.

Besides the hybridisation conditions, specificity of hybridisation typically also depends on the function of post-hybridisation washes. To remove background resulting from non-specific hybridisation, samples are washed with dilute salt solutions. Critical factors of such washes include the ionic strength and temperature of the final wash solution: the lower the salt concentration and the higher the wash temperature, the higher the stringency of the wash. Wash conditions are typically performed at or below hybridisation stringency. A positive hybridisation gives a signal that is at least twice of that of the background. Generally, suitable stringent conditions for nucleic acid hybridisation assays or gene amplification detection procedures are as set forth above. More or less stringent conditions may also be selected. The skilled artisan is aware of various parameters which may be altered during washing and which will either maintain or change the stringency conditions.

For example, typical high stringency hybridisation conditions for DNA hybrids longer than 50 nucleotides encompass hybridisation at 65°C in 1x SSC or at 42°C in 1x SSC and 50% formamide, followed by washing at 65°C in 0.3x SSC. Examples of medium stringency hybridisation conditions for DNA hybrids longer than 50 nucleotides encompass hybridisation at 50°C in 4x SSC or at 40°C in 6x SSC and 50% formamide, followed by washing at 50°C in 2x SSC. The length of the hybrid is the anticipated length for the hybridising nucleic acid. When nucleic acids of known sequence are hybridised, the hybrid length may be determined by aligning the sequences and identifying the conserved regions described herein. 1×SSC is 0.15M NaCl and 15mM sodium citrate; the hybridisation solution and wash solutions may additionally include 5x Denhardt's reagent, 0.5-1.0% SDS, 100 µg/ml denatured, fragmented salmon sperm DNA, 0.5% sodium pyrophosphate.

For the purposes of defining the level of stringency, reference can be made to Sambrook et al. (2001) Molecular Cloning: a laboratory manual, 3rd Edition, Cold Spring Harbor Laboratory Press, CSH, New York or to Current Protocols in Molecular Biology, John Wiley & Sons, N.Y. (1989 and yearly updates).

### Splice variant

The term "splice variant" as used herein encompasses variants of a nucleic acid sequence in which selected introns and/or exons have been excised, replaced, displaced or added, or in which introns have been shortened or lengthened. Such variants will be ones in which the biological activity of the protein is substantially retained; this may be achieved by selectively retaining functional segments of the protein. Such splice variants may be found in nature or may be manmade. Methods for predicting and isolating such splice variants are well known in the art (see for example Foissac and Schiex (2005) BMC Bioinformatics 6: 25).

### Allelic variant

Alleles or allelic variants are alternative forms of a given gene, located at the same chromosomal position. Allelic variants encompass Single Nucleotide Polymorphisms

(SNPs), as well as Small Insertion/Deletion Polymorphisms (INDELs). The size of INDELs is usually less than 100 bp. SNPs and INDELs form the largest set of sequence variants in naturally occurring polymorphic strains of most organisms.

### Gene shuffling/Directed evolution

Gene shuffling or directed evolution consists of iterations of DNA shuffling followed by appropriate screening and/or selection to generate variants of nucleic acids or portions thereof encoding proteins having a modified biological activity (Castle et al., (2004) Science 304(5674): 1151-4; US patents 5,811,238 and 6,395,547).

### Regulatory element/Control sequence/Promoter

The terms "regulatory element", "control sequence" and "promoter" are all used interchangeably herein and are to be taken in a broad context to refer to regulatory nucleic acid sequences capable of effecting expression of the sequences to which they are ligated. The term "promoter" typically refers to a nucleic acid control sequence located upstream from the transcriptional start of a gene and which is involved in recognising and binding of RNA polymerase and other proteins, thereby directing transcription of an operably linked nucleic acid. Encompassed by the aforementioned terms are transcriptional regulatory sequences derived from a classical eukaryotic genomic gene (including the TATA box which is required for accurate transcription initiation, with or without a CCAAT box sequence) and additional regulatory elements (i.e. upstream activating sequences, enhancers and silencers) which alter gene expression in response to developmental and/or external stimuli, or in a tissue-specific manner. Also included within the term is a transcriptional regulatory sequence of a classical prokaryotic gene, in which case it may include a -35 box sequence and/or -10 box transcriptional regulatory sequences. The term "regulatory element" also encompasses a synthetic fusion molecule or derivative that confers, activates or enhances expression of a nucleic acid molecule in a cell, tissue or organ.

A "plant promoter" comprises regulatory elements, which mediate the expression of a coding sequence segment in plant cells. Accordingly, a plant promoter need not be of plant origin, but may originate from viruses or micro-organisms, for example from viruses which attack plant cells. The "plant promoter" can also originate from a plant cell, e.g. from the plant which is transformed with the nucleic acid sequence to be expressed in the inventive process and described herein. This also applies to other "plant" regulatory signals, such as "plant" terminators. The promoters upstream of the nucleotide sequences useful in the methods of the present invention can be modified by one or more nucleotide substitution(s), insertion(s) and/or deletion(s) without interfering with the functionality or activity of either the promoters, the open reading frame (ORF) or the 3'-regulatory region such as terminators or other 3' regulatory regions which are located away from the ORF. It is furthermore possible that the activity of the promoters is increased by modification of their sequence, or that they are replaced completely by more active promoters, even promoters from heterologous organisms. For expression in plants, the nucleic acid molecule must, as described above, be linked operably to or comprise a suitable promoter which expresses the gene at the right point in time and with the required spatial expression pattern.

For the identification of functionally equivalent promoters, the promoter strength and/or expression pattern of a candidate promoter may be analysed for example by operably linking the promoter to a reporter gene and assaying the expression level and pattern of the reporter gene in various tissues of the plant. Suitable well-known reporter genes include for example beta-glucuronidase or beta-galactosidase. The promoter activity is assayed by measuring the enzymatic activity of the beta-glucuronidase or beta-galactosidase. The promoter strength and/or expression pattern may then be compared to that of a reference promoter (such as the one used in the methods of the present invention). Alternatively, promoter strength may be assayed by quantifying mRNA levels or by comparing mRNA levels of the nucleic acid used in the methods of the present invention, with mRNA levels of housekeeping genes such as 18S rRNA, using methods known in the art, such as Northern blotting with densitometric analysis of autoradiograms, quantitative real-time PCR or RT-PCR (Heid et al., 1996 Genome Methods 6: 986-994). Generally by "weak promoter" is intended a promoter that drives expression of a coding sequence at a low level. By "low level" is intended at levels of about 1/10,000 transcripts to about 1/100,000 transcripts, to about 1/500,0000 transcripts per cell. Conversely, a "strong promoter" drives expression of a coding sequence at high level, or at about 1/10 transcripts to about 1/100 transcripts to about 1/1000 transcripts per cell. Generally, by "medium strength promoter" is intended a promoter that drives expression of a coding sequence at a lower level than a strong promoter, in particular at a level that is in all instances below that obtained when under the control of a 35S CaMV promoter.

### Operably linked

The term "operably linked" as used herein refers to a functional linkage between the promoter sequence and the gene of interest, such that the promoter sequence is able to initiate transcription of the gene of interest.

### Constitutive promoter

A "constitutive promoter" refers to a promoter that is transcriptionally active during most, but not necessarily all, phases of growth and development and under most environmental conditions, in at least one cell, tissue or organ. Table 2a below gives examples of constitutive promoters.

**Table 2a: Examples of constitutive promoters**

| Gene Source | Reference |
|---|---|
| Actin | McElroy et al, Plant Cell, 2: 163-171, 1990 |
| HMGP | WO 2004/070039 |
| CAMV 35S | Odell et al, Nature, 313: 810-812, 1985 |
| CaMV 19S | Nilsson et al., Physiol. Plant. 100:456-462, 1997 |
| GOS2 | de Pater et al, Plant J Nov;2(6):837-44, 1992, WO 2004/065596 |
| Ubiquitin | Christensen et al, Plant Mol. Biol. 18: 675-689, 1992 |
| Rice cyclophilin | Buchholz et al, Plant Mol Biol. 25(5): 837-43, 1994 |
| Maize H3 histone | Lepetit et al, Mol. Gen. Genet. 231:276-285, 1992 |
| Alfalfa H3 histone | Wu et al. Plant Mol. Biol. 11:641-649, 1988 |
| Actin 2 | An et al, Plant J. 10(1); 107-121, 1996 |
| 34S FMV | Sanger et al., Plant. Mol. Biol., 14, 1990: 433-443 |
| Rubisco small subunit | US 4,962,028 |
| OCS | Leisner (1988) Proc Natl Acad Sci USA 85(5): 2553 |
| SAD1 | Jain et al., Crop Science, 39 (6), 1999: 1696 |
| SAD2 | Jain et al., Crop Science, 39 (6), 1999: 1696 |
| nos | Shaw et al. (1984) Nucleic Acids Res. 12(20):7831-7846 |
| V-ATPase | WO 01/14572 |
| Super promoter | WO 95/14098 |
| G-box proteins | WO 94/12015 |

### Ubiquitous promoter

A ubiquitous promoter is active in substantially all tissues or cells of an organism.

### Developmentally-regulated promoter

A developmentally-regulated promoter is active during certain developmental stages or in parts of the plant that undergo developmental changes.

### Inducible promoter

An inducible promoter has induced or increased transcription initiation in response to a chemical (for a review see Gatz 1997, Annu. Rev. Plant Physiol. Plant Mol. Biol., 48:89-108), environmental or physical stimulus, or may be "stress-inducible", i.e. activated when a plant is exposed to various stress conditions, or a "pathogen-inducible" i.e. activated when a plant is exposed to exposure to various pathogens.

### Organ-specific/Tissue-specific promoter

An organ-specific or tissue-specific promoter is one that is capable of preferentially initiating transcription in certain organs or tissues, such as the leaves, roots, seed tissue etc. For example, a "root-specific promoter" is a promoter that is transcriptionally active predominantly in plant roots, substantially to the exclusion of any other parts of a plant, whilst still allowing for any leaky expression in these other plant parts. Promoters able to initiate transcription in certain cells only are referred to herein as "cell-specific".

Examples of root-specific promoters are listed in Table 2b below:

**Table 2b: Examples of root-specific promoters**

| Gene Source | Reference |
|---|---|
| RCc3 | Plant Mol Biol. 1995 Jan;27(2):237-48 |
| Arabidopsis PHT1 | Kovama et al., 2005; Mudge et al. (2002, Plant J. 31:341) |
| Medicago phosphate transporter | Xiao et al., 2006 |
| Arabidopsis Pyk10 | Nitz et al. (2001) Plant Sci 161(2): 337-346 |
| root-expressible genes | Tingey et al., EMBO J. 6: 1, 1987. |
| tobacco auxin-inducible gene | Van der Zaal et al., Plant Mol. Biol. 16, 983, 1991. |
| β-tubulin | Oppenheimer, et al., Gene 63: 87, 1988. |
| tobacco root-specific genes | Conkling, et al., Plant Physiol. 93: 1203, 1990. |
| B. napus G1-3b gene | United States Patent No. 5, 401, 836 |
| SbPRP1 | Suzuki et al., Plant Mol. Biol. 21: 109-119, 1993. |
| LRX1 | Baumberger et al. 2001, Genes & Dev. 15:1128 |
| BTG-26 Brassica napus | US 20050044585 |
| LeAMT1 (tomato) | Lauter et al. (1996, PNAS 3:8139) |
| The LeNRT1-1 (tomato) | Lauter et al. (1996, PNAS 3:8139) |
| class I patatin gene (potato) | Liu et al., Plant Mol. Biol. 153:386-395, 1991. |
| KDC1 (Daucus carota) | Downey et al. (2000, J. Biol. Chem. 275:39420) |
| TobRB7 gene | W Song (1997) PhD Thesis, North Carolina State University, Raleigh, NC USA |
| OsRAB5a (rice) | Wang et al. 2002, Plant Sci. 163:273 |
| ALF5 (Arabidopsis) | Diener et al. (2001, Plant Cell 13:1625) |
| NRT2;1 Np (N. plumbaginifolia) | Quesada et al. (1997, Plant Mol. Biol. 34:265) |

A seed-specific promoter is transcriptionally active predominantly in seed tissue, but not necessarily exclusively in seed tissue (in cases of leaky expression). The seed-specific promoter may be active during seed development and/or during germination. The seed specific promoter may be endosperm/aleurone/embryo specific. Examples of seed-specific promoters (endosperm/aleurone/embryo specific) are shown in Table 2c to Table 2f below. Further examples of seed-specific promoters are given in Qing Qu and Takaiwa (Plant Biotechnol. J. 2, 113-125, 2004), which disclosure is incorporated by reference herein as if fully set forth.

**Table 2c: Examples of seed-specific promoters**

| Gene source | Reference |
|---|---|
| seed-specific genes | Simon et al., Plant Mol. Biol. 5: 191, 1985; |
| | Scofield et al., J. Biol. Chem. 262: 12202, 1987.; |
| | Baszczynski et al., Plant Mol. Biol. 14: 633, 1990. |
| Brazil Nut albumin | Pearson et al., Plant Mol. Biol. 18: 235-245, 1992. |
| legumin | Ellis et al., Plant Mol. Biol. 10: 203-214, 1988. |
| glutelin (rice) | Takaiwa et al., Mol. Gen. Genet. 208: 15-22, 1986; |
| | Takaiwa et al., FEBS Letts. 221: 43-47, 1987. |
| zein | Matzke et al Plant Mol Biol, 14(3):323-32 1990 |
| napA | Stalberg et al, Planta 199: 515-519, 1996. |
| wheat LMW and HMW glutenin-1 | Mol Gen Genet 216:81-90, 1989; NAR 17:461-2, 1989 |
| wheat SPA | Albani et al, Plant Cell, 9: 171-184, 1997 |
| wheat α, β, γ-gliadins | EMBO J. 3:1409-15, 1984 |
| barley Itr1 promoter | Diaz et al. (1995) Mol Gen Genet 248(5):592-8 |
| barley B1, C, D, hordein | Theor Appl Gen 98:1253-62, 1999; Plant J 4:343-55, 1993; Mol Gen Genet 250:750-60, 1996 |
| barley DOF | Mena et al, The Plant Journal, 116(1): 53-62, 1998 |
| blz2 | EP99106056.7 |
| synthetic promoter | Vicente-Carbajosa et al., Plant J. 13: 629-640, 1998. |
| rice prolamin NRP33 | Wu et al, Plant Cell Physiology 39(8) 885-889, 1998 |
| rice a-globulin Glb-1 | Wu et al, Plant Cell Physiology 39(8) 885-889, 1998 |
| rice OSH1 | Sato et al, Proc. Natl. Acad. Sci. USA, 93: 8117-8122, 1996 |
| rice α-globulin REB/OHP-1 | Nakase et al. Plant Mol. Biol. 33: 513-522, 1997 |
| rice ADP-glucose pyrophosphorylase | Trans Res 6:157-68, 1997 |
| maize ESR gene family | Plant J 12:235-46, 1997 |
| sorghum α-kafirin | DeRose et al., Plant Mol. Biol 32:1029-35, 1996 |
| KNOX | Postma-Haarsma et al, Plant Mol. Biol. 39:257-71, 1999 |
| rice oleosin | Wu et al, J. Biochem. 123:386,1998 |
| sunflower oleosin | Cummins et al., Plant Mol. Biol. 19: 873-876, 1992 |
| PRO0117, putative rice 40S ribosomal protein | WO 2004/070039 |
| PRO0136, rice alanine aminotransferase | unpublished |
| PRO0147, trypsin inhibitor ITR1 (barley) | unpublished |
| PRO0151, rice WSI18 | WO 2004/070039 |
| PRO0175, rice RAB21 | WO 2004/070039 |
| PRO005 | WO 2004/070039 |
| PRO0095 | WO 2004/070039 |
| α-amylase (Amy32b) | Lanahan et al, Plant Cell 4:203-211, 1992; Skriver et al, Proc Natl Acad Sci USA 88:7266-7270, 1991 |
| cathepsin β-like gene | Cejudo et al, Plant Mol Biol 20:849-856, 1992 |
| Barley Ltp2 | Kalla et al., Plant J. 6:849-60, 1994 |
| Chi26 | Leah et al., Plant J. 4:579-89, 1994 |
| Maize B-Peru | Selinger et al., Genetics 149;1125-38,1998 |

**Table 2d: examples of endosperm-specific promoters**

| Gene source | Reference |
|---|---|
| glutelin (rice) | Takaiwa et al. (1986) Mol Gen Genet 208:15-22; Takaiwa et al. (1987) FEBS Letts. 221:43-47 |
| zein | Matzke et al., (1990) Plant Mol Biol 14(3): 323-32 |
| wheat LMW and HMW glutein-1 | Colot et al. (1989) Mol Gen Genet 216:81-90, Anderson et al. (1989) NAR 17:461-2 |
| wheat SPA | Albani et al. (1997) Plant Cell 9:171-184 |
| wheat gliadins | Rafalski et al. (1984) EMBO 3:1409-15 |
| barley Itr1 promoter | Diaz et al. (1995) Mol Gen Genet 248(5):592-8 |
| barley B1, C, D, hordein | Cho et al. (1999) Theor Appl Genet 98:1253-62; Muller et al. (1993) Plant J 4:343-55; Sorenson et al. (1996) Mol Gen Genet 250:750-60 |
| barley DOF | Mena et al, (1998) Plant J 116(1): 53-62 |
| | Onate et al. (1999) J Biol Chem 274(14):9175-82 |
| synthetic promoter | Vicente-Carbajosa et al. (1998) Plant J 13:629-640 |
| rice prolamin NRP33 | Wu et al, (1998) Plant Cell Physiol 39(8) 885-889 |
| rice globulin Glb-1 | Wu et al. (1998) Plant Cell Physiol 39(8) 885-889 |
| rice globulin REB/OHP-1 | Nakase et al. (1997) Plant Molec Biol 33: 513-522 |
| rice ADP-glucose pyrophosphorylase | Russell et al. (1997) Trans Res 6:157-68 |
| maize ESR gene family | Opsahl-Ferstad et al. (1997) Plant J 12:235-46 |
| sorgnum kafirin | DeRose et al. (1996) Plant Mol Biol 32:1029-35 |

**Table 2e: Examples of embryo specific promoters:**

| Gene source | Reference |
|---|---|
| rice OSH1 | Sato et al, Proc. Natl. Acad. Sci. USA, 93: 8117-8122, 1996 |
| KNOX | Postma-Haarsma et al, Plant Mol. Biol. 39:257-71, 1999 |
| PRO0151 | WO 2004/070039 |
| PRO0175 | WO 2004/070039 |
| PRO005 | WO 2004/070039 |
| PRO0095 | WO 2004/070039 |

**Table 2f: Examples of aleurone-specific promoters:**

| Gene source | Reference |
|---|---|
| α-amylase (Amy32b) | Lanahan et al, Plant Cell 4:203-211, 1992; Skriver et al, Proc Natl Acad Sci USA 88:7266-7270, 1991 |
| cathepsin β-like gene | Cejudo et al, Plant Mol Biol 20:849-856, 1992 |
| Barley Ltp2 | Kalla et al., Plant J. 6:849-60, 1994 |
| Chi26 | Leah et al., Plant J. 4:579-89, 1994 |
| Maize B-Peru | Selinger et al., Genetics 149; 1125-38,1998 |

A green tissue-specific promoter as defined herein is a promoter that is transcriptionally active predominantly in green tissue, substantially to the exclusion of any other parts of a plant, whilst still allowing for any leaky expression in these other plant parts.

Examples of green tissue-specific promoters which may be used to perform the methods of the invention are shown in Table 2g below.

**Table 2g: Examples of green tissue-specific promoters**

| Gene | Expression | Reference |
|---|---|---|
| Maize Orthophosphate dikinase | Leaf specific | Fukavama et al., 2001 |
| Maize Phosphoenolpyruvate carboxylase | Leaf specific | Kausch et al., 2001 |
| Rice Phosphoenolpyruvate carboxylase | Leaf specific | Liu et al., 2003 |
| Rice small subunit Rubisco | Leaf specific | Nomura et al., 2000 |
| rice beta expansin EXBP9 | Shoot specific | WO 2004/070039 |
| Pigeonpea small subunit Rubisco | Leaf specific | Panguluri et al., 2005 |
| Pea RBCS3A | Leaf specific | |

Another example of a tissue-specific promoter is a meristem-specific promoter, which is transcriptionally active predominantly in meristematic tissue, substantially to the exclusion of any other parts of a plant, whilst still allowing for any leaky expression in these other plant parts. Examples of green meristem-specific promoters which may be used to perform the methods of the invention are shown in Table 2h below.

**Table 2h: Examples of meristem-specific promoters**

| Gene source | Expression pattern | Reference |
|---|---|---|
| rice OSH1 | Shoot apical meristem, from embryo globular stage to seedling stage | Sato et al. (1996) Proc. Natl. Acad. Sci. USA, 93: 8117-8122 |
| Rice metallothionein | Meristem specific | BAD87835.1 |
| WAK1 & WAK 2 | Shoot and root apical meristems, and in expanding leaves and sepals | Wagner & Kohorn (2001) Plant Cell 13(2): 303-318 |

### Terminator

The term "terminator" encompasses a control sequence which is a DNA sequence at the end of a transcriptional unit which signals 3' processing and polyadenylation of a primary transcript and termination of transcription. The terminator can be derived from the natural gene, from a variety of other plant genes, or from T-DNA. The terminator to be added may be derived from, for example, the nopaline synthase or octopine synthase genes, or alternatively from another plant gene, or less preferably from any other eukaryotic gene.

### Modulation

The term "modulation" means in relation to expression or gene expression, a process in which the expression level is changed by said gene expression in comparison to the control plant, the expression level may be increased or decreased. The original, unmodulated expression may be of any kind of expression of a structural RNA (rRNA, tRNA) or mRNA with subsequent translation. The term "modulating the activity" shall mean any change of the expression of the inventive nucleic acid sequences or encoded proteins, which leads to increased yield and/or increased growth of the plants.

### Expression

The term "expression" or "gene expression" means the transcription of a specific gene or specific genes or specific genetic construct. The term "expression" or "gene expression" in particular means the transcription of a gene or genes or genetic construct into structural RNA (rRNA, tRNA) or mRNA with or without subsequent translation of the latter into a protein. The process includes transcription of DNA and processing of the resulting mRNA product.

### Increased expression/overexpression

The term "increased expression" or "overexpression" as used herein means any form of expression that is additional to the original wild-type expression level.

Methods for increasing expression of genes or gene products are well documented in the art and include, for example, overexpression driven by appropriate promoters, the use of transcription enhancers or translation enhancers. Isolated nucleic acids which serve as promoter or enhancer elements may be introduced in an appropriate position (typically upstream) of a non-heterologous form of a polynucleotide so as to upregulate expression of a nucleic acid encoding the polypeptide of interest. For example, endogenous promoters may be altered in vivo by mutation, deletion, and/or substitution (see, Kmiec, US 5,565,350; Zarling et al., WO9322443), or isolated promoters may be introduced into a plant cell in the proper orientation and distance from a gene of the present invention so as to control the expression of the gene.

If polypeptide expression is desired, it is generally desirable to include a polyadenylation region at the 3'-end of a polynucleotide coding region. The polyadenylation region can be derived from the natural gene, from a variety of other plant genes, or from T-DNA. The 3' end sequence to be added may be derived from, for example, the nopaline synthase or octopine synthase genes, or alternatively from another plant gene, or less preferably from any other eukaryotic gene.

An intron sequence may also be added to the 5' untranslated region (UTR) or the coding sequence of the partial coding sequence to increase the amount of the mature message that accumulates in the cytosol. Inclusion of a spliceable intron in the transcription unit in both plant and animal expression constructs has been shown to increase gene expression at both the mRNA and protein levels up to 1000-fold (Buchman and Berg (1988) Mol. Cell biol. 8: 4395-4405; Callis et al. (1987) Genes Dev 1:1183-1200). Such intron enhancement of gene expression is typically greatest when placed near the 5' end of the transcription unit. Use of the maize introns Adh1-S intron 1, 2, and 6, the Bronze-1 intron are known in the art. For general information see: The Maize Handbook, Chapter 116, Freeling and Walbot, Eds., Springer, N.Y. (1994).

### Endogenous gene

Reference herein to an "endogenous" gene not only refers to the gene in question as found in a plant in its natural form (i.e., without there being any human intervention), but also refers to that same gene (or a substantially homologous nucleic acid/gene) in an isolated form subsequently (re)introduced into a plant (a transgene). For example, a transgenic plant containing such a transgene may encounter a substantial reduction of the transgene expression and/or substantial reduction of expression of the endogenous gene. The isolated gene may be isolated from an organism or may be manmade, for example by chemical synthesis.

### Decreased expression

Reference herein to "decreased expression" or "reduction or substantial elimination" of expression is taken to mean a decrease in endogenous gene expression and/or polypeptide levels and/or polypeptide activity relative to control plants. The reduction or substantial elimination is in increasing order of preference at least 10%, 20%, 30%, 40% or 50%, 60%, 70%, 80%, 85%, 90%, or 95%, 96%, 97%, 98%, 99% or more reduced compared to that of control plants. Methods for decreasing expression are known in the art and the skilled person would readily be able to adapt the known methods for silencing so as to achieve reduction of expression of an endogenous gene in a whole plant or in parts thereof through the use of an appropriate promoter, for example.

For the reduction or substantial elimination of expression an endogenous gene in a plant, a sufficient length of substantially contiguous nucleotides of a nucleic acid sequence is required. In order to perform gene silencing, this may be as little as 20, 19, 18, 17, 16, 15, 14, 13, 12, 11, 10 or fewer nucleotides, alternatively this may be as much as the entire gene (including the 5' and/or 3' UTR, either in part or in whole). The stretch of substantially contiguous nucleotides may be derived from the nucleic acid encoding the protein of interest (target gene), or from any nucleic acid capable of encoding an orthologue, paralogue or homologue of the protein of interest. Preferably, the stretch of substantially contiguous nucleotides is capable of forming hydrogen bonds with the target gene (either sense or antisense strand), more preferably, the stretch of substantially contiguous nucleotides has, in increasing order of preference, 50%, 60%, 70%, 80%, 85%, 90%, 95%, 96%, 97%, 98%, 99%, 100% sequence identity to the target gene (either sense or antisense strand). A nucleic acid sequence encoding a (functional) polypeptide is not a requirement for the various methods discussed herein for the reduction or substantial elimination of expression of an endogenous gene.

Examples of various methods for the reduction or substantial elimination of expression in a plant of an endogenous gene, or for lowering levels and/or activity of a protein, are known to the skilled in the art. A skilled person would readily be able to adapt the known methods for silencing, so as to achieve reduction of expression of an endogenous gene in a whole plant or in parts thereof through the use of an appropriate promoter, for example.

This reduction or substantial elimination of expression may be achieved using routine tools and techniques. A preferred method for the reduction or substantial elimination of endogenous gene expression is by introducing and expressing in a plant a genetic construct into which the nucleic acid (in this case a stretch of substantially contiguous nucleotides derived from the gene of interest, or from any nucleic acid capable of encoding an orthologue, paralogue or homologue of any one of the protein of interest) is cloned as an inverted repeat (in part or completely), separated by a spacer (non-coding DNA).

In such a preferred method, expression of the endogenous gene is reduced or substantially eliminated through RNA-mediated silencing using an inverted repeat of a nucleic acid or a part thereof (in this case a stretch of substantially contiguous nucleotides derived from the gene of interest, or from any nucleic acid capable of encoding an orthologue, paralogue or homologue of the protein of interest), preferably capable of forming a hairpin structure. The inverted repeat is cloned in an expression vector comprising control sequences. A non-coding DNA nucleic acid sequence (a spacer, for example a matrix attachment region fragment (MAR), an intron, a polylinker, etc.) is located between the two inverted nucleic acids forming the inverted repeat. After transcription of the inverted repeat, a chimeric RNA with a self-complementary structure is formed (partial or complete). This double-stranded RNA structure is referred to as the hairpin RNA (hpRNA). The hpRNA is processed by the plant into siRNAs that are incorporated into an RNA-induced silencing complex (RISC). The RISC further cleaves the mRNA transcripts, thereby substantially reducing the number of mRNA transcripts to be translated into polypeptides. For further general details see for example, Grierson et al. (1998) WO 98/53083; Waterhouse et al. (1999) WO 99/53050).

Performance of the methods of the invention does not rely on introducing and expressing in a plant a genetic construct into which the nucleic acid is cloned as an inverted repeat, but any one or more of several well-known "gene silencing" methods may be used to achieve the same effects.

One such method for the reduction of endogenous gene expression is RNA-mediated silencing of gene expression (downregulation). Silencing in this case is triggered in a plant by a double stranded RNA sequence (dsRNA) that is substantially similar to the target endogenous gene. This dsRNA is further processed by the plant into about 20 to about 26 nucleotides called short interfering RNAs (siRNAs). The siRNAs are incorporated into an RNA-induced silencing complex (RISC) that cleaves the mRNA transcript of the endogenous target gene, thereby substantially reducing the number of mRNA transcripts to be translated into a polypeptide. Preferably, the double stranded RNA sequence corresponds to a target gene.

Another example of an RNA silencing method involves the introduction of nucleic acid sequences or parts thereof (in this case a stretch of substantially contiguous nucleotides derived from the gene of interest, or from any nucleic acid capable of encoding an orthologue, paralogue or homologue of the protein of interest) in a sense orientation into a plant. "Sense orientation" refers to a DNA sequence that is homologous to an mRNA transcript thereof. Introduced into a plant would therefore be at least one copy of the nucleic acid sequence. The additional nucleic acid sequence will reduce expression of the endogenous gene, giving rise to a phenomenon known as co-suppression. The reduction of gene expression will be more pronounced if several additional copies of a nucleic acid sequence are introduced into the plant, as there is a positive correlation between high transcript levels and the triggering of co-suppression.

Another example of an RNA silencing method involves the use of antisense nucleic acid sequences. An "antisense" nucleic acid sequence comprises a nucleotide sequence that is complementary to a "sense" nucleic acid sequence encoding a protein, i.e. complementary to the coding strand of a double-stranded cDNA molecule or complementary to an mRNA transcript sequence. The antisense nucleic acid sequence is preferably complementary to the endogenous gene to be silenced. The complementarity may be located in the "coding region" and/or in the "non-coding region" of a gene. The term "coding region" refers to a region of the nucleotide sequence comprising codons that are translated into amino acid residues. The term "non-coding region" refers to 5' and 3' sequences that flank the coding region that are transcribed but not translated into amino acids (also referred to as 5' and 3' untranslated regions).

Antisense nucleic acid sequences can be designed according to the rules of Watson and Crick base pairing. The antisense nucleic acid sequence may be complementary to the entire nucleic acid sequence (in this case a stretch of substantially contiguous nucleotides derived from the gene of interest, or from any nucleic acid capable of encoding an orthologue, paralogue or homologue of the protein of interest), but may also be an oligonucleotide that is antisense to only a part of the nucleic acid sequence (including the mRNA 5' and 3' UTR). For example, the antisense oligonucleotide sequence may be complementary to the region surrounding the translation start site of an mRNA transcript encoding a polypeptide. The length of a suitable antisense oligonucleotide sequence is known in the art and may start from about 50, 45, 40, 35, 30, 25, 20, 15 or 10 nucleotides in length or less. An antisense nucleic acid sequence according to the invention may be constructed using chemical synthesis and enzymatic ligation reactions using methods known in the art. For example, an antisense nucleic acid sequence (e.g., an antisense oligonucleotide sequence) may be chemically synthesized using naturally occurring nucleotides or variously modified nucleotides designed to increase the biological stability of the molecules or to increase the physical stability of the duplex formed between the antisense and sense nucleic acid sequences, e.g., phosphorothioate derivatives and acridine substituted nucleotides may be used. Examples of modified nucleotides that may be used to generate the antisense nucleic acid sequences are well known in the art. Known nucleotide modifications include methylation, cyclization and 'caps' and substitution of one or more of the naturally occurring nucleotides with an analogue such as inosine. Other modifications of nucleotides are well known in the art.

The antisense nucleic acid sequence can be produced biologically using an expression vector into which a nucleic acid sequence has been subcloned in an antisense orientation (i.e., RNA transcribed from the inserted nucleic acid will be of an antisense orientation to a target nucleic acid of interest). Preferably, production of antisense nucleic acid sequences in plants occurs by means of a stably integrated nucleic acid construct comprising a promoter, an operably linked antisense oligonucleotide, and a terminator.

The nucleic acid molecules used for silencing in the methods of the invention (whether introduced into a plant or generated in situ) hybridize with or bind to mRNA transcripts and/or genomic DNA encoding a polypeptide to thereby inhibit expression of the protein, e.g., by inhibiting transcription and/or translation. The hybridization can be by conventional nucleotide complementarity to form a stable duplex, or, for example, in the case of an antisense nucleic acid sequence which binds to DNA duplexes, through specific interactions in the major groove of the double helix. Antisense nucleic acid sequences may be introduced into a plant by transformation or direct injection at a specific tissue site. Alternatively, antisense nucleic acid sequences can be modified to target selected cells and then administered systemically. For example, for systemic administration, antisense nucleic acid sequences can be modified such that they specifically bind to receptors or antigens expressed on a selected cell surface, e.g., by linking the antisense nucleic acid sequence to peptides or antibodies which bind to cell surface receptors or antigens. The antisense nucleic acid sequences can also be delivered to cells using the vectors described herein.

According to a further aspect, the antisense nucleic acid sequence is an a-anomeric nucleic acid sequence. An a-anomeric nucleic acid sequence forms specific double-stranded hybrids with complementary RNA in which, contrary to the usual b-units, the strands run parallel to each other (Gaultier et al. (1987) Nucl Ac Res 15: 6625-6641). The antisense nucleic acid sequence may also comprise a 2'-o-methylribonucleotide (Inoue et al. (1987) Nucl Ac Res 15, 6131-6148) or a chimeric RNA-DNA analogue (Inoue et al. (1987) FEBS Lett. 215, 327-330).

The reduction or substantial elimination of endogenous gene expression may also be performed using ribozymes. Ribozymes are catalytic RNA molecules with ribonuclease activity that are capable of cleaving a single-stranded nucleic acid sequence, such as an mRNA, to which they have a complementary region. Thus, ribozymes (e.g., hammerhead ribozymes (described in Haselhoff and Gerlach (1988) Nature 334, 585-591) can be used to catalytically cleave mRNA transcripts encoding a polypeptide, thereby substantially reducing the number of mRNA transcripts to be translated into a polypeptide. A ribozyme having specificity for a nucleic acid sequence can be designed (see for example: Cech et al. U.S. Patent No. 4,987,071; and Cech et al. U.S. Patent No. 5,116,742). Alternatively, mRNA transcripts corresponding to a nucleic acid sequence can be used to select a catalytic RNA having a specific ribonuclease activity from a pool of RNA molecules (Bartel and Szostak (1993) Science 261, 1411-1418). The use of ribozymes for gene silencing in plants is known in the art (e.g., Atkins et al. (1994) WO 94/00012; Lenne et al. (1995) WO 95/03404; Lutziger et al. (2000) WO 00/00619; Prinsen et al. (1997) WO 97/13865 and Scott et al. (1997) WO 97/38116).

Gene silencing may also be achieved by insertion mutagenesis (for example, T-DNA insertion or transposon insertion) or by strategies as described by, among others, Angell and Baulcombe ((1999) Plant J 20(3): 357-62), (Amplicon VIGS WO 98/36083), or Baulcombe (WO 99/15682).

Gene silencing may also occur if there is a mutation on an endogenous gene and/or a mutation on an isolated gene/nucleic acid subsequently introduced into a plant. The reduction or substantial elimination may be caused by a non-functional polypeptide. For example, the polypeptide may bind to various interacting proteins; one or more mutation(s) and/or truncation(s) may therefore provide for a polypeptide that is still able to bind interacting proteins (such as receptor proteins) but that cannot exhibit its normal function (such as signalling ligand).

A further approach to gene silencing is by targeting nucleic acid sequences complementary to the regulatory region of the gene (e.g., the promoter and/or enhancers) to form triple helical structures that prevent transcription of the gene in target cells. See Helene, C., Anticancer Drug Res. 6, 569-84, 1991; Helene et al., Ann. N.Y. Acad. Sci. 660, 27-36 1992; and Maher, L.J. Bioassays 14, 807-15, 1992.

Other methods, such as the use of antibodies directed to an endogenous polypeptide for inhibiting its function in planta, or interference in the signalling pathway in which a polypeptide is involved, will be well known to the skilled man. In particular, it can be envisaged that manmade molecules may be useful for inhibiting the biological function of a target polypeptide, or for interfering with the signalling pathway in which the target polypeptide is involved.

Alternatively, a screening program may be set up to identify in a plant population natural variants of a gene, which variants encode polypeptides with reduced activity. Such natural variants may also be used for example, to perform homologous recombination.

Artificial and/or natural microRNAs (miRNAs) may be used to knock out gene expression and/or mRNA translation. Endogenous miRNAs are single stranded small RNAs of typically 19-24 nucleotides long. They function primarily to regulate gene expression and/ or mRNA translation. Most plant microRNAs (miRNAs) have perfect or near-perfect complementarity with their target sequences. However, there are natural targets with up to five mismatches. They are processed from longer non-coding RNAs with characteristic fold-back structures by double-strand specific RNases of the Dicer family. Upon processing, they are incorporated in the RNA-induced silencing complex (RISC) by binding to its main component, an Argonaute protein. MiRNAs serve as the specificity components of RISC, since they base-pair to target nucleic acids, mostly mRNAs, in the cytoplasm. Subsequent regulatory events include target mRNA cleavage and destruction and/or translational inhibition. Effects of miRNA overexpression are thus often reflected in decreased mRNA levels of target genes.

Artificial microRNAs (amiRNAs), which are typically 21 nucleotides in length, can be genetically engineered specifically to negatively regulate gene expression of single or multiple genes of interest. Determinants of plant microRNA target selection are well known in the art. Empirical parameters for target recognition have been defined and can be used to aid in the design of specific amiRNAs, (Schwab et al., Dev. Cell 8, 517-527, 2005). Convenient tools for design and generation of amiRNAs and their precursors are also available to the public (Schwab et al., Plant Cell 18, 1121-1133, 2006).

For optimal performance, the gene silencing techniques used for reducing expression in a plant of an endogenous gene requires the use of nucleic acid sequences from monocotyledonous plants for transformation of monocotyledonous plants, and from dicotyledonous plants for transformation of dicotyledonous plants. Preferably, a nucleic acid sequence from any given plant species is introduced into that same species. For example, a nucleic acid sequence from rice is transformed into a rice plant. However, it is not an absolute requirement that the nucleic acid sequence to be introduced originates from the same plant species as the plant in which it will be introduced. It is sufficient that there is substantial homology between the endogenous target gene and the nucleic acid to be introduced.

Described above are examples of various methods for the reduction or substantial elimination of expression in a plant of an endogenous gene. A person skilled in the art would readily be able to adapt the aforementioned methods for silencing so as to achieve reduction of expression of an endogenous gene in a whole plant or in parts thereof through the use of an appropriate promoter, for example.

### Selectable marker (gene)/Reporter gene

"Selectable marker", "selectable marker gene" or "reporter gene" includes any gene that confers a phenotype on a cell in which it is expressed to facilitate the identification and/or selection of cells that are transfected or transformed with a nucleic acid construct of the invention. These marker genes enable the identification of a successful transfer of the nucleic acid molecules via a series of different principles. Suitable markers may be selected from markers that confer antibiotic or herbicide resistance, that introduce a new metabolic trait or that allow visual selection. Examples of selectable marker genes include genes conferring resistance to antibiotics (such as nptll that phosphorylates neomycin and kanamycin, or hpt, phosphorylating hygromycin, or genes conferring resistance to, for example, bleomycin, streptomycin, tetracyclin, chloramphenicol, ampicillin, gentamycin, geneticin (G418), spectinomycin or blasticidin), to herbicides (for example bar which provides resistance to Basta^{®}; aroA or gox providing resistance against glyphosate, or the genes conferring resistance to, for example, imidazolinone, phosphinothricin or sulfonylurea), or genes that provide a metabolic trait (such as manA that allows plants to use mannose as sole carbon source or xylose isomerase for the utilisation of xylose, or antinutritive markers such as the resistance to 2-deoxyglucose). Expression of visual marker genes results in the formation of colour (for example β-glucuronidase, GUS or β-galactosidase with its coloured substrates, for example X-Gal), luminescence (such as the luciferin/luceferase system) or fluorescence (Green Fluorescent Protein, GFP, and derivatives thereof). This list represents only a small number of possible markers. The skilled worker is familiar with such markers. Different markers are preferred, depending on the organism and the selection method.

It is known that upon stable or transient integration of nucleic acids into plant cells, only a minority of the cells takes up the foreign DNA and, if desired, integrates it into its genome, depending on the expression vector used and the transfection technique used. To identify and select these integrants, a gene coding for a selectable marker (such as the ones described above) is usually introduced into the host cells together with the gene of interest. These markers can for example be used in mutants in which these genes are not functional by, for example, deletion by conventional methods. Furthermore, nucleic acid molecules encoding a selectable marker can be introduced into a host cell on the same vector that comprises the sequence encoding the polypeptides of the invention or used in the methods of the invention, or else in a separate vector. Cells which have been stably transfected with the introduced nucleic acid can be identified for example by selection (for example, cells which have integrated the selectable marker survive whereas the other cells die). The marker genes may be removed or excised from the transgenic cell once they are no longer needed. Techniques for marker gene removal are known in the art, useful techniques are described above in the definitions section.

Since the marker genes, particularly genes for resistance to antibiotics and herbicides, are no longer required or are undesired in the transgenic host cell once the nucleic acids have been introduced successfully, the process according to the invention for introducing the nucleic acids advantageously employs techniques which enable the removal or excision of these marker genes. One such a method is what is known as co-transformation. The co-transformation method employs two vectors simultaneously for the transformation, one vector bearing the nucleic acid according to the invention and a second bearing the marker gene(s). A large proportion of transformants receives or, in the case of plants, comprises (up to 40% or more of the transformants), both vectors. In case of transformation with Agrobacteria, the transformants usually receive only a part of the vector, i.e. the sequence flanked by the T-DNA, which usually represents the expression cassette. The marker genes can subsequently be removed from the transformed plant by performing crosses. In another method, marker genes integrated into a transposon are used for the transformation together with desired nucleic acid (known as the Ac/Ds technology). The transformants can be crossed with a transposase source or the transformants are transformed with a nucleic acid construct conferring expression of a transposase, transiently or stable. In some cases (approx. 10%), the transposon jumps out of the genome of the host cell once transformation has taken place successfully and is lost. In a further number of cases, the transposon jumps to a different location. In these cases the marker gene must be eliminated by performing crosses. In microbiology, techniques were developed which make possible, or facilitate, the detection of such events. A further advantageous method relies on what is known as recombination systems; whose advantage is that elimination by crossing can be dispensed with. The best-known system of this type is what is known as the Cre/lox system. Cre1 is a recombinase that removes the sequences located between the loxP sequences. If the marker gene is integrated between the loxP sequences, it is removed once transformation has taken place successfully, by expression of the recombinase. Further recombination systems are the HIN/HIX, FLP/FRT and REP/STB system (Tribble et al., J. Biol. Chem., 275, 2000: 22255-22267; Velmurugan et al., J. Cell Biol., 149, 2000: 553-566). A site-specific integration into the plant genome of the nucleic acid sequences according to the invention is possible. Naturally, these methods can also be applied to microorganisms such as yeast, fungi or bacteria.

### Transgenic/Transgene/Recombinant

For the purposes of the invention, "transgenic", "transgene" or "recombinant" means with regard to, for example, a nucleic acid sequence, an expression cassette, gene construct or a vector comprising the nucleic acid sequence or an organism transformed with the nucleic acid sequences, expression cassettes or vectors according to the invention, all those constructions brought about by recombinant methods in which either
(a) the nucleic acid sequences encoding proteins useful in the methods of the invention, or
(b) genetic control sequence(s) which is operably linked with the nucleic acid sequence according to the invention, for example a promoter, or
(c) a) and b)
are not located in their natural genetic environment or have been modified by recombinant methods, it being possible for the modification to take the form of, for example, a substitution, addition, deletion, inversion or insertion of one or more nucleotide residues. The natural genetic environment is understood as meaning the natural genomic or chromosomal locus in the original plant or the presence in a genomic library. In the case of a genomic library, the natural genetic environment of the nucleic acid sequence is preferably retained, at least in part. The environment flanks the nucleic acid sequence at least on one side and has a sequence length of at least 50 bp, preferably at least 500 bp, especially preferably at least 1000 bp, most preferably at least 5000 bp. A naturally occurring expression cassette - for example the naturally occurring combination of the natural promoter of the nucleic acid sequences with the corresponding nucleic acid sequence encoding a polypeptide useful in the methods of the present invention, as defined above - becomes a transgenic expression cassette when this expression cassette is modified by non-natural, synthetic ("artificial") methods such as, for example, mutagenic treatment. Suitable methods are described, for example, in US 5,565,350 or WO 00/15815.

A transgenic plant for the purposes of the invention is thus understood as meaning, as above, that the nucleic acids used in the method of the invention are not at their natural locus in the genome of said plant, it being possible for the nucleic acids to be expressed homologously or heterologously. However, as mentioned, transgenic also means that, while the nucleic acids according to the invention or used in the inventive method are at their natural position in the genome of a plant, the sequence has been modified with regard to the natural sequence, and/or that the regulatory sequences of the natural sequences have been modified. Transgenic is preferably understood as meaning the expression of the nucleic acids according to the invention at an unnatural locus in the genome, i.e. homologous or, preferably, heterologous expression of the nucleic acids takes place. Preferred transgenic plants are mentioned herein.

### Transformation

The term "introduction" or "transformation" as referred to herein encompasses the transfer of an exogenous polynucleotide into a host cell, irrespective of the method used for transfer. Plant tissue capable of subsequent clonal propagation, whether by organogenesis or embryogenesis, may be transformed with a genetic construct of the present invention and a whole plant regenerated there from. The particular tissue chosen will vary depending on the clonal propagation systems available for, and best suited to, the particular species being transformed. Exemplary tissue targets include leaf disks, pollen, embryos, cotyledons, hypocotyls, megagametophytes, callus tissue, existing meristematic tissue (e.g., apical meristem, axillary buds, and root meristems), and induced meristem tissue (e.g., cotyledon meristem and hypocotyl meristem). The polynucleotide may be transiently or stably introduced into a host cell and may be maintained non-integrated, for example, as a plasmid. Alternatively, it may be integrated into the host genome. The resulting transformed plant cell may then be used to regenerate a transformed plant in a manner known to persons skilled in the art.

The transfer of foreign genes into the genome of a plant is called transformation. Transformation of plant species is now a fairly routine technique. Advantageously, any of several transformation methods may be used to introduce the gene of interest into a suitable ancestor cell. The methods described for the transformation and regeneration of plants from plant tissues or plant cells may be utilized for transient or for stable transformation. Transformation methods include the use of liposomes, electroporation, chemicals that increase free DNA uptake, injection of the DNA directly into the plant, particle gun bombardment, transformation using viruses or pollen and microprojection. Methods may be selected from the calcium/polyethylene glycol method for protoplasts (Krens, F.A. et al., (1982) Nature 296, 72-74; Negrutiu I et al. (1987) Plant Mol Biol 8: 363-373); electroporation of protoplasts (Shillito R.D. et al. (1985) Bio/Technol 3, 1099-1102); microinjection into plant material (Crossway A et al., (1986) Mol. Gen Genet 202: 179-185); DNA or RNA-coated particle bombardment (Klein TM et al., (1987) Nature 327: 70) infection with (non-integrative) viruses and the like. Transgenic plants, including transgenic crop plants, are preferably produced via Agrobacterium-mediated transformation. An advantageous transformation method is the transformation in planta. To this end, it is possible, for example, to allow the agrobacteria to act on plant seeds or to inoculate the plant meristem with agrobacteria. It has proved particularly expedient in accordance with the invention to allow a suspension of transformed agrobacteria to act on the intact plant or at least on the flower primordia. The plant is subsequently grown on until the seeds of the treated plant are obtained (Clough and Bent, Plant J. (1998) 16, 735-743). Methods for Agrobacterium-mediated transformation of rice include well known methods for rice transformation, such as those described in any of the following: European patent application EP 1198985 A1, Aldemita and Hodges (Planta 199: 612-617, 1996); Chan et al. (Plant Mol Biol 22 (3): 491-506, 1993), Hiei et al. (Plant J 6 (2): 271-282, 1994), which disclosures are incorporated by reference herein as if fully set forth. In the case of corn transformation, the preferred method is as described in either Ishida et al. (Nat. Biotechnol 14(6): 745-50, 1996) or Frame et al. (Plant Physiol 129(1): 13-22, 2002), which disclosures are incorporated by reference herein as if fully set forth. Said methods are further described by way of example in B. Jenes et al., Techniques for Gene Transfer, in: Transgenic Plants, Vol. 1, Engineering and Utilization, eds. S.D. Kung and R. Wu, Academic Press (1993) 128-143 and in Potrykus Annu. Rev. Plant Physiol. Plant Molec. Biol. 42 (1991) 205-225). The nucleic acids or the construct to be expressed is preferably cloned into a vector, which is suitable for transforming Agrobacterium tumefaciens, for example pBin19 (Bevan et al., Nucl. Acids Res. 12 (1984) 8711). Agrobacteria transformed by such a vector can then be used in known manner for the transformation of plants, such as plants used as a model, like Arabidopsis (Arabidopsis thaliana is within the scope of the present invention not considered as a crop plant), or crop plants such as, by way of example, tobacco plants, for example by immersing bruised leaves or chopped leaves in an agrobacterial solution and then culturing them in suitable media. The transformation of plants by means of Agrobacterium tumefaciens is described, for example, by Höfgen and Willmitzer in Nucl. Acid Res. (1988) 16, 9877 or is known inter alia from F.F. White, Vectors for Gene Transfer in Higher Plants; in Transgenic Plants, Vol. 1, Engineering and Utilization, eds. S.D. Kung and R. Wu, Academic Press, 1993, pp. 15-38.

In addition to the transformation of somatic cells, which then have to be regenerated into intact plants, it is also possible to transform the cells of plant meristems and in particular those cells which develop into gametes. In this case, the transformed gametes follow the natural plant development, giving rise to transgenic plants. Thus, for example, seeds of Arabidopsis are treated with agrobacteria and seeds are obtained from the developing plants of which a certain proportion is transformed and thus transgenic [Feldman, KA and Marks MD (1987). Mol Gen Genet 208:274-289; Feldmann K (1992). In: C Koncz, N-H Chua and J Shell, eds, Methods in Arabidopsis Research. Word Scientific, Singapore, pp. 274-289]. Alternative methods are based on the repeated removal of the inflorescences and incubation of the excision site in the center of the rosette with transformed agrobacteria, whereby transformed seeds can likewise be obtained at a later point in time (Chang (1994). Plant J. 5: 551-558; Katavic (1994). Mol Gen Genet, 245: 363-370). However, an especially effective method is the vacuum infiltration method with its modifications such as the "floral dip" method. In the case of vacuum infiltration of Arabidopsis, intact plants under reduced pressure are treated with an agrobacterial suspension [Bechthold, N (1993). C R Acad Sci Paris Life Sci, 316: 1194-1199], while in the case of the "floral dip" method the developing floral tissue is incubated briefly with a surfactant-treated agrobacterial suspension [Clough, SJ and Bent AF (1998) The Plant J. 16, 735-743]. A certain proportion of transgenic seeds are harvested in both cases, and these seeds can be distinguished from non-transgenic seeds by growing under the above-described selective conditions. In addition the stable transformation of plastids is of advantages because plastids are inherited maternally is most crops reducing or eliminating the risk of transgene flow through pollen. The transformation of the chloroplast genome is generally achieved by a process which has been schematically displayed in Klaus et al., 2004 [Nature Biotechnology 22 (2), 225-229]. Briefly the sequences to be transformed are cloned together with a selectable marker gene between flanking sequences homologous to the chloroplast genome. These homologous flanking sequences direct site specific integration into the plastome. Plastidal transformation has been described for many different plant species and an overview is given in Bock (2001) Transgenic plastids in basic research and plant biotechnology. J Mol Biol. 2001 Sep 21; 312 (3):425-38 or Maliga, P (2003) Progress towards commercialization of plastid transformation technology. Trends Biotechnol. 21, 20-28. Further biotechnological progress has recently been reported in form of marker free plastid transformants, which can be produced by a transient co-integrated maker gene (Klaus et al., 2004, Nature Biotechnology 22(2), 225-229).

### T-DNA activation tagging

T-DNA activation tagging (Hayashi et al. Science (1992) 1350-1353), involves insertion of T-DNA, usually containing a promoter (may also be a translation enhancer or an intron), in the genomic region of the gene of interest or 10 kb up- or downstream of the coding region of a gene in a configuration such that the promoter directs expression of the targeted gene. Typically, regulation of expression of the targeted gene by its natural promoter is disrupted and the gene falls under the control of the newly introduced promoter. The promoter is typically embedded in a T-DNA. This T-DNA is randomly inserted into the plant genome, for example, through Agrobacterium infection and leads to modified expression of genes near the inserted T-DNA. The resulting transgenic plants show dominant phenotypes due to modified expression of genes close to the introduced promoter.

### TILLING

The term "TILLING" is an abbreviation of "Targeted Induced Local Lesions In Genomes" and refers to a mutagenesis technology useful to generate and/or identify nucleic acids encoding proteins with modified expression and/or activity. TILLING also allows selection of plants carrying such mutant variants. These mutant variants may exhibit modified expression, either in strength or in location or in timing (if the mutations affect the promoter for example). These mutant variants may exhibit higher activity than that exhibited by the gene in its natural form. TILLING combines high-density mutagenesis with high-throughput screening methods. The steps typically followed in TILLING are: (a) EMS mutagenesis (Redei GP and Koncz C (1992) In Methods in Arabidopsis Research, Koncz C, Chua NH, Schell J, eds. Singapore, World Scientific Publishing Co, pp. 16-82; Feldmann et al., (1994) In Meyerowitz EM, Somerville CR, eds, Arabidopsis. Cold Spring Harbor Laboratory Press, Cold Spring Harbor, NY, pp 137-172; Lightner J and Caspar T (1998) In J Martinez-Zapater, J Salinas, eds, Methods on Molecular Biology, Vol. 82. Humana Press, Totowa, NJ, pp 91-104); (b) DNA preparation and pooling of individuals; (c) PCR amplification of a region of interest; (d) denaturation and annealing to allow formation of heteroduplexes; (e) DHPLC, where the presence of a heteroduplex in a pool is detected as an extra peak in the chromatogram; (f) identification of the mutant individual; and (g) sequencing of the mutant PCR product. Methods for TILLING are well known in the art (McCallum et al., (2000) Nat Biotechnol 18: 455-457; reviewed by Stemple (2004) Nat Rev Genet 5(2): 145-50).

### Homologous recombination

Homologous recombination allows introduction in a genome of a selected nucleic acid at a defined selected position. Homologous recombination is a standard technology used routinely in biological sciences for lower organisms such as yeast or the moss Physcomitrella. Methods for performing homologous recombination in plants have been described not only for model plants (Offringa et al. (1990) EMBO J 9(10): 3077-84) but also for crop plants, for example rice (Terada et al. (2002) Nat Biotech 20(10): 1030-4; lida and Terada (2004) Curr Opin Biotech 15(2): 132-8), and approaches exist that are generally applicable regardless of the target organism (Miller et al, Nature Biotechnol. 25, 778-785, 2007).

### Yield

The term "yield" in general means a measurable produce of economic value, typically related to a specified crop, to an area, and to a period of time. Individual plant parts directly contribute to yield based on their number, size and/or weight, or the actual yield is the yield per square meter for a crop and year, which is determined by dividing total production (includes both harvested and appraised production) by planted square meters. The term "yield" of a plant may relate to vegetative biomass (root and/or shoot biomass), to reproductive organs, and/or to propagules (such as seeds) of that plant.

### Early vigour

"Early vigour" refers to active healthy well-balanced growth especially during early stages of plant growth, and may result from increased plant fitness due to, for example, the plants being better adapted to their environment (i.e. optimizing the use of energy resources and partitioning between shoot and root). Plants having early vigour also show increased seedling survival and a better establishment of the crop, which often results in highly uniform fields (with the crop growing in uniform manner, i.e. with the majority of plants reaching the various stages of development at substantially the same time), and often better and higher yield. Therefore, early vigour may be determined by measuring various factors, such as thousand kernel weight, percentage germination, percentage emergence, seedling growth, seedling height, root length, root and shoot biomass and many more.

### Increase/Improve/Enhance

The terms "increase", "improve" or "enhance" are interchangeable and shall mean in the sense of the application at least a 3%, 4%, 5%, 6%, 7%, 8%, 9% or 10%, preferably at least 15% or 20%, more preferably 25%, 30%, 35% or 40% more yield and/or growth in comparison to control plants as defined herein.

### Seed yield

Increased seed yield may manifest itself as one or more of the following: a) an increase in seed biomass (total seed weight) which may be on an individual seed basis and/or per plant and/or per square meter; b) increased number of flowers per plant; c) increased number of (filled) seeds; d) increased seed filling rate (which is expressed as the ratio between the number of filled seeds divided by the total number of seeds); e) increased harvest index, which is expressed as a ratio of the yield of harvestable parts, such as seeds, divided by the total biomass; and f) increased thousand kernel weight (TKW), and g) increased number of primary panicles, which is extrapolated from the number of filled seeds counted and their total weight. An increased TKW may result from an increased seed size and/or seed weight, and may also result from an increase in embryo and/or endosperm size.

An increase in seed yield may also be manifested as an increase in seed size and/or seed volume. Furthermore, an increase in seed yield may also manifest itself as an increase in seed area and/or seed length and/or seed width and/or seed perimeter. Increased seed yield may also result in modified architecture, or may occur because of modified architecture.

### Greenness Index

The "greenness index" as used herein is calculated from digital images of plants. For each pixel belonging to the plant object on the image, the ratio of the green value versus the red value (in the RGB model for encoding color) is calculated. The greenness index is expressed as the percentage of pixels for which the green-to-red ratio exceeds a given threshold. Under normal growth conditions, under salt stress growth conditions, and under reduced nutrient availability growth conditions, the greenness index of plants is measured in the last imaging before flowering. In contrast, under drought stress growth conditions, the greenness index of plants is measured in the first imaging after drought.

### Plant

The term "plant" as used herein encompasses whole plants, ancestors and progeny of the plants and plant parts, including seeds, shoots, stems, leaves, roots (including tubers), flowers, and tissues and organs, wherein each of the aforementioned comprise the gene/nucleic acid of interest. The term "plant" also encompasses plant cells, suspension cultures, callus tissue, embryos, meristematic regions, gametophytes, sporophytes, pollen and microspores, again wherein each of the aforementioned comprises the gene/nucleic acid of interest.

Plants that are particularly useful in the methods of the invention include all plants which belong to the superfamily Viridiplantae, in particular monocotyledonous and dicotyledonous plants including fodder or forage legumes, ornamental plants, food crops, trees or shrubs selected from the list comprising Acer spp., Actinidia spp., Abelmoschus spp., Agave sisalana, Agropyron spp., Agrostis stolonifera, Allium spp., Amaranthus spp., Ammophila arenaria, Ananas comosus, Annona spp., Apium graveolens, Arachis spp, Artocarpus spp., Asparagus officinalis, Avena spp. (e.g. Avena sativa, Avena fatua, Avena byzantina, Avena fatua var. sativa, Avena hybrida), Averrhoa carambola, Bambusa sp., Benincasa hispida, Bertholletia excelsea, Beta vulgaris, Brassica spp. (e.g. Brassica napus, Brassica rapa ssp. [canola, oilseed rape, turnip rape]), Cadaba farinosa, Camellia sinensis, Canna indica, Cannabis sativa, Capsicum spp., Carex elata, Carica papaya, Carissa macrocarpa, Carya spp., Carthamus tinctorius, Castanea spp., Ceiba pentandra, Cichorium endivia, Cinnamomum spp., Citrullus lanatus, Citrus spp., Cocos spp., Coffea spp., Colocasia esculenta, Cola spp., Corchorus sp., Coriandrum sativum, Corylus spp., Crataegus spp., Crocus sativus, Cucurbita spp., Cucumis spp., Cynara spp., Daucus carota, Desmodium spp., Dimocarpus longan, Dioscorea spp., Diospyros spp., Echinochloa spp., Elaeis (e.g. Elaeis guineensis, Elaeis oleifera), Eleusine coracana, Eragrostis tef, Erianthus sp., Eriobotrya japonica, Eucalyptus sp., Eugenia uniflora, Fagopyrum spp., Fagus spp., Festuca arundinacea, Ficus carica, Fortunella spp., Fragaria spp., Ginkgo biloba, Glycine spp. (e.g. Glycine max, Soja hispida or Soja max), Gossypium hirsutum, Helianthus spp. (e.g. Helianthus annuus), Hemerocallis fulva, Hibiscus spp., Hordeum spp. (e.g. Hordeum vulgare), Ipomoea batatas, Juglans spp., Lactuca sativa, Lathyrus spp., Lens culinaris, Linum usitatissimum, Litchi chinensis, Lotus spp., Luffa acutangula, Lupinus spp., Luzula sylvatica, Lycopersicon spp. (e.g. Lycopersicon esculentum, Lycopersicon lycopersicum, Lycopersicon pyriforme), Macrotyloma spp., Malus spp., Malpighia emarginata, Mammea americana, Mangifera indica, Manihot spp., Manilkara zapota, Medicago sativa, Melilotus spp., Mentha spp., Miscanthus sinensis, Momordica spp., Morus nigra, Musa spp., Nicotiana spp., Olea spp., Opuntia spp., Ornithopus spp., Oryza spp. (e.g. Oryza sativa, Oryza latifolia), Panicum miliaceum, Panicum virgatum, Passiflora edulis, Pastinaca sativa, Pennisetum sp., Persea spp., Petroselinum crispum, Phalaris arundinacea, Phaseolus spp., Phleum pratense, Phoenix spp., Phragmites australis, Physalis spp., Pinus spp., Pistacia vera, Pisum spp., Poa spp., Populus spp., Prosopis spp., Prunus spp., Psidium spp., Punica granatum, Pyrus communis, Quercus spp., Raphanus sativus, Rheum rhabarbarum, Ribes spp., Ricinus communis, Rubus spp., Saccharum spp., Salix sp., Sambucus spp., Secale cereale, Sesamum spp., Sinapis sp., Solanum spp. (e.g. Solanum tuberosum, Solanum integrifolium or Solanum lycopersicum), Sorghum bicolor, Spinacia spp., Syzygium spp., Tagetes spp., Tamarindus indica, Theobroma cacao, Trifolium spp., Tripsacum dactyloides, Triticale sp., Triticosecale rimpaui, Triticum spp. (e.g. Triticum aestivum, Triticum durum, Triticum turgidum, Triticum hybernum, Triticum macha, Triticum sativum, Triticum monococcum or Triticum vulgare), Tropaeolum minus, Tropaeolum majus, Vaccinium spp., Vicia spp., Vigna spp., Viola odorata, Vitis spp., Zea mays, Zizania palustris, Ziziphus spp., amongst others.

### Detailed description of the invention

Surprisingly, it has now been found that modulating expression in a plant of a nucleic acid encoding an alfin-like polypeptide gives plants having enhanced abiotic stress tolerance relative to control plants. According to a first embodiment, the present invention provides a method for enhancing tolerance to various abiotic stresses in plants relative to control plants, comprising modulating expression in a plant of a nucleic acid encoding an alfin-like polypeptide and optionally selecting for plants having enhanced tolerance to abiotic stress.

Furthermore, it has now surprisingly been found that modulating expression in a plant of a nucleic acid encoding a YRP polypeptide gives plants having enhanced abiotic stress tolerance relative to control plants. According to a first embodiment, the present invention provides a method for enhancing tolerance to various abiotic stresses in plants relative to control plants, comprising modulating expression in a plant of a nucleic acid encoding a YRP polypeptide and optionally selecting for plants having enhanced tolerance to abiotic stress.

Furthermore, it has now surprisingly been found that increasing expression in a plant of a nucleic acid sequence encoding a BRXL polypeptide as defined herein, gives plants having increased yield-related traits relative to control plants. According to a first embodiment, the present invention provides a method for increasing yield-related traits in plants relative to control plants, comprising increasing expression in a plant of a nucleic acid sequence encoding a BRXL polypeptide.

The invention also provides hitherto unknown nucleic acid sequences encoding BRXL polypeptides, and BRXL polypeptides.

According to one embodiment of the present invention, there is therefore provided an isolated nucleic acid molecule selected from:
(i) a nucleic acid sequence as represented by any one of SEQ ID NO: 75, SEQ ID NO: 77, or SEQ ID NO: 79;
(ii) the complement of a nucleic acid sequence as represented by any one of SEQ ID NO: 75, SEQ ID NO: 77, or SEQ ID NO: 79;
(iii) a nucleic acid sequence encoding a BRXL polypeptide having, in increasing order of preference, at least 50%, 55%, 60%, 65%, 70%, 75%, 80%, 85%, 90%, 95%, 96%, 97%, 98%, 99% or more amino acid sequence identity to the polypeptide sequence represented by any one of SEQ ID NO: 76, SEQ ID NO: 78, or SEQ ID NO: 80.

According to a further embodiment of the present invention, there is also provided an isolated polypeptide selected from:
(i) a polypeptide sequence as represented by any one of SEQ ID NO: 76, SEQ ID NO: 78, or SEQ ID NO: 80;
(ii) a polypeptide sequence having, in increasing order of preference, at least 50%, 55%, 60%, 65%, 70%, 75%, 80%, 85%, 90%, 95%, 96%, 97%, 98%, 99% or more amino acid sequence identity to a polypeptide sequence as represented by any one of SEQ ID NO: 76, SEQ ID NO: 78, or SEQ ID NO: 80;
(iii) derivatives of any of the polypeptide sequences given in (i) or (ii) above.

Furthermore, it has now surprisingly been found that modulating expression in a plant of a nucleic acid encoding a silky-1-like polypeptide gives plants having enhanced abiotic stress tolerance relative to control plants. According to a first embodiment, the present invention provides a method for enhancing tolerance to various abiotic stresses in plants relative to control plants, comprising modulating expression in a plant of a nucleic acid encoding a silky-1-like polypeptide and optionally selecting for plants having enhanced tolerance to abiotic stress.

Furthermore, it has now surprisingly been found that modulating expression in a plant of a nucleic acid encoding an ARP6 polypeptide gives plants having enhanced yield-related traits relative to control plants. According to a first embodiment, the present invention provides a method for enhancing yield-related traits in plants relative to control plants, comprising modulating expression in a plant of a nucleic acid encoding an ARP6 polypeptide and optionally selecting for plants having enhanced yield-related traits.

Furthermore, it has now surprisingly been found that modulating expression in a plant of a nucleic acid encoding a Prolyl-oligopeptidase, hereafter named "POP polypeptide" gives plants having enhanced yield-related traits relative to control plants. According to a first embodiment, the present invention provides a method for enhancing yield-related traits in plants relative to control plants, comprising modulating expression in a plant of a nucleic acid encoding a POP polypeptide and optionally selecting for plants having enhanced yield-related traits.

Furthermore, it has now surprisingly found that modulating expression in a plant of a nucleic acid encoding a CRL polypeptide gives plants having enhanced yield-related traits relative to control plants. According to a first embodiment, the present invention provides a method for enhancing yield-related traits in plants relative to control plants, comprising modulating expression in a plant of a nucleic acid encoding a CRL polypeptide and optionally selecting for plants having enhanced yield-related traits, preferably any one of total seed yield (Totalwgseeds), number of filled seeds (nrfilledseed), fill rate (fillrate), and harvest index (harvestindex), and/or identifying the transgenic plant by selecting the transgenic plant that overexpresses CRL gene (introduced or endogeneous) and/or the CRL protein.

A preferred method for modulating (preferably, increasing) expression of a nucleic acid encoding an alfin-like polypeptide, or a YRP polypeptide, or a BRXL polypeptide, or a silky-1-like polypeptide, or an ARP6 polypeptide, or a POP polypeptide, or a CRL polypeptide, is by introducing and expressing in a plant a nucleic acid encoding an alfin-like polypeptide, a YRP polypeptide, or a BRXL polypeptide, or a silky-1-like polypeptide, or an ARP6 polypeptide, or a POP polypeptide, or a CRL polypeptide.

Concerning alfin-like polypeptides, any reference hereinafter to a "protein useful in the methods of the invention" is taken to mean an alfin-like polypeptide as defined herein. Any reference hereinafter to a "nucleic acid useful in the methods of the invention" is taken to mean a nucleic acid capable of encoding such an alfin-like polypeptide. The nucleic acid to be introduced into a plant (and therefore useful in performing the methods of the invention) is any nucleic acid encoding the type of protein which will now be described, hereafter also named "alfin-like nucleic acid" or "alfin-like gene".

Concerning YRP polypeptides, any reference hereinafter to a "protein useful in the methods of the invention" is taken to mean a YRP polypeptide as defined herein. Any reference hereinafter to a "nucleic acid useful in the methods of the invention" is taken to mean a nucleic acid capable of encoding such a YRP polypeptide. The nucleic acid to be introduced into a plant (and therefore useful in performing the methods of the invention) is any nucleic acid encoding the type of protein which will now be described, hereafter also named "YRP nucleic acid" or "YRP gene".

The YRP of Hordeum vulgare (SEQ ID NO: 11 and 13) are transcription factors encoding members of the GARP class of transcription factors.

Concerning BRXL polypeptides, any reference hereinafter to a "protein useful in the methods of the invention" is taken to mean a BRXL polypeptide as defined herein. Any reference hereinafter to a "nucleic acid sequence useful in the methods of the invention" is taken to mean a nucleic acid sequence capable of encoding such a BRXL polypeptide. The nucleic acid sequence to be introduced into a plant (and therefore useful in performing the methods of the invention) is any nucleic acid sequence encoding the type of polypeptide, which will now be described, hereafter also named "BRXL nucleic acid sequence" or "BRXL gene".

Concerning silky-1-like polypeptides, any reference hereinafter to a "protein useful in the methods of the invention" is taken to mean a silky-1-like polypeptide as defined herein. Any reference hereinafter to a "nucleic acid useful in the methods of the invention" is taken to mean a nucleic acid capable of encoding such a silky-1-like polypeptide. The nucleic acid to be introduced into a plant (and therefore useful in performing the methods of the invention) is any nucleic acid encoding the type of protein which will now be described, hereafter also named "silky-1-like nucleic acid" or "silky-1-like gene".

Concerning ARP6 polypeptides, any reference hereinafter to a "protein useful in the methods of the invention" is taken to mean an ARP6 polypeptide as defined herein. Any reference hereinafter to a "nucleic acid useful in the methods of the invention" is taken to mean a nucleic acid capable of encoding such an ARP6 polypeptide. The nucleic acid to be introduced into a plant (and therefore useful in performing the methods of the invention) is any nucleic acid encoding the type of protein which will now be described, hereafter also named "ARP6 nucleic acid" or "ARP6 gene".

Concerning POP polypeptides, any reference hereinafter to a "protein useful in the methods of the invention" is taken to mean a POP polypeptide as defined herein. Any reference hereinafter to a "nucleic acid useful in the methods of the invention" is taken to mean a nucleic acid capable of encoding such a POP polypeptide. The nucleic acid to be introduced into a plant (and therefore useful in performing the methods of the invention) is any nucleic acid encoding the type of protein which will now be described, hereafter also named "POP nucleic acid" or "POP gene".

Concerning CRL polypeptides, any reference hereinafter to a "protein useful in the methods of the invention" is taken to mean a CRL polypeptide as defined herein. Any reference hereinafter to a "nucleic acid useful in the methods of the invention" is taken to mean a nucleic acid capable of encoding such a CRL polypeptide. The nucleic acid to be introduced into a plant (and therefore useful in performing the methods of the invention) is any nucleic acid encoding the type of protein which will now be described, hereafter also named "CRL nucleic acid" or "CRL gene".

An "alfin-like polypeptide" as defined herein refers to any polypeptide comprising a core hexamer motif of either GNGGTG or GTGGNG.

Examples of such alfin-like polypeptides include orthologues and paralogues of the sequences represented by any of SEQ ID NO: 2 and SEQ ID NO: 4.

Alfin-like polypeptides and orthologues and paralogues thereof typically have in increasing order of preference at least 25%, 26%, 27%, 28%, 29%, 30%, 31 %, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% overall sequence identity to the amino acid represented by any of SEQ ID NO: 2 and SEQ ID NO: 4.

The overall sequence identity is determined using a global alignment algorithm, such as the Needleman Wunsch algorithm in the program GAP (GCG Wisconsin Package, Accelrys), preferably with default parameters and preferably with sequences of mature proteins (i.e. without taking into account secretion signals or transit peptides). Compared to overall sequence identity, the sequence identity will generally be higher when only conserved domains or motifs are considered.

Preferably, the polypeptide sequence which when used in the construction of a phylogenetic tree, clusters with the group of alfin-like polypeptides comprising the amino acid sequences represented by SEQ ID NO: 2 and SEQ ID NO: 4. rather than with any other group. Tools and techniques for the construction and analysis of phylogenetic trees are well known in the art.

A "YRP polypeptide" as defined herein refers to any polypeptide according to SEQ ID NO: 11 and SEQ ID NO: 13 and orthologues and paralogues of the sequences represented by any of SEQ ID NO: 11 and SEQ ID NO: 13.

YRP polypeptides and orthologues and paralogues thereof typically have in increasing order of preference at least 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% overall sequence identity to the amino acid represented by any of SEQ ID NO: 11 and SEQ ID NO: 13.

The overall sequence identity is determined using a global alignment algorithm, such as the Needleman Wunsch algorithm in the program GAP (GCG Wisconsin Package, Accelrys), preferably with default parameters and preferably with sequences of mature proteins (i.e. without taking into account secretion signals or transit peptides). Compared to overall sequence identity, the sequence identity will generally be higher when only conserved domains or motifs are considered.

Preferably, the polypeptide sequence which when used in the construction of a phylogenetic tree, clusters with the group of YRP polypeptides comprising the amino acid sequences represented by SEQ ID NO: 11 and SEQ ID NO: 13 rather than with any other group. Tools and techniques for the construction and analysis of phylogenetic trees are well known in the art.

A "BRXL polypeptide" as defined herein refers to any polypeptide comprising at least two BRX domains with an InterPro entry IPR013591 DZC domain (PFAM entry PF08381 DZC).

Alternatively or additionally, "BRXL polypeptide" as defined herein refers to any polypeptide comprising (i) in increasing order of preference at least 50%, 55%, 60%, 65%, 70%, 75%, 80%, 85%, 90%, 95%, 98%, 99% or more amino acid sequence identity to a BRX domain as represented by SEQ ID NO: 81; and (ii) in increasing order of preference at least 50%, 55%, 60%, 65%, 70%, 75%, 80%, 85%, 90%, 95%, 98%, 99% or more amino acid sequence identity to a BRX domain as represented by SEQ ID NO: 82.

Alternatively or additionally, "BRXL polypeptide" as defined herein refers to any polypeptide comprising (i) in increasing order of preference at least 50%, 55%, 60%, 65%, 70%, 75%, 80%, 85%, 90%, 95%, 98%, 99% or more amino acid sequence identity to a Conserved Domain 1 (comprising a BRX domain) as represented by SEQ ID NO: 83; and (ii) in increasing order of preference at least 50%, 55%, 60%, 65%, 70%, 75%, 80%, 85%, 90%, 95%, 98%, 99% or more amino acid sequence identity to a Conserved Domain 2 (comprising a BRX domain) as represented by SEQ ID NO: 84.

Additionally, a "BRXL polypeptide" as defined herein further comprises (1) in increasing order of preference at least 50%, 55%, 60%, 65%, 70%, 75%, 80%, 85%, 90%, 95%, 98%, 99% or more amino acid sequence identity to a Conserved domain 3 as represented by SEQ ID NO: 85; and (1) in increasing order of preference at least 50%, 55%, 60%, 65%, 70%, 75%, 80%, 85%, 90%, 95%, 98%, 99% or more amino acid sequence identity to a Conserved domain 4 as represented by SEQ ID NO: 86.

Alternatively or additionally, a "BRXL polypeptide" as defined herein refers to any polypeptide sequence having in increasing order of preference at least 40%, 45%, 50%, 55%, 60%, 65%, 70%, 75%, 80%, 85%, 90%, 95%, 98%, 99% or more amino acid sequence identity to a polypeptide as represented by SEQ ID NO: 18.

Alternatively or additionally, a "BRXL polypeptide" as defined herein refers to any polypeptide having in increasing order of preference at least 40%, 45%, 50%, 55%, 60%, 65%, 70%, 75%, 80%, 85%, 90%, 95%, 98%, 99% or more amino acid sequence identity to any of the polypeptide sequences given in Table A3 herein.

Alternatively or additionally, a "BRXL polypeptide" as defined herein refers to any polypeptide, which in a yeast two hybrid assay, interacts with itself or with another BRLX polypeptide.

An "silky-1-like polypeptide" as defined herein refers to any polypeptide represented by any of SEQ ID NO: 91, SEQ ID NO: 93 and SEQ ID NO: 95 and orthologues and paralogues thereof.

Silky-1-like polypeptides and orthologues and paralogues thereof typically have in increasing order of preference at least 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% overall sequence identity to the amino acid represented by any of SEQ ID NO: 91, SEQ ID NO: 93 and SEQ ID NO: 95.

The overall sequence identity is determined using a global alignment algorithm, such as the Needleman Wunsch algorithm in the program GAP (GCG Wisconsin Package, Accelrys), preferably with default parameters and preferably with sequences of mature proteins (i.e. without taking into account secretion signals or transit peptides). Compared to overall sequence identity, the sequence identity will generally be higher when only conserved domains or motifs are considered.

Preferably, the polypeptide sequence which when used in the construction of a phylogenetic tree, clusters with the group of silky-1-like polypeptides comprising the amino acid sequences represented by SEQ ID NO: 91, SEQ ID NO: 93 and SEQ ID NO: 95 rather than with any other group. Tools and techniques for the construction and analysis of phylogenetic trees are well known in the art.

An "ARP6 polypeptide" as defined herein refers to any polypeptide comprising an Actin domain and having in increasing order of preference at least 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% overall sequence identity to the amino acid represented by SEQ ID NO: 102 or by any polypeptide of Table A5.

ARP6 polypeptides typically comprise an Actin domain. Actin domains are well known in the art. For example, the Pfam database of protein domains (Bateman et al., Nucleic Acids Research 30(1): 276-280 (2002)) refers to the Actin domain as having the reference number: PF00022.

The Pfam PF00022 domain is based around hidden Markov model (HMM) searches as provided by the HMMER2 package. In HMMER2, like BLAST, E-values (expectation values) are calculated. The E-value is the number of hits that would be expected to have a score equal or better than this by chance alone. A good E-value is much less than 1. Around 1 is what we expect just by chance. In principle, all you need to decide on the significance of a match is the E-value. Bellow are the domain scores that define the Actin domain as provided in the Pfam database.

| Parameter | HMM model | | | |
|---|---|---|---|---|
| | Is model | | fs model | |
| | Sequence | Domain Score | Sequence | Domain Score |
| Gathering cut-off | -144.0 | -144.0 | 12.4 | 12.4 |
| Trusted cut-off | -144.0 | -144.0 | 12.6 | 12.6 |
| Noise cut-off | -145.0 | -145.0 | 12.3 | 12.3 |

The HMM model used to build the Actin domain is indicated. The order that the Is (global) and fs (fragment) matches are aligned to the model to give the full alignment. The build method can be global first, where Is matches are aligned first followed by fs matches that do not overlap, byscore, where matches are aligned in order of evalue score, or localfirst, where fs matches are aligned first followed by Is matches that do not overlap. The score of a single domain aligned to a HMM is indicated. If there is more than one domain, the sequence score is the sum of all the domain scores for that Pfam entry. If there is only a single domain, the sequence and the domains score for the protein will be identical.

The gathering cut-off used of the Actin domain is indicated. This value is the search threshold used to build the full alignment. The gathering cut-off is the minimum score a sequence must attain in order to belong the the full alignment of a Pfam entry. For each Pfam HMM there are two cutoff values, a sequence cutoff and a domain cutoff.

The trusted cutoff refers to the bit scores of the lowest scoring match in the full alignment.

The noise cutoff (NC) refers to the bit scores of the highest scoring match not in the full alignment.

Alternatively, the homologue of an ARP6 protein has in increasing order of preference at least 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% overall sequence identity to the amino acid represented by SEQ ID NO: 102, provided that the homologous protein comprises the conserved motifs as outlined above. The overall sequence identity is determined using a global alignment algorithm, such as the Needleman Wunsch algorithm in the program GAP (GCG Wisconsin Package, Accelrys), preferably with default parameters and preferably with sequences of mature proteins (i.e. without taking into account secretion signals or transit peptides). Compared to overall sequence identity, the sequence identity will generally be higher when only conserved domains or motifs are considered.

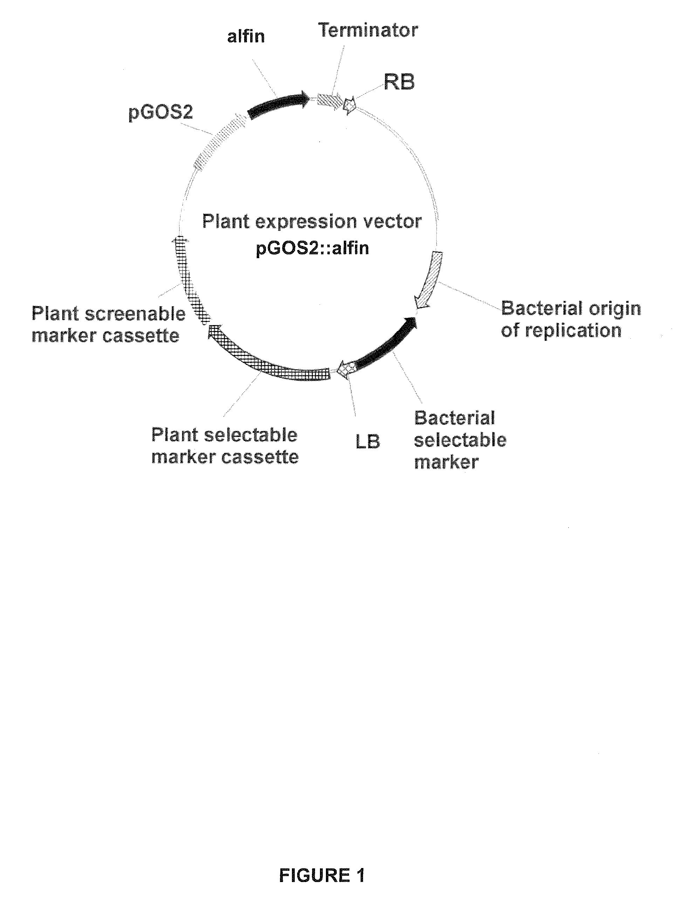

Preferably, the polypeptide sequence which when used in the construction of a phylogenetic tree, such as the one depicted in Figure 1 of Kandasamy et al. 2004. Trends Plant Sci 9: 196-202, clusters with the ARP6 group of polypeptides comprising the amino acid sequence of AtARP6 as represented by SEQ ID NO: 102 rather than with any other group.

A "Prolyl-oligopeptidase" or "POP polypeptide" as defined herein refers to any serine protease that classifies as an S9 family peptidase in the MEROPS database. Within the serine proteases, the POP proteins belong to the α/β hydrolase fold within the SC clan (Tripathi & Sowdhamini, BMC Genomics, 7, 200, 2006). Preferably, the POP polypeptide belongs to the S9B subfamily of the Prolyl-oligopeptidases. The POP polypeptide useful in the methods of the invention comprises a Peptidase_S9 domain (Pfam entry PF00326) and preferably also a DPPIV_N domain (Pfam entry PF00930).

Preferably, the POP polypeptide also comprises one or more of the following motifs:
Motif 1 (SEQ ID NO: 118):
   (V/S/L)(Y/H)GGP
Motif 2 (SEQ ID NO: 119):
   (Q/A)(Y/F)(L/W)(R/T/S)(S/N)(R/Q/K/I)G(I/W/Y)(L/A/S)(V/F/Q)(W/V/A/L)(K/D/I)(L/M/V/I)(D/N )(N/Y)(R/G)G(S/T)(A/S/T/L)(R/G)(R/Y)G(L/R/E)
Motif 3 (SEQ ID NO: 120):
   (R/H)(I/L)(G/C/T)(I/L/V)(Y/C/S/T/L)G(W/G/R)S(Y/A/H)GG(Y/F)(M/L/T)(A/S/T)
Motif 4 (SEQ ID NO: 121):
   (Y/F)(D/E)(T/S/A)(Y/H/F/R)(Y/G)(T/I/D)(E/D/Q)(K/N/S)(Y/L/H)(M/V/Y)(G/T)
Motif 5 (SEQ ID NO: 122):
   S(V/I/P)(M/I)(H/N/S)(H/F)(V/I)
Motif 6 (SEQ ID NO: 123):
   (H/Q/L)G(M/L/T)(I/E/K)D(E/K/L)(N/V/R)V(H/T/P)(F/P/I)
Motif 7 (SEQ ID NO: 124):
   (F/Y)(P/E)(D/G/N)(E/D)(R/Q/N)H(M/G/P)(P/F/L)(R/D)(G/R/K).

Further preferably, the POP polypeptide also comprises one or more of the following motifs:
Motif 8 (SEQ ID NO: 125):
   KLRRERLR(Q/E)RGLGVT(C/R)YEW
Motif 9 (SEQ ID NO: 126):
   HG(L/I)AEYIAQEEM(D/E)R(K/R)(N/T/M)G(Y/F)WWS(L/P)DS
Motif 10 (SEQ ID NO: 127):
   GFIWASE(K/R)(S/T)GFRHL
Motif 11 (SEQ ID NO: 128):
   LR(S/N)(Q/K/R)GILVWK(L/M)D
Motif 12 (SEQ ID NO: 129):
   IG(L/V/I)(C/Y)GWSYGG(Y/F)
Motif 13 (SEQ ID NO: 130):
   CAV(S/A)GAPVT(S/A)WDGYD
Motif 14 (SEQ ID NO: 131):
   HGMIDENVHFRHTARL

More preferably, the POP polypeptide comprises in increasing order of preference, at least 2, at least 3, at least 4, at least 5, at least 6, at least 7, at least 8, at least 9, at least 10, at least 11, or all 12 motifs.

Alternatively, the homologue of a POP protein has in increasing order of preference at least 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% overall sequence identity to the amino acid represented by SEQ ID NO: 117, provided that the homologous protein comprises at least one of the conserved motifs as outlined above. The overall sequence identity is determined using a global alignment algorithm, such as the Needleman Wunsch algorithm in the program GAP (GCG Wisconsin Package, Accelrys), preferably with default parameters and preferably with sequences of mature proteins (i.e. without taking into account secretion signals or transit peptides). Compared to overall sequence identity, the sequence identity will generally be higher when only conserved domains or motifs are considered. Preferably the motifs in a POP polypeptide have, in increasing order of preference, at least 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% sequence identity to the motifs represented by SEQ ID NO: 118 to SEQ ID NO: 129 (Motifs 1 to 12).

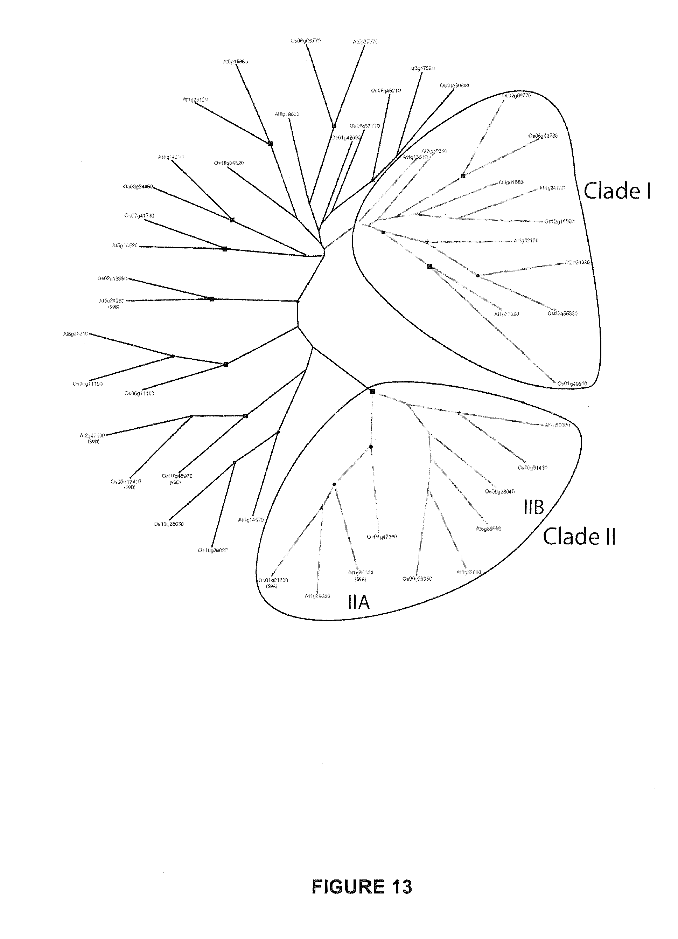

Preferably, the polypeptide sequence which when used in the construction of a phylogenetic tree, such as the one depicted in Figure 13 (Tripathi & Sowdhamini 2006), clusters with the group of POP polypeptides indicated by the arrow comprising the amino acid sequence represented by SEQ ID NO: 117 rather than with any other group.

A "CRL polypeptide" as defined herein refers to any polypeptide comprising a protein domain having in increasing order of preference at least 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% overall sequence identity to the amino acid domain represented by the sequence of amino acids 42-236 of SEQ ID NO: 156 and optionally having a transmembrane domain, preferably as represented by the sequence of amino acids 19-36 of SEQ ID NO: SEQ ID NO: 156.

Methods to determine the presence of a transmembrane domain are in a protein are well know in the art (Further details are provided in the Example section). A protein having a transmembrane domain is expected to be localized to a membranous structure. Preferably the protein of the invention, when present in a cell localizes to a membrane.

A preferred protein of the invention refers to a CRL polypeptide having in increasing order of preference at least 50%, 51%, 52%, 53%, 54%, 55%, 56%, 57%, 58%, 59%, 60%, 61 %, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% sequence identity to any one or more of the following motifs:
(i) Motif 1: EQAFWRxPXKPFRQR (SEQ ID NO: 207);
(ii) Motif 2: NFCDR (SEQ ID NO: 208);
(iii) Motif 3: RGKRCLYEGS (SEQ ID NO: 209);
(iv) Motif 4: QVWGxKXGPYEFK (SEQ ID NO: 210);
wherein X represents any amino acid.

Alternatively, the homologue of a CRL protein has in increasing order of preference at least 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% overall sequence identity to any of the amino acid set forth in Table A7, preferably to the sequence represented by SEQ ID NO: 156. The overall sequence identity is determined using a global alignment algorithm, such as the Needleman Wunsch algorithm in the program GAP (GCG Wisconsin Package, Accelrys), preferably with default parameters and preferably with sequences of mature proteins (i.e. without taking into account secretion signals or transit peptides). Compared to overall sequence identity, the sequence identity will generally be higher when only conserved domains or motifs are considered. For local alignments, the Smith-Waterman algorithm is particularly useful (Smith TF, Waterman MS (1981) J. Mol. Biol 147(1);195-7). An alternative method to determine sequence identity is provided by the method known as MATGAT (Examples section).

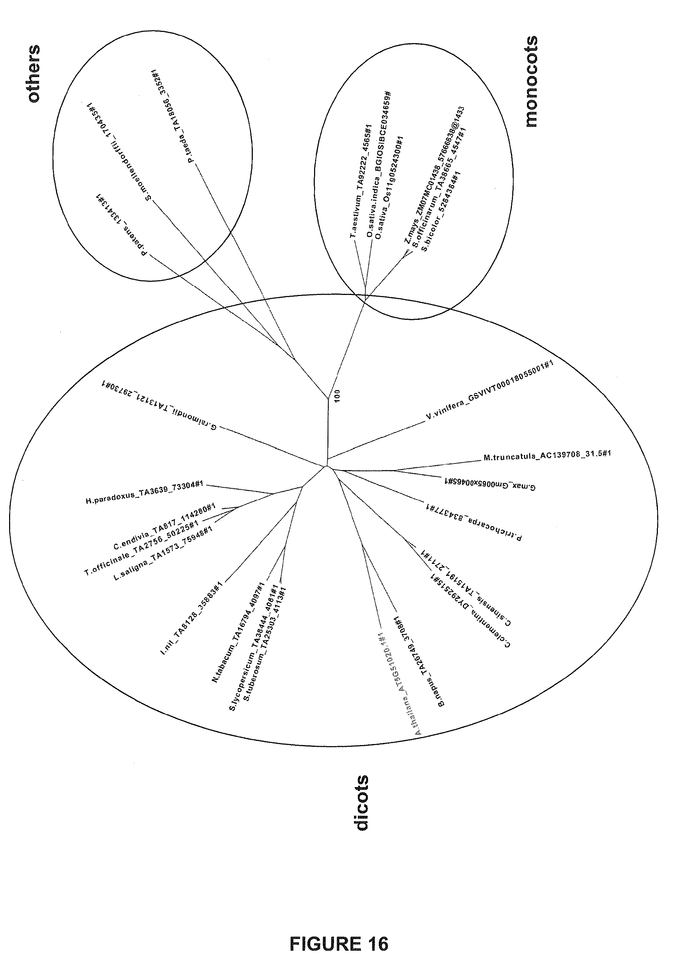

Preferably, the polypeptide sequence which when used in the construction of a phylogenetic tree, such as the one depicted in Figure 16, clusters with the group of CRL polypeptides comprising the amino acid sequence encoded by a monocotyledonous plant, more preferably by a sequence encoded by a dicotyledonous plant, most preferably by the sequence represented by SEQ ID NO: 156 rather than with any other group.

The terms "domain", "signature" and "motif" are defined in the "definitions" section herein. Specialist databases exist for the identification of domains, for example, SMART (Schultz et al. (1998) Proc. Natl. Acad. Sci. USA 95, 5857-5864; Letunic et al. (2002) Nucleic Acids Res 30, 242-244), InterPro (Mulder et al., (2003) Nucl. Acids. Res. 31, 315-318), Prosite (Bucher and Bairoch (1994), A generalized profile syntax for biomolecular sequences motifs and its function in automatic sequence interpretation. (In) ISMB-94; Proceedings 2nd International Conference on Intelligent Systems for Molecular Biology. Altman R., Brutlag D., Karp P., Lathrop R., Searls D., Eds., pp53-61, AAAI Press, Menlo Park; Hulo et al., Nucl. Acids. Res. 32:D134-D137, (2004)), or Pfam (Bateman et al., Nucleic Acids Research 30(1): 276-280 (2002)). A set of tools for in silico analysis of protein sequences is available on the ExPASy proteomics server (Swiss Institute of Bioinformatics (Gasteiger et al., ExPASy: the proteomics server for in-depth protein knowledge and analysis, Nucleic Acids Res. 31:3784-3788(2003)). Domains or motifs may also be identified using routine techniques, such as by sequence alignment.

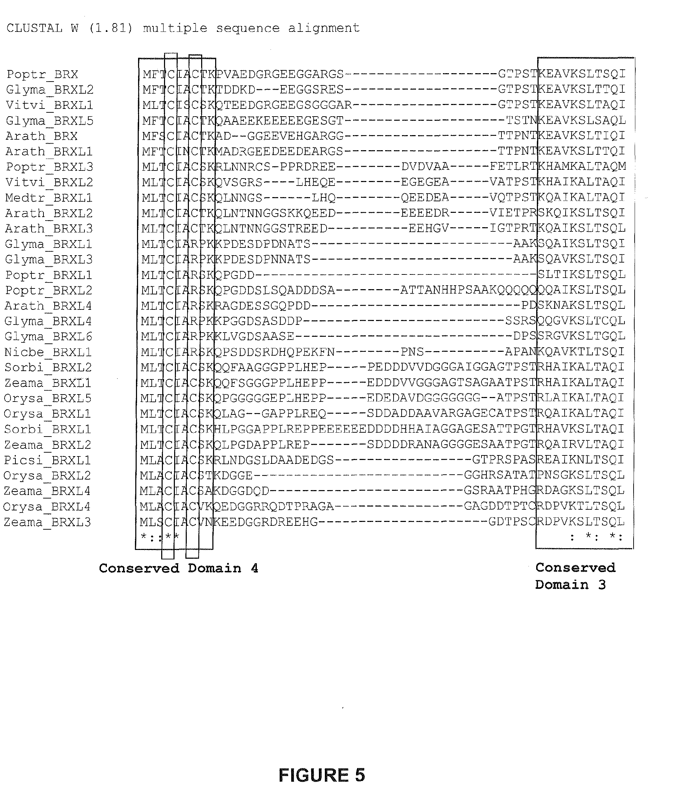

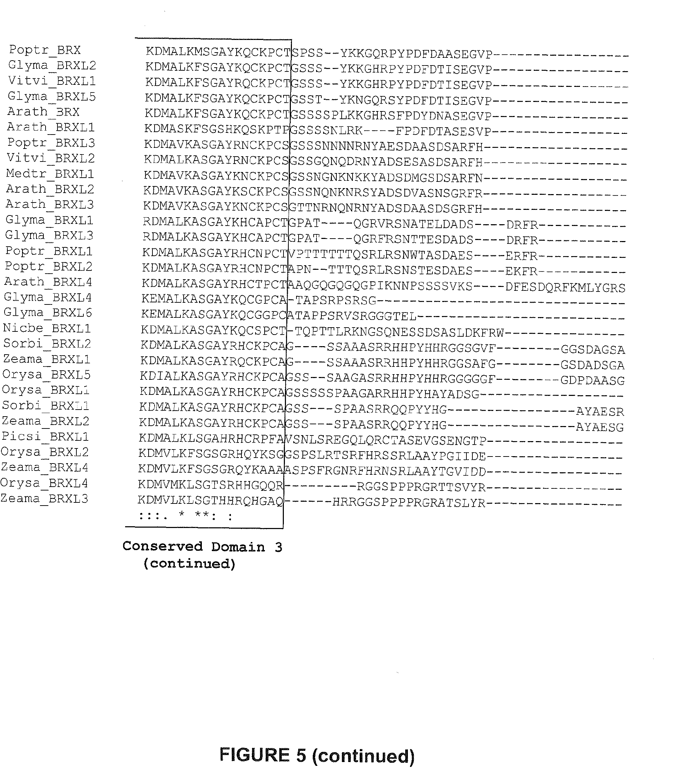

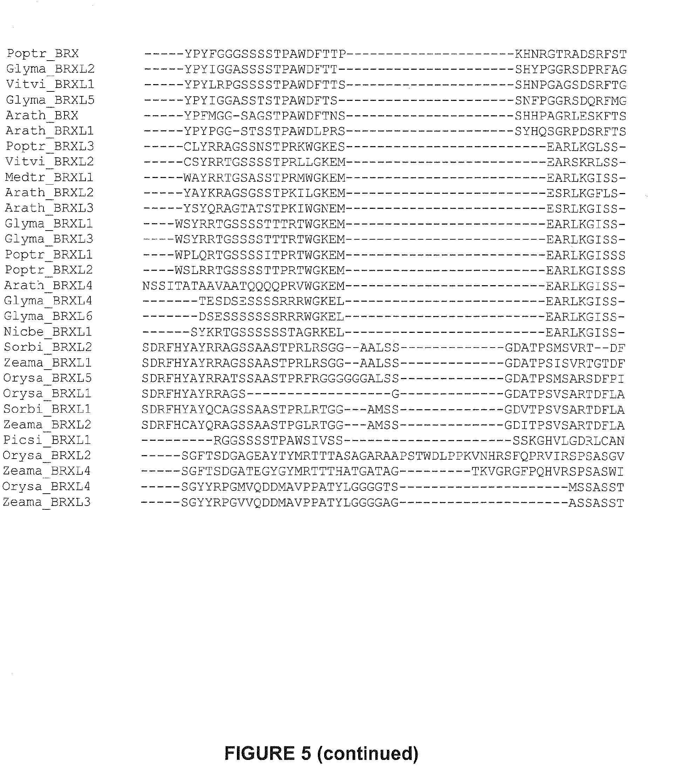

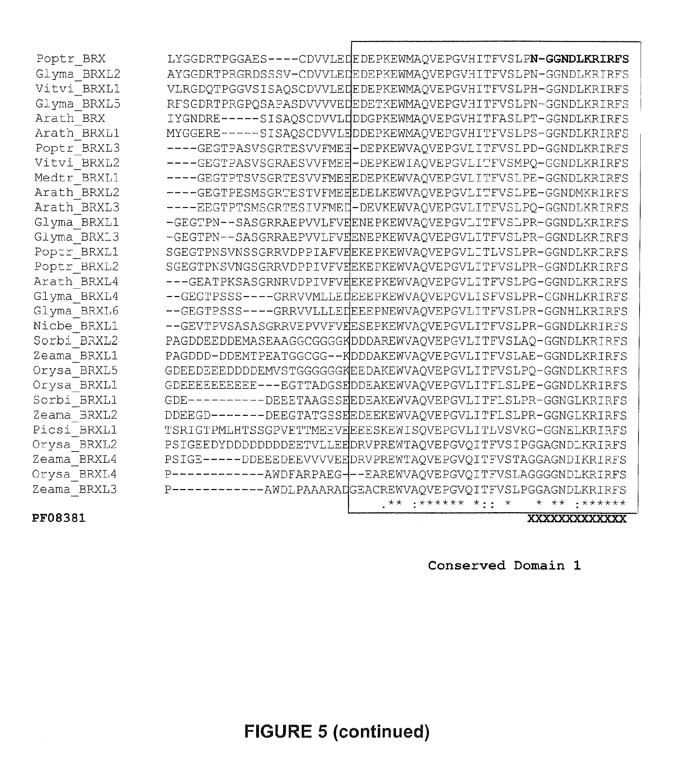

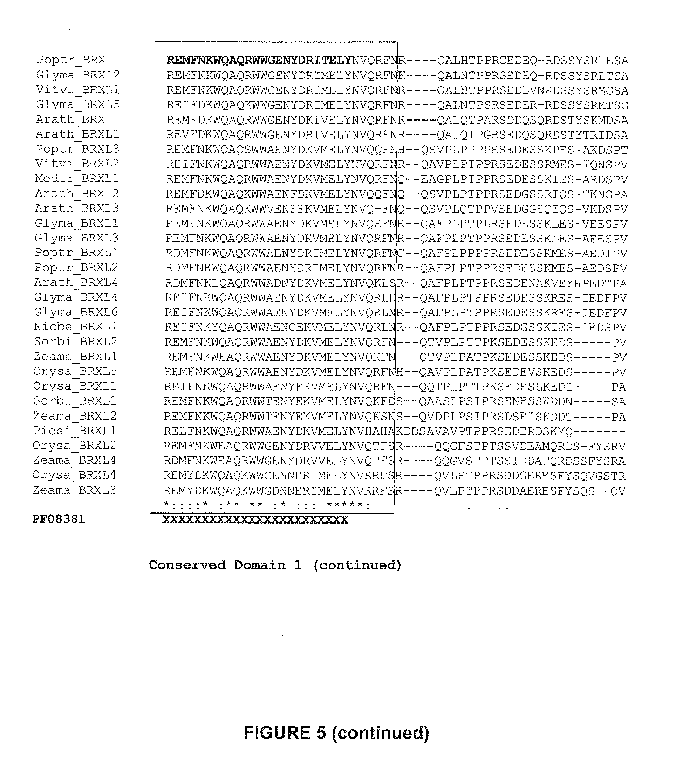

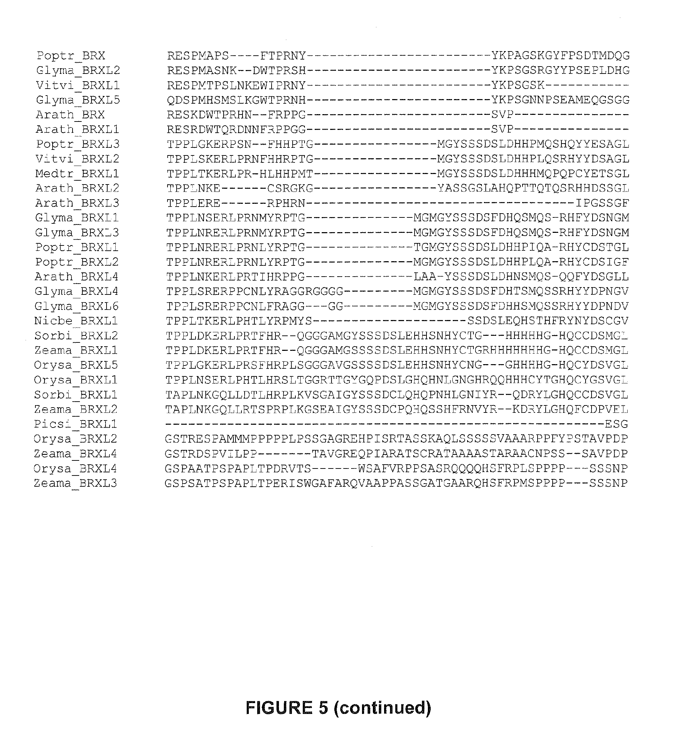

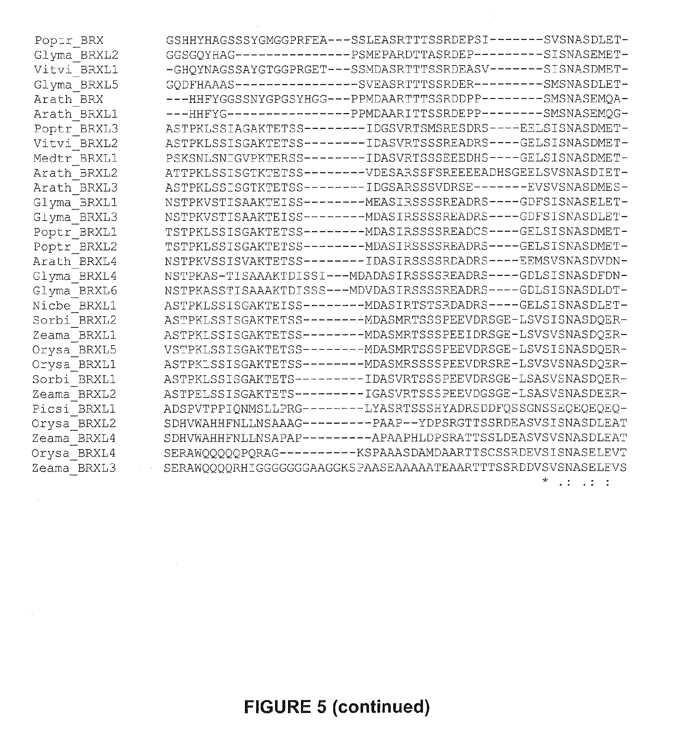

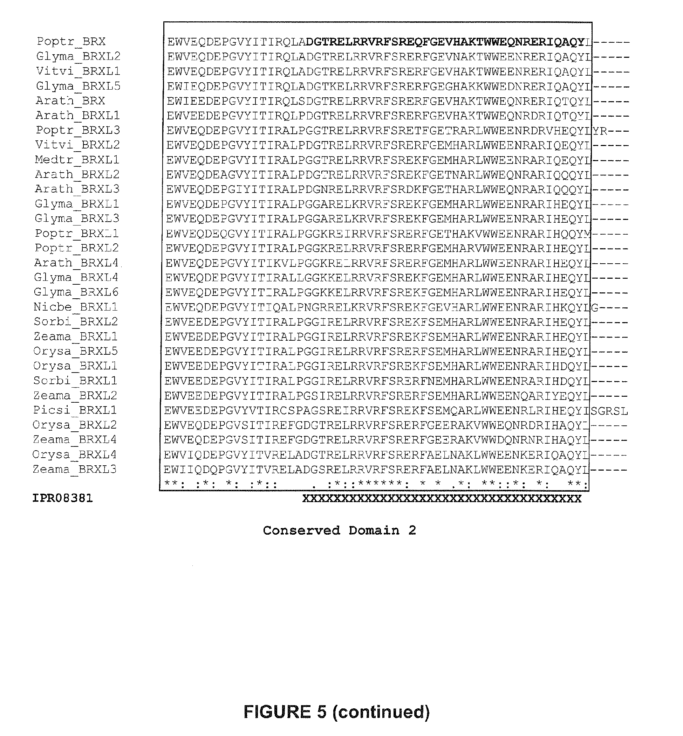

Concerning BRXL polypeptides, an alignment of the polypeptides of Table A3 herein, is shown in Figure 5. Such alignments are useful for identifying the most conserved domains or motifs between the BRXL polypeptides as defined herein. Four such domains are (i) a Conserved Domain 1 representing a BRX domain, which comprises IPR013591 DZC domain (PFAM entry PF08381 DZC; marked by X's in Figure 5); (2) a Conserved Domain 2 representing a BRX domain, which comprises a C-terminal IPR013591 DZC domain (PFAM entry PF08381 DZC; marked by X's in Figure 5); (3) a Conserved Domain 3 and (4) a Conserved Domain 4, both containing conserved Cys's, whose spacing is indicative of a potential zinc-binding motif. All four Conserved Domains are boxed in Figure 5.

Methods for the alignment of sequences for comparison are well known in the art, such methods include GAP, BESTFIT, BLAST, FASTA and TFASTA. GAP uses the algorithm of Needleman and Wunsch ((1970) J Mol Biol 48: 443-453) to find the global (i.e. spanning the complete sequences) alignment of two sequences that maximizes the number of matches and minimizes the number of gaps. The BLAST algorithm (Altschul et al. (1990) J Mol Biol 215: 403-10) calculates percent sequence identity and performs a statistical analysis of the similarity between the two sequences. The software for performing BLAST analysis is publicly available through the National Centre for Biotechnology Information (NCBI). Homologues may readily be identified using, for example, the ClustalW multiple sequence alignment algorithm (version 1.83), with the default pairwise alignment parameters, and a scoring method in percentage. Global percentages of similarity and identity may also be determined using one of the methods available in the MatGAT software package (Campanella et al., BMC Bioinformatics. 2003 Jul 10;4:29. MatGAT: an application that generates similarity/identity matrices using protein or DNA sequences.). Minor manual editing may be performed to optimise alignment between conserved motifs, as would be apparent to a person skilled in the art. Furthermore, instead of using full-length sequences for the identification of homologues, specific domains may also be used. The sequence identity values may be determined over the entire nucleic acid or amino acid sequence or over selected domains or conserved motif(s), using the programs mentioned above using the default parameters. For local alignments, the Smith-Waterman algorithm is particularly useful (Smith TF, Waterman MS (1981) J. Mol. Biol 147(1);195-7).

Concerning BRXL polypeptides, the Examples Section herein describes in Table B1 the percentage identity between the BRXL polypeptide as represented by SEQ ID NO: 18 and the BRXL polypeptides listed in Table A3, which can be as low as 43% amino acid sequence identity. In some instances, the default parameters may be adjusted to modify the stringency of the search. For example using BLAST, the statistical significance threshold (called "expect" value) for reporting matches against database sequences may be increased to show less stringent matches. This way, short nearly exact matches may be identified.

The task of protein subcellular localisation prediction is important and well studied. Knowing a protein's localisation helps elucidate its function. Experimental methods for protein localization range from immunolocalization to tagging of proteins using green fluorescent protein (GFP) or beta-glucuronidase (GUS). Such methods are accurate although labor-intensive compared with computational methods. Recently much progress has been made in computational prediction of protein localisation from sequence data. Among algorithms well known to a person skilled in the art are available at the ExPASy Proteomics tools hosted by the Swiss Institute for Bioinformatics, for example, PSort, TargetP, ChloroP, LocTree, Predotar, LipoP, MITOPROT, PATS, PTS1, SignalP, TMHMM, and others.

Concerning BRXL polypeptides, according to TargetP, the predicted subcellular localisation of a BRXL polypeptide as represented by SEQ ID NO: 18 is the nucleus (see the Example Section).

Alfin-like polypeptides, when expressed in plants, in particular in rice plants, confer enhanced tolerance to abiotic stresses to those plants.

Furthermore, YRP polypeptides, when expressed in plants, in particular in rice plants, confer enhanced tolerance to abiotic stresses to those plants.

Furthermore, silky-1-like polypeptides, when expressed in plants, in particular in rice plants, confer enhanced tolerance to abiotic stresses to those plants.

Furthermore, tools and techniques for measuring activity of ARP6 polypeptides are well known in the art as for example described in the literature references included herein. In addition, ARP6 polypeptides, when expressed in rice according to the methods of the present invention as outlined in the Examples section give plants having increased yield related traits, in particular in any one more of seed weight, harvest index, seed fill rate and the number of filled seeds per plant.

Furthermore, POP polypeptides (at least in their native form) typically have endopeptidase activity, cleaving after Proline residues, and to a lesser extent after Ala residues. Tools and techniques for measuring Prolyl endopeptidase activity are well known in the art, see for example Nomura (FEBS Letters 209, 235-237, 1986). Further details are provided in the Example Section. In addition, POP polypeptides, when expressed in rice according to the methods of the present invention as outlined in the Examples Section, give plants having increased yield related traits, in particular increased biomass and/or increased seed yield.

Furthermore, CRL polypeptides typically have the activity of modulating photosynthesis when present in a plant. Tools and techniques for measuring modulation of photosynthesis are well known in the art, for example by determination of the chlorophyll content or of the chlorophyll fluorescence (Asano et al 2004, The Plant Journal, Volume 38, pp. 448-459(12). In addition, CRL polypeptides, when expressed in rice according to the methods of the present invention as outlined in the Examples Section, give plants having increased yield related traits, in particular in any or more selected from the total seed yield (Totalwgseeds), number of filled seeds (nrfilledseed), fill rate (fillrate), and harvest index (harvestindex).

Concerning alfin-like polypeptides, the present invention may be performed, for example, by transforming plants with the nucleic acid sequence represented by any of SEQ ID NO: 1 encoding the polypeptide sequence of SEQ ID NO: 2, or SEQ ID NO: 3 encoding the polypeptide sequence of SEQ ID NO: 4. However, performance of the invention is not restricted to these sequences; the methods of the invention may advantageously be performed using any alfin-like-encoding nucleic acid or alfin-like polypeptide as defined herein.

Examples of nucleic acids encoding alfin-like polypeptides are given in Table A1 of the Examples section herein. Such nucleic acids are useful in performing the methods of the invention. Orthologues and paralogues of the amino acid sequences given in Table A1 may be readily obtained using routine tools and techniques, such as a reciprocal blast search. Typically, this involves a first BLAST involving BLASTing a query sequence (for example using any of the sequences listed in Table A1 of the Examples section) against any sequence database, such as the publicly available NCBI database. BLASTN or TBLASTX (using standard default values) are generally used when starting from a nucleotide sequence, and BLASTP or TBLASTN (using standard default values) when starting from a protein sequence. The BLAST results may optionally be filtered. The full-length sequences of either the filtered results or non-filtered results are then BLASTed back (second BLAST) against sequences from the organism from which the query sequence is derived (where the query sequence is SEQ ID NO: 1 or SEQ ID NO: 2, the second BLAST would therefore be against Solanum lycopersicum sequences; where the query sequence is SEQ ID NO: 3 or SEQ ID NO: 4, the second BLAST would therefore be against Populus trichocarpa sequences). The results of the first and second BLASTs are then compared. A paralogue is identified if a high-ranking hit from the first blast is from the same species as from which the query sequence is derived, a BLAST back then ideally results in the query sequence amongst the highest hits; an orthologue is identified if a high-ranking hit in the first BLAST is not from the same species as from which the query sequence is derived, and preferably results upon BLAST back in the query sequence being among the highest hits.

Concerning YRP polypeptides, the present invention may be performed, for example, by transforming plants with the nucleic acid sequence represented by any of SEQ ID NO: 10 encoding the polypeptide sequence of SEQ ID NO: 11, or SEQ ID NO: 12 encoding the polypeptide sequence of SEQ ID NO: 13. However, performance of the invention is not restricted to these sequences; the methods of the invention may advantageously be performed using any YRP-encoding nucleic acid or YRP polypeptide as defined herein.

Examples of nucleic acids encoding YRP polypeptides are given in Table A2 of the Examples section herein. Such nucleic acids are useful in performing the methods of the invention. Orthologues and paralogues of the amino acid sequences given in Table A2 may be readily obtained using routine tools and techniques, such as a reciprocal blast search. Typically, this involves a first BLAST involving BLASTing a query sequence (for example using any of the sequences listed in Table A2 of the Examples section) against any sequence database, such as the publicly available NCBI database. BLASTN or TBLASTX (using standard default values) are generally used when starting from a nucleotide sequence, and BLASTP or TBLASTN (using standard default values) when starting from a protein sequence. The BLAST results may optionally be filtered. The full-length sequences of either the filtered results or non-filtered results are then BLASTed back (second BLAST) against sequences from the organism from which the query sequence is derived (where the query sequence is SEQ ID NO: 10 or SEQ ID NO: 11, the second BLAST would therefore be against Hordeum vulgare sequences; where the query sequence is SEQ ID NO: 12 or SEQ ID NO: 13, the second BLAST would therefore be against Hordeum vulgare sequences). The results of the first and second BLASTs are then compared. A paralogue is identified if a high-ranking hit from the first blast is from the same species as from which the query sequence is derived, a BLAST back then ideally results in the query sequence amongst the highest hits; an orthologue is identified if a high-ranking hit in the first BLAST is not from the same species as from which the query sequence is derived, and preferably results upon BLAST back in the query sequence being among the highest hits.

Concerning BRXL polypeptides, the present invention is illustrated by transforming plants with the nucleic acid sequence represented by SEQ ID NO: 17, encoding the BRXL polypeptide sequence of SEQ ID NO: 18. However, performance of the invention is not restricted to these sequences; the methods of the invention may advantageously be performed using any nucleic acid sequence encoding a BRXL polypeptide as defined herein.

Examples of nucleic acid sequences encoding BRXL polypeptides are given in Table A3 of Example 1 herein. Such nucleic acid sequences are useful in performing the methods of the invention. The polypeptide sequences given in Table A3 of Example 1 are example sequences of orthologues and paralogues of the BRXL polypeptide represented by SEQ ID NO: 18, the terms "orthologues" and "paralogues" being as defined herein. Further orthologues and paralogues may readily be identified by performing a so-called reciprocal blast search. Typically, this involves a first BLAST involving BLASTing a query sequence (for example using any of the sequences listed in Table A3 of Example 1) against any sequence database, such as the publicly available NCBI database. BLASTN or TBLASTX (using standard default values) are generally used when starting from a nucleotide sequence, and BLASTP or TBLASTN (using standard default values) when starting from a protein sequence. The BLAST results may optionally be filtered. The full-length sequences of either the filtered results or non-filtered results are then BLASTed back (second BLAST) against sequences from the organism from which the query sequence is derived (where the query sequence is SEQ ID NO: 17 or SEQ ID NO: 18, the second BLAST would therefore be against poplar sequences). The results of the first and second BLASTs are then compared. A paralogue is identified if a high-ranking hit from the first blast is from the same species as from which the query sequence is derived, a BLAST back then ideally results in the query sequence amongst the highest hits; an orthologue is identified if a high-ranking hit in the first BLAST is not from the same species as from which the query sequence is derived, and preferably results upon BLAST back in the query sequence being among the highest hits.

Concerning silky-1-like polypeptides, the present invention may be performed, for example, by transforming plants with the nucleic acid sequence represented by any of SEQ ID NO: 90 encoding the polypeptide sequence of SEQ ID NO: 91, SEQ ID NO: 92 encoding the polypeptide sequence of SEQ ID NO: 93, or SEQ ID NO: 94 encoding the polypeptide sequence of SEQ ID NO: 95. However, performance of the invention is not restricted to these sequences; the methods of the invention may advantageously be performed using any silky-1-like-encoding nucleic acid or silky-1-like polypeptide as defined herein.

Examples of nucleic acids encoding silky-1-like polypeptides are given in Table A4 of the Examples section herein. Such nucleic acids are useful in performing the methods of the invention. Orthologues and paralogues of the amino acid sequences given in Table A4 may be readily obtained using routine tools and techniques, such as a reciprocal blast search. Typically, this involves a first BLAST involving BLASTing a query sequence (for example using any of the sequences listed in Table A4 of the Examples section) against any sequence database, such as the publicly available NCBI database. BLASTN or TBLASTX (using standard default values) are generally used when starting from a nucleotide sequence, and BLASTP or TBLASTN (using standard default values) when starting from a protein sequence. The BLAST results may optionally be filtered. The full-length sequences of either the filtered results or non-filtered results are then BLASTed back (second BLAST) against sequences from the organism from which the query sequence is derived (where the query sequence is SEQ ID NO: 90 or SEQ ID NO: 91, the second BLAST would therefore be against Populus trichocarpa sequences; where the query sequence is SEQ ID NO: 92 or SEQ ID NO: 93, the second BLAST would therefore be against Solanum lycopersicum sequences; where the query sequence is SEQ ID NO: 94 or SEQ ID NO: 95, the second BLAST would therefore be against Triticum aestivum sequences). The results of the first and second BLASTs are then compared. A paralogue is identified if a high-ranking hit from the first blast is from the same species as from which the query sequence is derived, a BLAST back then ideally results in the query sequence amongst the highest hits; an orthologue is identified if a high-ranking hit in the first BLAST is not from the same species as from which the query sequence is derived, and preferably results upon BLAST back in the query sequence being among the highest hits.

Concerning ARP6 polypeptides, the present invention is illustrated by transforming plants with the nucleic acid sequence represented by SEQ ID NO: 101, encoding the polypeptide sequence of SEQ ID NO: 102. However, performance of the invention is not restricted to these sequences; the methods of the invention may advantageously be performed using any ARTP6-encoding nucleic acid or ARP6 polypeptide as defined herein.

Examples of nucleic acids encoding ARP6 polypeptides are given in Table A5 of the Examples section herein. Such nucleic acids are useful in performing the methods of the invention. The amino acid sequences given in Table A5 of the Examples section are example sequences of orthologues and paralogues of the ARP6 polypeptide represented by SEQ ID NO: 102, the terms "orthologues" and "paralogues" being as defined herein. Further orthologues and paralogues may readily be identified by performing a so-called reciprocal blast search. Typically, this involves a first BLAST involving BLASTing a query sequence (for example using any of the sequences listed in Table A5 of the Examples section) against any sequence database, such as the publicly available NCBI database. BLASTN or TBLASTX (using standard default values) are generally used when starting from a nucleotide sequence, and BLASTP or TBLASTN (using standard default values) when starting from a protein sequence. The BLAST results may optionally be filtered. The full-length sequences of either the filtered results or non-filtered results are then BLASTed back (second BLAST) against sequences from the organism from which the query sequence is derived (where the query sequence is SEQ ID NO: 101 or SEQ ID NO: 102, the second BLAST would therefore be against Arabidopsis thaliana sequences). The results of the first and second BLASTs are then compared. A paralogue is identified if a high-ranking hit from the first blast is from the same species as from which the query sequence is derived, a BLAST back then ideally results in the query sequence amongst the highest hits; an orthologue is identified if a high-ranking hit in the first BLAST is not from the same species as from which the query sequence is derived, and preferably results upon BLAST back in the query sequence being among the highest hits.

Concerning POP polypeptides, the present invention is illustrated by transforming plants with the nucleic acid sequence represented by SEQ ID NO: 116, encoding the polypeptide sequence of SEQ ID NO: 117. However, performance of the invention is not restricted to these sequences; the methods of the invention may advantageously be performed using any POP-encoding nucleic acid or POP polypeptide as defined herein.

Examples of nucleic acids encoding POP polypeptides are given in Table A6 of the Examples section herein. Such nucleic acids are useful in performing the methods of the invention. The amino acid sequences given in Table A6 of the Examples section are example sequences of orthologues and paralogues of the POP polypeptide represented by SEQ ID NO: 2, the terms "orthologues" and "paralogues" being as defined herein. Further orthologues and paralogues may readily be identified by performing a so-called reciprocal blast search. Typically, this involves a first BLAST involving BLASTing a query sequence (for example using any of the sequences listed in Table A6 of the Examples section) against any sequence database, such as the publicly available NCBI database. BLASTN or TBLASTX (using standard default values) are generally used when starting from a nucleotide sequence, and BLASTP or TBLASTN (using standard default values) when starting from a protein sequence. The BLAST results may optionally be filtered. The full-length sequences of either the filtered results or non-filtered results are then BLASTed back (second BLAST) against sequences from the organism from which the query sequence is derived (where the query sequence is SEQ ID NO: 116 or SEQ ID NO: 117, the second BLAST would therefore be against Arabidopsis sequences). The results of the first and second BLASTs are then compared. A paralogue is identified if a high-ranking hit from the first blast is from the same species as from which the query sequence is derived, a BLAST back then ideally results in the query sequence amongst the highest hits; an orthologue is identified if a high-ranking hit in the first BLAST is not from the same species as from which the query sequence is derived, and preferably results upon BLAST back in the query sequence being among the highest hits.

Concerning CRL polypeptides, the present invention is illustrated by transforming plants with the nucleic acid sequence represented by SEQ ID NO: 155, encoding the polypeptide sequence of SEQ ID NO: 156. However, performance of the invention is not restricted to these sequences; the methods of the invention may advantageously be performed using any CRL-encoding nucleic acid or CRL polypeptide as defined herein.

Examples of nucleic acids encoding CRL polypeptides are given in Table A7 of the Examples section herein. Such nucleic acids are useful in performing the methods of the invention. The amino acid sequences given in Table A7 of the Examples section are example sequences of orthologues and paralogues of the CRL polypeptide represented by SEQ ID NO: 2, the terms "orthologues" and "paralogues" being as defined herein. Further orthologues and paralogues may readily be identified by performing a so-called reciprocal blast search. Typically, this involves a first BLAST involving BLASTing a query sequence (for example using any of the sequences listed in Table A7 of the Examples section) against any sequence database, such as the publicly available NCBI database. BLASTN or TBLASTX (using standard default values) are generally used when starting from a nucleotide sequence, and BLASTP or TBLASTN (using standard default values) when starting from a protein sequence. The BLAST results may optionally be filtered. The full-length sequences of either the filtered results or non-filtered results are then BLASTed back (second BLAST) against sequences from the organism from which the query sequence is derived (where the query sequence is SEQ ID NO: 155 or SEQ ID NO: 156, the second BLAST would therefore be against Arabidopsis thaliana sequences). The results of the first and second BLASTs are then compared. A paralogue is identified if a high-ranking hit from the first blast is from the same species as from which the query sequence is derived, a BLAST back then ideally results in the query sequence amongst the highest hits; an orthologue is identified if a high-ranking hit in the first BLAST is not from the same species as from which the query sequence is derived, and preferably results upon BLAST back in the query sequence being among the highest hits.

High-ranking hits are those having a low E-value. The lower the E-value, the more significant the score (or in other words the lower the chance that the hit was found by chance). Computation of the E-value is well known in the art. In addition to E-values, comparisons are also scored by percentage identity. Percentage identity refers to the number of identical nucleotides (or amino acids) between the two compared nucleic acid (or polypeptide) sequences over a particular length. In the case of large families, ClustalW may be used, followed by a neighbour joining tree, to help visualize clustering of related genes and to identify orthologues and paralogues.

Nucleic acid variants may also be useful in practising the methods of the invention. Examples of such variants include nucleic acids encoding homologues and derivatives of any one of the amino acid sequences given in Table A1 to A7 of the Examples section, the terms "homologue" and "derivative" being as defined herein. Also useful in the methods of the invention are nucleic acids encoding homologues and derivatives of orthologues or paralogues of any one of the amino acid sequences given in Table A1 to A7 of the Examples section. Homologues and derivatives useful in the methods of the present invention have substantially the same biological and functional activity as the unmodified protein from which they are derived. Further variants useful in practising the methods of the invention are variants in which codon usage is optimised or in which miRNA target sites are removed.

Further nucleic acid variants useful in practising the methods of the invention include portions of nucleic acids encoding alfin-like polypeptides, or YRP polypeptides, or BRXL polypeptides, or silky-1-like polypeptides, or ARP6 polypeptides, or POP polypeptides, or CRL polypeptides, nucleic acids hybridising to nucleic acids encoding alfin-like polypeptides, or YRP polypeptides, or BRXL polypeptides, or silky-1-like polypeptides, or ARP6 polypeptides, or POP polypeptides, or CRL polypeptides, splice variants of nucleic acids encoding alfin-like polypeptides, or YRP polypeptides, or BRXL polypeptides, or silky-1-like polypeptides, or ARP6 polypeptides, or POP polypeptides, or CRL polypeptides, allelic variants of nucleic acids encoding alfin-like polypeptides, or YRP polypeptides, or BRXL polypeptides, or silky-1-like polypeptides, or ARP6 polypeptides, or POP polypeptides, or CRL polypeptides, and variants of nucleic acids encoding alfin-like polypeptides, or YRP polypeptides, or BRXL polypeptides, or silky-1-like polypeptides, or ARP6 polypeptides, or POP polypeptides, or CRL polypeptides, obtained by gene shuffling. The terms hybridising sequence, splice variant, allelic variant and gene shuffling are as described herein.

Nucleic acids encoding alfin-like polypeptides, or YRP polypeptides, or BRXL polypeptides, or silky-1-like polypeptides, or ARP6 polypeptides, or POP polypeptides, or CRL polypeptides, need not be full-length nucleic acids, since performance of the methods of the invention does not rely on the use of full-length nucleic acid sequences. According to the present invention, there is provided a method for enhancing abiotic stress tolerance in plants, comprising introducing and expressing in a plant a portion of any one of the nucleic acid sequences given in Table A1 to A7 of the Examples section, or a portion of a nucleic acid encoding an orthologue, paralogue or homologue of any of the amino acid sequences given in Table A1 to A7 of the Examples section.

A portion of a nucleic acid may be prepared, for example, by making one or more deletions to the nucleic acid. The portions may be used in isolated form or they may be fused to other coding (or non-coding) sequences in order to, for example, produce a protein that combines several activities. When fused to other coding sequences, the resultant polypeptide produced upon translation may be bigger than that predicted for the protein portion.

Concerning alfin-like polypeptides, portions useful in the methods of the invention, encode an alfin-like polypeptide as defined herein, and have substantially the same biological activity as the amino acid sequences given in Table A1 of the Examples section. Preferably, the portion is a portion of any one of the nucleic acids given in Table A1 of the Examples section, or is a portion of a nucleic acid encoding an orthologue or paralogue of any one of the amino acid sequences given in Table A1 of the Examples section. Preferably the portion is at least 650, 700, 750, 800, 850, 900, 950, 1000, 1050, 1100, 1150, 1200, 1250 or more consecutive nucleotides in length, the consecutive nucleotides being of any one of the nucleic acid sequences given in Table A1 of the Examples section, or of a nucleic acid encoding an orthologue or paralogue of any one of the amino acid sequences given in Table A1 of the Examples section. Most preferably the portion is a portion of the nucleic acid of SEQ ID NO: 1 or SEQ ID NO: 3. Preferably, the portion encodes a fragment of an amino acid sequence which, when used in the construction of a phylogenetic tree, clusters with the group of alfin-like polypeptides comprising the amino acid sequence represented by SEQ ID NO: 2 or SEQ ID NO: 4, rather than with any other group.

Concerning YRP polypeptides, portions useful in the methods of the invention, encode a YRP polypeptide as defined herein, and have substantially the same biological activity as the amino acid sequences given in Table A2 of the Examples section. Preferably, the portion is a portion of any one of the nucleic acids given in Table A2 of the Examples section, or is a portion of a nucleic acid encoding an orthologue or paralogue of any one of the amino acid sequences given in Table A2 of the Examples section. Preferably the portion is at least 700, 750, 800, 850, 900, 950, 1000, 1050, 1100, 1150, 1200, 1250, 1300, 1350, 1350, 1400, or more consecutive nucleotides in length, the consecutive nucleotides being of any one of the nucleic acid sequences given in Table A2 of the Examples Section, or of a nucleic acid encoding an orthologue or paralogue of any one of the amino acid sequences given in Table A2 of the Examples section. Most preferably the portion is a portion of the nucleic acid of SEQ ID NO: 10 or SEQ ID NO: 12. Preferably, the portion encodes a fragment of an amino acid sequence which, when used in the construction of a phylogenetic tree, clusters with the group of YRP polypeptides comprising the amino acid sequence represented by SEQ ID NO: 11 or SEQ ID NO: 13, rather than with any other group.

Concerning BRXL polypeptides, portions useful in the methods of the invention, encode a BRXL polypeptide as defined herein, and have substantially the same biological activity as the polypeptide sequences given in Table A3 of Example 1. Preferably, the portion is a portion of any one of the nucleic acid sequences given in Table A3 of Example 1, or is a portion of a nucleic acid sequence encoding an orthologue or paralogue of any one of the polypeptide sequences given in Table A3 of Example 1. Preferably the portion is, in increasing order of preference at least 600, 650, 700, 750, 800, 850, 900, 950, 1000, 1050, 1060, 1070, 1080 or more consecutive nucleotides in length, the consecutive nucleotides being of any one of the nucleic acid sequences given in Table A3 of Example 1, or of a nucleic acid sequence encoding an orthologue or paralogue of any one of the polypeptide sequences given in Table A3 of Example 1. Preferably, the portion is a portion of a nucleic sequence encoding a polypeptide sequence comprising (i) in increasing order of preference at least 50%, 55%, 60%, 65%, 70%, 75%, 80%, 85%, 90%, 95%, 98%, 99% or more amino acid sequence identity to a Conserved Domain 1 (comprising a BRX domain) as represented by SEQ ID NO: 83; and (ii) in increasing order of preference at least 50%, 55%, 60%, 65%, 70%, 75%, 80%, 85%, 90%, 95%, 98%, 99% or more amino acid sequence identity to a Conserved Domain 2 (comprising a BRX domain) as represented by SEQ ID NO: 84. More preferably, the portion is a portion of a nucleic sequence encoding a polypeptide sequence having in increasing order of preference at least 50%, 55%, 60%, 65%, 70%, 75%, 80%, 85%, 90%, 95%, 98%, 99% or more amino acid sequence identity to the BRXL polypeptide as represented by SEQ ID NO: 18 or to any of the polypeptide sequences given in Table A3 herein. Most preferably, the portion is a portion of the nucleic acid sequence of SEQ ID NO: 17.

Concerning silky-1-like polypeptides, portions useful in the methods of the invention, encode a silky-1-like polypeptide as defined herein, and have substantially the same biological activity as the amino acid sequences given in Table A4 of the Examples section. Preferably, the portion is a portion of any one of the nucleic acids given in Table A4 of the Examples section, or is a portion of a nucleic acid encoding an orthologue or paralogue of any one of the amino acid sequences given in Table A4 of the Examples section. Preferably the portion is at least 550, 600, 650, 700, 750, 800, 850, 900, 950, 1000, 1050, 1100 or more consecutive nucleotides in length, the consecutive nucleotides being of any one of the nucleic acid sequences given in Table A4 of the Examples section, or of a nucleic acid encoding an orthologue or paralogue of any one of the amino acid sequences given in Table A4 of the Examples section. Most preferably the portion is a portion of the nucleic acid of SEQ ID NO: 90, SEQ ID NO: 92 or SEQ ID NO: 94. Preferably, the portion encodes a fragment of an amino acid sequence which, when used in the construction of a phylogenetic tree, clusters with the group of silky-1-like polypeptides comprising the amino acid sequence represented by SEQ ID NO: 91, SEQ ID NO: 93 or SEQ ID NO: 95, rather than with any other group.

Concerning ARP6 polypeptides, portions useful in the methods of the invention, encode an ARP6 polypeptide as defined herein, and have substantially the same biological activity as the amino acid sequences given in Table A5 of the Examples section. Preferably, the portion is a portion of any one of the nucleic acids given in Table A5 of the Examples section, or is a portion of a nucleic acid encoding an orthologue or paralogue of any one of the amino acid sequences given in Table A5 of the Examples section. Preferably the portion is at least 100, 200, 300, 400, 500, 550, 600, 650, 700, 750, 800, 850, 900, 950, 1000 consecutive nucleotides in length, the consecutive nucleotides being of any one of the nucleic acid sequences given in Table A5 of the Examples section, or of a nucleic acid encoding an orthologue or paralogue of any one of the amino acid sequences given in Table A5 of the Examples section. Most preferably the portion is a portion of the nucleic acid of SEQ ID NO: 101. Preferably, the portion encodes a fragment of an amino acid sequence which, when used in the construction of a phylogenetic tree, such as the one depicted in Figure 1 of Kandasamy et al. 2004. Trends Plant Sci 9: 196-202, clusters with the ARP6 group of polypeptides comprising the amino acid sequence of AtARP6 as represented by SEQ ID NO: 102 rather than with any other group.

Concerning POP polypeptides, portions useful in the methods of the invention, encode a POP polypeptide as defined herein, and have substantially the same biological activity as the amino acid sequences given in Table A6 of the Examples section. Preferably, the portion is a portion of any one of the nucleic acids given in Table A6 of the Examples section, or is a portion of a nucleic acid encoding an orthologue or paralogue of any one of the amino acid sequences given in Table A6 of the Examples section. Preferably the portion is at least 500, 600, 700, 800, 900, 1000, 1100, 1200, 1300, 1400, 1500, 1600, 1700, 1800, 1900, 2000, 2100, 2200, 2300 consecutive nucleotides in length, the consecutive nucleotides being of any one of the nucleic acid sequences given in Table A6 of the Examples section, or of a nucleic acid encoding an orthologue or paralogue of any one of the amino acid sequences given in Table A6 of the Examples section. Most preferably the portion is a portion of the nucleic acid of SEQ ID NO: 1. Preferably, the portion encodes a fragment of an amino acid sequence which, when used in the construction of a phylogenetic tree, such as the one depicted in Figure 13 (Tripathi & Sowdhamini 2006), clusters with the group of POP polypeptides indicated by the arrow comprising the amino acid sequence represented by SEQ ID NO: 117 rather than with any other group.

Concerning CRL polypeptides, portions useful in the methods of the invention, encode a CRL polypeptide as defined herein, and have substantially the same biological activity as the amino acid sequences given in Table A7 of the Examples section. Preferably, the portion is a portion of any one of the nucleic acids given in Table A7 of the Examples section, or is a portion of a nucleic acid encoding an orthologue or paralogue of any one of the amino acid sequences given in Table A7 of the Examples section. Preferably the portion is at least 100, 200, 300, 400, 500, 550, 600, 650, 700, 750, 800, 850, 900, 950, 1000 consecutive nucleotides in length, the consecutive nucleotides being of any one of the nucleic acid sequences given in Table A7 of the Examples section, or of a nucleic acid encoding an orthologue or paralogue of any one of the amino acid sequences given in Table A7 of the Examples section. Most preferably the portion is a portion of the nucleic acid of SEQ ID NO: 155. Preferably, the portion encodes a fragment of an amino acid sequence which, when used in the construction of a phylogenetic tree, such as the one depicted in Figure 16, clusters with the group of CRL polypeptides comprising the amino acid sequence encoded by a monocotyledonous plant, more preferably by a sequence encoded by a dicotyledonous plant, most preferably by the sequence represented by SEQ ID NO: 156 rather than with any other group.

Another nucleic acid variant useful in the methods of the invention is a nucleic acid capable of hybridising, under reduced stringency conditions, preferably under stringent conditions, with a nucleic acid encoding an alfin-like polypeptide, or a YRP polypeptide, or a BRXL polypeptide, or a silky-1-like polypeptide, or an ARP6 polypeptide, or a POP polypeptide, or a CRL polypeptide, as defined herein, or with a portion as defined herein.

According to the present invention, there is provided a method for enhancing abiotic stress tolerance in plants, comprising introducing and expressing in a plant a nucleic acid capable of hybridizing to any one of the nucleic acids given in Table A1 to A7 of the Examples Section, or comprising introducing and expressing in a plant a nucleic acid capable of hybridising to a nucleic acid encoding an orthologue, paralogue or homologue of any of the nucleic acid sequences given in Table A1 to A7 of the Examples Section.

Concerning alfin-like polypeptides, hybridising sequences useful in the methods of the invention encode an alfin-like polypeptide as defined herein, having substantially the same biological activity as the amino acid sequences given in Table A1 of the Examples section. Preferably, the hybridising sequence is capable of hybridising to the complement of any one of the nucleic acids given in Table A1, or to a portion of any of these sequences, a portion being as defined above, or the hybridising sequence is capable of hybridising to the complement of a nucleic acid encoding an orthologue or paralogue of any one of the amino acid sequences given in Table A1. Most preferably, the hybridising sequence is capable of hybridising to the complement of a nucleic acid as represented by SEQ ID NO: 1 or SEQ ID NO: 3 or to a portion thereof.

Preferably, the hybridising sequence encodes a polypeptide with an amino acid sequence which, when full-length and used in the construction of a phylogenetic tree, clusters with the group of alfin-like polypeptides comprising the amino acid sequence represented by SEQ ID NO: 2 or SEQ ID NO: 4 rather than with any other group.

Concerning YRP polypeptides, hybridising sequences useful in the methods of the invention encode a YRP polypeptide as defined herein, having substantially the same biological activity as the amino acid sequences given in Table A2 of the Examples section. Preferably, the hybridising sequence is capable of hybridising to the complement of any one of the nucleic acids given in Table A2, or to a portion of any of these sequences, a portion being as defined above, or the hybridising sequence is capable of hybridising to the complement of a nucleic acid encoding an orthologue or paralogue of any one of the amino acid sequences given in Table A2. Most preferably, the hybridising sequence is capable of hybridising to the complement of a nucleic acid as represented by SEQ ID NO: 10 or SEQ ID NO: 12 or to a portion thereof.

Preferably, the hybridising sequence encodes a polypeptide with an amino acid sequence which, when full-length and used in the construction of a phylogenetic tree, clusters with the group of YRP polypeptides comprising the amino acid sequence represented by SEQ ID NO: 11 or SEQ ID NO: 13 rather than with any other group.

Concerning BRXL polypeptides, hybridising sequences useful in the methods of the invention encode a BRXL polypeptide as defined herein, and have substantially the same biological activity as the polypeptide sequences given in Table A3 of Example 1. Preferably, the hybridising sequence is capable of hybridising to any one of the nucleic acid sequences given in Table A3 of Example 1, or to a complement thereof, or to a portion of any of these sequences, a portion being as defined above, or wherein the hybridising sequence is capable of hybridising to a nucleic acid sequence encoding an orthologue or paralogue of any one of the polypeptide sequences given in Table A3 of Example 1, or to a complement thereof. Preferably, the hybridising sequence is capable of hybridising to a nucleic acid sequence encoding a polypeptide sequence comprising (i) in increasing order of preference at least 50%, 55%, 60%, 65%, 70%, 75%, 80%, 85%, 90%, 95%, 98%, 99% or more amino acid sequence identity to a Conserved Domain 1 (comprising a BRX domain) as represented by SEQ ID NO: 83; and (ii) in increasing order of preference at least 50%, 55%, 60%, 65%, 70%, 75%, 80%, 85%, 90%, 95%, 98%, 99% or more amino acid sequence identity to a Conserved Domain 2 (comprising a BRX domain) as represented by SEQ ID NO: 84. More preferably, the hybridising sequence is capable of hybridising to a nucleic acid sequence encoding a polypeptide sequence having in increasing order of preference at least 40%, 45%, 50%, 55%, 60%, 65%, 70%, 75%, 80%, 85%, 90%, 95%, 98%, 99% or more amino acid sequence identity to the BRXL polypeptide as represented by SEQ ID NO: 18 or to any of the polypeptide sequences given in Table A3 herein. Most preferably, the hybridising sequence is capable of hybridising to a nucleic acid sequence as represented by SEQ ID NO: 17 or to a portion thereof.

Concerning silky-1-like polypeptides, hybridising sequences useful in the methods of the invention encode a silky-1-like polypeptide as defined herein, having substantially the same biological activity as the amino acid sequences given in Table A4 of the Examples section. Preferably, the hybridising sequence is capable of hybridising to the complement of any one of the nucleic acids given in Table A4, or to a portion of any of these sequences, a portion being as defined above, or the hybridising sequence is capable of hybridising to the complement of a nucleic acid encoding an orthologue or paralogue of any one of the amino acid sequences given in Table A4. Most preferably, the hybridising sequence is capable of hybridising to the complement of a nucleic acid as represented by SEQ ID NO: 90, SEQ ID NO: 92 or SEQ ID NO: 94 or to a portion thereof.

Preferably, the hybridising sequence encodes a polypeptide with an amino acid sequence which, when full-length and used in the construction of a phylogenetic tree, clusters with the group of silky-1-like polypeptides comprising the amino acid sequence represented by SEQ ID NO: 91, SEQ ID NO: 93 or SEQ ID NO: 95 rather than with any other group.

Concerning ARP6 polypeptides, hybridising sequences useful in the methods of the invention encode an ARP6 polypeptide as defined herein, having substantially the same biological activity as the amino acid sequences given in Table A5 of the Examples section. Preferably, the hybridising sequence is capable of hybridising to the complement of any one of the nucleic acids given in Table A5 of the Examples section, or to a portion of any of these sequences, a portion being as defined above, or the hybridising sequence is capable of hybridising to the complement of a nucleic acid encoding an orthologue or paralogue of any one of the amino acid sequences given in Table A5 of the Examples section. Most preferably, the hybridising sequence is capable of hybridising to the complement of a nucleic acid as represented by SEQ ID NO: 101 or to a portion thereof.

Preferably, the hybridising sequence encodes a polypeptide with an amino acid sequence which, when full-length and used in the construction of a phylogenetic tree, such as the one depicted in Figure 1 of Kandasamy et al. 2004. Trends Plant Sci 9: 196-202, clusters with the ARP6 group of polypeptides comprising the amino acid sequence of AtARP6 as represented by SEQ ID NO: 102 rather than with any other group.

Concerning POP polypeptides, hybridising sequences useful in the methods of the invention encode a POP polypeptide as defined herein, having substantially the same biological activity as the amino acid sequences given in Table A6 of the Examples section. Preferably, the hybridising sequence is capable of hybridising to the complement of any one of the nucleic acids given in Table A6 of the Examples section, or to a portion of any of these sequences, a portion being as defined above, or the hybridising sequence is capable of hybridising to the complement of a nucleic acid encoding an orthologue or paralogue of any one of the amino acid sequences given in Table A6 of the Examples section. Most preferably, the hybridising sequence is capable of hybridising to the complement of a nucleic acid as represented by SEQ ID NO: 116 or to a portion thereof.

Preferably, the hybridising sequence encodes a polypeptide with an amino acid sequence which, when full-length and used in the construction of a phylogenetic tree, such as the one depicted in Figure 13 (Tripathi & Sowdhamini 2006), clusters with the group of POP polypeptides indicated by the arrow comprising the amino acid sequence represented by SEQ ID NO: 117 rather than with any other group.

Concerning CRL polypeptides, hybridising sequences useful in the methods of the invention encode a CRL polypeptide as defined herein, having substantially the same biological activity as the amino acid sequences given in Table A7 of the Examples section. Preferably, the hybridising sequence is capable of hybridising to the complement of any one of the nucleic acids given in Table A7 of the Examples section, or to a portion of any of these sequences, a portion being as defined above, or the hybridising sequence is capable of hybridising to the complement of a nucleic acid encoding an orthologue or paralogue of any one of the amino acid sequences given in Table A7 of the Examples section. Most preferably, the hybridising sequence is capable of hybridising to the complement of a nucleic acid as represented by SEQ ID NO: 155 or to a portion thereof.

Preferably, the hybridising sequence encodes a polypeptide with an amino acid sequence which, when full-length and used in the construction of a phylogenetic tree, such as the one depicted in Figure 16, clusters with the group of CRL polypeptides comprising the amino acid sequence encoded by a monocotyledonous plant, more preferably by a sequence encoded by a dicotyledonous plant, most preferably by the sequence represented by SEQ ID NO: 156 rather than with any other group.

Another nucleic acid variant useful in the methods of the invention is a splice variant encoding an alfin-like polypeptide, or a YRP polypeptide, or a BRXL polypeptide, or a silky-1-like polypeptide, or an ARP6 polypeptide, or a POP polypeptide, or a CRL polypeptide, as defined hereinabove, a splice variant being as defined herein.

According to the present invention, there is provided a method for enhancing abiotic stress tolerance and/or enhancing yield-related traits in plants, comprising introducing and expressing in a plant a splice variant of any one of the nucleic acid sequences given in Table A1 to A7 of the Examples Section, or a splice variant of a nucleic acid encoding an orthologue, paralogue or homologue of any of the amino acid sequences given in Table A1 to A7 of the Examples Section.

Concerning alfin-like polypeptides, preferred splice variants are splice variants of a nucleic acid represented by any of SEQ ID NO: 1 or SEQ ID NO: 3, or a splice variant of a nucleic acid encoding an orthologue or paralogue of any of SEQ ID NO: 2 or SEQ ID NO: 4. Preferably, the amino acid sequence encoded by the splice variant, when used in the construction of a phylogenetic tree, clusters with the group of alfin-like polypeptides comprising the amino acid sequence represented by SEQ ID NO: 2 or SEQ ID NO: 4 rather than with any other group.

Concerning YRP polypeptides, preferred splice variants are splice variants of a nucleic acid represented by any of SEQ ID NO: 10 or SEQ ID NO: 12, or a splice variant of a nucleic acid encoding an orthologue or paralogue of any of SEQ ID NO: 11 or SEQ ID NO: 13. Preferably, the amino acid sequence encoded by the splice variant, when used in the construction of a phylogenetic tree, clusters with the group of YRP polypeptides comprising the amino acid sequence represented by SEQ ID NO: 11 or SEQ ID NO: 13 rather than with any other group.

Concerning BRXL polypeptides, preferred splice variants are splice variants of a nucleic acid sequence represented by SEQ ID NO: 17, or a splice variant of a nucleic acid sequence encoding an orthologue or paralogue of SEQ ID NO: 18. Preferably, the splice variant is a splice variant of a nucleic acid sequence encoding a polypeptide sequence comprising (i) in increasing order of preference at least 50%, 55%, 60%, 65%, 70%, 75%, 80%, 85%, 90%, 95%, 98%, 99% or more amino acid sequence identity to a Conserved Domain 1 (comprising a BRX domain) as represented by SEQ ID NO: 83; and (ii) in increasing order of preference at least 50%, 55%, 60%, 65%, 70%, 75%, 80%, 85%, 90%, 95%, 98%, 99% or more amino acid sequence identity to a Conserved Domain 2 (comprising a BRX domain) as represented by SEQ ID NO: 84. More preferably, the splice variant is a splice variant of a nucleic acid sequence encoding a polypeptide sequence having in increasing order of preference at least 40%, 45%, 50%, 55%, 60%, 65%, 70%, 75%, 80%, 85%, 90%, 95%, 98%, 99% or more amino acid sequence identity to the BRXL polypeptide as represented by SEQ ID NO: 18 or to any of the polypeptide sequences given in Table A3 herein. Most preferably, the splice variant is a splice variant of a nucleic acid sequence as represented by SEQ ID NO: 17, or of a nucleic acid sequence encoding a polypeptide sequence as represented by SEQ ID NO: 18.

Concerning silky-1-like polypeptides, preferred splice variants are splice variants of a nucleic acid represented by any of SEQ ID NO: 90, SEQ ID NO: 92 or SEQ ID NO: 94, or a splice variant of a nucleic acid encoding an orthologue or paralogue of any of SEQ ID NO: 91, SEQ ID NO: 93 or SEQ ID NO: 95. Preferably, the amino acid sequence encoded by the splice variant, when used in the construction of a phylogenetic tree, clusters with the group of silky-1-like polypeptides comprising the amino acid sequence represented by SEQ ID NO: 91, SEQ ID NO: 93 or SEQ ID NO: 95 rather than with any other group.

Concerning ARP6 polypeptides, preferred splice variants are splice variants of a nucleic acid represented by SEQ ID NO: 101, or a splice variant of a nucleic acid encoding an orthologue or paralogue of SEQ ID NO: 102. Preferably, the amino acid sequence encoded by the splice variant, when used in the construction of a phylogenetic tree, such as the one depicted in Figure 1 of Kandasamy et al. 2004. Trends Plant Sci 9: 196-202, clusters with the ARP6 group of polypeptides comprising the amino acid sequence of AtARP6 as represented by SEQ ID NO: 102 rather than with any other group.

Concerning POP polypeptides, preferred splice variants are splice variants of a nucleic acid represented by SEQ ID NO: 116, or a splice variant of a nucleic acid encoding an orthologue or paralogue of SEQ ID NO: 117. Preferably, the amino acid sequence encoded by the splice variant, when used in the construction of a phylogenetic tree, such as the one depicted in Figure 13 (Tripathi & Sowdhamini 2006), clusters with the group of POP polypeptides indicated by the arrow comprising the amino acid sequence represented by SEQ ID NO: 117 rather than with any other group.

Concerning CRL polypeptides, preferred splice variants are splice variants of a nucleic acid represented by SEQ ID NO: 155, or a splice variant of a nucleic acid encoding an orthologue or paralogue of SEQ ID NO: 156. Preferably, the amino acid sequence encoded by the splice variant, when used in the construction of a phylogenetic tree, , such as the one depicted in Figure 16, clusters with the group of CRL polypeptides comprising the amino acid sequence encoded by a monocotyledonous plant, more preferably by a sequence encoded by a dicotyledonous plant, most preferably by the sequence represented by SEQ ID NO: 156 rather than with any other group.

Another nucleic acid variant useful in performing the methods of the invention is an allelic variant of a nucleic acid encoding an alfin-like polypeptide, or a YRP polypeptide, or a BRXL polypeptide, or a silky-1-like polypeptide, or an ARP6 polypeptide, or a POP polypeptide, or a CRL polypeptide, as defined hereinabove, an allelic variant being as defined herein.

According to the present invention, there is provided a method for enhancing abiotic stress tolerance and/or enhancing yield-related traits in plants, comprising introducing and expressing in a plant an allelic variant of any one of the nucleic acids given in Table A1 to A7, or comprising introducing and expressing in a plant an allelic variant of a nucleic acid encoding an orthologue, paralogue or homologue of any of the amino acid sequences given in Table A1 to A7.

Concerning alfin-like polypeptides, the polypeptides encoded by allelic variants useful in the methods of the present invention have substantially the same biological activity as the alfin-like polypeptide of any of SEQ ID NO: 2 or any of the amino acids depicted in Table A1 of the Examples section. Allelic variants exist in nature, and encompassed within the methods of the present invention is the use of these natural alleles. Preferably, the allelic variant is an allelic variant of any of SEQ ID NO: 1 or SEQ ID NO: 3 or an allelic variant of a nucleic acid encoding an orthologue or paralogue of SEQ ID NO: 2 or SEQ ID NO: 4. Preferably, the amino acid sequence encoded by the allelic variant, clusters with the alfin-like polypeptides comprising the amino acid sequence represented by SEQ ID NO: 2 or SEQ ID NO: 4 rather than with any other group.

Concerning YRP polypeptides, the polypeptides encoded by allelic variants useful in the methods of the present invention have substantially the same biological activity as the YRP polypeptide of any of SEQ ID NO: 11 or any of the amino acids depicted in Table A2 of the Examples section. Allelic variants exist in nature, and encompassed within the methods of the present invention is the use of these natural alleles. Preferably, the allelic variant is an allelic variant of any of SEQ ID NO: 10 or SEQ ID NO: 12 or an allelic variant of a nucleic acid encoding an orthologue or paralogue of SEQ ID NO: 11 or SEQ ID NO: 13. Preferably, the amino acid sequence encoded by the allelic variant, clusters with the YRP polypeptides comprising the amino acid sequence represented by SEQ ID NO: 11 or SEQ ID NO: 13 rather than with any other group.

Concerning BRXL polypeptides, the allelic variants useful in the methods of the present invention have substantially the same biological activity as the BRXL polypeptide of SEQ ID NO: 18 and any of the polypeptide sequences depicted in Table A3 of Example 1. Allelic variants exist in nature, and encompassed within the methods of the present invention is the use of these natural alleles. Preferably, the allelic variant is an allelic variant of a polypeptide sequence comprising (i) in increasing order of preference at least 50%, 55%, 60%, 65%, 70%, 75%, 80%, 85%, 90%, 95%, 98%, 99% or more amino acid sequence identity to a Conserved Domain 1 (comprising a BRX domain) as represented by SEQ ID NO: 83; and (ii) in increasing order of preference at least 50%, 55%, 60%, 65%, 70%, 75%, 80%, 85%, 90%, 95%, 98%, 99% or more amino acid sequence identity to a Conserved Domain 2 (comprising a BRX domain) as represented by SEQ ID NO: 84. More preferably the allelic variant is an allelic variant encoding a polypeptide sequence having in increasing order of preference at least 40%, 45%, 50%, 55%, 60%, 65%, 70%, 75%, 80%, 85%, 90%, 95%, 98%, 99% or more amino acid sequence identity to the BRXL polypeptide as represented by SEQ ID NO: 18 or to any of the polypeptide sequences given in Table A herein. Most preferably, the allelic variant is an allelic variant of SEQ ID NO: 17 or an allelic variant of a nucleic acid sequence encoding an orthologue or paralogue of SEQ ID NO: 18.

Concerning silky-1-like polypeptides, the polypeptides encoded by allelic variants useful in the methods of the present invention have substantially the same biological activity as the silky-1-like polypeptide of any of SEQ ID NO: 91 or any of the amino acids depicted in Table A4 of the Examples section. Allelic variants exist in nature, and encompassed within the methods of the present invention is the use of these natural alleles. Preferably, the allelic variant is an allelic variant of any of SEQ ID NO: 90, SEQ ID NO: 92 or SEQ ID NO: 94 or an allelic variant of a nucleic acid encoding an orthologue or paralogue of SEQ ID NO: 91, SEQ ID NO: 93 or SEQ ID NO: 95. Preferably, the amino acid sequence encoded by the allelic variant, clusters with the silky-1-like polypeptides comprising the amino acid sequence represented by SEQ ID NO: 91, SEQ ID NO: 93 or SEQ ID NO: 95 rather than with any other group.

Concerning ARP6 polypeptides, the polypeptides encoded by allelic variants useful in the methods of the present invention have substantially the same biological activity as the ARP6 polypeptide of SEQ ID NO: 102 and any of the amino acids depicted in Table A5 of the Examples section. Allelic variants exist in nature, and encompassed within the methods of the present invention is the use of these natural alleles. Preferably, the allelic variant is an allelic variant of SEQ ID NO: 101 or an allelic variant of a nucleic acid encoding an orthologue or paralogue of SEQ ID NO: 102. Preferably, the amino acid sequence encoded by the allelic variant, when used in the construction of a phylogenetic tree, such as the one depicted in Figure 1 of Kandasamy et al. 2004. Trends Plant Sci 9: 196-202, clusters with the ARP6 group of polypeptides comprising the amino acid sequence of AtARP6 as represented by SEQ ID NO: 102 rather than with any other group.

Concerning POP polypeptides, the polypeptides encoded by allelic variants useful in the methods of the present invention have substantially the same biological activity as the POP polypeptide of SEQ ID NO: 117 and any of the amino acids depicted in Table A6 of the Examples section. Allelic variants exist in nature, and encompassed within the methods of the present invention is the use of these natural alleles. Preferably, the allelic variant is an allelic variant of SEQ ID NO: 116 or an allelic variant of a nucleic acid encoding an orthologue or paralogue of SEQ ID NO: 117. Preferably, the amino acid sequence encoded by the allelic variant, when used in the construction of a phylogenetic tree, such as the one depicted in Figure 13 (Tripathi & Sowdhamini 2006), clusters with the group of POP polypeptides indicated by the arrow comprising the amino acid sequence represented by SEQ ID NO: 117 rather than with any other group.

Concerning CRL polypeptides, the polypeptides encoded by allelic variants useful in the methods of the present invention have substantially the same biological activity as the CRL polypeptide of SEQ ID NO: 156 and any of the amino acids depicted in Table A7 of the Examples section. Allelic variants exist in nature, and encompassed within the methods of the present invention is the use of these natural alleles. Preferably, the allelic variant is an allelic variant of SEQ ID NO: 155 or an allelic variant of a nucleic acid encoding an orthologue or paralogue of SEQ ID NO: 156. Preferably, the amino acid sequence encoded by the allelic variant, when used in the construction of a phylogenetic tree, such as the one depicted in Figure 16, clusters with the group of CRL polypeptides comprising the amino acid sequence encoded by a monocotyledonous plant, more preferably by a sequence encoded by a dicotyledonous plant, most preferably by the sequence represented by SEQ ID NO: 156 rather than with any other group.

Gene shuffling or directed evolution may also be used to generate variants of nucleic acids encoding alfin-like polypeptides, or YRP polypeptides, or BRXL polypeptides, or silky-1-like polypeptides, or ARP6 polypeptides, or POP polypeptides, or CRL polypeptides, as defined above; the term "gene shuffling" being as defined herein.

According to the present invention, there is provided a method for enhancing abiotic stress tolerance and/or yield-related traits in plants, comprising introducing and expressing in a plant a variant of any one of the nucleic acid sequences given in Table A1 to A7 of the Examples section, or comprising introducing and expressing in a plant a variant of a nucleic acid encoding an orthologue, paralogue or homologue of any of the amino acid sequences given in Table A1 to A7 of the Examples section, which variant nucleic acid is obtained by gene shuffling.

Concerning alfin-like polypeptides, preferably, the amino acid sequence encoded by the variant nucleic acid obtained by gene shuffling, when used in the construction of a phylogenetic tree, clusters with the group of alfin-like polypeptides comprising the amino acid sequence represented by SEQ ID NO: 2 or SEQ ID NO: 4 rather than with any other group.

Concerning YRP polypeptides, preferably, the amino acid sequence encoded by the variant nucleic acid obtained by gene shuffling, when used in the construction of a phylogenetic tree, clusters with the group of YRP polypeptides comprising the amino acid sequence represented by SEQ ID NO: 11 or SEQ ID NO: 13 rather than with any other group.

Concerning BRXL polypeptides, preferably, the variant nucleic acid sequence obtained by gene shuffling encodes a polypeptide sequence comprising (i) in increasing order of preference at least 50%, 55%, 60%, 65%, 70%, 75%, 80%, 85%, 90%, 95%, 98%, 99% or more amino acid sequence identity to a Conserved Domain 1 (comprising a BRX domain) as represented by SEQ ID NO: 83; and (ii) in increasing order of preference at least 50%, 55%, 60%, 65%, 70%, 75%, 80%, 85%, 90%, 95%, 98%, 99% or more amino acid sequence identity to a Conserved Domain 2 (comprising a BRX domain) as represented by SEQ ID NO: 84. More preferably, the variant nucleic acid sequence obtained by gene shuffling encodes a polypeptide sequence having in increasing order of preference at least 40%, 45%, 50%, 55%, 60%, 65%, 70%, 75%, 80%, 85%, 90%, 95%, 98%, 99% or more amino acid sequence identity to the BRXL polypeptide as represented by SEQ ID NO: 18 or to any of the polypeptide sequences given in Table A3 herein. Most preferably, the nucleic acid sequence obtained by gene shuffling encodes a polypeptide sequence as represented by SEQ ID NO: 18.

Concerning silky-1-like polypeptides, preferably, the amino acid sequence encoded by the variant nucleic acid obtained by gene shuffling, when used in the construction of a phylogenetic tree, clusters with the group of silky-1-like polypeptides comprising the amino acid sequence represented by SEQ ID NO: 91, SEQ ID NO: 93 or SEQ ID NO: 95 rather than with any other group.

Concerning ARP6 polypeptides, preferably, the amino acid sequence encoded by the variant nucleic acid obtained by gene shuffling, when used in the construction of a phylogenetic tree such as the one depicted in Figure 1 of Kandasamy et al. 2004. Trends Plant Sci 9: 196-202, clusters with the ARP6 group of polypeptides comprising the amino acid sequence of AtARP6 as represented by SEQ ID NO: 102 rather than with any other group.

Concerning POP polypeptides, preferably, the amino acid sequence encoded by the variant nucleic acid obtained by gene shuffling, when used in the construction of a phylogenetic tree such as the one depicted in Figure 13 (Tripathi & Sowdhamini 2006), clusters with the group of POP polypeptides indicated by the arrow comprising the amino acid sequence represented by SEQ ID NO: 117 rather than with any other group.

Concerning CRL polypeptides, preferably, the amino acid sequence encoded by the variant nucleic acid obtained by gene shuffling, when used in the construction of a phylogenetic tree such as the one depicted in Figure 16, clusters with the group of CRL polypeptides comprising the amino acid sequence encoded by a monocotyledonous plant, more preferably by a sequence encoded by a dicotyledonous plant, most preferably by the sequence represented by SEQ ID NO: 156 rather than with any other group.

Furthermore, nucleic acid variants may also be obtained by site-directed mutagenesis. Several methods are available to achieve site-directed mutagenesis, the most common being PCR based methods (Current Protocols in Molecular Biology. Wiley Eds.).

Nucleic acids encoding alfin-like polypeptides may be derived from any natural or artificial source. The nucleic acid may be modified from its native form in composition and/or genomic environment through deliberate human manipulation. Preferably the alfin-like polypeptide-encoding nucleic acid is from a plant, further preferably from a monocotyledonous or dicotyledonous plant, more preferably from the family Allium or Hordeum.

Nucleic acids encoding YRP polypeptides may be derived from any natural or artificial source. The nucleic acid may be modified from its native form in composition and/or genomic environment through deliberate human manipulation. Preferably the YRP polypeptide-encoding nucleic acid is from a plant, further preferably from a monocotyledonous or dicotyledonous plant, more preferably from the family Populus or Solanum.

Nucleic acid sequences encoding BRXL polypeptides may be derived from any natural or artificial source. The nucleic acid sequence may be modified from its native form in composition and/or genomic environment through deliberate human manipulation. The nucleic acid sequence encoding a BRXL polypeptide is from a plant, further preferably from a dicotyledonous plant, more preferably from the family Salicaceae, most preferably the nucleic acid sequence is from Populus trichocarpa.

Nucleic acids encoding silky-1-like polypeptides may be derived from any natural or artificial source. The nucleic acid may be modified from its native form in composition and/or genomic environment through deliberate human manipulation. Preferably the silky-1-like polypeptide-encoding nucleic acid is from a plant, further preferably from a monocotyledonous or dicotyledonous plant, more preferably from the family Allium or Hordeum.

Nucleic acids encoding ARP6 polypeptides may be derived from any natural or artificial source. The nucleic acid may be modified from its native form in composition and/or genomic environment through deliberate human manipulation. Preferably the ARP6 polypeptide-encoding nucleic acid is from a plant, further preferably from a dicocotyledonous plant, more preferably from the family Brassicaceae, most preferably the nucleic acid is from Arabidopsis thaliana.

Nucleic acids encoding POP polypeptides may be derived from any natural or artificial source. The nucleic acid may be modified from its native form in composition and/or genomic environment through deliberate human manipulation. Preferably the POP polypeptide-encoding nucleic acid is from a plant, further preferably from a dicotyledonous plant, more preferably from the family Brassicaceae, most preferably the nucleic acid is from Arabidopsis thaliana.

Nucleic acids encoding CRL polypeptides may be derived from any natural or artificial source. The nucleic acid may be modified from its native form in composition and/or genomic environment through deliberate human manipulation. Preferably the CRL polypeptide-encoding nucleic acid is from a plant, further preferably from a dicotyledonous plant, more preferably from the family Brassicaceae, most preferably the nucleic acid is from Arabidopsis thaliana.

Advantageously, the invention also provides hitherto unknown CRL-encoding nucleic acids and CRL polypeptides.

According to a further embodiment of the present invention, there is therefore provided an isolated nucleic acid molecule selected from:
(i) a nucleic acid represented by SEQ ID NO: 41;
(ii) the complement of a nucleic acid represented by SEQ ID NO: 41:
(iii) a nucleic acid encoding the polypeptide as represented by SEQ ID NO: 42, preferably as a result of the degeneracy of the genetic code, said isolated nucleic acid can be derived from a polypeptide sequence as represented by SEQ ID NO: 42 and further preferably confers enhanced yield-related traits relative to control plants;
(iv) a nucleic acid having, in increasing order of preference at least 30 %, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% sequence identity with any of the nucleic acid sequences of Table A and further preferably conferring enhanced yield-related traits relative to control plants;
(v) a nucleic acid molecule which hybridizes with a nucleic acid molecule of (i) to (iv) under stringent hybridization conditions and preferably confers enhanced yield-related traits relative to control plants;
(vi) a nucleic acid encoding an ASPAT polypeptide having, in increasing order of preference, at least 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% sequence identity to the amino acid sequence represented by SEQ ID NO: 42 and any of the other amino acid sequences in Table A and preferably conferring enhanced yield-related traits relative to control plants.

According to a further embodiment of the present invention, there is also provided an isolated polypeptide selected from:
(i) an amino acid sequence represented by SEQ ID NO: 42;
(ii) an amino acid sequence having, in increasing order of preference, at least 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% sequence identity to the amino acid sequence represented by SEQ ID NO: 42, and any of the other amino acid sequences in Table A and preferably conferring enhanced yield-related traits relative to control plants.
(iii) derivatives of any of the amino acid sequences given in (i) or (ii) above.

Concerning alfin-like polypeptides, or YRP polypeptides, or silky-1-like polypeptides, performance of the methods of the invention gives plants having enhanced tolerance to abiotic stress.

Concerning BRXL polypeptides, performance of the methods of the invention gives plants having increased yield-related traits relative to control plants. The terms "yield" and "seed yield" are described in more detail in the "definitions" section herein.

Concerning ARP6 polypeptides, or CRL polypeptides, performance of the methods of the invention gives plants having enhanced yield-related traits. In particular performance of the methods of the invention gives plants having increased yield, especially increased seed yield relative to control plants. The terms "yield" and "seed yield" are described in more detail in the "definitions" section herein.

Concerning POP polypeptides, performance of the methods of the invention gives plants having enhanced yield-related traits. In particular performance of the methods of the invention gives plants having increased yield, especially increased (root and/or shoot) biomass and seed yield relative to control plants. The terms "yield" and "seed yield" are described in more detail in the "definitions" section herein. Performance of the methods of the invention gives plants having modified flowering time (preferably earlier flowering time).

Reference herein to enhanced yield-related traits is taken to mean an increase in biomass (weight) of one or more parts of a plant, which may include aboveground (harvestable) parts and/or (harvestable) parts below ground. In particular, such harvestable parts are seeds, and performance of the methods of the invention results in plants having increased seed yield relative to the seed yield of control plants.

Taking corn as an example, a yield increase may be manifested as one or more of the following: increase in the number of plants established per square meter, an increase in the number of ears per plant, an increase in the number of rows, number of kernels per row, kernel weight, thousand kernel weight, ear length/diameter, increase in the seed filling rate (which is the number of filled seeds divided by the total number of seeds and multiplied by 100), among others. Taking rice as an example, a yield increase may manifest itself as an increase in one or more of the following: number of plants per square meter, number of panicles per plant, number of spikelets per panicle, number of flowers (florets) per panicle (which is expressed as a ratio of the number of filled seeds over the number of primary panicles), increase in the seed filling rate (which is the number of filled seeds divided by the total number of seeds and multiplied by 100), increase in thousand kernel weight, among others.

Concerning abiotic stress, the present invention provides a method for enhancing stress tolerance in plants, relative to control plants, which method comprises modulating expression in a plant of a nucleic acid encoding an alfin-like polypeptide, or a YRP polypeptide, or a silky-1-like polypeptide, as defined herein.

Plants typically respond to exposure to stress by growing more slowly. In conditions of severe stress, the plant may even stop growing altogether. Mild stress on the other hand is defined herein as being any stress to which a plant is exposed which does not result in the plant ceasing to grow altogether without the capacity to resume growth. Mild stress in the sense of the invention leads to a reduction in the growth of the stressed plants of less than 40%, 35%, 30% or 25%, more preferably less than 20% or 15% in comparison to the control plant under non-stress conditions. Due to advances in agricultural practices (irrigation, fertilization, pesticide treatments) severe stresses are not often encountered in cultivated crop plants. As a consequence, the compromised growth induced by mild stress is often an undesirable feature for agriculture. Mild stresses are the everyday biotic and/or abiotic (environmental) stresses to which a plant is exposed. Abiotic stresses may be due to drought or excess water, anaerobic stress, salt stress, chemical toxicity, oxidative stress and hot, cold or freezing temperatures. The abiotic stress may be an osmotic stress caused by a water stress (particularly due to drought), salt stress, oxidative stress or an ionic stress. Biotic stresses are typically those stresses caused by pathogens, such as bacteria, viruses, fungi, nematodes and insects.

In particular, the methods of the present invention may be performed under conditions of (mild) drought to give plants having enhanced drought tolerance relative to control plants, which might manifest itself as an increased yield relative to control plants. As reported in Wang et al. (Planta (2003) 218: 1-14), abiotic stress leads to a series of morphological, physiological, biochemical and molecular changes that adversely affect plant growth and productivity. Drought, salinity, extreme temperatures and oxidative stress are known to be interconnected and may induce growth and cellular damage through similar mechanisms. Rabbani et al. (Plant Physiol (2003) 133: 1755-1767) describes a particularly high degree of "cross talk" between drought stress and high-salinity stress. For example, drought and/or salinisation are manifested primarily as osmotic stress, resulting in the disruption of homeostasis and ion distribution in the cell. Oxidative stress, which frequently accompanies high or low temperature, salinity or drought stress, may cause denaturing of functional and structural proteins. As a consequence, these diverse environmental stresses often activate similar cell signalling pathways and cellular responses, such as the production of stress proteins, up-regulation of anti-oxidants, accumulation of compatible solutes and growth arrest. The term "non-stress" conditions as used herein are those environmental conditions that allow optimal growth of plants. Persons skilled in the art are aware of normal soil conditions and climatic conditions for a given location. Plants with optimal growth conditions, (grown under non-stress conditions) typically yield in increasing order of preference at least 97%, 95%, 92%, 90%, 87%, 85%, 83%, 80%, 77% or 75% of the average production of such plant in a given environment. Average production may be calculated on harvest and/or season basis. Persons skilled in the art are aware of average yield productions of a crop.

Performance of the methods of the invention gives plants grown under (mild) drought conditions enhanced drought tolerance relative to control plants grown under comparable conditions. Therefore, according to the present invention, there is provided a method for enhancing drought tolerance in plants grown under (mild) drought conditions, which method comprises modulating expression in a plant of a nucleic acid encoding an alfin-like polypeptide, or a YRP polypeptide, or a silky-1-like polypeptide.

Performance of the methods of the invention gives plants grown under conditions of nutrient deficiency, particularly under conditions of nitrogen deficiency, enhanced tolerance to stresses caused by nutrient deficiency relative to control plants. Therefore, according to the present invention, there is provided a method for enhancing tolerance to stresses caused by nutrient deficiency, which method comprises modulating expression in a plant of a nucleic acid encoding an alfin-like polypeptide, or a YRP polypeptide, or a silky-1-like polypeptide. Nutrient deficiency may result from a lack of nutrients such as nitrogen, phosphates and other phosphorous-containing compounds, potassium, calcium, magnesium, manganese, iron and boron, amongst others.

Performance of the methods of the invention gives plants grown under conditions of salt stress, enhanced tolerance to salt relative to control plants grown under comparable conditions. Therefore, according to the present invention, there is provided a method for enhancing salt tolerance in plants grown under conditions of salt stress, which method comprises modulating expression in a plant of a nucleic acid encoding an alfin-like polypeptide, or a YRP polypeptide, or a silky-1-like polypeptide. The term salt stress is not restricted to common salt (NaCl), but may be any one or more of: NaCl, KCI, LiCl, MgCl₂, CaCl₂, amongst others.

Concerning yield-related traits, the present invention provides a method for increasing yield-related traits of plants relative to control plants, which method comprises increasing expression in a plant of a nucleic acid sequence encoding a BRXL polypeptide as defined herein.

The present invention provides a method for increasing yield, especially seed yield of plants, relative to control plants, which method comprises modulating expression in a plant of a nucleic acid encoding or an ARP6 polypeptide, or a POP polypeptide, or a CRL polypeptide, as defined herein.

Since the transgenic plants according to the present invention have increased yield-related traits and/or yield, it is likely that these plants exhibit an increased growth rate (during at least part of their life cycle), relative to the growth rate of control plants at a corresponding stage in their life cycle.

The increased growth rate may be specific to one or more parts of a plant (including seeds), or may be throughout substantially the whole plant. Plants having an increased growth rate may have a shorter life cycle. The life cycle of a plant may be taken to mean the time needed to grow from a dry mature seed up to the stage where the plant has produced dry mature seeds, similar to the starting material. This life cycle may be influenced by factors such as speed of germination, early vigour, growth rate, greenness index, flowering time and speed of seed maturation. The increase in growth rate may take place at one or more stages in the life cycle of a plant or during substantially the whole plant life cycle. Increased growth rate during the early stages in the life cycle of a plant may reflect enhanced vigour. The increase in growth rate may alter the harvest cycle of a plant allowing plants to be sown later and/or harvested sooner than would otherwise be possible (a similar effect may be obtained with earlier flowering time). If the growth rate is sufficiently increased, it may allow for the further sowing of seeds of the same plant species (for example sowing and harvesting of rice plants followed by sowing and harvesting of further rice plants all within one conventional growing period). Similarly, if the growth rate is sufficiently increased, it may allow for the further sowing of seeds of different plants species (for example the sowing and harvesting of corn plants followed by, for example, the sowing and optional harvesting of soybean, potato or any other suitable plant). Harvesting additional times from the same rootstock in the case of some crop plants may also be possible. Altering the harvest cycle of a plant may lead to an increase in annual biomass production per square meter (due to an increase in the number of times (say in a year) that any particular plant may be grown and harvested). An increase in growth rate may also allow for the cultivation of transgenic plants in a wider geographical area than their wild-type counterparts, since the territorial limitations for growing a crop are often determined by adverse environmental conditions either at the time of planting (early season) or at the time of harvesting (late season). Such adverse conditions may be avoided if the harvest cycle is shortened. The growth rate may be determined by deriving various parameters from growth curves, such parameters may be: T-Mid (the time taken for plants to reach 50% of their maximal size) and T-90 (time taken for plants to reach 90% of their maximal size), amongst others.

According to a preferred feature of the present invention, performance of the methods of the invention gives plants having an increased growth rate relative to control plants. Therefore, according to the present invention, there is provided a method for increasing the growth rate of plants, which method comprises increasing expression in a plant of a nucleic acid encoding a BRXL polypeptide, or an ARP6 polypeptide, or a POP polypeptide, or a CRL polypeptide, as defined herein.

Performance of the methods of the invention gives plants grown under non-stress conditions or under mild stress conditions having increased yield-related traits, relative to control plants grown under comparable conditions. Therefore, according to the present invention, there is provided a method for increasing yield-related traits in plants grown under non-stress conditions or under mild stress conditions, which method comprises increasing expression in a plant of a nucleic acid sequence encoding a BRXL polypeptide, or an ARP6 polypeptide, or a POP polypeptide, or a CRL polypeptide.

Performance of the methods of the invention gives plants grown under conditions of reduced nutrient availability, particularly under conditions of reduced nitrogen availablity, having increased yield-related traits and/or yield relative to control plants grown under comparable conditions. Therefore, according to the present invention, there is provided a method for increasing yield-related traits in plants grown under conditions of reduced nutrient availablity, preferably reduced nitrogen availability, which method comprises increasing expression in a plant of a nucleic acid sequence encoding a BRXL polypeptide, or an ARP6 polypeptide, or a POP polypeptide, or a CRL polypeptide. Reduced nutrient availability may result from a deficiency or excess of nutrients such as nitrogen, phosphates and other phosphorous-containing compounds, potassium, calcium, cadmium, magnesium, manganese, iron and boron, amongst others. Preferably, reduced nutrient availablity is reduced nitrogen availability.

Performance of the methods of the invention gives plants grown under conditions of salt stress, increased yield relative to control plants grown under comparable conditions. Therefore, according to the present invention, there is provided a method for increasing yield in plants grown under conditions of salt stress, which method comprises modulating expression in a plant of a nucleic acid encoding an ARP6 polypeptide, or a POP polypeptide, or a CRL polypeptide. The term salt stress is not restricted to common salt (NaCl), but may be any one or more of: NaCl, KCI, LiCl, MgCl₂, CaCl₂, amongst others.

The present invention encompasses plants or parts thereof (including seeds) obtainable by the methods according to the present invention. The plants or parts thereof comprise a nucleic acid transgene encoding an alfin-like polypeptide, or a YRP polypeptide, or a BRXL polypeptide, or a silky-1-like polypeptide, or an ARP6 polypeptide, or a POP polypeptide, or a CRL polypeptide, as defined above, operably linked to a promoter functioning in plants.

The invention also provides genetic constructs and vectors to facilitate introduction and/or expression in plants of nucleic acids encoding alfin-like polypeptides, or YRP polypeptides, or BRXL polypeptides, or silky-1-like polypeptides, or ARP6 polypeptides, or POP polypeptides, or CRL polypeptides. The gene constructs may be inserted into vectors, which may be commercially available, suitable for transforming into plants and suitable for expression of the gene of interest in the transformed cells. The invention also provides use of a gene construct as defined herein in the methods of the invention.

More specifically, the present invention provides a construct comprising:
(a) a nucleic acid encoding an alfin-like polypeptide, or a YRP polypeptide, or a BRXL polypeptide, or a silky-1-like polypeptide, or an ARP6 polypeptide, or a POP polypeptide, or a CRL polypeptide, as defined above;
(b) one or more control sequences capable of driving expression of the nucleic acid sequence of (a); and optionally
(c) a transcription termination sequence.

Preferably, the nucleic acid encoding an alfin-like polypeptide, or a YRP polypeptide, or a BRXL polypeptide, or a silky-1-like polypeptide, or an ARP6 polypeptide, or a POP polypeptide, or a CRL polypeptide, is as defined above. The term "control sequence" and "termination sequence" are as defined herein.

Concerning BRXL polypeptides, preferably, one of the control sequences of a construct is a consitituve promoter isolated from a plant genome. An example of a constitutive promoter is a GOS2 promoter, preferably a GOS2 promoter from rice, most preferably a GOS2 sequence as represented by SEQ ID NO: 87.

Plants are transformed with a vector comprising any of the nucleic acids described above. The skilled artisan is well aware of the genetic elements that must be present on the vector in order to successfully transform, select and propagate host cells containing the sequence of interest. The sequence of interest is operably linked to one or more control sequences (at least to a promoter).

Advantageously, any type of promoter, whether natural or synthetic, may be used to drive expression of the nucleic acid sequence, but preferably the promoter is of plant origin. A constitutive promoter is particularly useful in the methods. Preferably the constitutive promoter is also a ubiquitous promoter of medium strength. See the "Definitions" section herein for definitions of the various promoter types.

Concerning BRXL polypeptides, advantageously, any type of promoter, whether natural or synthetic, may be used to increase expression of the nucleic acid sequence. A constitutive promoter is particularly useful in the methods, preferably a constitutive promoter isolated from a plant genome. The plant constitutive promoter drives expression of a coding sequence at a level that is in all instances below that obtained under the control of a 35S CaMV viral promoter. An example of such a promoter is a GOS2 promoter as represented by SEQ ID NO: 87.

In the case of BRXL genes, organ-specific promoters, for example for preferred expression in leaves, stems, tubers, meristems, seeds, are useful in performing the methods of the invention. Developmentally-regulated and inducible promoters are also useful in performing the methods of the invention. See the "Definitions" section herein for definitions of the various promoter types.

Concerning alfin-like polypeptides, it should be clear that the applicability of the present invention is not restricted to the alfin-like polypeptide-encoding nucleic acid represented by SEQ ID NO: 1 or SEQ ID NO: 3, nor is the applicability of the invention restricted to expression of an alfin-like polypeptide-encoding nucleic acid when driven by a constitutive promoter.

The constitutive promoter is preferably a medium strength promoter, more preferably selected from a plant derived promoter, such as a GOS2 promoter, more preferably is the promoter GOS2 promoter from rice. Further preferably the constitutive promoter is represented by a nucleic acid sequence substantially similar to SEQ ID NO: 7, most preferably the constitutive promoter is as represented by SEQ ID NO: 7. See the "Definitions" section herein for further examples of constitutive promoters.

Optionally, one or more terminator sequences may be used in the construct introduced into a plant. Preferably, the construct comprises an expression cassette comprising a (GOS2) promoter, substantially similar to SEQ ID NO: 7, and the nucleic acid encoding the alfin-like polypeptide.

Concerning YRP polypeptides, it should be clear that the applicability of the present invention is not restricted to the YRP polypeptide-encoding nucleic acid represented by SEQ ID NO: 10 or SEQ ID NO: 12, nor is the applicability of the invention restricted to expression of a YRP polypeptide-encoding nucleic acid when driven by a constitutive promoter.

The constitutive promoter is preferably a medium strength promoter, more preferably selected from a plant derived promoter, such as a GOS2 promoter, more preferably is the promoter GOS2 promoter from rice. Further preferably the constitutive promoter is represented by a nucleic acid sequence substantially similar to SEQ ID NO: 16, most preferably the constitutive promoter is as represented by SEQ ID NO: 16. See the "Definitions" section herein for further examples of constitutive promoters.

Optionally, one or more terminator sequences may be used in the construct introduced into a plant. Preferably, the construct comprises an expression cassette comprising a (GOS2) promoter, substantially similar to SEQ ID NO: 16, and the nucleic acid encoding the YRP polypeptide.

Concerning BRXL polypeptides, it should be clear that the applicability of the present invention is not restricted to a nucleic acid sequence encoding the BRXL polypeptide, as represented by SEQ ID NO: 17, nor is the applicability of the invention restricted to expression of a BRXL polypeptide-encoding nucleic acid sequence when driven by a constitituve promoter.

Optionally, one or more terminator sequences may be used in the construct introduced into a plant.

Concerning silky-1-like polypeptides, it should be clear that the applicability of the present invention is not restricted to the silky-1-like polypeptide-encoding nucleic acid represented by SEQ ID NO: 90, SEQ ID NO: 92 or SEQ ID NO: 94, nor is the applicability of the invention restricted to expression of a silky-1-like polypeptide-encoding nucleic acid when driven by a constitutive promoter.

The constitutive promoter is preferably a medium strength promoter, more preferably selected from a plant derived promoter, such as a GOS2 promoter, more preferably is the promoter GOS2 promoter from rice. Further preferably the constitutive promoter is represented by a nucleic acid sequence substantially similar to SEQ ID NO: 96, most preferably the constitutive promoter is as represented by SEQ ID NO: 96. See the "Definitions" section herein for further examples of constitutive promoters.

Optionally, one or more terminator sequences may be used in the construct introduced into a plant. Preferably, the construct comprises an expression cassette comprising a (GOS2) promoter, substantially similar to SEQ ID NO: 96, and the nucleic acid encoding the silky-1-like polypeptide.

Concerning ARP6 polypeptides, it should be clear that the applicability of the present invention is not restricted to the ARP6 polypeptide-encoding nucleic acid represented by SEQ ID NO: 101, nor is the applicability of the invention restricted to expression of an ARP6 polypeptide-encoding nucleic acid when driven by a constitutive promoter.

The constitutive promoter is preferably a medium strength promoter, more preferably selected from a plant derived promoter, such as a GOS2 promoter, more preferably is the promoter GOS2 promoter from rice. Further preferably the constitutive promoter is represented by a nucleic acid sequence substantially similar to SEQ ID NO: 115, most preferably the constitutive promoter is as represented by SEQ ID NO: 115. See the "Definitions" section herein for further examples of constitutive promoters.

Optionally, one or more terminator sequences may be used in the construct introduced into a plant. Preferably, the construct comprises an expression cassette comprising a GOS2 promoter, substantially similar to SEQ ID NO: 115, and the nucleic acid encoding the ARP6 polypeptide.

Concerning POP polypeptides, it should be clear that the applicability of the present invention is not restricted to the POP polypeptide-encoding nucleic acid represented by SEQ ID NO: 116, nor is the applicability of the invention restricted to expression of a POP polypeptide-encoding nucleic acid when driven by a constitutive promoter.

The constitutive promoter is preferably a medium strength promoter, more preferably selected from a plant derived promoter, such as a GOS2 promoter, more preferably is the promoter GOS2 promoter from rice. Further preferably the constitutive promoter is represented by a nucleic acid sequence substantially similar to SEQ ID NO: 134, most preferably the constitutive promoter is as represented by SEQ ID NO: 134. See the "Definitions" section herein for further examples of constitutive promoters.

Optionally, one or more terminator sequences may be used in the construct introduced into a plant. Preferably, the construct comprises an expression cassette comprising a GOS2 promoter, substantially similar to SEQ ID NO: 134, and the nucleic acid encoding the POP polypeptide.

Concerning CRL polypeptides, it should be clear that the applicability of the present invention is not restricted to the CRL polypeptide-encoding nucleic acid represented by SEQ ID NO: 155, nor is the applicability of the invention restricted to expression of a CRL polypeptide-encoding nucleic acid when driven by a constitutive promoter, or when driven by a root-specific promoter.

The constitutive promoter is preferably a medium strength promoter, more preferably selected from a plant derived promoter, such as a GOS2 promoter, more preferably is the promoter GOS2 promoter from rice. Further preferably the constitutive promoter is represented by a nucleic acid sequence substantially similar to SEQ ID NO: 213, most preferably the constitutive promoter is as represented by SEQ ID NO: 213. See the "Definitions" section herein for further examples of constitutive promoters.

Optionally, one or more terminator sequences may be used in the construct introduced into a plant. Preferably, the construct comprises an expression cassette comprising a (GOS2) promoter, substantially similar to SEQ ID NO: 213, and the nucleic acid encoding the CRL polypeptide.

Additional regulatory elements may include transcriptional as well as translational enhancers. Those skilled in the art will be aware of terminator and enhancer sequences that may be suitable for use in performing the invention. An intron sequence may also be added to the 5' untranslated region (UTR) or in the coding sequence to increase the amount of the mature message that accumulates in the cytosol, as described in the definitions section. Other control sequences (besides promoter, enhancer, silencer, intron sequences, 3'UTR and/or 5'UTR regions) may be protein and/or RNA stabilizing elements. Such sequences would be known or may readily be obtained by a person skilled in the art.

The genetic constructs of the invention may further include an origin of replication sequence that is required for maintenance and/or replication in a specific cell type. One example is when a genetic construct is required to be maintained in a bacterial cell as an episomal genetic element (e.g. plasmid or cosmid molecule). Preferred origins of replication include, but are not limited to, the f1-ori and colE1.

For the detection of the successful transfer of the nucleic acid sequences as used in the methods of the invention and/or selection of transgenic plants comprising these nucleic acids, it is advantageous to use marker genes (or reporter genes). Therefore, the genetic construct may optionally comprise a selectable marker gene. Selectable markers are described in more detail in the "definitions" section herein. The marker genes may be removed or excised from the transgenic cell once they are no longer needed. Techniques for marker removal are known in the art, useful techniques are described above in the definitions section.

It is known that upon stable or transient integration of nucleic acid sequences into plant cells, only a minority of the cells takes up the foreign DNA and, if desired, integrates it into its genome, depending on the expression vector used and the transfection technique used. To identify and select these integrants, a gene coding for a selectable marker (such as the ones described above) is usually introduced into the host cells together with the gene of interest. These markers can for example be used in mutants in which these genes are not functional by, for example, deletion by conventional methods. Furthermore, nucleic acid sequence molecules encoding a selectable marker can be introduced into a host cell on the same vector that comprises the sequence encoding the polypeptides of the invention or used in the methods of the invention, or else in a separate vector. Cells which have been stably transfected with the introduced nucleic acid sequence can be identified for example by selection (for example, cells which have integrated the selectable marker survive whereas the other cells die). The marker genes may be removed or excised from the transgenic cell once they are no longer needed. Techniques for marker gene removal are known in the art, useful techniques are described above in the definitions section.

The invention also provides a method for the production of transgenic plants having enhanced abiotic stress tolerance and/or yield-related traits relative to control plants, comprising introduction and expression in a plant of any nucleic acid encoding an alfin-like polypeptide, or a YRP polypeptide, or a BRXL polypeptide, or a silky-1-like polypeptide, or an ARP6 polypeptide, or a POP polypeptide, or a CRL polypeptide, as defined hereinabove.

More specifically, the present invention provides a method for the production of transgenic plants having enhanced abiotic stress tolerance, particularly increased (mild) drought tolerance, which method comprises:
(i) introducing and expressing in a plant or plant cell a nucleic acid encoding an alfin-like polypeptide, or a YRP polypeptide, or a silky-1-like polypeptide; and
(ii) cultivating the plant cell under abiotic stress conditions.

The nucleic acid of (i) may be any of the nucleic acids capable of encoding an alfin-like polypeptide, or a YRP polypeptide, or a silky-1-like polypeptide, as defined herein.

More specifically, the present invention also provides a method for the production of transgenic plants having increased yield-related traits relative to control plants, which method comprises:
(i) introducing and expressing in a plant, plant part, or plant cell a nucleic acid sequence encoding a BRXL polypeptide; and
(ii) cultivating the plant cell, plant part or plant under conditions promoting plant growth and development.

The nucleic acid sequence of (i) may be any of the nucleic acid sequences capable of encoding a BRXL polypeptide as defined herein.

More specifically, the present invention also provides a method for the production of transgenic plants having enhanced yield-related traits, particularly increased seed yield, which method comprises:
(i) introducing and expressing in a plant or plant cell an ARP6 polypeptide-encoding nucleic acid; and
(ii) cultivating the plant cell under conditions promoting plant growth and development.

The nucleic acid of (i) may be any of the nucleic acids capable of encoding an ARP6 polypeptide as defined herein.

More specifically, the present invention also provides a method for the production of transgenic plants having enhanced yield-related traits, in particular increased yield which method comprises:
(i) introducing and expressing in a plant or plant cell a POP polypeptide-encoding nucleic acid; and
(ii) cultivating the plant cell under conditions promoting plant growth and development.

The nucleic acid of (i) may be any of the nucleic acids capable of encoding a POP polypeptide as defined herein.

More specifically, the present invention also provides a method for the production of transgenic plants having enhanced yield-related traits, particularly increased (seed) yield and/or harvest index, which method comprises:
(i) introducing and expressing in a plant or plant cell a CRL polypeptide-encoding nucleic acid; and
(ii) cultivating the plant cell under conditions promoting plant growth and development.

The nucleic acid of (i) may be any of the nucleic acids capable of encoding a CRL polypeptide as defined herein.

The nucleic acid may be introduced directly into a plant cell or into the plant itself (including introduction into a tissue, organ or any other part of a plant). According to a preferred feature of the present invention, the nucleic acid is preferably introduced into a plant by transformation. The term "transformation" is described in more detail in the "definitions" section herein.

The genetically modified plant cells can be regenerated via all methods with which the skilled worker is familiar. Suitable methods can be found in the abovementioned publications by S.D. Kung and R. Wu, Potrykus or Höfgen and Willmitzer.

Generally after transformation, plant cells or cell groupings are selected for the presence of one or more markers which are encoded by plant-expressible genes co-transferred with the gene of interest, following which the transformed material is regenerated into a whole plant. To select transformed plants, the plant material obtained in the transformation is, as a rule, subjected to selective conditions so that transformed plants can be distinguished from untransformed plants. For example, the seeds obtained in the above-described manner can be planted and, after an initial growing period, subjected to a suitable selection by spraying. A further possibility consists in growing the seeds, if appropriate after sterilization, on agar plates using a suitable selection agent so that only the transformed seeds can grow into plants. Alternatively, the transformed plants are screened for the presence of a selectable marker such as the ones described above.

Following DNA transfer and regeneration, putatively transformed plants may also be evaluated, for instance using Southern analysis, for the presence of the gene of interest, copy number and/or genomic organisation. Alternatively or additionally, expression levels of the newly introduced DNA may be monitored using Northern and/or Western analysis, both techniques being well known to persons having ordinary skill in the art.

The generated transformed plants may be propagated by a variety of means, such as by clonal propagation or classical breeding techniques. For example, a first generation (or T1) transformed plant may be selfed and homozygous second-generation (or T2) transformants selected, and the T2 plants may then further be propagated through classical breeding techniques. The generated transformed organisms may take a variety of forms. For example, they may be chimeras of transformed cells and non-transformed cells; clonal transformants (e.g., all cells transformed to contain the expression cassette); grafts of transformed and untransformed tissues (e.g., in plants, a transformed rootstock grafted to an untransformed scion).

The present invention clearly extends to any plant cell or plant produced by any of the methods described herein, and to all plant parts and propagules thereof. The present invention extends further to encompass the progeny of a primary transformed or transfected cell, tissue, organ or whole plant that has been produced by any of the aforementioned methods, the only requirement being that progeny exhibit the same genotypic and/or phenotypic characteristic(s) as those produced by the parent in the methods according to the invention.

The invention also includes host cells containing an isolated nucleic acid encoding an alfin-like polypeptide, or a YRP polypeptide, or a BRXL polypeptide, or a silky-1-like polypeptide, or an ARP6 polypeptide, or a POP polypeptide, or a CRL polypeptide, as defined hereinabove. Preferred host cells according to the invention are plant cells. Host plants for the nucleic acids or the vector used in the method according to the invention, the expression cassette or construct or vector are, in principle, advantageously all plants, which are capable of synthesizing the polypeptides used in the inventive method.

The methods of the invention are advantageously applicable to any plant. Plants that are particularly useful in the methods of the invention include all plants which belong to the superfamily Viridiplantae, in particular monocotyledonous and dicotyledonous plants including fodder or forage legumes, ornamental plants, food crops, trees or shrubs. According to a preferred embodiment of the present invention, the plant is a crop plant. Examples of crop plants include soybean, sunflower, canola, alfalfa, rapeseed, linseed, cotton, tomato, potato and tobacco. Further preferably, the plant is a monocotyledonous plant. Examples of monocotyledonous plants include sugarcane. More preferably the plant is a cereal. Examples of cereals include rice, maize, wheat, barley, millet, rye, triticale, sorghum, emmer, spelt, secale, einkorn, teff, milo and oats.

The invention also extends to harvestable parts of a plant such as, but not limited to seeds, leaves, fruits, flowers, stems, roots, rhizomes, tubers and bulbs, which harvestable parts comprise a recombinant nucleic acid encoding an alfin-like polypeptide, or a YRP polypeptide, or a BRXL polypeptide, or a silky-1-like polypeptide, or an ARP6 polypeptide, or a POP polypeptide, or a CRL polypeptide. The invention furthermore relates to products derived, preferably directly derived, from a harvestable part of such a plant, such as dry pellets or powders, oil, fat and fatty acids, starch or proteins.

According to a preferred feature of the invention, the modulated expression is increased expression. Methods for increasing expression of nucleic acids or genes, or gene products, are well documented in the art and examples are provided in the definitions section.

As mentioned above, a preferred method for modulating expression of a nucleic acid encoding an alfin-like polypeptide, or a YRP polypeptide, or a BRXL polypeptide, or a silky-1-like polypeptide, or an ARP6 polypeptide, or a POP polypeptide, or a CRL polypeptide, is by introducing and expressing in a plant a nucleic acid encoding an alfin-like polypeptide, or a YRP polypeptide, or a BRXL polypeptide, or a silky-1-like polypeptide, or an ARP6 polypeptide, or a POP polypeptide, or a CRL polypeptide; however the effects of performing the method, i.e. enhancing abiotic stress tolerance may also be achieved using other well known techniques, including but not limited to T-DNA activation tagging, TILLING, homologous recombination. A description of these techniques is provided in the definitions section.

The present invention also encompasses use of nucleic acids encoding alfin-like polypeptides, or YRP polypeptides, or silky-1-like polypeptides, as described herein and use of these alfin-like polypeptides in enhancing any of the aforementioned abiotic stresses in plants.

The present invention also encompasses use of nucleic acid sequences encoding BRXL polypeptides as described herein and use of these BRXL polypeptides in increasing any of the aforementioned yield-related traits in plants, under normal growth conditions, under abiotic stress growth (preferably osmotic stress growth conditions) conditions, and under growth conditions of reduced nutrient availability, preferably under conditions of reduced nitrogen availability.

The present invention also encompasses use of nucleic acids encoding ARP6 polypeptides as described herein and use of these ARP6 polypeptides, or POP polypeptides, or CRL polypeptides, in enhancing any of the aforementioned yield-related traits in plants.

Nucleic acids encoding alfin-like polypeptide, or YRP polypeptide, or BRXL polypeptide, or silky-1-like polypeptide, or ARP6 polypeptide, or POP polypeptide, or CRL polypeptide, described herein, or the alfin-like polypeptides, or YRP polypeptides, or BRXL polypeptides, or silky-1-like polypeptides, or ARP6 polypeptides, or POP polypeptides, or CRL polypeptides, themselves, may find use in breeding programmes in which a DNA marker is identified which may be genetically linked to agene encoding alfin-like polypeptides, or YRP polypeptides, or BRXL polypeptides, or silky-1-like polypeptides, or ARP6 polypeptides, or POP polypeptides, or CRL polypeptides. The nucleic acids/genes, or the alfin-like polypeptides, or YRP polypeptides, or BRXL polypeptides, or silky-1-like polypeptides, or ARP6 polypeptides, or POP polypeptides, or CRL polypeptides, themselves may be used to define a molecular marker. This DNA or protein marker may then be used in breeding programmes to select plants having enhanced abiotic stress tolerance as defined hereinabove in the methods of the invention.

Allelic variants of a nucleic acid/gene encoding alfin-like polypeptides, or YRP polypeptides, or BRXL polypeptides, or silky-1-like polypeptides, or ARP6 polypeptides, or POP polypeptides, or CRL polypeptides, may also find use in marker-assisted breeding programmes. Such breeding programmes sometimes require introduction of allelic variation by mutagenic treatment of the plants, using for example EMS mutagenesis; alternatively, the programme may start with a collection of allelic variants of so called "natural" origin caused unintentionally. Identification of allelic variants then takes place, for example, by PCR. This is followed by a step for selection of superior allelic variants of the sequence in question and which give enhanced stress tolerance which may be manifested as increased yield. Selection is typically carried out by monitoring growth performance of plants containing different allelic variants of the sequence in question. Growth performance may be monitored in a greenhouse or in the field. Further optional steps include crossing plants in which the superior allelic variant was identified with another plant. This could be used, for example, to make a combination of interesting phenotypic features.

Nucleic acids encoding alfin-like polypeptides, or YRP polypeptides, or BRXL polypeptides, or silky-1-like polypeptides, or ARP6 polypeptides, or POP polypeptides, or CRL polypeptides, may also be used as probes for genetically and physically mapping the genes that they are a part of, and as markers for traits linked to those genes. Such information may be useful in plant breeding in order to develop lines with desired phenotypes. Such use of nucleic acids encoding alfin-like polypeptides, or YRP polypeptides, or BRXL polypeptides, or silky-1-like polypeptides, or ARP6 polypeptides, or POP polypeptides, or CRL polypeptides, requires only a nucleic acid sequence of at least 15 nucleotides in length. The nucleic acids encoding alfin-like polypeptides, or YRP polypeptides, or BRXL polypeptides, or silky-1-like polypeptides, or ARP6 polypeptides, or POP polypeptides, or CRL polypeptides, may be used as restriction fragment length polymorphism (RFLP) markers. Southern blots (Sambrook J, Fritsch EF and Maniatis T (1989) Molecular Cloning, A Laboratory Manual) of restriction-digested plant genomic DNA may be probed with the nucleic acids encoding alfin-like polypeptides, or YRP polypeptides, or BRXL polypeptides, or silky-1-like polypeptides, or ARP6 polypeptides, or POP polypeptides, or CRL polypeptides. The resulting banding patterns may then be subjected to genetic analyses using computer programs such as MapMaker (Lander et al. (1987) Genomics 1: 174-181) in order to construct a genetic map. In addition, the nucleic acids may be used to probe Southern blots containing restriction endonuclease-treated genomic DNAs of a set of individuals representing parent and progeny of a defined genetic cross. Segregation of the DNA polymorphisms is noted and used to calculate the position of the nucleic acid encoding alfin-like polypeptides, or YRP polypeptides, or BRXL polypeptides, or silky-1-like polypeptides, or ARP6 polypeptides, or POP polypeptides, or CRL polypeptides, in the genetic map previously obtained using this population (Botstein et al. (1980) Am. J. Hum. Genet. 32:314-331).

The production and use of plant gene-derived probes for use in genetic mapping is described in Bernatzky and Tanksley (1986) Plant Mol. Biol. Reporter 4: 37-41. Numerous publications describe genetic mapping of specific cDNA clones using the methodology outlined above or variations thereof. For example, F2 intercross populations, backcross populations, randomly mated populations, near isogenic lines, and other sets of individuals may be used for mapping. Such methodologies are well known to those skilled in the art.

The nucleic acid probes may also be used for physical mapping (i.e., placement of sequences on physical maps; see Hoheisel et al. In: Non-mammalian Genomic Analysis: A Practical Guide, Academic press 1996, pp. 319-346, and references cited therein).

In another embodiment, the nucleic acid probes may be used in direct fluorescence in situ hybridisation (FISH) mapping (Trask (1991) Trends Genet. 7:149-154). Although current methods of FISH mapping favour use of large clones (several kb to several hundred kb; see Laan et al. (1995) Genome Res. 5:13-20), improvements in sensitivity may allow performance of FISH mapping using shorter probes.

A variety of nucleic acid amplification-based methods for genetic and physical mapping may be carried out using the nucleic acids. Examples include allele-specific amplification (Kazazian (1989) J. Lab. Clin. Med 11:95-96), polymorphism of PCR-amplified fragments (CAPS; Sheffield et al. (1993) Genomics 16:325-332), allele-specific ligation (Landegren et al. (1988) Science 241:1077-1080), nucleotide extension reactions (Sokolov (1990) Nucleic Acid Res. 18:3671), Radiation Hybrid Mapping (Walter et al. (1997) Nat. Genet. 7:22-28) and Happy Mapping (Dear and Cook (1989) Nucleic Acid Res. 17:6795-6807). For these methods, the sequence of a nucleic acid is used to design and produce primer pairs for use in the amplification reaction or in primer extension reactions. The design of such primers is well known to those skilled in the art. In methods employing PCR-based genetic mapping, it may be necessary to identify DNA sequence differences between the parents of the mapping cross in the region corresponding to the instant nucleic acid sequence. This, however, is generally not necessary for mapping methods.

The methods according to the present invention result in plants having enhanced enhanced abiotic stress tolerance and/or enhanced yield-related traits, as described hereinbefore. These traits may also be combined with other economically advantageous traits, such as further abiotic or biotic stress tolerance-enhancing traits, enhanced yield-related traits, enhanced yield-related traits, tolerance to herbicides, insectides, traits modifying various architectural features and/or biochemical and/or physiological features.

### Items

### 1. Alfin-like polypeptides

1. Method for enhancing abiotic stress tolerance in plants by modulating expression in a plant of a nucleic acid encoding an alfin-like polypeptide or an orthologue or paralogue thereof.
2. Method according to item 1, wherein said modulated expression is effected by introducing and expressing in a plant a nucleic acid encoding alfin-like polypeptide.
3. Method according to items 2 or 3, wherein said nucleic acid encoding an alfin-like polypeptide encodes any one of the proteins listed in Table A1 or is a portion of such a nucleic acid, or a nucleic acid capable of hybridising with such a nucleic acid.
4. Method according to any one of items 1 to 4, wherein said nucleic acid sequence encodes an orthologue or paralogue of any of the proteins given in Table A1.
5. Method according to items 3 or 4, wherein said nucleic acid is operably linked to a constitutive promoter, preferably to a GOS2 promoter, most preferably to a GOS2 promoter from rice.
6. Method according to any one of items 1 to 5, wherein said nucleic acid encoding an alfin-like polypeptide is of Allium cepa.
7. Plant or part thereof, including seeds, obtainable by a method according to any one of items 1 to 6, wherein said plant or part thereof comprises a recombinant nucleic acid encoding an alfin-like polypeptide.
8. Construct comprising:
   (i) nucleic acid encoding an alfin-like polypeptide as defined in items 1 or 2;
   (ii) one or more control sequences capable of driving expression of the nucleic acid sequence of (a); and optionally
   (iii) a transcription termination sequence.
9. Construct according to item 9, wherein one of said control sequences is a constitutive promoter, preferably a GOS2 promoter, most preferably a GOS2 promoter from rice.
10. Use of a construct according to item 8 or 9 in a method for making plants having increased abiotic stress tolerance relative to control plants.
11. Plant, plant part or plant cell transformed with a construct according to item 8 or 9.
12. Method for the production of a transgenic plant having increased abiotic stress tolerance relative to control plants, comprising:
   (i) introducing and expressing in a plant a nucleic acid encoding an alfin-like polypeptide; and
   (ii) cultivating the plant cell under conditions promoting abiotic stress.
13. Transgenic plant having abiotic stress tolerance, relative to control plants, resulting from modulated expression of a nucleic acid encoding an alfin-like polypeptide, or a transgenic plant cell derived from said transgenic plant.
14. Transgenic plant according to item 7, 11 or 13, or a transgenic plant cell derived thereof, wherein said plant is a crop plant or a monocot or a cereal, such as rice, maize, wheat, barley, millet, rye, triticale, sorghum, sugarcane, emmer, spelt, secale, einkorn, teff, milo and oats.
15. Harvestable parts of a plant according to item 14, wherein said harvestable parts are preferably shoot biomass and/or seeds.
16. Products derived from a plant according to item 14 and/or from harvestable parts of a plant according to item 15.
17. Use of a nucleic acid encoding an alfin-like polypeptide in increasing yield, particularly in increasing abiotic stress tolerance, relative to control plants.

### 2. YRP polypeptides

1. Method for enhancing abiotic stress tolerance in plants by modulating expression in a plant of a nucleic acid encoding a YRP polypeptide or an orthologue or paralogue thereof.
2. Method according to item 1, wherein said modulated expression is effected by introducing and expressing in a plant a nucleic acid encoding YRP polypeptide.
3. Method according to items 2 or 3, wherein said nucleic acid encoding a YRP polypeptide encodes any one of the proteins listed in Table A2 or is a portion of such a nucleic acid, or a nucleic acid capable of hybridising with such a nucleic acid.
4. Method according to any one of items 1 to 4, wherein said nucleic acid sequence encodes an orthologue or paralogue of any of the proteins given in Table A2.
5. Method according to items 3 or 4, wherein said nucleic acid is operably linked to a constitutive promoter, preferably to a GOS2 promoter, most preferably to a GOS2 promoter from rice.
6. Method according to any one of items 1 to 5, wherein said nucleic acid encoding a YRP polypeptide is of Hordeum Vulgare.
7. Plant or part thereof, including seeds, obtainable by a method according to any one of items 1 to 6, wherein said plant or part thereof comprises a recombinant nucleic acid encoding a YRP polypeptide.
8. Construct comprising:
   (i) nucleic acid encoding a YRP polypeptide as defined in items 1 or 2;
   (ii) one or more control sequences capable of driving expression of the nucleic acid sequence of (a); and optionally
   (iii) a transcription termination sequence.
9. Construct according to item 9, wherein one of said control sequences is a constitutive promoter, preferably a GOS2 promoter, most preferably a GOS2 promoter from rice.
10. Use of a construct according to item 8 or 9 in a method for making plants having increased abiotic stress tolerance relative to control plants.
11. Plant, plant part or plant cell transformed with a construct according to item 8 or 9.
12. Method for the production of a transgenic plant having increased abiotic stress tolerance relative to control plants, comprising:
   (i) introducing and expressing in a plant a nucleic acid encoding a YRP polypeptide; and
   (ii) cultivating the plant cell under conditions promoting abiotic stress.
13. Transgenic plant having abiotic stress tolerance, relative to control plants, resulting from modulated expression of a nucleic acid encoding a YRP polypeptide, or a transgenic plant cell derived from said transgenic plant.
14. Transgenic plant according to item 7, 11 or 13, or a transgenic plant cell derived thereof, wherein said plant is a crop plant or a monocot or a cereal, such as rice, maize, wheat, barley, millet, rye, triticale, sorghum, sugarcane, emmer, spelt, secale, einkorn, teff, milo and oats.
15. Harvestable parts of a plant according to item 14, wherein said harvestable parts are preferably shoot biomass and/or seeds.
16. Products derived from a plant according to item 14 and/or from harvestable parts of a plant according to item 15.
17. Use of a nucleic acid encoding a YRP polypeptide in increasing yield, particularly in increasing abiotic stress tolerance, relative to control plants.

### 3. Brevis Radix-like (BRXL) polypeptides

1) A method for increasing yield-related traits in plants relative to control plants, comprising increasing expression in a plant of a nucleic acid sequence encoding a Breves Radix-like (BRXL) polypeptide, which BRXL polypeptide comprises at least two BRX domains with an InterPro entry IPR013591 DZC domain (PFAM entry PF08381 DZC), and optionally selecting for plants having increased yield-related traits.
2) Method according to item 1, wherein said BRXL polypeptide comprises (i) in increasing order of preference at least 50%, 55%, 60%, 65%, 70%, 75%, 80%, 85%, 90%, 95%, 98%, 99% or more amino acid sequence identity to a BRX domain as represented by SEQ ID NO: 65; and (ii) in increasing order of preference at least 50%, 55%, 60%, 65%, 70%, 75%, 80%, 85%, 90%, 95%, 98%, 99% or more amino acid sequence identity to a BRX domain as represented by SEQ ID NO: 82.
3) Method according to item 1 or 2, wherein said BRXL polypeptide comprises (i) in increasing order of preference at least 50%, 55%, 60%, 65%, 70%, 75%, 80%, 85%, 90%, 95%, 98%, 99% or more amino acid sequence identity to a Conserved Domain 1 (comprising a BRX domain) as represented by SEQ ID NO: 83; and (ii) in increasing order of preference at least 50%, 55%, 60%, 65%, 70%, 75%, 80%, 85%, 90%, 95%, 98%, 99% or more amino acid sequence identity to a Conserved Domain 2 (comprising a BRX domain) as represented by SEQ ID NO: 84.
4) Method according to item 3, wherein said BRXL polypeptide comprises (1) in increasing order of preference at least 50%, 55%, 60%, 65%, 70%, 75%, 80%, 85%, 90%, 95%, 98%, 99% or more amino acid sequence identity to a Conserved domain 3 as represented by SEQ ID NO: 85; and (1) in increasing order of preference at least 50%, 55%, 60%, 65%, 70%, 75%, 80%, 85%, 90%, 95%, 98%, 99% or more amino acid sequence identity to a Conserved domain 4 as represented by SEQ ID NO: 86.
5) Method according to any preceding item, wherein said BRXL polypeptide has in increasing order of preference at least 40%, 45%, 50%, 55%, 60%, 65%, 70%, 75%, 80%, 85%, 90%, 95%, 98%, 99% or more amino acid sequence identity to a polypeptide as represented by SEQ ID NO: 18, or to any of the polypeptide sequences given in Table A herein.
6) Method according to any preceding item, wherein said BRXL polypeptide interacts with itself or with another BRLX polypeptide in a yeast two hybrid assay.
7) Method according to any preceding item, wherein said nucleic acid sequence encoding a BRXL polypeptide is represented by any one of the nucleic acid sequence SEQ ID NOs given in Table A3 or a portion thereof, or a sequence capable of hybridising with any one of the nucleic acid sequences SEQ ID NOs given in Table A3, or to a complement thereof.
8) Method according to any preceding item, wherein said nucleic acid sequence encodes an orthologue or paralogue of any of the polypeptide sequence SEQ ID NOs given in Table A3.
9) Method according to any preceding item, wherein said increased expression is effected by any one or more of: T-DNA activation tagging, TILLING, or homologous recombination.
10) Method according to any preceding item, wherein said increased expression is effected by introducing and expressing in a plant a nucleic acid sequence encoding a BRXL polypeptide.
11) Method according to any preceding item, wherein said increased yield-related trait is one or more of: increased plant height, and increased Thousand Kernel Weight (TKW).
12) Method according to any preceding item, wherein said nucleic acid sequence is operably linked to a constitutive promoter.
13) Method according to item 12, wherein said constitutive promoter is a GOS2 promoter, preferably a GOS2 promoter from rice, most preferably a GOS2 sequence as represented by SEQ ID NO: 87.
14) Method according to any preceding item, wherein said nucleic acid sequence encoding a BRXL polypeptide is from a plant, further preferably from a dicotyledonous plant, more preferably from the family Salicaceae, most preferably the nucleic acid sequence is from Populus trichocarpa.
15) Plants, parts thereof (including seeds), or plant cells obtainable by a method according to any preceding item, wherein said plant, part or cell thereof comprises an isolated nucleic acid transgene encoding a BRXL polypeptide.
16)An isolated nucleic acid molecule selected from:
   (i) a nucleic acid sequence as represented by any one of SEQ ID NO: 75, SEQ ID NO: 77, or SEQ ID NO: 79;
   (ii) the complement of a nucleic acid sequence as represented by any one of SEQ ID NO: 75, SEQ ID NO: 77, or SEQ ID NO: 79;
   (iii) a nucleic acid sequence encoding a BRXL polypeptide having, in increasing order of preference, at least 50%, 55%, 60%, 65%, 70%, 75%, 80%, 85%, 90%, 95%, 96%, 97%, 98%, 99% or more amino acid sequence identity to the polypeptide sequence represented by any one of SEQ ID NO: 76, SEQ ID NO: 78, or SEQ ID NO: 80.
17)An isolated polypeptide selected from:
   (i) a polypeptide sequence as represented by any one of SEQ ID NO: 76, SEQ ID NO: 78, or SEQ ID NO: 80;
   (ii) a polypeptide sequence having, in increasing order of preference, at least 50%, 55%, 60%, 65%, 70%, 75%, 80%, 85%, 90%, 95%, 96%, 97%, 98%, 99% or more amino acid sequence identity to a polypeptide sequence as represented by any one of SEQ ID NO: 76, SEQ ID NO: 78, or SEQ ID NO: 80;
   (iii) derivatives of any of the polypeptide sequences given in (i) or (ii) above.
18) Construct comprising:
   (a) a nucleic acid sequence encoding a BRXL polypeptide as defined in any one of items 1 to 8,or 16;
   (b) one or more control sequences capable of driving expression of the nucleic acid sequence of (a); and optionally
   (c) a transcription termination sequence.
19) Construct according to item 18 wherein said control sequence is a consitituve promoter.
20) Construct according to item 19 wherein said consitituve promoter is a GOS2 promoter, preferably a GOS2 promoter from rice, most preferably a GOS2 sequence as represented by SEQ ID NO: 87.
21) Use of a construct according to any one of items 18 to 20 in a method for making plants having increased yield-related traits relative to control plants, which increased yield-related traits are one or more of: increased plant height, increased seed yield per plant, increased number of filled seeds, and increased Thousand Kernel Weight (TKW).
22) Plant, plant part or plant cell transformed with a construct according to any one of items 18 to 20.
23) Method for the production of transgenic plants having increased yield-related traits relative to control plants, comprising:
   (i) introducing and expressing in a plant, plant part, or plant cell, a nucleic acid sequence encoding a BRXL polypeptide as defined in any one of items 1 to 8, or 16; and
   (ii) cultivating the plant cell, plant part, or plant under conditions promoting plant growth and development.
24)Transgenic plant having increased yield-related traits relative to control plants, resulting from increased expression of an isolated nucleic acid sequence encoding a BRXL polypeptide as defined in any one of items 1 to 8, or 16, or a transgenic plant cell or transgenic plant part derived from said transgenic plant.
25)Transgenic plant according to item 14, 22, or 24, wherein said plant is a crop plant or a monocot or a cereal, such as rice, maize, wheat, barley, millet, rye, triticale, sorghum and oats, or a transgenic plant cell derived from said transgenic plant.
26) Harvestable parts comprising an isolated nucleic acid sequence encoding a BRXL polypeptide, of a plant according to item 25, wherein said harvestable parts are preferably seeds.
27) Products derived from a plant according to item 25 and/or from harvestable parts of a plant according to item 26.
28) Use of a nucleic acid sequence encoding a BRXL polypeptide as defined in any one of items 1 to 8, or 16, in increasing yield-related traits, comprising one or more of: increased plant height, and increased Thousand Kernel Weight (TKW).

### 4. silky1-like polypeptides

a) Method for enhancing abiotic stress tolerance in plants by modulating expression in a plant of a nucleic acid encoding a silky-1-like polypeptide or an orthologue or paralogue thereof.
b) Method according to item 1, wherein said modulated expression is effected by introducing and expressing in a plant a nucleic acid encoding silky-1-like polypeptide.
c) Method according to items 2 or 3, wherein said nucleic acid encoding a silky-1-like polypeptide encodes any one of the proteins listed in Table A4 or is a portion of such a nucleic acid, or a nucleic acid capable of hybridising with such a nucleic acid.
d) Method according to any one of items 1 to 4, wherein said nucleic acid sequence encodes an orthologue or paralogue of any of the proteins given in Table A4.
e) Method according to items 3 or 4, wherein said nucleic acid is operably linked to a constitutive promoter, preferably to a GOS2 promoter, most preferably to a GOS2 promoter from rice.
f) Method according to any one of items 1 to 5, wherein said nucleic acid encoding a silky-1-like polypeptide is of Populus trichocarpa.
g) Plant or part thereof, including seeds, obtainable by a method according to any one of items 1 to 6, wherein said plant or part thereof comprises a recombinant nucleic acid encoding a silky-1-like polypeptide.
h) Construct comprising:
   (i) nucleic acid encoding a silky-1-like polypeptide as defined in items 1 or 2;
   (ii) one or more control sequences capable of driving expression of the nucleic acid sequence of (a); and optionally
   (iii) a transcription termination sequence.
i) Construct according to item 9, wherein one of said control sequences is a constitutive promoter, preferably a GOS2 promoter, most preferably a GOS2 promoter from rice.
j) Use of a construct according to item 8 or 9 in a method for making plants having increased abiotic stress tolerance relative to control plants.
k) Plant, plant part or plant cell transformed with a construct according to item 8 or 9.
l) Method for the production of a transgenic plant having increased abiotic stress tolerance relative to control plants, comprising:
   (i) introducing and expressing in a plant a nucleic acid encoding a silky-1-like polypeptide; and
   (ii) cultivating the plant cell under conditions promoting abiotic stress.
m) Transgenic plant having abiotic stress tolerance, relative to control plants, resulting from modulated expression of a nucleic acid encoding a silky-1-like polypeptide, or a transgenic plant cell derived from said transgenic plant.
n) Transgenic plant according to item 7, 11 or 13, or a transgenic plant cell derived thereof, wherein said plant is a crop plant or a monocot or a cereal, such as rice, maize, wheat, barley, millet, rye, triticale, sorghum, sugarcane, emmer, spelt, secale, einkorn, teff, milo and oats.
o) Harvestable parts of a plant according to item 14, wherein said harvestable parts are preferably shoot biomass and/or seeds.
p) Products derived from a plant according to item 14 and/or from harvestable parts of a plant according to item 15.
q) Use of a nucleic acid encoding a silky-1-like polypeptide in increasing yield, particularly in increasing abiotic stress tolerance, relative to control plants.

### 5. ARP6 polypeptides

1. A method for enhancing yield-related traits in plants relative to control plants, comprising modulating expression in a plant of a nucleic acid encoding an ARP6 polypeptide.
2. Method according to item 1, wherein said ARP6 polypeptide has in increasing order of preference at least 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% overall sequence identity to the amino acid represented by any one of SEQ ID NO: 102, SEQ ID NO: 104 , SEQ ID NO: 106, SEQ ID NO: 108, SEQ ID NO: 110, and SEQ ID NO: 112.
3. Method according to item 1 or 2, wherein said modulated expression is effected by introducing and expressing in a plant a nucleic acid encoding an ARP6 polypeptide.
4. Method according to any one of items 1 to 3, wherein said nucleic acid encoding an ARP6 polypeptide encodes any one of the proteins listed in Table A5 or is a portion of such a nucleic acid, or a nucleic acid capable of hybridising with such a nucleic acid.
5. Method according to any one of items 1 to 4, wherein said nucleic acid sequence encodes an orthologue or paralogue of any of the proteins given in Table A5.
6. Method according to any preceding item, wherein said enhanced yield-related traits comprise increased yield, preferably increased biomass and/or increased seed yield relative to control plants.
7. Method according to any one of items 1 to 6, wherein said enhanced yield-related traits are obtained under non-stress conditions.
8. Method according to any one of items 1 to 6, wherein said enhanced yield-related traits are obtained under conditions of drought stress, salt stress or nitrogen deficiency.
9. Method according to any one of items 3 to 8, wherein said nucleic acid is operably linked to a constitutive promoter, preferably to a GOS2 promoter, most preferably to a GOS2 promoter from rice.
10. Method according to any one of items 1 to 9, wherein said nucleic acid encoding an ARP6 polypeptide is of plant origin, preferably from a dicotyledonous plant, further preferably from the family Brassicaceae, more preferably from the genus Arabidopsis, most preferably from Arabidopsis thaliana.
11. Plant or part thereof, including seeds, obtainable by a method according to any one of items 1 to 10, wherein said plant or part thereof comprises a recombinant nucleic acid encoding an ARP6 polypeptide.
12. Construct comprising:
   (ii) nucleic acid encoding an ARP6 polypeptide as defined in items 1 or 2;
   (iii) one or more control sequences capable of driving expression of the nucleic acid sequence of (a); and optionally
   (iv) a transcription termination sequence.
13. Construct according to item 12, wherein one of said control sequences is a constitutive promoter, preferably a GOS2 promoter, most preferably a GOS2 promoter from rice.
14. Use of a construct according to item 12 or 13 in a method for making plants having increased yield, particularly increased biomass and/or increased seed yield relative to control plants.
15. Plant, plant part or plant cell transformed with a construct according to item 12 or 13.
16. Method for the production of a transgenic plant having increased yield, particularly increased biomass and/or increased seed yield relative to control plants, comprising:
   (i) introducing and expressing in a plant a nucleic acid encoding an ARP6 polypeptide as defined in item 1 or 2; and
   (ii) cultivating the plant cell under conditions promoting plant growth and development.
17. Transgenic plant having increased yield, particularly increased biomass and/or increased seed yield, relative to control plants, resulting from modulated expression of a nucleic acid encoding an ARP6 polypeptide as defined in item 1 or 2, or a transgenic plant cell derived from said transgenic plant.
18. Transgenic plant according to item 11, 15 or 17, or a transgenic plant cell derived thereof, wherein said plant is a crop plant or a monocot or a cereal, such as rice, maize, wheat, barley, millet, rye, triticale, sorghum emmer, spelt, secale, einkorn, teff, milo and oats.
19. Harvestable parts of a plant according to item 18, wherein said harvestable parts are preferably shoot biomass and/or seeds.
20. Products derived from a plant according to item 18 and/or from harvestable parts of a plant according to item 19.
21. Use of a nucleic acid encoding an ARP6 polypeptide in increasing yield, particularly in increasing seed yield and/or shoot biomass in plants, relative to control plants.

### 6. POP polypeptides

1. A method for enhancing yield-related traits in plants relative to control plants, comprising modulating expression in a plant of a nucleic acid encoding a prolyl oligopeptidase (POP) polypeptide, wherein said POP polypeptide comprises a Peptidase_S9 domain (Pfam entry PF00326) and preferably also a DPPIV_N domain (Pfam entry PF00930).
2. Method according to item 1, wherein said POP polypeptide comprises one or more of the motifs 1 to 14 (SEQ ID NO: 118 to SEQ ID NO: 131).
3. Method according to item 1 or 2, wherein said modulated expression is effected by introducing and expressing in a plant a nucleic acid encoding a POP polypeptide.
4. Method according to any one of items 1 to 3, wherein said nucleic acid encoding a POP polypeptide encodes any one of the proteins listed in Table A6 or is a portion of such a nucleic acid, or a nucleic acid capable of hybridising with such a nucleic acid.
5. Method according to any one of items 1 to 4, wherein said nucleic acid sequence encodes an orthologue or paralogue of any of the proteins given in Table A6.
6. Method according to any preceding item, wherein said enhanced yield-related traits comprise increased yield, preferably increased biomass and/or increased seed yield, and or modified flowering time relative to control plants.
7. Method according to any one of items 1 to 6, wherein said enhanced yield-related traits are obtained under non-stress conditions.
8. Method according to any one of items 3 to 7, wherein said nucleic acid is operably linked to a constitutive promoter, preferably to a GOS2 promoter, most preferably to a GOS2 promoter from rice.
9. Method according to any one of items 1 to 8, wherein said nucleic acid encoding a POP polypeptide is of plant origin, preferably from a dicotyledonous plant, further preferably from the family Brassicaceae, more preferably from the genus Arabidopsis, most preferably from Arabidopsis thaliana.
10. Plant or part thereof, including seeds, obtainable by a method according to any one of items 1 to 9, wherein said plant or part thereof comprises a recombinant nucleic acid encoding a POP polypeptide.
11. Construct comprising:
   (i) nucleic acid encoding a POP polypeptide as defined in items 1 or 2;
   (ii) one or more control sequences capable of driving expression of the nucleic acid sequence of (a); and optionally
   (iii) a transcription termination sequence.
12. Construct according to item 11, wherein one of said control sequences is a constitutive promoter, preferably a GOS2 promoter, most preferably a GOS2 promoter from rice.
13. Use of a construct according to item 11 or 12 in a method for making plants having increased yield, particularly increased biomass and/or increased seed yield relative to control plants.
14. Use of a construct according to item 11 or 12 in a method for making plants having modified flowering time relative to control plants.
15. Plant, plant part or plant cell transformed with a construct according to item 11 or 12.
16. Method for the production of a transgenic plant having increased yield and/or modified flowering time, particularly increased biomass and/or increased seed yield relative to control plants, comprising:
   (i) introducing and expressing in a plant a nucleic acid encoding a POP polypeptide as defined in item 1 or 2; and
   (ii) cultivating the plant cell under conditions promoting plant growth and development.
17. Transgenic plant having increased yield, particularly increased biomass and/or increased seed yield, relative to control plants, resulting from modulated expression of a nucleic acid encoding a POP polypeptide as defined in item 1 or 2, or a transgenic plant cell derived from said transgenic plant.
18. Transgenic plant according to item 10, 15 or 17, or a transgenic plant cell derived thereof, wherein said plant is a crop plant or a monocot or a cereal, such as rice, maize, wheat, barley, millet, rye, triticale, sorghum emmer, spelt, secale, einkorn, teff, milo and oats.
19. Harvestable parts of a plant according to item 18, wherein said harvestable parts are preferably shoot biomass and/or seeds.
20. Products derived from a plant according to item 18 and/or from harvestable parts of a plant according to item 18.
21. Use of a nucleic acid encoding a POP polypeptide in increasing yield, particularly in increasing seed yield and/or shoot biomass in plants, relative to control plants.
22. Use of a nucleic acid encoding a POP polypeptide for modifying flowering time of plants, relative to control plants.

### 7. CRL polypeptides

1. A method for enhancing yield-related traits in plants relative to control plants, comprising modulating expression in a plant of a nucleic acid encoding a CRL polypeptide, wherein said CRL polypeptide comprises a DUF206 domain.
2. Method according to item 1, wherein said CRL polypeptide comprises a motif having in increasing order of preference at least 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% sequence identity to any one or more of the following motifs:
   (i) Motif 15: EQAFWRxPXKPFRQR (SEQ ID NO: 207);
   (ii) Motif 16: NFCDR (SEQ ID NO: 208);
   (iii) Motif 17: RGKRCLYEGS (SEQ ID NO: 209);
   (iv) Motif 18: QVWGxKXGPYEFK (SEQ ID NO: 210);
   wherein X represents any amino acid.
3. Method according to item 1 or 2, wherein said modulated expression is effected by introducing and expressing in a plant a nucleic acid encoding a CRL polypeptide.
4. Method according to any one of items 1 to 3, wherein said nucleic acid encoding a CRL polypeptide encodes any one of the proteins listed in Table A7 or is a portion of such a nucleic acid, or a nucleic acid capable of hybridising with such a nucleic acid.
5. Method according to any one of items 1 to 4, wherein said nucleic acid sequence encodes an orthologue or paralogue of any of the proteins given in Table A7.
6. Method according to any preceding item, wherein said enhanced yield-related traits comprise increased yield, preferably increased biomass and/or increased seed yield relative to control plants.
7. Method according to any one of items 1 to 6, wherein said enhanced yield-related traits are obtained under non-stress conditions.
8. Method according to any one of items 1 to 6, wherein said enhanced yield-related traits are obtained under conditions of drought stress, salt stress or nitrogen deficiency.
9. Method according to any one of items 3 to 8, wherein said nucleic acid is operably linked to a constitutive promoter, preferably to a GOS2 promoter, most preferably to a GOS2 promoter from rice.
10. Method according to any one of items 1 to 9, wherein said nucleic acid encoding a CRL polypeptide is of plant origin, preferably from a dicotyledonous plant, further preferably from the family Brassicaceae, more preferably from the genus Arabidopsis, most preferably from Arabidopsis thaliana.
11. Plant or part thereof, including seeds, obtainable by a method according to any one of items 1 to 10, wherein said plant or part thereof comprises a recombinant nucleic acid encoding a CRL polypeptide.
12. Construct comprising:
   (i) nucleic acid encoding a CRL polypeptide as defined in items 1 or 2;
   (ii) one or more control sequences capable of driving expression of the nucleic acid sequence of (a); and optionally
   (iii) a transcription termination sequence.
13. Construct according to item 12, wherein one of said control sequences is a constitutive promoter, preferably a GOS2 promoter, most preferably a GOS2 promoter from rice.
14. Use of a construct according to item 12 or 13 in a method for making plants having increased yield, particularly increased biomass and/or increased seed yield relative to control plants.
15. Plant, plant part or plant cell transformed with a construct according to item 12 or 13.
16. Method for the production of a transgenic plant having increased yield, particularly increased biomass and/or increased seed yield relative to control plants, comprising:
   (i) introducing and expressing in a plant a nucleic acid encoding a CRL polypeptide as defined in item 1 or 2; and
   (ii) cultivating the plant cell under conditions promoting plant growth and development.
17. Transgenic plant having increased yield, particularly increased biomass and/or increased seed yield, relative to control plants, resulting from modulated expression of a nucleic acid encoding a CRL polypeptide as defined in item 1 or 2, or a transgenic plant cell derived from said transgenic plant.
18. Transgenic plant according to item 11, 15 or 17, or a transgenic plant cell derived thereof, wherein said plant is a crop plant or a monocot or a cereal, such as rice, maize, wheat, barley, millet, rye, triticale, sorghum emmer, spelt, secale, einkorn, teff, milo and oats.
19. Harvestable parts of a plant according to item 18, wherein said harvestable parts are preferably shoot biomass and/or seeds.
20. Products derived from a plant according to item 18 and/or from harvestable parts of a plant according to item 19.
21. Use of a nucleic acid encoding a CRL polypeptide in increasing yield, particularly in increasing seed yield and/or shoot biomass in plants, relative to control plants.
22. An isolated nucleic acid molecule selected from:
   (i) a nucleic acid represented by SEQ ID NO: 195;
   (ii) the complement of a nucleic acid represented by SEQ ID NO: 195;
   (iii) a nucleic acid encoding the polypeptide as represented by SEQ ID NO: 196, preferably as a result of the degeneracy of the genetic code, said isolated nucleic acid can be derived from a polypeptide sequence as represented by SEQ ID NO: 196 and further preferably confers enhanced yield-related traits relative to control plants;
   (iv) a nucleic acid having, in increasing order of preference at least 30 %, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% sequence identity with any of the nucleic acid sequences of Table A7 and further preferably conferring enhanced yield-related traits relative to control plants;
   (v) a nucleic acid molecule which hybridizes with a nucleic acid molecule of (i) to (iv) under stringent hybridization conditions and preferably confers enhanced yield-related traits relative to control plants;
   (vi) a nucleic acid encoding an ASPAT polypeptide having, in increasing order of preference, at least 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% sequence identity to the amino acid sequence represented by SEQ ID NO: 196 and any of the other amino acid sequences in Table A7 and preferably conferring enhanced yield-related traits relative to control plants.
23. An isolated polypeptide selected from:
   (i) an amino acid sequence represented by SEQ ID NO: 196;
   (ii) an amino acid sequence having, in increasing order of preference, at least 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91 %, 92%, 93%, 94%, 95%, 96%, 97%, 98%, or 99% sequence identity to the amino acid sequence represented by SEQ ID NO: 196, and any of the other amino acid sequences in Table A7 and preferably conferring enhanced yield-related traits relative to control plants.
   (iii) derivatives of any of the amino acid sequences given in (i) or (ii) above.

### Description of figures

The present invention will now be described with reference to the following figures in which:
Figure 1 represents the binary vector used for increased expression in Oryza sativa of an - like-encoding nucleic acid under the control of a rice GOS2 promoter (pGOS2)
Figure 2 represents the binary vector used for increased expression in Oryza sativa of a YRP-encoding nucleic acid under the control of a rice GOS2 promoter (pGOS2)
Figure 3 represents how brassinosteroid biosynthesis and auxin signalling are connected through a feedback loop, which involves BRX, required for optimal root growth, according to Mouchel et al. (2006) Nature 443: 458-461.
Figure 4 represents a cartoon of a BRXL polypeptide as represented by SEQ ID NO: 18, which comprises the following features: (i) a Conserved Domain 1 representing a BRX domain, which comprises IPR013591 DZC domain (PFAM entry PF08381 DZC); (2) a Conserved Domain 2 representing a BRX domain, which comprises a C-terminal IPR013591 DZC domain (PFAM entry PF08381 DZC); (3) a Conserved Domain 3 and (4) a Conserved Domain 4, both containing conserved Cys's, whose spacing is indicative of a potential zinc-binding motif.
Figure 5 shows a ClustalW 1.81 multiple sequence alignment of the BRXL polypeptides from Table A3. The following features are heavily boxed (i) a Conserved Domain 1 representing a BRX domain, which comprises IPR013591 DZC domain (PFAM entry PF08381 DZC; marked with X's); (2) a Conserved Domain 2 representing a BRX domain, which comprises a C-terminal IPR013591 DZC domain (PFAM entry PF08381 DZC;
marked with X's); (3) a Conserved Domain 3 and (4) a Conserved Domain 4, both containing conserved Cys's (lightly boxed), whose spacing is indicative of a potential zinc-binding motif.
Figure 6 shows the binary vector for increased expression in Oryza sativa plants of a nucleic acid sequence encoding a BRXL polypeptide under the control of a constitutive promoter functioning in plants.
Figure 7 represents the binary vector used for increased expression in Oryza sativa of a silky-1-like-encoding nucleic acid under the control of a rice GOS2 promoter (pGOS2).
Figure 8 represents a multiple alignment of ARP6 polypeptides.

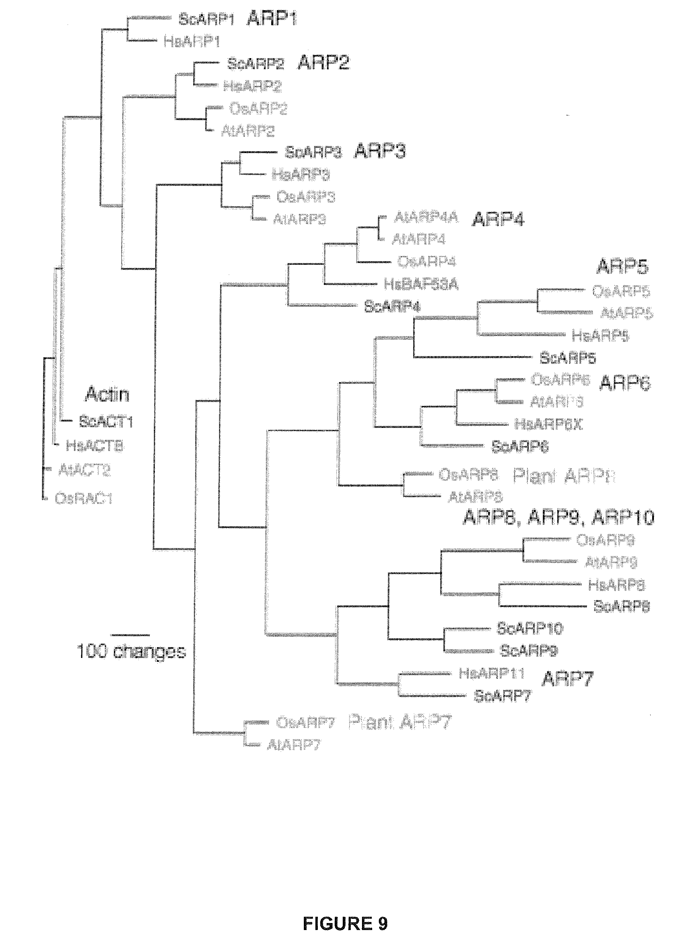

Figure 9 phylogenetic tree of ARP polypeptides as described by Kandasamy et al. 2004.
The Group of ARP6 polypeptides is indicated.
Figure 10 represents the binary vector used for increased expression in Oryza sativa of a ARP6-encoding nucleic acid under the control of a rice GOS2 promoter (pGOS2).

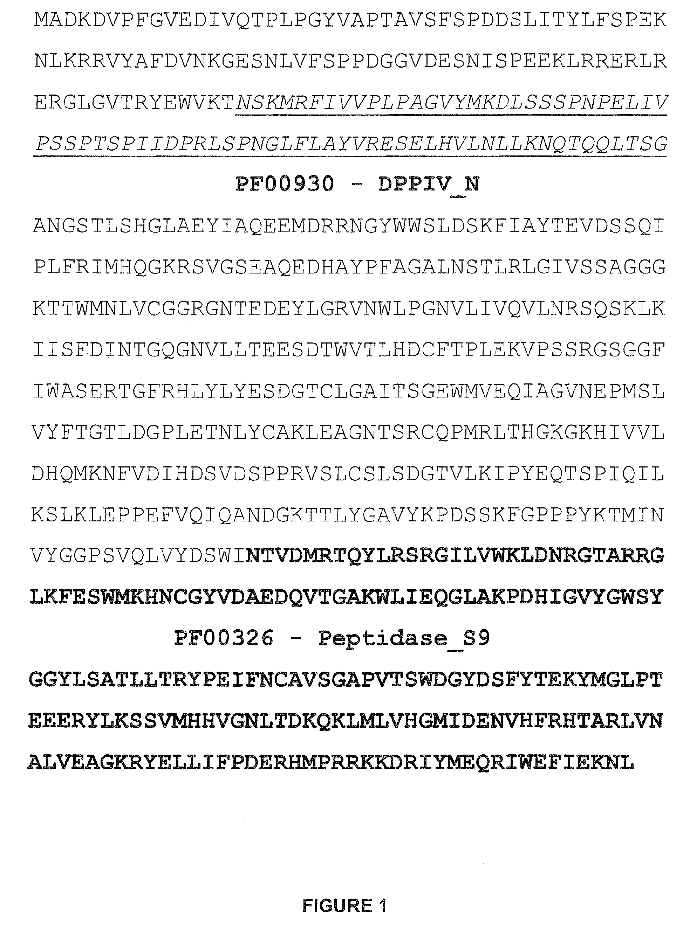

Figure 11 represents SEQ ID NO: 117 with the conserved DPPIV_N and Peptidase_S9 domains indicated in italics underlined and bold respectively.
Figure 12 represents a multiple alignment of various POP sequences
Figure 13 shows a phylogenetic tree of prolyl peptidases (Tripathi & Sowdhamini, 2006).
The branch with SEQ ID NO: 117 and its rice orthologue are indicated by the arrow.

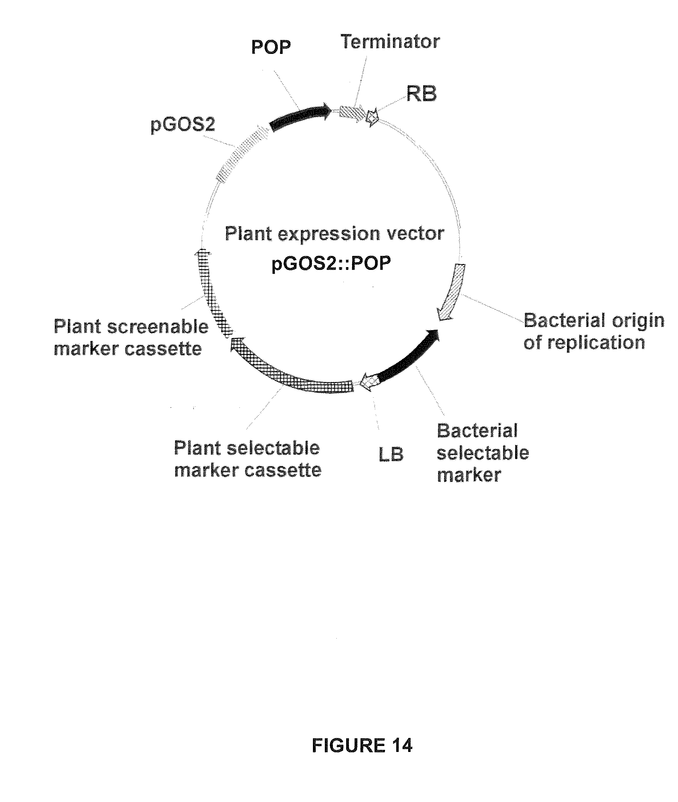

Figure 14 represents the binary vector used for increased expression in Oryza sativa of a POP-encoding nucleic acid under the control of a rice GOS2 promoter (pGOS2).
Figure 15 represents a multiple alignment of CRL proteins.
Figure 16 shows a phylogenetic tree of CRL proteins. Clusters for dicots, monocots and other viridiplantae CRL proteins are shown.

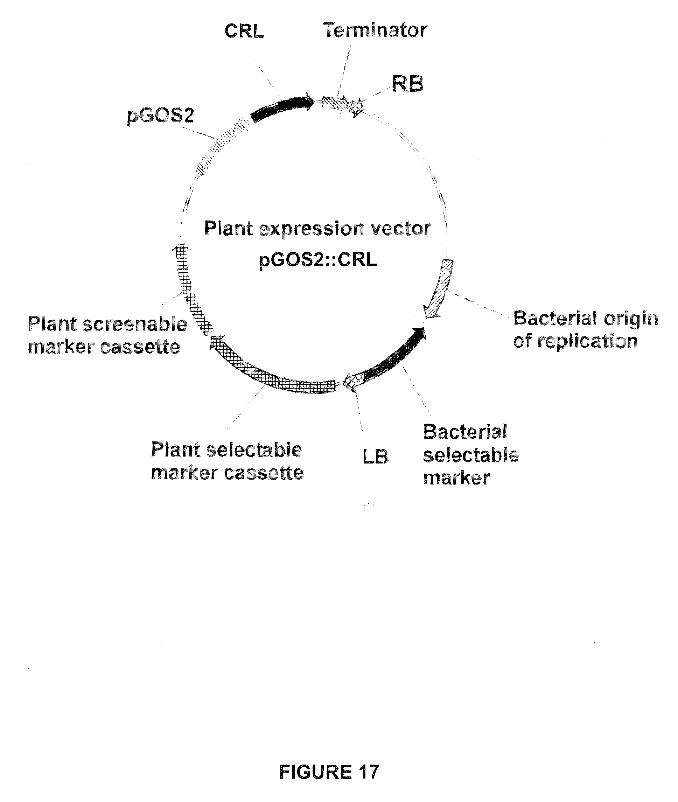

Figure 17 represents the binary vector used for increased expression in Oryza sativa of a CRL-encoding nucleic acid under the control of a rice GOS2 promoter (pGOS2).

### Examples

The present invention will now be described with reference to the following examples, which are by way of illustration alone. The following examples are not intended to completely define or otherwise limit the scope of the invention.

DNA manipulation: unless otherwise stated, recombinant DNA techniques are performed according to standard protocols described in (Sambrook (2001) Molecular Cloning: a laboratory manual, 3rd Edition Cold Spring Harbor Laboratory Press, CSH, New York) or in Volumes 1 and 2 of Ausubel et al. (1994), Current Protocols in Molecular Biology, Current Protocols. Standard materials and methods for plant molecular work are described in Plant Molecular Biology Labfax (1993) by R.D.D. Croy, published by BIOS Scientific Publications Ltd (UK) and Blackwell Scientific Publications (UK).

### 1.1. Alfin-like polypeptides

Sequences (full length cDNA, ESTs or genomic) related to SEQ ID NO: 1 and SEQ ID NO: 3 are identified amongst those maintained in the Entrez Nucleotides database at the National Center for Biotechnology Information (NCBI) using database sequence search tools, such as the Basic Local Alignment Tool (BLAST) (Altschul et al. (1990) J. Mol. Biol. 215:403-410; and Altschul et al. (1997) Nucleic Acids Res. 25:3389-3402). The program is used to find regions of local similarity between sequences by comparing nucleic acid or polypeptide sequences to sequence databases and by calculating the statistical significance of matches. For example, the polypeptide encoded by the nucleic acid of SEQ ID NO: 1 and SEQ ID NO: 3 is used in the TBLASTN algorithm, with default settings and the filter to ignore low complexity sequences set off. The output of the analysis was viewed by pairwise comparison, and ranked according to the probability score (E-value), where the score reflects the probability that a particular alignment occurs by chance (the lower the E-value, the more significant the hit). In addition to E-values, comparisons are also scored by percentage identity. Percentage identity refers to the number of identical nucleotides (or amino acids) between the two compared nucleic acid (or polypeptide) sequences over a particular length. In some instances, the default parameters are adjusted to modify the stringency of the search. For example the E-value is increased to show less stringent matches. This way, short nearly exact matches are identified.

Table A1 provides a list of alfin-like nucleic acid sequences.

**Table A1: Examples alfin-like polypeptides:**

| Name | Organism | Nucleic acid SEQ ID NO | Polypeptide SEQ ID NO |
|---|---|---|---|
| Ac_ALFIN-LIKE | Allium cepa | 1 | 2 |
| Hv_ALFIN-LIKE | Hordeum vulgare | 3 | 4 |

In some instances, related sequences are tentatively assembled and publicly disclosed by research institutions, such as The Institute for Genomic Research (TIGR; beginning with TA). The Eukaryotic Gene Orthologs (EGO) database is used to identify such related sequences, either by keyword search or by using the BLAST algorithm with the nucleic acid sequence or polypeptide sequence of interest. In other instances, special nucleic acid sequence databases are created for particular organisms, such as by the Joint Genome Institute.

### 1.2. YRP polypeptides

Sequences (full length cDNA, ESTs or genomic) related to SEQ ID NO: 10 and SEQ ID NO: 12 are identified amongst those maintained in the Entrez Nucleotides database at the National Center for Biotechnology Information (NCBI) using database sequence search tools, such as the Basic Local Alignment Tool (BLAST) (Altschul et al. (1990) J. Mol. Biol. 215:403-410; and Altschul et al. (1997) Nucleic Acids Res. 25:3389-3402). The program is used to find regions of local similarity between sequences by comparing nucleic acid or polypeptide sequences to sequence databases and by calculating the statistical significance of matches. For example, the polypeptide encoded by the nucleic acid of SEQ ID NO: 10 and SEQ ID NO: 12 is used in the TBLASTN algorithm, with default settings and the filter to ignore low complexity sequences set off. The output of the analysis was viewed by pairwise comparison, and ranked according to the probability score (E-value), where the score reflects the probability that a particular alignment occurs by chance (the lower the E-value, the more significant the hit). In addition to E-values, comparisons are also scored by percentage identity. Percentage identity refers to the number of identical nucleotides (or amino acids) between the two compared nucleic acid (or polypeptide) sequences over a particular length. In some instances, the default parameters are adjusted to modify the stringency of the search. For example the E-value is increased to show less stringent matches. This way, short nearly exact matches are identified.

Table A2 provides a list of YRP nucleic acid sequences.

**Table A2: Examples YRP polypeptides:**

| Name | Organism | Nucleic acid SEQ ID NO | Polypeptide SEQ ID NO |
|---|---|---|---|
| Hv_YRP | Hordeum vulgare | 10 | 11 |
| Hv_YRP | Hordeum vulgare | 12 | 13 |

In some instances, related sequences are tentatively assembled and publicly disclosed by research institutions, such as The Institute for Genomic Research (TIGR; beginning with TA). The Eukaryotic Gene Orthologs (EGO) database is used to identify such related sequences, either by keyword search or by using the BLAST algorithm with the nucleic acid sequence or polypeptide sequence of interest. In other instances, special nucleic acid sequence databases are created for particular organisms, such as by the Joint Genome Institute.

### 1.3. Brevis Radix-like (BRXL) polypeptides

Sequences (full length cDNA, ESTs or genomic) related to the nucleic acid sequence used in the methods of the present invention were identified amongst those maintained in the Entrez Nucleotides database at the National Center for Biotechnology Information (NCBI) using database sequence search tools, such as the Basic Local Alignment Tool (BLAST) (Altschul et al. (1990) J. Mol. Biol. 215:403-410; and Altschul et al. (1997) Nucleic Acids Res. 25:3389-3402). The program is used to find regions of local similarity between sequences by comparing nucleic acid sequence or polypeptide sequences to sequence databases and by calculating the statistical significance of matches. For example, the polypeptide encoded by the nucleic acid sequence of the present invention was used for the TBLASTN algorithm, with default settings and the filter to ignore low complexity sequences set off. The output of the analysis was viewed by pairwise comparison, and ranked according to the probability score (E-value), where the score reflect the probability that a particular alignment occurs by chance (the lower the E-value, the more significant the hit). In addition to E-values, comparisons were also scored by percentage identity. Percentage identity refers to the number of identical nucleotides (or amino acids) between the two compared nucleic acid sequence (or polypeptide) sequences over a particular length. In some instances, the default parameters may be adjusted to modify the stringency of the search. For example the E-value may be increased to show less stringent matches. This way, short nearly exact matches may be identified.

Table A3 provides a list of nucleic acid sequences related to the nucleic acid sequence used in the methods of the present invention.

**Table A3: Examples of BRXL polypeptide sequences, and encoding nucleic acid sequences**

| Name | Public database accession number | Nucleic acid SEQ ID NO: | Polypeptide SEQ ID NO: |
|---|---|---|---|
| Poptr_BRX | JGI_scaff_III.746#1 | 17 | 18 |
| Arath_BRX | NM_102925.2 | 19 | 20 |
| Arath_BRXL1 | NM_129113.4 | 21 | 22 |
| Arath_BRXL2 | NM_112254.2 | 23 | 24 |
| Arath_BRXL3 | NM_104296.2 | 25 | 26 |
| Arath_BRXL4 | NM_122061.3 | 27 | 28 |
| Glyma_BRXL1 | JGI_Gm0025x00418#1 | 29 | 30 |
| Glyma_BRXL2 | JGI_Gm0061x00041#1 | 31 | 32 |
| Glyma_BRXL3 | JGI_Gm0084x00169#1 | 33 | 34 |
| Glyma_BRXL4 | JGI_Gm0097x00095#1 | 35 | 36 |
| Glyma_BRXL5 | JGI_Gm0138x00217#1 | 37 | 38 |
| Glyma_BRXL6 | TIGR_TA62929_3847 | 39 | 40 |
| Medtr_BRXL1 | TIGR_TA28312_3880#1 | 41 | 42 |
| Nicbe_BRXL1 | TIGR_TA8873_4100#1 | 43 | 44 |
| Orysa_BRXL1 | Os08g36020 | 45 | 46 |
| Orysa_BRXL2 | Os02g47230 | 47 | 48 |
| Orysa_BRXL3 | Os04g51170 | 49 | 50 |
| Orysa_BRXL4 | Os03g63650 | 51 | 52 |
| Orysa_BRXL5 | CF299403.1,CI189950.1 | 53 | 54 |
| Phypa_BRXL1 | JGI_P.patens_161871#1 | 55 | 56 |
| Picsi_BRXL1 | TIGR_TA17584_3332#1 | 57 | 58 |
| Poptr_BRXL1 | JGI_P.trichocarpa_808986 | 59 | 60 |
| Poptr_BRXL2 | JGI_scaff_VI.979#1 | 61 | 62 |
| Poptr_BRXL3 | TIGR_TA12611_3695#1 | 63 | 64 |
| Sorbi_BRXL1 | Sb07g022540 | 65 | 66 |
| Sorbi_BRXL2 | Sb02g026020 | 67 | 68 |
| Vitvi_BRXL1 | GSVIVT00003671001#1 | 69 | 70 |
| Vitvi_BRXL2 | GGSVIVT00034110001#1 | 71 | 72 |
| Zeama_BRXL1 | TIGR_TA198521_4577#1 | 73 | 74 |
| Zeama_BRXL2 | Proprietary_ZM07MC01509_57718871 @1504#1 | 75 | 76 |
| Zeama_BRXL3 | Proprietary_ZM07MC22150_BFb0050E 10@22088#1 | 77 | 78 |
| Zeama_BRXL4 | Proprietary_ZM07MC27026_BFb0199F1 9@26946#1 | 79 | 80 |

In some instances, related sequences have tentatively been assembled and publicly disclosed by research institutions, such as The Institute for Genomic Research (TIGR; beginning with TA), or Genoscope (beginning with GS). The Eukaryotic Gene Orthologs (EGO) database may be used to identify such related sequences, either by keyword search or by using the BLAST algorithm with the nucleic acid sequence or polypeptide sequence of interest. On other instances, special nucleic acid sequence databases have been created for particular organisms, such as by the Joint Genome Institute. Further, access to proprietary databases, has allowed the identification of novel nucleic acid and polypeptide sequences.

### 1.4. silky-1-like polypeptides

Sequences (full length cDNA, ESTs or genomic) related to SEQ ID NO: 90, SEQ ID NO: 92 or SEQ ID NO: 94 are identified amongst those maintained in the Entrez Nucleotides database at the National Center for Biotechnology Information (NCBI) using database sequence search tools, such as the Basic Local Alignment Tool (BLAST) (Altschul et al. (1990) J. Mol. Biol. 215:403-410; and Altschul et al. (1997) Nucleic Acids Res. 25:3389-3402). The program is used to find regions of local similarity between sequences by comparing nucleic acid or polypeptide sequences to sequence databases and by calculating the statistical significance of matches. For example, the polypeptide encoded by the nucleic acid of SEQ ID NO: 90, SEQ ID NO: 92 or SEQ ID NO: 94 is used in the TBLASTN algorithm, with default settings and the filter to ignore low complexity sequences set off. The output of the analysis was viewed by pairwise comparison, and ranked according to the probability score (E-value), where the score reflects the probability that a particular alignment occurs by chance (the lower the E-value, the more significant the hit). In addition to E-values, comparisons are also scored by percentage identity. Percentage identity refers to the number of identical nucleotides (or amino acids) between the two compared nucleic acid (or polypeptide) sequences over a particular length. In some instances, the default parameters are adjusted to modify the stringency of the search. For example the E-value is increased to show less stringent matches. This way, short nearly exact matches are identified.

Table A4 provides a list of silky-1-like nucleic acid sequences.

**Table A4: Examples silky-1-like polypeptides:**

| Name | Organism | Nucleic acid SEQ ID NO | Polypeptide SEQ ID NO |
|---|---|---|---|
| Pt_silky homologue | Populus trichocarpa | 90 | 91 |
| Le-silky homologue | Solanum lycopersicum | 92 | 93 |
| Ta_silky | Triticum aestivum | 94 | 95 |

In some instances, related sequences are tentatively assembled and publicly disclosed by research institutions, such as The Institute for Genomic Research (TIGR; beginning with TA). The Eukaryotic Gene Orthologs (EGO) database is used to identify such related sequences, either by keyword search or by using the BLAST algorithm with the nucleic acid sequence or polypeptide sequence of interest. In other instances, special nucleic acid sequence databases are created for particular organisms, such as by the Joint Genome Institute.

### 1.5. ARP6 polypeptides

Sequences (full length cDNA, ESTs or genomic) related to the nucleic acid sequence used in the methods of the present invention were identified amongst those maintained in the Entrez Nucleotides database at the National Center for Biotechnology Information (NCBI) using database sequence search tools, such as the Basic Local Alignment Tool (BLAST) (Altschul et al. (1990) J. Mol. Biol. 215:403-410; and Altschul et al. (1997) Nucleic Acids Res. 25:3389-3402). The program is used to find regions of local similarity between sequences by comparing nucleic acid or polypeptide sequences to sequence databases and by calculating the statistical significance of matches. For example, the polypeptide encoded by the nucleic acid used in the present invention was used for the TBLASTN algorithm, with default settings and the filter to ignore low complexity sequences set off. The output of the analysis was viewed by pairwise comparison, and ranked according to the probability score (E-value), where the score reflect the probability that a particular alignment occurs by chance (the lower the E-value, the more significant the hit). In addition to E-values, comparisons were also scored by percentage identity. Percentage identity refers to the number of identical nucleotides (or amino acids) between the two compared nucleic acid (or polypeptide) sequences over a particular length. In some instances, the default parameters may be adjusted to modify the stringency of the search. For example the E-value may be increased to show less stringent matches. This way, short nearly exact matches may be identified.

Table A5 provides a list of nucleic acid sequences related to the nucleic acid sequence used in the methods of the present invention.

**Table A5: Examples of ARP6 nucleic acids and encoded polypeptides thereof:**

| ARP6 | Source Organism | Nucleic acid SEQ ID NO: | Polypeptide SEQ ID NO: |
|---|---|---|---|
| A.thaliana_AT3G33520.1 | Arabidopsis thaliana | 101 | 102 |
| O.sativa_LOC_Os01g16414.1 | Oryza sativa | 103 | 104 |
| P.patens_126969 | Physcomitrella patens | 105 | 106 |
| P.trichocarpa_scaff_XVIII.1149 | Populus trichoparca | 107 | 108 |
| Gm0195x00030 | Glycine max | 109 | 110 |
| Gm0057x00075 | Glycine max | 111 | 112 |

In some instances, related sequences have tentatively been assembled and publicly disclosed by research institutions, such as The Institute for Genomic Research (TIGR; beginning with TA). The Eukaryotic Gene Orthologs (EGO) database may be used to identify such related sequences, either by keyword search or by using the BLAST algorithm with the nucleic acid sequence or polypeptide sequence of interest. On other instances, special nucleic acid sequence databases have been created for particular organisms, such as by the Joint Genome Institute. Further, access to proprietary databases, has allowed the identification of novel nucleic acid and polypeptide sequences.

### 1.6. POP polypeptides

Sequences (full length cDNA, ESTs or genomic) related to the nucleic acid sequence used in the methods of the present invention were identified amongst those maintained in the Entrez Nucleotides database at the National Center for Biotechnology Information (NCBI) using database sequence search tools, such as the Basic Local Alignment Tool (BLAST) (Altschul et al. (1990) J. Mol. Biol. 215:403-410; and Altschul et al. (1997) Nucleic Acids Res. 25:3389-3402). The program is used to find regions of local similarity between sequences by comparing nucleic acid or polypeptide sequences to sequence databases and by calculating the statistical significance of matches. For example, the polypeptide encoded by the nucleic acid used in the present invention was used for the TBLASTN algorithm, with default settings and the filter to ignore low complexity sequences set off. The output of the analysis was viewed by pairwise comparison, and ranked according to the probability score (E-value), where the score reflect the probability that a particular alignment occurs by chance (the lower the E-value, the more significant the hit). In addition to E-values, comparisons were also scored by percentage identity. Percentage identity refers to the number of identical nucleotides (or amino acids) between the two compared nucleic acid (or polypeptide) sequences over a particular length. In some instances, the default parameters may be adjusted to modify the stringency of the search. For example the E-value may be increased to show less stringent matches. This way, short nearly exact matches may be identified.

Table A6 provides a list of nucleic acid sequences related to the nucleic acid sequence used in the methods of the present invention.

**Table A6: Examples of POP polypeptides:**

| Name | Plant source | Nucleic acid SEQ ID NO | Polypeptide SEQ ID NO |
|---|---|---|---|
| | Arabidopsis thaliana | 116 | 117 |
| AC13782818.5#1 | Medicago truncatula | 135 | 145 |
| Os02g0290600#1 | Oryza sativa | 136 | 146 |
| 104540#1 | Physcomitrella patens | 137 | 147 |
| 105959#1 | Physcomitrella patens | 138 | 148 |
| scaff_XII.203#1 | Populus trichocarpa | 139 | 149 |
| scaff_XV.146#1 | Populus trichocarpa | 140 | 150 |
| AT4G14570 | Arabidopsis thaliana | 141 | 151 |
| AT5G36210 | Arabidopsis thaliana | 142 | 152 |
| EF085857.1 | Picea sitchensis | 143 | 153 |
| AY108871 | Zea mays | 144 | 154 |

In some instances, related sequences have tentatively been assembled and publicly disclosed by research institutions, such as The Institute for Genomic Research (TIGR; beginning with TA). The Eukaryotic Gene Orthologs (EGO) database may be used to identify such related sequences, either by keyword search or by using the BLAST algorithm with the nucleic acid sequence or polypeptide sequence of interest. On other instances, special nucleic acid sequence databases have been created for particular organisms, such as by the Joint Genome Institute. Further, access to proprietary databases, has allowed the identification of novel nucleic acid and polypeptide sequences.

### 1.7. Crumpled Leaf (CRL) polypeptides

Sequences (full length cDNA, ESTs or genomic) related to the nucleic acid sequence used in the methods of the present invention were identified amongst those maintained in the Entrez Nucleotides database at the National Center for Biotechnology Information (NCBI) using database sequence search tools, such as the Basic Local Alignment Tool (BLAST) (Altschul et al. (1990) J. Mol. Biol. 215:403-410; and Altschul et al. (1997) Nucleic Acids Res. 25:3389-3402). The program is used to find regions of local similarity between sequences by comparing nucleic acid or polypeptide sequences to sequence databases and by calculating the statistical significance of matches. For example, the polypeptide encoded by the nucleic acid used in the present invention was used for the TBLASTN algorithm, with default settings and the filter to ignore low complexity sequences set off. The output of the analysis was viewed by pairwise comparison, and ranked according to the probability score (E-value), where the score reflect the probability that a particular alignment occurs by chance (the lower the E-value, the more significant the hit). In addition to E-values, comparisons were also scored by percentage identity. Percentage identity refers to the number of identical nucleotides (or amino acids) between the two compared nucleic acid (or polypeptide) sequences over a particular length. In some instances, the default parameters may be adjusted to modify the stringency of the search. For example the E-value may be increased to show less stringent matches. This way, short nearly exact matches may be identified.

Table A7 provides a list of nucleic acid sequences related to the nucleic acid sequence used in the methods of the present invention.

**Table A7: Examples of CRL polypeptides:**

| Name | Nucleic acid SEQ ID NO: | Protein SEQ ID NO: |
|---|---|---|
| A.thaliana_AT5G51020.1#1 | 155 | 156 |
| B.napus_TA26749_3708#1 | 157 | 158 |
| C.clementina_DY292515#1 | 159 | 160 |
| C.endivia_TA817_114280#1 | 161 | 162 |
| C.sinensis_TA15191_2711#1 | 163 | 164 |
| G.max_Gm0065x00465#1 | 165 | 166 |
| G.raimondii_TA13121_29730#1 | 167 | 168 |
| H.paradoxus_TA3639_73304#1 | 169 | 170 |
| I.nil_TA8128_35883#1 | 171 | 172 |
| L.saligna_TA1573_75948#1 | 173 | 174 |
| M.truncatula_AC139708_31.5#1 | 175 | 176 |
| N.tabacum_TA16794_4097#1 | 177 | 178 |
| T.officinale_TA2756_50225#1 | 179 | 180 |
| V.vinifera_GSVIVT00018055001#1 | 181 | 182 |
| P.trichocarpa_834377#1 | 183 | 184 |
| S.lycopersicum_TA38444_4081#1 | 185 | 186 |
| S.tuberosum_TA25303_4113#1 | 187 | 188 |
| S,officinarum_TA38665_4547#1 | 189 | 190 |
| T.aestivum_TA92222_4565#1 | 191 | 192 |
| S.bicolor_5284384#1 | 193 | 194 |
| Z.mays_ZM07MC01438_57666838@1433#1 | 195 | 196 |
| O.sativa.indica_BGIOSIBCE034659#1 | 197 | 198 |
| O.sativa_Os11g0524300#1 | 199 | 200 |
| P. patens_133413#1 | 201 | 202 |
| P.taeda_TA18056_3352#1 | 203 | 204 |
| S.moellendorffii_170435#1 | 205 | 206 |

In some instances, related sequences have tentatively been assembled and publicly disclosed by research institutions, such as The Institute for Genomic Research (TIGR; beginning with TA). The Eukaryotic Gene Orthologs (EGO) database may be used to identify such related sequences, either by keyword search or by using the BLAST algorithm with the nucleic acid sequence or polypeptide sequence of interest. On other instances, special nucleic acid sequence databases have been created for particular organisms, such as by the Joint Genome Institute. Further, access to proprietary databases, has allowed the identification of novel nucleic acid and polypeptide sequences.

### Example 2: Alignment of sequences related to the polypeptide sequences used in the methods of the invention

### 2.1. Alfin-like polypeptides

Alignment of polypeptide sequences is performed using the ClustalW 2.0 algorithm of progressive alignment (Thompson et al. (1997) Nucleic Acids Res 25:4876-4882; Chenna et al. (2003). Nucleic Acids Res 31:3497-3500) with standard setting (slow alignment, similarity matrix: Gonnet (or Blosum 62 (if polypeptides are aligned), gap opening penalty 10, gap extension penalty: 0.2). Minor manual editing is done to further optimise the alignment.

A phylogenetic tree of alfin-like polypeptides is constructed using a neighbour-joining clustering algorithm as provided in the AlignX programme from the Vector NTI (Invitrogen).

Alignment of polypeptide sequences is performed using the ClustalW 2.0 algorithm of progressive alignment (Thompson et al. (1997) Nucleic Acids Res 25:4876-4882; Chenna et al. (2003). Nucleic Acids Res 31:3497-3500) with standard setting (slow alignment, similarity matrix: Gonnet, gap opening penalty 10, gap extension penalty: 0.2). Minor manual editing is done to further optimise the alignment.

### 2.2. YRP polypeptides

Alignment of polypeptide sequences is performed using the ClustalW 2.0 algorithm of progressive alignment (Thompson et al. (1997) Nucleic Acids Res 25:4876-4882; Chenna et al. (2003). Nucleic Acids Res 31:3497-3500) with standard setting (slow alignment, similarity matrix: Gonnet (or Blosum 62 (if polypeptides are aligned), gap opening penalty 10, gap extension penalty: 0.2). Minor manual editing is done to further optimise the alignment.

A phylogenetic tree of YRP polypeptides is constructed using a neighbour-joining clustering algorithm as provided in the AlignX programme from the Vector NTI (Invitrogen). Alignment of polypeptide sequences is performed using the ClustalW 2.0 algorithm of progressive alignment (Thompson et al. (1997) Nucleic Acids Res 25:4876-4882; Chenna et al. (2003). Nucleic Acids Res 31:3497-3500) with standard setting (slow alignment, similarity matrix: Gonnet, gap opening penalty 10, gap extension penalty: 0.2). Minor manual editing is done to further optimise the alignment.

### 2.3. Brevis Radix-like (BRXL) polypeptides

Mutliple sequence alignment of all the BRXL polypeptide sequences in Table A3 was performed using the ClustalW 1.81 algorithm. Results of the alignment are shown in Figure 5 of the present application. The following features are heavily boxed (i) a Conserved Domain 1 representing a BRX domain, which comprises IPR013591 DZC domain (PFAM entry PF08381 DZC; marked with X's); (2) a Conserved Domain 2 representing a BRX domain, which comprises a C-terminal IPR013591 DZC domain (PFAM entry PF08381 DZC; marked with X's); (3) a Conserved Domain 3 and (4) a Conserved Domain 4, both containing conserved Cys's (lightly boxed), whose spacing is indicative of a potential zinc-binding motif.

### 2.4. silky-1-like polypeptides

Alignment of polypeptide sequences is performed using the ClustalW 2.0 algorithm of progressive alignment (Thompson et al. (1997) Nucleic Acids Res 25:4876-4882; Chenna et al. (2003). Nucleic Acids Res 31:3497-3500) with standard setting (slow alignment, similarity matrix: Gonnet (or Blosum 62 (if polypeptides are aligned), gap opening penalty 10, gap extension penalty: 0.2). Minor manual editing is done to further optimise the alignment.

A phylogenetic tree of silky-1-like polypeptides is constructed using a neighbour-joining clustering algorithm as provided in the AlignX programme from the Vector NTI (Invitrogen).

Alignment of polypeptide sequences is performed using the ClustalW 2.0 algorithm of progressive alignment (Thompson et al. (1997) Nucleic Acids Res 25:4876-4882; Chenna et al. (2003). Nucleic Acids Res 31:3497-3500) with standard setting (slow alignment, similarity matrix: Gonnet, gap opening penalty 10, gap extension penalty: 0.2). Minor manual editing is done to further optimise the alignment.

### 2.5. ARP6 polypeptides

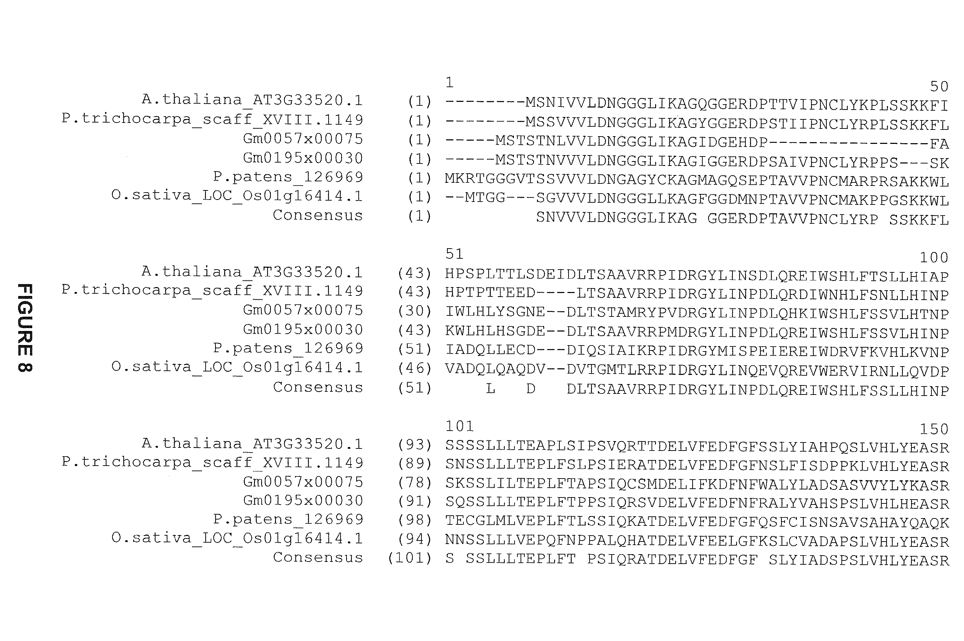

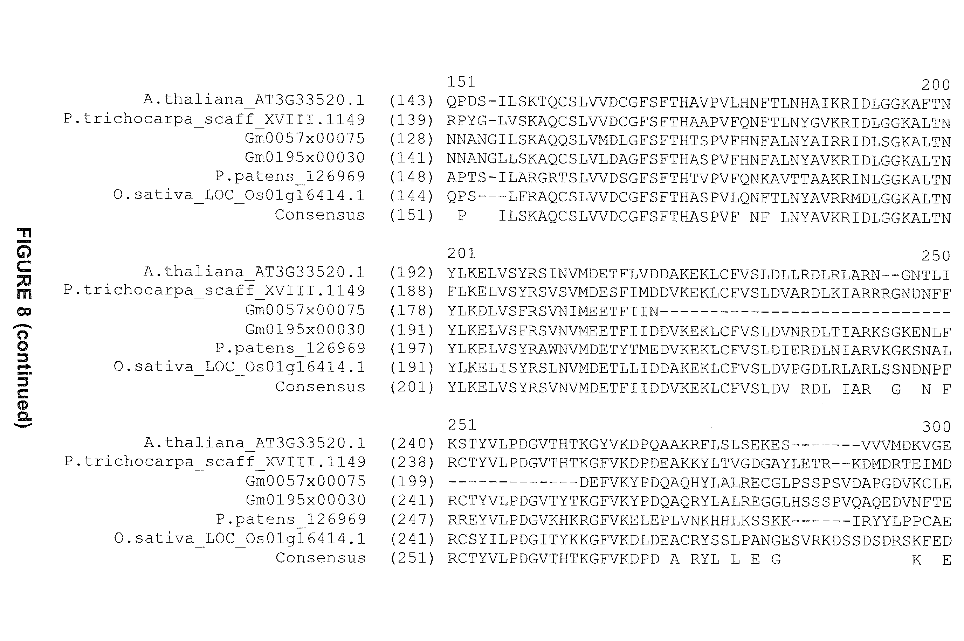

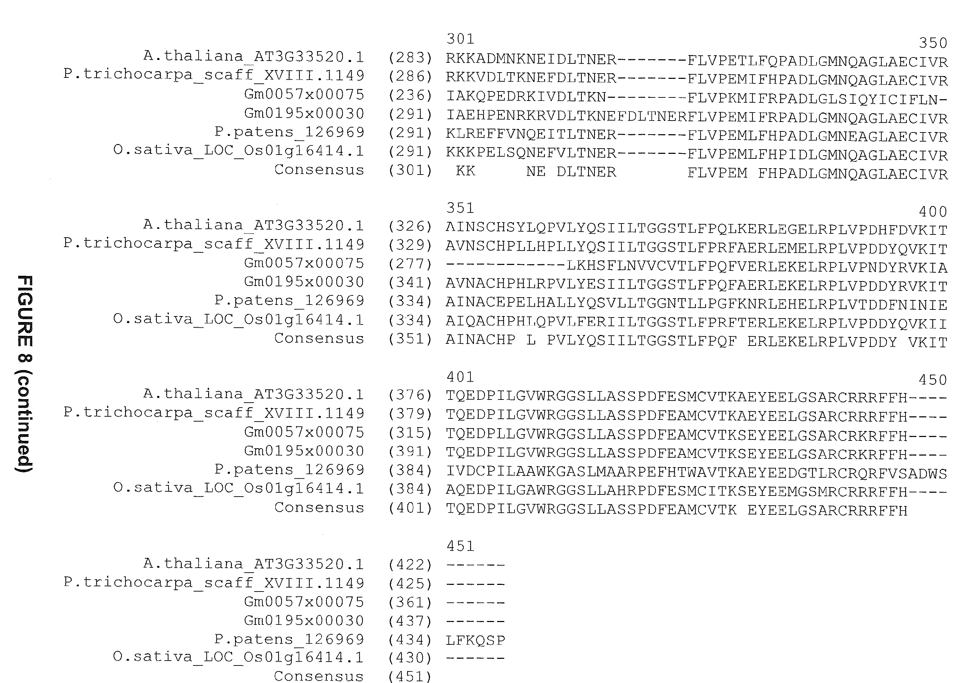

Alignment of polypeptide sequences was performed using the ClustalW 2.0 algorithm of progressive alignment (Thompson et al. (1997) Nucleic Acids Res 25:4876-4882; Chenna et al. (2003). Nucleic Acids Res 31:3497-3500) with standard setting (slow alignment, similarity matrix: Gonnet (or Blosum 62 (if polypeptides are aligned), gap opening penalty 10, gap extension penalty: 0.2). Minor manual editing was done to further optimise the alignment. The ARP6 polypeptides are aligned in Figure 8.

Alignment of polypeptide sequences was performed using the ClustalW 2.0 algorithm of progressive alignment (Thompson et al. (1997) Nucleic Acids Res 25:4876-4882; Chenna et al. (2003). Nucleic Acids Res 31:3497-3500) with standard setting (slow alignment, similarity matrix: Gonnet, gap opening penalty 10, gap extension penalty: 0.2). Minor manual editing was done to further optimise the alignment.

### 2.6. POP polypeptides

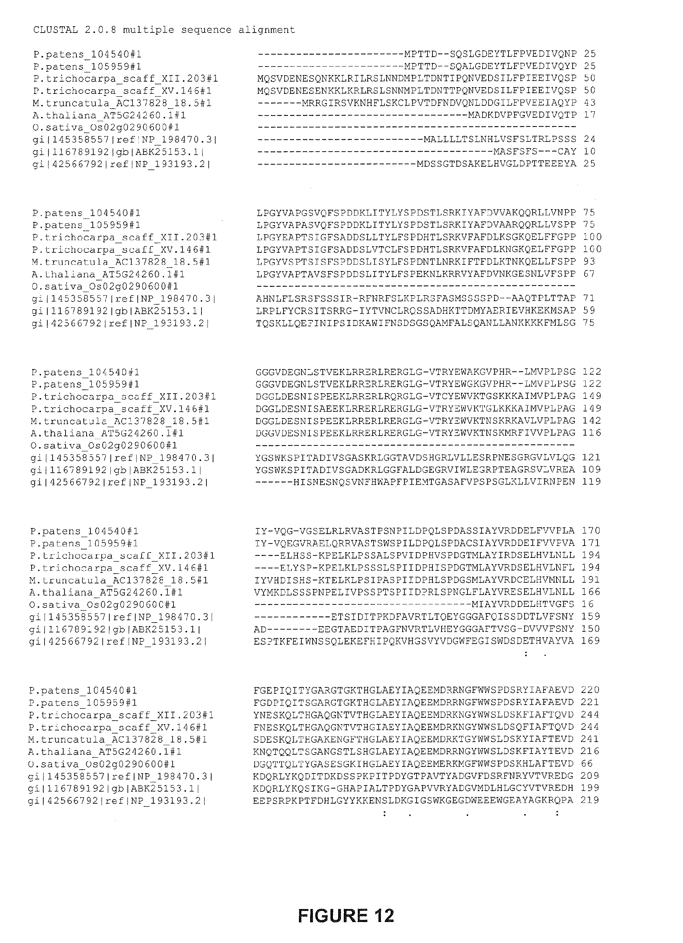

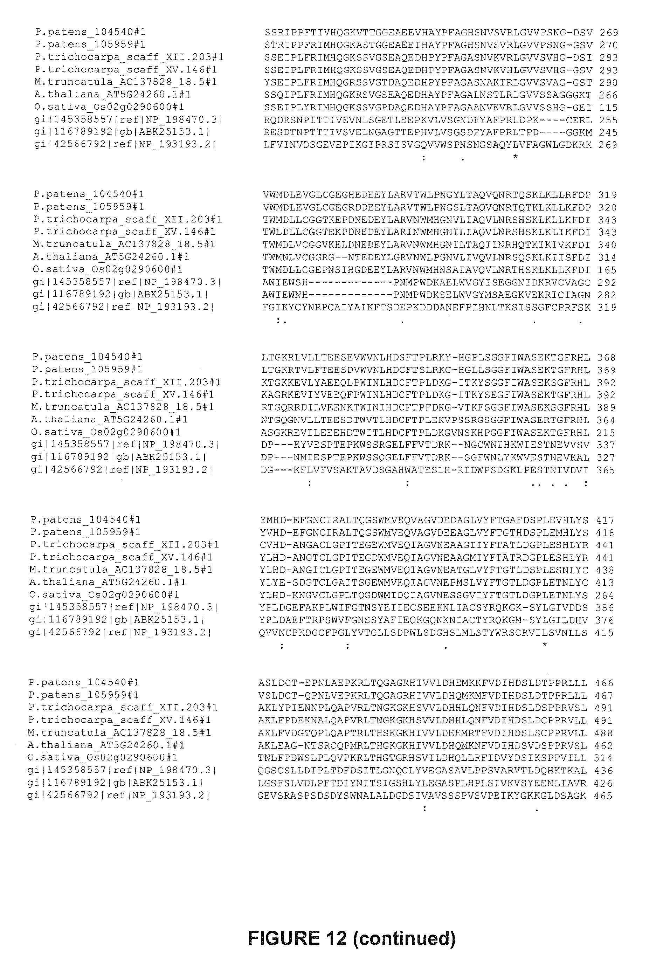

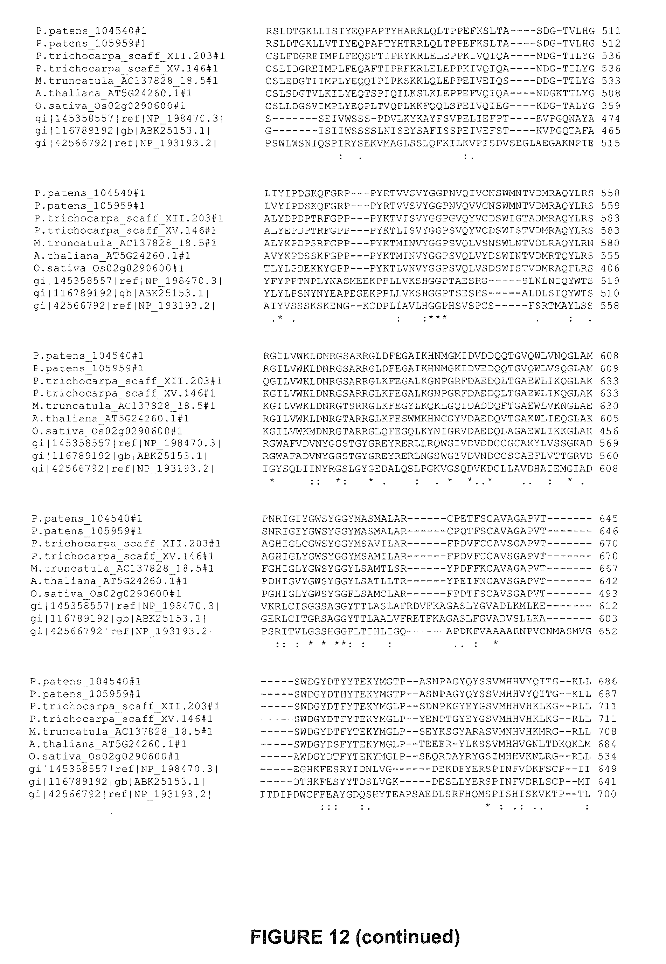

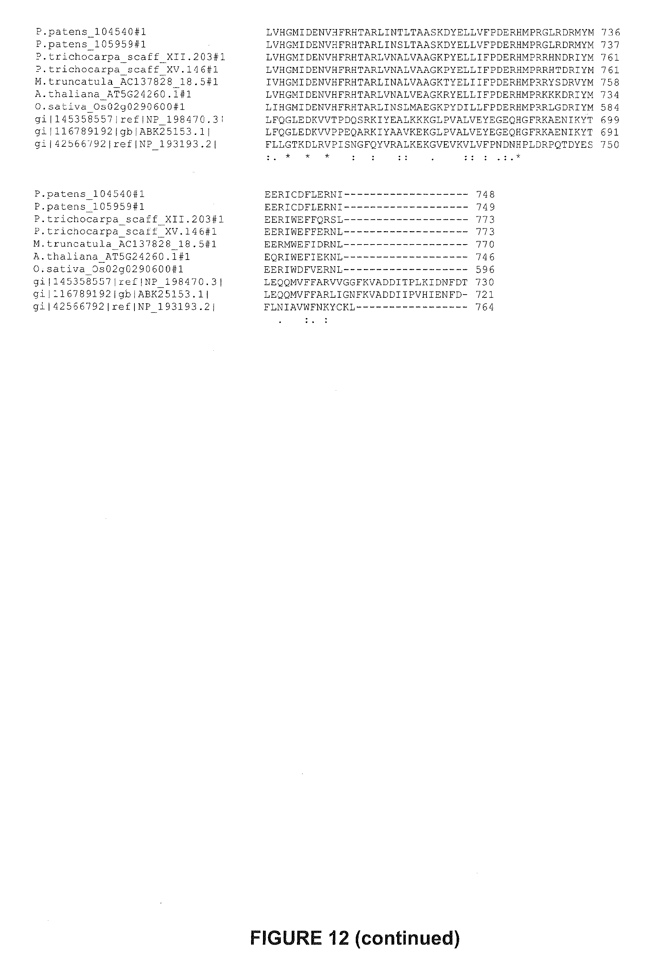

Alignment of polypeptide sequences was performed using the ClustalW 2.0 algorithm of progressive alignment (Thompson et al. (1997) Nucleic Acids Res 25:4876-4882; Chenna et al. (2003). Nucleic Acids Res 31:3497-3500) with standard settings (slow alignment, similarity matrix: Gonnet, gap opening penalty 10, gap extension penalty: 0.2). Minor manual editing was done to further optimise the alignment. The POP polypeptides are aligned in Figure 12.

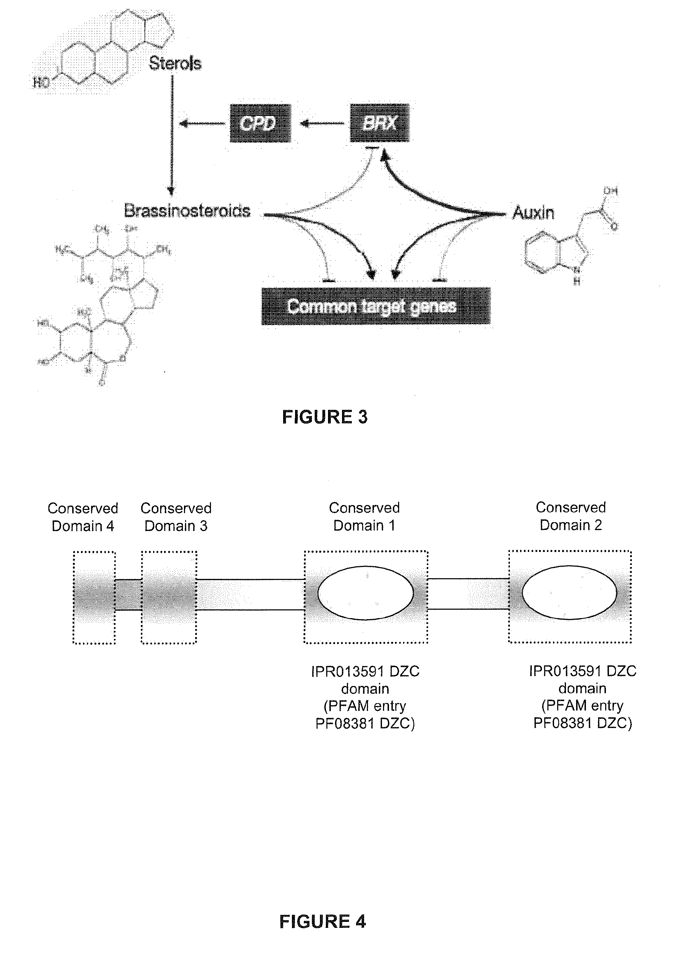

The phylogenetic tree of POP polypeptides (Figure 3, Tripathi & Sowdhamini, 2006) was constructed as follows:

Multiple sequence alignments of serine-protease domains were constructed using the CLUSTALW program. In order to compare equivalent regions, the domain regions were retrieved employing HMMALIGN, a sequence to profile matching method against the PfamA database. Proteins lacking a significant portion of the protease-like domain were not included in alignments. A Blosum 30 matrix, an open gap penalty of 10 and an extension penalty of 0.05 were employed. An overall phylogenetic tree was inferred from the multiple sequence alignment with PHYLIP (Phylogeny Inference Package) 3.65. Bootstrapping was performed 100 times using SEQBOOT to obtain support values for each internal branch. Pairwise distances were determined with PROTDIST. Neighbourjoining phylogenetic trees were calculated with NEIGHBOR using standard parameters. The majority-rule consensus trees of all bootstrapped sequences were obtained with the program CONSENSE. Representations of the calculated trees were constructed using TreeView. Clusters with bootstrap values greater than 50% were defined as confirmed subgroups, and sequences with lower values added to these subgroups according to their sequence similarity in the alignment as judged by visual inspection.

### 2.7. Crumpled Leaf (CRL) polypeptides

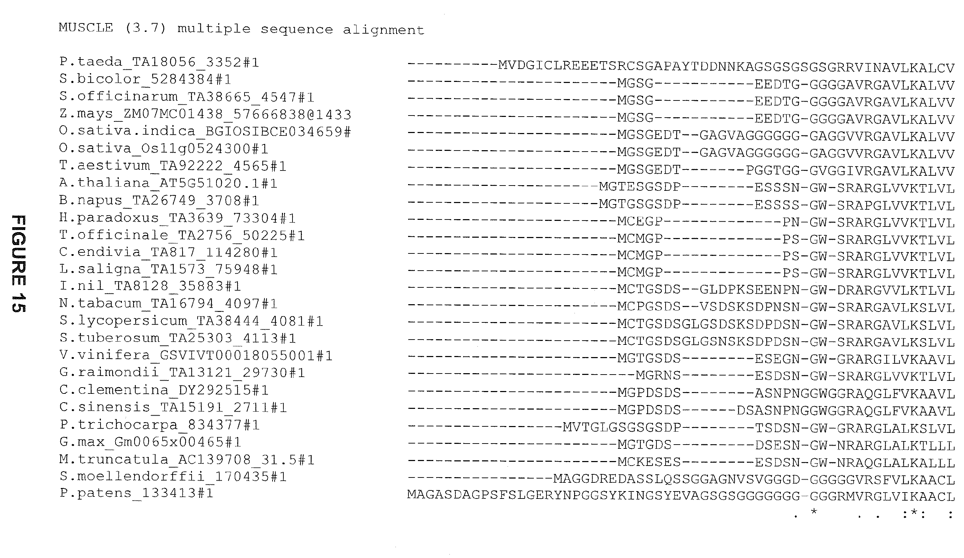

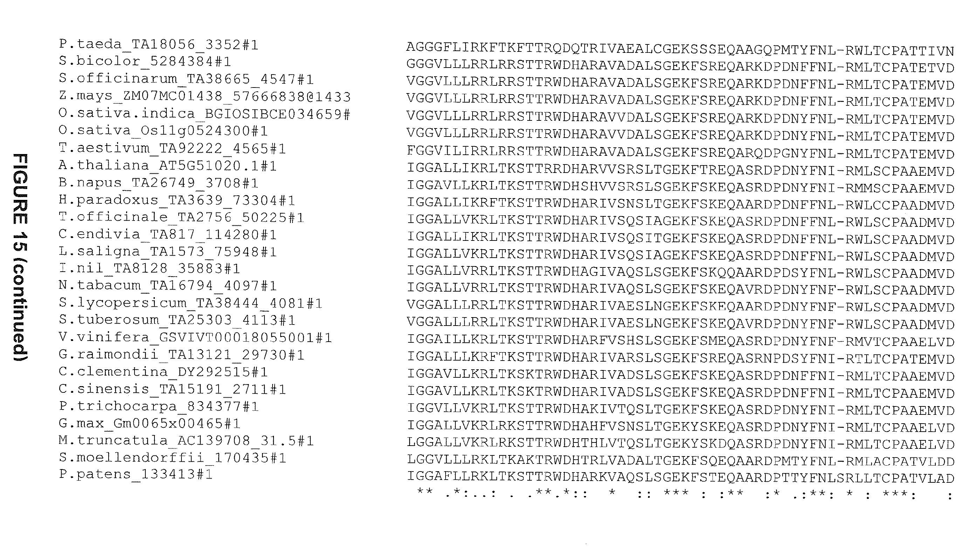

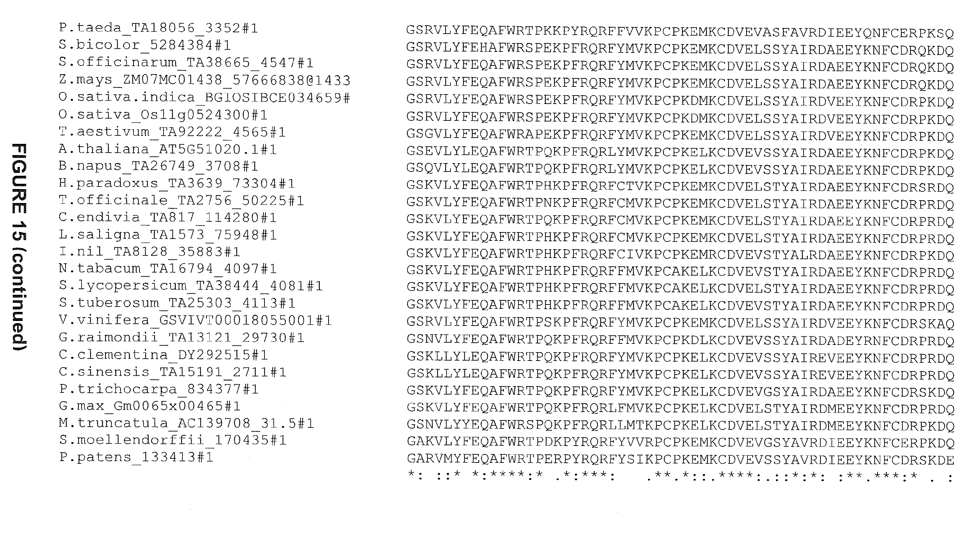

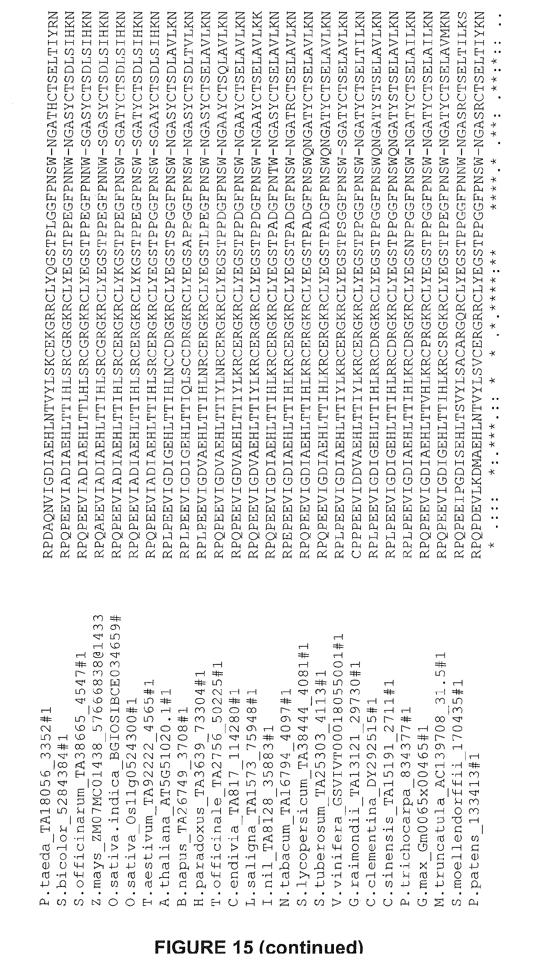

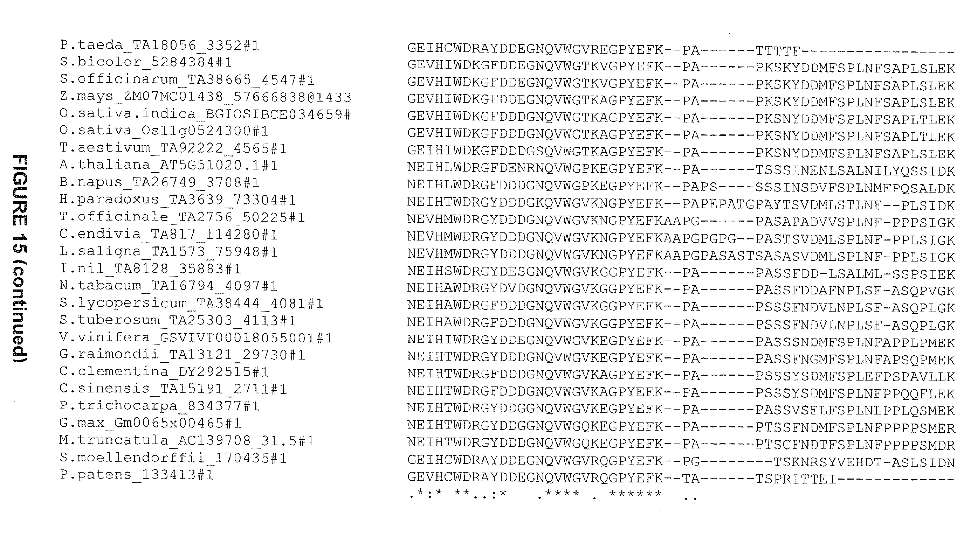

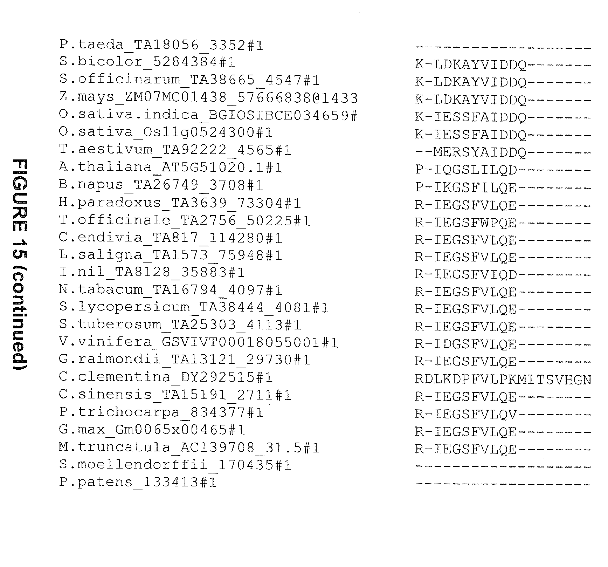

Alignment of polypeptide sequences was performed using the MUSCLE 3.7 algorithm (MUltiple Sequence Comparison by Log-Expectation) (Edgard 2004 of Nucleic Acids Research, 2004, Vol. 32, No. 5 1792-1797) with standard setting (Figure 15).

A Neighbour-Joining tree was calculated using QuickTree (Howe et al. (2002), Bioinformatics 18(11): 1546-7). Support of the major branching after 100 bootstrap repetitions is indicated. A circular phylogram was drawn using Dendroscope (Huson et al. (2007), BMC Bioinformatics 8(1):460). See Figure 16. CRL protein seems is a unique in most organisms.

### Example 3: Calculation of global percentage identity between polypeptide sequences useful in performing the methods of the invention

### 3.1. Alfin-like polypeptides

Global percentages of similarity and identity between full length polypeptide sequences is determined using one of the methods available in the art, the MatGAT (Matrix Global Alignment Tool) software (BMC Bioinformatics. 2003 4:29. MatGAT: an application that generates similarity/identity matrices using protein or DNA sequences. Campanella JJ, Bitincka L, Smalley J; software hosted by Ledion Bitincka). MatGAT software generates similarity/identity matrices for DNA or protein sequences without needing pre-alignment of the data. The program performs a series of pair-wise alignments using the Myers and Miller global alignment algorithm (with a gap opening penalty of 12, and a gap extension penalty of 2), calculates similarity and identity using for example Blosum 62 (for polypeptides), and then places the results in a distance matrix. Sequence similarity is shown in the bottom half of the dividing line and sequence identity is shown in the top half of the diagonal dividing line.

Parameters used in the comparison are:

| | |
|---|---|
| Scoring matrix: | Blosum62 |
| First Gap: | 12 |
| Extending gap: | 2 |

A MATGAT table for local alignment of a specific domain, or data on % identity/similarity between specific domains may also be performed.

### 3.2. YRP polypeptides

Global percentages of similarity and identity between full length polypeptide sequences is determined using one of the methods available in the art, the MatGAT (Matrix Global Alignment Tool) software (BMC Bioinformatics. 2003 4:29. MatGAT: an application that generates similarity/identity matrices using protein or DNA sequences. Campanella JJ, Bitincka L, Smalley J; software hosted by Ledion Bitincka). MatGAT software generates similarity/identity matrices for DNA or protein sequences without needing pre-alignment of the data. The program performs a series of pair-wise alignments using the Myers and Miller global alignment algorithm (with a gap opening penalty of 12, and a gap extension penalty of 2), calculates similarity and identity using for example Blosum 62 (for polypeptides), and then places the results in a distance matrix. Sequence similarity is shown in the bottom half of the dividing line and sequence identity is shown in the top half of the diagonal dividing line.

Parameters used in the comparison are:

| | |
|---|---|
| Scoring matrix: | Blosum62 |
| First Gap: | 12 |
| Extending gap: | 2 |

A MATGAT table for local alignment of a specific domain, or data on % identity/similarity between specific domains may also be performed.

### 3.3. Brevis Radix-like (BRXL) polypeptides

Global percentages of similarity and identity between full length polypeptide sequences useful in performing the methods of the invention were determined using one of the methods available in the art, the MatGAT (Matrix Global Alignment Tool) software (BMC Bioinformatics. 2003 4:29. MatGAT: an application that generates similarity/identity matrices using protein or DNA sequences. Campanella JJ, Bitincka L, Smalley J; software hosted by Ledion Bitincka). MatGAT software generates similarity/identity matrices for DNA or protein sequences without needing pre-alignment of the data. The program performs a series of pair-wise alignments using the Myers and Miller global alignment algorithm (with a gap opening penalty of 12, and a gap extension penalty of 2), calculates similarity and identity using for example Blosum 62 (for polypeptides), and then places the results in a distance matrix. Sequence similarity is shown in the bottom half of the dividing line and sequence identity is shown in the top half of the diagonal dividing line.

Parameters used in the comparison were:

| | |
|---|---|
| Scoring matrix: | Blosum62 |
| First Gap: | 12 |
| Extending gap: | 2 |

Results of the software analysis are shown in Table B1 for the global similarity and identity over the full length of the polypeptide sequences (excluding the partial polypeptide sequences).

The percentage identity between the full length polypeptide sequences useful in performing the methods of the invention can be as low as 43% amino acid identity compared to SEQ ID NO: 18.

**Table B1: MatGAT results for global similarity and identity over the full length of the polypeptide sequences of Table A3.**

| | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 | 27 | 28 | 29 | 30 |
|---|---|---|---|---|---|---|---|---|---|---|---|---|---|---|---|---|---|---|---|---|---|---|---|---|---|---|---|---|---|---|
| **1**. **Poptr**_**BRX** | | **67** | **65** | **50** | **50** | **46** | **51** | **78** | **52** | **46** | **69** | **48** | **53** | **50** | **44** | **47** | **45** | **45** | **43** | **50** | **50** | **51** | **45** | **45** | **79** | **56** | **45** | **43** | **45** | **47** |
| 2. Arath_BRX | 79 | | 78 | 46 | 48 | 42 | 44 | 70 | 44 | 43 | 64 | 43 | 48 | 45 | 43 | 44 | 42 | 42 | 41 | 43 | 44 | 44 | 42 | 44 | 69 | 49 | 43 | 41 | 42 | 45 |
| 3. Arath_BRXL1 | 76 | 86 | | 47 | 50 | 42 | 44 | 67 | 44 | 42 | 61 | 42 | 48 | 45 | 42 | 45 | 42 | 42 | 43 | 42 | 44 | 46 | 43 | 42 | 66 | 50 | 41 | 41 | 41 | 43 |
| 4. Arath_BRXL2 | 64 | 61 | 59 | | 73 | 55 | 61 | 49 | 61 | 54 | 49 | 55 | 70 | 60 | 56 | 39 | 43 | 53 | 43 | 59 | 59 | 68 | 53 | 54 | 52 | 69 | 54 | 51 | 38 | 39 |
| 5. Arath_BRXL3 | 66 | 68 | 64 | 82 | | 55 | 58 | 49 | 59 | 53 | 51 | 53 | 67 | 60 | 54 | 39 | 40 | 52 | 44 | 57 | 58 | 67 | 50 | 53 | 53 | 66 | 52 | 51 | 40 | 40 |
| 6. Arath_BRXL4 | 60 | 57 | 56 | 72 | 69 | | 67 | 47 | 68 | 63 | 44 | 62 | 62 | 64 | 54 | 40 | 39 | 53 | 44 | 66 | 67 | 62 | 51 | 53 | 49 | 63 | 53 | 51 | 38 | 41 |
| 7. Glyma_BRXL1 | 66 | 58 | 58 | 77 | 75 | 78 | | 50 | 97 | 72 | 48 | 73 | 69 | 72 | 56 | 41 | 40 | 55 | 46 | 75 | 78 | 66 | 52 | 55 | 52 | 72 | 55 | 52 | 40 | 39 |
| 8. Glyma_BRXL2 | 86 | 83 | 79 | 63 | 66 | 60 | 64 | | 50 | 46 | 76 | 46 | 52 | 48 | 44 | 46 | 44 | 44 | 45 | 48 | 48 | 50 | 44 | 44 | 77 | 53 | 44 | 42 | 45 | 46 |
| 9. Glyma_BRXL3 | 66 | 59 | 60 | 77 | 75 | 79 | 98 | 65 | | 73 | 48 | 74 | 71 | 73 | 56 | 42 | 41 | 55 | 46 | 76 | 80 | 68 | 53 | 56 | 53 | 72 | 56 | 52 | 39 | 38 |
| 10. Glyma_BRXL4 | 63 | 59 | 57 | 72 | 72 | 74 | 82 | 62 | 83 | | 45 | 91 | 62 | 64 | 52 | 39 | 41 | 51 | 44 | 66 | 68 | 61 | 50 | 53 | 47 | 63 | 52 | 50 | 39 | 40 |
| 11. Glyma_BRXL5 | 81 | 77 | 74 | 62 | 66 | 60 | 66 | 87 | 66 | 63 | | 45 | 50 | 48 | 42 | 43 | 45 | 41 | 42 | 46 | 46 | 47 | 43 | 42 | 71 | 53 | 42 | 41 | 45 | 44 |
| 12. Glyma_BRXL6 | 64 | 60 | 57 | 72 | 70 | 75 | 83 | 62 | 83 | 94 | 63 | | 62 | 65 | 53 | 40 | 41 | 53 | 46 | 66 | 67 | 61 | 49 | 54 | 48 | 65 | 54 | 49 | 41 | 40 |
| 13. Medtr_BRXL1 | 70 | 63 | 62 | 83 | 81 | 76 | 82 | 66 | 82 | 75 | 66 | 76 | | 67 | 59 | 42 | 44 | 60 | 48 | 65 | 68 | 76 | 57 | 61 | 57 | 80 | 59 | 56 | 38 | 42 |
| 14. Nicbe_BRXL1 | 66 | 60 | 60 | 75 | 74 | 78 | 83 | 61 | 83 | 78 | 63 | 77 | 78 | | 56 | 39 | 42 | 54 | 45 | 67 | 70 | 63 | 53 | 55 | 51 | 70 | 54 | 53 | 39 | 38 |
| 15. Orysa_BRXL1 | 58 | 56 | 55 | 69 | 65 | 67 | 67 | 57 | 67 | 63 | 55 | 64 | 70 | 68 | | 37 | 40 | 70 | 40 | 54 | 58 | 58 | 72 | 72 | 48 | 61 | 71 | 69 | 37 | 39 |
| 16. Orysa_BRXL2 | 59 | 56 | 55 | 54 | 52 | 54 | 53 | 57 | 53 | 51 | 56 | 51 | 56 | 53 | 52 | | 44 | 41 | 35 | 41 | 41 | 41 | 38 | 39 | 47 | 42 | 39 | 38 | 42 | 72 |
| 17. Orysa_BRXL4 | 61 | 58 | 56 | 59 | 56 | 54 | 56 | 60 | 56 | 52 | 62 | 55 | 61 | 58 | 52 | 57 | | 39 | 38 | 40 | 40 | 40 | 38 | 41 | 47 | 44 | 38 | 40 | 76 | 45 |
| 18. Orysa_BRXL5 | 57 | 54 | 53 | 65 | 63 | 64 | 66 | 55 | 66 | 62 | 54 | 63 | 69 | 66 | 78 | 54 | 52 | | 42 | 55 | 57 | 59 | 69 | 85 | 48 | 63 | 82 | 68 | 38 | 41 |
| 19. Picsi_BRXL1 | 58 | 59 | 59 | 60 | 66 | 60 | 61 | 59 | 61 | 59 | 58 | 61 | 65 | 59 | 54 | 48 | 55 | 52 | | 45 | 47 | 44 | 40 | 41 | 44 | 47 | 42 | 40 | 35 | 36 |
| 20. Poptr_BRXL1 | 64 | 60 | 56 | 75 | 73 | 78 | 86 | 63 | 87 | 79 | 61 | 78 | 78 | 80 | 66 | 51 | 54 | 66 | 60 | | 86 | 66 | 53 | 56 | 52 | 70 | 55 | 51 | 37 | 39 |
| 21. Poptr_BRXL2 | 63 | 58 | 57 | 74 | 71 | 81 | 86 | 61 | 87 | 78 | 60 | 77 | 80 | 80 | 70 | 53 | 53 | 70 | 60 | 88 | | 67 | 55 | 58 | 53 | 73 | 58 | 54 | 38 | 40 |
| 22. Poptr_BRXL3 | 65 | 59 | 60 | 82 | 78 | 76 | 78 | 63 | 79 | 74 | 63 | 74 | 83 | 75 | 69 | 54 | 55 | 67 | 59 | 79 | 78 | | 58 | 60 | 53 | 80 | 59 | 55 | 38 | 40 |
| 23. Sorbi_BRXL1 | 56 | 55 | 55 | 66 | 62 | 64 | 66 | 56 | 66 | 62 | 56 | 60 | 70 | 66 | 80 | 52 | 49 | 79 | 55 | 65 | 69 | 68 | | 70 | 46 | 59 | 70 | 84 | 38 | 37 |
| 24. Sorbi_BRXL2 | 58 | 54 | 54 | 67 | 64 | 65 | 68 | 57 | 69 | 65 | 55 | 64 | 70 | 65 | 79 | 52 | 53 | 89 | 54 | 67 | 70 | 69 | 82 | | 48 | 64 | 92 | 68 | 38 | 40 |
| 25. Vitvi_BRXL1 | 87 | 79 | 76 | 66 | 69 | 62 | 66 | 84 | 67 | 63 | 79 | 64 | 71 | 66 | 61 | 57 | 63 | 59 | 58 | 65 | 65 | 67 | 58 | 60 | | 58 | 47 | 46 | 45 | 47 |
| 26. Vitvi_BRXL2 | 70 | 63 | 63 | 84 | 81 | 78 | 85 | 66 | 85 | 76 | 69 | 78 | 89 | 83 | 73 | 54 | 60 | 72 | 62 | 82 | 82 | 87 | 71 | 73 | 71 | | 61 | 58 | 40 | 43 |
| 27. Zeama_BRXL1 | 58 | 55 | 54 | 66 | 63 | 65 | 66 | 57 | 67 | 64 | 56 | 64 | 69 | 67 | 79 | 52 | 52 | 88 | 53 | 66 | 68 | 70 | 82 | 95 | 60 | 72 | | 68 | 39 | 39 |
| 28. Zeama_BRXL2 | 54 | 53 | 53 | 65 | 63 | 64 | 65 | 53 | 65 | 63 | 56 | 62 | 67 | 67 | 78 | 53 | 50 | 78 | 54 | 62 | 67 | 66 | 89 | 79 | 57 | 69 | 79 | | 38 | 37 |
| 29. Zeama_BRXL3 | 59 | 59 | 58 | 56 | 55 | 57 | 56 | 60 | 56 | 52 | 59 | 54 | 57 | 55 | 50 | 57 | 86 | 52 | 51 | 53 | 55 | 54 | 53 | 52 | 60 | 58 | 54 | 52 | | 42 |
| 30. Zeama_BRXL4 | 61 | 57 | 55 | 54 | 54 | 56 | 53 | 60 | 54 | 53 | 58 | 53 | 56 | 53 | 52 | 82 | 59 | 51 | 51 | 50 | 56 | 53 | 48 | 50 | 61 | 56 | 52 | 49 | 58 | |

The percentage amino acid identity can be significantly increased if the most conserved region of the polypeptides are compared. For example, when comparing the amino acid sequence of a Conserved Domain 1 as represented by SEQ ID NO: 83, or of a Conserved domain 2 as represented by SEQ ID NO: 84 with the respective corresponding domains of the polypeptides of Table A3, the percentage amino acid identity increases significantly (in order of preference at least 50%, 55%, 60%, 65%, 70%, 75%, 80%, 85%, 90%, 95%, 98%, 99% or more amino acid sequence identity).

### 3.4. silky-1-like polypeptides

Global percentages of similarity and identity between full length polypeptide sequences is determined using one of the methods available in the art, the MatGAT (Matrix Global Alignment Tool) software (BMC Bioinformatics. 2003 4:29. MatGAT: an application that generates similarity/identity matrices using protein or DNA sequences. Campanella JJ, Bitincka L, Smalley J; software hosted by Ledion Bitincka). MatGAT software generates similarity/identity matrices for DNA or protein sequences without needing pre-alignment of the data. The program performs a series of pair-wise alignments using the Myers and Miller global alignment algorithm (with a gap opening penalty of 12, and a gap extension penalty of 2), calculates similarity and identity using for example Blosum 62 (for polypeptides), and then places the results in a distance matrix. Sequence similarity is shown in the bottom half of the dividing line and sequence identity is shown in the top half of the diagonal dividing line.

Parameters used in the comparison are:

| | |
|---|---|
| Scoring matrix: | Blosum62 |
| First Gap: | 12 |
| Extending gap: | 2 |

A MATGAT table for local alignment of a specific domain, or data on % identity/similarity between specific domains may also be performed.

### 3.5. ARP6 polypeptides

Global percentages of similarity and identity between full length polypeptide sequences useful in performing the methods of the invention were determined using one of the methods available in the art, the MatGAT (Matrix Global Alignment Tool) software (BMC Bioinformatics. 2003 4:29. MatGAT: an application that generates similarity/identity matrices using protein or DNA sequences. Campanella JJ, Bitincka L, Smalley J; software hosted by Ledion Bitincka). MatGAT software generates similarity/identity matrices for DNA or protein sequences without needing pre-alignment of the data. The program performs a series of pair-wise alignments using the Myers and Miller global alignment algorithm (with a gap opening penalty of 12, and a gap extension penalty of 2), calculates similarity and identity using for example Blosum 62 (for polypeptides), and then places the results in a distance matrix. Sequence similarity is shown in the bottom half of the dividing line and sequence identity is shown in the top half of the diagonal dividing line.

Parameters used in the comparison were:
Scoring matrix: Blosum62
First Gap: 12
Extending gap: 2

Results of the software analysis are shown in Table B2 for the global similarity and identity over the full length of the polypeptide sequences. Percentage identity is given above the diagonal in bold and percentage similarity is given below the diagonal (normal face).

The percentage identity between the ARP6 polypeptide sequences useful in performing the methods of the invention can be as low as 50.8 % amino acid identity compared to SEQ ID NO: 102 (A.thaliana_AT3G33520.1).

**Table B2: MatGAT results for global similarity and identity over the full length of the polypeptide sequences.**

| | 1 | 2 | 3 | 4 | 5 | 6 |
|---|---|---|---|---|---|---|
| 1. Gm0057x00075 | | 65.1 | 53.6 | 37.0 | 44.1 | 50.8 |
| 2. Gm0195x00030 | 72.7 | | 76.7 | 50.7 | 64.5 | 70.1 |
| 3. P.trichocarpa_scaff_XVIII.1149 | 67.5 | 88.8 | | 52.8 | 65.4 | 74.5 |
| 4. P.patens_126969 | 54.9 | 71.1 | 69.9 | | 51.8 | 50.8 |
| 5. O.sativa_LOC_Os01g16414.1 | 62.0 | 81.9 | 83.0 | 70.4 | | 63.1 |
| 6. A.thaliana_AT3G33520.1 | 63.9 | 83.5 | 88.9 | 68.6 | 79.3 | |

### 3.6. POP polypeptides

Global percentages of similarity and identity between full length polypeptide sequences useful in performing the methods of the invention were determined using one of the methods available in the art, the MatGAT (Matrix Global Alignment Tool) software (BMC Bioinformatics. 2003 4:29. MatGAT: an application that generates similarity/identity matrices using protein or DNA sequences. Campanella JJ, Bitincka L, Smalley J; software hosted by Ledion Bitincka). MatGAT software generates similarity/identity matrices for DNA or protein sequences without needing pre-alignment of the data. The program performs a series of pair-wise alignments using the Myers and Miller global alignment algorithm (with a gap opening penalty of 12, and a gap extension penalty of 2), calculates similarity and identity using for example Blosum 62 (for polypeptides), and then places the results in a distance matrix. Sequence similarity is shown in the bottom half of the dividing line and sequence identity is shown in the top half of the diagonal dividing line.

Parameters used in the comparison were:

| | |
|---|---|
| Scoring matrix: | Blosum62 |
| First Gap: | 12 |
| Extending gap: | 2 |

Results of the software analysis are shown in Table B3 for the global similarity and identity over the full length of the polypeptide sequences. Percentage identity is given above the diagonal in bold and percentage similarity is given below the diagonal (normal face).

The percentage identity between the POP polypeptide sequences useful in performing the methods of the invention can be as low as 16.8 % amino acid identity compared to SEQ ID NO: 117 (At5g24260).

**Table B3: MatGAT results for global similarity and identity over the full length of the polypeptide sequences.**

| | 1 | 2 | 3 | 4 | 5 | 6 | 7 | 8 | 9 | 10 |
|---|---|---|---|---|---|---|---|---|---|---|
| 1. At5g24260 | | 65.7 | 51.1 | 59.3 | 59.4 | 65.8 | 65.5 | 18.1 | 16.8 | 16.9 |
| 2. Mt_AC137828 | 80.4 | | 51.4 | 58.6 | 58.6 | 72.1 | 72.6 | 18.5 | 18.6 | 18.5 |
| 3. Os02g0290600 | 64.3 | 64.3 | | 47.8 | 48.1 | 53.0 | 54.0 | 18.3 | 18.4 | 20.7 |
| 4. Pp_104540 | 74.7 | 74.0 | 61.6 | | 91.9 | 58.2 | 58.5 | 18.8 | 17.0 | 18.2 |
| 5. Pp_105959 | 74.1 | 73.9 | 61.3 | 96.0 | | 57.9 | 58.7 | 18.8 | 16.6 | 18.0 |
| 6. Pt_scaff_XII.203 | 78.9 | 85.0 | 63.5 | 73.0 | 73.2 | | 91.1 | 19.3 | 17.7 | 18.8 |
| 7. Pt_scaff_XV.146 | 78.3 | 85.4 | 63.9 | 72.7 | 73.5 | 96.0 | | 19.6 | 17.4 | 17.6 |
| 8. NP_193193.2At | 36.0 | 37.5 | 32.6 | 36.3 | 36.3 | 37.8 | 36.2 | | 19.5 | 18.9 |
| 9. NP_198470.3At | 36.9 | 36.6 | 33.6 | 33.4 | 33.9 | 35.2 | 35.3 | 37.6 | | 60.5 |
| 10. ABK25153.1Ps | 35.9 | 35.1 | 35.2 | 35.2 | 34.6 | 34.3 | 34.2 | 38.7 | 74.7 | |

### 3.7. Crumpled Leaf (CRL) polypeptides

Global percentages of similarity and identity between full length polypeptide sequences useful in performing the methods of the invention were determined using one of the methods available in the art, the MatGAT (Matrix Global Alignment Tool) software (BMC Bioinformatics. 2003 4:29. MatGAT: an application that generates similarity/identity matrices using protein or DNA sequences. Campanella JJ, Bitincka L, Smalley J; software hosted by Ledion Bitincka). MatGAT software generates similarity/identity matrices for DNA or protein sequences without needing pre-alignment of the data. The program performs a series of pair-wise alignments using the Myers and Miller global alignment algorithm (with a gap opening penalty of 12, and a gap extension penalty of 2), calculates similarity and identity using for example Blosum 62 (for polypeptides), and then places the results in a distance matrix. Sequence similarity is shown in the bottom half of the dividing line and sequence identity is shown in the top half of the diagonal dividing line.

Parameters used in the comparison were:

| | |
|---|---|
| Scoring matrix: | Blosum62 |
| First Gap: | 12 |
| Extending gap: | 2 |

Results of the software analysis are shown in Table B4 for the global similarity and identity over the full length of the polypeptide sequences. Percentage identity is given above the diagonal in bold and percentage similarity is given below the diagonal (normal face).

The percentage identity between the CRL polypeptide sequences useful in performing the methods of the invention can be as low as 48 % amino acid identity compared to SEQ ID NO: 156.

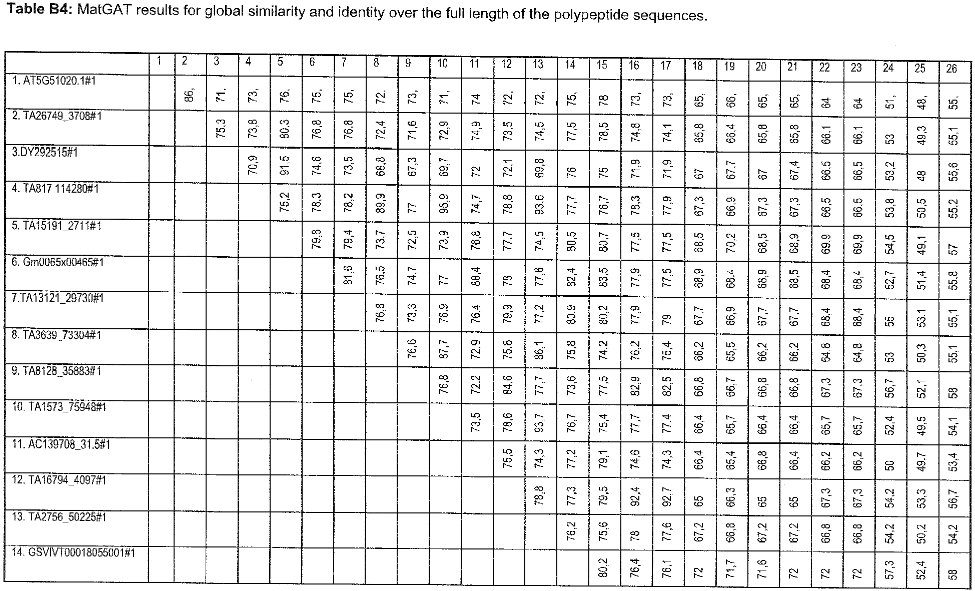

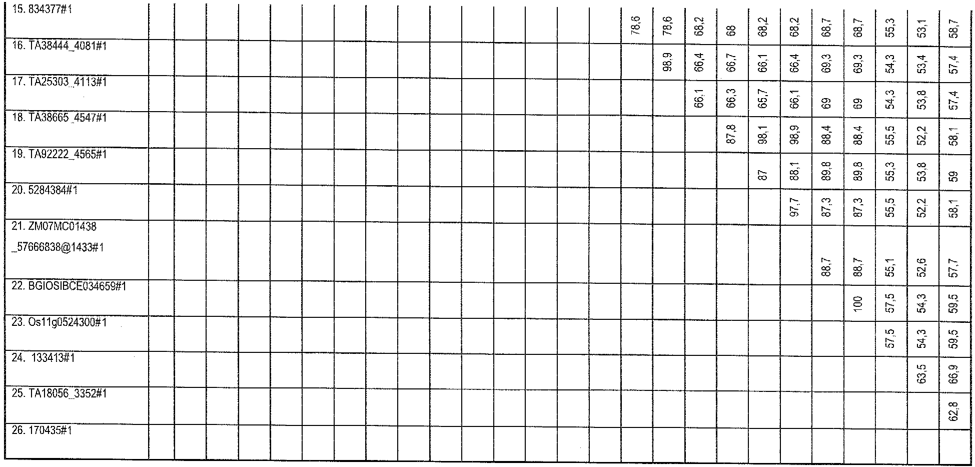

### Example 4: Identification of domains comprised in polypeptide sequences useful in performing the methods of the invention

### 4.1. Alfin-like polypeptides

The Integrated Resource of Protein Families, Domains and Sites (InterPro) database is an integrated interface for the commonly used signature databases for text- and sequence-based searches. The InterPro database combines these databases, which use different methodologies and varying degrees of biological information about well-characterized proteins to derive protein signatures. Collaborating databases include SWISS-PROT, PROSITE, TrEMBL, PRINTS, ProDom and Pfam, Smart and TIGRFAMs. Pfam is a large collection of multiple sequence alignments and hidden Markov models covering many common protein domains and families. Pfam is hosted at the Sanger Institute server in the United Kingdom. Interpro is hosted at the European Bioinformatics Institute in the United Kingdom.

### 4.2. YRP polypeptides

The Integrated Resource of Protein Families, Domains and Sites (InterPro) database is an integrated interface for the commonly used signature databases for text- and sequence-based searches. The InterPro database combines these databases, which use different methodologies and varying degrees of biological information about well-characterized proteins to derive protein signatures. Collaborating databases include SWISS-PROT, PROSITE, TrEMBL, PRINTS, ProDom and Pfam, Smart and TIGRFAMs. Pfam is a large collection of multiple sequence alignments and hidden Markov models covering many common protein domains and families. Pfam is hosted at the Sanger Institute server in the United Kingdom. Interpro is hosted at the European Bioinformatics Institute in the United Kingdom.

### 4.3. Brevis Radix-like (BRXL) polypeptides

The Integrated Resource of Protein Families, Domains and Sites (InterPro) database is an integrated interface for the commonly used signature databases for text- and sequence-based searches. The InterPro database combines these databases, which use different methodologies and varying degrees of biological information about well-characterized proteins to derive protein signatures. Collaborating databases include SWISS-PROT, PROSITE, TrEMBL, PRINTS, ProDom and Pfam, Smart and TIGRFAMs. Interpro is hosted at the European Bioinformatics Institute in the United Kingdom.

The results of the InterPro scan of the polypeptide sequence as represented by SEQ ID NO: 18 are presented in Table C1.

**Table C1: InterPro scan results of the polypeptide sequence as represented by SEQ ID NO: 18**

| **InterPro accession number and name** | **Integrated database name** | **Integrated database accession number** | **Integrated database accession name** |
|---|---|---|---|
| IPR13591 Disease resistance / zinc finger / chromosome condensation-like region (DZC) | PFAM | PF08381 | DZC |
| No IPR integrated | Panther | PTHR22870 | Regulator of chromosome condensation |
| | Panther | PTHR22870_SF25 | Regulator of chromosome condensation |

### 4.4. silky-1-like polypeptides

The Integrated Resource of Protein Families, Domains and Sites (InterPro) database is an integrated interface for the commonly used signature databases for text- and sequence-based searches. The InterPro database combines these databases, which use different methodologies and varying degrees of biological information about well-characterized proteins to derive protein signatures. Collaborating databases include SWISS-PROT, PROSITE, TrEMBL, PRINTS, ProDom and Pfam, Smart and TIGRFAMs. Pfam is a large collection of multiple sequence alignments and hidden Markov models covering many common protein domains and families. Pfam is hosted at the Sanger Institute server in the United Kingdom. Interpro is hosted at the European Bioinformatics Institute in the United Kingdom.

### 4.5. ARP6 polypeptides

The Integrated Resource of Protein Families, Domains and Sites (InterPro) database is an integrated interface for the commonly used signature databases for text- and sequence-based searches. The InterPro database combines these databases, which use different methodologies and varying degrees of biological information about well-characterized proteins to derive protein signatures. Collaborating databases include SWISS-PROT, PROSITE, TrEMBL, PRINTS, ProDom and Pfam, Smart and TIGRFAMs. Pfam is a large collection of multiple sequence alignments and hidden Markov models covering many common protein domains and families. Pfam is hosted at the Sanger Institute server in the United Kingdom. Interpro is hosted at the European Bioinformatics Institute in the United Kingdom.

### 4.6. POP polypeptides

The Integrated Resource of Protein Families, Domains and Sites (InterPro) database is an integrated interface for the commonly used signature databases for text- and sequence-based searches. The InterPro database combines these databases, which use different proteins to derive protein signatures. Collaborating databases include SWISS-PROT, PROSITE, TrEMBL, PRINTS, ProDom and Pfam, Smart and TIGRFAMs. Pfam is a large collection of multiple sequence alignments and hidden Markov models covering many common protein domains and families. Pfam is hosted at the Sanger Institute server in the United Kingdom. Interpro is hosted at the European Bioinformatics Institute in the United Kingdom.

The results of the InterPro scan of the polypeptide sequence as represented by SEQ ID NO: 117 are presented in Table C2.

**Table C2: InterPro scan results (major accession numbers) of the polypeptide sequence as represented by SEQ ID NO: 117.**

| **Database** | **Accession number** | **Accession name** | **Amino acid coordinates on SEQ ID NO 117** |
|---|---|---|---|
| InterPro | IPR001375 | Peptidase S9, prolyl oligopeptidase active site region | |
| HMMPfam | PF00326 | Peptidase_S9 | T[544-746] 1.3e-57 |
| InterPro | IPR002469 | Peptidase S9B, dipeptidylpeptidase IV N-terminal | |
| HMMPfam | PF00930 | DPPIV_N | T[103-458] 2.1e-129 |
| Gene3D | G3DSA:2.140.10.30 | no description | T[77-488] 6.3e-90 |
| Gene3D | G3DSA:3.40.50.1820 | no description | T[490-746] 4.7e-73 |
| HMMPanther | PTHR11731 | PROTEASE FAMILY S9B,C DIPEPTIDYL-PEPTIDASE IV-RELATED | T[116-746] 6.1e-293 |
| HMMPanther | PTHR11731SF12 | DIPEPTIDYL PEPTIDASE IV | T[116-746] 6.1e-293 |
| Superfamily | SSF53474 | alpha/beta-Hydrolases | T[490-746] 3.4e-65 |
| Superfamily | SSF82171 | Dipeptidyl peptidase IV/CD26, N-terminal domain | T[4-486] 9e-78 |

### 4.7. Crumpled Leaf (CRL) polypeptides

The Integrated Resource of Protein Families, Domains and Sites (InterPro) database is an integrated interface for the commonly used signature databases for text- and sequence-based searches. The InterPro database combines these databases, which use different methodologies and varying degrees of biological information about well-characterized proteins to derive protein signatures. Collaborating databases include SWISS-PROT, PROSITE, TrEMBL, PRINTS, ProDom and Pfam, Smart and TIGRFAMs. Pfam is a large collection of multiple sequence alignments and hidden Markov models covering many common protein domains and families. Pfam is hosted at the Sanger Institute server in the United Kingdom. Interpro is hosted at the European Bioinformatics Institute in the United Kingdom.

The results of the InterPro scan of the polypeptide sequence as represented by SEQ ID NO: 156 are presented in Table C3.

**Table C3: InterPro scan results (major accession numbers) of the polypeptide sequence as represented by SEQ ID NO: 156.**

| Database | Accession number | Description | evalue | amino acid coordinates [start-end] |
|---|---|---|---|---|
| Interpro | IPR010404 | | | |
| PFAM | PF06206 | DUF1001 | 0.043 | [42-236] |

### Example 5: Topology prediction of the polypeptide sequences useful in performing the methods of the invention

### 5.1. Alfin-like polypeptides

TargetP 1.1 predicts the subcellular location of eukaryotic proteins. The location assignment is based on the predicted presence of any of the N-terminal pre-sequences: chloroplast transit peptide (cTP), mitochondrial targeting peptide (mTP) or secretory pathway signal peptide (SP). Scores on which the final prediction is based are not really probabilities, and they do not necessarily add to one. However, the location with the highest score is the most likely according to TargetP, and the relationship between the scores (the reliability class) may be an indication of how certain the prediction is. The reliability class (RC) ranges from 1 to 5, where 1 indicates the strongest prediction. TargetP is maintained at the server of the Technical University of Denmark. For the sequences predicted to contain an N-terminal presequence a potential cleavage site can also be predicted.

A number of parameters are selected, such as organism group (non-plant or plant), cutoff sets (none, predefined set of cutoffs, or user-specified set of cutoffs), and the calculation of prediction of cleavage sites (yes or no).

Many other algorithms can be used to perform such analyses, including:
- ChloroP 1.1 hosted on the server of the Technical University of Denmark;
- Protein Prowler Subcellular Localisation Predictor version 1.2 hosted on the server of the Institute for Molecular Bioscience, University of Queensland, Brisbane, Australia;
- PENCE Proteome Analyst PA-GOSUB 2.5 hosted on the server of the University of Alberta, Edmonton, Alberta, Canada;
- TMHMM, hosted on the server of the Technical University of Denmark
- PSORT (URL: psort.org)
- PLOC (Park and Kanehisa, Bioinformatics, 19, 1656-1663, 2003).

### 5.2. YRP polypeptides

TargetP 1.1 predicts the subcellular location of eukaryotic proteins. The location assignment is based on the predicted presence of any of the N-terminal pre-sequences: chloroplast transit peptide (cTP), mitochondrial targeting peptide (mTP) or secretory pathway signal peptide (SP). Scores on which the final prediction is based are not really probabilities, and they do not necessarily add to one. However, the location with the highest score is the most likely according to TargetP, and the relationship between the scores (the reliability class) may be an indication of how certain the prediction is. The reliability class (RC) ranges from 1 to 5, where 1 indicates the strongest prediction. TargetP is maintained at the server of the Technical University of Denmark. For the sequences predicted to contain an N-terminal presequence a potential cleavage site can also be predicted.

A number of parameters are selected, such as organism group (non-plant or plant), cutoff sets (none, predefined set of cutoffs, or user-specified set of cutoffs), and the calculation of prediction of cleavage sites (yes or no).

Many other algorithms can be used to perform such analyses, including:
- ChloroP 1.1 hosted on the server of the Technical University of Denmark;
- Protein Prowler Subcellular Localisation Predictor version 1.2 hosted on the server of the Institute for Molecular Bioscience, University of Queensland, Brisbane, Australia;
- PENCE Proteome Analyst PA-GOSUB 2.5 hosted on the server of the University of Alberta, Edmonton, Alberta, Canada;
- TMHMM, hosted on the server of the Technical University of Denmark
- PSORT (URL: psort.org)
- PLOC (Park and Kanehisa, Bioinformatics, 19, 1656-1663, 2003).

### 5.3. silky-1-like polypeptides

TargetP 1.1 predicts the subcellular location of eukaryotic proteins. The location assignment is based on the predicted presence of any of the N-terminal pre-sequences: chloroplast transit peptide (cTP), mitochondrial targeting peptide (mTP) or secretory pathway signal peptide (SP). Scores on which the final prediction is based are not really probabilities, and they do not necessarily add to one. However, the location with the highest score is the most likely according to TargetP, and the relationship between the scores (the reliability class) may be an indication of how certain the prediction is. The reliability class (RC) ranges from 1 to 5, where 1 indicates the strongest prediction. TargetP is maintained at the server of the Technical University of Denmark. For the sequences predicted to contain an N-terminal presequence a potential cleavage site can also be predicted.

A number of parameters are selected, such as organism group (non-plant or plant), cutoff sets (none, predefined set of cutoffs, or user-specified set of cutoffs), and the calculation of prediction of cleavage sites (yes or no).

Many other algorithms can be used to perform such analyses, including:
- ChloroP 1.1 hosted on the server of the Technical University of Denmark;
- Protein Prowler Subcellular Localisation Predictor version 1.2 hosted on the server of the Institute for Molecular Bioscience, University of Queensland, Brisbane, Australia;
- PENCE Proteome Analyst PA-GOSUB 2.5 hosted on the server of the University of Alberta, Edmonton, Alberta, Canada;
- TMHMM, hosted on the server of the Technical University of Denmark
- PSORT (URL: psort.org)
- PLOC (Park and Kanehisa, Bioinformatics, 19, 1656-1663, 2003).

### 5.4. ARP6 polypeptides

TargetP 1.1 predicts the subcellular location of eukaryotic proteins. The location assignment is based on the predicted presence of any of the N-terminal pre-sequences: chloroplast transit peptide (cTP), mitochondrial targeting peptide (mTP) or secretory pathway signal peptide (SP). Scores on which the final prediction is based are not really probabilities, and they do not necessarily add to one. However, the location with the highest score is the most likely according to TargetP, and the relationship between the scores (the reliability class) may be an indication of how certain the prediction is. The reliability class (RC) ranges from 1 to 5, where 1 indicates the strongest prediction. TargetP is maintained at the server of the Technical University of Denmark.

For the sequences predicted to contain an N-terminal presequence a potential cleavage site can also be predicted.

A number of parameters are selected, such as organism group (non-plant or plant), cutoff sets (none, predefined set of cutoffs, or user-specified set of cutoffs), and the calculation of prediction of cleavage sites (yes or no).

Many other algorithms can be used to perform such analyses, including:
- ChloroP 1.1 hosted on the server of the Technical University of Denmark;
- Protein Prowler Subcellular Localisation Predictor version 1.2 hosted on the server of the Institute for Molecular Bioscience, University of Queensland, Brisbane, Australia;
- PENCE Proteome Analyst PA-GOSUB 2.5 hosted on the server of the University of Alberta, Edmonton, Alberta, Canada;
- TMHMM, hosted on the server of the Technical University of Denmark
- PSORT (URL: psort.org)
- PLOC (Park and Kanehisa, Bioinformatics, 19, 1656-1663, 2003).

### 5.5. POP polypeptides

TargetP 1.1 predicts the subcellular location of eukaryotic proteins. The location assignment is based on the predicted presence of any of the N-terminal pre-sequences: chloroplast transit peptide (cTP), mitochondrial targeting peptide (mTP) or secretory pathway signal peptide (SP). Scores on which the final prediction is based are not really probabilities, and they do not necessarily add to one. However, the location with the highest score is the most likely according to TargetP, and the relationship between the scores (the reliability class) may be an indication of how certain the prediction is. The reliability class (RC) ranges from 1 to 5, where 1 indicates the strongest prediction. TargetP is maintained at the server of the Technical University of Denmark.

For the sequences predicted to contain an N-terminal presequence a potential cleavage site can also be predicted.

A number of parameters were selected, such as organism group (non-plant or plant), cutoff sets (none, predefined set of cutoffs, or user-specified set of cutoffs), and the calculation of prediction of cleavage sites (yes or no).

The results of TargetP 1.1 analysis of the polypeptide sequence as represented by SEQ ID NO: 117 are presented Table D1. The "plant" organism group has been selected, no cutoffs defined, and the predicted length of the transit peptide requested. The subcellular localization of the polypeptide sequence as represented by SEQ ID NO: 117 may be the cytoplasm or nucleus, no transit peptide is predicted.

Table D1: TargetP 1.1 analysis of the polypeptide sequence as represented by SEQ ID NO: 117. Abbreviations: Len, Length; cTP, Chloroplastic transit peptide; mTP, Mitochondrial transit peptide, SP, Secretory pathway signal peptide, other, Other subcellular targeting, Loc, Predicted Location; RC, Reliability class; TPlen, Predicted transit peptide length.

| Name | Len | cTP | mTP | SP | other | Loc | RC | TPlen |
|---|---|---|---|---|---|---|---|---|
| POP | 746 | 0.083 | 0.100 | 0.079 | 0.914 | _ | 1 | - |
| cutoff | | 0.000 | 0.000 | 0.000 | 0.000 | | | |

Many other algorithms can be used to perform such analyses, including:
- ChloroP 1.1 hosted on the server of the Technical University of Denmark;
- Protein Prowler Subcellular Localisation Predictor version 1.2 hosted on the server of the Institute for Molecular Bioscience, University of Queensland, Brisbane, Australia;
- PENCE Proteome Analyst PA-GOSUB 2.5 hosted on the server of the University of Alberta, Edmonton, Alberta, Canada;
- TMHMM, hosted on the server of the Technical University of Denmark
- PSORT (URL: psort.org)
- PLOC (Park and Kanehisa, Bioinformatics, 19, 1656-1663, 2003).

### 5.6. Crumpled Leaf (CRL) polypeptides

TargetP 1.1 predicts the subcellular location of eukaryotic proteins. The location assignment is based on the predicted presence of any of the N-terminal pre-sequences: chloroplast transit peptide (cTP), mitochondrial targeting peptide (mTP) or secretory pathway signal peptide (SP). Scores on which the final prediction is based are not really probabilities, and they do not necessarily add to one. However, the location with the highest score is the most likely according to TargetP, and the relationship between the scores (the reliability class) may be an indication of how certain the prediction is. The reliability class (RC) ranges from 1 to 5, where 1 indicates the strongest prediction. TargetP is maintained at the server of the Technical University of Denmark.

For the sequences predicted to contain an N-terminal presequence a potential cleavage site can also be predicted.

A number of parameters are selected, such as organism group (non-plant or plant), cutoff sets (none, predefined set of cutoffs, or user-specified set of cutoffs), and the calculation of prediction of cleavage sites (yes or no).

Many other algorithms can be used to perform such analyses, including:
- ChloroP 1.1 hosted on the server of the Technical University of Denmark;
- Protein Prowler Subcellular Localisation Predictor version 1.2 hosted on the server of the Institute for Molecular Bioscience, University of Queensland, Brisbane, Australia;
- PENCE Proteome Analyst PA-GOSUB 2.5 hosted on the server of the University of Alberta, Edmonton, Alberta, Canada;
- TMHMM, hosted on the server of the Technical University of Denmark
- PSORT (URL: psort.org)
- PLOC (Park and Kanehisa, Bioinformatics, 19, 1656-1663, 2003).

The result of the protein analysis using some of the above mentioned algorithms is shown below:
Psort: endoplasmic reticulum 0.600
WOLFPsort: cyto: 4.0, extr: 3.0, E.R.: 2.5, E.R._plas: 2.5, plas: 1.5, nucl: 1.0, mito: 1.0
TargetP: chloro:0.775 quality 4
ChloroP: not chloroplastic 0.497
MitoProtII: not mitochondrial 0.027
PTS1 peroxisome: not targeted
Phobius: there is a membrane domain [19-36].

### Example 6: Subcellular localisation prediction of the polypeptide sequences useful in performing the methods of the invention

### 6.1. Brevis Radix-like (BRXL) polypeptides

Experimental methods for protein localization range from immunolocalization to tagging of proteins using green fluorescent protein (GFP) or beta-glucuronidase (GUS). Such methods to identify subcellular compartmentalisation of BRXL polypeptides are well known in the art. Computational prediction of protein localisation from sequence data was performed. Among algorithms well known to a person skilled in the art are available at the ExPASy Proteomics tools hosted by the Swiss Institute for Bioinformatics, for example, PSort, TargetP, ChloroP, LocTree, Predotar, LipoP, MITOPROT, PATS, PTS1, SignalP, TMHMM, TMpred, and others.

TargetP 1.1 predicts the subcellular location of eukaryotic proteins. The location assignment is based on the predicted presence of any of the N-terminal pre-sequences: chloroplast transit peptide (cTP), mitochondrial targeting peptide (mTP) or secretory pathway signal peptide (SP). Scores on which the final prediction is based are not really probabilities, and they do not necessarily add to one. However, the location with the highest score is the most likely according to TargetP, and the relationship between the scores (the reliability class) may be an indication of how certain the prediction is. The reliability class (RC) ranges from 1 to 5, where 1 indicates the strongest prediction. TargetP is maintained at the server of the Technical University of Denmark.

For the sequences predicted to contain an N-terminal presequence a potential cleavage site can also be predicted.

A number of parameters were selected, such as organism group (non-plant or plant), cutoff sets (none, predefined set of cutoffs, or user-specified set of cutoffs), and the calculation of prediction of cleavage sites (yes or no).

The results of TargetP 1.1 analysis of the polypeptide sequence as represented by SEQ ID NO: 117 are presented in the Table below. The "plant" organism group has been selected, and no cutoffs defined. The predicted subcellular localization of the polypeptide sequence as represented by SEQ ID NO: 117 is not chloroplastic, not mitochondrial and not the secretory pathway, but most likely the nucleus.

**Table showing TargetP 1.1 analysis of the polypeptide sequence as represented by SEQ ID NO: 117**

| | |
|---|---|
| Length (AA) | 362 |
| Chloroplastic transit peptide | 0.132 |
| Mitochondrial transit peptide | 0.089 |
| Secretory pathway signal peptide | 0.112 |
| Other subcellular targeting | 0.909 |
| Predicted Location | Other |
| Reliability class | 2 |

### Example 7: Assay related to the polypeptide sequences useful in performing the methods of the invention

### 7.1. Brevis Radix-like (BRXL) polypeptides

BRXL polypeptides useful in the methods of the present invention (at least in their native form) typically, but not necessarily, have transcriptional regulatory activity and the capacity to interact with other proteins. DNA-binding activity and protein-protein interactions may readily be determined in vitro or in vivo using techniques well known in the art (for example in Current Protocols in Molecular Biology, Volumes 1 and 2, Ausubel et al. (1994), Current Protocols). BRXL polypeptides comprise the conserved BRX domains, which have been shown to mediate protein-protein interaction in yeast two-hybrid experiments. Homodimerization and heterodimerization within and/or between the BRXL and also PRAF-like protein families is known in th art (Briggs et al. (2006) Plant Physiol 140: 1307-1316; van Leeuwen et al. (2004) Trends Plant Sci 9: 378-384).

### 7.2. POP polypeptides

POP activity can be measured as described by Bastos et al. (Biochem J. 388: 29-38, 2005): POP activity is determined by measuring the fluorescence of AMC (7-amido-4-methylcoumarin) released by hydrolysis of the enzyme substrate N-Suc-Gly-Pro-Leu-Gly-Pro-AMC, where Suc stands for succinyl. POP is assayed in reaction buffer [25 mM Hepes and 5 mM DTT (dithiothreitol), pH 7.5] containing 20 µM substrate in 100 µl final volume. The fluorescence of AMC released by the enzymatic reaction is recorded as described in Grellier et al. (J. Biol. Chem. 276, 47078-47086, 2001). The POP activity can also be assayed using different peptides under the same experimental conditions (N-Boc-Val-Leu-Lys-AMC, N-Boc-Leu-Lys-Arg-AMC, N-Cbz-Val-Lys-Met-AMC, N-Boc-Leu-Gly-Arg-AMC, N-Boc-Ile-Gly-Gly-Arg-AMC, N-Suc-Leu-Tyr-AMC, N-Suc-Ala-Ala-Ala-AMC, N-Boc-Val-Pro-Arg-AMC, N-Suc-Gly-Pro-AMC, N-Cbz-Gly-Gly-Arg-AMC, N-Suc-Ala-Ala-Pro-Phe-AMC, N-Cbz-Phe-Arg-AMC, H-Gly-Arg-AMC, H-Gly-Phe-AMC, Ala-Ala-Phe-AMC, L-Arg-AMC and L-Ala-AMC and L-Lys-Ala-AMC, where Boc and Cbz stand for t-butoxycarbonyl and benzyloxycarbonyl respectively). To determine kinetic parameters, recombinant (0.67 ng) or native (0.26 ng) POP is incubated in reaction buffer with variable N-Suc-Gly-Pro-Leu-Gly-Pro-AMC substrate concentrations (3.12-100 µM) and the AMC release is measured as described above. Kₘ and Vₘₐₓ values are determined by hyperbolic regression using the method of Cornish-Bowden. The k_{cat} is calculated using k_{cat}=Vₘₐₓ/[E]₀, where [E]o represents the active enzyme concentration. Quantification of active POP is performed by incubation of the protein with serial concentrations of the irreversible chloromethane POP Tc80 inhibitor as described in Grellier et al. (2001).

### Example 8: Cloning of the nucleic acid sequence used in the methods of the invention 8.1. Alfin-like polypeptides

The nucleic acid sequence is amplified by PCR using as template a cDNA library (in pCMV Sport 6.0; Invitrogen, Paisley, UK). PCR is performed using Hifi Taq DNA polymerase in standard conditions, using 200 ng of template in a 50 µl PCR mix. The primers include the AttB sites for Gateway recombination. The amplified PCR fragment is purified also using standard methods. The first step of the Gateway procedure, the BP reaction, is then performed, during which the PCR fragment recombines in vivo with the pDONR201 plasmid to produce, according to the Gateway terminology, an "entry clone". Plasmid pDONR201 is purchased from Invitrogen, as part of the Gateway^{®} technology.

The entry clone comprising SEQ ID NO: 1 or SEQ ID NO: 3 is then used in an LR reaction with a destination vector used for Oryza sativa transformation. This vector contains as functional elements within the T-DNA borders: a plant selectable marker; a screenable marker expression cassette; and a Gateway cassette intended for LR in vivo recombination with the nucleic acid sequence of interest already cloned in the entry clone. A rice GOS2 promoter (SEQ ID NO: 7) for constitutive expression is located upstream of this Gateway cassette.

After the LR recombination step, the resulting expression vector pGOS2:: alfin-like (Figure 1) is transformed into Agrobacterium strain LBA4044 according to methods well known in the art.

### 8.2. YRP polypeptides

The nucleic acid sequence is amplified by PCR using as template a cDNA library (in pCMV Sport 6.0; Invitrogen, Paisley, UK). PCR is performed using Hifi Taq DNA polymerase in standard conditions, using 200 ng of template in a 50 µl PCR mix. The primers include the AttB sites for Gateway recombination. The amplified PCR fragment is purified also using standard methods. The first step of the Gateway procedure, the BP reaction, is then performed, during which the PCR fragment recombines in vivo with the pDONR201 plasmid to produce, according to the Gateway terminology, an "entry clone". Plasmid pDONR201 is purchased from Invitrogen, as part of the Gateway^{®} technology.

The entry clone comprising SEQ ID NO: 10 or SEQ ID NO: 12 is then used in an LR reaction with a destination vector used for Oryza sativa transformation. This vector contains as functional elements within the T-DNA borders: a plant selectable marker; a screenable marker expression cassette; and a Gateway cassette intended for LR in vivo recombination with the nucleic acid sequence of interest already cloned in the entry clone. A rice GOS2 promoter (SEQ ID NO: 16) for constitutive expression is located upstream of this Gateway cassette.

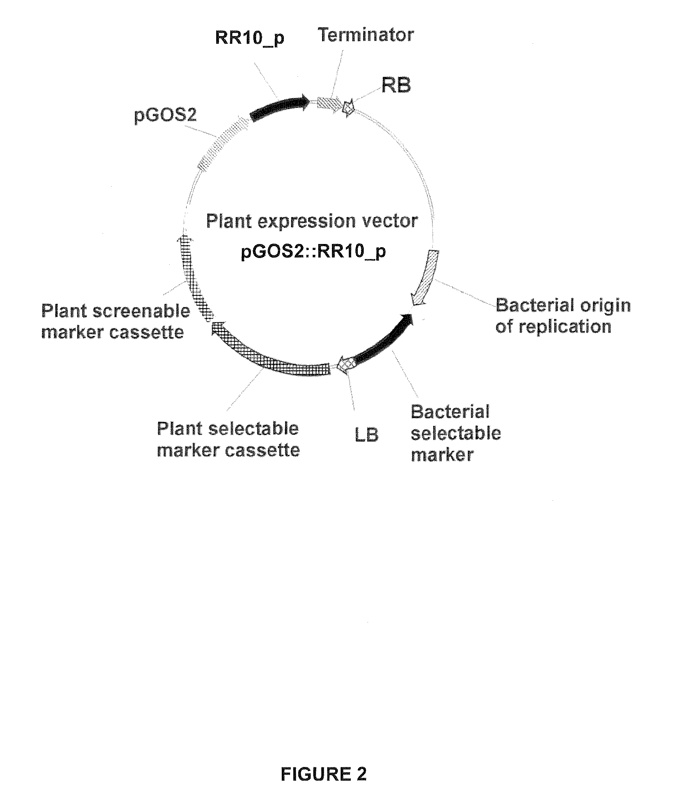

After the LR recombination step, the resulting expression vector pGOS2::YRP (Figure 2) is transformed into Agrobacterium strain LBA4044 according to methods well known in the art.

### 8.3. Brevis Radix-like (BRXL) polypeptides

Unless otherwise stated, recombinant DNA techniques are performed according to standard protocols described in (Sambrook (2001) Molecular Cloning: a laboratory manual, 3rd Edition Cold Spring Harbor Laboratory Press, CSH, New York) or in Volumes 1 and 2 of Ausubel et al. (1994), Current Protocols in Molecular Biology, Current Protocols. Standard materials and methods for plant molecular work are described in Plant Molecular Biology Labfax (1993) by R.D.D. Croy, published by BIOS Scientific Publications Ltd (UK) and Blackwell Scientific Publications (UK).

The poplar nucleic acid sequence encoding a BRXL polypeptide sequence as represented by SEQ ID NO: 18 was amplified by PCR using as template a cDNA bank constructed using RNA from poplar plants at different developmental stages. The following primers, which include the AttB sites for Gateway recombination, were used for PCR amplification:
1) prm 11475 (SEQ ID NO: 88, sense):
   5'-ggggacaagtttgtacaaaaaagcaggcttaaacaatgtttacgtgcatagc-3'
2) prm11476 (SEQ ID NO: 89, reverse, complementary):
   5'-ggggaccactttgtacaagaaagctgggtgcaatttaggtcatgggaaat-3'

PCR was performed using Hifi Taq DNA polymerase in standard conditions. A PCR fragment of the expected length (including attB sites) was amplified and purified also using standard methods. The first step of the Gateway procedure, the BP reaction, was then performed, during which the PCR fragment recombined in vivo with the pDONR201 plasmid to produce, according to the Gateway terminology, an "entry clone". Plasmid pDONR201 was purchased from Invitrogen, as part of the Gateway® technology.

The entry clone comprising SEQ ID NO: 17 was subsequently used in an LR reaction with a destination vector used for Oryza sativa transformation. This vector contained as functional elements within the T-DNA borders: a plant selectable marker; a screenable marker expression cassette; and a Gateway cassette intended for LR in vivo recombination with the nucleic acid sequence of interest already cloned in the entry clone. A rice GOS2 promoter (SEQ ID NO: 87) for constitutive expression was located upstream of this Gateway cassette.

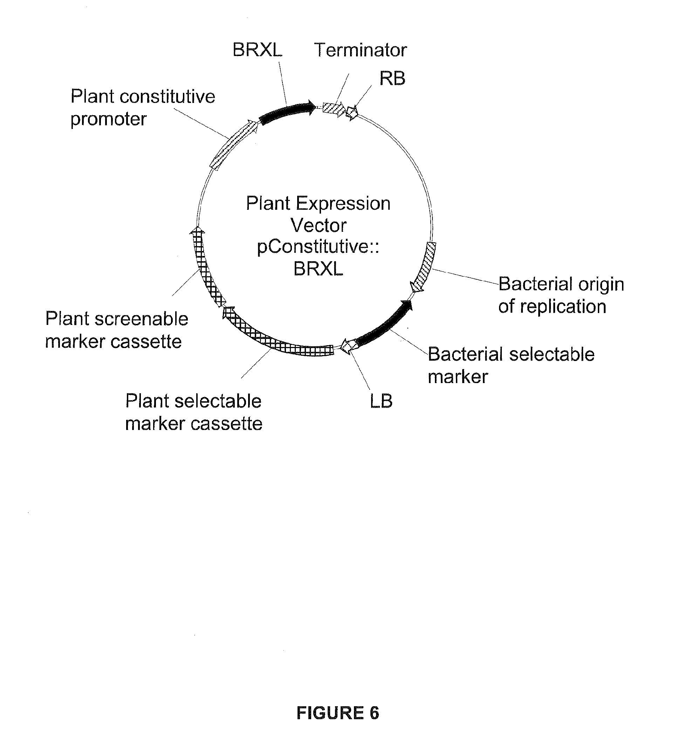

After the LR recombination step, the resulting expression vector pGOS2::BRXL (Figure 6) for constitutive expression was transformed into Agrobacterium strain LBA4044 according to methods well known in the art.

### 8.4. silky-1-like polypeptides

The nucleic acid sequence is amplified by PCR using as template a cDNA library (in pCMV Sport 6.0; Invitrogen, Paisley, UK). PCR is performed using Hifi Taq DNA polymerase in standard conditions, using 200 ng of template in a 50 µl PCR mix. The primers include the AttB sites for Gateway recombination. The amplified PCR fragment is purified also using standard methods. The first step of the Gateway procedure, the BP reaction, is then performed, during which the PCR fragment recombines in vivo with the pDONR201 plasmid to produce, according to the Gateway terminology, an "entry clone". Plasmid pDONR201 is purchased from Invitrogen, as part of the Gateway^{®} technology.

The entry clone comprising SEQ ID NO: 90, SEQ ID NO: 92 or SEQ ID NO: 94 is then used in an LR reaction with a destination vector used for Oryza sativa transformation. This vector contains as functional elements within the T-DNA borders: a plant selectable marker; a screenable marker expression cassette; and a Gateway cassette intended for LR in vivo recombination with the nucleic acid sequence of interest already cloned in the entry clone. A rice GOS2 promoter (SEQ ID NO: 96) for constitutive expression is located upstream of this Gateway cassette.

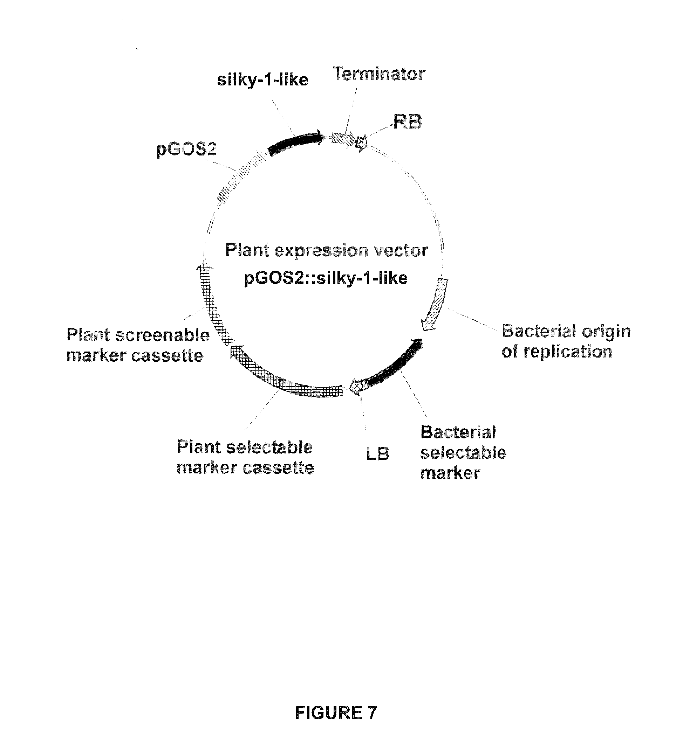

After the LR recombination step, the resulting expression vector pGOS2:: silky-1-like (Figure 7) is transformed into Agrobacterium strain LBA4044 according to methods well known in the art.

### 8.5. ARP6 polypeptides

The nucleic acid sequence used in the methods of the invention was amplified by PCR using as template a custom-made Arabidopsis thaliana seedlings cDNA library (in pCMV Sport 6.0; Invitrogen, Paisley, UK). PCR was performed using Hifi Taq DNA polymerase in standard conditions, using 200 ng of template in a 50 µl PCR mix. The primers used were: (SEQ ID NO: 113; sense, start codon in bold): 5'-ggggacaagtttgtacaaaaaagcaggcttaaacaatgtc aaacatcgttgttcta-3' and (SEQ ID NO: 114; reverse, complementary): 5'-ggggaccact ttgtacaagaaagctgggttcatgtgatattgttttggtt-3', which include the AttB sites for Gateway recombination. The amplified PCR fragment was purified also using standard methods. The first step of the Gateway procedure, the BP reaction, was then performed, during which the PCR fragment recombined in vivo with the pDONR201 plasmid to produce, according to the Gateway terminology, an "entry clone", pARP6. Plasmid pDONR201 was purchased from Invitrogen, as part of the Gateway^{®} technology.

The entry clone comprising SEQ ID NO: 101 was then used in an LR reaction with a destination vector used for Oryza sativa transformation. This vector contained as functional elements within the T-DNA borders: a plant selectable marker; a screenable marker expression cassette; and a Gateway cassette intended for LR in vivo recombination with the nucleic acid sequence of interest already cloned in the entry clone. A rice GOS2 promoter (SEQ ID NO: 115) for constitutive specific expression was located upstream of this Gateway cassette.

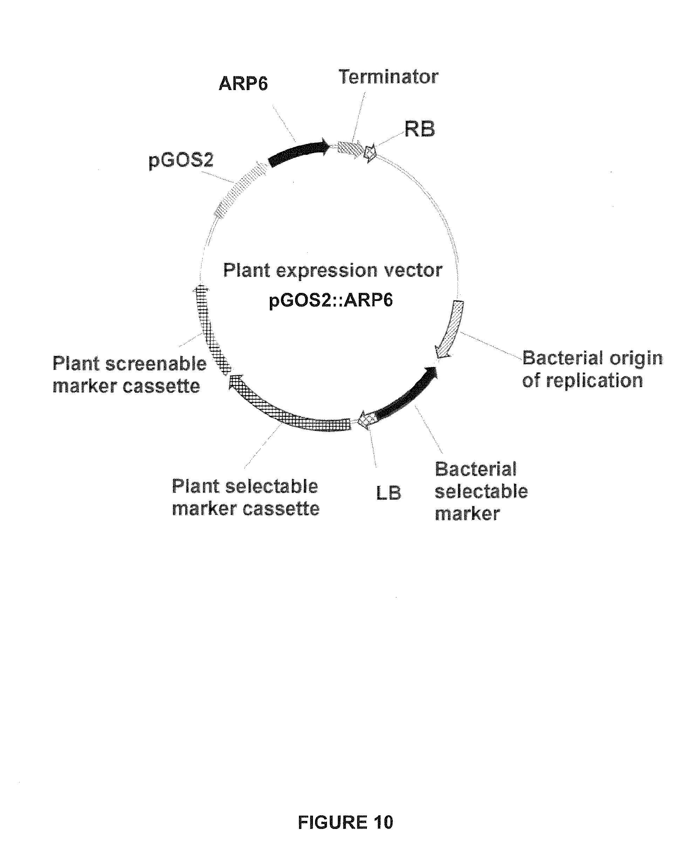

After the LR recombination step, the resulting expression vector pGOS2::ARP6 (Figure 10) was transformed into Agrobacterium strain LBA4044 according to methods well known in the art.

### 8.6. POP polypeptides

The nucleic acid sequence used in the methods of the invention was amplified by PCR using as template a custom-made Arabidopsis thaliana seedlings cDNA library (in pCMV Sport 6.0; Invitrogen, Paisley, UK). PCR was performed using Hifi Taq DNA polymerase in standard conditions, using 200 ng of template in a 50 µl PCR mix. The primers used were prm05611 (SEQ ID NO: 132; sense, start codon in bold): 5'-ggggacaagtttgtacaaaaaagcaggc ttaaacaatggcggataaggacgtt-3' and prm05612 (SEQ ID NO: 133; reverse, complementary): 5'-ggggaccactttgtacaagaaagctgggtaagcaacaacaggttctg tga-3', which include the AttB sites for Gateway recombination. The amplified PCR fragment was purified also using standard methods. The first step of the Gateway procedure, the BP reaction, was then performed, during which the PCR fragment recombined in vivo with the pDONR201 plasmid to produce, according to the Gateway terminology, an "entry clone", pPOP. Plasmid pDONR201 was purchased from Invitrogen, as part of the Gateway^{®} technology.

The entry clone comprising SEQ ID NO: 116 was then used in an LR reaction with a destination vector used for Oryza sativa transformation. This vector contained as functional elements within the T-DNA borders: a plant selectable marker; a screenable marker expression cassette; and a Gateway cassette intended for LR in vivo recombination with the nucleic acid sequence of interest already cloned in the entry clone. A rice GOS2 promoter (SEQ ID NO: 131) for constitutive specific expression was located upstream of this Gateway cassette.

After the LR recombination step, the resulting expression vector pGOS2::POP (Figure 13) was transformed into Agrobacterium strain LBA4044 according to methods well known in the art.

### 8.7. Crumpled Leaf (CRL) polypeptides

The nucleic acid sequence used in the methods of the invention was amplified by PCR using as template a custom-made Arabidopsis thaliana seedlings cDNA library (in pCMV Sport 6.0; Invitrogen, Paisley, UK). PCR was performed using Hifi Taq DNA polymerase in standard conditions, using 200 ng of template in a 50 µl PCR mix. The primers used were a primer as represented by (SEQ ID NO: 211; sense, start codon in bold): 5'-ggggacaagtttgta caaaaaagcaggcttaaacaatgggtaccgagtcggg-3' and a primer as represented by SEQ ID NO: 212; reverse, complementary): 5'-ggggaccactttgtacaagaaagctgggt tcagacaatagaaaagggggt-3', which include the AttB sites for Gateway recombination. The amplified PCR fragment was purified also using standard methods. The first step of the Gateway procedure, the BP reaction, was then performed, during which the PCR fragment recombined in vivo with the pDONR201 plasmid to produce, according to the Gateway terminology, an "entry clone", pCRL. Plasmid pDONR201 was purchased from Invitrogen, as part of the Gateway® technology.

The entry clone comprising SEQ ID NO: 155 was then used in an LR reaction with a destination vector used for Oryza sativa transformation. This vector contained as functional elements within the T-DNA borders: a plant selectable marker; a screenable marker expression cassette; and a Gateway cassette intended for LR in vivo recombination with the nucleic acid sequence of interest already cloned in the entry clone. A rice GOS2 promoter (SEQ ID NO: 213) for constitutive specific expression was located upstream of this Gateway cassette.

After the LR recombination step, the resulting expression vector pGOS2::CRL (Figure 3) was transformed into Agrobacterium strain LBA4044 according to methods well known in the art.

### Example 9: Plant transformation

### Rice transformation

The Agrobacterium containing the expression vector is used to transform Oryza sativa plants. Mature dry seeds of the rice japonica cultivar Nipponbare are dehusked. Sterilization is carried out by incubating for one minute in 70% ethanol, followed by 30 minutes in 0.2% HgCl₂, followed by a 6 times 15 minutes wash with sterile distilled water. The sterile seeds are then germinated on a medium containing 2,4-D (callus induction medium). After incubation in the dark for four weeks, embryogenic, scutellum-derived calli are excised and propagated on the same medium. After two weeks, the calli are multiplied or propagated by subculture on the same medium for another 2 weeks. Embryogenic callus pieces are sub-cultured on fresh medium 3 days before co-cultivation (to boost cell division activity).

Agrobacterium strain LBA4404 containing the expression vector is used for co-cultivation. Agrobacterium is inoculated on AB medium with the appropriate antibiotics and cultured for 3 days at 28°C. The bacteria are then collected and suspended in liquid co-cultivation medium to a density (OD₆₀₀) of about 1. The suspension is then transferred to a Petri dish and the calli immersed in the suspension for 15 minutes. Callus tissue is then blotted dry on a filter paper and transferred to solidified, co-cultivation medium and incubated for 3 days in the dark at 25°C. Co-cultivated calli are grown on 2,4-D-containing medium for 4 weeks in the dark at 28°C in the presence of a selection agent. During this period, rapidly growing resistant callus islands developed. After transfer of this material to a regeneration medium and incubation in the light, the embryogenic potential is released and shoots developed in the next four to five weeks. Shoots are excised from the calli and incubated for 2 to 3 weeks on an auxin-containing medium from which they are transferred to soil. Hardened shoots are grown under high humidity and short days in a greenhouse. Approximately 35 independent T0 rice transformants are generated for one construct. The primary transformants are transferred from a tissue culture chamber to a greenhouse. After a quantitative PCR analysis to verify copy number of the T-DNA insert, only single copy transgenic plants that exhibit tolerance to the selection agent are kept for harvest of T1 seed. Seeds are then harvested three to five months after transplanting. See Aldemita and Hodges1996, Chan et al. 1993, Hiei et al. 1994.

### Corn transformation

Transformation of maize (Zea mays) is performed with a modification of the method described by Ishida et al. (1996) Nature Biotech 14(6): 745-50. Transformation is genotype-dependent in corn and only specific genotypes are amenable to transformation and regeneration. The inbred line A188 (University of Minnesota) or hybrids with A188 as a parent are good sources of donor material for transformation, but other genotypes can be used successfully as well. Ears are harvested from corn plant approximately 11 days after pollination (DAP) when the length of the immature embryo is about 1 to 1.2 mm. Immature embryos are cocultivated with Agrobacterium tumefaciens containing the expression vector, and transgenic plants are recovered through organogenesis. Excised embryos are grown on callus induction medium, then maize regeneration medium, containing the selection agent (for example imidazolinone but various selection markers can be used). The Petri plates are incubated in the light at 25 °C for 2-3 weeks, or until shoots develop. The green shoots are transferred from each embryo to maize rooting medium and incubated at 25 °C for 2-3 weeks, until roots develop. The rooted shoots are transplanted to soil in the greenhouse. T1 seeds are produced from plants that exhibit tolerance to the selection agent and that contain a single copy of the T-DNA insert.

### Wheat transformation

Transformation of wheat is performed with the method described by Ishida et al. (1996) Nature Biotech 14(6): 745-50. The cultivar Bobwhite (available from CIMMYT, Mexico) is commonly used in transformation. Immature embryos are co-cultivated with Agrobacterium tumefaciens containing the expression vector, and transgenic plants are recovered through organogenesis. After incubation with Agrobacterium, the embryos are grown in vitro on callus induction medium, then regeneration medium, containing the selection agent (for example imidazolinone but various selection markers can be used). The Petri plates are incubated in the light at 25 °C for 2-3 weeks, or until shoots develop. The green shoots are transferred from each embryo to rooting medium and incubated at 25 °C for 2-3 weeks, until roots develop. The rooted shoots are transplanted to soil in the greenhouse. T1 seeds are produced from plants that exhibit tolerance to the selection agent and that contain a single copy of the T-DNA insert.

### Soybean transformation

Soybean is transformed according to a modification of the method described in the Texas A&M patent US 5,164,310. Several commercial soybean varieties are amenable to transformation by this method. The cultivar Jack (available from the Illinois Seed foundation) is commonly used for transformation. Soybean seeds are sterilised for in vitro sowing. The hypocotyl, the radicle and one cotyledon are excised from seven-day old young seedlings. The epicotyl and the remaining cotyledon are further grown to develop axillary nodes. These axillary nodes are excised and incubated with Agrobacterium tumefaciens containing the expression vector. After the cocultivation treatment, the explants are washed and transferred to selection media. Regenerated shoots are excised and placed on a shoot elongation medium. Shoots no longer than 1 cm are placed on rooting medium until roots develop. The rooted shoots are transplanted to soil in the greenhouse. T1 seeds are produced from plants that exhibit tolerance to the selection agent and that contain a single copy of the T-DNA insert.

### Rapeseed/canola transformation

Cotyledonary petioles and hypocotyls of 5-6 day old young seedling are used as explants for tissue culture and transformed according to Babic et al. (1998, Plant Cell Rep 17: 183-188). The commercial cultivar Westar (Agriculture Canada) is the standard variety used for transformation, but other varieties can also be used. Canola seeds are surface-sterilized for in vitro sowing. The cotyledon petiole explants with the cotyledon attached are excised from the in vitro seedlings, and inoculated with Agrobacterium (containing the expression vector) by dipping the cut end of the petiole explant into the bacterial suspension. The explants are then cultured for 2 days on MSBAP-3 medium containing 3 mg/l BAP, 3 % sucrose, 0.7 % Phytagar at 23 °C, 16 hr light. After two days of co-cultivation with Agrobacterium, the petiole explants are transferred to MSBAP-3 medium containing 3 mg/l BAP, cefotaxime, carbenicillin, or timentin (300 mg/l) for 7 days, and then cultured on MSBAP-3 medium with cefotaxime, carbenicillin, or timentin and selection agent until shoot regeneration. When the shoots are 5 - 10 mm in length, they are cut and transferred to shoot elongation medium (MSBAP-0.5, containing 0.5 mg/l BAP). Shoots of about 2 cm in length are transferred to the rooting medium (MS0) for root induction. The rooted shoots are transplanted to soil in the greenhouse. T1 seeds are produced from plants that exhibit tolerance to the selection agent and that contain a single copy of the T-DNA insert.

### Alfalfa transformation

A regenerating clone of alfalfa (Medicago sativa) is transformed using the method of (McKersie et al., 1999 Plant Physiol 119: 839-847). Regeneration and transformation of alfalfa is genotype dependent and therefore a regenerating plant is required. Methods to obtain regenerating plants have been described. For example, these can be selected from the cultivar Rangelander (Agriculture Canada) or any other commercial alfalfa variety as described by Brown DCW and A Atanassov (1985. Plant Cell Tissue Organ Culture 4: 111-112). Alternatively, the RA3 variety (University of Wisconsin) has been selected for use in tissue culture (Walker et al., 1978 Am J Bot 65:654-659). Petiole explants are cocultivated with an overnight culture of Agrobacterium tumefaciens C58C1 pMP90 (McKersie et al., 1999 Plant Physiol 119: 839-847) or LBA4404 containing the expression vector. The explants are cocultivated for 3 d in the dark on SH induction medium containing 288 mg/ L Pro, 53 mg/ L thioproline, 4.35 g/ L K2SO4, and 100 µm acetosyringinone. The explants are washed in half-strength Murashige-Skoog medium (Murashige and Skoog, 1962) and plated on the same SH induction medium without acetosyringinone but with a suitable selection agent and suitable antibiotic to inhibit Agrobacterium growth. After several weeks, somatic embryos are transferred to BOi2Y development medium containing no growth regulators, no antibiotics, and 50 g/ L sucrose. Somatic embryos are subsequently germinated on half-strength Murashige-Skoog medium. Rooted seedlings were transplanted into pots and grown in a greenhouse. T1 seeds are produced from plants that exhibit tolerance to the selection agent and that contain a single copy of the T-DNA insert.

### Cotton transformation

Cotton is transformed using Agrobacterium tumefaciens according to the method described in US 5,159,135. Cotton seeds are surface sterilised in 3% sodium hypochlorite solution during 20 minutes and washed in distilled water with 500 µg/ml cefotaxime. The seeds are then transferred to SH-medium with 50µg/ml benomyl for germination. Hypocotyls of 4 to 6 days old seedlings are removed, cut into 0.5 cm pieces and are placed on 0.8% agar. An Agrobacterium suspension (approx. 108 cells per ml, diluted from an overnight culture transformed with the gene of interest and suitable selection markers) is used for inoculation of the hypocotyl explants. After 3 days at room temperature and lighting, the tissues are transferred to a solid medium (1.6 g/l Gelrite) with Murashige and Skoog salts with B5 vitamins (Gamborg et al., Exp. Cell Res. 50:151-158 (1968)), 0.1 mg/l 2,4-D, 0.1 mg/l 6-furfurylaminopurine and 750 µg/ml MgCL2, and with 50 to 100 µg/ml cefotaxime and 400-500 µg/ml carbenicillin to kill residual bacteria. Individual cell lines are isolated after two to three months (with subcultures every four to six weeks) and are further cultivated on selective medium for tissue amplification (30°C, 16 hr photoperiod). Transformed tissues are subsequently further cultivated on non-selective medium during 2 to 3 months to give rise to somatic embryos. Healthy looking embryos of at least 4 mm length are transferred to tubes with SH medium in fine vermiculite, supplemented with 0.1 mg/l indole acetic acid, 6 furfurylaminopurine and gibberellic acid. The embryos are cultivated at 30°C with a photoperiod of 16 hrs, and plantlets at the 2 to 3 leaf stage are transferred to pots with vermiculite and nutrients. The plants are hardened and subsequently moved to the greenhouse for further cultivation.

### Example 10: Phenotypic evaluation procedure

### 10.1 Evaluation setup

Approximately 35 independent T0 rice transformants are generated. The primary transformants are transferred from a tissue culture chamber to a greenhouse for growing and harvest of T1 seed. Six events, of which the T1 progeny segregated 3:1 for presence/absence of the transgene, are retained. For each of these events, approximately 10 T1 seedlings containing the transgene (hetero- and homo-zygotes) and approximately 10 T1 seedlings lacking the transgene (nullizygotes) are selected by monitoring visual marker expression. The transgenic plants and the corresponding nullizygotes are grown side-by-side at random positions. Greenhouse conditions are for shorts days (12 hours light), 28°C in the light and 22°C in the dark, and a relative humidity of 70%.

Four T1 events were further evaluated in the T2 generation following the same evaluation procedure as for the T1 generation but with more individuals per event. From the stage of sowing until the stage of maturity the plants are passed several times through a digital imaging cabinet. At each time point digital images (2048x1536 pixels, 16 million colours) are taken of each plant from at least 6 different angles.

### Drought screen

Plants from T2 seeds are grown in potting soil under normal conditions until they approached the heading stage. They are then transferred to a "dry" section where irrigation is withheld. Humidity probes are inserted in randomly chosen pots to monitor the soil water content (SWC). When SWC goes below certain thresholds, the plants are automatically re-watered continuously until a normal level is reached again. The plants are then retransferred again to normal conditions. The rest of the cultivation (plant maturation, seed harvest) is the same as for plants not grown under abiotic stress conditions. Growth and yield parameters are recorded as detailed for growth under normal conditions.

### Nitrogen use efficiency screen

Rice plants from T2 seeds are grown in potting soil under normal conditions except for the nutrient solution. The pots are watered from transplantation to maturation with a specific nutrient solution containing reduced N nitrogen (N) content, usually between 7 to 8 times less. The rest of the cultivation (plant maturation, seed harvest) is the same as for plants not grown under abiotic stress. Growth and yield parameters are recorded as detailed for growth under normal conditions.

### Salt stress screen

Plants are grown on a substrate made of coco fibers and argex (3 to 1 ratio). A normal nutrient solution is used during the first two weeks after transplanting the plantlets in the greenhouse. After the first two weeks, 25 mM of salt (NaCl) is added to the nutrient solution, until the plants are harvested. Seed-related parameters are then measured.

### 10.2 Statistical analysis: F test

A two factor ANOVA (analysis of variants) was used as a statistical model for the overall evaluation of plant phenotypic characteristics. An F test was carried out on all the parameters measured of all the plants of all the events transformed with the gene of the present invention. The F test was carried out to check for an effect of the gene over all the transformation events and to verify for an overall effect of the gene, also known as a global gene effect. The threshold for significance for a true global gene effect was set at a 5% probability level for the F test. A significant F test value points to a gene effect, meaning that it is not only the mere presence or position of the gene that is causing the differences in phenotype.

Because two experiments with overlapping events were carried out, a combined analysis was performed. This is useful to check consistency of the effects over the two experiments, and if this is the case, to accumulate evidence from both experiments in order to increase confidence in the conclusion. The method used was a mixed-model approach that takes into account the multilevel structure of the data (i.e. experiment - event - segregants). P values were obtained by comparing likelihood ratio test to chi square distributions.

### 10.3 Parameters measured

### Biomass-related parameter measurement

From the stage of sowing until the stage of maturity the plants were passed several times through a digital imaging cabinet. At each time point digital images (2048x1536 pixels, 16 million colours) were taken of each plant from at least 6 different angles.

The plant aboveground area (or leafy biomass) was determined by counting the total number of pixels on the digital images from aboveground plant parts discriminated from the background. This value was averaged for the pictures taken on the same time point from the different angles and was converted to a physical surface value expressed in square mm by calibration. Experiments show that the aboveground plant area measured this way correlates with the biomass of plant parts above ground. The above ground area is the area measured at the time point at which the plant had reached its maximal leafy biomass. The early vigour is the plant (seedling) aboveground area three weeks post-germination. Increase in root biomass is expressed as an increase in total root biomass (measured as maximum biomass of roots observed during the lifespan of a plant); or as an increase in the root/shoot index (measured as the ratio between root mass and shoot mass in the period of active growth of root and shoot).

Early vigour was determined by counting the total number of pixels from aboveground plant parts discriminated from the background. This value was averaged for the pictures taken on the same time point from different angles and was converted to a physical surface value expressed in square mm by calibration. The results described below are for plants three weeks post-germination.

Determination of the start of flowering was as described in WO 2007/093444

### Seed-related parameter measurements

The mature primary panicles were harvested, counted, bagged, barcode-labelled and then dried for three days in an oven at 37°C. The panicles were then threshed and all the seeds were collected and counted. The filled husks were separated from the empty ones using an air-blowing device. The empty husks were discarded and the remaining fraction was counted again. The filled husks were weighed on an analytical balance. The number of filled seeds was determined by counting the number of filled husks that remained after the separation step. The total seed weight per plant was measured by weighing all filled husks harvested from one plant. Total seed number per plant was measured by counting the number of husks harvested from a plant. Thousand Kernel Weight (TKW) is extrapolated from the number of filled seeds counted and their total weight. The Harvest Index (HI) in the present invention is defined as the ratio between the total seed weight per plant and the above ground area (mm²), multiplied by a factor 10⁶. The total number of flowers per panicle as defined in the present invention is the ratio between the total number of seeds and the number of mature primary panicles. The seed fill rate as defined in the present invention is the proportion (expressed as a %) of the number of filled seeds over the total number of seeds (or florets).

### Examples 11: Results of the phenotypic evaluation of the transgenic plants

### 11.1. Brevis Radix-like (BRXL) polypeptides

The results of the evaluation of T1 generation transgenic rice plants expressing the nucleic acid sequence encoding a BRXL polypeptide as represented by SEQ ID NO: 18, under the control of a constitutive promoter, are presented in Table D below.

There was a significant increase in plant height, and Thousand Kernel Weight (TKW), of the transgenic plants relative to the controls.

**Table D1: Results of the evaluation of T1 generation transgenic rice plants expressing the nucleic acid sequence encoding a BRXL polypeptide as represented by SEQ ID NO: 18, under the control of a promoter for constitutive expression.**

| Trait | Overall average % increase in 6 events in the T1 generation |
|---|---|
| Plant height | 4% |
| Thousand kernel weight | 7% |

### 11.2. ARP6 polypeptides

The results of the evaluation of transgenic rice plants in the T2 generation and expressing a nucleic acid comprising the longest Open Reading Frame in SEQ ID NO: 101 under non-stress conditions are presented below. See previous Examples for details on the generations of the transgenic plants.

The results of the evaluation of transgenic rice plants under non-stress conditions are presented below. An increase of at least 5 % was observed for total seed yield (totalwgseeds), number of filled seeds (nrfilledseed), fill rate (fillrate), and harvest index (harvestindex).

**Table D2:**

| Yield-related trait | % increase in transgenic plant compared to control plant |
|---|---|
| totalwgseeds | 12.0 |
| fillrate | 13.2 |
| harvestindex | 13.0 |
| nrfilledseed | 9.8 |

### 11.3. POP polypeptides

The results of the evaluation of transgenic rice plants in the T2 generation and expressing a nucleic acid encoding the POP polypeptide of SEQ ID NO: 117 under non-stress conditions are presented below in Table D3. When grown under non-stress conditions, an increase of at least 5 % was observed for aboveground biomass (AreaMax), root biomass (RootMax and RootThickMax), and for seed yield (total weight of seeds, number of filled seeds, fill rate, harvest index). In addition, plants expressing a POP nucleic acid showed a faster growth rate (a shorter time (in days) needed between sowing and the day the plant reaches 90 % of its final biomass (AreaCycle) and an earlier start of flowering (TimetoFlower: time (in days) between sowing and the emergence of the first panicle).

**Table D3: Data summary for transgenic rice plants; for each parameter, the overall percent increase is shown for the confirmation (T2 generation), for each parameter the p-value is <0.05.**

| Parameter | Overall increase |
|---|---|
| AreaMax | 11.0 |
| TimetoFlower | 7.6 |
| RootMax | 7.9 |
| totalwgseeds | 35.0 |
| nrfilledseed | 30.4 |
| fillrate | 31.3 |
| harvestindex | 21.0 |
| AreaCycl | 5.1 |
| RootThickMax | 12.6 |

### 11.4. Crumpled Leaf (CRL) polypeptides

The results of the evaluation of transgenic rice plants in the T2 generation and expressing a nucleic acid comprising the longest Open Reading Frame in SEQ ID NO: 155 evaluated under non-stress conditions are presented below. See previous Examples for details on the generations of the transgenic plants.

The results of the evaluation of transgenic rice plants under non-stress conditions are presented below. An increase of at least 5 % was observed for total seed yield (Totalwgseeds), number of filled seeds (nrfilledseed), fill rate (fillrate), and harvest index (harvestindex).

**Table D4.**

| Parameter | % increase in transgenic compared to control plants |
|---|---|
| Totalwgseeds | 17.4 |
| nrfilledseed | 15.5 |
| fillrate | 17.7 |
| harvestindex | 15.9 |

## Claims

1. A method for enhancing yield-related traits in plants relative to control plants, comprising modulating expression in a plant of a nucleic acid encoding an ARP6 polypeptide.

2. Method according to claim 1, wherein said ARP6 polypeptide has at least 30% overall sequence identity to the amino acid represented by any one of SEQ ID NO: 102, SEQ ID NO: 104 , SEQ ID NO: 106, SEQ ID NO: 108, SEQ ID NO: 110, and SEQ ID NO: 112.

3. Method according to claim 1 or 2, wherein said nucleic acid encoding an ARP6 polypeptide encodes any one of the proteins listed in Table A5 or is a portion of such a nucleic acid, or a nucleic acid capable of hybridising with such a nucleic acid; and/or wherein said nucleic acid sequence encodes an orthologue or paralogue of any of the proteins given in Table A5.

4. Method according to any preceding claim, wherein said modulated expression is effected by introducing and expressing in a plant a nucleic acid encoding an ARP6 polypeptide.

5. Method according to any preceding claim, wherein said enhanced yield-related traits comprise increased yield, preferably increased biomass and/or increased seed yield relative to control plants.

6. Method according to any one of claims 1 to 5, wherein said enhanced yield-related traits are obtained under non-stress conditions or under conditions of drought stress, salt stress or nitrogen deficiency.

7. Method according to any one of claims 4 to 6, wherein said nucleic acid is operably linked to a constitutive promoter, preferably to a GOS2 promoter, most preferably to a GOS2 promoter from rice.

8. Method according to any one of claims 1 to 7, wherein said nucleic acid encoding an ARP6 polypeptide is of plant origin, preferably from a dicotyledonous plant, further preferably from the family *Brassicaceae,* more preferably from the genus *Arabidopsis,* most preferably from *Arabidopsis thaliana.*

9. Construct comprising:
(i) nucleic acid encoding an ARP6 polypeptide as defined in any of claims 1 to 3;
(ii) one or more control sequences capable of driving expression of the nucleic acid sequence of (a); and optionally
(iii) a transcription termination sequence.

10. Construct according to claim 9, wherein one of said control sequences is a constitutive promoter, preferably a GOS2 promoter, most preferably a GOS2 promoter from rice.

11. Method for the production of a transgenic plant having increased yield, particularly increased biomass and/or increased seed yield relative to control plants, comprising:
(i) introducing and expressing in a plant a nucleic acid encoding an ARP6 polypeptide as defined in any of claims 1 to 3; and
(ii) cultivating the plant cell under conditions promoting plant growth and development.

12. Plant or part thereof, including seeds, obtainable by a method according to any one of claims 1 to 8 and 11, wherein said plant or part thereof comprises a recombinant nucleic acid encoding an ARP6 polypeptide; or plant, plant part or plant cell transformed with a construct according to claim 9 or 10; or transgenic plant having increased yield, particularly increased biomass and/or increased seed yield, relative to control plants, resulting from modulated expression of a nucleic acid encoding an ARP6 polypeptide as defined in any of claims 1 to 3, or a transgenic plant cell derived from said transgenic plant.

13. Transgenic plant according to claim 12, or a transgenic plant cell derived therefrom, wherein said plant is a crop plant or a monocot or a cereal, such as rice, maize, wheat, barley, millet, rye, triticale, sorghum emmer, spelt, secale, einkorn, teff, milo and oats.

14. Harvestable parts of a plant according to claim 12 or 13, wherein said harvestable parts are preferably shoot biomass and/or seeds and/or products derived from a plant according to claim 12 or 13 and/or from harvestable parts of said plant.

15. Use of a nucleic acid encoding an ARP6 polypeptide as defined in any of claims 1 to 3 in increasing yield, particularly in increasing seed yield and/or shoot biomass in plants, relative to control plants; or use of a construct according to claim 9 or 10 in a method for making plants having increased yield, particularly increased biomass and/or increased seed yield relative to control plants.
